# EUROPEAN PATENT APPLICATION

(11) **EP 4 813 984 A1**
(43) Date of publication of application: **30.09.2026**
(21) Application number: 24893516.5
(22) Date of filing: 21.11.2024
(51) Int. Cl.: C07D 471/04, A61K 31/437, A61K 31/444, A61P 3/04, A61P 3/06, A61P 3/10

(54) **HETEROCYCLIC COMPOUND, AND PREPARATION METHOD THEREFOR AND USE THEREOF IN MEDICINE**

(30) Priority: 21.11.2023 CN 202311556992; 12.01.2024 CN 202410048709; 31.01.2024 CN 202410135819; 28.03.2024 CN 202410367539; 23.04.2024 CN 202410493077; 06.06.2024 CN 202410729135; 16.07.2024 CN 202410952072
(71) Applicant: Jiangsu Hengrui Pharmaceuticals Co., Ltd., Lianyungang, Jiangsu 222047 (CN); Shanghai Hengrui Pharmaceutical Co., Ltd., Shanghai 200245 (CN)
(72) Inventor: DONG, Ping, Shanghai 200245 (CN); LI, Xin, Shanghai 200245 (CN); SHU, Chunfeng, Shanghai 200245 (CN); ZHANG, Jing, Shanghai 200245 (CN); CAO, Hu, Shanghai 200245 (CN); FEI, Hongbo, Shanghai 200245 (CN); HE, Feng, Shanghai 200245 (CN)
(74) Representative: Dragotti & Associati S.P.A.
(86) International application number: PCT/CN2024/133436
(87) International publication number: WO 2025/108361

(57) **Abstract**

The present invention relates to a heterocyclic compound, and a preparation method therefor and a use thereof in medicine. In particular, the present invention relates to a heterocyclic compound represented by general formula (I), a preparation method therefor, a pharmaceutical composition containing the compound, and a use thereof as a therapeutic agent, particularly as a GLP-1 receptor agonist. The definition of each group in the general formula (I) is as stated in the description.

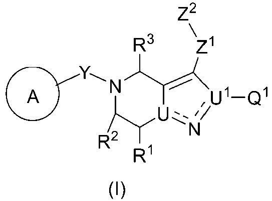

## Description

### Technical Field

The present invention belongs to the field of medicine and relates to a heterocyclic compound, a preparation method therefor, and the use thereof in medicine. Specifically, the present invention relates to a heterocyclic compound represented by general formula (I), a preparation method therefor, a pharmaceutical composition containing the compound, and the use thereof as a GLP-1 receptor agonist.

### Background Art

Diabetes is a metabolic disease with multiple causes, which is characterized by chronic hyperglycemia, accompanied by sugar, lipid and protein metabolism disorders caused by insulin secretion or action defects. Diabetes is a very ancient disease, which is caused by the absolute or relative deficiency of insulin in the human body, leading to elevated blood glucose concentrations and thus excessive glucose excretion via urine, and presents with symptoms such as polydipsia, polyuria, polyphagia, and emaciation.

Generally, there are two types of diabetes. Patients with type I diabetes, i.e., patients with insulin-dependent diabetes, produce little or no insulin by themselves. Insulin is a hormone used in the body to regulate glucose utilization. Patients with type II diabetes, i.e., patients with insulin-independent diabetes, have the same or higher plasma insulin levels as non-diabetic patients. However, such patients develop resistance to insulin, which stimulates glucose and lipid metabolism in major insulin-sensitive tissues and cells, such as muscle, liver, and adipose tissues. The significant resistance to insulin in the patient cannot be overcome even if the plasma insulin level increases.

Insulin resistance is not only caused by the decreased number of insulin receptors, but also by insulin receptor deficiencies. So far, this mechanism remains incompletely elucidated. Insulin resistance leads to the failure of insulin to activate glucose uptake, oxidation, and storage in muscle tissues, and to effectively inhibit lipolysis in adipose tissues as well as hepatic glucose production and secretion.

Glucagon-like peptide-1 (GLP-1) is an incretin hormone secreted by L-cells in the lower digestive tract. GLP-1 plays a corresponding role by binding to its ubiquitous specific receptor. Organs in which GLP-1 receptor is now clearly present include islet cells, gastrointestinal, pulmonary, brain, kidney, hypothalamus and cardiovascular systems, and GLP-1 receptor may also be present in liver, adipose tissues and skeletal muscle. GLP-1 not only acts on β cells to promote insulin secretion, but also acts on α cells to inhibit glucagon secretion. There is generally no significant difference in serum GLP-1 levels in patients with normal glucose tolerance, impaired glucose tolerance, and type II diabetes. However, there is a deficiency of the response of β cells to GLP-1 after eating, and under certain conditions, the response is significantly enhanced after continuous infusion of GLP-1. Since the duration of action of human GLP-1 is very short (t1/2 < 1.5 minutes via intravenous injection), endogenous human GLP-1 is not suitable for clinical treatment of diabetes.

Peptidic GLP-1 receptor agonists (e.g., liraglutide and exenatide) have the effect of lowering fasting and postprandial glucose and improving blood glucose level in patients with type II diabetes. However, since the peptidic GLP-1 has poor oral bioavailability and is inconvenient to take, small molecule agonists of GLP-1 receptors with good oral bioavailability are highly desirable.

Patent applications disclosing GLP-1 receptor small molecule agonists include WO 2018056453 A1, JP 2019099571 A, WO 2021155841 A1, WO 2022017338 A1, WO 2022048665 A1, WO 2022052958 A1, WO 2022127868 A1, WO 2022246230 A1, WO 2022258805 A1, WO 2023016546 A1, WO 2023038039 A1, WO 2023169456 A1, etc.

### Summary of the Invention

An object of the present invention is to provide a compound represented by general formula (I) or a pharmaceutically acceptable salt thereof: 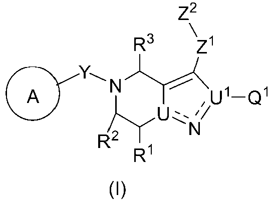 wherein: 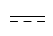
is a single bond or a double bond;
Q¹ is aryl or heteroaryl, and the aryl and heteroaryl are each independently and optionally substituted with one or more R^{Q1};
Z² is selected from the group consisting of a hydrogen atom, a deuterium atom, cycloalkyl, heterocyclyl, 6- to 10-membered aryl, and 5- to 10-membered heteroaryl, wherein the cycloalkyl, heterocyclyl, 6- to 10-membered aryl, and 5- to 10-membered heteroaryl are each independently and optionally substituted with one or more R^{Z2};
Z¹ is selected from the group consisting of a bond, -heterocyclyl-, - heterocyclyl-L^{2A}-, -L^{2A}-heterocyclyl-, -L^{2A}-heterocyclyl-L^{2A}-, -heteroaryl-L^{2A-}, - L^{2A}-heteroaryl-L^{2A}-, -N=, -NR^{aa}-L^{2A}-, and -NR^{aa}-C(O)-NR^{bb}-L^{2A}-, wherein the heterocyclyl and heteroaryl are each independently and optionally substituted with one or more R^{Z1};
L^{2A} is selected from the group consisting of a bond, alkyl, deuterioalkyl, heteroalkyl, and -N=; 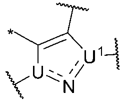 is 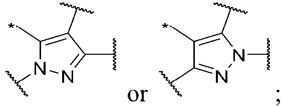
Y is selected from the group consisting of C(O), S(O), S(O)₂, deuterioalkylene, and alkylene;
R¹, R², and R³ are the same or different and are each independently selected from the group consisting of a hydrogen atom, a deuterium atom, halogen, alkyl, alkenyl, alkynyl, alkoxy, alkoxyalkyl, haloalkyl, haloalkoxy, hydroxyalkyl, cyano, amino, nitro, hydroxyl, oxo, -(CH₂)ₓNR^{11a}R^{11b}, -(CH₂)ₓC(O)NR^{11a}R^{11b}, - NR⁰⁰C(O)R¹², -C(O)R¹², -C(O)OR¹², -OC(O)R¹², -S(O)ₜR¹², -S(O)ₜNR^{11a}R^{11b}, - NR⁰⁰S(O)ₜR¹², -(CH₂)ₓP(O)R¹²R¹³, -S(O)ₜOR¹², -OS(O)ₜR¹², -OR¹², =CR^{12a}R^{13a}, =NR⁰⁰, C(=NR⁰⁰)R¹², S(=NR⁰⁰)(O)R¹², S(=NR⁰⁰)R¹², cycloalkyl, cycloalkylalkyl, heterocyclyl, heterocyclylalkyl, aryl, and heteroaryl; the alkyl, alkenyl, alkynyl, alkoxy, cycloalkyl, cycloalkylalkyl, heterocyclyl, heterocyclylalkyl, aryl, and heteroaryl are each independently and optionally substituted with one or more R⁰¹;
ring A is selected from the group consisting of 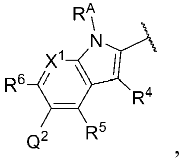 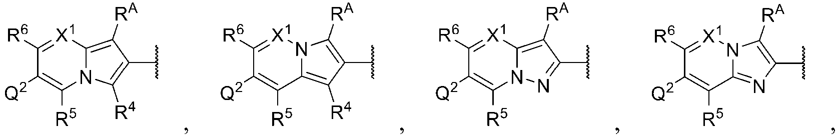 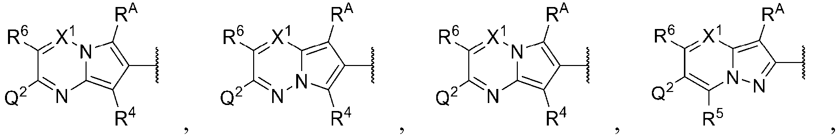 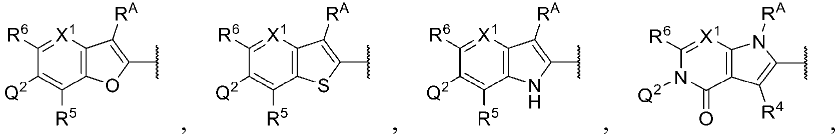 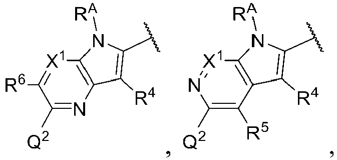 and bicyclic heteroaryl substituted with R^{A} and Q²; the bicyclic heteroaryl is optionally substituted with one or more R⁰¹;
R^{A} is 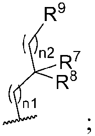
Q² is selected from the group consisting of cycloalkyl, heterocyclyl, aryl, and heteroaryl, wherein the cycloalkyl, heterocyclyl, aryl, and heteroaryl are each independently and optionally substituted with one or more R^{Q2};
X¹ is CR^{x1} or N;
R⁴, R⁵, R⁶, and R^{x1}, when present, are the same or different and are each independently selected from the group consisting of a hydrogen atom, a deuterium atom, halogen, alkyl, alkenyl, alkynyl, alkoxy, alkoxyalkyl, haloalkyl, haloalkoxy, hydroxyalkyl, cyano, amino, nitro, hydroxyl, -(CH₂)ₓNR^{11a}R^{11b}, -NR⁰⁰C(O)R¹², - C(O)R¹², -C(O)OR¹², -(CH₂)ₓC(O)NR^{11a}R^{11b}, -OC(O)R¹², -S(O)ₜR¹², - S(O)ₜNR^{11a}R^{11b}, -NR⁰⁰S(O)ₜR¹², -(CH₂)ₓP(O)R¹²R¹³, -S(O)ₜOR¹², -OS(O)ₜR¹², -OR¹², C(=NR⁰⁰)R¹², S(=NR⁰⁰)(O)R¹², S(=NR⁰⁰)R¹², cycloalkyl, cycloalkylalkyl, heterocyclyl, heterocyclylalkyl, aryl, and heteroaryl; the alkyl, alkenyl, alkynyl, alkoxy, cycloalkyl, cycloalkylalkyl, heterocyclyl, heterocyclylalkyl, aryl, and heteroaryl are each independently and optionally substituted with one or more R⁰¹;
or Q² and R⁶, together with the carbon atoms to which they are attached, form ring B, or Q² and R⁵, together with the carbon atoms to which they are attached, form ring B; or R^{Q2} and R⁶, together with the ring atoms to which they are attached, form ring B⁰, or R^{Q2} and R⁵, together with the ring atoms to which they are attached, form ring B⁰;
each ring B is independently selected from the group consisting of cycloalkyl, heterocyclyl, aryl, and heteroaryl, and the ring B is optionally substituted with one or more R^{B}; each ring B⁰ is independently selected from the group consisting of cycloalkyl, heterocyclyl, aryl, and heteroaryl, and the ring B⁰ is optionally substituted with one or more R^{B};
R⁹ is selected from the group consisting of -C(O)OR¹², -C(O)NR^{11a}R^{11b}, - S(O)ₜR¹², -S(O)ₜNR^{11a}R^{11b}, heterocyclyl, and heteroaryl, wherein the heterocyclyl and heteroaryl are each independently and optionally substituted with one or more R^{9a};
R⁷ and R⁸ are the same or different and are each independently selected from the group consisting of a hydrogen atom, a deuterium atom, halogen, alkyl, alkenyl, alkynyl, alkoxy, alkoxyalkyl, haloalkyl, haloalkoxy, hydroxyalkyl, cyano, amino, nitro, hydroxyl, oxo, -(CH₂)ₓNR^{11a}R^{11b}, -(CH₂)ₓC(O)NR^{11a}R^{11b}, -NR⁰⁰C(O)R¹², - C(O)R¹², -C(O)OR¹², -OC(O)R¹², -S(O)ₜR¹², -S(O)ₜNR^{11a}R^{11b}, -NR⁰⁰S(O)ₜR¹², - (CH₂)ₓP(O)R¹²R¹³, -S(O)ₜOR¹², -OS(O)ₜR¹², -OR¹², =CR^{12a}R^{13a}, =NR⁰⁰, C(=NR⁰⁰)R¹², S(=NR⁰⁰)(O)R¹², S(=NR⁰⁰)R¹², cycloalkyl, cycloalkylalkyl, heterocyclyl, heterocyclylalkyl, aryl, and heteroaryl; the alkyl, alkenyl, alkynyl, alkoxy, cycloalkyl, cycloalkylalkyl, heterocyclyl, heterocyclylalkyl, aryl, and heteroaryl are each independently and optionally substituted with one or more R⁰¹; or
R⁷ and R⁸, together with the atoms to which they are attached, form 3- to 5-membered cycloalkyl or heterocyclyl, wherein the 3- to 5-membered cycloalkyl and heterocyclyl are each independently and optionally substituted with one or more R⁷⁸;
n1 is 0, 1, 2, 3, 4, or 5; n2 is 0, 1, 2, 3, 4, or 5;
R^{Q1}, R^{Z2}, R^{Z1}, R⁰¹, R^{Q2}, R^{B}, R^{9a}, and R⁷⁸ are each the same or different and are each independently selected from the group consisting of a deuterium atom, halogen, alkyl, alkenyl, alkynyl, alkoxy, haloalkyl, deuterioalkyl, haloalkoxy, hydroxyalkyl, cyano, amino, nitro, hydroxyl, oxo, =S, -(CH₂)ₓNR^{11a}R^{11b}, -NR⁰⁰C(O)R¹², -C(O)R¹², -C(O)OR¹², -OC(O)R¹², -(CH₂)ₓC(O)NR^{11a}R^{11b}, -S(O)ₜR¹², -S(O)ₜNR^{11a}R^{11b}, - NR⁰⁰S(O)ₜR¹², -(CH₂)ₓP(O)R¹²R¹³, -S(O)ₜOR¹², -OS(O)ₜR¹², -OR¹², =CR^{12a}R^{13a}, =NR⁰⁰, C(=NR⁰⁰)R¹², S(=NR⁰⁰)(O)R¹², S(=NR⁰⁰)R¹², cycloalkyl, heterocyclyl, 6- to 10-membered aryl, and 5- to 10-membered heteroaryl, wherein the alkyl, alkenyl, alkynyl, alkoxy, cycloalkyl, heterocyclyl, 6- to 10-membered aryl, and 5- to 10-membered heteroaryl are each independently and optionally substituted with one or more R*;
or R^{Z2} and R^{Z1}, together with the ring atoms to which they are attached, form ring C;
ring C is selected from the group consisting of cycloalkyl, heterocyclyl, aryl, and heteroaryl, and the ring C is optionally substituted with one or more R^{C};
R* and R^{C} are each the same or different and are each independently selected from the group consisting of a deuterium atom, halogen, alkyl, alkenyl, alkynyl, alkoxy, haloalkyl, haloalkoxy, hydroxyalkyl, cyano, amino, nitro, hydroxyl, oxo, =S, -(CH₂)ₓNR^{11a}R^{11b}, -NR⁰⁰C(O)R¹², -C(O)R¹², -C(O)OR¹², -(CH₂)ₓC(O)NR^{11a}R^{11b}, - S(O)ₜR¹², -S(O)ₜNR^{11a}R^{11b}, -NR⁰⁰S(O)ₜR¹², -(CH₂)ₓP(O)R¹²R¹³, -OC(O)R¹², - S(O)ₜOR¹², -OS(O)ₜR¹², -OR¹², =CR^{12a}R^{13a}, =NR⁰⁰, C(=NR⁰⁰)R¹², S(=NR⁰⁰)(O)R¹², S(=NR⁰⁰)R¹², cycloalkyl, heterocyclyl, aryl, and heteroaryl, wherein the alkyl, alkenyl, alkynyl, alkoxy, cycloalkyl, heterocyclyl, aryl, and heteroaryl are each independently and optionally substituted with one or more identical or different substituents selected from the group consisting of a deuterium atom, oxo, halogen, cyano, amino, hydroxyl, alkyl, haloalkyl, hydroxyalkyl, alkoxy, haloalkoxy, cycloalkyl, heterocyclyl, aryl, and heteroaryl;
R^{11a}, R^{11b}, R¹², and R¹³ are each the same or different and are each independently selected from the group consisting of a hydrogen atom, a deuterium atom, alkyl, alkoxy, hydroxyalkyl, haloalkyl, -C(O)R¹⁵, -C(O)OR¹⁵, -OC(O)R¹⁵ - (CH₂)_{y}NR^{14a}R^{14b}, -(CH₂)_{y}C(O)NR^{14a}R^{14b}, -NR⁰C(O)R¹⁵, -S(O)ₜR¹⁵, -S(O)ₜNR^{14a}R^{14b}, -NR⁰S(O)ₜR¹⁵, -S(O)ₜOR¹⁵, - OS(O)ₜR¹⁵, -OR¹⁵, 4- to 12-membered cycloalkyl, and heterocyclyl; or R^{11a} and R^{11b}, together with the nitrogen atom to which they are attached, form heterocyclyl, wherein the heterocyclyl is optionally substituted with one or more identical or different substituents selected from the group consisting of a deuterium atom, oxo, halogen, cyano, amino, hydroxyl, alkyl, haloalkyl, hydroxyalkyl, alkoxy, and haloalkoxy;
R^{aa}, R^{bb}, R⁰⁰, and R⁰ are each the same or different and are each independently selected from the group consisting of a hydrogen atom, a deuterium atom, alkyl, hydroxyalkyl, haloalkyl, C(O) alkyl, amido, cycloalkyl, and heterocyclyl;
R^{12a} and R^{13a} are each the same or different and are each independently selected from the group consisting of a hydrogen atom, a deuterium atom, halogen, cyano, amino, hydroxyl, alkyl, haloalkyl, hydroxyalkyl, alkoxy, haloalkoxy, cycloalkyl, and heterocyclyl; or R^{12a} and R^{13a}, together with the carbon atoms to which they are attached, form cycloalkyl or heterocyclyl, wherein the cycloalkyl and heterocyclyl are each independently and optionally substituted with one or more identical or different substituents selected from the group consisting of a deuterium atom, halogen, cyano, amino, hydroxyl, oxo, alkenyl, alkynyl, alkyl, haloalkyl, hydroxyalkyl, alkoxy, haloalkoxy, cycloalkyl, and heterocyclyl;
R¹⁵, R^{14a}, and R^{14b} are each the same or different and are each independently selected from the group consisting of a hydrogen atom, a deuterium atom, alkyl, alkoxy, hydroxyalkyl, haloalkyl, C(O) alkyl, amino, amido, cycloalkyl, heterocyclyl, aryl, and heteroaryl, wherein the alkyl, alkoxy, cycloalkyl, heterocyclyl, aryl, and heteroaryl are each independently and optionally substituted with one or more identical or different substituents selected from the group consisting of a deuterium atom, halogen, cyano, nitro, amino, hydroxyl, oxo, alkenyl, alkynyl, alkyl, haloalkyl, hydroxyalkyl, alkoxy, haloalkoxy, cycloalkyl, and heterocyclyl;
x is 0, 1, 2, 3, 4, 5, or 6; y is 0, 1, 2, 3, 4, 5, or 6; and
t is 0, 1, or 2.

An object of the present invention is to provide a compound represented by general formula (I) or a pharmaceutically acceptable salt thereof: 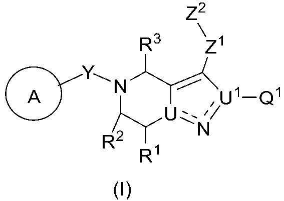 wherein: 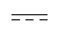
is a single bond or a double bond;
Q¹ is aryl or heteroaryl, and the aryl and heteroaryl are each independently and optionally substituted with one or more R^{Q1};
Z² is selected from the group consisting of a hydrogen atom, cycloalkyl, heterocyclyl, 6- to 10-membered aryl, and 5- to 10-membered heteroaryl, wherein the cycloalkyl, heterocyclyl, 6- to 10-membered aryl, and 5- to 10-membered heteroaryl are each independently and optionally substituted with one or more R^{Z2};
Z¹ is selected from the group consisting of a bond, -heterocyclyl-, - heterocyclyl-L^{2A}-, -L^{2A}-heterocyclyl-, -heteroaryl-L^{2A}-, -N=, -NR^{aa}-L^{2A}-, and -NR^{aa}-C(O)-NR^{bb}-L^{2A}-, wherein the heterocyclyl and heteroaryl are each independently and optionally substituted with one or more R^{Z1};
L^{2A} is selected from the group consisting of a bond, alkyl, and -N=; 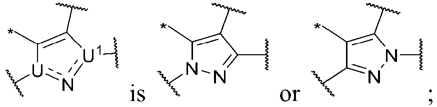
Y is selected from the group consisting of C(O), S(O), S(O)₂, and alkylene;
R¹, R², and R³ are the same or different and are each independently selected from the group consisting of a hydrogen atom, halogen, alkyl, alkenyl, alkynyl, alkoxy, alkoxyalkyl, haloalkyl, haloalkoxy, hydroxyalkyl, cyano, amino, nitro, hydroxyl, oxo, -(CH₂)ₓNR^{11a}R^{11b}, -(CH₂)ₓC(O)NR^{11a}R^{11b}, -NR⁰⁰C(O)R¹², -C(O)R¹², -C(O)OR¹², -OC(O)R¹², -S(O)ₜR¹², -S(O)ₜNR^{11a}R^{11b}, -NR⁰⁰S(O)ₜR¹², - (CH₂)ₓP(O)R¹²R¹³, -S(O)ₜOR¹², -OS(O)ₜR¹², -OR¹², =CR^{12a}R^{13a}, =NR⁰⁰, C(=NR⁰⁰)R¹², S(=NR⁰⁰)(O)R¹², cycloalkyl, cycloalkylalkyl, heterocyclyl, heterocyclylalkyl, aryl, and heteroaryl; the alkyl, alkenyl, alkynyl, alkoxy, cycloalkyl, cycloalkylalkyl, heterocyclyl, heterocyclylalkyl, aryl, and heteroaryl are each independently and optionally substituted with one or more R⁰¹;
ring A is selected from the group consisting of 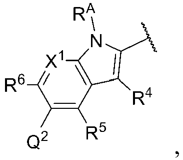 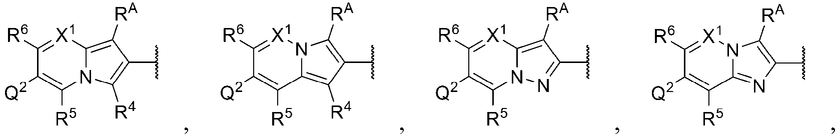 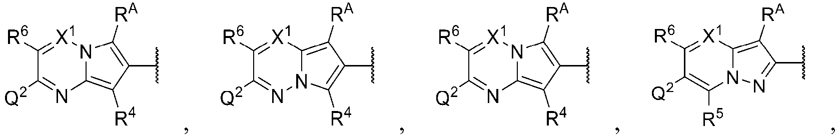 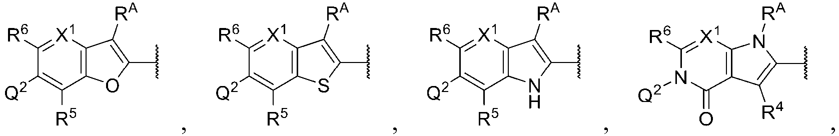 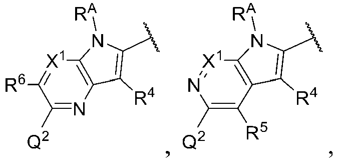 and bicyclic heteroaryl substituted with R^{A} and Q²; the bicyclic heterocyclyl is optionally substituted with one or more R⁰¹;
R^{A} is 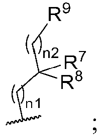
Q² is selected from the group consisting of cycloalkyl, heterocyclyl, aryl, and heteroaryl, wherein the cycloalkyl, heterocyclyl, aryl, and heteroaryl are each independently and optionally substituted with one or more R^{Q2};
X¹ is CR^{x1} or N;
R⁴, R⁵, R⁶, and R^{x1}, when present, are the same or different and are each independently selected from the group consisting of a hydrogen atom, halogen, alkyl, alkenyl, alkynyl, alkoxy, alkoxyalkyl, haloalkyl, haloalkoxy, hydroxyalkyl, cyano, amino, nitro, hydroxyl, -(CH₂)ₓNR^{11a}R^{11b}, -NR⁰⁰C(O)R¹², -C(O)R¹², -C(O)OR¹², - (CH₂)ₓC(O)NR^{11a}R^{11b}, -OC(O)R¹², -S(O)ₜR¹², -S(O)ₜNR^{11a}R^{11b}, -NR⁰⁰S(O)ₜR¹², - (CH₂)ₓP(O)R¹²R¹³, -S(O)ₜOR¹², -OS(O)ₜR¹², -OR¹², C(=NR⁰⁰)R¹², S(=NR⁰⁰)(O)R¹², cycloalkyl, cycloalkylalkyl, heterocyclyl, heterocyclylalkyl, aryl, and heteroaryl; the alkyl, alkenyl, alkynyl, alkoxy, cycloalkyl, cycloalkylalkyl, heterocyclyl, heterocyclylalkyl, aryl, and heteroaryl are each independently and optionally substituted with one or more R⁰¹;
or Q² and R⁶, together with the carbon atoms to which they are attached, form ring B, or Q² and R⁵, together with the carbon atoms to which they are attached, form ring B; or R^{Q2} and R⁶, together with the ring atoms to which they are attached, form ring B⁰, or R^{Q2} and R⁵, together with the ring atoms to which they are attached, form ring B⁰;
each ring B is independently selected from the group consisting of cycloalkyl, heterocyclyl, aryl, and heteroaryl, and the ring B is optionally substituted with one or more R^{B}; each ring B⁰ is independently selected from the group consisting of cycloalkyl, heterocyclyl, aryl, and heteroaryl, and the ring B⁰ is optionally substituted with one or more R^{B};
R⁹ is selected from the group consisting of -C(O)OR¹², -C(O)NR^{11a}R^{11b}, - S(O)ₜR¹², -S(O)ₜNR^{11a}R^{11b}, heterocyclyl, and heteroaryl, wherein the heterocyclyl and heteroaryl are each independently and optionally substituted with one or more R^{9a};
R⁷ and R⁸ are the same or different and are each independently selected from the group consisting of a hydrogen atom, halogen, alkyl, alkenyl, alkynyl, alkoxy, alkoxyalkyl, haloalkyl, haloalkoxy, hydroxyalkyl, cyano, amino, nitro, hydroxyl, oxo, -(CH₂)ₓNR^{11a}R^{11b}, -(CH₂)ₓC(O)NR^{11a}R^{11b}, -NR⁰⁰C(O)R¹², -C(O)R¹², - C(O)OR¹², -OC(O)R¹², -S(O)ₜR¹², -S(O)ₜNR^{11a}R^{11b}, -NR⁰⁰S(O)ₜR¹², - (CH₂)ₓP(O)R¹²R¹³, -S(O)ₜOR¹², -OS(O)ₜR¹², -OR¹², =CR^{12a}R^{13a}, =NR⁰⁰, C(=NR⁰⁰)R¹², S(=NR⁰⁰)(O)R¹², cycloalkyl, cycloalkylalkyl, heterocyclyl, heterocyclylalkyl, aryl, and heteroaryl; the alkyl, alkenyl, alkynyl, alkoxy, cycloalkyl, cycloalkylalkyl, heterocyclyl, heterocyclylalkyl, aryl, and heteroaryl are each independently and optionally substituted with one or more R⁰¹; or
R⁷ and R⁸, together with the atoms to which they are attached, form 3- to 5-membered cycloalkyl or heterocyclyl, wherein the 3- to 5-membered cycloalkyl and heterocyclyl are each independently and optionally substituted with one or more R⁷⁸;
n1 is 0, 1, 2, 3, 4, or 5; n2 is 0, 1, 2, 3, 4, or 5;
R^{Q1}, R^{Z2}, R^{Z1}, R⁰¹, R^{Q2}, R^{B} , R^{9a}, and R⁷⁸ are each the same or different and are each independently selected from the group consisting of halogen, alkyl, alkenyl, alkynyl, alkoxy, haloalkyl, haloalkoxy, hydroxyalkyl, cyano, amino, nitro, hydroxyl, oxo, -(CH₂)ₓNR^{11a}R^{11b}, -NR⁰⁰C(O)R¹², -C(O)R¹², -C(O)OR¹², -OC(O)R¹², - (CH₂)ₓC(O)NR^{11a}R^{11d}, -S(O)ₜR¹², -S(O)ₜNR^{11a}R^{11b}, -NR⁰⁰S(O)ₜR¹², - (CH₂)ₓP(O)R¹²R¹³, -S(O)ₜOR¹², -OS(O)ₜR¹², -OR¹², =CR^{12a}R^{13a}, =NR⁰⁰, C(=NR⁰⁰)R¹², S(=NR⁰⁰)(O)R¹², cycloalkyl, heterocyclyl, 6- to 10-membered aryl, and 5- to 10-membered heteroaryl, wherein the alkyl, alkenyl, alkynyl, alkoxy, cycloalkyl, heterocyclyl, 6- to 10-membered aryl, and 5- to 10-membered heteroaryl are each independently and optionally substituted with one or more R*;
or R^{Z2} and R^{Z1}, together with the ring atoms to which they are attached, form ring C;
ring C is selected from the group consisting of cycloalkyl, heterocyclyl, aryl, and heteroaryl, and the ring C is optionally substituted with one or more R^{C};
R* and R^{C} are each the same or different and are each independently selected from the group consisting of halogen, alkyl, alkenyl, alkynyl, alkoxy, haloalkyl, haloalkoxy, hydroxyalkyl, cyano, amino, nitro, hydroxyl, oxo, -(CH₂)ₓNR^{11a}R^{11b}, - NR⁰⁰C(O)R¹², -C(O)R¹², -C(O)OR¹², -(CH₂)ₓC(O)NR^{11a}R^{11d}, -S(O)ₜR¹², - S(O)ₜNR^{11a}R^{11b}, -NR⁰⁰S(O)ₜR¹², -(CH₂)ₓP(O)R¹²R¹³, -OC(O)R¹², -S(O)ₜOR¹², - OS(O)ₜR¹², -OR¹², =CR^{12a}R^{13a}, =NR⁰⁰, C(=NR⁰⁰)R¹², S(=NR⁰⁰)(O)R¹², cycloalkyl, heterocyclyl, aryl, and heteroaryl, wherein the alkyl, alkenyl, alkynyl, alkoxy, cycloalkyl, heterocyclyl, aryl, and heteroaryl are each independently and optionally substituted with one or more identical or different substituents selected from the group consisting of oxo, halogen, cyano, amino, hydroxyl, alkyl, haloalkyl, hydroxyalkyl, alkoxy, haloalkoxy, cycloalkyl, heterocyclyl, aryl, and heteroaryl;
R^{11a}, R^{11b}, R¹², and R¹³ are each the same or different and are each independently selected from the group consisting of a hydrogen atom, alkyl, alkoxy, hydroxyalkyl, haloalkyl, -C(O)R¹⁵, -C(O)OR¹⁵, -OC(O)R¹⁵, -(CH₂)_{y}NR1^{4a}R^{14b}, - (CH₂)_{y}C(O)NR^{14a}R^{14b}, -NR⁰C(O)R¹⁵, -S(O)ₜR¹⁵, -S(O)ₜNR^{14a}R^{14b}, -NR⁰S(O)ₜR¹⁵, - S(O)ₜOR¹⁵, - OS(O)ₜR¹⁵, -OR¹⁵, 4- to 12-membered cycloalkyl, and heterocyclyl; or R^{11a} and R^{11b}, together with the nitrogen atom to which they are attached, form heterocyclyl, wherein the heterocyclyl is optionally substituted with one or more identical or different substituents selected from the group consisting of oxo, halogen, cyano, amino, hydroxyl, alkyl, haloalkyl, hydroxyalkyl, alkoxy, and haloalkoxy;
R^{aa}, R^{bb}, R⁰⁰, and R⁰ are each the same or different and are each independently selected from the group consisting of a hydrogen atom, alkyl, hydroxyalkyl, haloalkyl, C(O) alkyl, amido, cycloalkyl, and heterocyclyl;
R^{12a} and R^{13a} are each the same or different and are each independently selected from the group consisting of a hydrogen atom, halogen, cyano, amino, hydroxyl, alkyl, haloalkyl, hydroxyalkyl, alkoxy, haloalkoxy, cycloalkyl, and heterocyclyl; or R^{12a} and R^{13a}, together with the carbon atoms to which they are attached, form cycloalkyl or heterocyclyl, wherein the cycloalkyl and heterocyclyl are each independently and optionally substituted with one or more identical or different substituents selected from the group consisting of halogen, cyano, amino, hydroxyl, oxo, alkenyl, alkynyl, alkyl, haloalkyl, hydroxyalkyl, alkoxy, haloalkoxy, cycloalkyl, and heterocyclyl;
R¹⁵, R^{14a}, and R^{14b} are each the same or different and are each independently selected from the group consisting of a hydrogen atom, alkyl, alkoxy, hydroxyalkyl, haloalkyl, C(O) alkyl, amino, amido, cycloalkyl, heterocyclyl, aryl, and heteroaryl, wherein the alkyl, alkoxy, cycloalkyl, heterocyclyl, aryl, and heteroaryl are each independently and optionally substituted with one or more identical or different substituents selected from the group consisting of halogen, cyano, nitro, amino, hydroxyl, oxo, alkenyl, alkynyl, alkyl, haloalkyl, hydroxyalkyl, alkoxy, haloalkoxy, cycloalkyl, and heterocyclyl;
x is 0, 1, 2, 3, 4, 5, or 6; y is 0, 1, 2, 3, 4, 5, or 6; and
t is 0, 1, or 2.

In some embodiments of the present invention, the compound represented by general formula (I) or the pharmaceutically acceptable salt thereof is a compound represented by general formula (IM) or a pharmaceutically acceptable salt thereof: 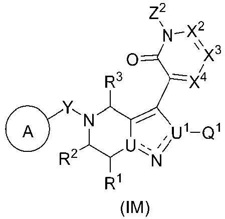 wherein
X², X³, and X⁴ are the same or different and are each independently CR^{Z1a} or N; R^{Z1a} is a hydrogen atom or R^{Z1}; and
-̅ -̅ -̅, 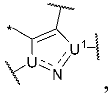 ring A, Y, R¹, R², R³, Q¹, Z², and R^{Z1} are as defined for general formula (I).

In some embodiments of the present invention, the compound represented by general formula (I) or (IM) or the pharmaceutically acceptable salt thereof is a compound represented by general formula (IM-1) or a pharmaceutically acceptable salt thereof: 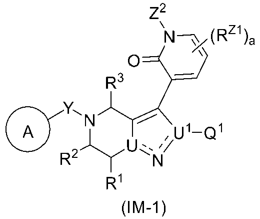
wherein a is 0, 1, 2, or 3; and
-̅ -̅ -̅, 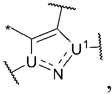 ring A, Y, R¹, R², R³, Q¹, R^{Z1}, and Z² are as defined for general formula (I).

In some embodiments of the present invention, the compound represented by general formula (I), (IM), or (IM-1) or the pharmaceutically acceptable salt thereof is a compound represented by general formula (IM-2) or a pharmaceutically acceptable salt thereof: 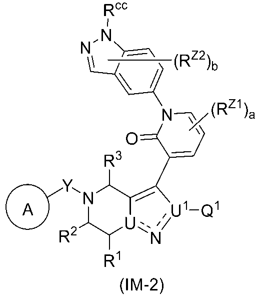
wherein R^{cc} is selected from the group consisting of a hydrogen atom, alkyl, hydroxyalkyl, and cycloalkyl;
a is 0, 1, 2, or 3; b is 0, 1, 2, 3, or 4; and
-̅ -̅ -̅, 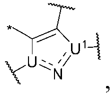 ring A, Y, R¹, R², R³, Q¹, R^{Z1}, and R^{Z2} are as defined for general formula (I).

In some embodiments of the present invention, the compound represented by general formula (I) or the pharmaceutically acceptable salt thereof is a compound represented by general formula (IN) or a pharmaceutically acceptable salt thereof: 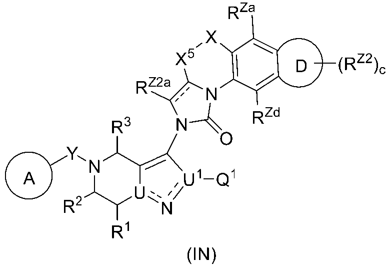
wherein -̅ -̅ -̅ is a single bond or a double bond;
X is selected from the group consisting of a bond, CR^{Z2c}R^{z2d}CR^{z2c}R^{z2d}, CR^{Z2c}R^{Z2d}, O, NR^{cc}, CR^{Z2c}R^{Z2d}O, OCR^{Z2c}R^{z2d}, and C(O); X⁵ is selected from the group consisting of a bond, CR^{Z2e}R^{Z2f}CR^{Z2e}R^{Z2f}, CR^{Z2e}R^{Z2f}, O, NR^{cc}, CR^{Z2e}R^{Z2f}O, OCR^{Z2e}R^{Z2f}, and C(O);
R^{Z2a}, R^{Z2c}, R^{Z2d}, R^{Z2e}, and R^{Z2f} are the same or different and are each independently a hydrogen atom or R^{Z2}; or R^{Z2c} and R^{Z2d}, together with the carbon atoms to which they are attached, form cycloalkyl or heterocyclyl, or R^{Z2e} and R^{Z2f}, together with the carbon atoms to which they are attached, form cycloalkyl or heterocyclyl, or R^{Z2c} and R^{Z2e}, together with the carbon atoms to which they are attached, form cycloalkyl or heterocyclyl, wherein the formed cycloalkyl and heterocyclyl are each independently and optionally substituted with one or more selected from the group consisting of halogen, hydroxyl, alkyl, alkoxy, cycloalkyl, and heterocyclyl;
R^{Za} and R^{Zd} are the same or different and are each independently a hydrogen atom or R^{Z2},
ring D is selected from the group consisting of cycloalkyl, heterocyclyl, aryl, and heteroaryl; and c is 0, 1, 2, 3, 4, 5, or 6;
R^{cc} is selected from the group consisting of a hydrogen atom, alkyl, and cycloalkyl, wherein the alkyl and cycloalkyl are each independently and optionally substituted with one or more selected from the group consisting of a deuterium atom, halogen, hydroxyl, alkyl, alkoxy, and cycloalkyl; and 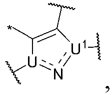 ring A, Y, R¹, R², R³, Q¹, and R^{Z2} are as defined for general formula (I).

In some embodiments of the present invention, the compound represented by general formula (I) or the pharmaceutically acceptable salt thereof is a compound represented by general formula (IN) or a pharmaceutically acceptable salt thereof: 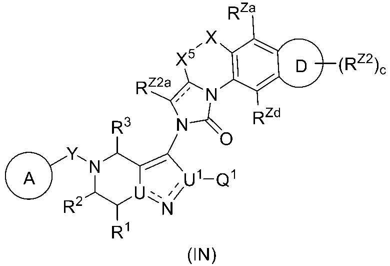 wherein 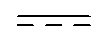
is a single bond or a double bond;
X is selected from the group consisting of a bond, CR^{Z2c}R^{Z2d}CR^{Z2c}R^{Z2d}, CR^{Z2c}R^{Z2d}, O, NR^{cc}, CR^{Z2c}R^{z2d}O, OCR^{Z2c}R^{z2d}, and C(O); X⁵ is selected from the group consisting of a bond, CR^{Z2e}R^{Z2f}CR^{Z2e}R^{Z2f}, CR^{Z2e}R^{Z2f}, O, NR^{cc}, CR^{Z2e}R^{Z2f}O, OCR^{Z2e}R^{Z2f}, and C(O);
R^{Z2a}, R^{Z2c}, R^{Z2d}, R^{Z2e}, and R^{Z2f} are the same or different and are each independently a hydrogen atom or R^{Z2};
R^{Za} and R^{Zd} are the same or different and are each independently a hydrogen atom or R^{Z2};
ring D is selected from the group consisting of cycloalkyl, heterocyclyl, aryl, and heteroaryl; and
R^{cc} is selected from the group consisting of a hydrogen atom, alkyl, hydroxyalkyl, and cycloalkyl; c is 0, 1, 2, 3, 4, 5, or 6; and 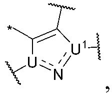 ring A, Y, R¹, R², R³, Q¹, and R^{Z2}; are as defined for general formula (I).

In some embodiments of the present invention, the compound represented by general formula (I) or (IN) or the pharmaceutically acceptable salt thereof is a compound represented by general formula (IN-1) or a pharmaceutically acceptable salt thereof: 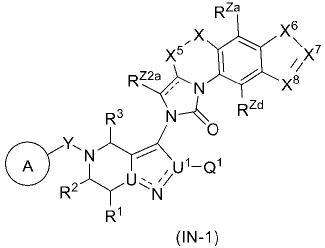 wherein
X⁶ is selected from the group consisting of NR°°, O, and S; X⁷ is CR^{Zh} or N; X⁸ is CR^{Ze} or N;
R^{Ze} and R^{Zh} are the same or different and each independently a hydrogen atom or R^{Z2}; and 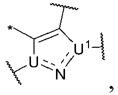 ring A, Y, R¹, R², R, Q¹ R^{z2a} -̅ -̅ -̅, X X⁵, R^{Za}, R^{Zd} R^{cc}, and R^{Z2} are as defined for general formula (IN).

In some embodiments of the present invention, the compound represented by general formula (I) or the pharmaceutically acceptable salt thereof is a compound represented by general formula (IP) or a pharmaceutically acceptable salt thereof: 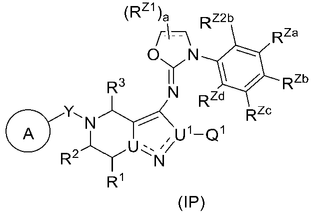
wherein a is 0, 1, 2, or 3; and
R^{Z2b}, R^{Za}, R^{Zb}, R^{Zc}, and R^{Zd} are the same or different and each independently a hydrogen atom or R^{Z2};
or R^{Zb} and R^{Zc}, together with the carbon atoms to which they are attached, form ring D, or R^{Zc} and R^{Zd}, together with the carbon atoms to which they are attached, form ring D; ring D is optionally substituted with c R^{Z2}; and 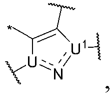 ring A, Y, R¹, R², R³, Q¹, -̅ -̅ -̅, R^{Z1}, R^{Z2}, ring D, and c are as defined for general formula (IN).

In some embodiments of the present invention, the compound represented by general formula (I) or (IP) or the pharmaceutically acceptable salt thereof is a compound represented by general formula (IP-1) or a pharmaceutically acceptable salt thereof: 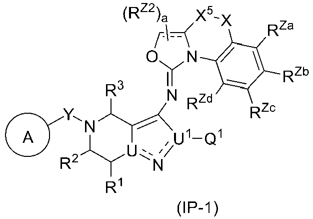 wherein
R^{Za}, R^{Zb}, R^{Zc}, and R^{Zd} are the same or different and each independently a hydrogen atom or R^{Z2};
or R^{Za} and R^{Zb}, together with the carbon atoms to which they are attached, form ring D, or R^{Zb} and R^{Zc}, together with the carbon atoms to which they are attached, form ring D, or R^{Zc} and R^{Zd}, together with the carbon atoms to which they are attached, form ring D; ring D is optionally substituted with c R^{Z2}; a is 0, 1, 2, or 3; and 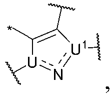 ring A, Y, R¹, R², R³, Q¹, -̅ -̅ -̅, R^{Z2}, X, X⁵, ring D, and c are as defined for general formula (IN).

In some embodiments of the present invention, the compound represented by general formula (I), (IP), or (IP-1) or the pharmaceutically acceptable salt thereof is a compound represented by general formula (IP-2) or (IP-3) or a pharmaceutically acceptable salt thereof: 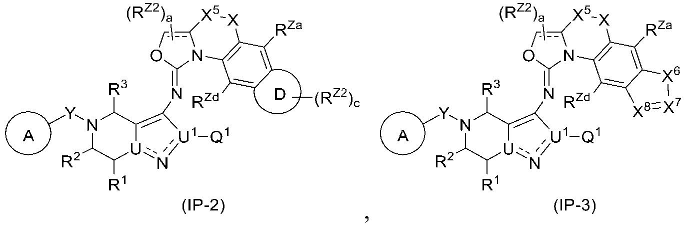 wherein
a is 0, 1, 2, or 3; 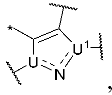 ring A, Y, R¹, R², R³ Q¹, -̅ -̅ -̅, R^{Z2}, X, X⁵, R^{Za}, R^{Za}, ring D, and c are as defined for general formula (IN); and X⁶, X⁷, and X⁸ are as defined for general formula (IN-1).

In some embodiments of the present invention, the compound represented by general formula (I) or the pharmaceutically acceptable salt thereof is a compound represented by general formula (IO) or a pharmaceutically acceptable salt thereof: 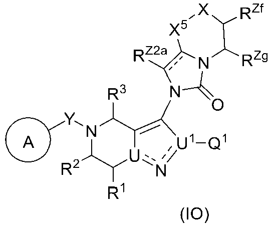 wherein
R^{Zf} and R^{Zg} are the same or different and each independently a hydrogen atom or R^{Z2}, or R^{Zf} and R^{Zg}, together with the carbon atoms to which they are attached, form ring E; ring E is selected from the group consisting of cycloalkyl, heterocyclyl, aryl, and heteroaryl, and ring E is optionally substituted with 1, 2, 3, or 4 R^{Z2}; and 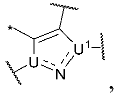 ring A, Y, R¹, R², R³, Q¹, R^{Z2a}, -̅ -̅ -̅, X, X⁵ , and R^{Z2} are as defined for general formula (IN).

In some embodiments of the present invention, the compound represented by general formula (I) or the pharmaceutically acceptable salt thereof is a compound represented by general formula (II) or a pharmaceutically acceptable salt thereof: 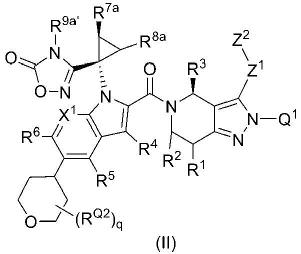 wherein
R^{7a} and R^{8a} are different and are each independently a hydrogen atom or R⁷⁸;
R^{9a}' is a hydrogen atom or alkyl; q is 0, 1, 2, 3, 4, 5, or 6; and
Q¹, Z¹, Z², R¹ to R⁶, X¹, R⁷⁸, and R^{Q2} are as defined for general formula (I).

In some embodiments of the present invention, the compound represented by general formula (I) or the pharmaceutically acceptable salt thereof is a compound represented by general formula (III) or a pharmaceutically acceptable salt thereof: 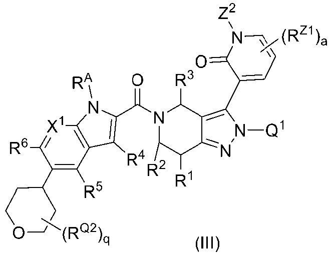 wherein
q is 0, 1, 2, 3, 4, 5, or 6; a is 0, 1, 2, or 3; and
Q¹, Z², R^{Z1}, R¹ to R⁶, X¹, R^{A}, and R^{Q2} are as defined for general formula (I).

In some embodiments of the present invention, the compound represented by general formula (I) or the pharmaceutically acceptable salt thereof is a compound represented by general formula (IV) or (IV') or a pharmaceutically acceptable salt thereof: 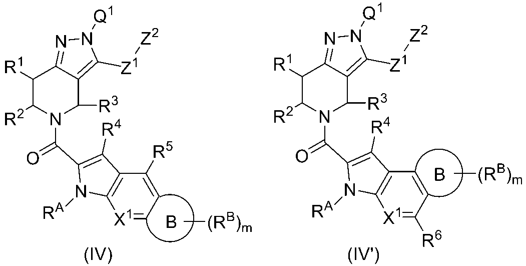 wherein
m is 0, 1, 2, 3, 4, 5, or 6; and
ring B, X¹, Q¹, Z¹, Z², R¹ to R⁴, R⁵, R⁶, R^{A}, and R^{B} are as defined for general formula (I).

In some embodiments of the present invention, in the compound represented by general formula (I), (IM), (IM-1), (IM-2), (IN), (IN-1), (IP) to (IP-3), or (IO) or the pharmaceutically acceptable salt thereof, 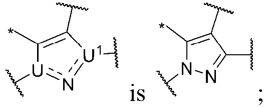 and in some embodiments,

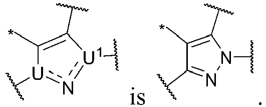

In some embodiments of the present invention, in the compound represented by general formula (I), (IM), (IM-1), (IM-2), (IN), (IN-1), (IP) to (IP-3), or (IO) or the pharmaceutically acceptable salt thereof, Y is C(O).

In some embodiments of the present invention, in the compound represented by general formula (I), (III), (IV), (IV'), (IM), (IM-1), (IM-2), (IN), (IN-1), (IP) to (IP-3), or (IO) or the pharmaceutically acceptable salt thereof, 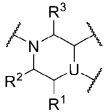 is 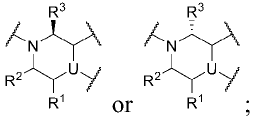 in some embodiments, 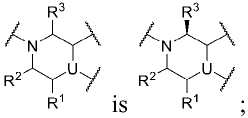 in some embodiments, 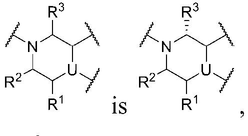 R¹, R², and R³ are as defined for general formula (I), and R³ is not a hydrogen atom; and in some embodiments, 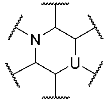 is optionally substituted with 1, 2, or 3 substituents selected from the group consisting of R⁰¹, and R⁰¹ is as defined for general formula (I).

In some embodiments of the present invention, in the compound represented by general formula (I), (IM), (IM-1), (IM-2), (IN), (IN-1), (IP) to (IP-3), or (IO) or the pharmaceutically acceptable salt thereof, ring A is 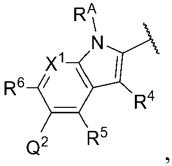 and X¹, Q², R^{A}, R⁴, R⁵, and R⁶ are as defined for general formula (I); in some embodiments, ring A is 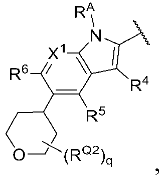 q is 0, 1, 2, 3, 4, 5, or 6; X¹, R^{A}, R⁴, R⁵, R⁶, and R^{Q2} are as defined for general formula (I);
in some embodiments, ring A is 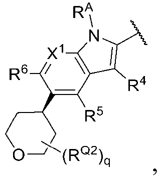 and q is 1, 2, 3, 4, 5, or 6; X¹, R^{A}, R⁴, R⁵, R⁶, and R^{Q2} are as defined for general formula (I);
in some embodiments, ring A is 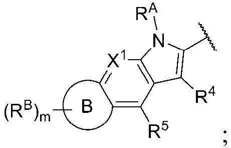 ring B, R^{B}, m, X¹, R^{A}, R⁴, and R⁵ are as defined for general formula (IV); in some embodiments, ring A is 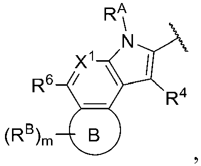 and ring B, R^{B}, m, X¹, R^{A}, R⁴, and R⁶ are as defined for general formula (IV'); and
in some embodiments, ring A is

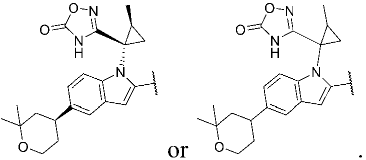

In some embodiments of the present invention, in the compound represented by general formula (I), (IV), or (IV') or the pharmaceutically acceptable salt thereof, ring B is 3- to 12-membered heterocyclyl; in some embodiments, ring B is 7- or 12-membered bicyclic heterocyclyl; in some embodiments, ring B is 10-membered bicyclic heterocyclyl; in some embodiments, ring B is selected from the group consisting of 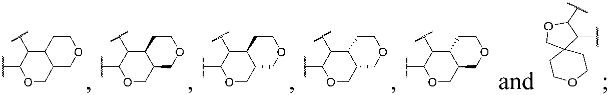 in some embodiments, ring B is 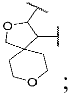 in some embodiments, ring B is 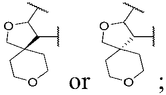 and 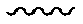 represents the site of fusion with the ring where X¹ is located.

In some embodiments of the present invention, in the compound represented by general formula (I) or the pharmaceutically acceptable salt thereof, ring B⁰ is 3-to 10-membered cycloalkyl or 3- to 10-membered heterocyclyl; in some embodiments, ring B⁰ is 5- or 6-membered cycloalkyl or 5- or 6-membered heterocyclyl; in some embodiments, ring B⁰ is 5- or 6-membered heterocyclyl; in some embodiments, ring B⁰ is hydrofuryl or hydropyranyl; and in some embodiments, ring B⁰ is hydrofuryl.

In some embodiments of the present invention, in the compound represented by general formula (I), (IV), or (IV') or the pharmaceutically acceptable salt thereof, each R^{B} is the same or different and is each independently selected from the group consisting of halogen, C₁₋₆ alkyl, and C₁₋₆ haloalkyl; in some embodiments, each R^{B} is the same or different and is each independently C₁₋₆ alkyl; and in some embodiments, each R^{B} is the same or different and is each independently methyl.

In some embodiments of the present invention, in the compound represented by general formula (I), (IV), or (IV') or the pharmaceutically acceptable salt thereof, m is 0, 1, or 2; and in some embodiments, m is 0.

In some embodiments of the present invention, in the compound represented by general formula (I), (IM), (IM-1), (IM-2), (IN), (IN-1), (IP) to (IP-3), or (IO) or the pharmaceutically acceptable salt thereof, Q² is 3- to 12-membered heterocyclyl or 5- to 10-membered heteroaryl, wherein the 3- to 12-membered heterocyclyl and 5- to 10-membered heteroaryl are each independently and optionally substituted with one or more R^{Q2}, and R^{Q2} is as defined for general formula (I); in some embodiments, Q² is 3- to 6-membered heterocyclyl optionally substituted with one or more R^{Q2}, and R^{Q2} is as defined for general formula (I); in some embodiments, Q² is tetrahydropyranyl optionally substituted with one or more R^{Q2}, and R^{Q2} is as defined for general formula (I);
in some embodiments, Q² is 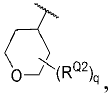 q is 0, 1, 2, 3, 4, 5, or 6, and R^{Q2} is as defined for general formula (I); in some embodiments, Q² is 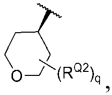 q is 1, 2, 3, 4, 5, or 6, and R^{Q2} is as defined for general formula (I);
in some embodiments, Q² is 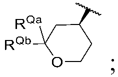 R^{Qa} is selected from the group consisting of halogen, alkyl, alkenyl, alkynyl, alkoxy, haloalkyl, haloalkoxy, hydroxyalkyl, cyano, amino, hydroxyl, cycloalkyl, and heterocyclyl, R^{Qb} is a hydrogen atom or R^{Q2}, and R^{Q2} is as defined for general formula (I); and in some embodiments, Q² is

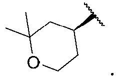

In some embodiments of the present invention, in the compound represented by general formula (I) to (III), (IM), (IM-1), (IM-2), (IN), (IN-1), (IP) to (IP-3), or (IO) or the pharmaceutically acceptable salt thereof, 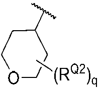 is 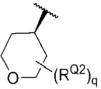 or 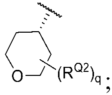 in some embodiments, 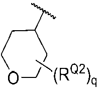 is 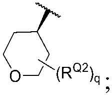 in some embodiments, 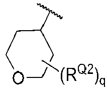 is 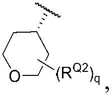 q is 1, 2, 3, 4, 5, or 6, and R^{Q2} is as defined for general formula (I);
in some embodiments, 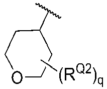 is 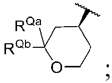 R^{Qa} is selected from the group consisting of halogen, alkyl, alkenyl, alkynyl, alkoxy, haloalkyl, haloalkoxy, hydroxyalkyl, cyano, amino, hydroxyl, cycloalkyl, and heterocyclyl, R^{Qb} is a hydrogen atom or R^{Q2}, and R^{Q2} is as defined for general formula (I); and in some embodiments, 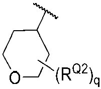 is

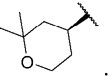

In some embodiments of the present invention, in the compound represented by general formula (I) to (III), (IM), (IM-1), (IM-2), (IN), (IN-1), (IP) to (IP-3), or (IO) or the pharmaceutically acceptable salt thereof, each R^{Q2} is the same or different and is each independently selected from the group consisting of halogen, C₁₋₆ alkyl, and C₁₋₆ haloalkyl; in some embodiments, each R^{Q2} is the same or different and is each independently C₁₋₆ alkyl; in some embodiments, R^{Q2} is methyl; in some embodiments, each R^{Q2} is the same or different and is each independently selected from the group consisting of halogen, C₁₋₆ alkyl, C₁₋₆ haloalkyl, and 3- to 6-membered cycloalkyl;
in some embodiments, each R^{Q2} is the same or different and is each independently C₁₋₆ alkyl, or R^{Q2} and R⁶, together with the carbon atoms to which they are attached, form ring B⁰, or R^{Q2} and R⁵, together with the carbon atoms to which they are attached, form ring B⁰, and ring B⁰ is 5- or 6-membered heterocyclyl; in some embodiments, each R^{Q2} is the same or different and is each independently C₁₋₆ alkyl; or R^{Q2} and R⁶, together with the carbon atoms to which they are attached, form ring B⁰, and ring B⁰ is 5- or 6-membered heterocyclyl.

In some embodiments of the present invention, in the compound represented by general formula (I) to (III), (IM), (IM-1), (IM-2), (IN), (IN-1), (IP) to (IP-3), or (IO) or the pharmaceutically acceptable salt thereof, q is 1, 2, or 3; in some embodiments, q is 2; and in some embodiments, q is 0, 1, or 2.

In some embodiments of the present invention, R^{Qa} is selected from the group consisting of halogen, C₁₋₆ alkyl, and C₁₋₆ haloalkyl; and/or R^{Qb} is selected from the group consisting of a hydrogen atom, halogen, C₁₋₆ alkyl, and C₁₋₆ haloalkyl; in some embodiments, R^{Qa} is C₁₋₆ alkyl, and/or R^{Qb} is C₁₋₆ alkyl; and in some embodiments, R^{Qa} is methyl, and/or R^{Qb} is methyl.

In some embodiments of the present invention, in the compound represented by general formula (I), (II), (IV), or (IV') or the pharmaceutically acceptable salt thereof, Z¹ is 3- to 12-membered heterocyclyl optionally substituted with one or more R^{Z1}, and R^{Z1} is as defined for general formula (I); in some embodiments, Z¹ is selected from the group consisting of a bond, -N=, 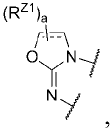 and 5- or 6-membered heterocyclyl, wherein the 5- or 6-membered heterocyclyl is optionally substituted with one or more R^{Z1}, a is 0, 1, 2, or 3, -̅ -̅ -̅ is a single bond or a double bond, and R^{Z1} is as defined for general formula (I); in some embodiments, Z¹ is 5- or 6-membered heterocyclyl optionally substituted with one or more R^{Z1}, and R^{Z1} is as defined for general formula (I); in some embodiments, Z¹ is dihydroimidazolyl optionally substituted with one or more R^{Z1}, and R^{Z1} is as defined for general formula (I); in some embodiments, Z¹ is selected from the group consisting of 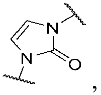 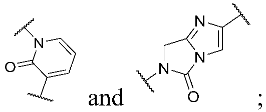 in some embodiments, Z¹ is 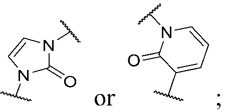 in some embodiments, Z¹ is 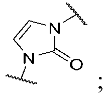 in some embodiments, Z¹ is 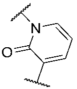 or 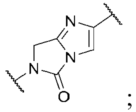 in some embodiments, Z¹ is a bond;
in some embodiments, Z¹ is 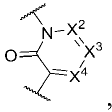 and X², X³, and X⁴ are the same or different and are each independently CR^{Z1a} or N; R^{Z1a} is a hydrogen atom or R^{Z1}; and R^{Z1} is as defined for general formula (I);
in some embodiments, Z¹ is selected from the group consisting of a bond, -N=, 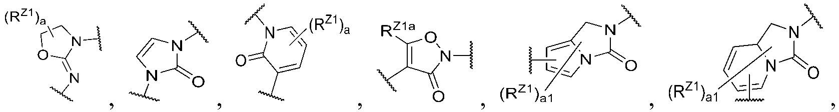 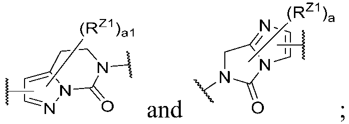 R^{Z1a} is a hydrogen atom or R^{Z1}; a is 0, 1, 2, or 3; a1 is 0, 1, 2, 3, or 4; and R^{Z1} is as defined for general formula (I);
in some embodiments, Z¹ is selected from the group consisting of a bond, -N=, 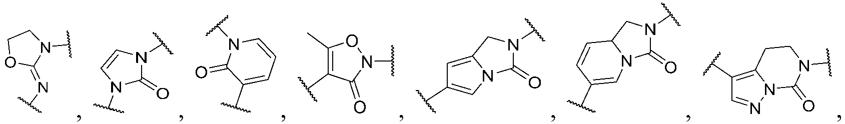 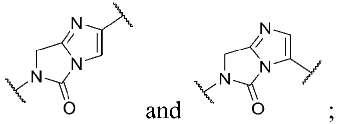 in some embodiments, Z¹ is selected from the group consisting of a bond, 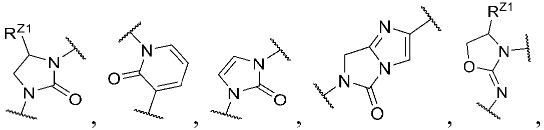 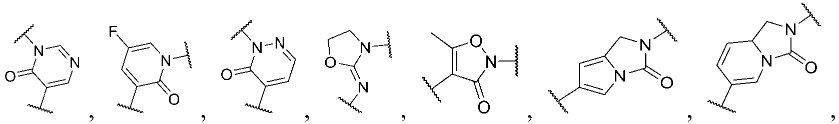 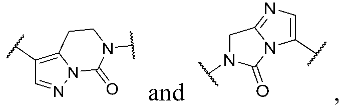 and R^{Z1} is a hydrogen atom, or R^{Z1} and R^{Z2}, together with the atoms to which they are attached, form 6- or 7-membered heterocyclyl; in some embodiments, Z¹ is selected from the group consisting of 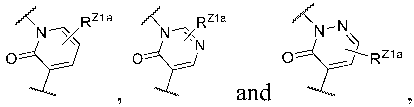 R^{Z1a} is a hydrogen atom or R^{Z1}, and R^{Z1} is as defined for general formula (I); in some embodiments, Z¹ is selected from the group consisting of 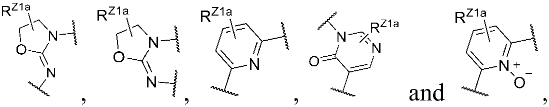 and R^{Z1a} is selected from the group consisting of a hydrogen atom, halogen, C₁₋₆ alkyl, and 3-to 6-membered cycloalkyl; and in some embodiments, Z¹ is selected from the group consisting of

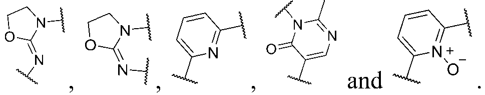

In some embodiments of the present invention, in the compound represented by general formula (I) to (IV'), (IM), (IM-1), or (IM-2) or the pharmaceutically acceptable salt thereof, each R^{Z1} is the same or different and is each independently selected from the group consisting of halogen, C₁₋₆ alkyl, C₁₋₆ alkoxy, C₁₋₆ haloalkyl, C₁₋₆ haloalkoxy, oxo, and amino; in some embodiments, each R^{Z1} is the same or different and is each independently selected from the group consisting of oxo, C₁₋₆ alkyl, and C₁₋₆ haloalkyl; in some embodiments, R^{Z1} is oxo; in some embodiments, R^{Z1} is methyl; and in some embodiments, each R^{Z1} is the same or different and is each independently selected from the group consisting of halogen, C₁₋₆ alkyl, and C₁₋₆ haloalkyl.

In some embodiments of the present invention, in the compound represented by general formula (I), (IM-1), (IM-2), (IP) to (IP-3), or (III) or the pharmaceutically acceptable salt thereof, a is 0.

In some embodiments of the present invention, in the compound represented by general formula (I) or (IM) or the pharmaceutically acceptable salt thereof, X², in some embodiments, is selected from the group consisting of C-methyl, CF, CH, and N; X² is CH or N; and in some embodiments, X² is CH.

In some embodiments of the present invention, in the compound represented by general formula (I) or (IM) or the pharmaceutically acceptable salt thereof, X³ is CR^{Z1a} or N, and R^{Z1a} is selected from the group consisting of a hydrogen atom, halogen, C₁₋₆ alkyl, and C₁₋₆ haloalkyl; in some embodiments, X³ is CH or N; and in some embodiments, X³ is CH.

In some embodiments of the present invention, in the compound represented by general formula (I) or (IM) or the pharmaceutically acceptable salt thereof, X⁴ is CH or N; and in some embodiments, X⁴ is CH.

In some embodiments of the present invention, in the compound represented by general formula (I) or (IM-2) or the pharmaceutically acceptable salt thereof, b is 0, 1, or 2; in some embodiments, b is 0 or 1; and in some embodiments, b is 0.

In some embodiments of the present invention, in the compound represented by general formula (I) or (IM) or the pharmaceutically acceptable salt thereof, R^{Z1a} is selected from the group consisting of a hydrogen atom, halogen, C₁₋₆ alkyl, C₁₋₆ alkoxy, C₁₋₆ haloalkyl, and C₁₋₆ haloalkoxy; in some embodiments, R^{Z1a} is a hydrogen atom or C₁₋₆ alkyl; in some embodiments, R^{Z1a} is a hydrogen atom or methyl; in some embodiments, R^{Z1a} is a hydrogen atom; in some embodiments, R^{Z1a} is selected from the group consisting of a hydrogen atom, halogen, C₁₋₆ alkyl, and 3-to 6-membered cycloalkyl; in some embodiments, R ^{Zla} is a hydrogen atom or halogen; and in some embodiments, R^{Z1a} is selected from the group consisting of a hydrogen atom, F, methyl, and cyclopropyl.

In some embodiments of the present invention, in the compound represented by general formula (I) to (IV'), (IM), or (IM-1) or the pharmaceutically acceptable salt thereof, Z² is 6- to 10-membered aryl or 5- to 10-membered heteroaryl, wherein the 6- to 10-membered aryl and 5- to 10-membered heteroaryl are each independently and optionally substituted with one or more R^{Z2}; in some embodiments, Z² is a hydrogen atom or 5- to 10-membered heteroaryl optionally substituted with one or more R^{Z2}; in some embodiments, Z² is 5- to 10-membered heteroaryl optionally substituted with one or more R^{Z2}; in some embodiments, Z² is indazolyl optionally substituted with one or more R^{Z2}; R^{Z2} is as defined for general formula (I);
in some embodiments, Z² is 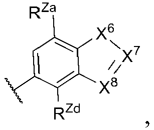 and R^{Za}, R^{Za}, X⁶, X⁷, and X⁸ are as defined for general formula (IN-1);
in some embodiments, Z² is 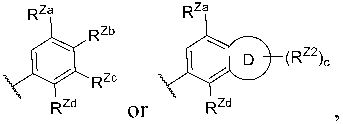 and R^{Za}, R^{Zb}, R^{Zc}, and R^{Zd} are the same or different and each independently a hydrogen atom or R^{Z2}; c is 0, 1, 2, 3, 4, 5, or 6; ring D is selected from the group consisting of cycloalkyl, heterocyclyl, aryl, and heteroaryl; and R^{Z2} is as defined for general formula (I);
in some embodiments, Z² is selected from the group consisting of a hydrogen atom, 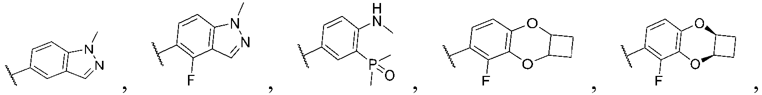 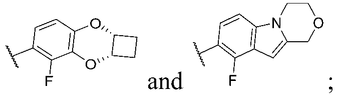 in some embodiments, Z² is 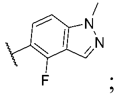 in some embodiments, Z² is selected from the group consisting of a hydrogen atom, 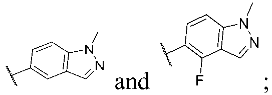
in some embodiments, Z² is 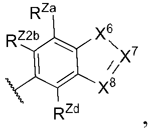 R^{Z2b} is a hydrogen atom or R^{Z2}, and R^{Z2}, R^{Za}, R^{Zd}, X⁶, X⁷, and X⁸ are as defined for general formula (IN-1); in some embodiments, Z² is 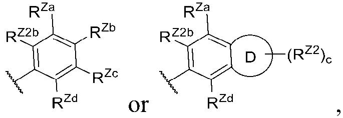 R^{Z2b} is a hydrogen atom or R^{Z2}, and R^{Za}, R^{Zb}, R^{Zc}, and R^{Zd} are the same or different and each independently a hydrogen atom or R^{Z2}; c is 0, 1, 2, 3, 4, 5, or 6; ring D is heteroaryl; and R^{Z2} is selected from the group consisting of a hydrogen atom, halogen, C₁₋₆ alkyl, C₁₋₆ haloalkyl, and 3- to 6-membered cycloalkyl, or R^{Z2} and R^{Z1}, together with the ring atoms to which they are attached, form heterocyclyl, wherein the heterocyclyl is optionally substituted with one or more selected from the group consisting of halogen, C₁₋₆ alkyl, C₁₋₆ haloalkyl, and 3- to 6-membered cycloalkyl;
in some embodiments, Z² is selected from the group consisting of a hydrogen atom, 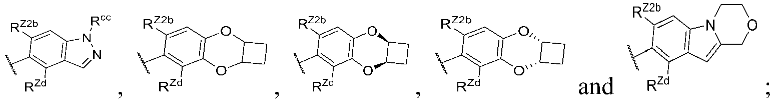 R^{Z2b} is selected from the group consisting of a hydrogen atom, halogen, C₁₋₆ alkyl, and 3- to 6-membered cycloalkyl, or R^{Z2b} and R^{Z1}, together with the ring atoms to which they are attached, form 6- or 7-membered heterocyclyl, R^{Zd} is selected from the group consisting of a hydrogen atom, halogen, C₁₋₆ alkyl, C₁₋₆ haloalkyl, and 3-to 6-membered cycloalkyl, and R^{cc} is selected from the group consisting of a hydrogen atom, C₁₋₆ alkyl, 3- to 6-membered cycloalkyl, and C₁₋₆ deuterioalkyl;
in some embodiments, Z² is selected from the group consisting of a hydrogen atom, 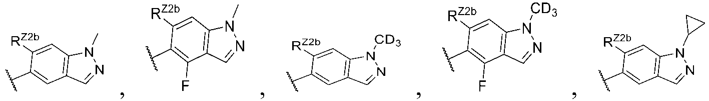 and 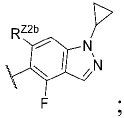 R^{Z2b} is a hydrogen atom, or R^{Z2b} and R^{Z1}, together with the ring atoms to which they are attached, form 6- or 7-membered heterocyclyl; in some embodiments, Z² is selected from the group consisting of a hydrogen atom, 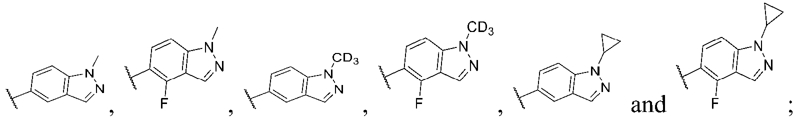
in some embodiments, Z² is a hydrogen atom or 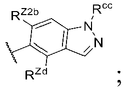 R^{Z2b} is a hydrogen atom, R^{Zd} is selected from the group consisting of a hydrogen atom, halogen, and C₁₋₆ alkyl, and R^{cc} is selected from the group consisting of a hydrogen atom, C₁₋₆ alkyl, 3- to 6-membered cycloalkyl, and C₁₋₆ deuterioalkyl; and in some embodiments, Z² is selected from the group consisting of

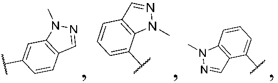

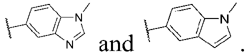

In some embodiments of the present invention, in the compound represented by general formula (I), (II), (IV), or (IV') or the pharmaceutically acceptable salt thereof, 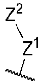 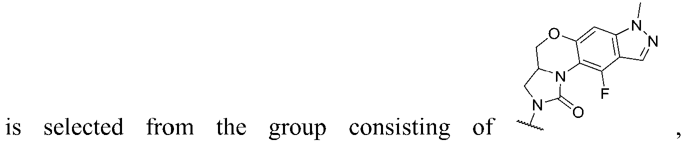 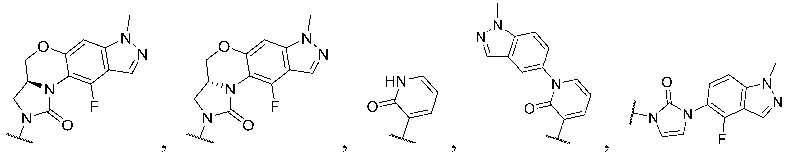 and 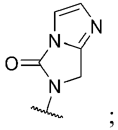 in some embodiments, 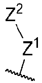 is selected from the group consisting of 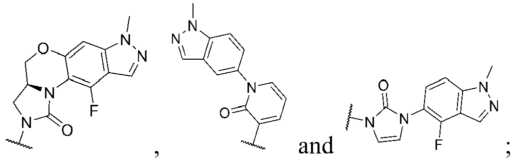 in some embodiments, 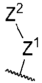 is 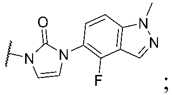
in some embodiments, 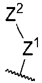 is selected from the group consisting of group A: 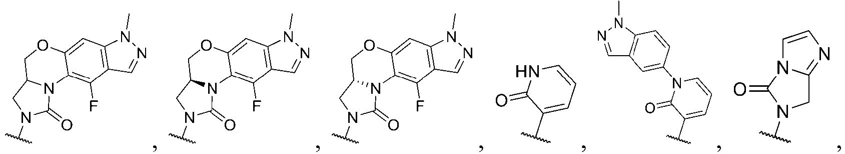 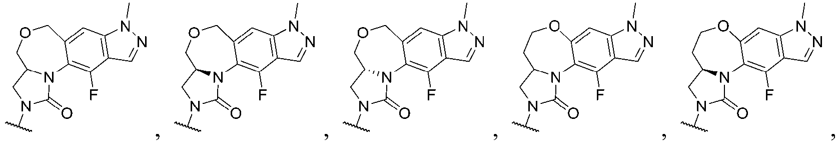 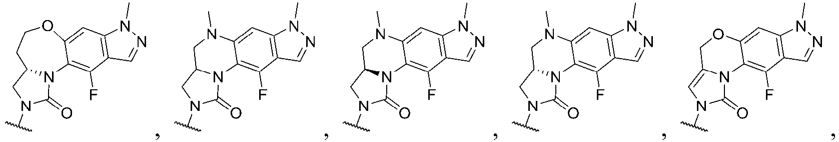 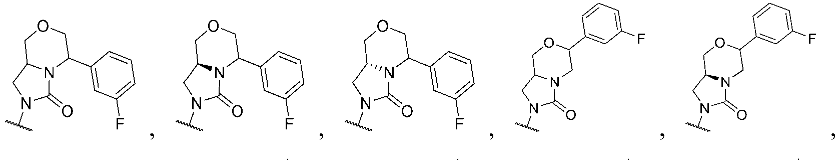 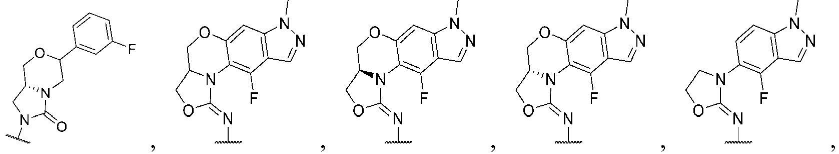 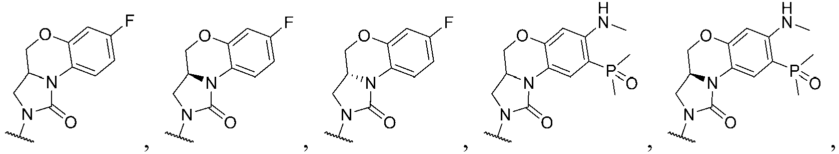 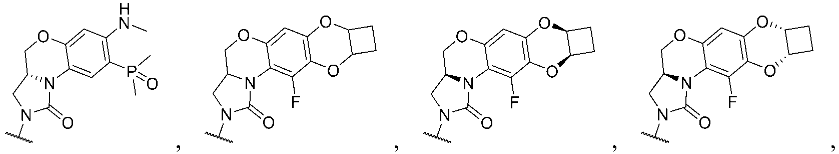 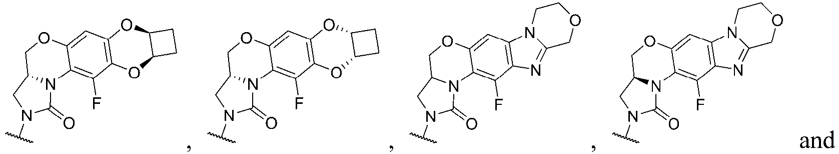 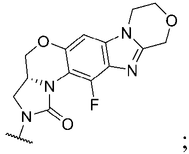 in some embodiments, 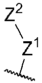 is selected from the group consisting of group B: 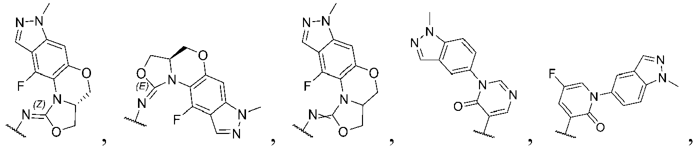 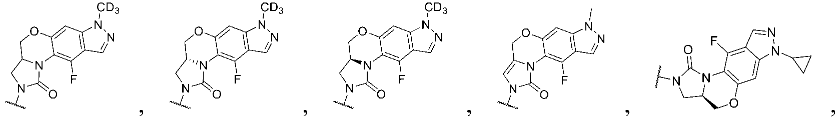 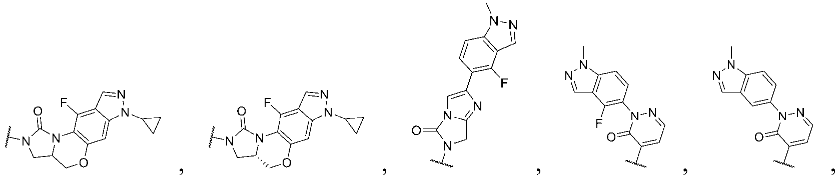 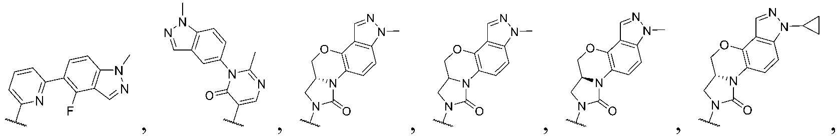 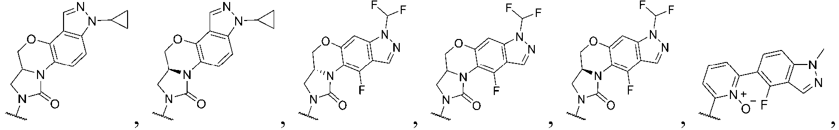 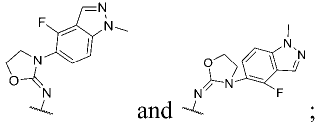
in some embodiments, 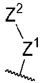 is selected from the group consisting of group C: 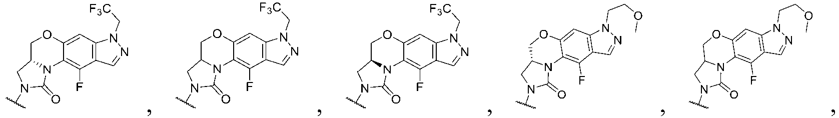 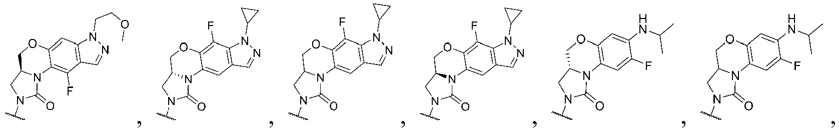 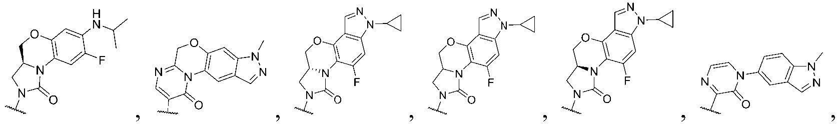 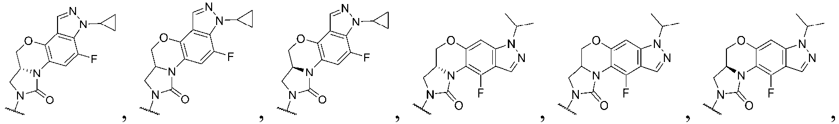 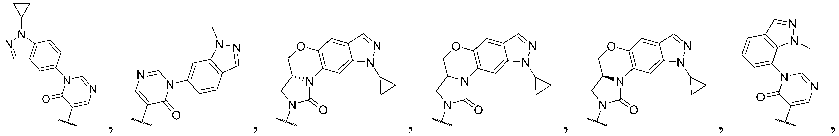 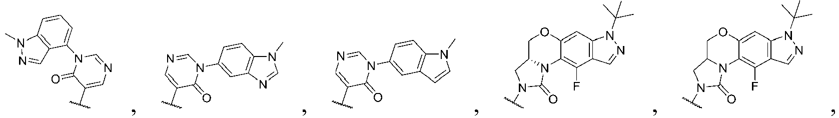 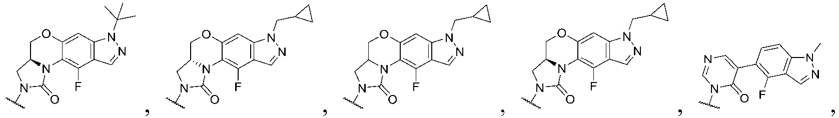 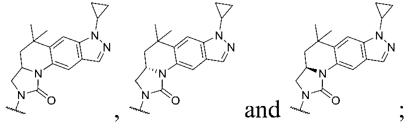
in some embodiments, 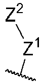 is selected from the group consisting of group A, group B, and group C (in some embodiments, the groups in group C);
in some embodiments, 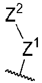 is selected from the group consisting of 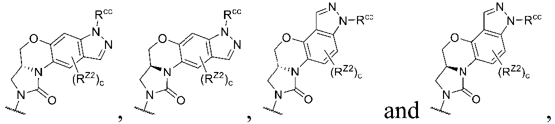 c is 0, 1, or 2, and R^{cc} is selected from the group consisting of a hydrogen atom, C₁₋₆ alkyl, and 3- to 6-membered cycloalkyl, wherein the C₁₋₆ alkyl and 3- to 6-membered cycloalkyl are each independently and optionally substituted with one or more selected from the group consisting of a deuterium atom, halogen, C₁₋₆ alkyl, C₁₋₆ alkoxy, hydroxyl, and 3- to 6-membered cycloalkyl; each R^{Z2} is the same or different and is each independently selected from the group consisting of halogen, cyano, C₁₋₆ alkyl, C₁₋₆ haloalkyl, C₁₋₆ alkoxy, C₁₋₆ haloalkoxy, and 3- to 6-membered cycloalkyl;
in some embodiments, 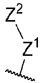 is 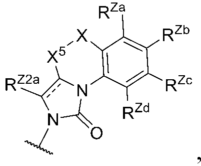 and R^{Za}, R^{Zb}, R^{Zc}, and R^{Zd} are the same or different and each independently a hydrogen atom or R^{Z2}; or R^{Za} and R^{Zb}, together with the carbon atoms to which they are attached, form ring D, or R^{Zb} and R^{Zc}, together with the carbon atoms to which they are attached, form ring D, or R^{Zc} and R^{Zd}, together with the carbon atoms to which they are attached, form ring D; the ring D is optionally substituted with c R^{Z2}; and -̅ -̅ -̅, R^{Z2a}, X, X⁵, R^{Z2}, ring D, and c are as defined for general formula (IN).

In some embodiments of the present invention, in the compound represented by general formula (I), (IN), (IN-1), and (IO) or the pharmaceutically acceptable salt thereof, R^{Z2a} is selected from the group consisting of a hydrogen atom, halogen, C₁₋₆ alkyl, C₁₋₆ alkoxy, C₁₋₆ haloalkyl, and C₁₋₆ haloalkoxy; and in some embodiments, R^{Z2a} is a hydrogen atom.

In some embodiments of the present invention, in the compound represented by general formula (I), (IN), (IN-1), (IP) to (IP-3), and (IO) or the pharmaceutically acceptable salt thereof, -̅ -̅ -̅ is a single bond; and in some embodiments, -̅ -̅ -̅ is a double bond.

In some embodiments of the present invention, in the compound represented by general formula (I), (IN), (IN-1), (IP-1) to (IP-3), and (IO) or the pharmaceutically acceptable salt thereof, X is selected from the group consisting of CH₂, CH₂CH₂, CH₂O, OCH₂, O, NH, and N(methyl); in some embodiments, X is selected from the group consisting of CH₂O, OCH₂, CH₂, O, and N(methyl); in some embodiments, X is O; and in some embodiments, X is selected from the group consisting of CH₂, CH₂CH₂, CH₂O, OCH₂, O, NH, N(methyl), CH(methyl), CH cyclopropyl, CF₂, and

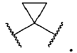

In some embodiments of the present invention, in the compound represented by general formula (I), (IN), (IN-1), (IP-1) to (IP-3), or (IO) or the pharmaceutically acceptable salt thereof, X⁵ is selected from the group consisting of CH₂, CH₂CH₂, CH₂O, OCH₂, O, NH, and N(methyl); in some embodiments, X⁵ is CH₂; and in some embodiments, X⁵ is O or CH₂.

In some embodiments of the present invention, in the compound represented by general formula (I), (IN), (IN-1), (IP-1) to (IP-3), or (IO) or the pharmaceutically acceptable salt thereof, 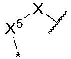 is selected from the group consisting of 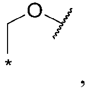 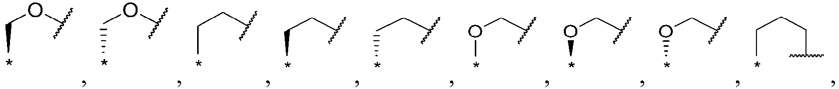 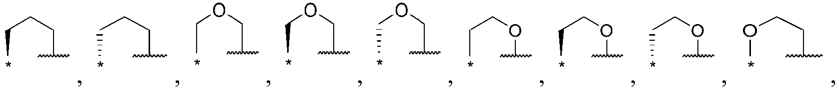 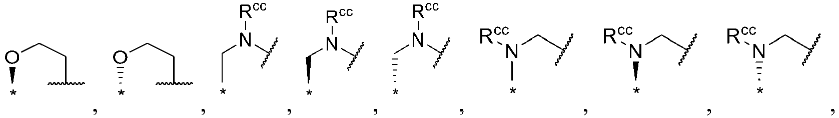 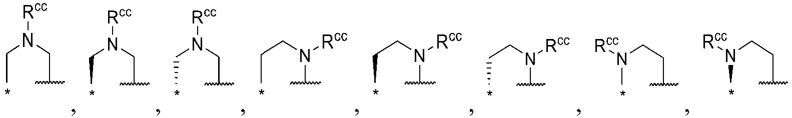 and 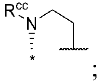 in some embodiments, 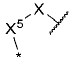 is selected from the group consisting of 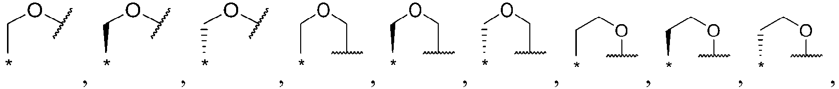 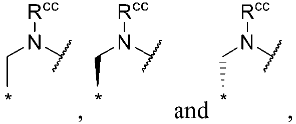 wherein R^{cc} is a hydrogen atom or C₁₋₆ alkyl (in some embodiments, R^{cc} is a hydrogen atom or methyl); in some embodiments, 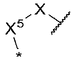 is selected from the group consisting of 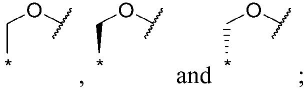 and in some embodiments, 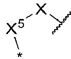 is selected from the group consisting of

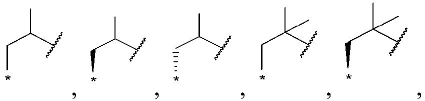

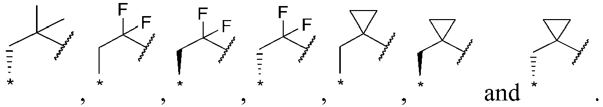

In some embodiments of the present invention, in the compound represented by general formula (I), (IP), or (IP-1) or the pharmaceutically acceptable salt thereof, R^{Za}, R^{Zb}, R^{Zc}, and R^{Zd} are the same or different and are each independently selected from the group consisting of a hydrogen atom, halogen, C₁₋₆ alkyl, C₁₋₆ alkoxy, C₁₋₆ haloalkyl, C₁₋₆ haloalkoxy, NR^{11a}R^{11b}, and P(O)R¹²R¹³, and R^{11a}, R^{11b}, R¹², and R¹³ are as defined for general formula (I); in some embodiments, R^{Za}, R^{Zb}, R^{Zc}, and R^{Zd} are the same or different and are each independently selected from the group consisting of a hydrogen atom, halogen, NR^{11a}R^{11b}, and P(O)R¹²R¹³; and R^{11a}, R^{11b}, R¹², and R¹³ are as defined for general formula (I); and in some embodiments, R^{Za}, R^{Zb}, R^{Zc}, and R^{Zd} are the same or different and are each independently selected from the group consisting of a hydrogen atom, F, methylamino, and

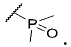

In some embodiments of the present invention, in the compound represented by general formula (I), (IP), or (IP-1) or the pharmaceutically acceptable salt thereof, R^{Zb} and R^{Zc} are the same or different and are each independently selected from the group consisting of a hydrogen atom, halogen, C₁₋₆ alkyl, C₁₋₆ alkoxy, C₁₋₆ haloalkyl, C₁₋₆ haloalkoxy, NR^{11a}R^{11b}, and P(O)R¹²R¹³, or R^{Zb} and R^{Zc}, together with the carbon atoms to which they are attached, form ring D, wherein ring D is optionally substituted with c R^{Z2}, and R^{11a}, R^{11b}, R¹², R¹³, R^{Z2}, ring D, and c are as defined for general formula (IN); in some embodiments, R^{Zb} and R^{Zc} are the same or different and are each independently selected from the group consisting of a hydrogen atom, halogen, NR^{11a}R^{11b}, and P(O)R¹²R¹³, or R^{Zb} and R^{Zc}, together with the carbon atoms to which they are attached, form ring D, wherein ring D is optionally substituted with c R^{Z2}, and R^{11a}, R^{11b}, R¹², R¹³, R^{Z2}, ring D, and c are as defined for general formula (IN); in some embodiments, R^{Zb} and R^{Zc}, together with the carbon atoms to which they are attached, form ring D, wherein ring D is optionally substituted with c R^{Z2}; and ring D, c, and R^{Z2} are as defined for general formula (IN).

In some embodiments of the present invention, in the compound represented by general formula (I), (IP), or (IP-1) or the pharmaceutically acceptable salt thereof, R^{Zb} is selected from the group consisting of a hydrogen atom, halogen, and NR^{11a}R^{11b}, and R^{11a} and R^{11b} are as defined for general formula (I); in some embodiments, R^{Zb} is NR^{11a}R^{11b}, and R^{11a} and R^{11b} are the same or different and each independently a hydrogen atom or C₁₋₆ alkyl; and in some embodiments, R^{Zb} is methylamino.

In some embodiments of the present invention, in the compound represented by general formula (I), (IP), or (IP-1) or the pharmaceutically acceptable salt thereof, R^{Zc} is selected from the group consisting of a hydrogen atom, halogen, and P(O)R¹²R¹³, and R¹² and R¹³ are as defined for general formula (I); in some embodiments, R^{Zc} is F or 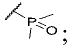 in some embodiments, R^{Zc} is halogen; in some embodiments, R^{Zc} is P(O)R¹²R¹³, and R¹² and R¹³ are the same or different and are each independently C₁₋₆ alkyl; and in some embodiments, R^{Zc} is

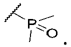

In some embodiments of the present invention, in the compound represented by general formula (I), (IN), (IN-1), (IP), or (IP-1) to (IP-3) or the pharmaceutically acceptable salt thereof, R^{Za} and R^{Zd} are the same or different and are each independently selected from the group consisting of a hydrogen atom, halogen, C₁₋₆ alkyl, and C₁₋₆ haloalkyl; in some embodiments, R^{Za} and R^{Zd} are the same or different and are each independently a hydrogen atom or halogen; in some embodiments, R^{Za} is a hydrogen atom, and/or R^{Zd} is halogen; and in some embodiments, R^{Za} is a hydrogen atom, and/or R^{Zd} is F.

In some embodiments of the present invention, in the compound represented by general formula (I), (IN), or (IP-2) or the pharmaceutically acceptable salt thereof, ring D is 3- to 10-membered heterocyclyl or 5- to 10-membered heteroaryl; in some embodiments, ring D is 4- to 7-membered heterocyclyl or 5- to 10-membered heteroaryl; in some embodiments, ring D is 5- to 10-membered heteroaryl; in some embodiments, ring D is 5-membered heteroaryl; in some embodiments, ring D is pyrazolyl; in some embodiments, ring D is selected from the group consisting of 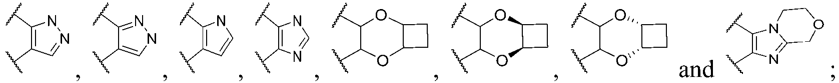 in some embodiments, ring D is 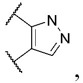 wherein each 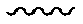 is the site of fusion with phenyl.

In some embodiments of the present invention, in the compound represented by general formula (I) to (IV'), (IM), (IM-1), (IM-2), (IN), (IN-1), (IP) to (IP-3), or (IO) or the pharmaceutically acceptable salt thereof, each R^{Z2} is the same or different and is each independently selected from the group consisting of halogen, cyano, C₁₋₆ alkyl, C₁₋₆ alkoxy, C₁₋₆ alkoxy C₁₋₆ alkyl, 3- to 6-membered cycloalkyl, 3- to 6-membered heterocyclyl, NR^{11a}R^{11b}, and P(O)R¹²R¹³, wherein the C₁₋₆ alkyl, C₁₋₆ alkoxy, C₁₋₆ alkoxy C₁₋₆ alkyl, 3- to 6-membered cycloalkyl, and 3- to 6-membered heterocyclyl are each independently and optionally substituted with one or more selected from the group consisting of a deuterium atom, oxo, halogen, cyano, C₁₋₆ alkyl, C₁₋₆ alkoxy, 3- to 6-membered cycloalkyl, and 3- to 6-membered heterocyclyl, and R^{11a}, R^{11b}, R¹², and R¹³ are as defined for general formula (I); in some embodiments, each R^{Z2} is the same or different and is each independently selected from the group consisting of halogen, C₁₋₆ alkyl, C₁₋₆ haloalkyl, NR^{11a}R^{11b}, and P(O)R¹²R¹³, and R^{11a}, R^{11b}, R¹², and R¹³ are as defined for general formula (I); in some embodiments, each R^{Z2} is the same or different and is each independently selected from the group consisting of halogen, C₁₋₆ alkyl, and C₁₋₆ haloalkyl; in some embodiments, R^{Z2} is methyl or F; in some embodiments, each R^{Z2} is the same or different and is each independently C₁₋₆ alkyl; in some embodiments, R^{Z2} is methyl; in some embodiments, each R^{Z2} is the same or different and is each independently selected from the group consisting of halogen, C₁₋₆ alkyl, C₁₋₆ haloalkyl, C₁₋₆ deuterioalkyl, and 3- to 6-membered cycloalkyl; in some embodiments, each R^{Z2} is the same or different and is each independently selected from the group consisting of F, methyl, deuteriomethyl, and cyclopropyl; in some embodiments, each R^{Z2} is the same or different and is each independently selected from the group consisting of halogen, cyano, C₁₋₆ alkyl, C₁₋₆ haloalkyl, C₁₋₆ deuterioalkyl, C₁₋₆ alkoxy, C₁₋₆ haloalkoxy, C₁₋₆ alkoxy C₁₋₆ alkyl, 3- to 6-membered cycloalkyl, NR^{11a}R^{11b}, and P(O)R¹²R¹³, and R^{11a}, R^{11b}, R¹², and R¹³ are the same or different and are each independently a hydrogen atom or C₁₋₆ alkyl; in some embodiments, each R^{Z2} is the same or different and is each independently selected from the group consisting of halogen, cyano, C₁₋₆ alkyl, C₁₋₆ haloalkyl, C₁₋₆ deuterioalkyl, C₁₋₆ alkoxy, C₁₋₆ haloalkoxy, 3- to 6-membered cycloalkyl, and C₁₋₆ alkoxy C₁₋₆ alkyl; in some embodiments, each R^{Z2} is the same or different and is each independently selected from the group consisting of F, cyano, methyl, methoxy, trideuteriomethyl, cyclopropyl, difluoromethyl, difluoroethyl, trifluoroethyl, and methoxymethyl; In some embodiments, each R^{Z2} is the same or different and is each independently selected from the group consisting of halogen, C₁₋₆ alkyl, C₁₋₆ alkoxy, C₁₋₆ alkoxy C₁₋₆ alkyl, and 3- to 6-membered cycloalkyl, wherein the C₁₋₆ alkyl, C₁₋₆ alkoxy, and 3- to 6-membered cycloalkyl are each independently and optionally substituted with one or more selected from the group consisting of a deuterium atom, halogen, and 3- to 6-membered cycloalkyl; in some embodiments, each R^{Z2} is the same or different and is each independently selected from the group consisting of halogen, C₁₋₆ alkyl, C₁₋₆ haloalkyl, C₁₋₆ deuterioalkyl, C₁₋₆ alkoxy, C₁₋₆ haloalkoxy, 3- to 6-membered cycloalkyl, C₁₋₆ alkoxy C₁₋₆ alkyl, and 3- to 6-membered cycloalkyl C₁₋₆ alkyl; in some embodiments, R^{Z2} is tert-butyl or -CH₂ cyclopropyl.

In some embodiments of the present invention, in the compound represented by general formula (I), (IN), or (IP-2) or the pharmaceutically acceptable salt thereof, c is 0, 1, or 2; in some embodiments, c is 0 or 1; and in some embodiments, c is 1.

In some embodiments of the present invention, in the compound represented by general formula (I), (IN-1), or (IP-3) or the pharmaceutically acceptable salt thereof, X⁶ is NR^{cc}, and R^{cc} is as defined for general formula (IN); in some embodiments, X⁶ is NR^{cc}, and R^{cc} is a hydrogen atom or methyl; and in some embodiments, X⁶ is N(methyl).

In some embodiments of the present invention, in the compound represented by general formula (I), (IN-1), or (IP-3) or the pharmaceutically acceptable salt thereof, X⁷ is CH or N; and in some embodiments, X⁷ is N.

In some embodiments of the present invention, in the compound represented by general formula (I), (IN-1), or (IP-3) or the pharmaceutically acceptable salt thereof, X⁸ is CH or N; and in some embodiments, X⁸ is CH.

In some embodiments of the present invention, in the compound represented by general formula (I), (IN), (IN-1), (IP-1) to (IP-3), or (IO) or the pharmaceutically acceptable salt thereof, R^{cc} is a hydrogen atom or C₁₋₆ alkyl; in some embodiments, R^{cc} is C₁₋₆ alkyl; in some embodiments, R^{cc} is methyl; in some embodiments, R^{cc} is a hydrogen atom; in some embodiments, R^{cc} is selected from the group consisting of a hydrogen atom, C₁₋₆ alkyl, 3- to 6-membered cycloalkyl, and C₁₋₆ deuterioalkyl; in some embodiments, R^{cc} is selected from the group consisting of a hydrogen atom, methyl, cyclopropyl, and deuteriomethyl; in some embodiments, R^{cc} is selected from the group consisting of a hydrogen atom, C₁₋₆ alkyl, C₁₋₆ haloalkyl, C₁₋₆ deuterioalkyl, C₁₋₆ alkoxy C₁₋₆ alkyl, 3- to 6-membered cycloalkyl, and C₁₋₆ hydroxyalkyl; in some embodiments, R^{cc} is selected from the group consisting of a hydrogen atom, methyl, cyclopropyl, deuteriomethyl, difluoromethyl, difluoroethyl, trifluoroethyl, and methoxymethyl; in some embodiments, R^{cc} is methoxyethyl or isopropyl; in some embodiments, R^{cc} is methoxyethyl; in some embodiments, R^{cc} is isopropyl; in some embodiments, R^{cc} is tert-butyl or -CH₂ cyclopropyl; in some embodiments, R^{cc} is a hydrogen atom or R^{Z2}; in some embodiments, R^{cc} is selected from the group consisting of a hydrogen atom, C₁₋₆ alkyl, and 3- to 8-membered cycloalkyl, wherein the C₁₋₆ alkyl and 3- to 8-membered cycloalkyl are each independently and optionally substituted with one or more R^{Z2}; and R^{Z2} is as defined for general formula (I).

In some embodiments of the present invention, in the compound represented by general formula (I), (IN-1), or (IP-3) or the pharmaceutically acceptable salt thereof, R^{Ze} is selected from the group consisting of a hydrogen atom, halogen, C₁₋₆ alkyl, C₁₋₆ alkoxy, C₁₋₆ haloalkyl, and C₁₋₆ haloalkoxy; and in some embodiments, R^{Ze} is a hydrogen atom.

In some embodiments of the present invention, in the compound represented by general formula (I), (IN-1), or (IP-3) or the pharmaceutically acceptable salt thereof, R^{Zh} is selected from the group consisting of a hydrogen atom, halogen, C₁₋₆ alkyl, C₁₋₆ alkoxy, C₁₋₆ haloalkyl, and C₁₋₆ haloalkoxy; and in some embodiments, R^{Zh} is a hydrogen atom.

In some embodiments of the present invention, in the compound represented by general formula (I) or (IP) or the pharmaceutically acceptable salt thereof, R^{Z2b} is selected from the group consisting of a hydrogen atom, halogen, C₁₋₆ alkyl, C₁₋₆ alkoxy, C₁₋₆ haloalkyl, and C₁₋₆ haloalkoxy; and in some embodiments, R^{Z2b} is a hydrogen atom.

In some embodiments of the present invention, in the compound represented by general formula (I) or (IO) or the pharmaceutically acceptable salt thereof, R^{Zf} and R^{Zg} are the same or different and are each independently selected from the group consisting of a hydrogen atom, halogen, C₁₋₆ alkyl, C₁₋₆ alkoxy, C₁₋₆ haloalkyl, C₁₋₆ haloalkoxy, phenyl, and 5- or 6-membered heteroaryl, wherein the phenyl and 5- or 6-membered heteroaryl are each independently and optionally substituted with one or more selected from the group consisting of halogen, C₁₋₆ alkyl, and C₁₋₆ haloalkyl; in some embodiments, R^{Zf} is a hydrogen atom, R^{Zg} is phenyl, or R^{Zg} is a hydrogen atom, and R^{Zf} is phenyl, wherein the phenyl is optionally substituted with one or more selected from the group consisting of halogen, C₁₋₆ alkyl, and C₁₋₆ haloalkyl; in some embodiments, R^{Zf} is a hydrogen atom, R^{Zg} is halophenyl, or R^{Zg} is a hydrogen atom, and R^{Zf} is halophenyl; in some embodiments, R^{Zf} is a hydrogen atom, R^{Zg} is 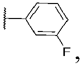 or R^{Zg} is a hydrogen atom, and R^{Zf} is 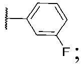 in some embodiments, R^{Zf} and R^{Zg}, together with the carbon atoms to which they are attached, form ring E, ring E is optionally substituted with 1, 2, 3, or 4 R^{Z2}, and ring E and R^{Z2} are as defined for general formula (IO); and in some embodiments, R^{Zf} and R^{Zg}, together with the carbon atoms to which they are attached, form indazolyl optionally substituted with 1, 2, 3, or 4 R^{Z2}, and R^{Z2} is as defined for general formula (I).

In some embodiments of the present invention, in the compound represented by general formula (I) or (IO) or the pharmaceutically acceptable salt thereof, ring E is 6- to 10-membered aryl or 5- to 14-membered heteroaryl; in some embodiments, ring E is phenyl or 5- to 14-membered heteroaryl; in some embodiments, ring E is phenyl or indazolyl; in some embodiments, ring E is 5- to 14-membered heteroaryl; in some embodiments, ring E is indazolyl; in some embodiments, ring E is 9- or 10-membered bicyclic heteroaryl; in some embodiments, ring E is 6- to 10-membered aryl; and in some embodiments, ring E is phenyl.

In some embodiments of the present invention, in the compound represented by general formula (I) to (IV'), (IM) to (IM-2), (IN), (IN-1), (IP) to (IP-3), or (IO) or the pharmaceutically acceptable salt thereof, Q¹ is 6- or 14-aryl or 5- to 14-membered heteroaryl, wherein the 6- or 14-aryl and 5- to 14-membered heteroaryl are each independently and optionally substituted with one or more R^{Q1}, and R^{Q1} is as defined for general formula (I); in some embodiments, Q¹ is phenyl or 6-membered heteroaryl, wherein the phenyl or 6-membered heteroaryl is optionally substituted with one or more R^{Q1}, and R^{Q1} is as defined for general formula (I); in some embodiments, Q¹ is phenyl optionally substituted with one or more R^{Q1}, and R^{Q1} is as defined for general formula (I); in some embodiments, Q¹ is 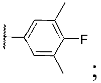 in some embodiments, Q¹ is 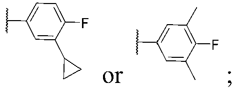 in some embodiments, Q¹ is 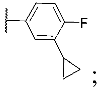
in some embodiments, Q¹ is 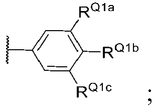 in some embodiments, Q¹ is 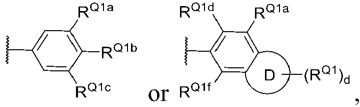 R^{Q1a}, R^{Q1b}, R^{Q1c}, R^{Q1d}, and R^{Q1f} are the same or different and are each independently a hydrogen atom or R^{Q1}, ring D is heterocyclyl or heteroaryl, and d is 0, 1, 2, 3, or 4; and R^{Q1} is as defined for general formula (I); in some embodiments, Q¹ is selected from the group consisting of 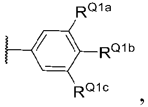 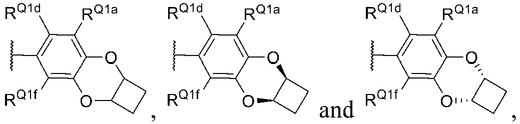 R^{Q1a}, R^{Q1b}, R^{Q1c}, R^{Q1d}, and R^{Q1f} are the same or different and are each independently a hydrogen atom or R^{Q1}, and R^{Q1} is as defined for general formula (I); in some embodiments, Q¹ is selected from the group consisting of 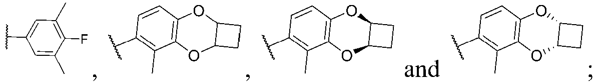 in some embodiments, Q¹ is 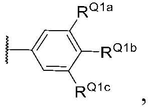 R^{Q1a}, R^{Q1b}, and R^{Q1c} are the same or different and are each independently selected from the group consisting of a hydrogen atom, halogen, C₁₋₆ alkyl, and C₁₋₆ haloalkyl; and
in some embodiments, Q¹ is cycloalkyl or heterocyclyl, the cycloalkyl and heterocyclyl are each independently and optionally substituted with one or more R^{Q1}, and R^{Q1} is as defined for general formula (I).

In some embodiments of the present invention, in the compound represented by general formula (I) to (IV'), (IM) to (IM-2), (IN), (IN-1), (IP) to (IP-3), or (IO) or the pharmaceutically acceptable salt thereof, each R^{Q1} is the same or different and is each independently selected from the group consisting of halogen, C₁₋₆ alkyl, and C₁₋₆ haloalkyl; and in some embodiments, each R^{Q1} is the same or different and is each independently methyl or F.

In some embodiments of the present invention, R^{Q1a}, R^{Q1b}, and R^{Q1c} are the same or different and are each independently selected from the group consisting of a hydrogen atom, halogen, C₁₋₆ alkyl, C₁₋₆ haloalkyl, and 3- to 6-membered cycloalkyl; in some embodiments, R^{Q1a} is C₁₋₆ alkyl, and/or R^{Q1b} is halogen, and/or R^{Q1c} is C₁₋₆ alkyl; in some embodiments, R^{Q1a} is methyl, and/or R^{Q1b} is F, and/or R^{Q1c} is methyl; in some embodiments, R^{Q1a}, R^{Q1b}, and R^{Q1c} are the same or different and are each independently selected from the group consisting of a hydrogen atom, F, methyl, and cyclopropyl; and in some embodiments, R^{Q1a}, R^{Q1b}, and R^{Q1c} are the same or different and are each independently selected from the group consisting of a hydrogen atom, halogen, C₁₋₆ alkyl, and 3- to 6-membered cycloalkyl.

In some embodiments of the present invention, R^{Q1a}, R^{Q1d}, and R^{Q1f} are the same or different and are each independently selected from the group consisting of a hydrogen atom, halogen, C₁₋₆ alkyl, and C₁₋₆ haloalkyl; in some embodiments, R^{Q1a} is a hydrogen atom, and/or R^{Q1d} is a hydrogen atom, and/or R^{Q1f} is C₁₋₆ alkyl; and in some embodiments, R^{Q1a} is a hydrogen atom, and/or R^{Q1c} is a hydrogen atom, and/or R^{Q1f} is methyl.

In some embodiments of the present invention, in the compound represented by general formula (I), (III), (IV), (IV'), (IM) to (IM-2), (IN), (IN-1), (IP) to (IP-3), or (IO) or the pharmaceutically acceptable salt thereof, R⁹ is 3- to 6-membered heterocyclyl or 5- or 6-membered heteroaryl, the 3- to 6-membered heterocyclyl and 5- or 6-membered heteroaryl are each independently and optionally substituted with one or more R^{9a}, and R^{9a} is as defined for general formula (I); in some embodiments, R⁹ is 5-membered heterocyclyl optionally substituted with one or more R^{9a}, and R^{9a} is as defined for general formula (I); in some embodiments, R⁹ is 5-membered heterocyclyl optionally substituted with one or more R^{9a}, and at least one R^{9a} is oxo; in some embodiments, R⁹ is 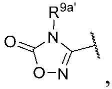 and R^{9a}, is a hydrogen atom or C₁₋₆ alkyl; in some embodiments, R⁹ is 5-membered heteroaryl optionally substituted with one or more R^{9a}, and R^{9a} is as defined for general formula (I); in some embodiments, R⁹ is 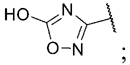 in some embodiments, R⁹ is 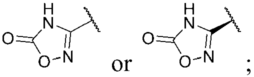 and in some embodiments, R⁹ is

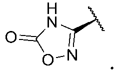

In some embodiments of the present invention, in the compound represented by general formula (I), (III), (IV), (IV'), (IM) to (IM-2), (IN), (IN-1), (IP) to (IP-3), or (IO) or the pharmaceutically acceptable salt thereof, R⁷ and R⁸, together with the atoms to which they are attached, form 3- to 5-membered cycloalkyl, the 3- to 5-membered cycloalkyl is optionally substituted with one or more R⁷⁸, and R⁷⁸ is as defined for general formula (I); in some embodiments, R⁷ and R⁸, together with the atoms to which they are attached, form cyclopropyl optionally substituted with one or more R⁷⁸, and R⁷⁸ is as defined for general formula (I); and in some embodiments, R⁷ and R⁸, together with the atoms to which they are attached, form cyclopropyl optionally substituted with methyl.

In some embodiments of the present invention, in the compound represented by general formula (I), (III), (IV), (IV'), (IM) to (IM-2), (IN), (IN-1), (IP) to (IP-3), or (IO) or the pharmaceutically acceptable salt thereof, n1 is 0 or 1, and/or n2 is 0 or 1; and in some embodiments, n1 is 0, and/or n2 is 0.

(IN), (IN-1) In some embodiments of the present invention, in the compound represented by general formula (I), (III), (IV), (IV'), (IM) to (IM-2), (IN), (IN-1), (IP) to (IP-3), or (IO) or the pharmaceutically acceptable salt thereof, R^{A} is 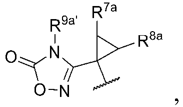 and R^{7a} and R^{8a} are the same or different and are each independently a hydrogen atom or R⁷⁸; R^{9a}' is a hydrogen atom or C₁₋₆ alkyl, and R⁷⁸ is as defined for general formula (I);
in some embodiments, R^{A} is 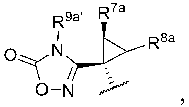 and R^{7a} and R^{8a} are different and are each independently a hydrogen atom or R⁷⁸; R^{9a}, is a hydrogen atom or C₁₋₆ alkyl, and R⁷⁸ is as defined for general formula (I);
in some embodiments, R^{A} is 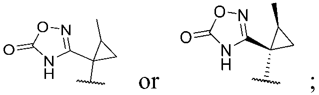 in some embodiments, R^{A} is 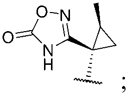 in some embodiments, R^{A} is 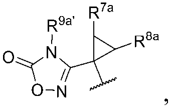 and R^{7a} and R^{8a} are the same or different and are each independently a hydrogen atom or R⁷⁸; R^{9a}, is selected from the group consisting of a hydrogen atom, C₁₋₆ alkyl, C₁₋₆ haloalkyl, and 3- to 6-membered cycloalkyl, and R⁷⁸ is as defined for general formula (I); and in some embodiments, R^{A} is

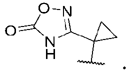

In some embodiments of the present invention, in the compound represented by general formula (I) or (II) or the pharmaceutically acceptable salt thereof, R^{7a} and R^{8a} are the same or different and are each independently selected from the group consisting of a hydrogen atom, halogen, C₁₋₆ alkyl, and C₁₋₆ haloalkyl; and in some embodiments, R^{7a} and R^{8a} are the same or different and are each independently a hydrogen atom or C₁₋₆ alkyl.

In some embodiments of the present invention, in the compound represented by general formula (I) or (II) or the pharmaceutically acceptable salt thereof, R^{7a} and R^{8a} are different and are each independently selected from the group consisting of a hydrogen atom, halogen, C₁₋₆ alkyl, and C₁₋₆ haloalkyl; and in some embodiments, R^{7a} and R^{8a} are different and are each independently a hydrogen atom or C₁₋₆ alkyl.

In some embodiments of the present invention, in the compound represented by general formula (I) or (II) or the pharmaceutically acceptable salt thereof, R^{7a} is a hydrogen atom or C₁₋₆ alkyl; in some embodiments, R^{7a} is C₁₋₆ alkyl; and in some embodiments, R^{7a} is methyl.

In some embodiments of the present invention, in the compound represented by general formula (I) or (II) or the pharmaceutically acceptable salt thereof, R^{8a} is a hydrogen atom or C₁₋₆ alkyl; and in some embodiments, R^{8a} is a hydrogen atom.

In some embodiments of the present invention, in the compound represented by general formula (I) or (II) or the pharmaceutically acceptable salt thereof, each R⁷⁸ is the same or different and is each independently selected from the group consisting of halogen, C₁₋₆ alkyl, and C₁₋₆ haloalkyl; in some embodiments, each R⁷⁸ is the same or different and is each independently C₁₋₆ alkyl; and in some embodiments, R⁷⁸ is methyl.

In some embodiments of the present invention, in the compound represented by general formula (I) or (II) or the pharmaceutically acceptable salt thereof, R^{9a'} is selected from the group consisting of a hydrogen atom, C₁₋₆ alkyl, C₁₋₆ haloalkyl, 3-to 6-membered cycloalkyl, and 3- to 6-membered cycloalkyl C₁₋₆ alkyl; in some embodiments, R^{9a'} is a hydrogen atom or C₁₋₆ alkyl; and in some embodiments, R^{9a'} is a hydrogen atom.

In some embodiments of the present invention, in the compound represented by general formula (I) or (II) or the pharmaceutically acceptable salt thereof, each R^{9a} is the same or different and is each independently selected from the group consisting of halogen, C₁₋₆ alkyl, and C₁₋₆ haloalkyl; in some embodiments, each R^{9a} is the same or different and is each independently C₁₋₆ alkyl; and in some embodiments, R^{9a} is methyl.

In some embodiments of the present invention, in the compound represented by general formula (I) to (IV'), (IM) to (IM-2), (IN), (IN-1), (IP) to (IP-3), or (IO) or the pharmaceutically acceptable salt thereof, R³ is selected from the group consisting of a hydrogen atom, halogen, C₁₋₆ alkyl, and 3- to 6-membered cycloalkyl; in some embodiments, R³ is C₁₋₆ alkyl; and in some embodiments, R³ is methyl.

In some embodiments of the present invention, in the compound represented by general formula (I) to (IV'), (IM) to (IM-2), (IN), (IN-1), (IP) to (IP-3), or (IO) or the pharmaceutically acceptable salt thereof, R¹ is a hydrogen atom, and/or R² is a hydrogen atom, and/or R³ is C₁₋₆ alkyl.

In some embodiments of the present invention, R¹ is a hydrogen atom, and/or R² is a hydrogen atom, and/or R³ is C₁₋₆ alkyl, and/or R⁴, R⁵, and R⁶ are hydrogen atoms, and/or X¹ is CH.

In some embodiments of the present invention, in the compound represented by general formula (I) to (IV'), (IM) to (IM-2), (IN), (IN-1), (IP) to (IP-3), or (IO) or the pharmaceutically acceptable salt thereof, R¹ is selected from the group consisting of a hydrogen atom, halogen, C₁₋₆ alkyl, and 3- to 6-membered cycloalkyl; in some embodiments, R¹ is a hydrogen atom or C₁₋₆ alkyl; and in some embodiments, R¹ is a hydrogen atom.

In some embodiments of the present invention, in the compound represented by general formula (I) to (IV'), (IM) to (IM-2), (IN), (IN-1), (IP) to (IP-3), or (IO) or the pharmaceutically acceptable salt thereof, R² is selected from the group consisting of a hydrogen atom, halogen, C₁₋₆ alkyl, and 3- to 6-membered cycloalkyl; in some embodiments, R² is a hydrogen atom or C₁₋₆ alkyl; and in some embodiments, R² is a hydrogen atom.

In some embodiments of the present invention, in the compound represented by general formula (I) to (IV'), (IM) to (IM-2), (IN), (IN-1), (IP) to (IP-3), or (IO) or the pharmaceutically acceptable salt thereof, R⁴ is selected from the group consisting of a hydrogen atom, halogen, C₁₋₆ alkyl, and C₁₋₆ haloalkyl; and in some embodiments, R⁴ is a hydrogen atom.

In some embodiments of the present invention, in the compound represented by general formula (I) to (IV'), (IM) to (IM-2), (IN), (IN-1), (IP) to (IP-3), or (IO) or the pharmaceutically acceptable salt thereof, R⁵ is selected from the group consisting of a hydrogen atom, halogen, C₁₋₆ alkyl, and C₁₋₆ haloalkyl; and in some embodiments, R⁵ is a hydrogen atom.

In some embodiments of the present invention, in the compound represented by general formula (I) to (IV'), (IM) to (IM-2), (IN), (IN-1), (IP) to (IP-3), or (IO) or the pharmaceutically acceptable salt thereof, R⁶ is selected from the group consisting of a hydrogen atom, halogen, C₁₋₆ alkyl, and C₁₋₆ haloalkyl; and in some embodiments, R⁶ is a hydrogen atom.

In some embodiments of the present invention, R⁴ is a hydrogen atom, and/or R⁵ is a hydrogen atom, and/or R⁶ is a hydrogen atom. In some embodiments of the present invention, R⁴ is a hydrogen atom, and/or R⁵ is a hydrogen atom. In some embodiments of the present invention, R⁴ is a hydrogen atom, and/or R⁶ is a hydrogen atom.

In some embodiments of the present invention, in the compound represented by general formula (I) to (IV'), (IM) to (IM-2), (IN), (IN-1), (IP) to (IP-3), or (IO) or the pharmaceutically acceptable salt thereof, X¹ is CH or N; in some embodiments, X¹ is CH; and in some embodiments, X¹ is CR^{x1}, and R^{x1} is selected from the group consisting of a hydrogen atom, halogen, C₁₋₆ alkyl, C₁₋₆ haloalkyl, and 3- to 6-membered cycloalkyl.

In some embodiments of the present invention, in the compound represented by the general formula or the pharmaceutically acceptable salt thereof, R^{x1} is selected from the group consisting of a hydrogen atom, C₁₋₆ alkyl, and C₁₋₆ haloalkyl; and in some embodiments, R^{x1} is a hydrogen atom.

In some embodiments of the present invention, in the compound represented by general formula (I) or the pharmaceutically acceptable salt thereof, ring C is 3-to 8-membered cycloalkyl or 3- to 8-membered heterocyclyl; in some embodiments, ring C is 5- or 6-membered heterocyclyl; and in some embodiments, ring C is 6- or 7-membered heterocyclyl.

In some embodiments of the present invention, each R* is the same or different and is each independently selected from the group consisting of a deuterium atom, halogen, C₁₋₆ alkyl, C₁₋₆ alkoxy, cyano, amino, hydroxyl, oxo, 3- to 6-membered cycloalkyl, 3- to 6-membered heterocyclyl, 3- to 6-membered cycloalkyl C₁₋₆ alkyl, 3- to 6-membered heterocyclyl C₁₋₆ alkyl, 6- to 10-membered aryl, and 5- to 10-membered heteroaryl; and in some embodiments, each R* is the same or different and is each independently selected from the group consisting of halogen, C₁₋₆ alkyl, and C₁₋₆ haloalkyl.

In some embodiments of the present invention, each R^{C} is the same or different and is each independently selected from the group consisting of halogen, C₁₋₆ alkyl, and C₁₋₆ haloalkyl.

In some embodiments of the present invention, R^{11a} and R^{11b} are the same or different and are each independently a hydrogen atom or C₁₋₆ alkyl; in some embodiments, R^{11a} and R^{11b} are the same or different and are each independently a hydrogen atom or methyl; in some embodiments, R^{11a} and R^{11b} are both hydrogen atoms; and in some embodiments, R^{11a} is a hydrogen atom, and/or R^{11b} is methyl.

In some embodiments of the present invention, R^{aa}, R^{bb}, R⁰⁰, and R⁰ are the same or different and are each independently a hydrogen atom or C₁₋₆ alkyl; and in some embodiments, R^{aa}, R^{bb}, R⁰⁰, and R⁰ are the same or different and are each independently a hydrogen atom or methyl.

In some embodiments of the present invention, R¹² and R¹³ are the same or different and are each independently a hydrogen atom or C₁₋₆ alkyl; in some embodiments, R¹² and R¹³ are the same or different and are each independently C₁₋₆ alkyl; and in some embodiments, R¹² and R¹³ are both methyl.

In some embodiments of the present invention, R¹² is a hydrogen atom or C₁₋₆ alkyl; and in some embodiments, R¹² is a hydrogen atom.

In some embodiments of the present invention, R¹⁵ is a hydrogen atom or C₁₋₆ alkyl; and in some embodiments, R¹⁵ is a hydrogen atom.

In some embodiments of the present invention, R^{14a} and R^{14b} are the same or different and are each independently a hydrogen atom or C₁₋₆ alkyl; in some embodiments, R^{14a} and R^{14b} are the same or different and are each independently a hydrogen atom or methyl; and in some embodiments, R^{14a} and R^{14b} are both hydrogen atoms.

In some embodiments of the present invention, R^{12a} and R^{13a} are the same or different and are each independently selected from the group consisting of a hydrogen atom, halogen, and C₁₋₆ alkyl.

In some embodiments of the present invention, x is 0, 1, or 2; and in some embodiments, x is 0.

In some embodiments of the present invention, y is 0, 1, or 2; and in some embodiments, y is 0.

In some embodiments of the present invention, R^{Z2c}, R^{Z2d}, R^{Z2e}, and R^{Z2f} are the same or different and are each independently selected from the group consisting of a hydrogen atom, halogen, cyano, C₁₋₆ alkyl, C₁₋₆ haloalkyl, C₁₋₆ alkoxy, C₁₋₆ haloalkoxy, and 3- to 6-membered cycloalkyl, or R^{Z2c} and R^{Z2d}, together with the same carbon atom to which they are attached, form3- to 6-membered cycloalkyl, or R^{Z2c} and R^{Z2d}, together with two adjacent carbon atoms to which they are attached, form3- to 6-membered cycloalkyl, or R^{Z2e} and R^{Z2f}, together with the same carbon atom to which they are attached, form3- to 6-membered cycloalkyl; in some embodiments, R^{Z2c}, R^{Z2d}, R^{Z2e}, and R^{Z2f} are the same or different and are each independently selected from the group consisting of a hydrogen atom, halogen, C₁₋₆ alkyl, and 3- to 6-membered cycloalkyl, or R^{Z2c} and R^{Z2d}, together with the same carbon atom to which they are attached, form cyclopropyl; and in some embodiments, R^{Z2c}, R^{Z2d}, R^{Z2e}, and R^{Z2f} are the same or different and are each independently selected from the group consisting of a hydrogen atom, F, methyl, and cyclopropyl, or R^{Z2c} and R^{Z2d}, together with the same carbon atom to which they are attached, form cyclopropyl.

In some embodiments of the present invention, R^{Z2c} and R^{Z2d} are the same or different and are each independently a hydrogen atom or C₁₋₆ alkyl; and in some embodiments, R^{Z2c} and R^{Z2d} are both hydrogen atoms.

In some embodiments of the present invention, R^{Z2e} and R^{Z2f} are the same or different and are each independently a hydrogen atom or C₁₋₆ alkyl; and in some embodiments, R^{Z2e} and R^{Z2f} are hydrogen atoms.

In the present invention, the * end in 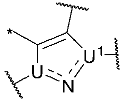 is the site of fusion with the ring to which R³ is attached.

In some embodiments of the present invention, in the compound represented by general formula (IM-2) or the pharmaceutically acceptable salt thereof, 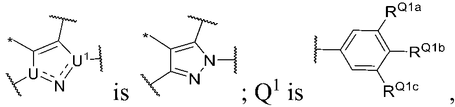 and R^{Q1a}, R^{Q1b}, and R^{Q1c} are the same or different and are each independently selected from the group consisting of a hydrogen atom, halogen, C₁₋₆ alkyl, and C₁₋₆ haloalkyl; a is 0; each R^{Z2} is the same or different and is each independently selected from the group consisting of halogen, C₁₋₆ alkyl, and C₁₋₆ haloalkyl; b is 0 or 1; R^{cc} is C₁₋₆ alkyl; 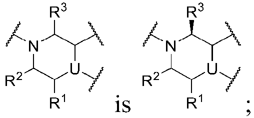 R¹ is a hydrogen atom, R² is a hydrogen atom, and R³ is C₁₋₆ alkyl; Y is C(O); ring A is 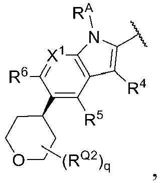 and X¹ is CH; each R^{Q2} is the same or different and is each independently selected from the group consisting of halogen, C₁₋₆ alkyl, and C₁₋₆ haloalkyl; q is 2; R^{A} is 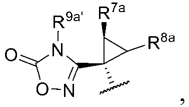 and R^{7a} and R^{8a} are different and each independently a hydrogen atom or R⁷⁸; R^{9a'} is a hydrogen atom or C₁₋₆ alkyl, and each R⁷⁸ is the same or different and is each independently selected from the group consisting of halogen, C₁₋₆ alkyl, and C₁₋₆ haloalkyl; and R⁴ is a hydrogen atom, R⁵ is a hydrogen atom, and R⁶ is a hydrogen atom.

In some embodiments of the present invention, in the compound represented by general formula (IN) or the pharmaceutically acceptable salt thereof, 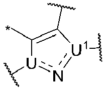 is 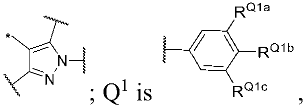 and R^{Q1a}, R^{Q1b}, and R^{Q1c} are the same or different and are each independently selected from the group consisting of a hydrogen atom, halogen, C₁₋₆ alkyl, and C₁₋₆ haloalkyl; -̅-̅-̅ is a single bond; R^{Z2a} is a hydrogen atom; X is selected from the group consisting of CH₂O, OCH₂, CH₂, O, and N(methyl); X⁵ is CH₂; ring D is 4- to 7-membered heterocyclyl or 5- to 10-membered heteroaryl; R^{Za} and R^{Zd} are the same or different and are each independently selected from the group consisting of a hydrogen atom, halogen, C₁₋₆ alkyl, and C₁₋₆ haloalkyl; each R^{Z2} is the same or different and is each independently selected from the group consisting of halogen, C₁₋₆ alkyl, and C₁₋₆ haloalkyl; c is 0 or 1; 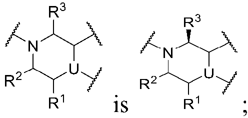 R¹ is a hydrogen atom, R² is a hydrogen atom, and R³ is C₁₋₆ alkyl; Y is C(O); ring A is 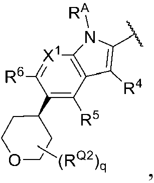 and X¹ is CH; each R^{Q2} is the same or different and is each independently selected from the group consisting of halogen, C₁₋₆ alkyl, and C₁₋₆ haloalkyl; q is 2; R^{A} is 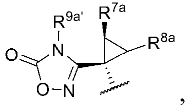 and R^{7a} and R^{8a} are different and each independently a hydrogen atom or R⁷⁸; R^{9a}' is a hydrogen atom or C₁₋₆ alkyl, and each R⁷⁸ is the same or different and is each independently selected from the group consisting of halogen, C₁₋₆ alkyl, and C₁₋₆ haloalkyl; and R⁴ is a hydrogen atom, R⁵ is a hydrogen atom, and R⁶ is a hydrogen atom.

In some embodiments of the present invention, in the compound represented by general formula (IN) or the pharmaceutically acceptable salt thereof, 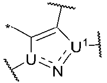 is 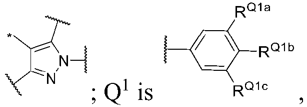 and R^{Q1a}, R^{Q1b}, and R^{Q1c} are the same or different and are each independently selected from the group consisting of a hydrogen atom, halogen, C₁₋₆ alkyl, and C₁₋₆ haloalkyl; -̅-̅-̅ is a single bond; R^{Z2a} is a hydrogen atom; 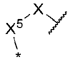 is selected from the group consisting of 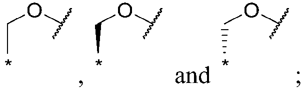 ring D is 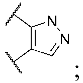 R^{Za} is a hydrogen atom, and R^{Zd} is halogen; each R^{Z2} is the same or different and is each independently C₁₋₆ alkyl; c is 0 or 1; 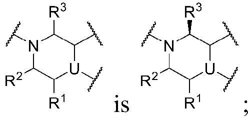 R¹ is a hydrogen atom, R² is a hydrogen atom, and R³ is C₁₋₆ alkyl; Y is C(O); ring A is 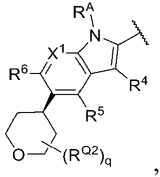 and X¹ is CH; each R^{Q2} is the same or different and is each independently selected from the group consisting of halogen, C₁₋₆ alkyl, and C₁₋₆ haloalkyl; q is 2; R^{A} is 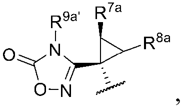 and R^{7a} and R^{8a} are different and each independently a hydrogen atom or R⁷⁸; R^{9a}' is a hydrogen atom or C₁₋₆ alkyl, and each R⁷⁸ is the same or different and is each independently selected from the group consisting of halogen, C₁₋₆ alkyl, and C₁₋₆ haloalkyl; and R⁴ is a hydrogen atom, R⁵ is a hydrogen atom, and R⁶ is a hydrogen atom.

In some embodiments of the present invention, in the compound represented by general formula (II) or the pharmaceutically acceptable salt thereof, Q¹ is 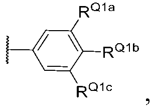 and R^{Q1a}, R^{Q1b}, and R^{Q1c} are the same or different and are each independently selected from the group consisting of a hydrogen atom, halogen, C₁₋₆ alkyl, and C₁₋₆ haloalkyl; R¹ is a hydrogen atom, R² is a hydrogen atom, and R³ is C₁₋₆ alkyl; R⁴ is a hydrogen atom, R⁵ is a hydrogen atom, and R⁶ is a hydrogen atom; X¹ is CH; 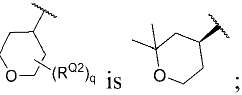 R^{7a} is C₁₋₆ alkyl; R^{8a} is a hydrogen atom; R^{9a'} is a hydrogen atom; Z¹ is 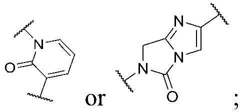 and Z² is selected from the group consisting of a hydrogen atom,

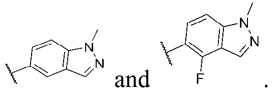

In some embodiments of the present invention, in the compound represented by general formula (IV) or (IV') or the pharmaceutically acceptable salt thereof, X¹ is CH; ring B is 10-membered bicyclic heterocyclyl; each R^{B} is the same or different and is each independently selected from the group consisting of halogen, C₁₋₆ alkyl, and C₁₋₆ haloalkyl; m is 0, 1, or 2; R⁴ is a hydrogen atom, R⁵ is a hydrogen atom, and R⁶ is a hydrogen atom; R^{A} is 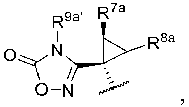 and R^{7a} and R^{8a} are different and each independently a hydrogen atom or R⁷⁸; R^{9a}' is a hydrogen atom or C₁₋₆ alkyl, and each R⁷⁸ is the same or different and is each independently selected from the group consisting of halogen, C₁₋₆ alkyl, and C₁₋₆ haloalkyl; 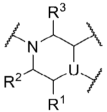 is 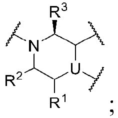 R¹ is a hydrogen atom, R² is a hydrogen atom, and R³ is C₁₋₆ alkyl; Q¹ is 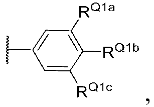 and R^{Q1a}, R^{Q1b}, and R^{Q1c} are the same or different and are each independently selected from the group consisting of a hydrogen atom, halogen, C₁₋₆ alkyl, and C₁₋₆ haloalkyl; Z¹ is 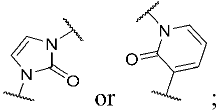 and Z² is a hydrogen atom or 5- to 10-membered heteroaryl optionally substituted with one or more R^{Z2}; and each R^{Z2} is the same or different and is each independently selected from the group consisting of halogen, C₁₋₆ alkyl, and C₁₋₆ haloalkyl.

In some embodiments of the present invention, in the compound represented by general formula (IV) or (IV') or the pharmaceutically acceptable salt thereof, X¹ is CH; ring B is selected from the group consisting of 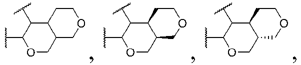 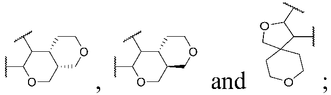 each R^{B} is the same or different and is each independently selected from the group consisting of halogen, C₁₋₆ alkyl, and C₁₋₆ haloalkyl; m is 0, 1, or 2; R⁴ is a hydrogen atom, R⁵ is a hydrogen atom, and R⁶ is a hydrogen atom; R^{A} is 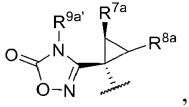 and R^{7a} and R^{8a} are different and each independently a hydrogen atom or R⁷⁸; R^{9a}' is a hydrogen atom or C₁₋₆ alkyl, and each R⁷⁸ is the same or different and is each independently selected from the group consisting of halogen, C₁₋₆ alkyl, and C₁₋₆ haloalkyl; 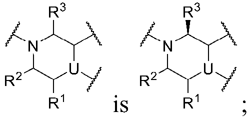 R¹ is a hydrogen atom, R² is a hydrogen atom, and R³ is C₁₋₆ alkyl; Q¹ is 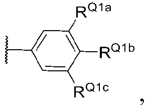 and R^{Q1a}, R^{Q1b}, and R^{Q1c} are the same or different and are each independently selected from the group consisting of a hydrogen atom, halogen, C₁₋₆ alkyl, and C₁₋₆ haloalkyl; Z¹ is 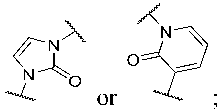 and Z² is 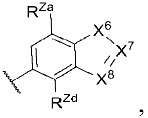 X⁶ is NR^{cc}, and R^{cc} is a hydrogen atom or methyl; X⁷ is CR^{Zh} or N; X⁸ is CR^{Ze} or N; R^{Za}, R^{Zd}, R^{Ze}, and R^{Zh} are the same or different and are each independently a hydrogen atom or R^{Z2}; and each R^{Z2} is the same or different and is each independently selected from the group consisting of halogen, C₁₋₆ alkyl, and C₁₋₆ haloalkyl.

In some embodiments of the present invention, in the compound represented by general formula (IN) or the pharmaceutically acceptable salt thereof, 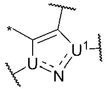 is 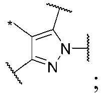 Q¹ is 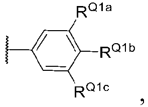 and R^{Q1a}, R^{Q1b}, and R^{Q1c} are the same or different and are each independently selected from the group consisting of a hydrogen atom, halogen, C₁₋₆ alkyl, and C₁₋₆ haloalkyl; -̅-̅-̅ is a single bond or a double bond; R^{Z2a} is a hydrogen atom; X is selected from the group consisting of CH₂O, OCH₂, CH₂, O, and N(methyl); X⁵ is O or CH₂; ring D is 5- to 10-membered heteroaryl; R^{Za} and R^{Zd} are the same or different and are each independently selected from the group consisting of a hydrogen atom, halogen, C₁₋₆ alkyl, and C₁₋₆ haloalkyl; each R^{Z2} is the same or different and is each independently selected from the group consisting of halogen, C₁₋₆ alkyl, C₁₋₆ haloalkyl, C₁₋₆ deuterioalkyl, and 3- to 6-membered cycloalkyl, and c is 0, 1, or 2; 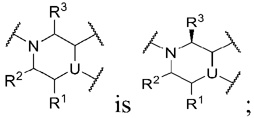 R¹ is a hydrogen atom, R² is a hydrogen atom, and R³ is C₁₋₆ alkyl; Y is C(O); ring A is 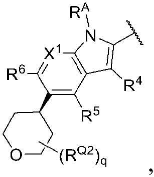 and X¹ is CH; each R^{Q2} is the same or different and is each independently selected from the group consisting of halogen, C₁₋₆ alkyl, and C₁₋₆ haloalkyl; q is 2; R^{A} is 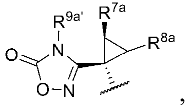 and R^{7a} and R^{8a} are different and each independently a hydrogen atom or R⁷⁸; R^{9a}' is a hydrogen atom or C₁₋₆ alkyl, and each R⁷⁸ is the same or different and is each independently selected from the group consisting of halogen, C₁₋₆ alkyl, and C₁₋₆ haloalkyl; and R⁴ is a hydrogen atom, R⁵ is a hydrogen atom, and R⁶ is a hydrogen atom.

In some embodiments of the present invention, in the compound represented by general formula (IM) or the pharmaceutically acceptable salt thereof, 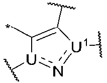 is 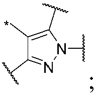 Q¹ is 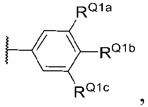 and R^{Q1a}, R^{Q1b}, and R^{Q1c} are the same or different and are each independently selected from the group consisting of a hydrogen atom, halogen, C₁₋₆ alkyl, and C₁₋₆ haloalkyl; X² is CH or N; and X³ is CR^{Z1a} or N, and R^{Z1a} is selected from the group consisting of a hydrogen atom, halogen, C₁₋₆ alkyl, and C₁₋₆ haloalkyl; X⁴ is CH or N; and Z² is a hydrogen atom or 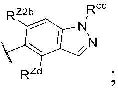 R^{Z2b} is a hydrogen atom, R^{Zd} is selected from the group consisting of a hydrogen atom, halogen, and C₁₋₆ alkyl, and R^{cc} is selected from the group consisting of a hydrogen atom, C₁₋₆ alkyl, 3- to 6-membered cycloalkyl, and C₁₋₆ deuterioalkyl; and 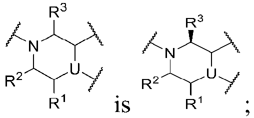 R¹ is a hydrogen atom, R² is a hydrogen atom, and R³ is C₁₋₆ alkyl; Y is C(O); ring A is 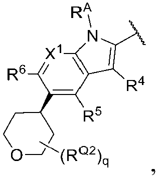 and X¹ is CH; each R^{Q2} is the same or different and is each independently selected from the group consisting of halogen, C₁₋₆ alkyl, and C₁₋₆ haloalkyl; q is 2; R^{A} is 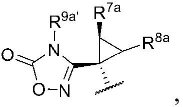 and R^{7a} and R^{8a} are different and each independently a hydrogen atom or R⁷⁸; R^{9a}' is a hydrogen atom or C₁₋₆ alkyl, and each R⁷⁸ is the same or different and is each independently selected from the group consisting of halogen, C₁₋₆ alkyl, and C₁₋₆ haloalkyl; and R⁴ is a hydrogen atom, R⁵ is a hydrogen atom, and R⁶ is a hydrogen atom.

In some embodiments of the present invention, in the compound represented by general formula (IP-2) or the pharmaceutically acceptable salt thereof, 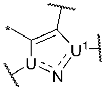 is 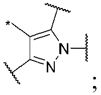 Q¹ is 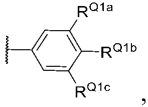 and R^{Q1a}, R^{Q1b}, and R^{Q1c} are the same or different and are each independently selected from the group consisting of a hydrogen atom, halogen, C₁₋₆ alkyl, and C₁₋₆ haloalkyl; -̅-̅-̅ is a single bond or a double bond; a is 0; X is selected from the group consisting of CH₂O, OCH₂, CH₂, O, and N(methyl); X⁵ is O or CH₂; ring D is 5- to 10-membered heteroaryl; R^{Za} and R^{Zd} are the same or different and are each independently selected from the group consisting of a hydrogen atom, halogen, C₁₋₆ alkyl, and C₁₋₆ haloalkyl; each R^{Z2} is the same or different and is each independently selected from the group consisting of halogen, C₁₋₆ alkyl, C₁₋₆ haloalkyl, C₁₋₆ deuterioalkyl, and 3- to 6-membered cycloalkyl, and c is 0, 1, or 2; 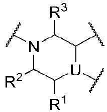 is 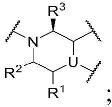 R¹ is a hydrogen atom, R² is a hydrogen atom, and R³ is C₁₋₆ alkyl; Y is C(O); ring A is 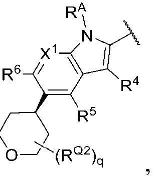 and X¹ is CH; each R^{Q2} is the same or different and is each independently selected from the group consisting of halogen, C₁₋₆ alkyl, and C₁₋₆ haloalkyl; q is 2; R^{A} is 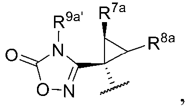 and R^{7a} and R^{8a} are different and each independently a hydrogen atom or R⁷⁸; R^{9a}' is a hydrogen atom or C₁₋₆ alkyl, and each R⁷⁸ is the same or different and is each independently selected from the group consisting of halogen, C₁₋₆ alkyl, and C₁₋₆ haloalkyl; and R⁴ is a hydrogen atom, R⁵ is a hydrogen atom, and R⁶ is a hydrogen atom.

In some embodiments of the present invention, in the compound represented by general formula (IM) or the pharmaceutically acceptable salt thereof, 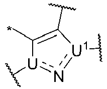 is 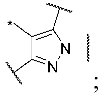 Q¹ is 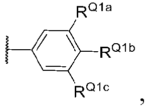 and R^{Q1a}, R^{Q1b}, and R^{Q1c} are the same or different and are each independently selected from the group consisting of a hydrogen atom, halogen, C₁₋₆ alkyl, C₁₋₆ haloalkyl, and 3- to 6-membered cycloalkyl; X², X³, and X⁴ are the same or different and are each independently CR^{Z1a} or N; R^{Z1a} is selected from the group consisting of a hydrogen atom, halogen, C₁₋₆ alkyl, and 3- to 6-membered cycloalkyl; Z² is 5- to 10-membered heteroaryl optionally substituted with one or more R^{Z2}, and each R^{Z2} is the same or different and is each independently selected from the group consisting of halogen, cyano, C₁₋₆ alkyl, C₁₋₆ haloalkyl, C₁₋₆ deuterioalkyl, C₁₋₆ alkoxy, C₁₋₆ haloalkoxy, 3- to 6-membered cycloalkyl, and C₁₋₆ alkoxy C₁₋₆ alkyl; 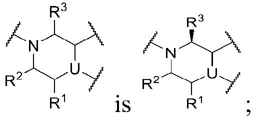 R¹ is a hydrogen atom, R² is a hydrogen atom, and R³ is C₁₋₆ alkyl; Y is C(O); ring A is 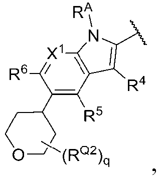 X¹ is CR^{x1} , and R^{x1} is selected from the group consisting of a hydrogen atom, halogen, C₁₋₆ alkyl, C₁₋₆ haloalkyl, and 3- to 6-membered cycloalkyl; each R^{Q2} is the same or different and is each independently selected from the group consisting of halogen, C₁₋₆ alkyl, and C₁₋₆ haloalkyl; q is 0, 1, or 2; R⁴ is a hydrogen atom, R⁵ is a hydrogen atom, and R⁶ is a hydrogen atom; R^{A} is 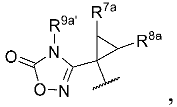 and R^{7a} and R^{8a} are different and each independently a hydrogen atom or R⁷⁸; R^{9a}' is a hydrogen atom or C₁₋₆ alkyl, and each R⁷⁸ is the same or different and is each independently selected from the group consisting of halogen, C₁₋₆ alkyl, and C₁₋₆ haloalkyl.

In some embodiments of the present invention, in the compound represented by general formula (IO) or the pharmaceutically acceptable salt thereof, 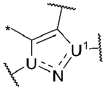 is 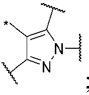 Q¹ is 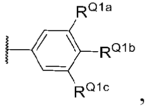 and R^{Q1a}, R^{Q1b}, and R^{Q1c} are the same or different and are each independently selected from the group consisting of a hydrogen atom, halogen, C₁₋₆ alkyl, C₁₋₆ haloalkyl, and 3- to 6-membered cycloalkyl; -̅ -̅ -̅ is a single bond or a double bond, and R^{Z2a} is selected from the group consisting of a hydrogen atom, halogen, C₁₋₆ alkyl, C₁₋₆ alkoxy, C₁₋₆ haloalkyl, and C₁₋₆ haloalkoxy; X is selected from the group consisting of CH₂O, OCH₂, CH₂, O, and N(methyl); X⁵ is O or CH₂; R^{Zf} and R^{Zg}, together with the carbon atoms to which they are attached, form ring E, ring E is optionally substituted with 1, 2, 3, or 4 R^{Z2}, ring E is 9- or 10-membered bicyclic heteroaryl, and each R^{Z2} is the same or different and is each independently selected from the group consisting of halogen, cyano, C₁₋₆ alkyl, C₁₋₆ haloalkyl, C₁₋₆ deuterioalkyl, C₁₋₆ alkoxy, C₁₋₆ haloalkoxy, 3- to 6-membered cycloalkyl, and C₁₋₆ alkoxy C₁₋₆ alkyl; R¹ is selected from the group consisting of a hydrogen atom, halogen, C₁₋₆ alkyl, and 3- to 6-membered cycloalkyl; R² is selected from the group consisting of a hydrogen atom, halogen, C₁₋₆ alkyl, and 3- to 6-membered cycloalkyl, and R³ is selected from the group consisting of a hydrogen atom, halogen, C₁₋₆ alkyl, and 3- to 6-membered cycloalkyl; Y is C(O); ring A is 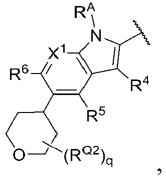 X¹ is CR^{x1}, and R^{x1} is selected from the group consisting of a hydrogen atom, halogen, C₁₋₆ alkyl, C₁₋₆ haloalkyl, and 3- to 6-membered cycloalkyl; each R^{Q2} is the same or different and is each independently selected from the group consisting of halogen, C₁₋₆ alkyl, C₁₋₆ haloalkyl, and 3- to 6-membered cycloalkyl; q is 0, 1, or 2; R⁴ is selected from the group consisting of a hydrogen atom, halogen, C₁₋₆ alkyl, and C₁₋₆ haloalkyl; R⁵ is selected from the group consisting of a hydrogen atom, halogen, C₁₋₆ alkyl, and C₁₋₆ haloalkyl; R⁶ is selected from the group consisting of a hydrogen atom, halogen, C₁₋₆ alkyl, and C₁₋₆ haloalkyl; R^{A} is 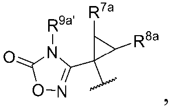 and R^{7a} and R^{8a} are different and each independently a hydrogen atom or R⁷⁸; R^{9a}' is a hydrogen atom or C₁₋₆ alkyl, and each R⁷⁸ is the same or different and is each independently selected from the group consisting of halogen, C₁₋₆ alkyl, and C₁₋₆ haloalkyl.

In some embodiments of the present invention, in the compound represented by general formula (IO) or the pharmaceutically acceptable salt thereof, 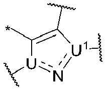 is 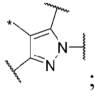 Q¹ is 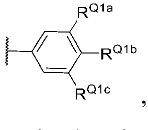 and R^{Q1a}, R^{Q1b}, and R^{Q1c} are the same or different and are each independently selected from the group consisting of a hydrogen atom, halogen, C₁₋₆ alkyl, C₁₋₆ haloalkyl, and 3- to 6-membered cycloalkyl; -̅ -̅ -̅ is a single bond or a double bond, and R^{Z2a} is selected from the group consisting of a hydrogen atom, halogen, C₁₋₆ alkyl, C₁₋₆ alkoxy, C₁₋₆ haloalkyl, and C₁₋₆ haloalkoxy; X is selected from the group consisting of CH₂, CH₂CH₂, CH₂O, OCH₂, O, NH, N(methyl), CH(methyl), CH cyclopropyl, CF₂, and 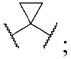 X³ is O or CH₂; R^{Zf} and R^{Zg}, together with the carbon atoms to which they are attached, form indazolyl optionally substituted with 1, 2, 3, or 4 R^{Z2}, each R^{Z2} is the same or different and is each independently selected from the group consisting of halogen, cyano, C₁₋₆ alkyl, C₁₋₆ haloalkyl, C₁₋₆ deuterioalkyl, C₁₋₆ alkoxy, C₁₋₆ haloalkoxy, C₁₋₆ alkoxy C₁₋₆ alkyl, 3- to 6-membered cycloalkyl, NR^{11a}R^{11b}, and P(O)R¹²R¹³, and R^{11a}, R^{11b}, R¹², and R¹³ are the same or different and are each independently a hydrogen atom or C₁₋₆ alkyl; R¹ is selected from the group consisting of a hydrogen atom, halogen, C₁₋₆ alkyl, and 3- to 6-membered cycloalkyl; R² is selected from the group consisting of a hydrogen atom, halogen, C₁₋₆ alkyl, and 3- to 6-membered cycloalkyl, and R³ is selected from the group consisting of a hydrogen atom, halogen, C₁₋₆ alkyl, and 3-to 6-membered cycloalkyl; Y is C(O); ring A is 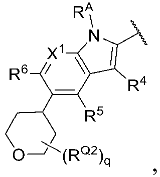 X¹ is CR^{x1}, and R^{x1} is selected from the group consisting of a hydrogen atom, halogen, C₁₋₆ alkyl, C₁₋₆ haloalkyl, and 3- to 6-membered cycloalkyl; each R^{Q2} is the same or different and is each independently selected from the group consisting of halogen, C₁₋₆ alkyl, C₁₋₆ haloalkyl, and 3- to 6-membered cycloalkyl; q is 0, 1, or 2; R⁴ is selected from the group consisting of a hydrogen atom, halogen, C₁₋₆ alkyl, and C₁₋₆ haloalkyl; R⁵ is selected from the group consisting of a hydrogen atom, halogen, C₁₋₆ alkyl, and C₁₋₆ haloalkyl; R⁶ is selected from the group consisting of a hydrogen atom, halogen, C₁₋₆ alkyl, and C₁₋₆ haloalkyl; R^{A} is 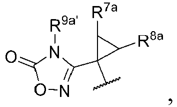 and R^{7a} and R^{8a} are different and each independently a hydrogen atom or R⁷⁸; R^{9a}' is a hydrogen atom or C₁₋₆ alkyl, and each R⁷⁸ is the same or different and is each independently selected from the group consisting of halogen, C₁₋₆ alkyl, and C₁₋₆ haloalkyl.

In some embodiments of the present invention, in the compound represented by general formula (II) or the pharmaceutically acceptable salt thereof, Q¹ is 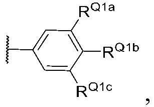 and R^{Q1a}, R^{Q1b}, and R^{Q1c} are the same or different and are each independently selected from the group consisting of a hydrogen atom, halogen, C₁₋₆ alkyl, C₁₋₆ haloalkyl, and 3- to 6-membered cycloalkyl; R¹ is a hydrogen atom, R² is a hydrogen atom, and R³ is C₁₋₆ alkyl; R⁴ is selected from the group consisting of a hydrogen atom, halogen, C₁₋₆ alkyl, and C₁₋₆ haloalkyl; R⁵ is selected from the group consisting of a hydrogen atom, halogen, C₁₋₆ alkyl, and C₁₋₆ haloalkyl; R⁶ is selected from the group consisting of a hydrogen atom, halogen, C₁₋₆ alkyl, and C₁₋₆ haloalkyl; X¹ is CR^{x1}, and R^{x1} is selected from the group consisting of a hydrogen atom, halogen, C₁₋₆ alkyl, C₁₋₆ haloalkyl, and 3- to 6-membered cycloalkyl; each R^{Q2} is the same or different and is each independently selected from the group consisting of halogen, C₁₋₆ alkyl, C₁₋₆ haloalkyl, and 3- to 6-membered cycloalkyl; q is 0, 1, or 2; R^{7a} is a hydrogen atom or C₁₋₆ alkyl; R^{8a} is a hydrogen atom; R^{9a}' is a hydrogen atom; Z¹ is selected from the group consisting of 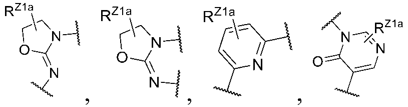 and 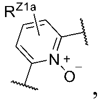 and R^{Z1a} is selected from the group consisting of a hydrogen atom, halogen, C₁₋₆ alkyl, C₁₋₆ haloalkyl, and 3- to 6-membered cycloalkyl; Z² is indazolyl optionally substituted with one or more R^{Z2}; and each R^{Z2} is the same or different and is each independently selected from the group consisting of halogen, cyano, C₁₋₆ alkyl, C₁₋₆ haloalkyl, C₁₋₆ deuterioalkyl, C₁₋₆ alkoxy, C₁₋₆ haloalkoxy, 3- to 6-membered cycloalkyl, and C₁₋₆ alkoxy C₁₋₆ alkyl.

In some embodiments of the present invention, in the compound represented by general formula (IO) or the pharmaceutically acceptable salt thereof, 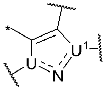 is 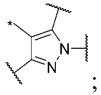 Q¹ is 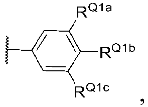 and R^{Q1a}, R^{Q1b}, and R^{Q1c} are the same or different and are each independently selected from the group consisting of a hydrogen atom, halogen, C₁₋₆ alkyl, C₁₋₆ haloalkyl, and 3- to 6-membered cycloalkyl; -̅ -̅ -̅ is a single bond, R^{Z2a} is selected from the group consisting of a hydrogen atom, halogen, C₁₋₆ alkyl, C₁₋₆ alkoxy, C₁₋₆ haloalkyl, and C₁₋₆ haloalkoxy; X is selected from the group consisting of CH₂O, OCH₂, CH₂, O, and N(methyl); X⁵ is CH₂; R^{Zf} and R^{Zg}, together with the carbon atoms to which they are attached, form ring E, ring E is optionally substituted with 1, 2, 3, or 4 R^{Z2}, ring E is 6- to 10-membered aryl or 5- to 14-membered heteroaryl, and each R^{Z2} is the same or different and is each independently selected from the group consisting of halogen, cyano, C₁₋₆ alkyl, C₁₋₆ alkoxy, C₁₋₆ alkoxy C₁₋₆ alkyl, 3- to 6-membered cycloalkyl, 3- to 6-membered heterocyclyl, NR^{11a}R^{11b}, and P(O)R¹²R¹³, wherein the C₁₋₆ alkyl, C₁₋₆ alkoxy, C₁₋₆ alkoxy C₁₋₆ alkyl, 3- to 6-membered cycloalkyl, and 3- to 6-membered heterocyclyl are each independently and optionally substituted with one or more selected from the group consisting of a deuterium atom, oxo, halogen, cyano, C₁₋₆ alkyl, C₁₋₆ alkoxy, 3- to 6-membered cycloalkyl, and 3- to 6-membered heterocyclyl, and R^{11a} and R^{11b} are the same or different and are each independently a hydrogen atom or C₁₋₆ alkyl; R¹² and R¹³ are the same or different and are each independently a hydrogen atom or C₁₋₆ alkyl; 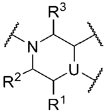 is 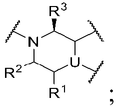 R¹ is a hydrogen atom; R² is a hydrogen atom, and R³ is C₁₋₆ alkyl; Y is C(O); ring A is 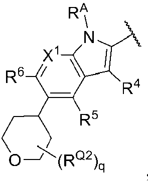 X¹ is CR^{x1}, and R^{x1} is selected from the group consisting of a hydrogen atom, halogen, C₁₋₆ alkyl, C₁₋₆ haloalkyl, and 3- to 6-membered cycloalkyl; 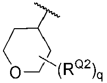 is 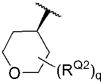 or 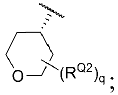 each R^{Q2} is the same or different and is each independently selected from the group consisting of halogen, C₁₋₆ alkyl, C₁₋₆ haloalkyl, and 3- to 6-membered cycloalkyl; q is 0, 1, or 2; R⁴ is selected from the group consisting of a hydrogen atom, halogen, C₁₋₆ alkyl, and C₁₋₆ haloalkyl; R⁵ is selected from the group consisting of a hydrogen atom, halogen, C₁₋₆ alkyl, and C₁₋₆ haloalkyl; R⁶ is selected from the group consisting of a hydrogen atom, halogen, C₁₋₆ alkyl, and C₁₋₆ haloalkyl; R^{A} is 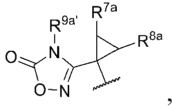 and R^{7a} and R^{8a} are different and each independently a hydrogen atom or R⁷⁸; R^{9a}' is a hydrogen atom or C₁₋₆ alkyl, and each R⁷⁸ is the same or different and is each independently selected from the group consisting of halogen, C₁₋₆ alkyl, and C₁₋₆ haloalkyl.

In some embodiments of the present invention, in the compound represented by general formula (IO) or the pharmaceutically acceptable salt thereof, 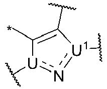 is 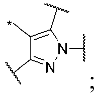 Q¹ is 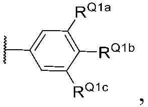 and R^{Q1a}, R^{Q1b}, and R^{Q1c} are the same or different and are each independently selected from the group consisting of a hydrogen atom, halogen, C₁₋₆ alkyl, C₁₋₆ haloalkyl, and 3- to 6-membered cycloalkyl; -̅ -̅ -̅ is a single bond, R^{Z2a} is selected from the group consisting of a hydrogen atom, halogen, C₁₋₆ alkyl, C₁₋₆ alkoxy, C₁₋₆ haloalkyl, and C₁₋₆ haloalkoxy; X is selected from the group consisting of CH₂O, OCH₂, CH₂, O, and N(methyl); X⁵ is CH₂; R^{Zf} and R^{Zg}, together with the carbon atoms to which they are attached, form ring E, ring E is optionally substituted with 1, 2, 3, or 4 R^{Z2}, ring E is phenyl, and each R^{Z2} is the same or different and is each independently selected from the group consisting of halogen, cyano, C₁₋₆ alkyl, C₁₋₆ haloalkyl, C₁₋₆ deuterioalkyl, C₁₋₆ alkoxy, C₁₋₆ haloalkoxy, C₁₋₆ alkoxy C₁₋₆ alkyl, 3- to 6-membered cycloalkyl, NR^{11a}R^{11b}, and P(O)R¹²R¹³, and R^{11a}, R^{11b}, R¹², and R¹³ are the same or different and are each independently a hydrogen atom or C₁₋₆ alkyl; 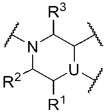 is 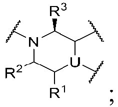 R¹ is a hydrogen atom; R² is a hydrogen atom, and R³ is C₁₋₆ alkyl; Y is C(O); ring A is 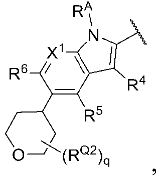 X¹ is CR^{x1}, and R^{x1} is selected from the group consisting of a hydrogen atom, halogen, C₁₋₆ alkyl, C₁₋₆ haloalkyl, and 3- to 6-membered cycloalkyl; 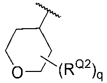 is 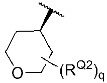 or 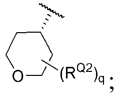 each R^{Q2} is the same or different and is each independently selected from the group consisting of halogen, C₁₋₆ alkyl, C₁₋₆ haloalkyl, and 3- to 6-membered cycloalkyl; q is 0, 1, or 2; R⁴ is selected from the group consisting of a hydrogen atom, halogen, C₁₋₆ alkyl, and C₁₋₆ haloalkyl; R⁵ is selected from the group consisting of a hydrogen atom, halogen, C₁₋₆ alkyl, and C₁₋₆ haloalkyl; R⁶ is selected from the group consisting of a hydrogen atom, halogen, C₁₋₆ alkyl, and C₁₋₆ haloalkyl; R^{A} is 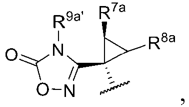 and R^{7a} is C₁₋₆ alkyl; R^{8a} is a hydrogen atom or C₁₋₆ alkyl; and R^{9a}' is a hydrogen atom or C₁₋₆ alkyl.

In some embodiments of the present invention, in the compound represented by general formula (IV) or (IV') or the pharmaceutically acceptable salt thereof, X¹ is CH; ring B is 3- to 12-membered heterocyclyl; each R^{B} is the same or different and is each independently selected from the group consisting of halogen, C₁₋₆ alkyl, and C₁₋₆ haloalkyl; m is 0, 1, or 2; R⁴ is a hydrogen atom, R⁵ is a hydrogen atom, and R⁶ is a hydrogen atom; R^{A} is 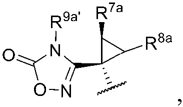 and R^{7a} and R^{8a} are different and each independently a hydrogen atom or R⁷⁸; R^{9a}' is a hydrogen atom or C₁₋₆ alkyl, and each R⁷⁸ is the same or different and is each independently selected from the group consisting of halogen, C₁₋₆ alkyl, and C₁₋₆ haloalkyl; 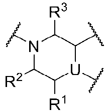 is 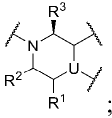 R¹ is a hydrogen atom, R² is a hydrogen atom, and R³ is C₁₋₆ alkyl; Q¹ is 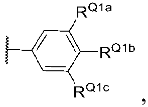 and R^{Q1a}, R^{Q1b}, and R^{Q1c} are the same or different and are each independently selected from the group consisting of a hydrogen atom, halogen, C₁₋₆ alkyl, C₁₋₆ haloalkyl, and 3- to 6-membered cycloalkyl; 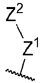 is selected from the group consisting of the groups from group A, group B, and group C (in some embodiments, the groups from group C).

In some embodiments of the present invention, in the compound represented by general formula (II) or the pharmaceutically acceptable salt thereof, Q¹ is 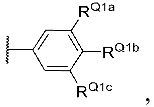 and R^{Q1a}, R^{Q1b}, and R^{Q1c} are the same or different and are each independently selected from the group consisting of a hydrogen atom, halogen, C₁₋₆ alkyl, C₁₋₆ haloalkyl, and 3- to 6-membered cycloalkyl; 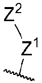 is selected from the group consisting of the groups from group C; R¹ is a hydrogen atom; R² is a hydrogen atom, and R³ is C₁₋₆ alkyl; X¹ is CR^{x1}, and R^{x1} is selected from the group consisting of a hydrogen atom, halogen, C₁₋₆ alkyl, C₁₋₆ haloalkyl, and 3- to 6-membered cycloalkyl; 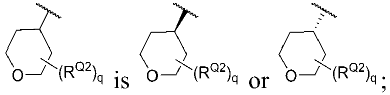 each R^{Q2} is the same or different and is each independently selected from the group consisting of halogen, C₁₋₆ alkyl, C₁₋₆ haloalkyl, and 3- to 6-membered cycloalkyl; q is 0, 1, or 2; R⁴ is selected from the group consisting of a hydrogen atom, halogen, C₁₋₆ alkyl, and C₁₋₆ haloalkyl; R⁵ is selected from the group consisting of a hydrogen atom, halogen, C₁₋₆ alkyl, and C₁₋₆ haloalkyl; R⁶ is selected from the group consisting of a hydrogen atom, halogen, C₁₋₆ alkyl, and C₁₋₆ haloalkyl; R^{7a} is C₁₋₆ alkyl; R^{8a} is a hydrogen atom or C₁₋₆ alkyl; and R^{9a}' is a hydrogen atom or C₁₋₆ alkyl.

In some embodiments of the present invention, in the compound represented by general formula (I) or the pharmaceutically acceptable salt thereof, Q¹ is 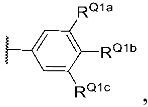 and R^{Q1a}, R^{Q1b}, and R^{Q1c} are the same or different and are each independently selected from the group consisting of a hydrogen atom, halogen, C₁₋₆ alkyl, C₁₋₆ haloalkyl, and 3- to 6-membered cycloalkyl; 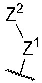 is selected from the group consisting of 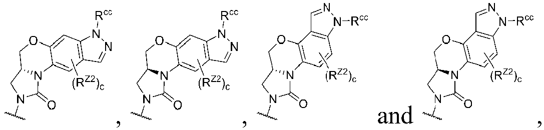 c is 0, 1, or 2, and R^{cc} is selected from the group consisting of a hydrogen atom, C₁₋₆ alkyl, and 3- to 6-membered cycloalkyl, wherein the C₁₋₆ alkyl and 3- to 6-membered cycloalkyl are each independently and optionally substituted with one or more selected from the group consisting of a deuterium atom, halogen, C₁₋₆ alkyl, C₁₋₆ alkoxy, hydroxyl, and 3- to 6-membered cycloalkyl; each R^{Z2} is the same or different and is each independently selected from the group consisting of halogen, cyano, C₁₋₆ alkyl, C₁₋₆ haloalkyl, C₁₋₆ alkoxy, C₁₋₆ haloalkoxy, and 3- to 6-membered cycloalkyl; 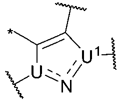 is 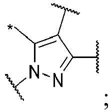 R¹ is a hydrogen atom; R² is a hydrogen atom, and R³ is C₁₋₆ alkyl; Y is C(O); ring A is 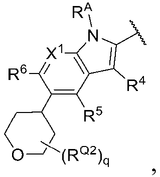 and X¹ is CH; R⁴, R⁵, and R⁶ is a hydrogen atom; 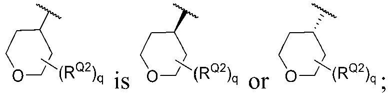 each R^{Q2} is the same or different and is each independently selected from the group consisting of halogen, C₁₋₆ alkyl, C₁₋₆ haloalkyl, and 3- to 6-membered cycloalkyl; q is 0, 1, or 2; R^{A} is 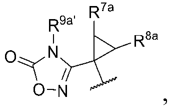 and R^{7a} and R^{8a} are different and each independently a hydrogen atom or R⁷⁸; and R^{9a}' is a hydrogen atom or C₁₋₆ alkyl, and each R⁷⁸ is the same or different and is each independently selected from the group consisting of halogen, C₁₋₆ alkyl, and C₁₋₆ haloalkyl.

In some embodiments of the present invention, in the compound represented by general formula (IN) or the pharmaceutically acceptable salt thereof, 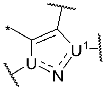 is 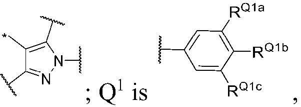 and R^{Q1a}, R^{Q1b}, and R^{Q1c} are the same or different and are each independently selected from the group consisting of a hydrogen atom, halogen, C₁₋₆ alkyl, and 3- to 6-membered cycloalkyl; -̅ -̅ -̅ is a single bond; R^{Z2a} is a hydrogen atom; X is O; X⁵ is CH₂; ring D is 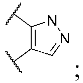 R^{Za} and R^{Zd} are the same or different and are each independently a hydrogen atom or halogen; each R^{Z2} is the same or different and is each independently selected from the group consisting of halogen, C₁₋₆ alkyl, C₁₋₆ alkoxy, C₁₋₆ alkoxy C₁₋₆ alkyl, and 3- to 6-membered cycloalkyl, wherein the C₁₋₆ alkyl, C₁₋₆ alkoxy, and 3- to 6-membered cycloalkyl are each independently and optionally substituted with one or more selected from the group consisting of a deuterium atom, halogen, and 3- to 6-membered cycloalkyl; c is 0 or 1; 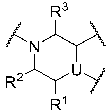 is 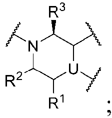 R¹ is a hydrogen atom, R² is a hydrogen atom, and R³ is C₁₋₆ alkyl; Y is C(O); ring A is 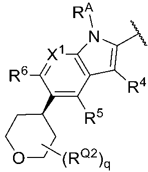 , and X¹ is CH; each R^{Q2} is the same or different and is each independently C₁₋₆ alkyl; q is 2; R^{A} is 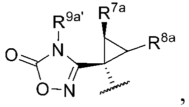 and R^{7a} is C₁₋₆ alkyl; R^{8a} is a hydrogen atom; R^{9a'} is a hydrogen atom; and R⁴ is a hydrogen atom, R⁵ is a hydrogen atom, and R⁶ is a hydrogen atom.

**Table A. Typical compounds of the present invention, including but not limited to:**

| Compound No. | Compound structure | Name |
|---|---|---|
| 1 | 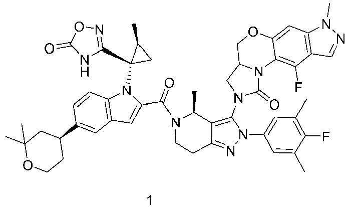 | 2-((*S*)-5-(5-((*S*)-2,2-dimethyltetrahydro-2*H-*pyran-4-yl)-1-((1*S*,2*S*)-2-methyl-1-(5-oxo-4,5-dihydro-1,2,4-oxadiazol-3-yl)cyclopropyl)-1*H*-indole-2-carbonyl)-2-(4-fluoro-3,5-dimethylphenyl)-4-methyl-4,5,6,7-tetrahydro-2*H*-pyrazolo[4,3-*c*]pyridin-3-yl)-10-fluoro-7-methyl-2,3,3*a*,4-tetrahydro-1*H*,7*H-*imidazo[1',5':4,5][1,4]oxazino[3,2-*f*]indazol-1-one **1 (mixture of diastereomers)** |
| 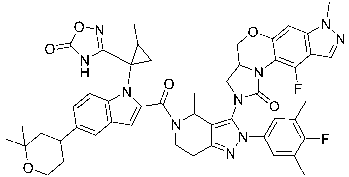 | | |
| **1-1** | 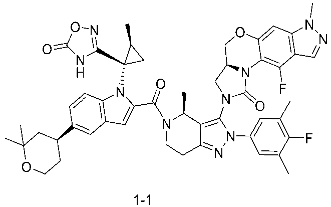 | (S)-2-((S)-5-(5-((S)-2,2-dimethyltetrahydro-2H-pyran-4-yl)-1-((1S,2S)-2-methyl-1-(5-oxo-4,5-dihydro-1,2,4-oxadiazol-3-yl)cyclopropyl)-1H-indole-2-carbonyl)-2-(4-fluoro-3,5-dimethylphenyl)-4-methyl-4,5,6,7-tetrahydro-2H-pyrazolo[4,3-c]pyridin-3-yl)-10-fluoro-7-methyl-2,3,3a,4-tetrahydro-1H,7H-imidazo[1',5':4,5][1,4]oxazino[3,2-f]indazol-1-one **1-1** |
| **1-2** | 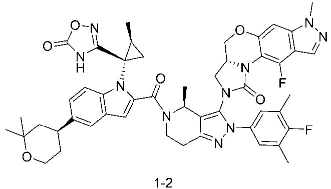 | (R)-2-((S)-5-(5-((S)-2,2-dimethyltetrahydro-2H-pyran-4-yl)-1-((1S,2S)-2-methyl-1-(5-oxo-4,5-dihydro-1,2,4-oxadiazol-3-yl)cyclopropyl)-1H-indole-2-carbonyl)-2-(4-fluoro-3,5-dimethylphenyl)-4-methyl-4,5,6,7-tetrahydro-2H-pyrazolo[4,3-c]pyridin-3 -yl)-10-fluoro-7-methyl-2,3,3a,4-tetrahydro-1H,7H-imidazo[1',5':4,5][1,4]oxazino[3,2-f]indazol-1-one **1-2** |
| **2** | 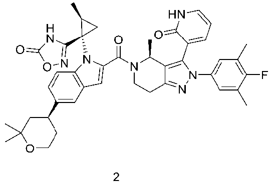 | 3-((1S,2S)-1-(5-((S)-2,2-dimethyltetrahydro-2H-pyran-4-yl)-2-((S)-2-(4-fluoro-3,5-dimethylphenyl)-4-methyl-3-(2-oxo-1,2-dihydropyridin-3-yl)-4,5,6,7-tetrahydro-2H-pyrazolo[4,3-c]pyridine-5-carbonyl)-1H-indol-1-yl)-2-methylcyclopropyl)-1,2,4-oxadiazol-5(4H)-one **2** |
| 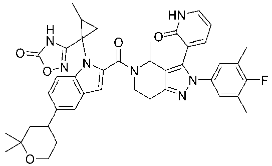 | | |
| **3** | 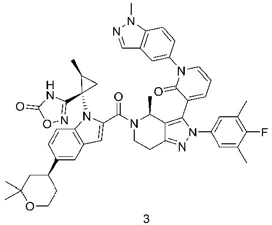 | 3-((1S,2S)-1-(5-((S)-2,2-dimethyltetrahydro-2H-pyran-4-yl)-2-((S)-2-(4-fluoro-3,5-dimethylphenyl)-4-methyl-3-(1-(1-methyl-1H-indazol-5-yl)-2-oxo-1,2-dihydropyridin-3-yl)-4,5,6,7-tetrahydro-2H-pyrazolo[4,3-c]pyridine-5-carbonyl)-1H-indol-1-yl)-2-methylcyclopropyl)-1,2,4-oxadiazol-5(4H)-one **3** |
| 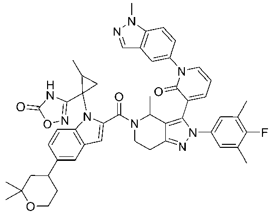 | | |
| **4** | 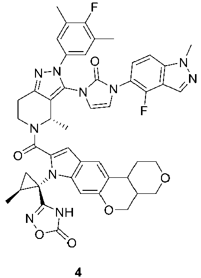 | 3-((1S,2S)-1-(9-((S)-3-(3-(4-fluoro-1-methyl-1H-indazol-5-yl)-2-oxo-2,3-dihydro-1H-imidazol-1-yl)-2-(4-fluoro-3,5-dimethylphenyl)-4-methyl-4,5,6,7-tetrahydro-2H-pyrazolo[4,3-c]pyridine-5-carbonyl)-1,4a,5,11b-tetrahydro-4H-pyrano[4',3':4,5]pyrano[3,2-f]indol-8(2H)-yl)-2-methylcyclopropyl)-1,2,4-oxadiazol-5(4H)-one **4** (a mixture of stereoisomers) |
| 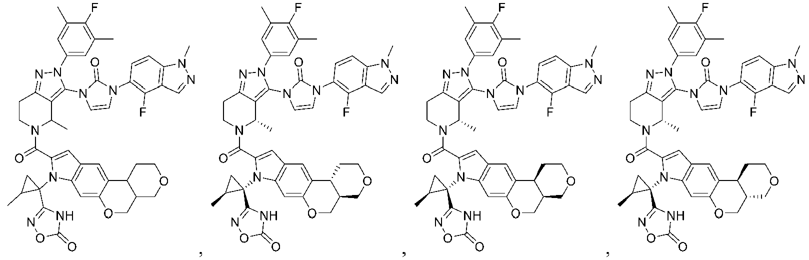 | | |
| and 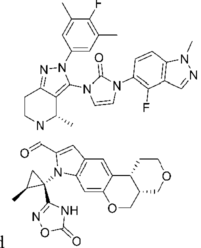 | | |
| **5** | 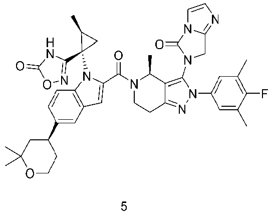 | 3-((1S,2S)-1-(5-((S)-2,2-dimethyltetrahydro-2H-pyran-4-yl)-2-((S)-2-(4-fluoro-3,5-dimethylphenyl)-4-methyl-3-(5-oxo-5H-imidazo[1,5-a]imidazol-6(7H)-yl)-4,5,6,7-tetrahydro-2H-pyrazolo[4,3-c]pyridine-5-carbonyl)-1H-indol-1-yl)-2-methylcyclopropyl)-1,2,4-oxadiazol-5(4H)-one **5** |
| 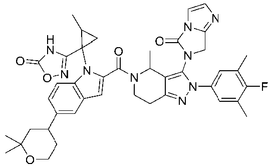 | | |
| 6 | 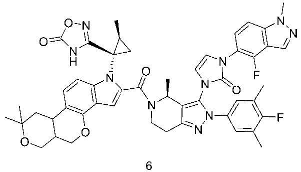 | 3-((1S,2S)-1-(2-((S)-3-(3-(4-fluoro-1-methyl-1H-indazol-5-yl)-2-oxo-2,3-dihydro-1H-imidazol-1-yl)-2-(4-fluoro-3,5-dimethylphenyl)-4-methyl-4,5,6,7-tetrahydro-2H-pyrazolo[4,3-c]pyridine-5-carbonyl)-8,8-dimethyl-5a,8,9,9a-tetrahydro-5H-pyrano[4',3':4,5]pyrano[2,3-e]indol-1(6H)-yl)-2-methylcyclopropyl)-1,2,4-oxadiazol-5(4H)-one **6** |
| 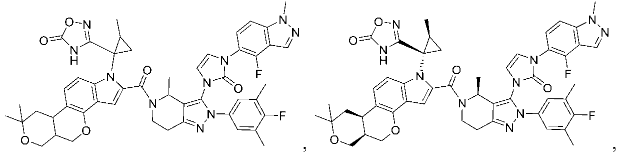 | | |
| 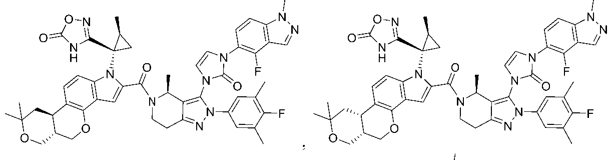 and | | |
| 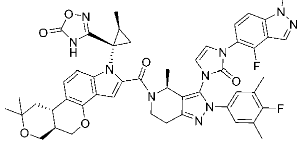 | | |
| 7 | 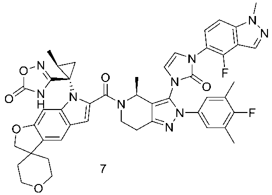 | 3-((1S,2S)-1-(6-((S)-3-(3-(4-fluoro-1-methyl-1H-imidazol-5-yl)-2-oxo-2,3-dihydro-1H-imidazol-1-yl)-2-(4-fluoro-3,5-dimethylphenyl)-4-methyl-4,5,6,7-tetrahydro-2H-pyrazolo[4,3-c]pyridine-5-carbonyl)-2',3',5',6'-tetrahydro-2H,7H-spiro [furo [3,2-f]indole-3,4'-pyran]-7-yl)-2-methylcyclopropyl)-1,2,4-oxadiazol-5(4H)-one 7 |
| 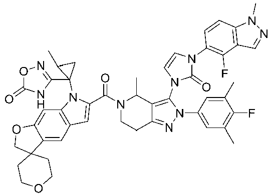 | | |
| **8-1** | 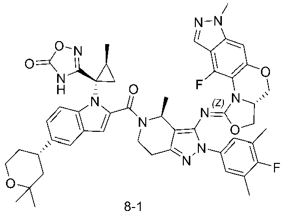 | 3-((1S,2S)-1-(5-((S)-2,2-dimethyltetrahydro-2H-pyran-4-yl)-2-((S)-2-(4-fluoro-3,5-dimethylphenyl)-3-((R,Z)-10-fluoro-7-methyl-3a,4-dihydro-1H,3H,7H-oxazolo[3',4':4,5][1,4]oxazino[3,2-f]indazol-1-ylidene)amino)-4-methyl-4,5,6,7-tetrahydro-2H-pyrazolo[4,3-c]pyridine-5-carbonyl)-1H-indol-1-yl)-2-methylcyclopropyl)-1,2,4-oxadiazol-5(4H)-one **8-1** |
| **8-2** | 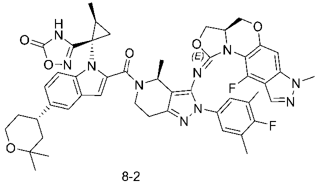 | 3-((1S,2S)-1-(5-((S)-2,2-dimethyltetrahydro-2H-pyran-4-yl)-2-((S)-2-(4-fluoro-3,5-dimethylphenyl)-3-((R,E)-10-fluoro-7-methyl-3a,4-dihydro-1H,3H,7H-oxazolo[3',4':4,5][1,4]oxazino[3,2-f]indazol-1-ylidene)amino)-4-methyl-4,5,6,7-tetrahydro-2H-pyrazolo[4,3-c]pyridine-5-carbonyl)-1H-indol-1-yl)-2-methylcyclopropyl)-1,2,4-oxadiazol-5(4H)-one **8-2** |
| 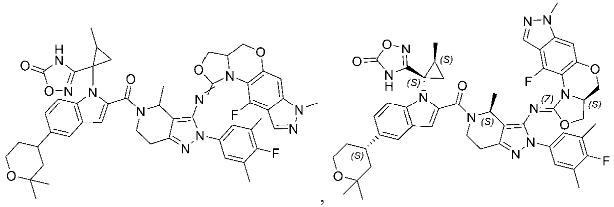 and | | |
| 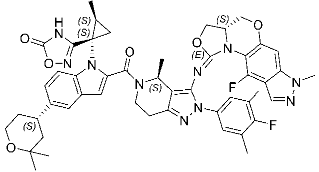 | | |
| **9** | 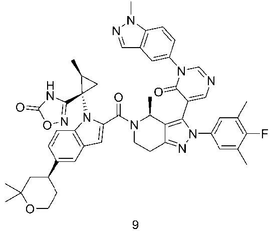 | 3-((1*S*,2*S*)-1-(5-((*S*)-2,2-dimethyltetrahydro-2*H*-pyran-4-yl)-2-((*S*)-2-(4-fluoro-3,5-dimethylphenyl)-4-methyl-3-(1-(1-methyl-1*H*-indazol-5-yl)-6-oxo-1,6-dihydropyrimidin-5-yl)-4,5,6,7-tetrahydro-2*H*-pyrazolo[4,3-*c*]pyridine-5-carbonyl)-1*H*-indol-1-yl)-2-methylcyclopropyl)-1,2,4-oxadiazol-5(4*H*)-one **9** |
| 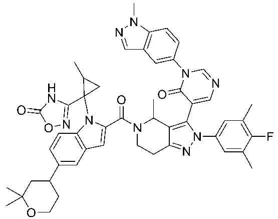 | | |
| **10** | 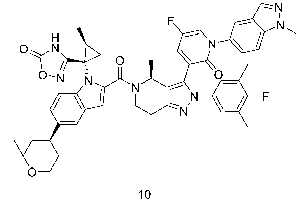 | 3-((1*S*,2*S*)-1-(5-((*S*)-2,2-dimethyltetrahydro-2*H*-pyran-4-yl)-2-((*S*)-3-(5-fluoro-1-(1-methyl-1*H*-indazol-5-yl)-2-oxo-1,2-dihydropyridin-3-yl)-2-(4-fluoro-3,5-dimethylphenyl)-4-methyl-4,5,6,7-tetrahydro-2*H*-pyrazolo[4,3-*c*]pyridine-5-carbonyl)-1*H*-indol-1-yl)-2-methylcyclopropyl)-1,2,4-oxadiazol-5(4*H*)-one **10** |
| 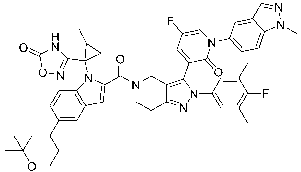 | | |
| **11** | 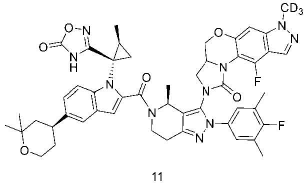 | 2-((*S*)-5-(5-((*S*)-2,2-dimethyltetrahydro-2*H-*pyran-4-yl)-1-((1*S*,2*S*)-2-methyl-1-(5-oxo-4,5-dihydro-1,2,4-oxadiazol-3-yl)cyclopropyl)-1*H*-indole-2-carbonyl)-2-(4-fluoro-3,5-dimethylphenyl)-4-methyl-4,5,6,7-tetrahydro-2*H*-pyrazolo[4,3-*c*]pyridin-3-yl)-10-fluoro-7-(trideuteriomethyl)-2,3,3*a*,4-tetrahydro-1*H*,7*H*-imidazo[1',5':4,5][1,4]oxazino[3,2-*f*]indazol-1-one **11 (mixture of diastereomers)** |
| 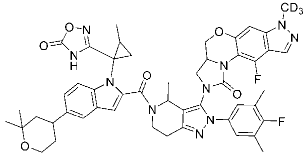 | | |
| **11-1** | 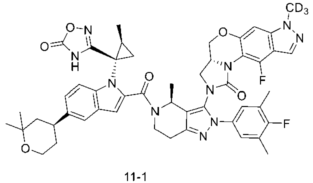 | (*R*)-2-((*S*)-5-(5-((*S*)-2,2-dimethyltetrahydro-2*H*-pyran-4-yl)-1-((1*S*,2*S*)-2-methyl-1-(5-oxo-4,5-dihydro-1,2,4-oxadiazol-3-yl)cyclopropyl)-1*H*-indole-2-carbonyl)-2-(4-fluoro-3,5-dimethylphenyl)-4-methyl-4,5,6,7-tetrahydro-2*H*-pyrazolo[4,3-*c*]pyridin-3-yl)-10-fluoro-7-(methyl-*d*₃)-2,3,3*a*,4-tetrahydro-1*H*,7*H-*imidazo[1',5':4,5][1,4]oxazino[3,2-*f*]indazol-1-one **11-1** |
| **11-2** | 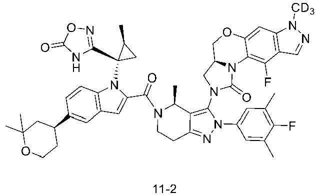 | (*S*)-2-((*S*)-5-(5-((*S*)-2,2-dimethyltetrahydro-2*H*-pyran-4-yl)-1-((1*S*,2*S*)-2-methyl-1-(5-oxo-4,5-dihydro-1,2,4-oxadiazol-3-yl)cyclopropyl)-1*H*-indole-2-carbonyl)-2-(4-fluoro-3,5-dimethylphenyl)-4-methyl-4,5,6,7-tetrahydro-2*H*-pyrazolo[4,3-*c*]pyridin-3-yl)-10-fluoro-7-(methyl-*d*₃)-2,3,3*a*,4-tetrahydro-1*H*,7*H-*imidazo[1',5':4,5][1,4]oxazino[3,2-*f*]indazol-1-one **11-2** |
| **12** | 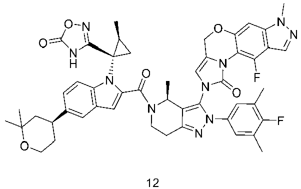 | 2-((*S*)-5-(5-((*S*)-2,2-dimethyltetrahydro-2*H-*pyran-4-yl)-1-((1*S*,2*S*)-2-methyl-1-(5-oxo-4,5-dihydro-1,2,4-oxadiazol-3-yl)cyclopropyl)-1*H*-indole-2-carbonyl)-2-(4-fluoro-3,5-dimethylphenyl)-4-methyl-4,5,6,7-tetrahydro-2*H*-pyrazolo[4,3-*c*]pyridin-3-yl)-10-fluoro-7-methyl-2,4-dihydro-1*H*,7*H*-imidazo[1', 5':4,5][1,4]oxazino[3,2-*f*]indazol-1-one **12** |
| 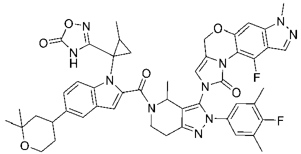 | | |
| **13** | 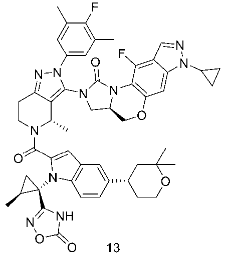 | (*R*)-7-cyclopropyl-2-((*S*)-5-(5-((*S*)-2,2-dimethyltetrahydro-2*H*-pyran-4-yl)-1-((1*S*,2*S*)-2-methyl-1-(5-oxo-4,5-dihydro-1,2,4-oxadiazol-3-yl)cyclopropyl)-1*H-*indole-2-carbonyl)-2-(4-fluoro-3,5-dimethylphenyl)-4-methyl-4,5,6,7-tetrahydro-2*H*-pyrazolo[4,3-*c*]pyridin-3-yl)-10-fluoro-2,3,3*a*,4-tetrahydro-1*H*,7*H-*imidazo[1',5':4,5][1,4]oxazino[3,2-*f*]indazol-1-one **13** |
| 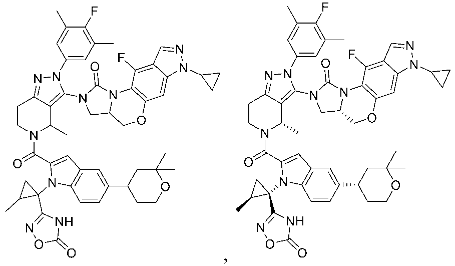 | | |
| **14** | 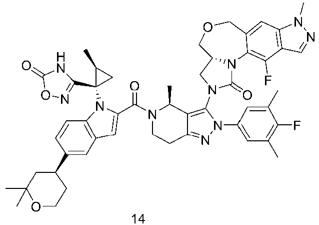 | (*R*)-2-((*S*)-5-(5-((*S*)-2,2-dimethyltetrahydro-2*H*-pyran-4-yl)-1-((1*S*,2*S*)-2-methyl-1-(5-oxo-4,5-dihydro-1,2,4-oxadiazol-3-yl)cyclopropyl)-1*H*-indole-2-carbonyl)-2-(4-fluoro-3,5-dimethylphenyl)-4-methyl-4,5,6,7-tetrahydro-2*H*-pyrazolo[4,3-*c*]pyridin-3-yl)-11-fluoro-8-methyl-2,3,3*a*,4,6,8-hexahydro-1*H-*imidazo[5',1':3,4][1,4]oxazepino[5,6-*f*]indazol-1-one **14** |
| 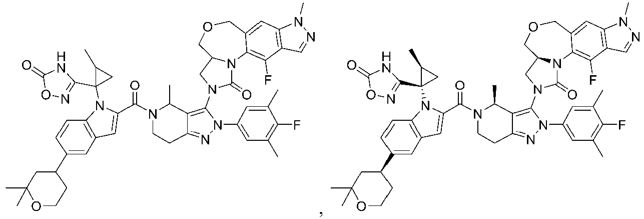 | | |
| **15** | 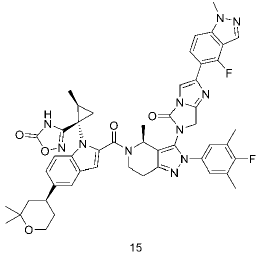 | 3-((1*S*,2*S*)-1-(5-((*S*)-2,2-dimethyltetrahydro-2*H*-pyran-4-yl)-2-((*S*)-3-(2-(4-fluoro-1-methyl-1*H*-indazol-5-yl)-5-oxo-5*H-*imidazo[1,5-*a*]imidazol-6(7*H*)-yl)-2-(4-fluoro-3,5-dimethylphenyl)-4-methyl-4,5,6,7-tetrahydro-2*H*-pyrazolo[4,3-*c*]pyridine-5-carbonyl)-1*H*-indol-1-yl)-2-methylcyclopropyl)-1,2,4-oxadiazol-5(4*H*)-one **15** |
| 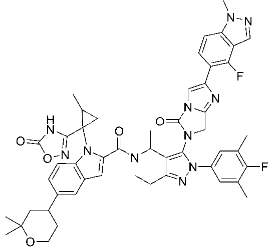 | | |
| **16-1** | 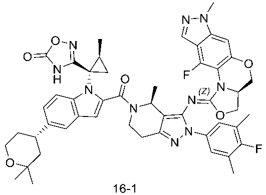 | 3-((1*S*,2*S*)-1-(5-((*S*)-2,2-dimethyltetrahydro-2*H*-pyran-4-yl)-2-((*S*)-2-(4-fluoro-3,5-dimethylphenyl)-3-(((*S*,*Z*)-10-fluoro-7-methyl-3*a*,4-dihydro-1*H*,3*H*,7*H-*oxazolo[3',4':4,5] [1,4]oxazino[3,2-*f*]indazol-1-ylidene)amino)-4-methyl-4,5,6,7-tetrahydro-2*H*-pyrazolo[4,3-*c*]pyridine-5-carbonyl)-1*H*-indol-1-yl)-2-methylcyclopropyl)-1,2,4-oxadiazol-5(4*H*)-one **16-1** |
| **16-2** | 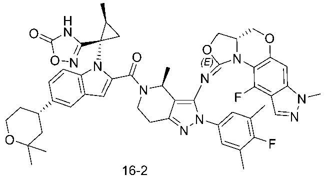 | 3-((1*S*,2*S*)-1-(5-((*S*)-2,2-dimethyltetrahydro-2*H*-pyran-4-yl)-2-((*S*)-2-(4-fluoro-3,5-dimethylphenyl)-3-(((*S*,*E*)-10-fluoro-7-methyl-3*a*,4-dihydro-1*H*,3*H*,7*H-*oxazolo[3',4':4,5] [1,4]oxazino[3,2-*f*]indazol-1-ylidene)amino)-4-methyl-4,5,6,7-tetrahydro-2*H*-pyrazolo[4,3-*c*]pyridine-5-carbonyl)-1*H*-indol-1-yl)-2-methylcyclopropyl)-1,2,4-oxadiazol-5(4*H*)-one **16-2** |
| 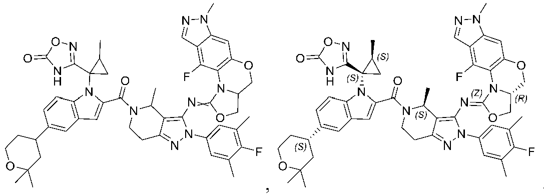 and | | |
| 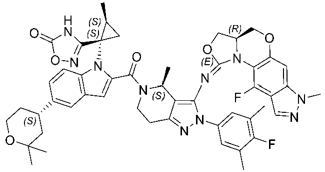 | | |
| **17** | 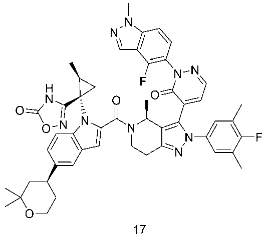 | 3-((1*S*,2*S*)-1-(5-((*S*)-2,2-dimethyltetrahydro-2*H*-pyran-4-yl)-2-((*S*)-3-(2-(4-fluoro-1-methyl-1*H*-indazol-5-yl)-3-oxo-2,3-dihydrohydropyridazin-4-yl)-2-(4-fluoro-3,5-dimethylphenyl)-4-methyl-4,5,6,7-tetrahydro-2*H*-pyrazolo[4,3-*c*]pyridine-5-carbonyl)-1*H*-indol-1-yl)-2-methylcyclopropyl)-1,2,4-oxadiazol-5(4*H*)-one **17** |
| 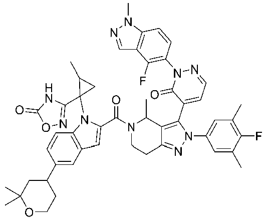 | | |
| **18** | 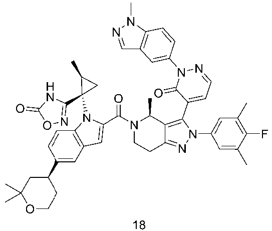 | 3-((1*S*,2*S*)-1-(5-((*S*)-2,2-dimethyltetrahydro-2*H*-pyran-4-yl)-2-((*S*)-2-(4-fluoro-3,5-dimethylphenyl)-4-methyl-3-(2-(1-methyl-1*H*-indazol-5-yl)-3-oxo-2,3-dihydropyridazin-4-yl)-4,5,6,7-tetrahydro-2*H*-pyrazolo[4,3-*c*]pyridine-5-carbonyl)-1*H*-indol-1-yl)-2-methylcyclopropyl)-1,2,4-oxadiazol-5(4*H*)-one **18** |
| 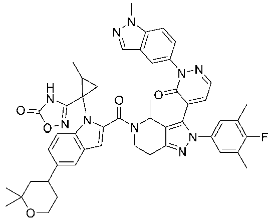 | | |
| **19-1** | 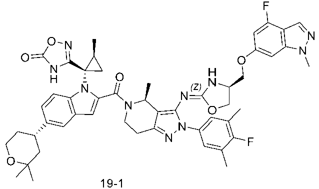 | 3-((1*S*,2*S*)-1-(5-((*S*)-2,2-dimethyltetrahydro-2*H*-pyran-4-yl)-2-((*S*)-3-(((*S,Z*)-4-(((4-fluoro-1-methyl-1*H*-indazol-6-yl)oxy)methyl)oxazolidin-2-ylidene)amino)-2-(4-fluoro-3,5-dimethylphenyl)-4-methyl-4,5,6,7-tetrahydro-2*H*-pyrazolo[4,3-*c*]pyridine-5-carbonyl)-1*H*-indol-1-yl)-2-methylcyclopropyl)-1,2,4-oxadiazol-5(4*H*)-one **19-1** |
| **19-2** | 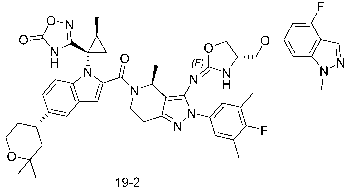 | 3-((1*S*,2*S*)-1-(5-((*S*)-2,2-dimethyltetrahydro-2*H*-pyran-4-yl)-2-((*S*)-3-(((*S,E*)-4-(((4-fluoro-1-methyl-1*H*-indazol-6-yl)oxy)methyl)oxazolidin-2-ylidene)amino)-2-(4-fluoro-3,5-dimethylphenyl)-4-methyl-4,5,6,7-tetrahydro-2*H*-pyrazolo[4,3-*c*]pyridine-5-carbonyl)-1*H*-indol-1-yl)-2-methylcyclopropyl)-1,2,4-oxadiazol-5(4*H*)-one **19-2** |
| 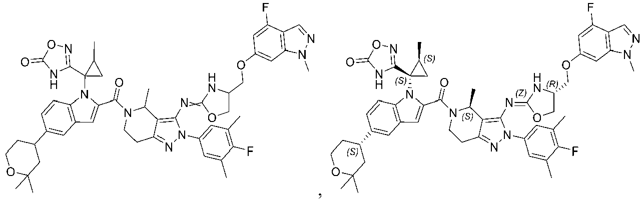 and | | |
| 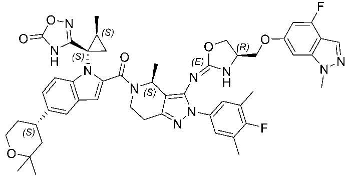 | | |
| **20** | 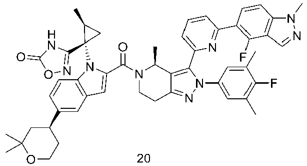 | 3-((1S,2S)-1-(5-((S)-2,2-dimethyltetrahydro-2H-pyran-4-yl)-2-((S)-3-(6-(4-fluoro-1-methyl-1H-indazol-5-yl)pyridin-2-yl)-2-(4-fluoro-3,5-dimethylphenyl)-4-methyl-4,5,6,7-tetrahydro-2H-pyrazolo[4,3-c]pyridine-5-carbonyl)-1H-indol-1-yl)-2-methylcyclopropyl)-1,2,4-oxadiazol-5(4H)-one **20** |
| 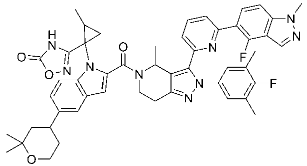 | | |
| **21-1** | 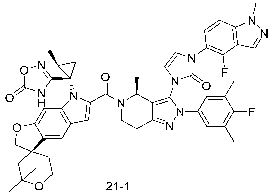 | 3-((1*S,*2*S*)-1-((*R*)-(6-((*S*)-3-(3-(4-fluoro-1-methyl-1*H*-indazol-5-yl)-2-oxo-2,3-dihydro-1*H*-imidazol-1-yl)-2-(4-fluoro-3,5-dimethylphenyl)-4-methyl-4,5,6,7-tetrahydro-2*H*-pyrazolo[4,3-*c*]pyridine-5-carbonyl)-2',2'-dimethyl-2',3',5',6'-tetrahydro-2*H*,7*H*-spiro[furo[3,2-*f*]indole-3,4'-pyran]-7-yl)-2-methylcyclopropyl)-1,2,4-oxadiazol-5(4*H*)-one **21-1** |
| 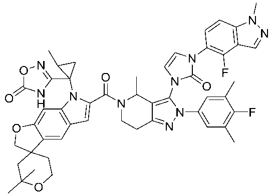 | | |
| **21-2** | 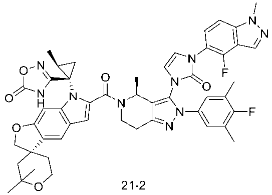 | 3-((1*S*,2*S*)-1-((*S*)-(6-((*S*)-3-(3-(4-fluoro-1-methyl-1*H*-indazol-5-yl)-2-oxo-2,3-dihydro-1*H*-imidazol-1-yl)-2-(4-fluoro-3,5-dimethylphenyl)-4-methyl-4,5,6,7-tetrahydro-2*H*-pyrazolo[4,3-*c*]pyridine-5-carbonyl)-2',2'-dimethyl-2',3',5',6'-tetrahydro-2*H*,7*H*-spiro[furo[3,2-*f*]indole-3,4'-pyran]-7-yl)-2-methylcyclopropyl)-1,2,4-oxadiazol-5(4*H*)-one **21-2** |
| **22** | 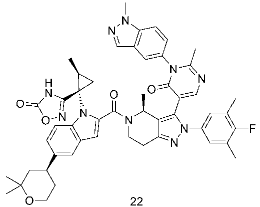 | 3-((1*S*,2*S*)-1-(5-((*S*)-2,2-dimethyltetrahydro-2*H*-pyran-4-yl)-2-((*S*)-2-(4-fluoro-3,5-dimethylphenyl)-4-methyl-3-(2-methyl-1-(1-methyl-1*H*-indazol-5-yl)-6-oxo-1,6-dihydropyrimidin-5-yl)-4,5,6,7-tetrahydro-2*H*-pyrazolo[4,3-*c*]pyridine-5-carbonyl)-1*H*-indol-1-yl)-2-methylcyclopropyl)-1,2,4-oxadiazol-5(4*H*)-one **22** |
| 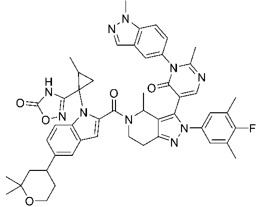 | | |
| **23** | 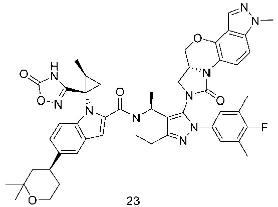 | (*R*)-7-((*S*)-5-(5-((*S*)-2,2-dimethyltetrahydro-2*H*-pyran-4-yl)-1-((1*S*,2*S*)-2-methyl-1-(5-oxo-4,5-dihydro-1,2,4-oxadiazol-3-yl)cyclopropyl)-1*H*-indole-2-carbonyl)-2-(4-fluoro-3,5-dimethylphenyl)-4-methyl-4,5,6,7-tetrahydro-2*H*-pyrazolo[4,3-*c*]pyridin-3-yl)-1-methyl-5,5*a*,6,7-tetrahydro-1*H*,8*H-*imidazo[1',5':4,5][1,4]oxazino[2,3-*e*]indazol-8-one **23** |
| 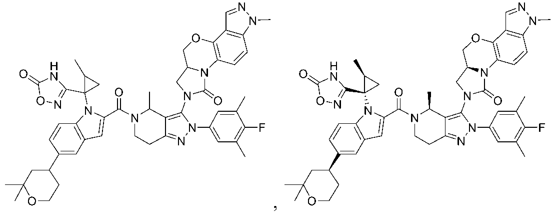 | | |
| **24** | 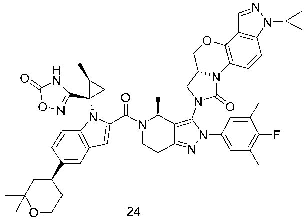 | (*R*)-1-cyclopropyl-7-((*S*)-5-(5-((*S*)-2,2-dimethyltetrahydro-2*H*-pyran-4-yl)-1-((1*S*,2*S*)-2-methyl-1-(5-oxo-4,5-dihydro-1,2,4-oxadiazol-3-yl)cyclopropyl)-1*H-*indole-2-carbonyl)-2-(4-fluoro-3,5-dimethylphenyl)-4-methyl-4,5,6,7-tetrahydro-2*H*-pyrazolo[4,3-*c*]pyridin-3-yl)-5,5*a*,6,7-tetrahydro-1*H*,8*H-*imidazo[1',5':4,5][1,4]oxazino[2,3-*e*]indazol-8-one **24** |
| 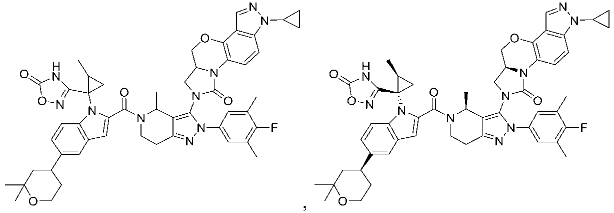 | | |
| **25** | 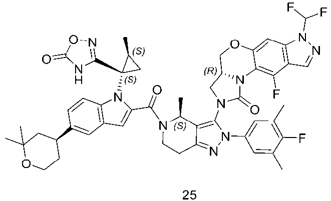 | (*R*)-7-(difluoromethyl)-2-((*S*)-5-(5-((*S*)-2,2-dimethyltetrahydro-2*H*-pyran-4-yl)-1-((1*S*,2*S*)-2-methyl-1-(5-oxo-4,5-dihydro-1,2,4-oxadiazol-3-yl)cyclopropyl)-1*H-*indole-2-carbonyl)-2-(4-fluoro-3,5-dimethylphenyl)-4-methyl-4,5,6,7-tetrahydro-2*H*-pyrazolo[4,3-*c*]pyridin-3-yl)-10-fluoro-2,3,3*a*,4-tetrahydro-1*H*,7*H-*imidazo[1',5':4,5][1,4]oxazino[3,2-*f*]indazol-1-one **25** |
| 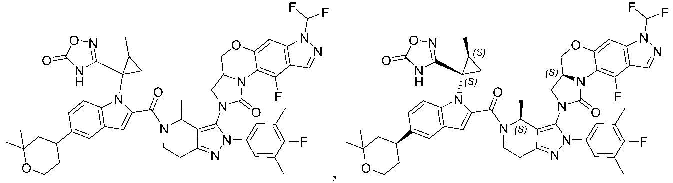 | | |
| **26-1** | 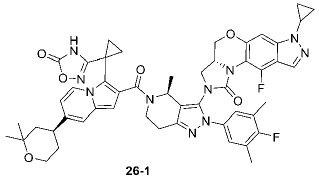 | (*R*)-7-cyclopropyl-2-((*S*)-5-(7-((*S*)-2,2-dimethyltetrahydro-2*H*-pyran-4-yl)-3-(1-(5-oxo-4,5-dihydro-1,2,4-oxadiazol-3-yl)cyclopropyl)indolizine-2-carbonyl)-2-(4-fluoro-3,5-dimethylphenyl)-4-methyl-4,5,6,7-tetrahydro-2*H*-pyrazolo[4,3-*c*]pyridin-3-yl)-10-fluoro-2,3,3*a*,4-tetrahydro-1H,7H-imidazo[1',5':4,5][1,4]oxazino[3,2-*f*]indazol-1-one **26-1** |
| 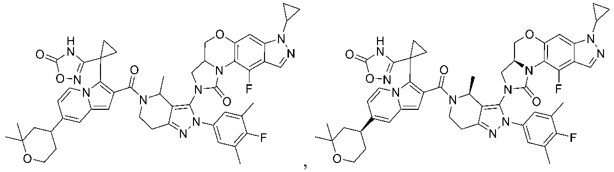 | | |
| **26-2** | 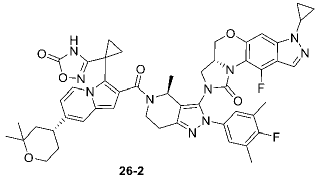 | (*R*)-7-cyclopropyl-2-((*S*)-5-(7-((*R*)-2,2-dimethyltetrahydro-2*H*-pyran-4-yl)-3-(1-(5-oxo-4,5-dihydro-1,2,4-oxadiazol-3-yl)cyclopropyl)indolizine-2-carbonyl)-2-(4-fluoro-3,5-dimethylphenyl)-4-methyl-4,5,6,7-tetrahydro-2*H*-pyrazolo[4,3-*c*]pyridin-3-yl)-10-fluoro-2,3,3*a*,4-tetrahydro-1*H*,7*H-*imidazo[1',5':4,5][1,4]oxazino[3,2-*f*]indazol-1-one **26-2** |
| 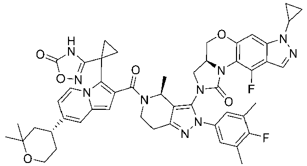 | | |
| **27** | 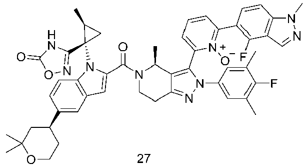 | 2-((4*S*)-5-(5-((*S*)-2,2-dimethyltetrahydro-2*H*-pyran-4-yl)-1-((1*S*,2*S*)-2-methyl-1-(5-oxo-4,5-dihydro-1,2,4-oxadiazol-3-yl)cyclopropyl)-1*H*-indole-2-carbonyl)-2-(4-fluoro-3,5-dimethylphenyl)-4-methyl-4,5,6,7-tetrahydro-2*H*-pyrazolo[4,3-*c*]pyridin-3-yl)-6-(4-fluoro-1-methyl-1*H-*indazol-5-yl)pyridine 1-oxide **27** |
| 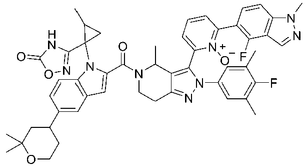 | | |
| **28-1** | 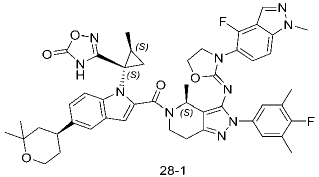 | 3-((1*S*,2*S*)-1-(5-((*S*)-2,2-dimethyltetrahydro-2*H*-pyran-4-yl)-2-((*S*)-3-(((*Z*)-3-(4-fluoro-1-methyl-1*H*-indazol-5-yl)oxazolidin-2-ylidene)amino)-2-(4-fluoro-3,5-dimethylphenyl)-4-methyl-4,5,6,7-tetrahydro-2*H*-pyrazolo[4,3-*c*]pyridine-5-carbonyl)-1*H*-indol-1-yl)-2-methylcyclopropyl)-1,2,4-oxadiazol-5(4*H*)-one **28-1** |
| 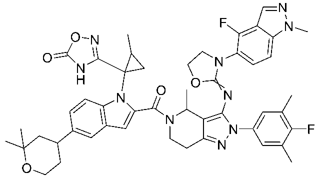 | | |
| **28-2** | 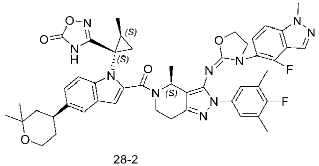 | 3-((1*S*,2*S*)-1-(5-((*S*)-2,2-dimethyltetrahydro-2*H*-pyran-4-yl)-2-((*S*)-3-(((*E*)-3-(4-fluoro-1-methyl-1*H*-indazol-5-yl)oxazolidin-2-ylidene)amino)-2-(4-fluoro-3,5-dimethylphenyl)-4-methyl-4,5,6,7-tetrahydro-2*H*-pyrazolo[4,3-*c*]pyridine-5-carbonyl)-1*H*-indol-1-yl)-2-methylcyclopropyl)-1,2,4-oxadiazol-5(4*H*)-one **28-2** |
| **29** | 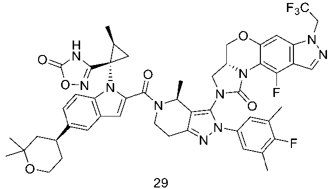 | (*R*)-2-((*S*)-5-(5-((*S*)-2,2-dimethyltetrahydro-2*H*-pyran-4-yl)-1-((1*S*,2*S*)-2-methyl-1-(5-oxo-4,5-dihydro-1,2,4-oxadiazol-3-yl)cyclopropyl)-1*H*-indole-2-carbonyl)-2-(4-fluoro-3,5-dimethylphenyl)-4-methyl-4,5,6,7-tetrahydro-2*H*-pyrazolo[4,3-*c*]pyridin-3-yl)-10-fluoro-7-(2,2,2-trifluoroethyl)-2,3,3*a*,4-tetrahydro-1*H*,7*H-*imidazo[1',5':4,5][1,4]oxazino[3,2-*f*]indazol-1-one **29** |
| 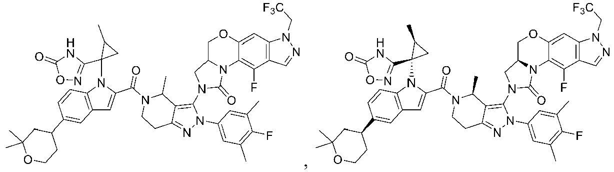 | | |
| **30** | 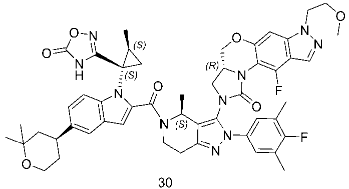 | (*R*)-2-((*S*)-5-(5-((*S*)-2,2-dimethyltetrahydro-2*H*-pyran-4-yl)-1-((1*S*,2*S*)-2-methyl-1-(5-oxo-4,5-dihydro-1,2,4-oxadiazol-3-yl)cyclopropyl)-1*H*-indole-2-carbonyl)-2-(4-fluoro-3,5-dimethylphenyl)-4-methyl-4,5,6,7-tetrahydro-2*H*-pyrazolo[4,3-*c*]pyridin-3-yl)-10-fluoro-7-(2-methoxyethyl)-2,3,3*a*,4-tetrahydro-1*H*,7*H-*imidazo[1',5':4,5][1,4]oxazino[3,2-*f*]indazol-1-one **30** |
| 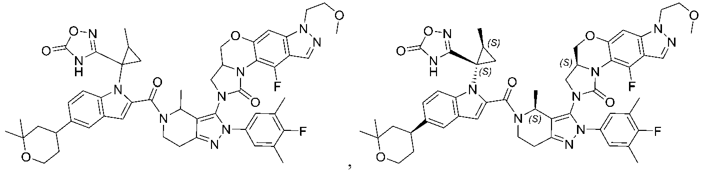 | | |
| **31-1** | 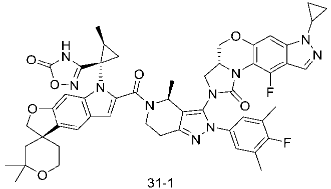 | (*R*)-7-cyclopropyl-2-((*S*)-5-((*R*)-2',2'-dimethyl-7-((1*S*,2*S*)-2-methyl-1-(5-oxo-4,5-dihydro-1,2,4-oxadiazol-3-yl)cyclopropyl)-2',3',5',6'-tetrahydro-2*H*,7*H*-spiro[furo[3,2-*f*]indole-3,4'-pyran]-6-carbonyl)-2-(4-fluoro-3,5-dimethylphenyl)-4-methyl-4,5,6,7-tetrahydro-2*H*-pyrazolo[4,3-c]pyridin-3-yl)-10-fluoro-2,3,3*a*,4-tetrahydro-1*H*,7*H-*imidazo[1',5':4,5][1,4]oxazino[3,2-*f*]indazol-1-one **31-1** |
| 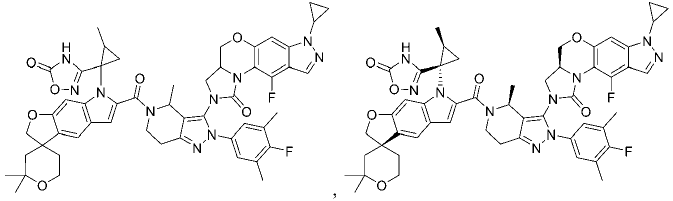 | | |
| **31-2** | 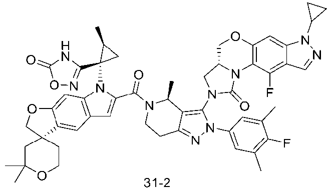 | (*R*)-7-cyclopropyl-2-((*S*)-5-((S)-2',2'-dimethyl-7-((1*S*,2*S*)-2-methyl-1-(5-oxo-4,5-dihydro-1,2,4-oxadiazol-3-yl)cyclopropyl)-2',3',5',6'-tetrahydro-2*H*,7*H*-spiro[furo[3,2-*f*]indole-3,4'-pyran]-6-carbonyl)-2-(4-fluoro-3,5-dimethylphenyl)-4-methyl-4,5,6,7-tetrahydro-2*H*-pyrazolo[4,3-*c*]pyridin-3-yl)-10-fluoro-2,3,3*a*,4-tetrahydro-1*H*,7*H-*imidazo[1',5':4,5][1,4]oxazino[3,2-*f*]indazol-1-one **31-2** |
| 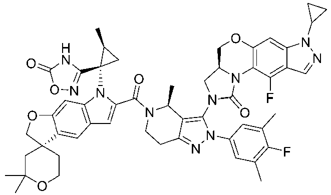 | | |
| **32** | 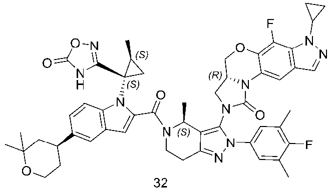 | (*R*)-7-cyclopropyl-2-((*S*)-5-(5-((*S*)-2,2-dimethyltetrahydro-2*H*-pyran-4-yl)-1-((1*S*,2*S*)-2-methyl-1-(5-oxo-4,5-dihydro-1,2,4-oxadiazol-3-yl)cyclopropyl)-1*H-*indole-2-carbonyl)-2-(4-fluoro-3,5-dimethylphenyl)-4-methyl-4,5,6,7-tetrahydro-2*H*-pyrazolo[4,3-*c*]pyridin-3-yl)-6-fluoro-2,3,3*a*,4-tetrahydro-1*H*,7*H-*imidazo[1',5':4,5][1,4]oxazino[3,2-*f*]indazol-1-one **32** |
| 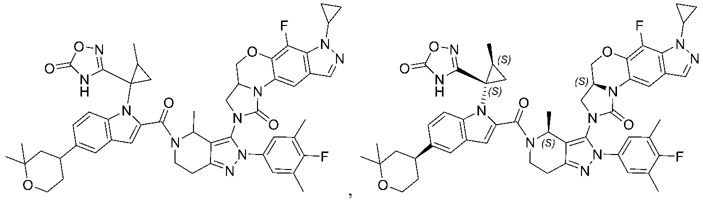 | | |
| **33** | 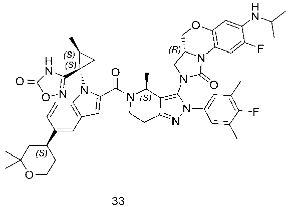 | (*R*)-2-((*S*)-5-(5-((*S*)-2,2-dimethyltetrahydro-2*H*-pyran-4-yl)-1-((1*S*,2*S*)-2-methyl-1-(5-oxo-4,5-dihydro-1,2,4-oxadiazol-3-yl)cyclopropyl)-1*H*-indole-2-carbonyl)-2-(4-fluoro-3,5-dimethylphenyl)-4-methyl-4,5,6,7-tetrahydro-2*H*-pyrazolo[4,3-*c*]pyridin-3-yl)-8-fluoro-7-(isopropylamino)-2,3,3*a*,4-tetrahydro-1*H-*benzo[*b*]imidazo[1,5-*d*][1,4]oxazin-1-one **33** |
| 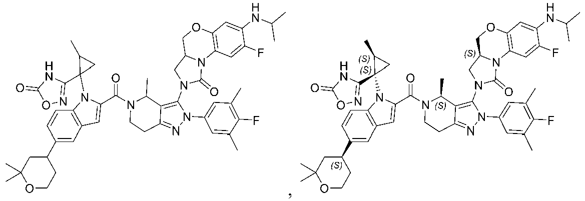 | | |
| **34-1** | 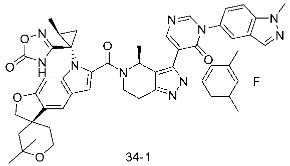 | 3-((1*S*,2*S*)-1-((*R*)-6-((*S*)-2-(4-fluoro-3,5-dimethylphenyl)-4-methyl-3-(1-(1-methyl-1*H*-indazol-5-yl)-6-oxo-1,6-dihydropyrimidin-5-yl)-4,5,6,7-tetrahydro-2*H*-pyrazolo[4,3-*c*]pyridine-5-carbonyl)-2',2'-dimethyl-2',3',5',6'-tetrahydro-2*H*,7*H-*spiro[furo[3,2-*f*]indole-3,4'-pyran]-7-yl)-2-methylcyclopropyl)-1,2,4-oxadiazol-5(4*H*)-one **34-1** |
| 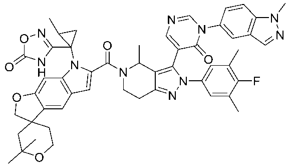 | | |
| **34-2** | 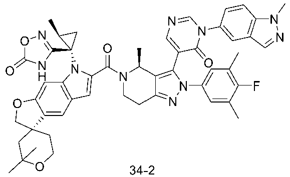 | 3-((1*S*,2*S*)-1-((*S*)-6-((*S*)-2-(4-fluoro-3,5-dimethylphenyl)-4-methyl-3-(1-(1-methyl-1*H*-indazol-5-yl)-6-oxo-1,6-dihydropyrimidin-5-yl)-4,5,6,7-tetrahydro-2*H*-pyrazolo[4,3-*c*]pyridine-5-carbonyl)-2',2'-dimethyl-2',3',5',6'-tetrahydro-2*H*,7*H-*spiro[furo[3,2-*f*]indole-3,4'-pyran]-7-yl)-2-methylcyclopropyl)-1,2,4-oxadiazol-5(4*H*)-one **34-2** |
| **35-1** | 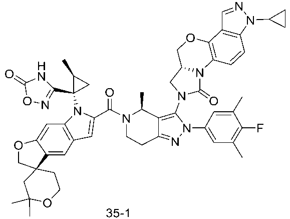 | (*R*)-1-cyclopropyl-7-((*S*)-5-((*R*)-2',2'-dimethyl-7-((1*S*,2*S*)-2-methyl-1-(5-oxo-4,5-dihydro-1,2,4-oxadiazol-3-yl)cyclopropyl)-2',3',5',6'-tetrahydro-2*H*,7*H*-spiro[furo[3,2-*f*]indole-3,4'-pyran]-6-carbonyl)-2-(4-fluoro-3,5-dimethylphenyl)-4-methyl-4,5,6,7-tetrahydro-2*H*-pyrazolo[4,3-*c*]pyridin-3-yl)-5,5*a*,6,7-tetrahydro-1*H*,8*H-*imidazo[1',5':4,5][1,4]oxazino[2,3-e]indazol-8-one **35-1** |
| 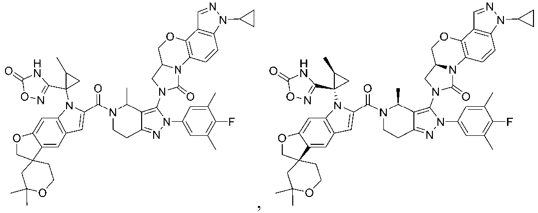 | | |
| **35-2** | 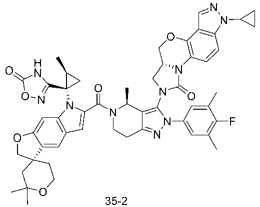 | (*R*)-1-cyclopropyl-7-((*S*)-5-((*S*)-2',2'-dimethyl-7-((1*S*,2*S*)-2-methyl-1-(5-oxo-4,5-dihydro-1,2,4-oxadiazol-3-yl)cyclopropyl)-2',3',5',6'-tetrahydro-2*H*,7*H*-spiro[furo[3,2-*f*]indole-3,4'-pyran]-6-carbonyl)-2-(4-fluoro-3,5-dimethylphenyl)-4-methyl-4,5,6,7-tetrahydro-2*H*-pyrazolo[4,3-*c*]pyridin-3-yl)-5,5*a*,6,7-tetrahydro-1*H*,8*H-*imidazo[1',5':4,5][1,4]oxazino[2,3-*e*]indazol-8-one **35-2** |
| 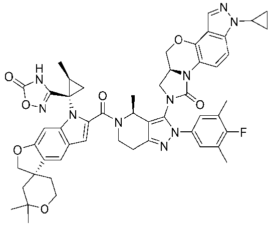 | | |
| **36** | 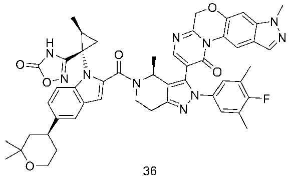 | 2-((*S*)-5-(5-((*S*)-2,2-dimethyltetrahydro-2*H-*pyran-4-yl)-1-((1*S*,2*S*)-2-methyl-1-(5-oxo-4,5-dihydro-1,2,4-oxadiazol-3-yl)cyclopropyl)-1*H*-indole-2-carbonyl)-2-(4-fluoro-3,5-dimethylphenyl)-4-methyl-4,5,6,7-tetrahydro-2*H*-pyrazolo[4,3-*c*]pyridin-3-yl)-8-methyl-8*H-*pyrimido[1',2':4,5][1,4]oxazino[3,2-*f*]indazol-1(5*H*)-one **36** |
| 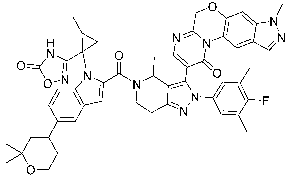 | | |
| **37** | 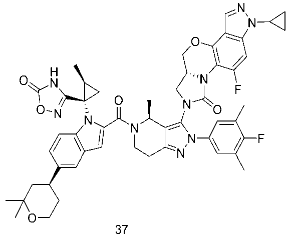 | (*R*)-1-cyclopropyl-7-((*S*)-5-(5-((*S*)-2,2-dimethyltetrahydro-2*H*-pyran-4-yl)-1-((1*S*,2*S*)-2-methyl-1-(5-oxo-4,5-dihydro-1,2,4-oxadiazol-3-yl)cyclopropyl)-1*H-*indole-2-carbonyl)-2-(4-fluoro-3,5-dimethylphenyl)-4-methyl-4,5,6,7-tetrahydro-2*H*-pyrazolo[4,3-*c*]pyridin-3-yl)-10-fluoro-5,5*a*,6,7-tetrahydro-1*H*,8*H-*imidazo[1',5':4,5][1,4]oxazino[2,3-*e*]indazol-8-one **37** |
| 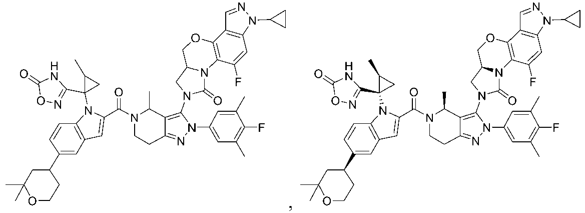 | | |
| **38** | 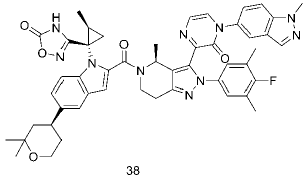 | 3-((1*S*,2*S*)-1-(5-((*S*)-2,2-dimethyltetrahydro-2*H*-pyran-4-yl)-2-((S)-2-(4-fluoro-3,5-dimethylphenyl)-4-methyl-3-(4-(1-methyl-1*H*-indazol-5-yl)-3-oxo-3,4-dihydropyrazin-2-yl)-4,5,6,7-tetrahydro-2*H*-pyrazolo[4,3-*c*]pyridine-5-carbonyl)-1*H*-indol-1-yl)-2-methylcyclopropyl)-1,2,4-oxadiazol-5(4*H*)-one **38** |
| 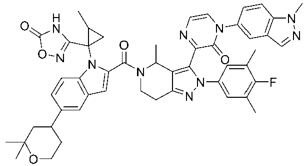 | | |
| **39** | 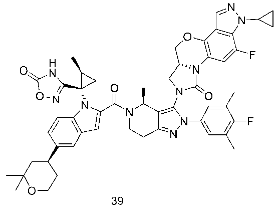 | (*R*)-1-cyclopropyl-7-((*S*)-5-(5-((*S*)-2,2-dimethyltetrahydro-2*H*-pyran-4-yl)-1-((1*S*,2*S*)-2-methyl-1-(5-oxo-4,5-dihydro-1,2,4-oxadiazol-3-yl)cyclopropyl)-1*H-*indole-2-carbonyl)-2-(4-fluoro-3,5-dimethylphenyl)-4-methyl-4,5,6,7-tetrahydro-2*H*-pyrazolo[4,3-*c*]pyridin-3-yl)-11-fluoro-5,5*a*,6,7-tetrahydro-1*H*,8*H-*imidazo[1',5':4,5][1,4]oxazino[2,3-*e*]indazol-8-one **39** |
| 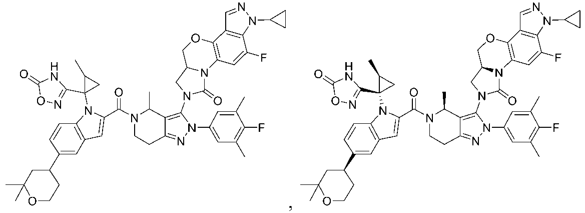 | | |
| **40** | 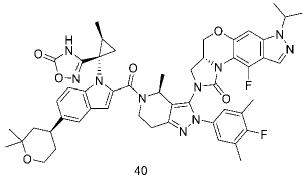 | (*R*)-2-((*S*)-5-(5-((*S*)-2,2-dimethyltetrahydro-2*H*-pyran-4-yl)-1-((1*S*,2*S*)-2-methyl-1-(5-oxo-4,5-dihydro-1,2,4-oxadiazol-3-yl)cyclopropyl)-1*H*-indole-2-carbonyl)-2-(4-fluoro-3,5-dimethylphenyl)-4-methyl-4,5,6,7-tetrahydro-2*H*-pyrazolo[4,3-*c*]pyridin-3-yl)-10-fluoro-7-isopropyl-2,3,3*a*,4-tetrahydro-1*H*,7*H-*imidazo[1',5':4,5][1,4]oxazino[3,2-*f*]indazol-1-one **40** |
| 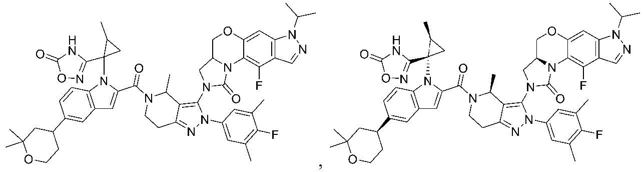 | | |
| **41** | 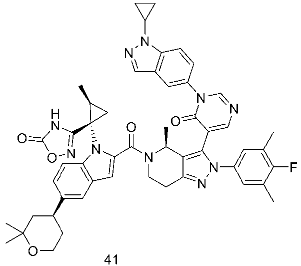 | 3-((1*S*,2*S*)-1-(2-((*S*)-3-(1-(1-cyclopropyl-1H-indazol-5-yl)-6-oxo-1,6-dihydropyrimidin-5-yl)-2-(4-fluoro-3,5-dimethylphenyl)-4-methyl-4,5,6,7-tetrahydro-2*H*-pyrazolo[4,3-*c*]pyridine-5-carbonyl)-5-((*S*)-2,2-dimethyltetrahydro-2*H*-pyran-4-yl)-1*H*-indol-1-yl)-2-methylcyclopropyl)-1,2,4-oxadiazol-5(4*H*)-one **41** |
| 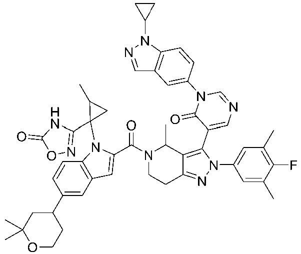 | | |
| **42** | 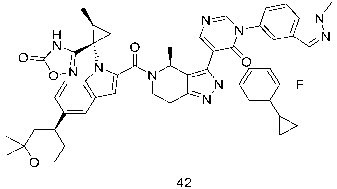 | 3-((1*S*,2*S*)-1-(2-((*S*)-2-(3-cyclopropyl-4-fluorophenyl)-4-methyl-3-(1-(1-methyl-1*H-*indazol-5-yl)-6-oxo-1,6-dihydropyrimidin-5-yl)-4,5,6,7-tetrahydro-2*H*-pyrazolo[4,3-*c*]pyridine-5-carbonyl)-5-((*S*)-2,2-dimethyltetrahydro-2*H*-pyran-4-yl)-1*H-*indol-1-yl)-2-methylcyclopropyl)-1,2,4-oxadiazol-5(4*H*)-one **42** |
| 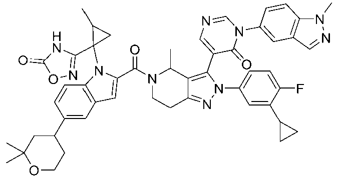 | | |
| **43** | 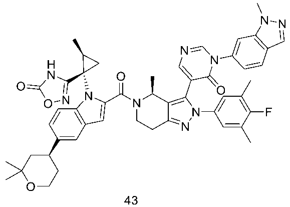 | 3-((1*S*,2*S*)-1-(5-((*S*)-2,2-dimethyltetrahydro-2*H*-pyran-4-yl)-2-((*S*)-2-(4-fluoro-3,5-dimethylphenyl)-4-methyl-3-(1-(1-methyl-1*H*-indazol-6-yl)-6-oxo-1,6-dihydropyrimidin-5-yl)-4,5,6,7-tetrahydro-2*H*-pyrazolo[4,3-*c*]pyridine-5-carbonyl)-1*H*-indol-1-yl)-2-methylcyclopropyl)-1,2,4-oxadiazol-5(4*H*)-one **43** |
| 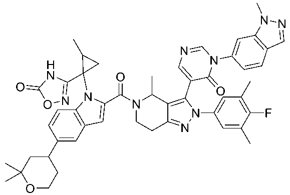 | | |
| **44** | 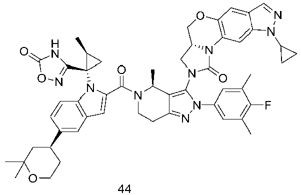 | (*R*)-9-cyclopropyl-2-((*S*)-5-(5-((*S*)-2,2-dimethyltetrahydro-2*H*-pyran-4-yl)-1-((1*S*,2*S*)-2-methyl-1-(5-oxo-4,5-dihydro-1,2,4-oxadiazol-3-yl)cyclopropyl)-1*H-*indole-2-carbonyl)-2-(4-fluoro-3,5-dimethylphenyl)-4-methyl-4,5,6,7-tetrahydro-2*H*-pyrazolo[4,3-*c*]pyridin-3-yl)-2,3,3*a*,4-tetrahydro-1*H*,9*H-*imidazo[1',5':4,5][1,4]oxazino[2,3-*f*]indazol-1-one **44** |
| 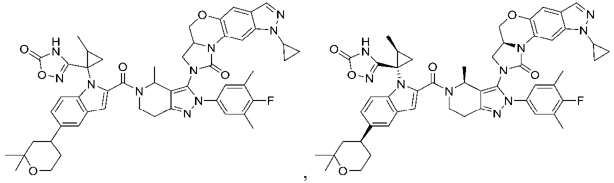 | | |
| **45** | 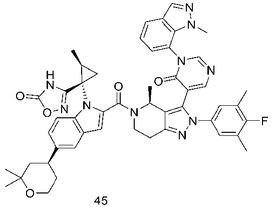 | 3-((1*S*,2*S*)-1-(5-((*S*)-2,2-dimethyltetrahydro-2*H*-pyran-4-yl)-2-((*S*)-2-(4-fluoro-3,5-dimethylphenyl)-4-methyl-3-(1-(1-methyl-1*H*-indazol-7-yl)-6-oxo-1,6-dihydropyrimidin-5-yl)-4,5,6,7-tetrahydro-2*H*-pyrazolo[4,3-*c*]pyridine-5-carbonyl)-1*H*-indol-1-yl)-2-methylcyclopropyl)-1,2,4-oxadiazol-5(4*H*)-one **45** |
| 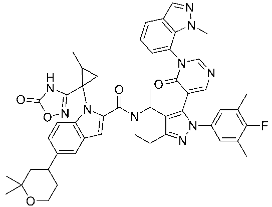 | | |
| **46** | 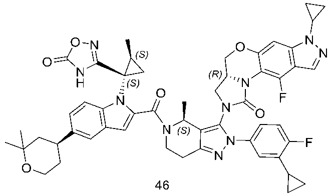 | (*R*)-7-cyclopropyl-2-((*S*)-2-(3-cyclopropyl-4-fluorophenyl)-5-(5-((*S*)-2,2-dimethyltetrahydro-2*H*-pyran-4-yl)-1-((1*S*,2*S*)-2-methyl-1-(5-oxo-4,5-dihydro-1,2,4-oxadiazol-3-yl)cyclopropyl)-1*H-*indole-2-carbonyl)-4-methyl-4,5,6,7-tetrahydro-2*H*-pyrazolo[4,3-*c*]pyridin-3-yl)-10-fluoro-2,3,3*a*,4-tetrahydro-1*H*,7*H-*imidazo[1',5':4,5][1,4]oxazino[3,2-*f*]indazol-1-one **46** |
| 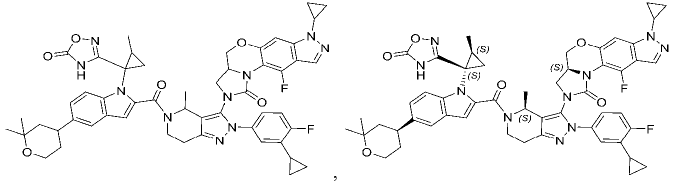 | | |
| **47** | 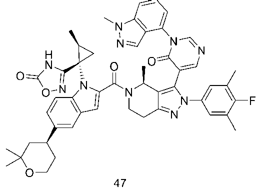 | 3-((1*S*,2*S*)-1-(5-((*S*)-2,2-dimethyltetrahydro-2*H*-pyran-4-yl)-2-((*S*)-2-(4-fluoro-3,5-dimethylphenyl)-4-methyl-3-(1-(1-methyl-1*H*-indazol-4-yl)-6-oxo-1,6-dihydropyrimidin-5-yl)-4,5,6,7-tetrahydro-2*H*-pyrazolo[4,3-*c*]pyridine-5-carbonyl)-1*H*-indol-1-yl)-2-methylcyclopropyl)-1,2,4-oxadiazol-5(4*H*)-one **47** |
| 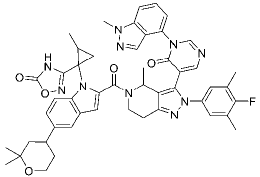 | | |
| **48** | 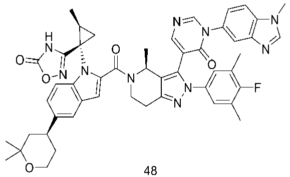 | 3-((1*S*,2*S*)-1-(5-((*S*)-2,2-dimethyltetrahydro-2*H*-pyran-4-yl)-2-((*S*)-2-(4-fluoro-3,5-dimethylphenyl)-4-methyl-3-(1-(1-methyl-1*H*-benzo[*d*]imidazol-5-yl)-6-oxo-1,6-dihydropyrimidin-5-yl)-4,5,6,7-tetrahydro-2*H*-pyrazolo[4,3-*c*]pyridine-5-carbonyl)-1*H*-indol-1-yl)-2-methylcyclopropyl)-1,2,4-oxadiazol-5(4*H*)-one **48** |
| 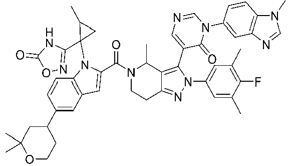 | | |
| **49** | 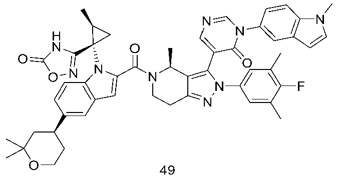 | 3-((1*S*,2*S*)-1-(5-((*S*)-2,2-dimethyltetrahydro-2*H*-pyran-4-yl)-2-((*S*)-2-(4-fluoro-3,5-dimethylphenyl)-4-methyl-3-(1-(1-methyl-1*H*-indol-5-yl)-6-oxo-1,6-dihydropyrimidin-5-yl)-4,5,6,7-tetrahydro-2*H*-pyrazolo[4,3-*c*]pyridine-5-carbonyl)-1*H*-indol-1-yl)-2-methylcyclopropyl)-1,2,4-oxadiazol-5(4*H*)-one **49** |
| 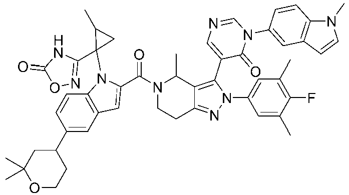 | | |
| **50** | 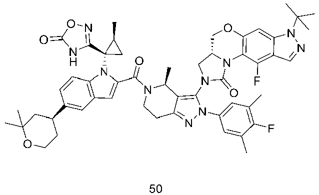 | (R)-7-(tert-butyl)-2-((S)-5-(5-((S)-2,2-dimethyltetrahydro-2H-pyran-4-yl)-1-((1S,2S)-2-methyl-1-(5-oxo-4,5-dihydro-1,2,4-oxadiazol-3-yl)cyclopropyl)-1H-indole-2-carbonyl)-2-(4-fluoro-3,5-dimethylphenyl)-4-methyl-4,5,6,7-tetrahydro-2H-pyrazolo[4,3-c]pyridin-3-yl)-10-fluoro-2,3,3a,4-tetrahydro-1H,7H-imidazo[1',5':4,5][1,4]oxazino[3,2-f]indazol-1-one **50** |
| 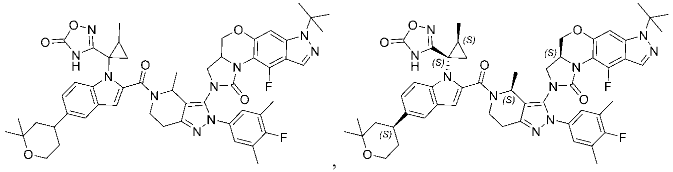 | | |
| **51** | 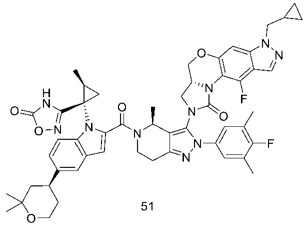 | (R)-7-(cyclopropylmethyl)-2-((S)-5-(5-((S)-2,2-dimethyltetrahydro-2H-pyran-4-yl)-1-((1S,2S)-2-methyl-1-(5-oxo-4,5-dihydro-1,2,4-oxadiazol-3-yl)cyclopropyl)-1H-indole-2-carbonyl)-2-(4-fluoro-3,5-dimethylphenyl)-4-methyl-4,5,6,7-tetrahydro-2H-pyrazolo[4,3-c]pyridin-3-yl)-10-fluoro-2,3,3a,4-tetrahydro-1H,7H-imidazo[1',5':4,5][1,4]oxazino[3,2-f]indazol-1-one **51** |
| 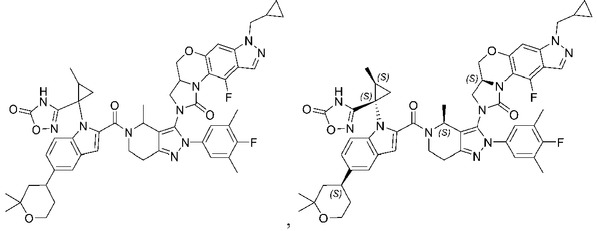 | | |
| **52-1** | 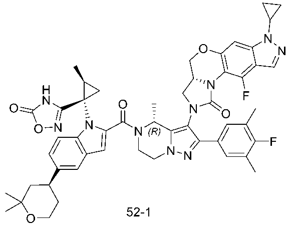 | (R)-7-cyclopropyl-2-((R)-5-(5-((S)-2,2-dimethyltetrahydro-2H-pyran-4-yl)-1-((1S,2S)-2-methyl-1-(5-oxo-4,5-dihydro-1,2,4-oxadiazol-3-yl)cyclopropyl)-1H-indole-2-carbonyl)-2-(4-fluoro-3,5-dimethylphenyl)-4-methyl-4,5,6,7-tetrahydropyrazolo[1,5-a]pyrazin-3-yl)-10-fluoro-2,3,3a,4-tetrahydro-1H,7H-imidazo[1',5':4,5][1,4]oxazino[3,2-f]indazol-1-one **52-1** |
| **52-2** | 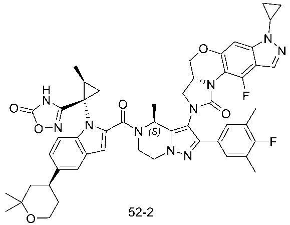 | (R)-7-cyclopropyl-2-((S)-5-(5-((S)-2,2-dimethyltetrahydro-2H-pyran-4-yl)-1-((1S,2S)-2-methyl-1-(5-oxo-4,5-dihydro-1,2,4-oxadiazol-3-yl)cyclopropyl)-1H-indole-2-carbonyl)-2-(4-fluoro-3,5-dimethylphenyl)-4-methyl-4,5,6,7-tetrahydropyrazolo[1,5-a]pyrazin-3-yl)-10-fluoro-2,3,3a,4-tetrahydro-1H,7H-imidazo[1',5':4,5][1,4]oxazino[3,2-f]indazol-1-one **52-2** |
| 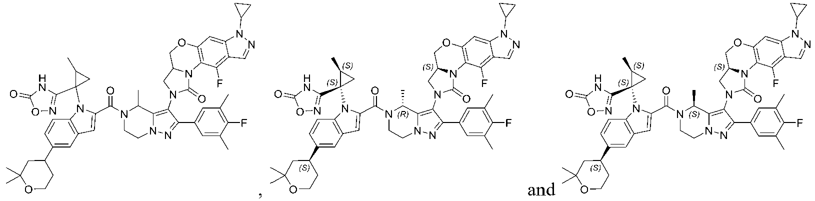 | | |
| **53-1** | 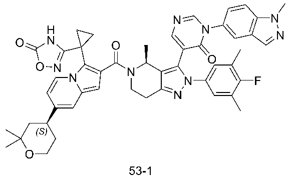 | 3-(1-(7-((S)-2,2-dimethyltetrahydro-2H-pyran-4-yl)-2-((S)-2-(4-fluoro-3,5-dimethylphenyl)-4-methyl-3-(1-(1-methyl-1H-indazol-5-yl)-6-oxo-1,6-dihydropyrimidin-5-yl)-4,5,6,7-tetrahydro-2H-pyrazolo[4,3-c]pyridine-5-carbonyl)indolizin-3-yl)cyclopropyl)-1,2,4-oxadiazol-5(4H)-one **53-1** |
| **53-2** | 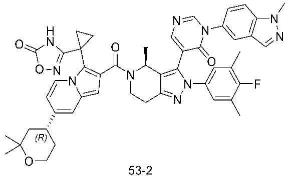 | 3-(1-(7-((R)-2,2-dimethyltetrahydro-2H-pyran-4-yl)-2-((S)-2-(4-fluoro-3,5-dimethylphenyl)-4-methyl-3-(1-(1-methyl-1H-indazol-5-yl)-6-oxo-1,6-dihydropyrimidin-5-yl)-4,5,6,7-tetrahydro-2H-pyrazolo[4,3-c]pyridine-5-carbonyl)indolizin-3-yl)cyclopropyl)-1,2,4-oxadiazol-5(4H)-one **53-2** |
| 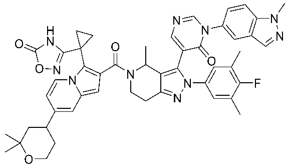 | | |

Another aspect of the present invention relates to a compound represented by general formula (IA) or a salt thereof: 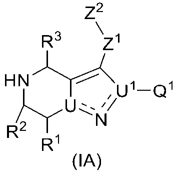 wherein -̅ -̅ -̅, 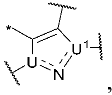 Q¹, Z¹, Z², R¹, R², and R³ are as defined for general formula (I).

Another aspect of the present invention relates to a compound represented by general formula (IMA), (IM-1A), or (IM-2A) or a salt thereof: 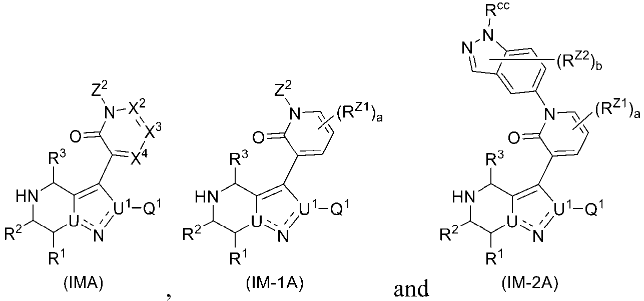 wherein 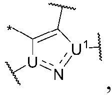 Q¹, Z², X², X³, X⁴, R^{Z1}, a, R¹, R², R³, R^{Z2}, b, and R^{cc} are as defined for general formula (IM), (IM-1), or (IM-2).

Another aspect of the present invention relates to a compound represented by general formula (INA) or a salt thereof: 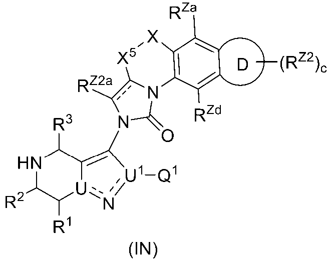 wherein 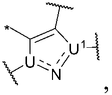 Q¹, R¹, R², R³, R^{Z2a}, -̅ -̅ -̅, X, X⁵, R^{Za}, R^{Zd} ring D, R^{Z2}, and c are as defined for general formula (IN).

Another aspect of the present invention relates to a compound represented by general formula (IN-1A) or a salt thereof: 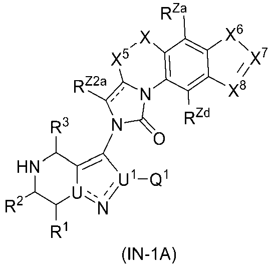 wherein 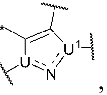 Q¹, R¹, R², R³, R^{Z2a}, -̅ -̅ -̅, X, X⁵, R^{Za}, R^{Zd}, X⁶, X⁷, and X⁸ are as defined for general formula (IN-1).

Another aspect of the present invention relates to a compound represented by general formula (IIA) or (IVA) or a salt thereof, 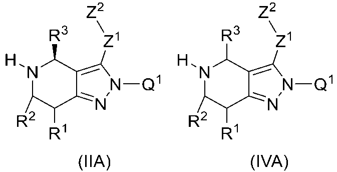 wherein
Q¹, Z¹, Z², R¹, R², and R³ are as defined for general formula (II).

Another aspect of the present invention relates to a compound represented by general formula (IIIA) or a salt thereof: 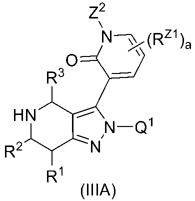 wherein
Q¹, Z², R^{Z1}, a, R¹, R², and R³ are as defined for general formula (III).

Another aspect of the present invention relates to a compound represented by general formula (IVB) or (IV'B) or a salt thereof, 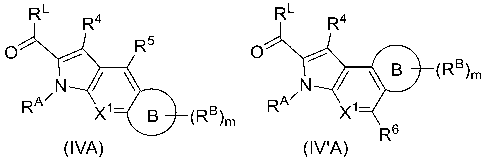
wherein R^{L} is selected from the group consisting of halogen, hydroxyl, and alkoxy; and in some embodiments, is hydroxyl;
ring B, X¹, R^{A}, R⁴, R⁵, R⁶, R^{B}, and m are as defined for general formula (IV) or (IV').

Another aspect of the present invention relates to a compound represented by general formula (IPA) or a salt thereof: 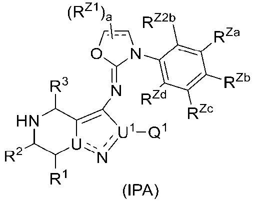 wherein 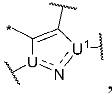 Q¹, R¹, R², R³, R^{Z1}, a, -̅ -̅ -̅, R^{Z2b}, R^{Za}, R^{Zb}, R^{Zc}, and R^{Zd} are as defined for general formula (IP).

Another aspect of the present invention relates to a compound represented by general formula (IP-1A) or a salt thereof: 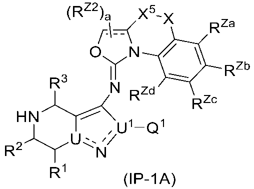 wherein 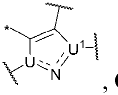 Q¹, R¹, R², R³, R^{Z2}, a, -̅ -̅ -̅, X, X⁵, R^{Za}, R^{Zb}, R^{Zc}, and R^{Zd} are as defined for general formula (IP-1).

Another aspect of the present invention relates to a compound represented by general formula (IOA) or a salt thereof: 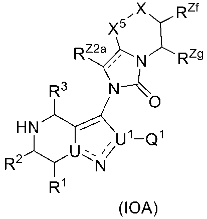 wherein 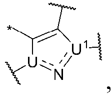 Q¹, R¹, R², R³, R^{Z2a}, -̅ -̅ -̅, X, X⁵, R^{Zf}, and R^{Zg} are as defined for general formula (IO).

Another aspect of the present invention relates to a compound represented by general formula (IIA-1) or a salt thereof: 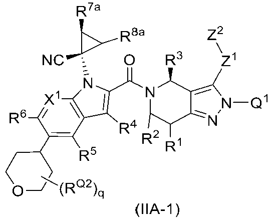 wherein X¹, Q¹, Z¹, Z², R¹ to R⁶, R^{7a}, R^{8a}, R^{Q2} and q are as defined for general formula (II).

**Table B. Typical compounds of the present invention, including but not limited to:**

| Compound No. | Compound structure | Name |
|---|---|---|
| **1v** | 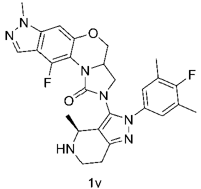 | 10-fluoro-2-((*S*)-2-(4-fluoro-3,5-dimethylphenyl)-4-methyl-4,5,6,7-tetrahydro-2*H*-pyrazolo[4,3-*c*]pyridin-3-yl)-7-methyl-2,3,3*a*,4-tetrahydro-1*H*,7*H*-imidazo[1',5':4,5][1,4]oxazino[3,2-*f*]indazol-1-one **1v** |
| 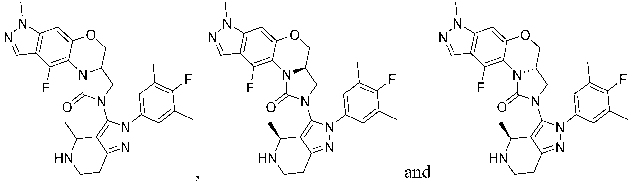 | | |
| **2c** | 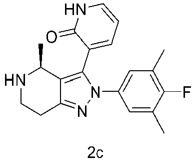 | (*S*)-3-(2-(4-fluoro-3,5-dimethylphenyl)-4-methyl-4,5,6,7-tetrahydro-2*H*-pyrazolo[4,3-*c*]pyridin-3-yl)pyridin-2(1*H*)-one **2c** |
| 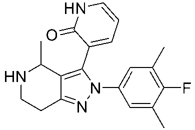 | | |
| **3b** | 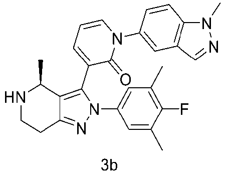 | (*S*)-3-(2-(4-fluoro-3,5-dimethylphenyl)-4-methyl-4,5,6,7-tetrahydro-2*H*-pyrazolo[4,3-*c*]pyridin-3-yl)-1-(1-methyl-1*H*-indazol-5-yl)pyridin-2(1*H*)-one **3b** |
| 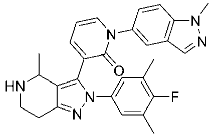 | | |
| **4p** | 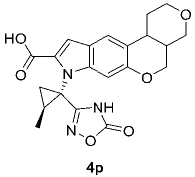 | 8-((1*S*,2*S*)-2-methyl-1-(5-oxo-4,5-dihydro-1,2,4-oxadiazol-3-yl)cyclopropyl)-1,2,4*a*,5,8,11*b-*hexahydro-4*H*-pyrano[4',3':4,5]pyrano[3,2-*f*]indole-9-carboxylic acid **4p** |
| 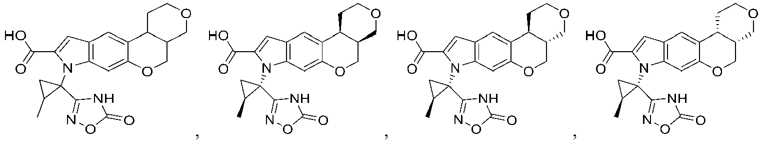 and | | |
| 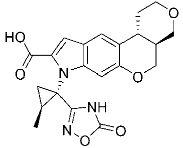 | | |
| **5b** | 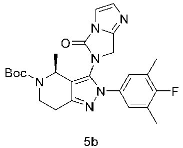 | Tert-butyl (*S*)-2-(4-fluoro-3,5-dimethylphenyl)-4-methyl-3-(5-oxo-5*H*-imidazo[1,5-*a*]imidazol-6(7*H*)-yl)-2,4,6,7-tetrahydro-5*H*-pyrazolo[4,3-*c*]pyridine-5-carboxylate **5b** |
| 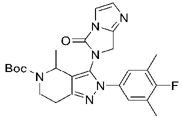 | | |
| **5c** | 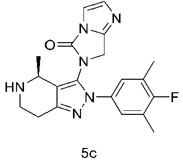 | (*S*)-6-(2-(4-fluoro-3,5-dimethylphenyl)-4-methyl-4,5,6,7-tetrahydro-2*H*-pyrazolo[4,3-*c*]pyridin-3-yl)-6,7-dihydro-5*H*-imidazo[1,5-*a*]imidazol-5-one **5c** |
| 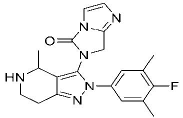 | | |
| **6u** | 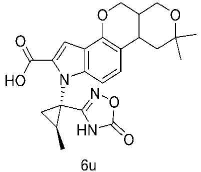 | 8,8-Dimethyl-1-((1*S*,2*S*)-2-methyl-1-(5-oxo-4,5-dihydro-1,2,4-oxadiazol-3-yl)cyclopropyl)-1,5*a*,6,8,9,9*a-*hexahydro-5*H-*pyrano[4',3':4,5]pyrano[2,3-*e*]indole-2-carboxylic acid **6u** |
| 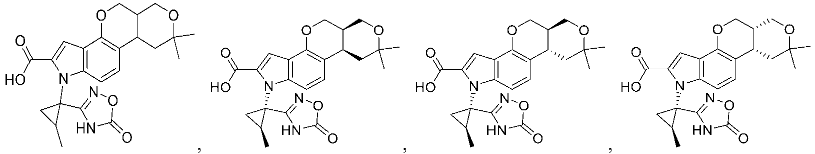 | | |
| and 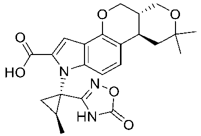 | | |
| **7m** | 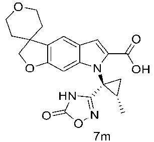 | 7-((1*S*,2*S*)-2-methyl-1-(5-oxo-4,5-dihydro-1,2,4-oxadiazol-3-yl)cyclopropyl)-2',3',5',6'-tetrahydro-2*H*,7*H*-spiro[furo[3,2-*f*]indole-3,4'-pyran]-6-carboxylic acid **7m** |
| 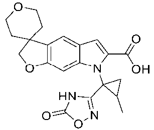 | | |
| **8h-1** | 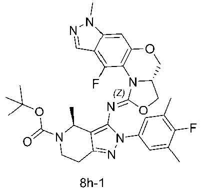 | Tert-butyl (*S*)-2-(4-fluoro-3,5-dimethylphenyl)-3-(((*R,Z*)-10-fluoro-7-methyl-3*a*,4-dihydro-1*H*,3*H*,7*H*-oxazolo[3 ',4':4,5][1,4]oxazino[3,2-*f*]indazol-1-ylidene)amino)-4-methyl-2,4,6,7-tetrahydro-5*H*-pyrazolo[4,3-*c*]pyridine-5-carboxylate **8h-1** |
| **8h-2** | 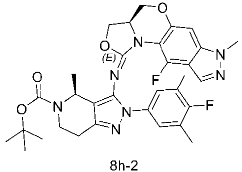 | Tert-butyl (*S*)-2-(4-fluoro-3,5-dimethylphenyl)-3-(((*R,E*)-10-fluoro-7-methyl-3*a*,4-dihydro-1*H*,3*H*,7*H*-oxazolo[3 ',4':4,5][1,4]oxazino[3,2-*f*]indazol-1-ylidene)amino)-4-methyl-2,4,6,7-tetrahydro-5*H*-pyrazolo[4,3-*c*]pyridine-5-carboxylate **8h-2** |
| **8i-1** | 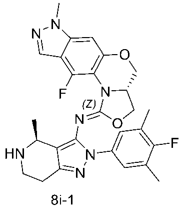 | (*R*,*Z*)-10-fluoro-*N*-((*S*)-2-(4-fluoro-3,5-dimethylphenyl)-4-methyl-4,5,6,7-tetrahydro-2*H-*pyrazolo[4,3-*c*]pyridin-3-yl)-7-methyl-3*a*,4-dihydro-1*H*,3*H*,7*H-*oxazolo[3',4':4,5][1,4]oxazino[3,2-*f*]indazol-1-imine **8i-1** |
| **8i-2** | 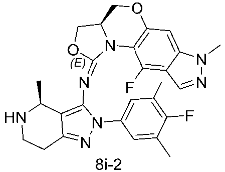 | (*R,E*)-10-fluoro-*N*-((*S*)-2-(4-fluoro-3,5-dimethylphenyl)-4-methyl-4,5,6,7-tetrahydro-2*H-*pyrazolo[4,3-*c*]pyridin-3-yl)-7-methyl-3*a*,4-dihydro-1*H*,3*H*,7*H-*oxazolo[3',4':4,5][1,4]oxazino[3,2-*f*]indazol-1-imine **8i-2** |
| 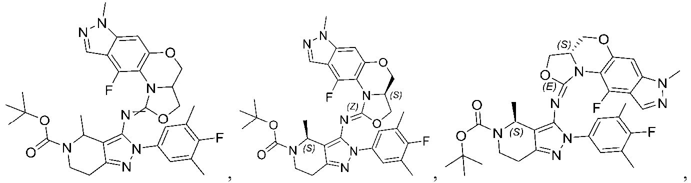 | | |
| 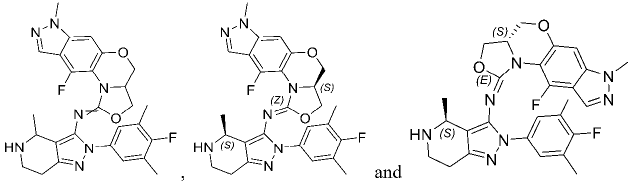 | | |
| **9d** | 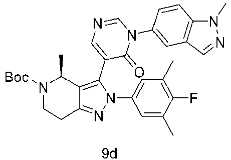 | Tert-butyl (*S*)-2-(4-fluoro-3,5-dimethylphenyl)-4-methyl-3-(1-(1-methyl-1*H*-indazol-5-yl)-6-oxo-1,6-dihydropyrimidin-5-yl)-2,4,6,7-tetrahydro-5*H-*pyrazolo[4,3-*c*]pyridine-5-carboxylate **9d** |
| **9e** | 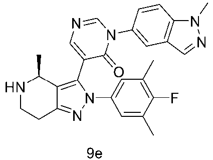 | (*S*)-5-(2-(4-fluoro-3,5-dimethylphenyl)-4-methyl-4,5,6,7-tetrahydro-2*H*-pyrazolo[4,3-*c*]pyridin-3-yl)-3-(1-methyl-1*H*-indazol-5-yl)pyrimidin-4(3*H*)-one **9e** |
| 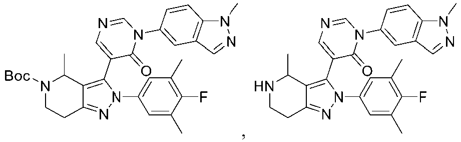 | | |
| **10d** | 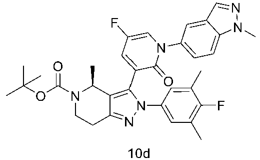 | Tert-butyl (*S*)-3-(5-fluoro-1-(1-methyl-1*H-*indazol-5-yl)-2-oxo-1,2-dihydropyridin-3-yl)-2-(4-fluoro-3,5-dimethylphenyl)-4-methyl-2,4,6,7-tetrahydro-5*H*-pyrazolo[4,3-*c*]pyridine-5-carboxylate **10d** |
| **10e** | 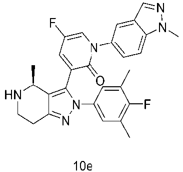 | (*S*)-5-fluoro-3-(2-(4-fluoro-3,5-dimethylphenyl)-4-methyl-4,5,6,7-tetrahydro-2*H*-pyrazolo[4,3-*c*]pyridin-3-yl)-1-(1-methyl-1*H*-indazol-5-yl)pyridin-2(1*H*)-one **10e** |
| 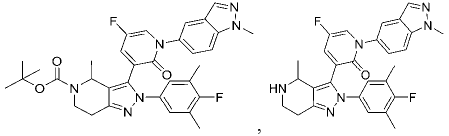 | | |
| **11h** | 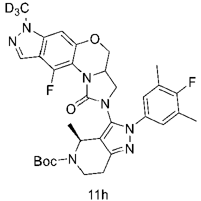 | Tert-butyl (4*S*)-2-(4-fluoro-3,5-dimethylphenyl)-3-(10-fluoro-7-(methyl-*d*₃)-1-oxo-3*a*,4-dihydro-1*H*,7*H*-imidazo[1',5':4,5][1,4]oxazino[3,2-*f*]indazol-2(3*H*)-yl)-4-methyl-2,4,6,7-tetrahydro-5*H*-pyrazolo[4,3-*c*]pyridine-5-carboxylate **11h** |
| **11i** | 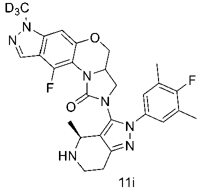 | 10-Fluoro-2-((*S*)-2-(4-fluoro-3,5-dimethylphenyl)-4-methyl-4,5,6,7-tetrahydro-2*H*-pyrazolo[4,3-*c*]pyridin-3-yl)-7-(methyl-*d*₃)-2,3,3*a*,4-tetrahydro-1*H*,7*H*-imidazo[1',5':4,5][1,4]oxazino[3,2-*f*]indazol-1-one **11i** |
| 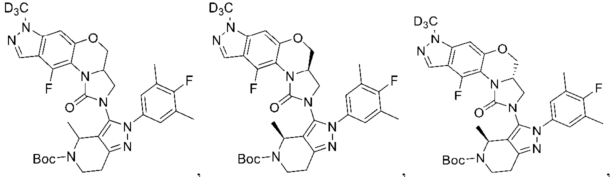 | | |
| 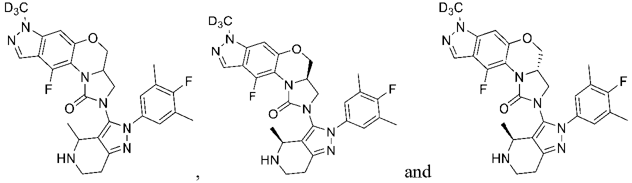 | | |
| **12g** | 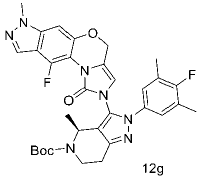 | Tert-butyl (*S*)-2-(4-fluoro-3,5-dimethylphenyl)-3-(10-fluoro-7-methyl-1-oxo-1*H*,7*H*-imidazo[1',5':4, 5][1,4]oxazino[3,2-*f*]indazol-2(4*H*)-yl)-4-methyl-2,4,6,7-tetrahydro-5*H*-pyrazolo[4,3-*c*]pyridine-5-carboxylate **12g** |
| 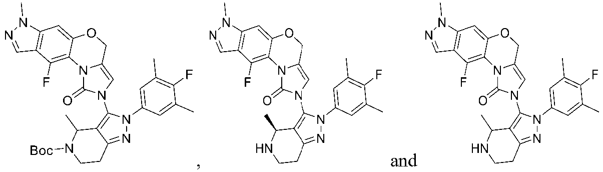 | | |
| **13q** | 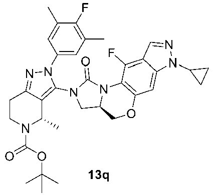 | Tert-butyl (*S*)-3-((*R*)-7-cyclopropyl-10-fluoro-1-oxo-3*a*,4-dihydro-1*H*,7*H-*imidazo[1',5':4,5][1,4]oxazino[3,2-*f*]indazol-2(3*H*)-yl)-2-(4-fluoro-3,5-dimethylphenyl)-4-methyl-2,4,6,7-tetrahydro-5*H*-pyrazolo[4,3-*c*]pyridine-5-carboxylate **13q** |
| **13r** | 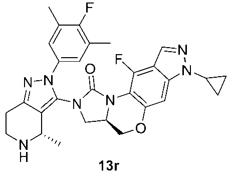 | (*R*)-7-cyclopropyl-10-fluoro-2-((*S*)-2-(4-fluoro-3,5-dimethylphenyl)-4-methyl-4,5,6,7-tetrahydro-2*H*-pyrazolo[4,3-*c*]pyridin-3-yl)-2,3,3*a*,4-tetrahydro-1*H*,7*H-*imidazo[1',5':4,5][1,4]oxazino[3,2-*f*]indazol-1-one **13r** |
| 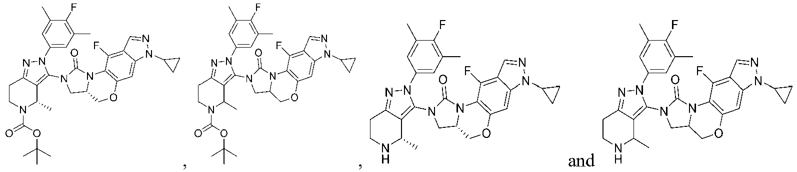 | | |
| **14f** | 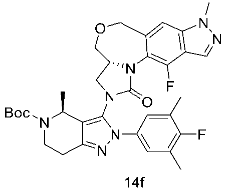 | Tert-butyl (*S*)-2-(4-fluoro-3,5-dimethylphenyl)-3-((*R*)-11-fluoro-8-methyl-1-oxo-3*a*,4,6,8-tetrahydro-1*H-*imidazo[5',1':3,4][1,4]oxazepino[5,6-*f*]indazol-2(3*H*)-yl)-4-methyl-2,4,6,7-tetrahydro-5*H-*pyrazolo[4,3-*c*]pyridine-5-carboxylate **14f** |
| **14g** | 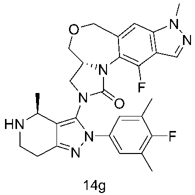 | (*R*)-11-fluoro-2-((*S*)-2-(4-fluoro-3,5-dimethylphenyl)-4-methyl-4,5,6,7-tetrahydro-2*H-*pyrazolo[4,3-*c*]pyridin-3-yl)-8-methyl-2,3,3a,4,6,8-hexahydro-1*H-*imidazo[5',1':3,4][1,4]oxazepino[5,6-*f*]indazol-1-one **14g** |
| 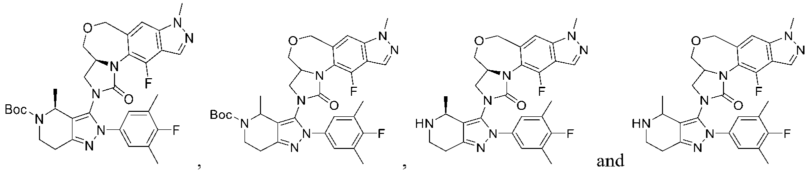 | | |
| **15i** | 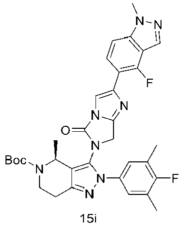 | Tert-butyl (*S*)-3-(2-(4-fluoro-1-methyl-1*H-*indazol-5-yl)-5-oxo-5*H*-imidazo[1,5-*a*]imidazol-6(7*H*)-yl)-2-(4-fluoro-3,5-dimethylphenyl)-4-methyl-2,4,6,7-tetrahydro-5*H*-pyrazolo[4,3-*c*]pyridine-5-carboxylate **15i** |
| **15j** | 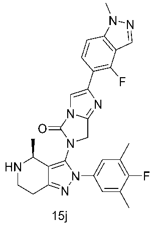 | (*S*)-2-(4-fluoro-1-methyl-1*H*-indazol-5-yl)-6-(2-(4-fluoro-3,5-dimethylphenyl)-4-methyl-4,5,6,7-tetrahydro-2*H*-pyrazolo[4,3-*c*]pyridin-3-yl)-6,7-dihydro-5*H*-imidazo[1,5-*a*]imidazol-5-one **15j** |
| 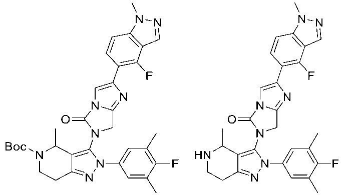 | | |
| **16e-1** | 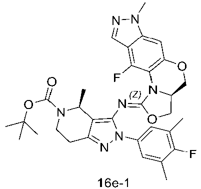 | Tert-butyl (*S*)-2-(4-fluoro-3,5-dimethylphenyl)-3-(((*S,Z*)-10-fluoro-7-methyl-3*a*,4-dihydro-1*H*,3*H*,7*H*-oxazolo[3',4':4,5][1,4]oxazino[3,2-*f*]indazol-1-ylidene)amino)-4-methyl-2,4,6,7-tetrahydro-5*H*-pyrazolo[4,3-*c*]pyridine-5-carboxylate **16e-1** |
| **16e-2** | 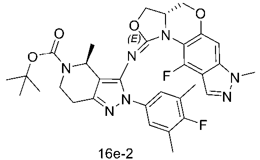 | Tert-butyl (*S*)-2-(4-fluoro-3,5-dimethylphenyl)-3-(((*S,E*)-10-fluoro-7-methyl-3*a*,4-dihydro-1*H*,3*H*,7*H*-oxazolo[3',4':4,5][1,4]oxazino[3,2-*f*]indazol-1-ylidene)amino)-4-methyl-2,4,6,7-tetrahydro-5*H*-pyrazolo[4,3-*c*]pyridine-5-carboxylate **16e-2** |
| **16f-1** | 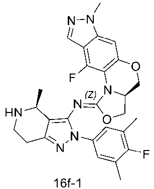 | (*S,Z*)-10-fluoro-*N*-((*S*)-2-(4-fluoro-3,5-dimethylphenyl)-4-methyl-4,5,6,7-tetrahydro-2*H-*pyrazolo[4,3-*c*]pyridin-3-yl)-7-methyl-3*a*,4-dihydro-1*H*,3*H*,7*H-*oxazolo[3',4':4,5][1,4]oxazino[3,2-*f*]indazol-1-imine **16f-1** |
| **16f-2** | 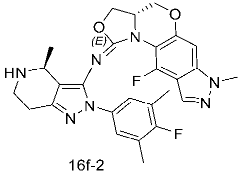 | (*S,E*)-10-fluoro-*N*-((*S*)-2-(4-fluoro-3,5-dimethylphenyl)-4-methyl-4,5,6,7-tetrahydro-2*H-*pyrazolo[4,3-*c*]pyridin-3-yl)-7-methyl-3*a*,4-dihydro-1*H*,3*H*,7*H-*oxazolo[3',4':4,5][1,4]oxazino[3,2-*f*]indazol-1-imine **16f-2** |
| 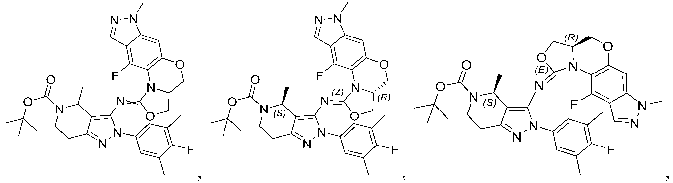 | | |
| 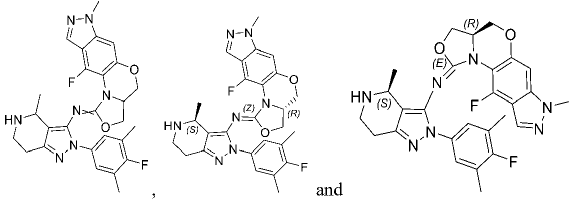 | | |
| **17c** | 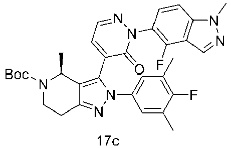 | Tert-butyl (*S*)-3-(2-(4-fluoro-1-methyl-1*H-*indazol-5-yl)-3-oxo-2,3-dihydropyridazin-4-yl)-2-(4-fluoro-3,5-dimethylphenyl)-4-methyl-2,4,6,7-tetrahydro-5*H*-pyrazolo[4,3-*c*]pyridine-5-carboxylate **17c** |
| **17d** | 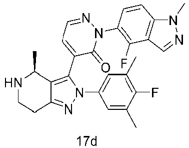 | (*S*)-2-(4-fluoro-1-methyl-1*H*-indazol-5-yl)-4-(2-(4-fluoro-3,5-dimethylphenyl)-4-methyl-4,5,6,7-tetrahydro-2*H*-pyrazolo[4,3-*c*]pyridin-3-yl)pyridazin-3(2*H*)-one **17d** |
| 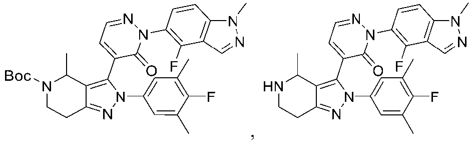 | | |
| **18b** | 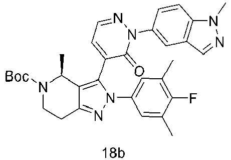 | Tert-butyl (*S*)-2-(4-fluoro-3,5-dimethylphenyl)-4-methyl-3-(2-(1-methyl-1*H*-indazol-5-yl)-3-oxo-2,3-dihydropyridazin-4-yl)-2,4,6,7-tetrahydro-5*H-*pyrazolo[4,3-*c*]pyridine-5-carboxylate **18b** |
| **18c** | 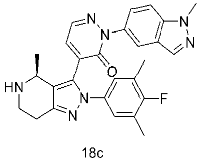 | (*S*)-4-(2-(4-fluoro-3,5-dimethylphenyl)-4-methyl-4,5,6,7-tetrahydro-2*H*-pyrazolo[4,3-*c*]pyridin-3-yl)-2-(1-methyl-1*H*-indazol-5-yl)pyridazin-3(2*H*)-one **18c** |
| 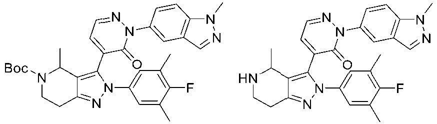 | | |
| **19b-1** | 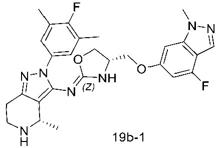 | (*S,Z*)-4-(((4-fluoro-1-methyl-1*H*-indazol-6-yl)oxy)methyl)-*N*-((*S*)-2-(4-fluoro-3,5-dimethylphenyl)-4-methyl-4,5,6,7-tetrahydro-2*H-*pyrazolo[4,3-*c*]pyridin-3-yl)oxazolidin-2-imine **19b-1** |
| **19b-2** | 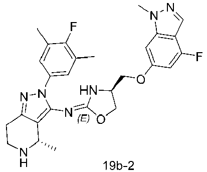 | (*S,E*)-4-(((4-fluoro-1-methyl- 1*H*-indazol-6-yl)oxy)methyl)-*N*-((*S*)-2-(4-fluoro-3,5-dimethylphenyl)-4-methyl-4,5,6,7-tetrahydro-2*H-*pyrazolo[4,3-*c*]pyridin-3-yl)oxazolidin-2-imine **19b-2** |
| 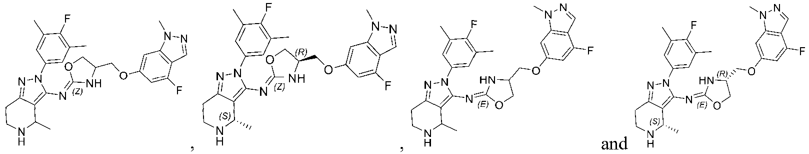 | | |
| **20c** | 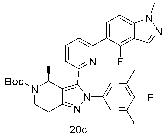 | Tert-butyl (*S*)-3-(6-(4-fluoro-1-methyl-1*H-*indazol-5-yl)pyridin-2-yl)-2-(4-fluoro-3,5-dimethylphenyl)-4-methyl-2,4,6,7-tetrahydro-5*H-*pyrazolo[4,3-*c*]pyridine-5-carboxylate **20c** |
| **20d** | 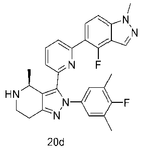 | (*S*)-3-(6-(4-fluoro-1-methyl-1*H*-indazol-5-yl)pyridin-2-yl)-2-(4-fluoro-3,5-dimethylphenyl)-4-methyl-4,5,6,7-tetrahydro-2*H*-pyrazolo[4,3-*c*]pyridine **20d** |
| 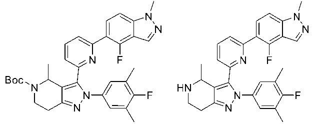 | | |
| **21o-1** | 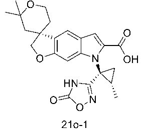 | (*R*)-2',2'-dimethyl-7-((1*S*,2*S*)-2-methyl-1-(5-oxo-4,5-dihydro-1,2,4-oxadiazol-3-yl)cyclopropyl)-2',3',5',6'-tetrahydro-2*H*,7*H*-spiro[furo[3,2-*f*]indole-3,4'-pyran]-6-carboxylic acid **21o-1** |
| **21o-2** | 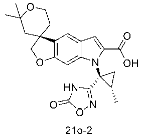 | (*S*)-2',2'-dimethyl-7-((1*S*,2*S*)-2-methyl-1-(5-oxo-4,5-dihydro-1,2,4-oxadiazol-3-yl)cyclopropyl)-2',3',5',6'-tetrahydro-2*H*,7*H*-spiro[furo[3,2-*f*]indole-3,4'-pyran]-6-carboxylic acid **21o-2** |
| 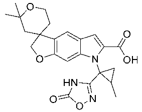 | | |
| **22c** | 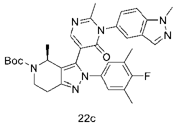 | Tert-butyl (*S*)-2-(4-fluoro-3,5-dimethylphenyl)-4-methyl-3-(2-methyl-1-(1-methyl-1*H*-indazol-5-yl)-6-oxo-1,6-dihydropyrimidin-5-yl)-2,4,6,7-tetrahydro-5*H*-pyrazolo[4,3-*c*]pyridine-5-carboxylate **22c** |
| **22d** | 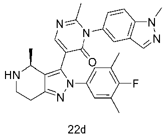 | (*S*)-5-(2-(4-fluoro-3,5-dimethylphenyl)-4-methyl-4,5,6,7-tetrahydro-2*H*-pyrazolo[4,3-*c*]pyridin-3-yl)-2-methyl-3-(1-methyl-1*H*-indazol-5-yl)pyrimidin-4(3*H*)-one **22d** |
| 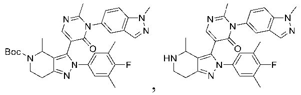 | | |
| **23b** | 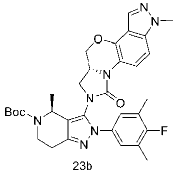 | Tert-butyl (*S*)-2-(4-fluoro-3,5-dimethylphenyl)-4-methyl-3-((*R*)-1-methyl-8-oxo-5*a*,6-dihydro-1*H*,5*H*-imidazo[1',5':4,5][1,4]oxazino[2,3-e]indazol-7(8*H*)-yl)-2,4,6,7-tetrahydro-5*H-*pyrazolo[4,3-*c*]pyridine-5-carboxylate **23b** |
| **23c** | 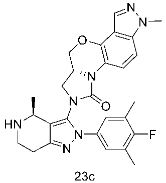 | (*R*)-7-((*S*)-2-(4-fluoro-3,5-dimethylphenyl)-4-methyl-4,5,6,7-tetrahydro-2*H*-pyrazolo[4,3-*c*]pyridin-3-yl)-1-methyl-5,5*a*,6,7-tetrahydro-1*H*,8*H*-imidazo[1',5':4,5][1,4]oxazino[2,3-*e*]indazol-8-one **23c** |
| 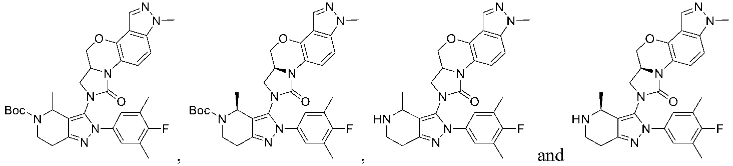 | | |
| **24c** | 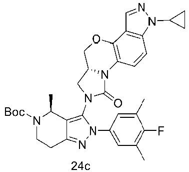 | Tert-butyl (*S*)-3-((*R*)-1-cyclopropyl-8-oxo-5*a*,6-dihydro-1*H*,5*H-*imidazo[1',5':4,5][1,4]oxazino[2,3-*e*]indazol-7(8*H*)-yl)-2-(4-fluoro-3,5-dimethylphenyl)-4-methyl-2,4,6,7-tetrahydro-5*H*-pyrazolo[4,3-*c*]pyridine-5-carboxylate **24c** |
| **24d** | 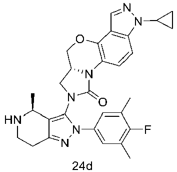 | (*R*)-1-cyclopropyl-7-((*S*)-2-(4-fluoro-3,5-dimethylphenyl)-4-methyl-4,5,6,7-tetrahydro-2*H-*pyrazolo[4,3-*c*]pyridin-3-yl)-5,5*a*,6,7-tetrahydro-1*H*,8*H*-imidazo[1',5':4,5][1,4]oxazino[2,3-*e*]indazol-8-one **24d** |
| 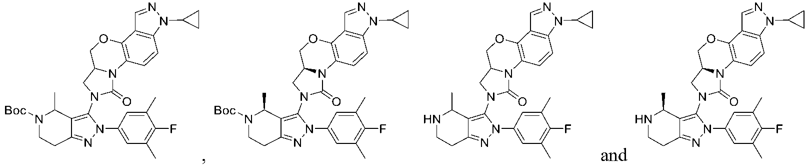 | | |
| **25j** | 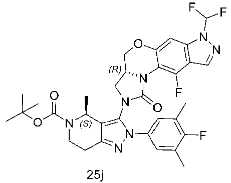 | Tert-butyl (*S*)-3-((*R*)-7-(difluoromethyl)-10-fluoro-1-oxo-3*a*,4-dihydro-1*H*,7*H-*imidazo[1',5':4,5][1,4]oxazino[3,2-*f*]indazol-2(3*H*)-yl)-2-(4-fluoro-3,5-dimethylphenyl)-4-methyl-2,4,6,7-tetrahydro-5*H*-pyrazolo[4,3-*c*]pyridine-5-carboxylate **25j** |
| **25k** | 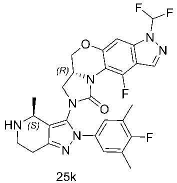 | (*R*)-7-(difluoromethyl)-10-fluoro-2-((*S*)-2-(4-fluoro-3,5-dimethylphenyl)-4-methyl-4,5,6,7-tetrahydro-2*H*-pyrazolo[4,3-*c*]pyridin-3-yl)-2,3,3*a*,4-tetrahydro-1*H*,7*H-*imidazo[1',5':4,5][1,4]oxazino[3,2-*f*]indazol-1-one **25k** |
| 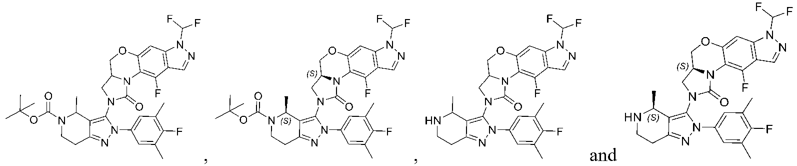 | | |
| **26b-1** | 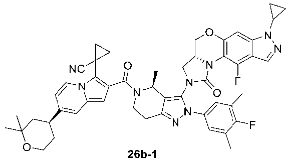 | 1-(2-((*S*)-3-((*R*)-7-cyclopropyl-10-fluoro-1-oxo-3*a*,4-dihydro-1*H*,7*H-*imidazo[1',5':4,5][1,4]oxazino[3,2-*f*]indazol-2(3*H*)-yl)-2-(4-fluoro-3,5-dimethylphenyl)-4-methyl-4,5,6,7-tetrahydro-2*H*-pyrazolo[4,3-*c*]pyridine-5-carbonyl)-7-((*S*)-2,2-dimethyltetrahydro-2*H*-pyran-4-yl)indolizin-3-yl)cyclopropane-1-carbonitrile **26b-1** |
| 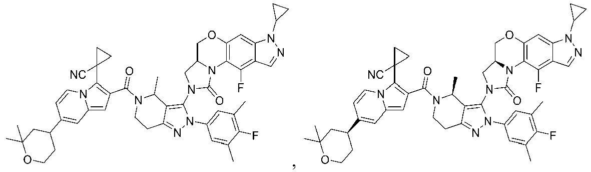 | | |
| **26b-2** | 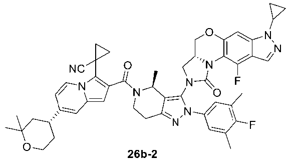 | 1-(2-((*S*)-3-((*R*)-7-cyclopropyl-10-fluoro-1-oxo-3*a*,4-dihydro-1*H*,7*H-*imidazo[1',5':4,5][1,4]oxazino[3,2-*f*]indazol-2(3*H*)-yl)-2-(4-fluoro-3,5-dimethylphenyl)-4-methyl-4,5,6,7-tetrahydro-2*H*-pyrazolo[4,3-*c*]pyridine-5-carbonyl)-7-((*R*)-2,2-dimethyltetrahydro-2*H*-pyran-4-yl)indolizin-3-yl)cyclopropane-1-carbonitrile **26b-2** |
| 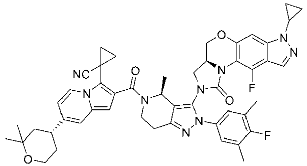 | | |
| **27c** | 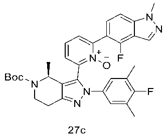 | (*S*)-2-(5-(tert-butoxycarbonyl)-2-(4-fluoro-3,5-dimethylphenyl)-4-methyl-4,5,6,7-tetrahydro-2*H-*pyrazolo[4,3-*c*]pyridin-3-yl)-6-(4-fluoro-1-methyl-1*H*-indazol-5-yl)pyridine 1-oxide **27c** |
| **27d** | 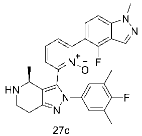 | (*S*)-2-(4-fluoro-1-methyl-1*H*-indazol-5-yl)-6-(2-(4-fluoro-3,5-dimethylphenyl)-4-methyl-4,5,6,7-tetrahydro-2*H*-pyrazolo[4,3-*c*]pyridin-3-yl)pyridine 1-oxide **27d** |
| 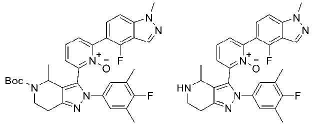 | | |
| **28e-1** | 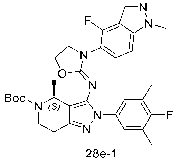 | Tert-butyl (*S,Z*)-3-((3-(4-fluoro-1-methyl-1*H-*indazol-5-yl)oxazolidin-2-ylidene)amino)-2-(4-fluoro-3,5-dimethylphenyl)-4-methyl-2,4,6,7-tetrahydro-5*H*-pyrazolo[4,3-*c*]pyridine-5-carboxylate **28e-1** |
| 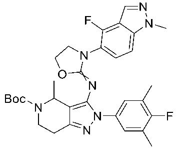 | | |
| **28e-2** | 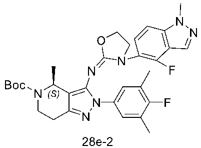 | Tert-butyl (*S,E*)-3-((3-(4-fluoro-1-methyl-1*H-*indazol-5-yl)oxazolidin-2-ylidene)amino)-2-(4-fluoro-3,5-dimethylphenyl)-4-methyl-2,4,6,7-tetrahydro-5*H*-pyrazolo[4,3-*c*]pyridine-5-carboxylate **28e-2** |
| **28f-1** | 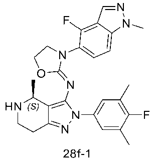 | (*S*,*Z*)-3-(4-fluoro-1-methyl-1*H*-indazol-5-yl)-N-(2-(4-fluoro-3,5-dimethylphenyl)-4-methyl-4,5,6,7-tetrahydro-2*H*-pyrazolo[4,3-*c*]pyridin-3-yl)oxazolidin-2-imine **28f-1** |
| 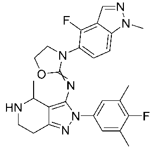 | | |
| **28f-2** | 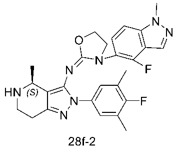 | (*S,E*)-3-(4-fluoro-1-methyl-1*H*-indazol-5-yl)-*N*-(2-(4-fluoro-3,5-dimethylphenyl)-4-methyl-4,5,6,7-tetrahydro-2*H*-pyrazolo[4,3-*c*]pyridin-3-yl)oxazolidin-2-imine **28f-2** |
| 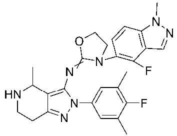 | | |
| **29d** | 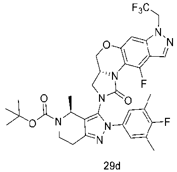 | Tert-butyl (*S*)-3-((*R*)-10-fluoro-1-oxo-7-(2,2,2-trifluoroethyl)-3*a*,4-dihydro-1*H*,7*H-*imidazo[1',5':4,5][1,4]oxazino[3,2-*f*]indazol-2(3*H*)-yl)-2-(4-fluoro-3,5-dimethylphenyl)-4-methyl-2,4,6,7-tetrahydro-5*H*-pyrazolo[4,3-*c*]pyridine-5-carboxylate **29d** |
| **29e** | 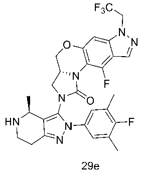 | (*R*)-10-fluoro-2-((*S*)-2-(4-fluoro-3,5-dimethylphenyl)-4-methyl-4,5,6,7-tetrahydro-2*H-*pyrazolo[4,3-*c*]pyridin-3-yl)-7-(2,2,2-trifluoroethyl)-2,3,3a,4-tetrahydro-1*H*,7*H-*imidazo[1',5"4,5][1,4]oxazino[3,2-*f*]indazol-1-one **29e** |
| 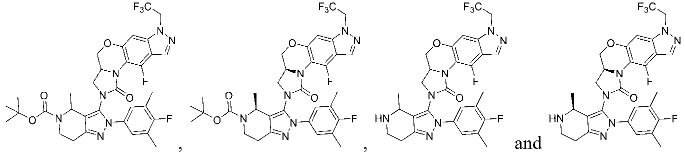 | | |
| **30h** | 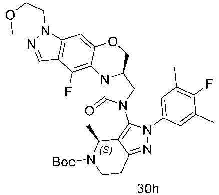 | Tert-butyl (*S*)-2-(4-fluoro-3,5-dimethylphenyl)-3-((*R*)-10-fluoro-7-(2-methoxyethyl)-1-oxo-3a,4-dihydro-1*H*,7*H-*imidazo[1',5':4,5][1,4]oxazino[3,2-*f*]indazol-2(3*H*)-yl)-4-methyl-2,4,6,7-tetrahydro-5*H-*pyrazolo[4,3-*c*]pyridine-5-carboxylate **30h** |
| | 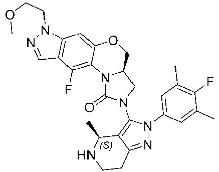 | |
| 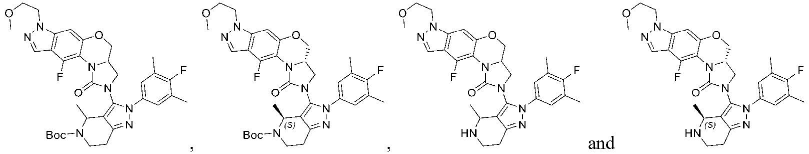 | | |
| **32h** | 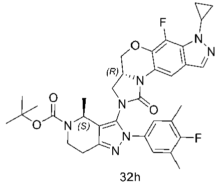 | Tert-butyl (*S*)-3-((*R*)-7-cyclopropyl-6-fluoro-1-oxo-3a,4-dihydro-1*H*,7*H-*imidazo[1',5':4,5][1,4]oxazino[3,2-*f*]indazol-2(3*H*)-yl)-2-(4-fluoro-3,5-dimethylphenyl)-4-methyl-2,4,6,7-tetrahydro-5*H*-pyrazolo[4,3-*c*]pyridine-5-carboxylate **32h** |
| **32i** | 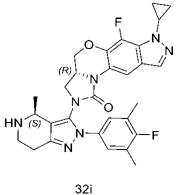 | (*R*)-7-cyclopropyl-6-fluoro-2-((*S*)-2-(4-fluoro-3,5-dimethylphenyl)-4-methyl-4,5,6,7-tetrahydro-2*H-*pyrazolo[4,3-*c*]pyridin-3-yl)-2,3,3*a*,4-tetrahydro-1*H*,7*H*-imidazo[1',5':4,5][1,4]oxazino[3,2-*f*]indazol-1-one **32i** |
| 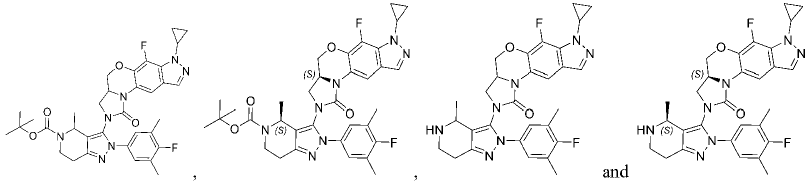 | | |
| **33e** | 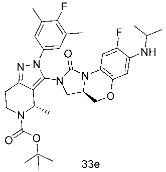 | Tert-butyl (*S*)-2-(4-fluoro-3,5-dimethylphenyl)-3-((*R*)-8-fluoro-7-(isopropylamino)-1-oxo-3*a*,4-dihydro-1*H*-benzo[*b*]imidazo[1,5-*d*][1,4]oxazin-2(3*H*)-yl)-4-methyl-2,4,6,7-tetrahydro-5*H-*pyrazolo[4,3-*c*]pyridine-5-carboxylate **33e** |
| **33f** | 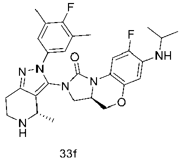 | (*R*)-8-fluoro-2-((*S*)-2-(4-fluoro-3,5-dimethylphenyl)-4-methyl-4,5,6,7-tetrahydro-2*H-*pyrazolo[4,3-*c*]pyridin-3-yl)-7-(isopropylamino)-2,3,3a,4-tetrahydro-1*H*-benzo[*b*]imidazo[1,5-*d*][1,4]oxazin-1-one **33f** |
| 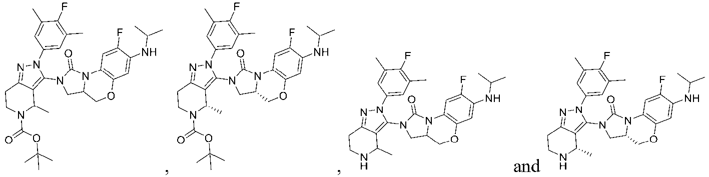 | | |
| **36h** | 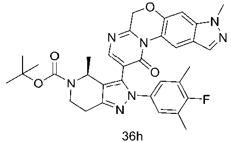 | Tert-butyl (*S*)-2-(4-fluoro-3,5-dimethylphenyl)-4-methyl-3-(8-methyl-1-oxo-1,5-dihydro-8*H-*pyrimido[1',2":4,5][1,4]oxazino[3,2-*f*]indazol-2-yl)-2,4,6,7-tetrahydro-5*H*-pyrazolo[4,3-*c*]pyridine-5-carboxylate **36h** |
| **36i** | 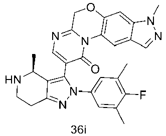 | (*S*)-2-(2-(4-fluoro-3,5-dimethylphenyl)-4-methyl-4,5,6,7-tetrahydro-2*H*-pyrazolo[4,3-*c*]pyridin-3-yl)-8-methyl-8*H-*pyrimido[1',2':4,5][1,4]oxazino[3,2-*f*]indazol-1(5*H*)-one **36i** |
| 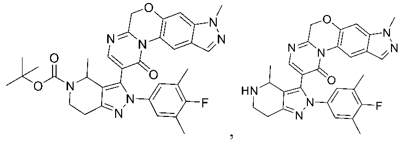 | | |
| **37g** | 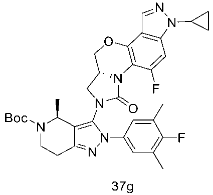 | Tert-butyl (*S*)-3-((*R*)-1-cyclopropyl-10-fluoro-8-oxo-5*a*,6-dihydro-1*H*,5*H-*imidazo[1',5':4,5][1,4]oxazino[2,3-*e*]indazol-7(8*H*)-yl)-2-(4-fluoro-3,5-dimethylphenyl)-4-methyl-2,4,6,7-tetrahydro-5*H*-pyrazolo[4,3-*c*]pyridine-5-carboxylate **37g** |
| **37h** | 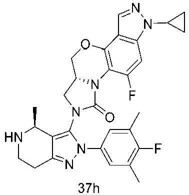 | (*R*)-1-cyclopropyl-10-fluoro-7-((*S*)-2-(4-fluoro-3,5-dimethylphenyl)-4-methyl-4,5,6,7-tetrahydro-2*H*-pyrazolo[4,3-*c*]pyridin-3-yl)-5,5*a*,6,7-tetrahydro-1*H*,8*H-*imidazo[1',5':4,5][1,4]oxazino[2,3-*e*]indazol-8-one **37h** |
| 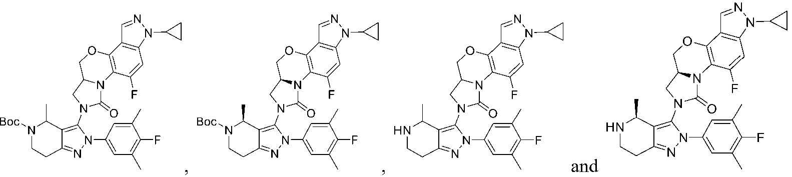 | | |
| **38e** | 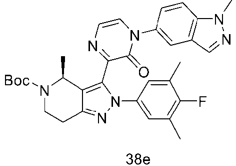 | Tert-butyl (*S*)-2-(4-fluoro-3,5-dimethylphenyl)-4-methyl-3-(4-(1-methyl-1H*-*indazol-5-yl)-3-oxo-3,4-dihydropyrazin-2-yl)-2,4,6,7-tetrahydro-5*H-*pyrazolo[4,3-*c*]pyridine-5-carboxylate **38e** |
| 38f | 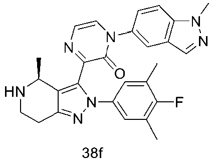 | (*S*)-3-(2-(4-fluoro-3,5-dimethylphenyl)-4-methyl-4,5,6,7-tetrahydro-2*H*-pyrazolo[4,3-*c*]pyridin-3-yl)-1-(1-methyl-1*H*-indazol-5-yl)pyrazin-2(1*H*)-one **38f** |
| 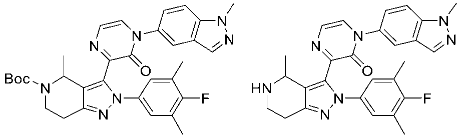 | | |
| **39g** | 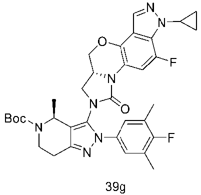 | Tert-butyl (*S*)-3-((R)-1-cyclopropyl-11-fluoro-8-oxo-5*a*,6-dihydro-1*H*,5*H-*imidazo[1',5':4,5][1,4]oxazino[2,3*-e*]indazol-7(8*H)*-yl)-2-(4-fluoro-3,5-dimethylphenyl)-4-methyl-2,4,6,7-tetrahydro-5*H*-pyrazolo[4,3-*c*]pyridine-5-carboxylate **39g** |
| **39h** | 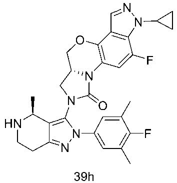 | (*R*)-1-cyclopropyl-11-fluoro-7-((*S*)-2-(4-fluoro-3,5-dimethylphenyl)-4-methyl-4,5,6,7-tetrahydro-2*H*-pyrazolo[4,3-*c*]pyridin-3-yl)-5,5*a*,6,7-tetrahydro-1*H*,8*H-*imidazo[1',5':4,5][1,4]oxazino[2,3-*e*]indazol-8-one **39h** |
| 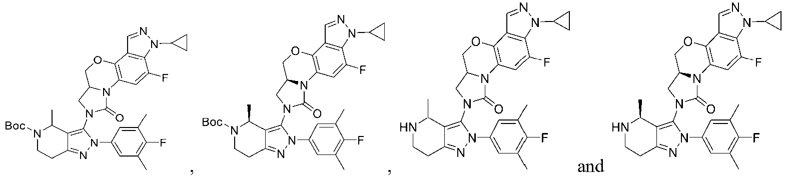 | | |
| **40d** | 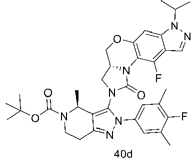 | Tert-butyl (*S*)-2-(4-fluoro-3,5-dimethylphenyl)-3-((*R*)-10-fluoro-7-isopropyl-1-oxo-3*a*,4-dihydro-1*H*,7*H*-imidazo[1',5':4,5][1,4]oxazino[3,2-*f*]indazol-2(3*H*)-yl)-4-methyl-2,4,6,7-tetrahydro-5*H*-pyrazolo[4,3-*c*]pyridine-5-carboxylate **40d** |
| **40e** | 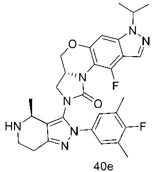 | (*R*)-10-fluoro-2-((*S*)-2-(4-fluoro-3,5-dimethylphenyl)-4-methyl-4,5,6,7-tetrahydro-2*H-*pyrazolo[4,3-*c*]pyridin-3-yl)-7-isopropyl-2,3,3a,4-tetrahydro-1*H*,7*H-*imidazo[1',5"4,5][1,4]oxazino[3,2-*f*]indazol-1-one **40e** |
| 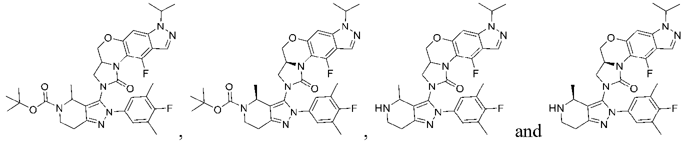 | | |
| **41e** | 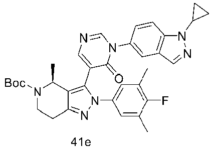 | Tert-butyl (*S*)-3-(1-(1-cyclopropyl-1*H*-indazol-5-yl)-6-oxo-1,6-dihydropyrimidin-5-yl)-2-(4-fluoro-3,5-dimethylphenyl)-4-methyl-2,4,6,7-tetrahydro-5*H*-pyrazolo[4,3-*c*]pyridine-5-carboxylate **41e** |
| **41f** | 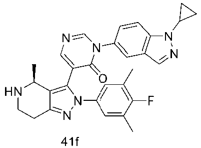 | (*S*)-3-(1-cyclopropyl-1*H*-indazol-5-yl)-5-(2-(4-fluoro-3,5-dimethylphenyl)-4-methyl-4,5,6,7-tetrahydro-2*H*-pyrazolo[4,3-*c*]pyridin-3-yl)pyrimidin-4(3*H*)-one **41f** |
| 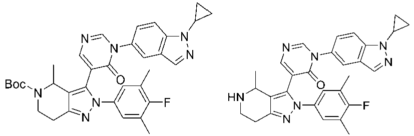 | | |
| **42d** | 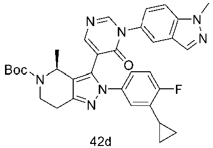 | Tert-butyl (*S*)-2-(3-cyclopropyl-4-fluorophenyl)-4-methyl-3-(1-(1-methyl-1*H*-indazol-5-yl)-6-oxo-1,6-dihydropyrimidin-5-yl)-2,4,6,7-tetrahydro-5*H-*pyrazolo[4,3-*c*]pyridine-5-carboxylate **42d** |
| **42e** | 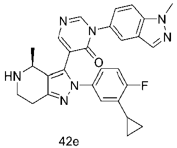 | (*S*)-5-(2-(3-cyclopropyl-4-fluorophenyl)-4-methyl-4,5,6,7-tetrahydro-2*H*-pyrazolo[4,3-*c*]pyridin-3-yl)-3-(1-methyl-1*H*-indazol-5-yl)pyrimidin-4(3*H*)-one **42e** |
| 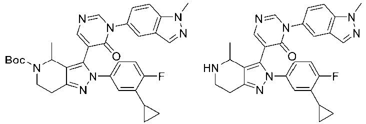 | | |
| **43e** | 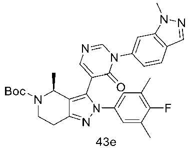 | Tert-butyl (*S*)-2-(4-fluoro-3,5-dimethylphenyl)-4-methyl-3-(1-(1-methyl-1*H*-indazol-6-yl)-6-oxo-1,6-dihydropyrimidin-5-yl)-2,4,6,7-tetrahydro-5*H-*pyrazolo[4,3-*c*]pyridine-5-carboxylate **43e** |
| **43f** | 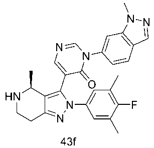 | (*S*)-5-(2-(4-fluoro-3,5-dimethylphenyl)-4-methyl-4,5,6,7-tetrahydro-2*H*-pyrazolo[4,3-*c*]pyridin-3-yl)-3-(1-methyl-1*H*-indazol-6-yl)pyrimidin-4(3*H*)-one **43f** |
| 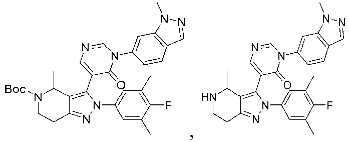 | | |
| **44d** | 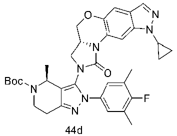 | Tert-butyl (*S*)-3-((R)-9-cyclopropyl-1-oxo-3a,4-dihydro-1*H*,9*H-*imidazo[1',5':4,5][1,4]oxazino[2,3-*f*]indazol-2(3*H*)-yl)-2-(4-fluoro-3,5-dimethylphenyl)-4-methyl-2,4,6,7-tetrahydro-5*H*-pyrazolo[4,3-*c*]pyridine-5-carboxylate **44d** |
| **44e** | 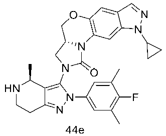 | (*R*)-9-cyclopropyl-2-((*S*)-2-(4-fluoro-3,5-dimethylphenyl)-4-methyl-4,5,6,7-tetrahydro-2*H-*pyrazolo[4,3-*c*]pyridin-3-yl)-2,3,3a,4-tetrahydro-1*H*,9*H*-imidazo[1',5':4,5][1,4]oxazino[2,3-*f*]indazol-1-one **44e** |
| 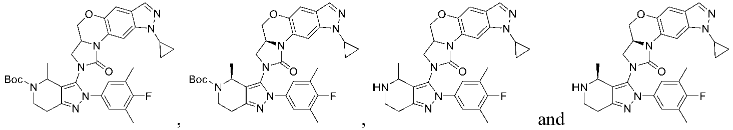 | | |
| **45e** | 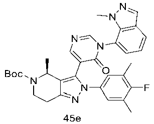 | Tert-butyl (*S*)-2-(4-fluoro-3,5-dimethylphenyl)-4-methyl-3-(1-(1-methyl-1*H*-indazol-7-yl)-6-oxo-1,6-dihydropyrimidin-5-yl)-2,4,6,7-tetrahydro-5*H-*pyrazolo[4,3-*c*]pyridine-5-carboxylate **45e** |
| **45f** | 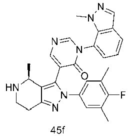 | (*S*)-5-(2-(4-fluoro-3,5-dimethylphenyl)-4-methyl-4*,*5,6,7-tetrahydro-2*H*-pyrazolo[4,3-*c*]pyridin-3-yl)-3-(1-methyl-1*H*-indazol-7-yl)pyrimidin-4(3*H*)-one **45f** |
| 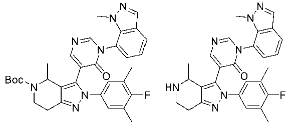 | | |
| **46f** | 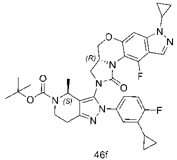 | Tert-butyl (*S*)-3-((*R*)-7-cyclopropyl-10-fluoro-1-oxo-3a,4-dihydro-1*H*,7*H-*imidazo[1',5':4,5][1,4]oxazino[3,2-*f*]indazol-2(3*H*)-yl)-2-(3-cyclopropyl-4-fluorophenyl)-4-methyl-2,4,6,7-tetrahydro-5*H*-pyrazolo[4,3-*c*]pyridine-5-carboxylate **46f** |
| **46g** | 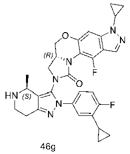 | (*R*)-7-cyclopropyl-2-((*S*)-2-(3-cyclopropyl-4-fluorophenyl)-4-methyl-4,5,6,7-tetrahydro-2*H-*pyrazolo[4,3-*c*]pyridin-3-yl)-10-fluoro-2,3,3a,4-tetrahydro-1*H*,7*H-*imidazo[1',5"4,5][1,4]oxazino[3,2-*f*]indazol-1-one **46g** |
| 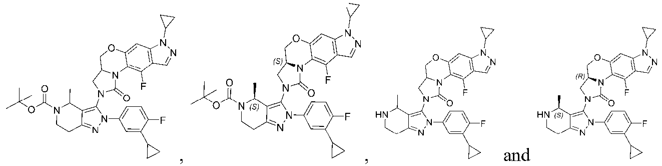 | | |
| **47c** | 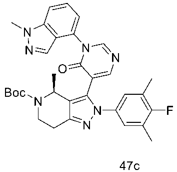 | Tert-butyl (*S*)-2-(4-fluoro-3,5-dimethylphenyl)-4-methyl-3-(1-(1-methyl-1*H*-indazol-4-yl)-6-oxo-1,6-dihydropyrimidin-5-yl)-2,4,6,7-tetrahydro-5*H-*pyrazolo[4,3-*c*]pyridine-5-carboxylate **47c** |
| **47d** | 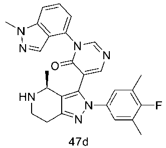 | (*S*)-5-(2-(4-fluoro-3,5-dimethylphenyl)-4-methyl-4,5,6,7-tetrahydro-2*H*-pyrazolo[4,3-*c*]pyridin-3-yl)-3-(1-methyl-1*H*-indazol-4-yl)pyrimidin-4(3*H*)-one **47d** |
| 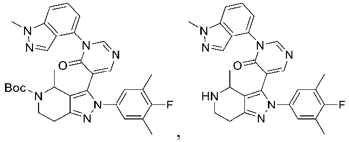 | | |
| **48e** | 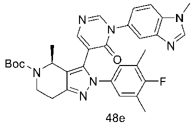 | Tert-butyl (*S*)-2-(4-fluoro-3,5-dimethylphenyl)-4-methyl-3-(1-(1-methyl-1*H*-benzo[*d*]imidazol-5-yl)-6-oxo-1,6-dihydropyrimidin-5-yl)-2,4,6,7-tetrahydro-5*H*-pyrazolo[4,3-*c*]pyridine-5-carboxylate **48e** |
| **48f** | 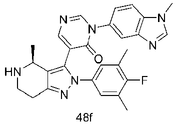 | (*S*)-5-(2-(4-fluoro-3,5-dimethylphenyl)-4-methyl-4,5,6,7-tetrahydro-2*H*-pyrazolo[4,3-*c*]pyridin-3-yl)-3-(1-methyl-1*H*-benzo[*d*]imidazol-5-yl)pyrimidin-4(3*H*)-one **48f** |
| 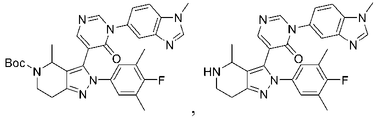 | | |
| **49c** | 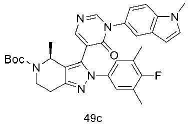 | Tert-butyl (*S*)-2-(4-fluoro-3,5-dimethylphenyl)-4-methyl-3-(1-(1-methyl-1*H*-indol-5-yl)-6-oxo-1,6-dihydropyrimidin-5-yl)-2,4,6,7-tetrahydro-5*H-*pyrazolo[4,3-*c*]pyridine-5-carboxylate **49c** |
| **49d** | 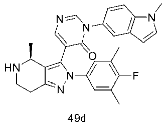 | (*S*)-5-(2-(4-fluoro-3,5-dimethylphenyl)-4-methyl-4,5,6,7-tetrahydro-2*H*-pyrazolo[4,3-*c*]pyridin-3-yl)- 3-(1-methyl-1*H*-indol-5-yl)pyrimidin-4(3*H*)-one **49d** |
| 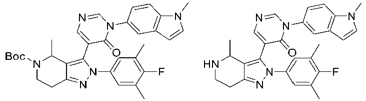 | | |
| **50e** | 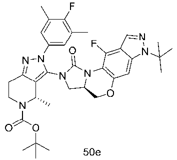 | Tert-butyl (*S*)-3-((*R*)-7-(tert-butyl)-10-fluoro-1-oxo-3a,4-dihydro-1*H*,7*H-*imidazo[1',5':4,5][1,4]oxazino[3,2-*f*]indazol-2(3*H*)-yl)-2-(4-fluoro-3,5-dimethylphenyl)-4-methyl-2,4,6,7-tetrahydro-5*H*-pyrazolo[4,3-*c*]pyridine-5-carboxylate **50e** |
| **50f** | 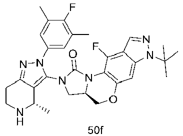 | (*R*)-7-(tert-butyl)-10-fluoro-2-((*S*)-2-(4-fluoro-3,5-dimethylphenyl)-4-methyl-4,5,6,7-tetrahydro-2*H-*pyrazolo[4,3-*c*]pyridin-3-yl)-2,3,3*a*,4-tetrahydro-1*H*,7*H*-imidazo[1',5':4,5][1,4]oxazino[3,2-*f*]indazol-1-one **50f** |
| 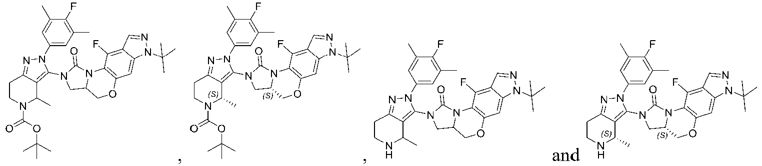 | | |
| **51d** | 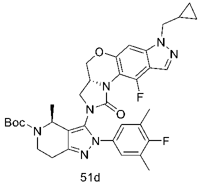 | Tert-butyl (S)-3-((R)-7-(cyclopropylmethyl)-10-fluoro-1-oxo-3a,4-dihydro-1H,7H-imidazo[1',5':4,5][1,4]oxazino[3,2-f]indazol-2(3H)-yl)-2-(4-fluoro-3,5-dimethylphenyl)-4-methyl-2,4,6,7-tetrahydro-5H-pyrazolo[4,3-c]pyridine-5-carboxylate **51d** |
| **51e** | 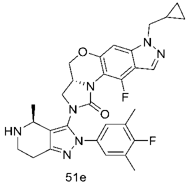 | (*R*)-7-(cyclopropylmethyl)-10-fluoro-2-((*S*)-2-(4-fluoro-3,5-dimethylphenyl)-4-methyl-4,5,6,7-tetrahydro-2*H*-pyrazolo[4,3-c]pyridin-3-yl)-2,3,3*a*,4-tetrahydro-1*H*,7*H-*imidazo[1',5':4,5][1,4]oxazino[3,2-*f*]indazol-1-one **51e** |
| 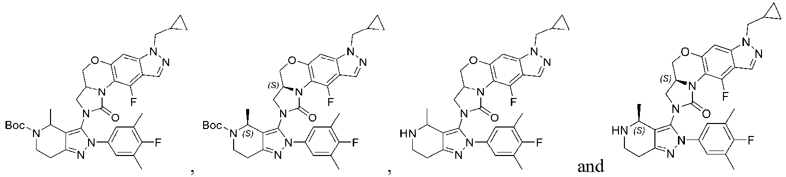 | | |
| **52f** | 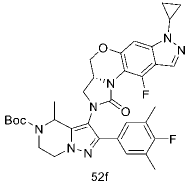 | Tert-butyl 3-((*R*)-7-cyclopropyl-10-fluoro-1-oxo-3a,4-dihydro-1*H*,7*H-*imidazo[1',5':4,5][1,4]oxazino[3,2-*f*]indazol-2(3*H*)-yl)-2-(4-fluoro-3,5-dimethylphenyl)-4-methyl-6,7-dihydropyrazolo[1,5-*a*]pyrazine-5(4*H*)-carboxylate **52f** |
| **52g** | 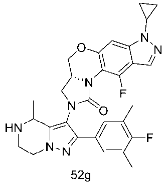 | (3*aR*)-7-cyclopropyl-10-fluoro-2-(2-(4-fluoro-3,5-dimethylphenyl)-4-methyl-4,5,6,7-tetrahydropyrazolo[1,5-*a*]pyrazin-3-yl)-2,3,3a,4-tetrahydro-1*H*,7*H-*imidazo[1',5"4,5][1,4]oxazino[3,2-*f*]indazol-1-one **52g** |
| 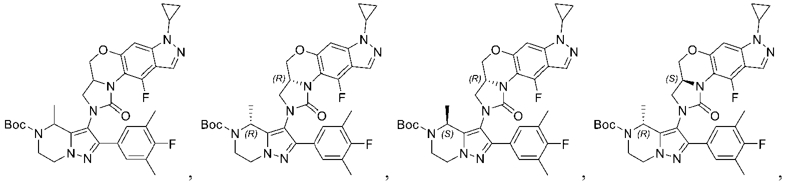 | | |
| 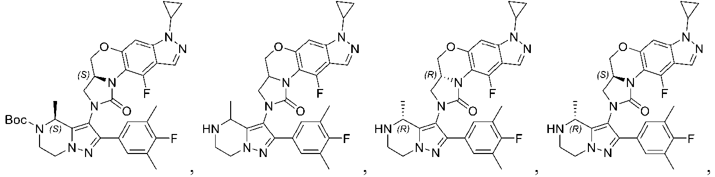 | | |
| 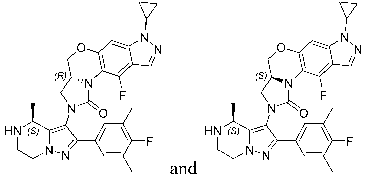 | | |
| **53a-1** | 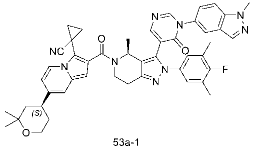 | 1-(7-((S)-2,2-dimethyltetrahydro-2H-pyran-4-yl)-2-((S)-2-(4-fluoro-3,5-dimethylphenyl)-4-methyl-3-(1-(1-methyl-1H-indazol-5-yl)-6-oxo-1,6-dihydropyrimidin-5-yl)-4,5,6,7-tetrahydro-2H-pyrazolo[4,3-c]pyridine-5-carbonyl)indolizin-3-yl)cyclopropane-1-carbonitrile **53a-1** |
| **53a-2** | 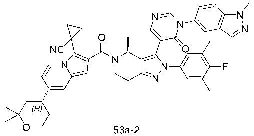 | 1-(7-((R)-2,2-dimethyltetrahydro-2H-pyran-4-yl)-2-((S)-2-(4-fluoro-3,5-dimethylphenyl)-4-methyl-3-(1-(1-methyl-1H-indazol-5-yl)-6-oxo-1,6-dihydropyrimidin-5-yl)-4,5,6,7-tetrahydro-2H-pyrazolo[4,3-c]pyridine-5-carbonyl)indolizin-3-yl)cyclopropane-1-carbonitrile **53a-2** |
| 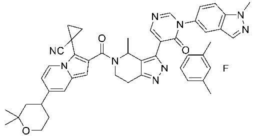 | | |

Another aspect of the present invention relates to a method for preparing a compound represented by general formula (I) or a pharmaceutically acceptable salt thereof, wherein the method comprises: 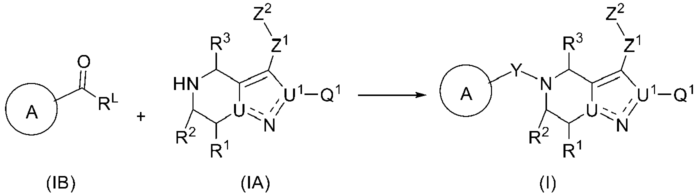
subjecting a compound represented by general formula (IA) or a salt thereof and a compound represented by general formula (IB) or a salt thereof to a condensation reaction to obtain the compound represented by general formula (I) or the pharmaceutically acceptable salt thereof,
wherein R^{L} is selected from the group consisting of halogen, hydroxyl, and alkoxy, and in some embodiments, is hydroxyl; Y is C(O); and
-̅ -̅ -̅, 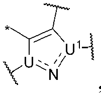 , ring A, Q¹, Z¹, Z², R¹, R², and R³ are as defined for general formula (I).

Another aspect of the present invention relates to a method for preparing a compound represented by general formula (IM) or a pharmaceutically acceptable salt thereof, wherein the method comprises: 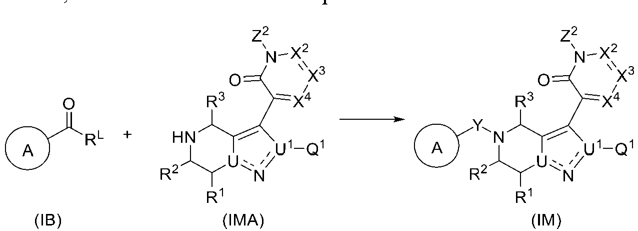
subjecting a compound represented by general formula (IMA) or a salt thereof and a compound represented by general formula (IB) or a salt thereof to a condensation reaction to obtain the compound represented by general formula (1M) or the pharmaceutically acceptable salt thereof,
wherein R^{L} is selected from the group consisting of halogen, hydroxyl, and alkoxy, and in some embodiments, is hydroxyl; Y is C(O); and
-̅ -̅ -̅, 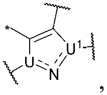 ring A, Q¹, X², X³, X⁴, Z², R¹, R², and R³ are as defined for general formula (IM).

Another aspect of the present invention relates to a method for preparing the compound represented by general formula (IM-1) or the pharmaceutically acceptable salt thereof, wherein the method comprises: 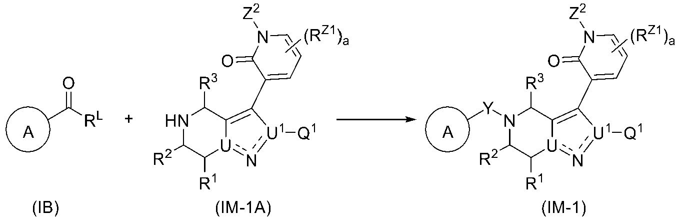
subjecting a compound represented by general formula (IM-1A) or a salt thereof and a compound represented by general formula (IB) or a salt thereof to a condensation reaction to obtain the compound represented by general formula (IM-1) or the pharmaceutically acceptable salt thereof,
wherein R^{L} is selected from the group consisting of halogen, hydroxyl, and alkoxy, and in some embodiments, is hydroxyl; Y is C(O); and
-̅ -̅ -̅, 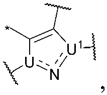 ring A, Q¹, Z², R^{Z1}, a, R¹, R², and R³ are as defined for general formula (IM-1).

Another aspect of the present invention relates to a method for preparing the compound represented by general formula (IM-2) or the pharmaceutically acceptable salt thereof, wherein the method comprises: 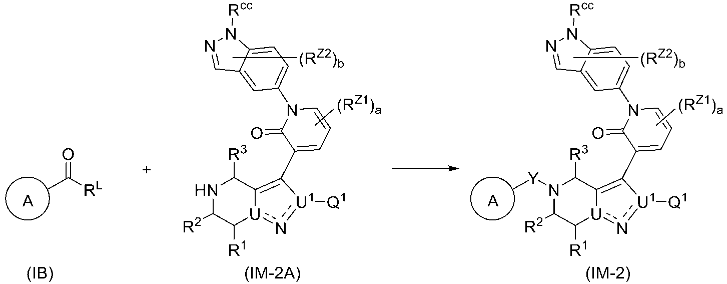
subjecting a compound represented by general formula (IM-2A) or a salt thereof and a compound represented by general formula (IB) or a salt thereof to a condensation reaction to obtain the compound represented by general formula (IM-2) or the pharmaceutically acceptable salt thereof,
wherein R^{L} is selected from the group consisting of halogen, hydroxyl, and alkoxy, and in some embodiments, is hydroxyl; Y is C(O);
-̅ -̅ -̅, 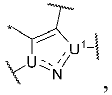 ring A, Q¹, R^{Z1}, a, R^{Z2}, b, R^{cc}, R¹, R², and R³ are as defined for general formula (IM-2).

Another aspect of the present invention relates to a method for preparing a compound represented by general formula (IN) or a pharmaceutically acceptable salt thereof, wherein the method comprises: 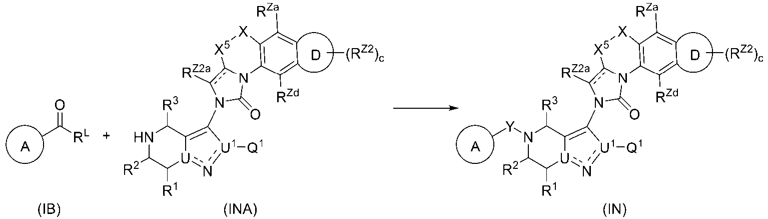
subjecting a compound represented by general formula (INA) or a salt thereof and a compound represented by general formula (IB) or a salt thereof to a condensation reaction to obtain the compound represented by general formula (IN) or the pharmaceutically acceptable salt thereof,
wherein R^{L} is selected from the group consisting of halogen, hydroxyl, and alkoxy, and in some embodiments, is hydroxyl; Y is C(O); 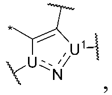
ring A, Q¹, R¹, R², R³, R^{Z2a}, -̅ -̅ -̅, X, X⁵, R^{Za}, R^{Zd}, ring D, R^{Z2}, and c are as defined for general formula (IN).

Another aspect of the present invention relates to a method for preparing the compound represented by general formula (IN-1) or the pharmaceutically acceptable salt thereof, wherein the method comprises: 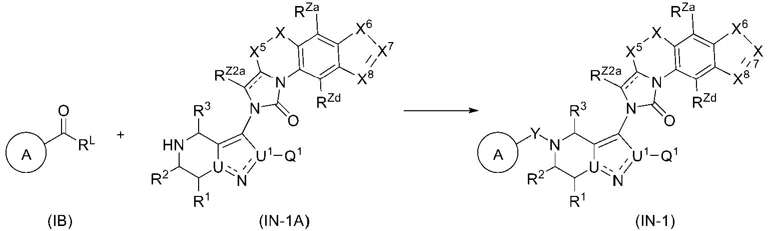
subjecting a compound represented by general formula (IN-1A) or a salt thereof and a compound represented by general formula (IB) or a salt thereof to a condensation reaction to obtain the compound represented by general formula (IN-1) or the pharmaceutically acceptable salt thereof,
wherein R^{L} is selected from the group consisting of halogen, hydroxyl, and alkoxy, and in some embodiments, is hydroxyl; Y is C(O); 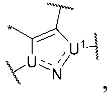 ring A, Q¹, R¹, R², R³ R^{Z2a} -̅ -̅ -̅, X, X⁵, R^{Za}, R^{Zd}, X⁶, X⁷ and X⁸ are as defined for general formula (IN-1).

Another aspect of the present invention relates to a method for preparing a compound represented by general formula (II) or a pharmaceutically acceptable salt thereof, wherein the method comprises: 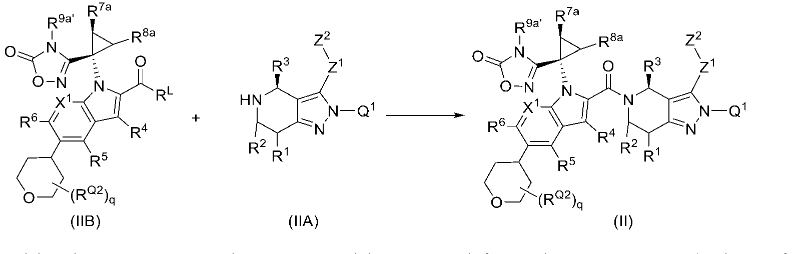
subjecting a compound represented by general formula (IIA) or a salt thereof and a compound represented by general formula (IIB) or a salt thereof to a condensation reaction to obtain the compound represented by general formula (II) or the pharmaceutically acceptable salt thereof,
wherein R^{L} is selected from the group consisting of halogen, hydroxyl, and alkoxy, and in some embodiments, is hydroxyl;
X¹, Q¹, Z¹, Z², R¹ to R⁶, R^{7a}, R^{8a}, R^{9a}', R^{Q2}, and q are as defined for general formula (II).

Another aspect of the present invention relates to a method for preparing a compound represented by general formula (III) or a pharmaceutically acceptable salt thereof, wherein the method comprises: 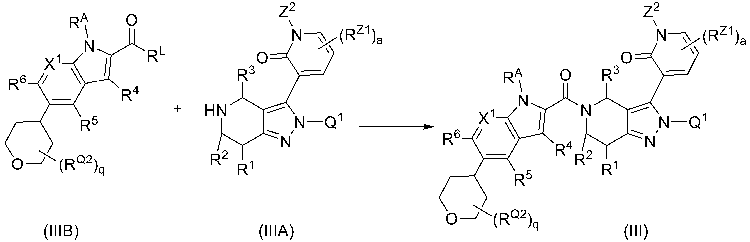
subjecting a compound represented by general formula (IIIA) or a salt thereof and a compound represented by general formula (IIIB) or a salt thereof to a condensation reaction to obtain the compound represented by general formula (III) or the pharmaceutically acceptable salt thereof,
wherein R^{L} is selected from the group consisting of halogen, hydroxyl, and alkoxy, and in some embodiments, is hydroxyl;
X¹, Q¹, Z², R^{Z1}, a, R¹ to R⁶, R^{A}, R^{Q2}, and q are as defined for general formula (III).

Another aspect of the present invention relates to a method for preparing a compound represented by general formula (IV) or (IV') or a pharmaceutically acceptable salt thereof, wherein the method comprises: 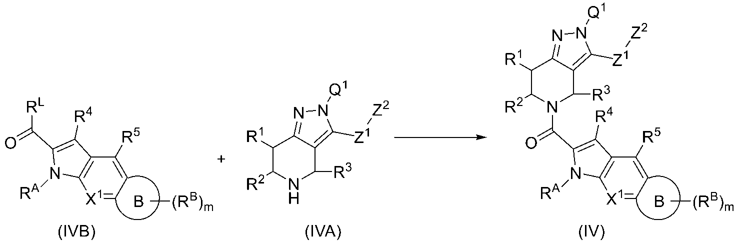
subjecting a compound represented by general formula (IVA) or a salt thereof and a compound represented by general formula (IVB) or a salt thereof to a condensation reaction to obtain the compound represented by general formula (IV) or the pharmaceutically acceptable salt thereof, 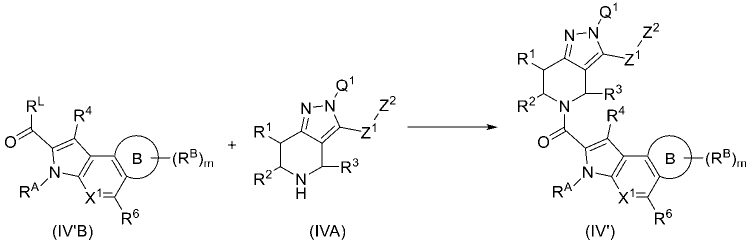
subjecting a compound represented by general formula (IVA) or a salt thereof and a compound represented by general formula (IV'B) or a salt thereof to a condensation reaction to obtain the compound represented by general formula (IV') or the pharmaceutically acceptable salt thereof,
wherein R^{L} is selected from the group consisting of halogen, hydroxyl, and alkoxy, and in some embodiments, is hydroxyl;
ring B, X¹, Q¹, Z¹, Z², R¹ to R⁴, R⁵, R⁶, R^{A}, R^{B}, and m are as defined for general formula (IV) or (IV').

Another aspect of the present invention relates to a method for preparing a compound represented by general formula (IO) or a pharmaceutically acceptable salt thereof, wherein the method comprises: 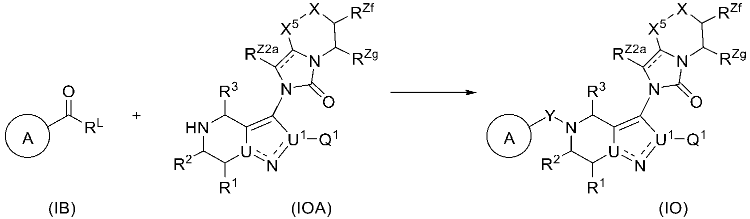
subjecting a compound represented by general formula (IOA) or a salt thereof and a compound represented by general formula (IB) or a salt thereof to a condensation reaction to obtain the compound represented by general formula (IO) or the pharmaceutically acceptable salt thereof,
wherein R^{L} is selected from the group consisting of halogen, hydroxyl, and alkoxy, and in some embodiments, is hydroxyl; Y is C(O); 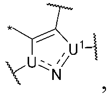 Q¹, ring A, R¹, R², R³, R^{Z2a}, -̅ -̅ -̅, X, X⁵ , R^{Zf}, and R^{Zg} are as defined for general formula (IO).

Another aspect of the present invention relates to a method for preparing a compound represented by general formula (II) or a pharmaceutically acceptable salt thereof, wherein the method comprises: 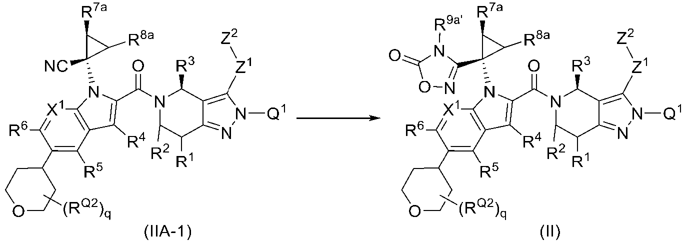
subjecting a compound represented by general formula (IIA-1) or a salt thereof and hydroxylamine or a salt thereof (in some embodiments, a hydrochloride thereof) to an addition reaction, followed by a further ring-closing reaction with *N,N'-*carbonyldiimidazole to obtain a compound represented by general formula (II) or a pharmaceutically acceptable salt thereof,
wherein R^{9a'} is a hydrogen atom; and
X¹, Q¹, Z¹, Z², R¹ to R⁶, R^{7a}, R^{8a}, R^{Q2}, and q are as defined for general formula (II).

Another aspect of the present invention relates to a method for preparing a compound represented by general formula (IP) or a pharmaceutically acceptable salt thereof, wherein the method comprises: 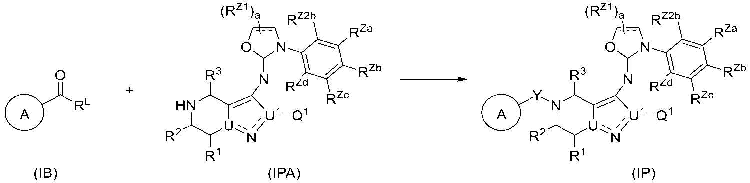
subjecting a compound represented by general formula (IB) or a salt thereof and a compound represented by general formula (IPA) or a salt thereof to a condensation reaction to obtain the compound represented by general formula (IP) or the pharmaceutically acceptable salt thereof,
wherein R^{L} is selected from the group consisting of halogen, hydroxyl, and alkoxy, and in some embodiments, is hydroxyl; Y is C(O);
ring A, 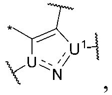 Q¹, R¹, R², R³, R^{Z1}, a, -̅ -̅ -̅, R^{Z2b}, R^{Za}, R^{Zb}, R^{Zc}, and R^{Zd} are as defined for general formula (IP).

Another aspect of the present invention relates to a method for preparing the compound represented by general formula (IP-1) or the pharmaceutically acceptable salt thereof, wherein the method comprises: 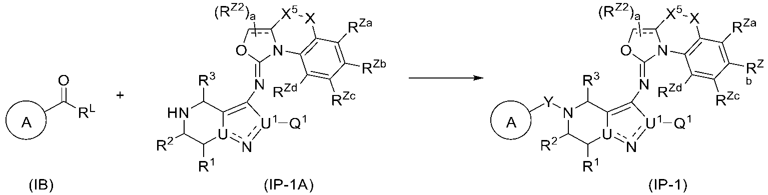
subjecting a compound represented by general formula (IB) or a salt thereof and a compound represented by general formula (IP-1A) or a salt thereof to a condensation reaction to obtain the compound represented by general formula (IP-1) or the pharmaceutically acceptable salt thereof,
wherein R^{L} is selected from the group consisting of halogen, hydroxyl, and alkoxy, and in some embodiments, is hydroxyl; Y is C(O);
ring A, 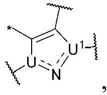 Q¹, R¹, R², R³, R^{Z2}, a, -̅ -̅ -̅, X, X⁵ , R^{Za}, R^{Zb}, R^{Zc}, and R^{Zd} are as defined for general formula (IP-1).

Another aspect of the present invention relates to a pharmaceutical composition, wherein the pharmaceutical composition comprises the compound represented by general formula (I) to (IV'), (IM) to (IM-2), (IN), (IN-1), (IP) to (IP-3), or (IO) of the present invention or as shown in Table A or a pharmaceutically acceptable salt thereof, and one or more pharmaceutically acceptable carriers, diluents or excipients.

The present invention further relates to the use of the compound represented by general formula (I) to (IV'), (IM) to (IM-2), (IN), (IN-1), (IP) to (IP-3), or (IO) or as shown in Table A or a pharmaceutically acceptable salt thereof, or a pharmaceutical composition comprising same in the preparation of a medicament for agonizing a GLP-1 receptor.

The present invention further relates to the use of the compound represented by general formula (I) to (IV'), (IM) to (IM-2), (IN), (IN-1), (IP) to (IP-3), or (IO) or as shown in Table A or a pharmaceutically acceptable salt thereof, or a pharmaceutical composition comprising same in the preparation of a GLP-1 receptor agonist.

The present invention further relates to the use of the compound represented by general formula (I) to (IV'), (IM) to (IM-2), (IN), (IN-1), (IP) to (IP-3), or (IO) or as shown in Table A or a pharmaceutically acceptable salt thereof, or a pharmaceutical composition comprising same in the preparation of a medicament for treating and/or preventing type I diabetes, type II diabetes, malnutrition-related diabetes, diabetic complications, obesity, hyperglycemia, glucose intolerance, cardiovascular diseases, hyperlipidemia, cerebral infarction, stroke, non-alcoholic steatohepatitis (NASH), Parkinson's disease, dementia, glaucoma, diarrhea, insulin resistance, and liver insulin resistance; and in some embodiments, in the preparation of a medicament for treating and/or preventing type I diabetes, type II diabetes, obesity, diabetic complications, non-alcoholic steatohepatitis, and cardiovascular diseases.

The present invention further relates to the use of the compound represented by general formula (I) to (IV'), (IM) to (IM-2), (IN), (IN-1), (IP) to (IP-3), or (IO) or as shown in Table A or a pharmaceutically acceptable salt thereof, or a pharmaceutical composition comprising same in the preparation of a medicament for treating and/or preventing idiopathic type I diabetes, latent immune diabetes in adults (LADA), maturity-onset diabetes of the young (MODY), gestational diabetes, non-alcoholic fatty liver disease (NAFLD), atherosclerosis, hypertension, and coronary heart disease.

The present invention further relates to a method for agonizing a GLP-1 receptor, comprising administering to a patient in need thereof a therapeutically effective amount of a compound represented by general formula (I) to (IV'), (IM) to (IM-2), (IN), (IN-1), (IP) to (IP-3), or (IO) or as shown in Table A or a pharmaceutically acceptable salt thereof, or a pharmaceutical composition comprising same.

The present invention further relates to a method for treating and/or preventing a disease or condition mediated by a GLP-1 receptor, comprising administering to a patient in need thereof a therapeutically effective amount of a compound represented by general formula (I) to (IV'), (IM) to (IM-2), (IN), (IN-1), (IP) to (IP-3), or (IO) or as shown in Table A or a pharmaceutically acceptable salt thereof, or a pharmaceutical composition comprising same.

The present invention further relates to a method for treating and/or preventing type I diabetes, type II diabetes, malnutrition-related diabetes, diabetic complications, obesity, hyperglycemia, glucose intolerance, cardiovascular diseases, hyperlipidemia, cerebral infarction, stroke, non-alcoholic steatohepatitis (NASH), Parkinson's disease, dementia, glaucoma, diarrhea, insulin resistance, and liver insulin resistance, comprising administering to a patient in need thereof a therapeutically effective amount of a compound represented by general formula (I) or (II) or as shown in Table A or a pharmaceutically acceptable salt thereof, or a pharmaceutical composition comprising same; and in some embodiments, the method is used for treating and/or preventing type I diabetes, type II diabetes, obesity, diabetic complications, non-alcoholic steatohepatitis, and cardiovascular diseases.

The present invention further relates to a method for treating and/or preventing idiopathic type I diabetes, latent immune diabetes in adults (LADA), maturity-onset diabetes of the young (MODY), gestational diabetes, non-alcoholic fatty liver disease (NAFLD), atherosclerosis, hypertension, and coronary heart disease, comprising administering to a patient in need thereof a therapeutically effective amount of a compound represented by general formula (I) or (II) or as shown in Table A or a pharmaceutically acceptable salt thereof, or a pharmaceutical composition comprising same.

The present invention further relates to a compound represented by general formula (I) to (IV'), (1M) to (IM-2), (IN), (IN-1), (IP) to (IP-3), or (IO) or as shown in Table A or a pharmaceutically acceptable salt thereof, or a pharmaceutical composition comprising same, for use as a medicament.

The present invention further relates to a compound represented by general formula (I) to (IV'), (1M) to (IM-2), (IN), (IN-1), (IP) to (IP-3), or (IO) or as shown in Table A or a pharmaceutically acceptable salt thereof, or a pharmaceutical composition comprising same, for use as a GLP-1 receptor agonist.

The present invention further relates to a compound represented by general formula (I) to (IV'), (IM) to (IM-2), (IN), (IN-1), (IP) to (IP-3), or (IO) or as shown in Table A or a pharmaceutically acceptable salt thereof, or a pharmaceutical composition comprising same, for agonizing a GLP-1 receptor.

The present invention further relates to a compound represented by general formula (I) to (IV'), (IM) to (IM-2), (IN), (IN-1), (IP) to (IP-3), or (IO) or as shown in Table A or a pharmaceutically acceptable salt thereof, or a pharmaceutical composition comprising same, for treating and/or preventing type I diabetes, type II diabetes, malnutrition-related diabetes, diabetic complications, obesity, hyperglycemia, glucose intolerance, cardiovascular diseases, hyperlipidemia, cerebral infarction, stroke, non-alcoholic steatohepatitis (NASH), Parkinson's disease, dementia, glaucoma, diarrhea, insulin resistance, and liver insulin resistance; and in some embodiments, for treating and/or preventing type I diabetes, type II diabetes, obesity, diabetic complications, non-alcoholic steatohepatitis, and cardiovascular diseases.

The present invention further relates to a compound represented by general formula (I) to (IV'), (1M) to (IM-2), (IN), (IN-1), (IP) to (IP-3), or (IO) or as shown in Table A or a pharmaceutically acceptable salt thereof, or a pharmaceutical composition comprising same, for treating and/or preventing idiopathic type I diabetes, latent immune diabetes in adults (LADA), maturity-onset diabetes of the young (MODY), gestational diabetes, non-alcoholic fatty liver disease (NAFLD), atherosclerosis, hypertension, and coronary heart disease.

In some embodiments, the disease or condition mediated by the GLP-1 receptor, as described in the present invention, is selected from the group consisting of type I diabetes, type II diabetes, malnutrition-related diabetes, diabetic complications, obesity, hyperglycemia, glucose intolerance, cardiovascular diseases, hyperlipidemia, cerebral infarction, stroke, non-alcoholic steatohepatitis (NASH), Parkinson's disease, dementia, glaucoma, diarrhea, insulin resistance, and liver insulin resistance; and in some embodiments, from type I diabetes, type II diabetes, obesity, diabetic complications, non-alcoholic steatohepatitis, and cardiovascular diseases.

In some embodiments, the disease or condition mediated by the GLP-1 receptor, as described in the present invention, is selected from the group consisting of idiopathic type I diabetes, latent immune diabetes in adults (LADA), maturity-onset diabetes of the young (MODY), gestational diabetes, non-alcoholic fatty liver disease (NAFLD), atherosclerosis, hypertension, and coronary heart disease.

"Diabetic complications" are complications caused by diabetes or hyperglycemia, and they can be acute complex or chronic complex. The term "acute complex" includes ketoacidosis and infectious diseases (such as skin infection, soft tissue infection, biliary tract infection, respiratory system infection, and urinary tract infection), and the "chronic complex" includes, for example, microangiopathy (such as nephropathy and retinopathy), neuropathy (such as sensory nerve disorder, motor nerve disorder and autonomic nerve disorder), and gangrene. Major diabetic complexes include diabetic retinopathy, diabetic nephropathy, and diabetic neuropathy.

"Coronary heart disease" includes myocardial infarction and angina pectoris.

"Dementia" includes, for example, Alzheimer's disease, (early-onset dementia) EOD, vascular dementia, and diabetic dementia.

The active compound can be formulated into a form suitable for administration via any appropriate route, and the compositions of the present invention can be formulated using conventional methods with one or more pharmaceutically acceptable carriers. Therefore, the active compounds of the present invention can be formulated into various dosage forms for oral administration, injection (e.g., intravenous injection, intramuscular injection, or subcutaneous injection), inhalation, or insufflation. The compounds of the present invention can also be formulated into, for example, dosage forms such as tablets, hard or soft capsules, aqueous or oily suspensions, emulsions, injectable solutions, dispersible powders or granules, suppositories, lozenges and syrups.

As a general guide, the active compound of the present invention is presented in a unit dosage form, or in a manner that allows the patient to self-administer a single dose. The compounds or compositions of the present invention can be presented in unit dosage forms such as tablets, capsules, cachets, bottled oral solutions, powders, granules, lozenges, suppositories, reconstitutable powders, or liquid formulations. An appropriate unit dose can be 0.1-1000 mg.

In addition to the active compound, the pharmaceutical composition of the present invention may contain one or more auxiliary materials selected from the group consisting of: fillers (diluents), binders, wetting agents, disintegrants, excipients, etc. Depending on the administration method, the composition can contain 0.1% to 99% by weight of the active compound.

In some embodiments, the unit dose of the pharmaceutical composition is 0.001-1000 mg.

In some embodiments, the pharmaceutical composition comprises 0.01%-99.99% of the aforementioned compound or a pharmaceutically acceptable salt thereof, or an isotopic substitute thereof, based on the total weight of the composition. In some embodiments, the pharmaceutical composition comprises 0.1%-99.9% of the aforementioned compound or a pharmaceutically acceptable salt thereof, or an isotopic substitute thereof. In some embodiments, the pharmaceutical composition comprises 0.5%-99.5% of the aforementioned compound or a pharmaceutically acceptable salt thereof, or an isotopic substitute thereof. In some embodiments, the pharmaceutical composition comprises 1%-99% of the aforementioned compound or a pharmaceutically acceptable salt thereof, or an isotopic substitute thereof. In some embodiments, the pharmaceutical composition comprises 2%-98% of the aforementioned compound or a pharmaceutically acceptable salt thereof, or an isotopic substitute thereof.

In some embodiments, the pharmaceutical composition comprises 0.01%-99.99% of the pharmaceutically acceptable excipient, based on the total weight of the composition. In some embodiments, the pharmaceutical composition comprises 0.1%-99.9% of the pharmaceutically acceptable excipient. In some embodiments, the pharmaceutical composition comprises 0.5%-99.5% of the pharmaceutically acceptable excipient. In some embodiments, the pharmaceutical composition comprises 1%-99% of the pharmaceutically acceptable excipient. In some embodiments, the pharmaceutical composition comprises 2%-98% of the pharmaceutically acceptable excipient.

The tablet contains an active ingredient, and a non-toxic, pharmaceutically acceptable excipient suitable for compounding into a tablet. These excipients can be inert excipients, granulators, disintegrants, binders, and lubricants. These tablets can be uncoated, or coated using known techniques to mask the taste of the drug or provide a sustained-release effect over an extended period by delaying disintegration and absorption in the gastrointestinal tract.

Oral formulations can also be provided as soft gelatin capsules in which the active ingredient is mixed with an inert solid diluent or in which the active ingredient is mixed with a water-soluble carrier or an oil-based vehicle.

The aqueous suspension contains an active substance, and an excipient suitable for compounding into an aqueous suspension. Such excipients are suspending agents, dispersants, or wetting agents. The aqueous suspension can also contain one or more preservatives, one or more colorants, one or more flavoring agents, and one or more sweeteners.

The oil suspension can be prepared by suspending active ingredients in vegetable oil or mineral oil. The oil suspension can contain thickeners. The above-described sweeteners and flavoring agents can be added to provide a palatable formulation. These compositions can be preserved by adding antioxidants.

The pharmaceutical composition of the present invention can also be in the form of an oil-in-water emulsion. The oil phase can be vegetable oil, mineral oil, or a mixture thereof. Suitable emulsifiers can be naturally occurring phospholipids, and emulsions can also contain sweeteners, flavoring agents, preservatives, and antioxidants. Such formulations can also contain demulcents, preservatives, colorants, and antioxidants.

The pharmaceutical composition of the present invention can be in the form of a sterile injectable aqueous solution. Acceptable vehicles or solvents that can be used include water, Ringer's solution, and isotonic sodium chloride solution. Sterile injectable formulations can be sterile injectable oil-in-water microemulsions in which the active ingredient is dissolved in the oil phase. The injectable solutions or microemulsions can be injected into the patient's bloodstream through local high-volume injection. Alternatively, the solutions and microemulsions are preferably administered in a manner that maintains a constant circulating concentration of the compounds of the present invention. To maintain such a constant concentration, a continuous intravenous delivery device can be used. An example of such a device is the Deltec CADD-PLUS. TM. 5400 intravenous injection pump.

The pharmaceutical composition of the present invention can be in the form of a sterile injectable water or an oil suspension for intramuscular and subcutaneous administration. The suspension can be prepared using the above-described suitable dispersants or wetting agents and suspending agents according to known techniques. Sterile injectable formulations can also be sterile injectable solutions or suspensions prepared in non-toxic diluents or solvents that are acceptable for parenteral administration. Furthermore, sterile fixed oils can conveniently be used as solvents or suspension media. For this purpose, any blended fixed oil can be used. In addition, fatty acids can also be used in the preparation of injections.

The compounds of the present invention can be administered in suppository form for rectal administration. These pharmaceutical compositions can be prepared by mixing the drug with a suitable, non-irritating excipient that is solid at normal temperatures but liquid in the rectum, and thus dissolves in the rectum to release the drug.

The compounds of the present invention can be prepared by adding water to form water-dispersible powders and granules. These pharmaceutical compositions can be prepared by mixing the active ingredient with a dispersant or a wetting agent, a suspending agent, or one or more preservatives.

As will be appreciated by a person skilled in the art, the administration dosage of the drug depends on a variety of factors, including, but not limited to: the activity of the specific compound used, the severity of the disease, the age, body weight, health status, behavior and diet of the patient, the administration time, the administration method, the excretion rate, the combination of drugs, etc. Furthermore, the optimal treatment method, such as the mode of treatment, the daily dosage of the compound, or the type of pharmaceutically acceptable salts, can be validated by conventional therapeutic protocols.

### Description of Terminology

Unless stated to the contrary, the terms used in the description and claims have the following meanings.

The term "alkyl" refers to a saturated aliphatic hydrocarbon group, which is a linear or branched group containing 1 to 20 carbon atoms. In some embodiments, it refers to an alkyl group having 1 to 12 carbon atoms (e.g., 1, 2, 3, 4, 5, 6, 7, 8, 9, 10, 11, and 12 carbon atoms) (i.e., C₁₋₁₂ alkyl), and in some embodiments, it refers to an alkyl group having 1 to 6 carbon atoms (i.e., C₁₋₆ alkyl). Non-limiting examples thereof include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, n-nonyl, 2-methyl-2-ethylhexyl, 2-methyl-3-ethylhexyl, 2,2-diethylpentyl, n-decyl, 3,3-diethylhexyl, 2,2-diethylhexyl, various branched-chain isomers thereof, etc. The alkyl can be substituted or unsubstituted. When substituted, the alkyl can be substituted at any available point of attachment, and in some embodiments, the substituents are selected from the group consisting of one or more of a D atom, halogen, alkoxy, haloalkyl, haloalkoxy, cycloalkyloxy, heterocyclyloxy, hydroxyl, hydroxyalkyl, cyano, amino, nitro, cycloalkyl, heterocyclyl, aryl, and heteroaryl.

The term "alkylene" refers to a divalent alkyl group, wherein the alkyl, as defined above, has 1 to 20 (e.g., 1, 2, 3, 4, 5, 6, 7, 8, 9, 10, 11, 12, 13, 14, 15, 16, 17, 18, 19, or 20) carbon atoms (i.e., C₁₋₂₀ alkylene). In some embodiments, the alkylene has 1 to 12 carbon atoms (i.e., C₁₋₁₂ alkylene). In some embodiments, the alkylene has 1 to 6 carbon atoms (i.e., C₁₋₆ alkylene). Non-limiting examples include: -CH₂-, -CH(CH₃)-, -C(CH₃)₂-, -CH₂CH₂-, -CH(CH₂CH₃)-, -CH₂CH(CH₃)-, -CH₂C(CH₃)₂-, -CH₂CH₂CH₂-, -CH₂CH₂CH₂CH₂-, etc. The alkylene can be substituted or unsubstituted. When substituted, the alkylene can be substituted at any available point of attachment, and in some embodiments, the substituents are selected from the group consisting of one or more of a D atom, halogen, alkoxy, haloalkyl, haloalkoxy, cycloalkyloxy, heterocyclyloxy, hydroxyl, hydroxyalkyl, cyano, amino, nitro, cycloalkyl, heterocyclyl, aryl, and heteroaryl.

The term "heteroalkyl" means that one or more carbon atoms of the alkyl group are replaced by one or more heteroatoms selected from the group consisting of nitrogen, oxygen, and sulfur, wherein the sulfur can be optionally oxidized (i.e., forming sulfoxide or sulfone), exclusive of the moiety of -O-O-, -O-S-, or -S-S-, the remaining atoms are carbon, and the alkyl group is as defined above.

The term "alkenyl" refers to an alkyl group containing at least one carbon-carbon double bond in the molecule, wherein the definition of alkyl is as described above. In some embodiments, it has 2 to 12 carbon atoms (e.g., 2, 3, 4, 5, 6, 7, 8, 9, 10, 11, and 12 carbon atoms) (i.e., C₂₋₁₂ alkenyl), and in some embodiments, it has 2 to 6 carbon atoms (i.e., C₂₋₆ alkenyl). Non-limiting examples include: ethenyl, propenyl, isopropenyl, butenyl, etc. The alkenyl can be substituted or unsubstituted. When substituted, in some embodiments, the substituents are selected from the group consisting of one or more of alkoxy, halogen, haloalkyl, haloalkoxy, cycloalkyloxy, heterocyclyloxy, hydroxyl, hydroxyalkyl, cyano, amino, nitro, cycloalkyl, heterocyclyl, aryl, and heteroaryl.

The term "alkynyl" refers to an alkyl group containing at least one carbon-carbon triple bond in the molecule, wherein the definition of alkyl is as described above. In some embodiments, it has 2 to 12 carbon atoms (e.g., 2, 3, 4, 5, 6, 7, 8, 9, 10, 11, and 12 carbon atoms) (i.e., C₂₋₁₂ alkynyl), and in some embodiments 2 to 6 carbon atoms (i.e., C₂₋₆ alkynyl). Non-limiting examples include: ethynyl, propynyl, butynyl, pentynyl, hexynyl, etc. The alkynyl can be substituted or unsubstituted. When substituted, in some embodiments, the substituents are selected from the group consisting of one or more of alkoxy, halogen, haloalkyl, haloalkoxy, cycloalkyloxy, heterocyclyloxy, hydroxyl, hydroxyalkyl, cyano, amino, nitro, cycloalkyl, heterocyclyl, aryl, and heteroaryl.

The term "cycloalkyl" refers to a saturated or partially unsaturated monocyclic or polycyclic cyclic hydrocarbon substituent. The cycloalkyl ring contains 3 to 20 carbon atoms. In some embodiments, it contains 3 to 12 carbon atoms (e.g., 3, 4, 5, 6, 7, 8, 9, 10, 11, and 12) carbon atoms (i.e., 3- to 12-membered cycloalkyl) or is 4-to 12-membered cycloalkyl. In some embodiments, it is 3- to 10-membered. In some embodiments, it contains 3 to 8 carbon atoms (e.g., 3, 4, 5, 6, 7, and 8) carbon atoms (i.e., 3- to 8-membered cycloalkyl). In some embodiments, it contains 3 to 6 carbon atoms (i.e., 3- to 6-membered cycloalkyl) or contains 3 to 5 carbon atoms (i.e., 3- to 5-membered cycloalkyl). Non-limiting examples of monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctyl, etc.; polycyclic cycloalkyl includes spirocycloalkyl, fused cycloalkyl, and bridged cycloalkyl.

The term "spirocycloalkyl" refers to a 5- to 20-membered polycyclic group in which monocycles share one carbon atom (referred to as the spiro atom), which may contain one or more double bonds. In some embodiments, the spirocycloalkyl is 6-to 14-membered. In some embodiments, the spirocycloalkyl is 7- to 12-membered. In some embodiments, the spirocycloalkyl is 7- to 10-membered (such as 7-, 8-, 9-, or 10-membered). The spirocycloalkyl can be classified into monospirocycloalkyl or polyspirocycloalkyl (e.g., dispirocycloalkyl) based on the number of spiro atoms shared between rings. In some embodiments, the spirocycloalkyl can be classified into monospirocycloalkyl and dispirocycloalkyl. In some embodiments, the spirocycloalkyl is 3-membered/4-membered, 3-membered/5-membered, 3-membered/6-membered, 4-membered/4-membered, 4-membered/5-membered, 4-membered/6-membered, 5-membered/3-membered, 5-membered/4-membered, 5-membered/5-membered, 5-membered/6-membered, 5-membered/7-membered, 6-membered/3-membered, 6-membered/4-membered, 6-membered/5-membered, 6-membered/6-membered, 6-membered/7-membered, 7-membered/5-membered, or 7-membered/6-membered monospirocycloalkyl. Non-limiting examples of spirocycloalkyl include: 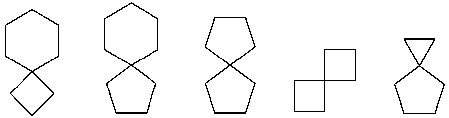 (the point of attachment can be at any position); 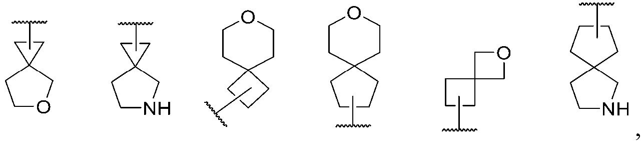 etc.

The term "fused cycloalkyl" refers to a 5- to 20-membered all-carbon polycyclic group in which each ring in the system shares an adjacent pair of carbon atoms with other rings in the system, and one or more rings may contain one or more double bonds. In some embodiments, the fused cycloalkyl is 6- to 14-membered. In some embodiments, the fused cycloalkyl is 7- to 12-membered. In some embodiments, the fused cycloalkyl is 7- to 10-membered (such as 7-, 8-, 9-, or 10-membered). Based on the number of constituent rings, the fused cycloalkyl can be classified into bicyclic or polycyclic fused cycloalkyl (e.g., tricyclic or tetracyclic). In some embodiments, the fused cycloalkyl is bicyclic or tricyclic. In some embodiments, the fused cycloalkyl is 3-membered/4-membered, 3-membered/5-membered, 3-membered/6-membered, 4-membered/4-membered, 4-membered/5-membered, 4-membered/6-membered, 5-membered/3-membered, 5-membered/4-membered, 5-membered/5-membered, 5-membered/6-membered, 5-membered/7-membered, 6-membered/3-membered, 6-membered/4-membered, 6-membered/5-membered, 6-membered/6-membered, 6-membered/7-membered, 7-membered/5-membered, or 7-membered/6-membered bicycloalkyl. Non-limiting examples of fused cycloalkyl include: 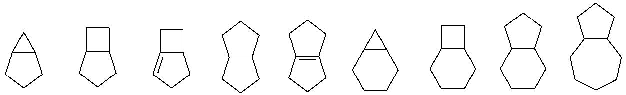 (the point of attachment can be at any position); 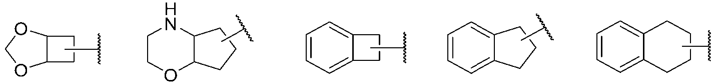 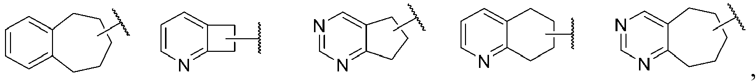 etc.

The term "bridged cycloalkyl" refers to a 5- to 20-membered all-carbon polycyclic group in which any two rings share two carbon atoms that are not directly connected, which may contain one or more double bonds. In some embodiments, the bridged cycloalkyl is 6- to 14-membered. In some embodiments, the bridged cycloalkyl is 7- to 10-membered (such as 7-, 8-, 9-, or 10-membered). According to the number of constituent rings, it can be classified into bicyclic or polycyclic (e.g., tricyclic or tetracyclic) bridged cycloalkyl. In some embodiments, it is bicyclic, tricyclic, or tetracyclic; in some embodiments, it is bicyclic or tricyclic. Non-limiting examples of bridged cycloalkyl include: 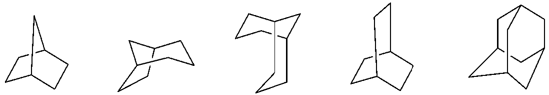 (the point of attachment can be at any position).

The cycloalkyl ring includes the cycloalkyl as described above (including monocyclic, spiro, fused, and bridged rings) fused to an aryl, heteroaryl, or heterocycloalkyl ring, wherein the ring connected to the parent structure is cycloalkyl. Non-limiting examples thereof include 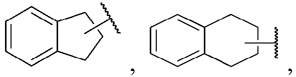 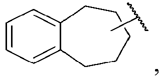 etc.; in some embodiments, the cycloalkyl ring is 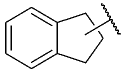 or

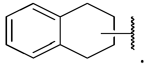

The cycloalkyl can be substituted or unsubstituted. When substituted, the cycloalkyl can be substituted at any available point of attachment, and in some embodiments, the substituents are selected from the group consisting of one or more of halogen, alkyl, alkoxy, haloalkyl, haloalkoxy, cycloalkyloxy, heterocyclyloxy, hydroxyl, hydroxyalkyl, cyano, amino, nitro, cycloalkyl, heterocyclyl, aryl, and heteroaryl.

The term "alkoxy" refers to -O-(alkyl), wherein the alkyl is as defined above. Non-limiting examples of alkoxy include: methoxy, ethoxy, propoxy, and butoxy. The alkoxy can be optionally substituted or unsubstituted. When substituted, in some embodiments, the substituents are selected from the group consisting of a D atom, halogen, alkoxy, haloalkyl, haloalkoxy, cycloalkyloxy, heterocyclyloxy, hydroxyl, hydroxyalkyl, cyano, amino, nitro, cycloalkyl, heterocyclyl, aryl, and heteroaryl.

The term "heterocyclyl" refers to a saturated or partially unsaturated monocyclic or polycyclic cyclic substituent, containing 3 to 20 ring atoms (e.g., 3, 4, 5, 6, 7, 8, 9, 10, 11, 12, 13, 14, 15, 16, 17, 18, 19, or 20 ring atoms) (i.e., 3- to 20-membered heterocyclyl), wherein one or more ring atoms are heteroatoms selected from the group consisting of nitrogen, oxygen, and sulfur, and the sulfur may be optionally oxidized (i.e., forming a sulfoxide or sulfone), but excluding ring moieties of -O-O-, -O-S-, or -S-S-, with the remaining ring atoms being carbon. In some embodiments, it contains 3 to 12 ring atoms (e.g., 3, 4, 5, 6, 7, 8, 9, 10, 11, and 12) ring atoms (i.e., 3- to 12-membered heterocyclyl), or 7- to 12-membered heterocyclyl, 1 to 4 (e.g., 1, 2, 3, and 4) of which are heteroatoms. In some embodiments, it is 3- to 10-membered. In some embodiments, it contains 3 to 8 ring atoms (e.g., 3, 4, 5, 6, 7, and 8) (i.e., 3- to 8-membered heterocyclyl), or it is 3- to 6-membered heterocyclyl or 4- to 7-membered heterocyclyl, or contains 6 to 14 ring atoms (e.g., 6, 7, 8, 9, 10, 11, 12, 13, and 14) (i.e., 6- to 14-membered heterocyclyl), 1 to 3 of which are heteroatoms (e.g., 1, 2, and 3); in some embodiments, it contains 5 or 6 ring atoms (i.e., 5-membered or 6-membered heterocyclyl), 1 to 3 of which are heteroatoms; and in some embodiments, it is 5-membered heterocyclyl. Non-limiting examples of monocyclic heterocyclyl include pyrrolidinyl, tetrahydropyranyl, 1,2,3,6-tetrahydropyridyl, piperidyl, piperazinyl, morpholinyl, thiomorpholinyl, homopiperazinyl, etc.

Polycyclic heterocyclyl includes spiroheterocyclyl, fused heterocyclyl, and bridged heterocyclyl.

The term "spiroheterocyclyl" refers to a 5- to 20-membered polycyclic heterocyclic group in which monocyclic rings share one atom (referred to as the spiro atom), wherein one or more ring atoms are heteroatoms selected from the group consisting of nitrogen, oxygen, and sulfur, and the sulfur may be optionally oxidized (i.e., forming a sulfoxide or sulfone), with the remaining ring atoms being carbon. It may contain one or more double bonds. In some embodiments, it is 6- to 14-membered (e.g., 6, 7, 8, 9, 10, 11, 12, 13, and 14-membered) (i.e., 6- to 14-membered spiroheterocyclyl), and in some embodiments, it is 7- to 10-membered (e.g., 7, 8, 9, or 10-membered) (i.e., 7- to 10-membered spiroheterocyclyl). The spiroheterocyclyl can be classified into monospiroheterocyclyl or polyspiroheterocyclyl (e.g., dispiroheterocyclyl) based on the number of spiro atoms shared between rings. In some embodiments, the spiro heterocyclyl is monospiroheterocyclyl or dispiroheterocyclyl. In some embodiments, the spiroheterocyclyl is 3-membered/4-membered, 3-membered/5-membered, 3-membered/6-membered, 4-membered/4-membered, 4-membered/5-membered, 4-membered/6-membered, 5-membered/3-membered, 5-membered/4-membered, 5-membered/5-membered, 5-membered/6-membered, 5-membered/7-membered, 6-membered/3-membered, 6-membered/4-membered, 6-membered/5-membered, 6-membered/6-membered, 6-membered/7-membered, 7-membered/5-membered, or 7-membered/6-membered monospiroheterocyclyl. Non-limiting examples of spiroheterocyclyl include:

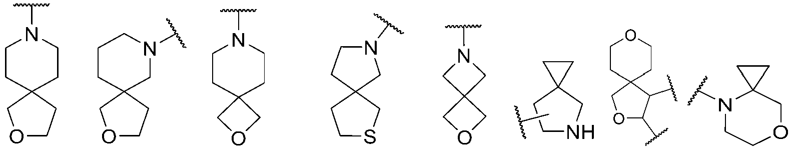

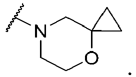

The term "fused heterocyclyl" refers to a 5- to 20-membered polycyclic heterocyclic group in which each ring in the system shares an adjacent pair of atoms with other rings in the system. One or more rings may contain one or more double bonds, wherein one or more ring atoms are heteroatoms selected from the group consisting of nitrogen, oxygen, and sulfur, and the sulfur may be optionally oxidized (i.e., forming a sulfoxide or sulfone), with the remaining ring atoms being carbon. In some embodiments, it is 6- to 14-membered (e.g., 6, 7, 8, 9, 10, 11, 12, 13, and 14-membered) (i.e., 6- to 14-membered fused heterocyclyl), and in some embodiments, it is 7- to 10-membered (e.g., 7, 8, 9, or 10-membered) (i.e., 7- to 10-membered fused heterocyclyl). Based on the number of constituent rings, the fused heterocyclyl can be classified into bicyclic or polycyclic (e.g., tricyclic or tetracyclic) fused heterocyclyl. In some embodiments, it is bicyclic or tricyclic. In some embodiments, the fused heterocyclyl is 3-membered/4-membered, 3-membered/5-membered, 3-membered/6-membered, 4-membered/4-membered, 4-membered/5-membered, 4-membered/6-membered, 5-membered/3-membered, 5-membered/4-membered, 5-membered/5-membered, 5-membered/6-membered, 5-membered/7-membered, 6-membered/3-membered, 6-membered/4-membered, 6-membered/5-membered, 6-membered/6-membered, 6-membered/7-membered, 7-membered/5-membered, or 7-membered/6-membered bicyclic fused heterocyclyl. Non-limiting examples of fused heterocyclyl include:

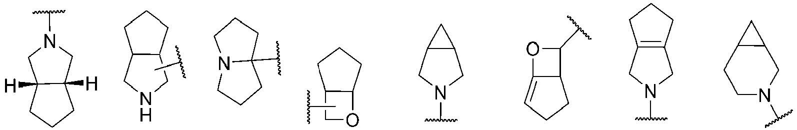

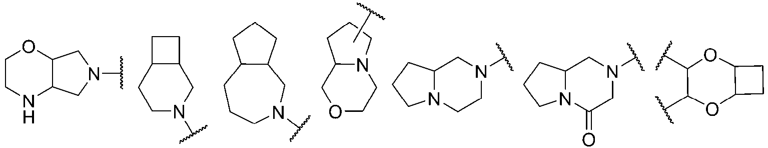

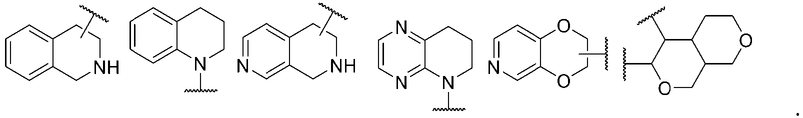

The term "bridged heterocyclyl" refers to a 5- to 20-membered polycyclic heterocyclic group in which any two rings share two atoms that are not directly connected, which may contain one or more double bonds, wherein one or more ring atoms are heteroatoms selected from the group consisting of nitrogen, oxygen, and sulfur, and the sulfur may be optionally oxidized (i.e., forming a sulfoxide or sulfone), with the remaining ring atoms being carbon. In some embodiments, it is 6-to 14-membered (e.g., 6, 7, 8, 9, 10, 11, 12, 13, and 14-membered) (i.e., 6- to 14-membered bridged heterocyclyl), and in some embodiments, it is 7- to 10-membered (e.g., 7, 8, 9, or 10-membered) (i.e., 7- to 10-membered bridged heterocyclyl). According to the number of constituent rings, it can be classified into bicyclic or polycyclic (e.g., tricyclic or tetracyclic) bridged heterocyclyl. In some embodiments, it is bicyclic, tricyclic, or tetracyclic; in some embodiments, it is bicyclic or tricyclic. Non-limiting examples of bridged heterocyclyl include:

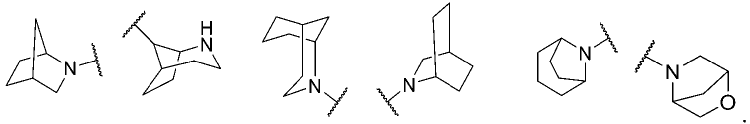

The heterocyclyl ring includes the heterocyclyl as described above (including monocyclic, spiroheterocyclic, fused heterocyclic, and bridged heterocyclic rings) fused to an aryl, heteroaryl, or cycloalkyl ring, wherein the ring connected to the parent structure is heterocyclyl. Non-limiting examples thereof include:

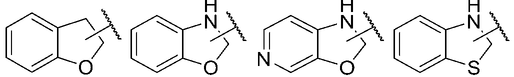

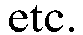

The heterocyclyl can be substituted or unsubstituted. When substituted, the heterocyclyl can be substituted at any available point of attachment, and in some embodiments, the substituents are selected from the group consisting of one or more of halogen, alkyl, alkoxy, haloalkyl, haloalkoxy, cycloalkyloxy, heterocyclyloxy, hydroxyl, hydroxyalkyl, cyano, amino, nitro, cycloalkyl, heterocyclyl, aryl, and heteroaryl.

The term "aryl" refers to a 6- to 14-membered all-carbon monocyclic or fused polycyclic (fused polycyclic rings are rings that share a pair of adjacent carbon atoms) group with a conjugated π-electron system. In some embodiments, it is 6- to 10-membered, for example, phenyl and naphthyl. The aryl ring includes the aryl ring as described above fused to a heteroaryl, heterocyclyl, or cycloalkyl ring, wherein the ring connected to the parent structure is an aryl ring. Non-limiting examples thereof include:

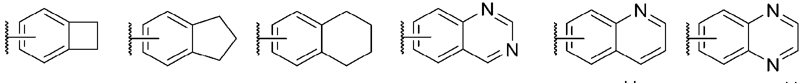

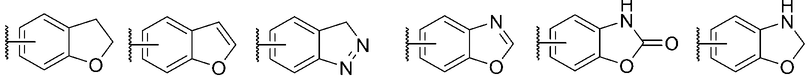

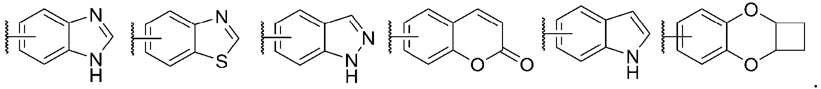

The aryl can be substituted or unsubstituted. When substituted, the aryl can be substituted at any available point of attachment, and in some embodiments, the substituents are selected from the group consisting of one or more of halogen, alkyl, alkoxy, haloalkyl, haloalkoxy, cycloalkyloxy, heterocyclyloxy, hydroxyl, hydroxyalkyl, cyano, amino, nitro, cycloalkyl, heterocyclyl, aryl, and heteroaryl.

The term "heteroaryl" refers to a heteroaromatic system containing 1 to 4 (e.g., 1, 2, 3, and 4) heteroatoms and 5 to 14 ring atoms, wherein the heteroatoms are selected from the group consisting of oxygen, sulfur and nitrogen. In some embodiments, the heteroaryl is 5- to 10-membered (e.g., 5-, 6-, 7-, 8-, 9-, or 10-membered) (i.e., 5- to 10-membered heteroaryl); in some embodiments, it is 8- to 10-membered (e.g., 8-, 9-, or 10-membered); in some embodiments, it is 5-membered or 6-membered (i.e., 5-membered or 6-membered heteroaryl), such as furyl, thienyl, pyridyl, pyrrolyl, N-alkylpyrrolyl, pyrimidinyl, pyrazinyl, pyridazinyl, imidazolyl, pyrazolyl, triazolyl, tetrazolyl, etc.; and in some embodiments, it is 5-membered heteroaryl. The heteroaryl ring includes the heteroaryl as described above fused to an aryl, heterocyclyl, or cycloalkyl ring, wherein the ring connected to the parent structure is a heteroaryl ring. Non-limiting examples thereof include: 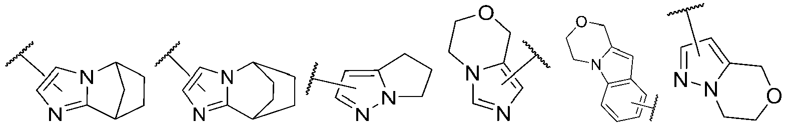 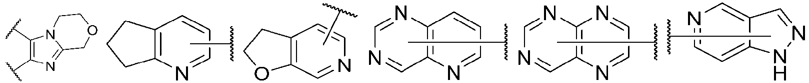 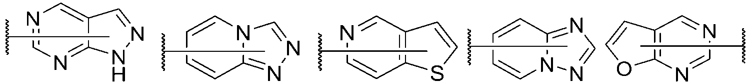 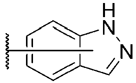 etc.

The heteroaryl can be substituted or unsubstituted. When substituted, the heteroaryl can be substituted at any available point of attachment, and in some embodiments, the substituents are selected from the group consisting of one or more of halogen, alkyl, alkoxy, haloalkyl, haloalkoxy, cycloalkyloxy, heterocyclyloxy, hydroxyl, hydroxyalkyl, cyano, amino, nitro, cycloalkyl, heterocyclyl, aryl, and heteroaryl.

The above-mentioned cycloalkyl, heterocyclyl, aryl, and heteroaryl include residues derived by removing one hydrogen atom from a parent ring atom, or residues derived by removing two hydrogen atoms from the same ring atom or two different ring atoms of the parent, i.e., "divalent cycloalkyl", "divalent heterocyclyl", "arylene", and "heteroarylene".

The term "amino protecting group" refers to a group that is easily removable and used to protect an amino group so that the amino group remains unchanged while reactions are carried out on other parts of the molecule. Non-limiting examples include (trimethylsilyl)ethoxymethyl (SEM), tetrahydropyranyl, tert-butoxycarbonyl (Boc), acetyl, benzyl, allyl, p-toluenesulfonyl (Ts), p-methoxybenzyl (PMB), etc. These groups may be optionally substituted with 1 to 3 substituents selected from the group consisting of halogen, alkoxy, and nitro; in some embodiments, the amino protecting group is Boc or PMB.

The term "hydroxy protecting group" refers to a derivative of a hydroxy group that is commonly used to block or protect the hydroxy group while reactions are carried out on other functional groups of the compound. By way of example, the hydroxy protecting groups include, for example, triethylsilyl, triisopropylsilyl, tert-butyldimethylsilyl (TBS), tert-butyldiphenylsilyl, methyl, tert-butyl, allyl, benzyl, methoxymethyl (MOM), tert-butyldiphenylsilyl (TBDPS), ethoxyethyl, 2-tetrahydropyranyl (THP), formyl, acetyl, benzoyl, and p-nitrobenzoyl; and in some embodiments, the hydroxy protecting group is MOM or TBDPS.

The term "cycloalkyloxy" refers to cycloalkyl-O-, wherein the cycloalkyl is as defined above.

The term "heterocyclyloxy" refers to heterocyclyl-O-, wherein the heterocyclyl is as defined above.

The term "aryloxy" refers to aryl-O-, wherein the aryl is as defined above.

The term "heteroaryloxy" refers to heteroaryl-O-, wherein the heteroaryl is as defined above.

The term "haloalkyl" refers to an alkyl group substituted with one or more halogen, wherein the alkyl is as defined above.

The term "haloalkoxy" refers to an alkoxy group substituted with one or more halogen, wherein the alkoxy is as defined above.

The term " deuterioalkyl" refers to an alkyl group substituted with one or more deuterium atoms, wherein the alkyl is as defined above.

The term "hydroxyalkyl" refers to alkyl substituted with one or more hydroxyl groups, wherein the alkyl is as defined above.

The term "cycloalkylalkyl" refers to an alkyl group substituted with one or more cycloalkyl groups, wherein the alkyl and cycloalkyl are as defined above.

The term "heterocyclylalkyl" refers to an alkyl group substituted with one or more heterocyclyl groups, wherein the alkyl and heterocyclyl are as defined above.

The term "arylalkyl" refers to an alkyl group substituted with one or more aryl groups, wherein the alkyl and aryl are as defined above.

The term "heteroarylalkyl" refers to an alkyl group substituted with one or more heteroaryl groups, wherein the alkyl and heteroaryl are as defined above.

The term " deuterioalkyl" refers to an alkyl group substituted with one or more deuterium atoms, wherein the alkyl is as defined above. Non-limiting examples include deuteriomethyl, deuterioethyl, etc. The deuteriomethyl is selected from the group consisting of CH₂D, CHD₂, and CD₃.

The term "halogen" refers to fluorine, chlorine, bromine, or iodine.

The term "hydroxyl" refers to -OH.

The term "mercapto" refers to -SH.

The term "amino" refers to -NH₂.

The term "cyano" refers to -CN.

The term "nitro" refers to -NO₂.

The term "oxo group" or "oxo" refers to "=O".

The term "carbonyl" refers to C=O.

The term "carboxyl" refers to -C(O)OH.

The term "carboxylate ester group" refers to -C(O)O(alkyl), - C(O)O(cycloalkyl), (alkyl)C(O)O-, or (cycloalkyl)C(O)O-, wherein the alkyl and cycloalkyl are as defined above.

MOM refers to methoxymethyl.

Boc refers to tert-butoxycarbonyl.

TIPS refers to triisopropylsilyl.

TBS refers to tert-butyldimethylsilyl.

The compounds of the present invention may include all forms of their rotamers and conformationally restricted states. Also included are atropisomers. The term "atropisomer" refers to stereoisomers resulting from hindered rotation around a single bond, where the energy difference due to steric strain or other contributing factors creates a sufficiently high rotational barrier to allow for the separation of individual conformers. For example, certain compounds of the present invention may exist as a mixture of atropisomers (such as an equal proportion mixture and a mixture enriched in one atropisomer) or as a purified single atropisomer.

The compounds and intermediates of the present invention may also exist in different tautomeric forms, and all such forms are encompassed within the scope of the present invention. The term "tautomer" or "tautomeric form" refers to structural isomers of different energies which are interconvertible via a low energy barrier. For example, proton tautomers (also known as proton transfer tautomers) include interconversion via proton migration, such as keto-enol, imine-enamine and lactam-lactim isomerization. An example of keto-enol equilibrium is as shown below: 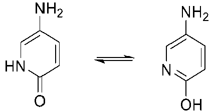 also for example, 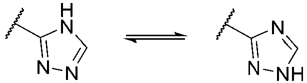 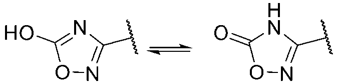 all tautomeric forms are included within the scope of the present invention. The names of the compounds do not exclude any tautomers.

The compounds of the present invention can exist in specific stereoisomeric forms. The term "stereoisomer" refers to isomers with the same structure but different spatial arrangements of atoms. It includes cis- and trans- (or *Z*- and *E*-) isomers, (-)- and (+)-isomers, (*R*)- and (*S*)-enantiomers, diastereomers, *(D*)- and (*L*)-isomers, tautomers, atropisomer, conformational isomers and mixtures thereof (such as mixtures of racemates and diastereomers). The substituents in the compounds of the present invention can contain additional asymmetric atoms. All these stereoisomer and mixtures thereof are encompassed within the scope of the present invention. For all carbon-carbon double bonds, both the *Z*- and *E*-forms are included, even if only one configuration is named. Optically active (-)- and (+)-isomers, (*R*)- and (*S*)-enantiomers, and *(D*)- and (*L*)-isomers can be prepared by using chiral synthesis, chiral reagents or other conventional techniques. An isomer of a compound of the present invention can be prepared by asymmetric synthesis or derivatization with a chiral auxiliary. Alternatively, where the molecule contains a basic functional group (such as amino) or an acidic functional group (such as carboxyl), diastereomeric salts can be formed with an appropriate optically active acid or base, followed by resolution of the diastereomers using conventional methods well known in the art to obtain the pure isomers. In addition, the separation of enantiomers and diastereomers is usually performed by chromatography.

In the chemical structure of the compound of the present invention, the bond " 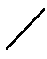 " represents an unspecified configuration, that is, if the chemical structure exists as a chiral isomer, the bond " 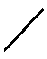 " can be " 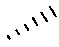 " or " 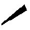 ", or both the configurations of " 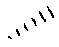 " and " 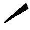 " are involved; the bond " 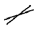 " represents an unspecified configuration, which can be in the Z configuration or the E configuration, or include both configurations.

The compounds of the present invention include all suitable isotopic derivatives thereof. The term "isotopic derivative" refers to a compound in which at least one atom is replaced by an atom having the same atomic number but a different atomic mass. Examples of isotopes that can be introduced into the compounds of the present invention include stable and radioactive isotopes of hydrogen, carbon, nitrogen, oxygen, phosphorus, sulfur, fluorine, chlorine, bromine, iodine, etc., such as ²H (deuterium, D),³H (tritium, T), ¹¹C, ¹³C, ¹⁴C, ¹⁵N, ¹⁷O, ¹³O, ³²p, ³³p, ³³S, ³⁴S, ³⁵S, ³⁶S, ¹⁸F, ³⁶Cl, ⁸²Br, ¹²³I, ¹²⁴I, ¹²⁵I, ¹²⁹I, and ¹³¹I. In some embodiments, the isotope is deuterium.

Deuterated drugs offer advantages over their non-deuterated counterparts, such as reduced toxicity and side effects, increased drug stability, enhanced efficacy, and prolonged biological half-life. All isotopic variations of the compounds of the present invention, whether radioactive or not, are encompassed within the scope of the present invention. Each available hydrogen atom attached to a carbon atom can be independently replaced by a deuterium atom, where such deuterium replacement can be partial or complete, and partial deuterium replacement means that at least one hydrogen atom is replaced by at least one deuterium atom.

"Optionally" or "optional" means that the event or circumstance subsequently described may but need not occur, and the description includes the case where the event or circumstance occurs or does not occur. For example, "C₁₋₆ alkyl optionally substituted with halogen or cyano" means that halogen or cyano may but need not be present. The description includes the case where the alkyl is substituted with halogen or cyano and the case where the alkyl is not substituted with halogen or cyano.

"Substituted" means that one or more hydrogen atoms in a group, in some embodiments 1 to 6 hydrogen atoms, and in some embodiments 1 to 3 hydrogen atoms, are each independently substituted with a corresponding number of substituents. A person skilled in the art would have been able to determine the possible or impossible substitutions (by experiment or theory) without undue effort. For example, an amino group or hydroxyl group having free hydrogen may be unstable when it binds to a carbon atom bearing an unsaturated (e.g., olefinic) bond.

The term "pharmaceutical composition" refers to a mixture of one or more of the compounds described herein, or pharmaceutically acceptable salts or prodrugs thereof, and other chemical components, as well as other ingredients such as pharmaceutically acceptable carriers and excipients. The purpose of the pharmaceutical compositions is to facilitate administration to living organisms and facilitate the absorption of active ingredients to exert biological activity.

The term "pharmaceutically acceptable salt" refers to a salt of the compound of the present invention, which can be selected from the group consisting of an inorganic salt or an organic salt. Such salts are safe and effective when used in mammals, and possess the expected biological activity. The salts can be prepared during the final isolation and purification of the compound, or separately by reacting the appropriate group with a suitable base or acid. The bases commonly used to form pharmaceutically acceptable salts include inorganic bases such as sodium hydroxide and potassium hydroxide, and organic bases such as ammonia. The acids commonly used to form pharmaceutically acceptable salts include inorganic acids and organic acids.

With respect to drugs or pharmacologically active agents, the term "therapeutically effective amount" refers to an amount of the drug or agent sufficient to achieve or at least partially achieve the desired effect. Determination of the therapeutically effective amount, varying from person to person, depends on the age and general condition of a subject and also depends on a specific active substance. The appropriate therapeutically effective amount in individual cases can be determined by a person skilled in the art according to routine experiments.

As used herein, the term "pharmaceutically acceptable" refers to those compounds, materials, compositions and/or dosage forms that are, within the scope of sound medical judgment, suitable for use in contact with patient tissues without excessive toxicity, irritation, allergic response, or other problems or complications, commensurate with a reasonable benefit/risk ratio, and effective for the intended use.

As used herein, the singular forms "a", "an", and "the" include plural referents and vice versa, unless otherwise clearly indicated in the context.

When the term "about" is applied to parameters such as pH, concentration, and temperature, it indicates that the parameters can vary by ±10%, and sometimes within ±5%. As will be understood by a person skilled in the art, numerical values for non-critical parameters are generally provided for illustrative purposes only and not as limitations.

### Methods for synthesizing compounds of the present invention

In order to achieve the objectives of the present invention, the following technical solutions are adopted:

### Scheme I

The present invention provides a method for preparing a compound represented by general formula (I) or a pharmaceutically acceptable salt thereof, wherein the method comprises: 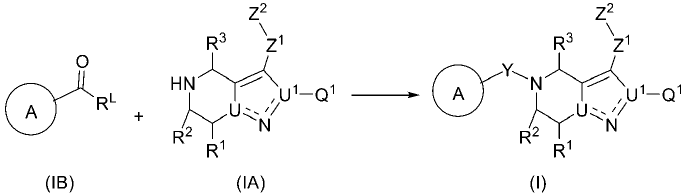
subjecting a compound represented by general formula (IA) or a salt thereof and a compound represented by general formula (IB) or a salt thereof to a condensation reaction under alkaline condition, optionally in the presence of a condensing agent, to obtain the compound represented by general formula (I) or the pharmaceutically acceptable salt thereof,
wherein R^{L} is selected from the group consisting of halogen, hydroxyl, and alkoxy, and in some embodiments, is hydroxyl; Y is C(O); 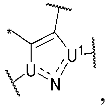 ring A, Q¹, Z¹, Z², R¹, R², and R³ are as defined for general formula (I).

### Scheme II

The present invention provides a method for preparing a compound represented by general formula (1M) or a pharmaceutically acceptable salt thereof, wherein the method comprises: 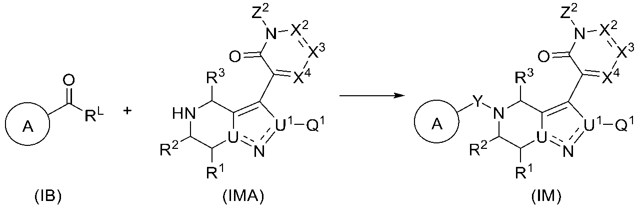
subjecting a compound represented by general formula (IMA) or a salt thereof and a compound represented by general formula (IB) or a salt thereof to a condensation reaction under alkaline condition, optionally in the presence of a condensing agent, to obtain the compound represented by general formula (IM) or the pharmaceutically acceptable salt thereof,
wherein R^{L} is selected from the group consisting of halogen, hydroxyl, and alkoxy, and in some embodiments, is hydroxyl; Y is C(O); 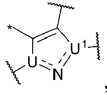 ring A, Q¹, X², X³, X⁴, Z², R¹, R², and R³ are as defined for general formula (1M).

### Scheme II'

The present invention provides a method for preparing a compound represented by general formula (IM-1) or a pharmaceutically acceptable salt thereof, wherein the method comprises: 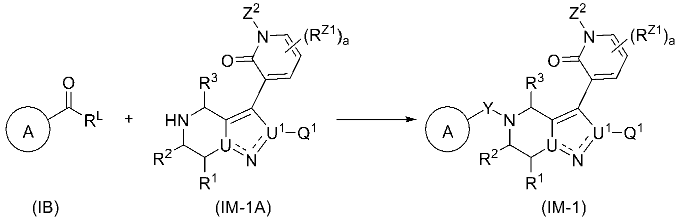
subjecting a compound represented by general formula (IM-1A) or a salt thereof and a compound represented by general formula (IB) or a salt thereof to a condensation reaction under alkaline condition, optionally in the presence of a condensing agent, to obtain the compound represented by general formula (IM-1) or the pharmaceutically acceptable salt thereof,
wherein R^{L} is selected from the group consisting of halogen, hydroxyl, and alkoxy, and in some embodiments, is hydroxyl; Y is C(O); 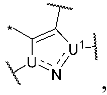 ring A, Q¹, Z², R^{Z1}, a, R¹, R², and R³ are as defined for general formula (IM-1).

### Scheme III

The present invention provides a method for preparing a compound represented by general formula (IM-2) or a pharmaceutically acceptable salt thereof, wherein the method comprises: 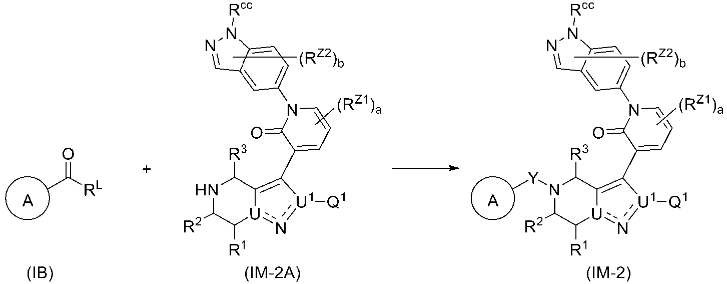
subjecting a compound represented by general formula (IM-2A) or a salt thereof and a compound represented by general formula (IB) or a salt thereof to a condensation reaction under alkaline condition, optionally in the presence of a condensing agent, to obtain the compound represented by general formula (IM-2) or the pharmaceutically acceptable salt thereof,
wherein R^{L} is selected from the group consisting of halogen, hydroxyl, and alkoxy, and in some embodiments, is hydroxyl; Y is C(O); 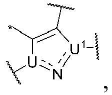 ring A, Q¹, R^{Z1}, a, R^{Z2}, b, R^{cc}, R¹, R², and R³ are as defined for general formula (IM-2).

### Scheme IV

The present invention provides a method for preparing a compound represented by general formula (IN) or a pharmaceutically acceptable salt thereof, wherein the method comprises: 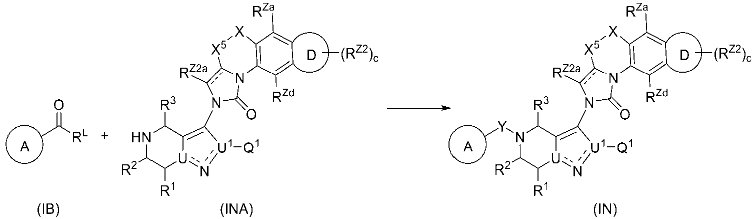
subjecting a compound represented by general formula (INA) or a salt thereof and a compound represented by general formula (IB) or a salt thereof to a condensation reaction under alkaline condition, optionally in the presence of a condensing agent, to obtain the compound represented by general formula (IN) or the pharmaceutically acceptable salt thereof,
wherein R^{L} is selected from the group consisting of halogen, hydroxyl, and alkoxy, and in some embodiments, is hydroxyl; Y is C(O); 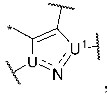 , ring A, Q¹, R¹, R², R³, R^{Z2a} -̅ -̅ -̅, X, X⁵ , R^{Za}, R^{Zd}, ring D, R^{Z2}, and c are as defined for general formula (IN).

### Scheme V

The present invention provides a method for preparing a compound represented by general formula (IN-1) or a pharmaceutically acceptable salt thereof, wherein the method comprises: 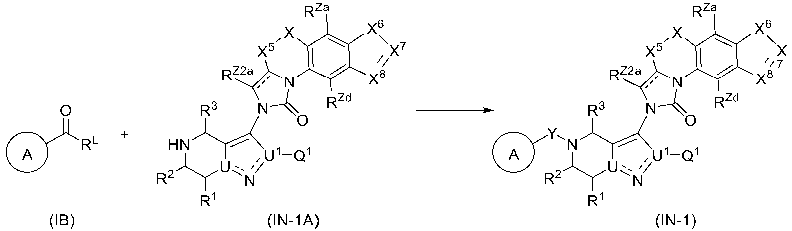
subjecting a compound represented by general formula (IN-1A) or a salt thereof and a compound represented by general formula (IB) or a salt thereof to a condensation reaction under alkaline condition, optionally in the presence of a condensing agent, to obtain the compound represented by general formula (IN-1) or the pharmaceutically acceptable salt thereof,
wherein R^{L} is selected from the group consisting of halogen, hydroxyl, and alkoxy, and in some embodiments, is hydroxyl; Y is C(O); 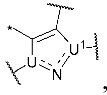 ring A, Q¹, R¹, R², R³ R^{Z2a}, -̅ -̅ -̅, X, X⁵, R^{Zd}, X⁶, X⁷, and X⁸ are as defined for general formula (IN-1).

### Scheme VI

The present invention provides a method for preparing a compound represented by general formula (II) or a pharmaceutically acceptable salt thereof, wherein the method comprises: 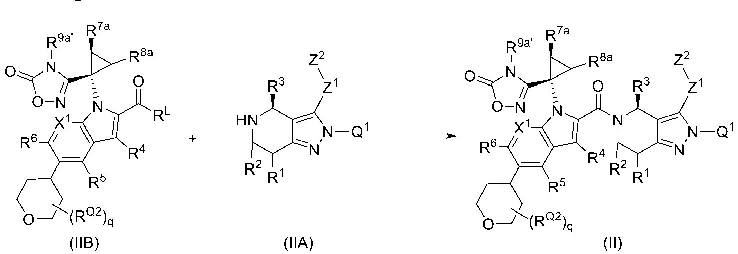
subjecting a compound represented by general formula (IIA) or a salt thereof and a compound represented by general formula (IIB) or a salt thereof to a condensation reaction under alkaline condition, optionally in the presence of a condensing agent, to obtain the compound represented by general formula (II) or the pharmaceutically acceptable salt thereof,
wherein R^{L} is selected from the group consisting of halogen, hydroxyl, and alkoxy, and in some embodiments, is hydroxyl; and
X¹, Q¹, Z¹, Z², R¹ to R⁶, R^{7a}, R^{8a}, R^{9a'}, R^{Q2}, and q are as defined for general formula (II).

### Scheme VI-1

The present invention provides a method for preparing a compound represented by general formula (II) or a pharmaceutically acceptable salt thereof, wherein the method comprises: 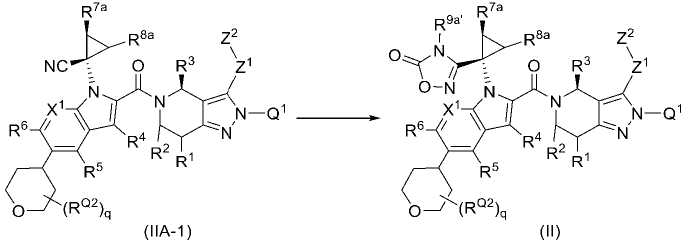
subjecting a compound represented by general formula (IIA-1) or a salt thereof and hydroxylamine or a salt thereof (in some embodiments, a hydrochloride thereof) to an addition reaction, followed by a further ring-closing reaction with *N,N'-*carbonyldiimidazole to obtain a compound represented by general formula (II) or a pharmaceutically acceptable salt thereof,
wherein R^{9a'} is a hydrogen atom; and
X¹, Q¹, Z¹, Z², R¹ to R⁶, R^{7a}, R^{8a}, R^{Q2}, and q are as defined for general formula (II).

### Scheme VII

The present invention provides a method for preparing a compound represented by general formula (III) or a pharmaceutically acceptable salt thereof, wherein the method comprises: 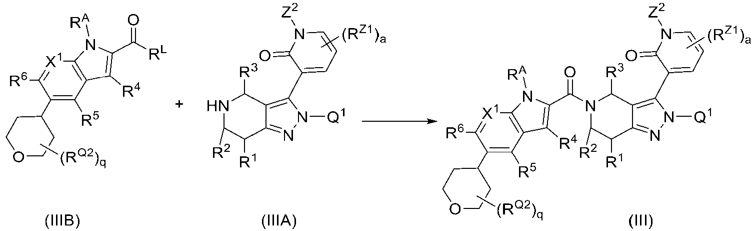
subjecting a compound represented by general formula (IIIA) or a salt thereof and a compound represented by general formula (IIIB) or a salt thereof to a condensation reaction under alkaline condition, optionally in the presence of a condensing agent, to obtain the compound represented by general formula (III) or the pharmaceutically acceptable salt thereof,
wherein R^{L} is selected from the group consisting of halogen, hydroxyl, and alkoxy, and in some embodiments, is hydroxyl;
X¹, Q¹, Z², R^{Z1}, a, R¹ to R⁶, R^{A}, R^{Q2}, and q are as defined for general formula (III).

### Scheme VIII

The present invention provides a method for preparing a compound represented by general formula (IV) or a pharmaceutically acceptable salt thereof, wherein the method comprises: 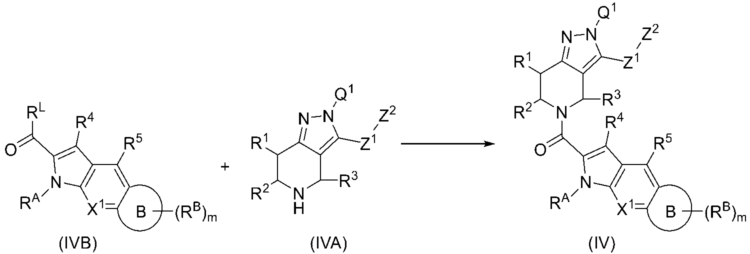
subjecting a compound represented by general formula (IVA) or a salt thereof and a compound represented by general formula (IVB) or a salt thereof to a condensation reaction under alkaline condition, optionally in the presence of a condensing agent, to obtain the compound represented by general formula (IV) or the pharmaceutically acceptable salt thereof,
wherein R^{L} is selected from the group consisting of halogen, hydroxyl, and alkoxy, and in some embodiments, is hydroxyl;
ring B, X¹, Q¹, Z¹, Z², R¹ to R⁴, R⁵, R^{A}, R^{B}, and m are as defined for general formula (IV).

### Scheme IX

The present invention provides a method for preparing a compound represented by general formula (IV') or a pharmaceutically acceptable salt thereof, wherein the method comprises: 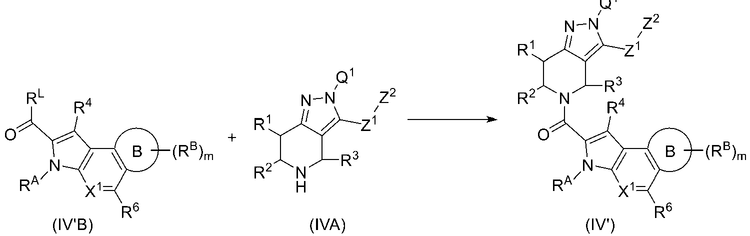
subjecting a compound represented by general formula (IVA) or a salt thereof and a compound represented by general formula (IV'B) or a salt thereof to a condensation reaction under alkaline condition, optionally in the presence of a condensing agent, to obtain the compound represented by general formula (IV') or the pharmaceutically acceptable salt thereof,
wherein R^{L} is selected from the group consisting of halogen, hydroxyl, and alkoxy, and in some embodiments, is hydroxyl;
ring B, X¹, Q¹, Z¹, Z², R¹ to R⁴, R⁶, R^{A}, R^{B}, and m are as defined for general formula (IV').

### Scheme X

The present invention provides a method for preparing a compound represented by general formula (IO) or a pharmaceutically acceptable salt thereof, wherein the method comprises: 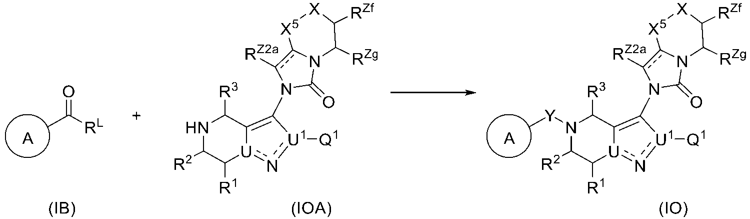
subjecting a compound represented by general formula (IOA) or a salt thereof and a compound represented by general formula (IB) or a salt thereof to a condensation reaction under alkaline condition, optionally in the presence of a condensing agent, to obtain the compound represented by general formula (IO) or the pharmaceutically acceptable salt thereof,
wherein R^{L} is selected from the group consisting of halogen, hydroxyl, and alkoxy, and in some embodiments, is hydroxyl; Y is C(O); 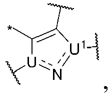 Q¹, ring A, R¹, R², R³, R^{Z2a}, -̅ -̅ -̅, X, X⁵, R^{Zf}, and R^{Zg} are as defined for general formula (IO).

### Scheme XI

The present invention provides a method for preparing a compound represented by general formula (IP) or a pharmaceutically acceptable salt thereof, wherein the method comprises: 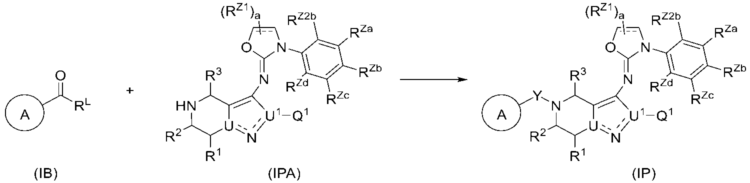
subjecting a compound represented by general formula (IB) or a salt thereof and a compound represented by general formula (IPA) or a salt thereof to a condensation reaction under alkaline condition, optionally in the presence of a condensing agent, to obtain the compound represented by general formula (IP) or the pharmaceutically acceptable salt thereof,
wherein R^{L} is selected from the group consisting of halogen, hydroxyl, and alkoxy, and in some embodiments, is hydroxyl; Y is C(O);
ring A, 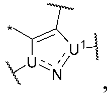 Q¹, R¹, R², R³, R^{Z1} _{"} a, -̅ -̅ -̅, R^{Z2b}, R^{Za}, R^{Zb}, R^{Zc}, and R^{Zd} are as defined for general formula (IP).

### Scheme XII

The present invention provides a method for preparing a compound represented by general formula (IP-1) or a pharmaceutically acceptable salt thereof, wherein the method comprises: 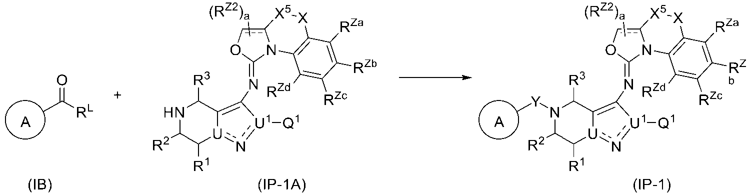
subjecting a compound represented by general formula (IB) or a salt thereof and a compound represented by general formula (IP-1A) or a salt thereof to a condensation reaction under alkaline condition, optionally in the presence of a condensing agent, to obtain the compound represented by general formula (IP-1) or the pharmaceutically acceptable salt thereof,
wherein R^{L} is selected from the group consisting of halogen, hydroxyl, and alkoxy, and in some embodiments, is hydroxyl; Y is C(O);
ring A, 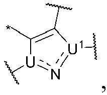 Q¹, R¹, R², R³, R^{Z2}, a, -̅ -̅ -̅, X, X⁵ , R^{Zb}, R^{Zc}, and R^{Zd} are as defined for general formula (IP-1).

The addition reaction in the above-mentioned synthesis Scheme VI-1 occurs under alkaline condition. In some embodiments, the reagent providing alkaline condition is anhydrous sodium bicarbonate. The ring-closing reaction in Scheme XI-1 occurs in the presence of a catalyst. In some embodiments, the catalyst is 1,8-diazabicyclo[5,4,0]undec-7-ene.

Reagents for providing the alkaline conditions in the above-mentioned synthesis schemes include organic bases and inorganic bases, wherein the organic bases include but are not limited to triethylamine, *N,N*-diisopropylethylamine, n-butyllithium, lithium diisopropylamide, potassium acetate, sodium tert-butoxide, potassium tert-butoxide, tetrabutylammonium fluoride, a solution of tetrabutylammonium fluoride in tetrahydrofuran, or 1,8-diazabicycloundec-7-ene, and the inorganic bases include but are not limited to sodium hydride, potassium phosphate, sodium carbonate, sodium acetate, potassium acetate, potassium carbonate, cesium carbonate, sodium hydroxide, lithium hydroxide, cesium fluoride, and potassium hydroxide. In some embodiments, the reagent is *N,N-*diisopropylethylamine.

The condensing agent mentioned in the above-mentioned synthesis schemes includes but is not limited to 1-(3-dimethylaminopropyl)-3-ethylcarbodiimide hydrochloride, *N,N'*-dicyclohexylcarbodiimide, *N,N*'-diisopropylcarbodiimide, O-benzotriazole-*N,N,N',N'*-tetramethyluronium tetrafluoroborate, 1-hydroxybenzotriazole, 1-hydroxy-7-azabenzotriazole, O-benzotriazole-*N,N,N',N'-*tetramethyluronium hexafluorophosphate, *O*-(7-azabenzotriazol-1-yl)-*N,N,N',N'-*tetramethyluronium hexafluorophosphate (HATU), 2-(7-azabenzotriazole)-*N,N,N',N'*-tetramethyluronium hexafluorophosphate (HATU), 2-(7-oxobenzotriazole)-*N,N,N',N'*-tetramethyluronium hexafluorophosphate, benzotriazol-1-yloxytris(dimethylamino)phosphonium hexafluorophosphate, or benzotriazol-1-yloxytris(pyrrolidino)phosphonium hexafluorophosphate. In some embodiments, the condensing agent is HATU.

The reaction of the above step is optionally carried out in a solvent, and the solvent used includes but is not limited to pyridine, ethylene glycol dimethyl ether, acetic acid, methanol, ethanol, acetonitrile, n-butanol, toluene, tetrahydrofuran, dichloromethane, petroleum ether, ethyl acetate, n-hexane, dimethyl sulfoxide, 1,4-dioxane, water, *N,N*-dimethylformamide, *N,N*-dimethylacetamide, 1,2-dibromoethane, and mixtures thereof.

### Detailed Description of Embodiments

The present invention will be further described below in conjunction with examples, but these examples are not intended to limit the scope of the present invention.

### Examples

The structures of the compounds are determined by nuclear magnetic resonance (NMR) or/and mass spectrometry (MS). The NMR shift (δ) is given in units of 10⁻⁶ (ppm). NMR is determined by Bruker AVANCE-400 nuclear magnetic resonance spectrometer; the solvents for determination are deuterated dimethyl sulfoxide (DMSO-*d₆*), deuterated chloroform (CDCl₃) and deuterated methanol (CD₃OD); and the internal standard is tetramethylsilane (TMS).

MS is determined by FINNIGAN LCQAd (ESI) mass spectrometer (manufacturer: Thermo, model: Finnigan LCQ advantage MAX).

High-performance liquid chromatography (HPLC) analysis is carried out by Agilent HPLC 1200DAD, Agilent HPLC 1200VWD and Waters HPLC e2695-2489 high-pressure liquid chromatographs.

Chiral HPLC analysis is determined by Agilent 1260 DAD high-performance liquid chromatograph.

Preparative high-performance liquid chromatography is carried out by Waters 2767, Waters 2767-SQ Detecor2, Shimadzu LC-20AP and Gilson-281 preparative chromatographs.

Chiral preparative chromatography is carried out by Shimadzu LC-20AP preparative chromatograph.

The CombiFlash flash preparative instrument used is Combiflash Rf200 (TELEDYNE ISCO).

Yantai Huanghai HSGF254 or Qingdao GF254 silica gel plate is used as a thin layer chromatography silica plate, and the silica gel plate for the thin layer chromatography (TLC) is of the specification of 0.15-0.2 mm, and the specification when separating and purifying a product by thin layer chromatography is 0.4-0.5 mm.

For silica gel column chromatography, Yantai Huanghai silica gel 200-300 mesh silica gel is generally used as a carrier.

The determination of the average kinase inhibition rate and IC₅₀ value is performed using NovoStar microplate reader (BMG Company, Germany).

Known starting materials of the present invention may be synthesized using or according to methods known in the art, or may be purchased from ABCR GmbH & Co. KG, Acros Organics, Aldrich Chemical Company, Accela Inc., Chembee Chemicals, and other companies.

If there is no special instruction in the examples, reactions can all be carried out under argon atmosphere or nitrogen atmosphere.

The argon atmosphere or nitrogen atmosphere means that the reaction flask is connected to an argon or nitrogen balloon with a volume of about 1 L.

The hydrogen atmosphere means that the reaction flask is connected to a hydrogen balloon with a volume of about 1 L.

Pressurized hydrogenation reactions are performed using Parr 3916EKX hydrogenator, Qinglan QL-500 hydrogen generator or HC2-SS hydrogenator.

The hydrogenation reaction usually comprises the steps of vacuumizing and charging with hydrogen, and the operation is repeated three times.

CEM Discover-S 908860 microwave reactor is used for the microwave reaction.

Unless otherwise specified in the examples, the solution refers to an aqueous solution.

Unless otherwise specified in the examples, the reaction temperature refers to room temperature.

The progress of the reaction in the examples is monitored by means of thin layer chromatography (TLC). The developing solvent used in the reaction, the eluent system of column chromatography used in the purification of compounds and the developing solvent system of thin layer chromatography include: A: dichloromethane/methanol system, B: n-hexane/ethyl acetate system, C: petroleum ether/ethyl acetate system, wherein the volume ratio of the solvent is adjusted according to the polarity of compounds and can also be adjusted by adding a small quantity of basic reagents such as triethylamine or acidic reagents such as acetic acid.

### Example 1

### 2-((S)-5-(5-((S)-2,2-dimethyltetrahydro-2H-pyran-4-yl)-1-((1S,2S)-2-methyl-1-(5-oxo-4,5-dihydro-1,2,4-oxadiazol-3-yl)cyclopropyl)-1H-indole-2-carbonyl)-2-(4-fluoro-3,5-dimethylphenyl)-4-methyl-4,5,6,7-tetrahydro-2H-pyrazolo[4,3-c]pyridin-3-yl)-10-fluoro-7-methyl-2,3,3a,4-tetrahydro-1H,7H-imidazo[1',5':4,5][1,4]oxazino[3,2-f]indazol-1-one 1

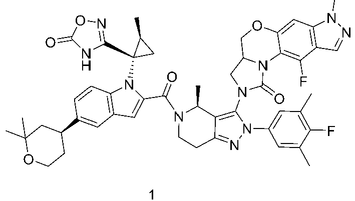

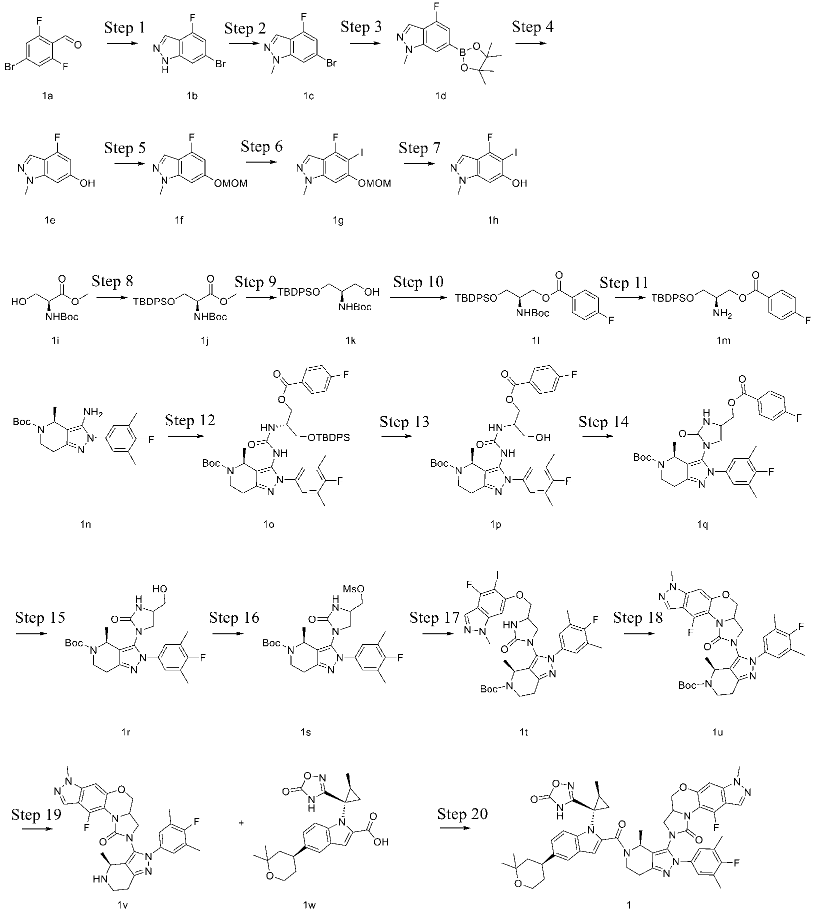

### Step 1

### 6-Bromo-4-fluoro-1H-indazole 1b

4-Bromo-2,6-difluorobenzaldehyde **1a** (14.6 g, 66.1 mmol, Shaoyuan Technology (Shanghai) Co., Ltd.), and hydrazine hydrate (28 g, 475 mmol, 85% wt aqueous solution) were dissolved in dioxane (80 mL) and stirred at 100°C for 7 hours. The reaction solution was cooled to room temperature. 200 mL of water was added. A solid was precipitated. After filtration, the solid was washed with water (100 mL × 2) and dried to obtain the crude title compound **1b** (13.9 g).

MS m/z (ESI): 213.1 [M-1].

### Step 2

### 6-Bromo-4-fluoro-1-methyl-1H-indazole 1c

Compound **1b** (13.9 g, 64.6 mmol) was dissolved in *N*,*N*-dimethylformamide (150 mL). Sodium hydride (3.4 g, 85.0 mmol, 60% wt) was added in portions at 0°C. The mixture was stirred for 30 minutes. Iodomethane (11 g, 77.5 mmol) was dropwise added. The mixture was reacted under stirring at room temperature for 2 hours. The reaction solution was quenched with a saturated ammonium chloride solution (300 mL), extracted with ethyl acetate (100 mL × 2), and washed with a saturated sodium chloride solution. The organic phase was dried over anhydrous sodium sulfate and filtered. The filtrate was concentrated under reduced pressure. The residue was purified by silica gel column chromatography with eluent system C to obtain the title compound **1c** (8.57 g, yield: 57.8%).

MS m/z (ESI): 228.9 [M+1].

### Step 3

### 4-Fluoro-1-methyl-6-(4,4,5,5-tetramethyl-1,3,2-dioxaborolan-2-yl)-1H-indazole 1d

Compound **1c** (8.57 g, 37.4 mmol), potassium acetate (11.1 g, 113.1mmol), bis(pinacolato)diboron (14 g, 55.1 mmol, Shaoyuan Technology (Shanghai) Co., Ltd.), and [1,1'-bis(diphenylphosphino)ferrocene]palladium dichloride (2.7 g, 3.69 mmol, Shanghai Bidepharm) were dissolved in dioxane (120 mL). After displacement with nitrogen three times, the mixture was reacted at 85°C for 16 hours. Water (100 mL) was added. The mixture was extracted with ethyl acetate (100 mL × 3). The organic phases were combined and washed with a saturated sodium chloride solution. The organic phase was dried over anhydrous sodium sulfate. After filtration, the filtrate was concentrated under reduced pressure. The residue was purified by silica gel column chromatography with eluent system C to obtain the title compound **1d** (10 g, yield: 96.7%).

MS m/z (ESI): 277.0 [M+1].

### Step 4

### 4-Fluoro-1-methyl-1H-indazol-6-ol 1e

Compound **1d** (10 g, 36.2 mmol) was dissolved in tetrahydrofuran (100 mL). An solution of sodium perborate tetrahydrate (12 g, 78.0 mmol) in water (50 mL) was dropwise added at 0°C and the mixture was reacted at room temperature for 2 hours. The reaction solution was poured into 100 mL of water, extracted with dichloromethane (100 mL × 3), and dried over anhydrous sodium sulfate. After filtration, the filtrate was concentrated under reduced pressure. The residue was purified by silica gel column chromatography with eluent system C to obtain the title compound **1e** (6.0 g, yield: 99.7%).

MS m/z (ESI): 166.9 [M+1].

### Step 5

### 4-Fluoro-6-(methoxymethoxy)-1-methyl-1H-indazole 1f

Compound **1e** (6.0 g, 36.1 mmol) and *N,N*-diisopropylethylamine (9.4 g, 72.7 mmol) was dissolved in dichloromethane (100 mL). Bromomethyl methyl ether (6.8 g, 54.4 mmol) was dropwise added at 0°C. After the addition was complete, the mixture was stirred at room temperature for 2 hours, quenched with water (100 mL), and extracted with dichloromethane (100 mL × 3). The organic phases were combined and washed with a saturated sodium chloride solution. The organic phase was dried over anhydrous sodium sulfate. After filtration, the filtrate was concentrated under reduced pressure. The residue was purified by silica gel column chromatography with eluent system C to obtain the title compound **1f** (7.2 g, yield: 94.8%).

MS m/z (ESI): 211.1 [M+1].

### Step 6

### 4-Fluoro-5-iodo-6-(methoxymethoxy)-1-methyl-1H-indazole 1g

Compound **1f** (4.0 g, 19.0 mmol) and tetramethylethylenediamine (3.3 g, 28.4 mmol, Adamas) were dissolved in tetrahydrofuran (60 mL). Sec-butyllithium (21.6 mL, 28.1 mmol, 1.3 M, Energy Chemical) was dropwise added in a nitrogen atmosphere at -78°C. After the addition was complete, stirring was continued at - 78°C for 2 hours. A solution of iodine (5.0 g, 19.7 mmol)/tetrahydrofuran (10 mL) was then dropwise added. After the addition was complete, stirring was continued at -78°C for 1 hour. The reaction was quenched with a saturated sodium thiosulfate solution and extracted with ethyl acetate (100 mL × 3). The organic phases were combined and washed with a saturated sodium chloride solution. The organic phase was dried over anhydrous sodium sulfate. After filtration, the filtrate was concentrated under reduced pressure. The residue was purified by silica gel column chromatography with eluent system C to obtain the title compound **1g** (1.0 g, yield: 15.6%).

MS m/z (ESI): 336.9 [M+1].

### Step 7

### 4-Fluoro-5-iodo-1-methyl-1H indazol-6-ol 1h

Compound **1g** (700 mg, 2.08 mmol) was dissolved in dichloromethane (5.0 mL). A hydrogen chloride/dioxane solution (5.0 mL, 4.0 M) was dropwise added at 0°C. After the addition was complete, the mixture was stirred at room temperature for 1 hour. After concentration, 30 mL of water was added. The mixture was extracted with ethyl acetate (30 mL × 3). The organic phases were combined, washed with a saturated aqueous sodium chloride solution, and dried over anhydrous sodium sulfate. After filtration, the filtrate was concentrated under reduced pressure. The residue was purified by silica gel column chromatography with eluent system C to obtain the title compound **1h** (430 mg, yield: 70.1%).

MS m/z (ESI): 292.9 [M+1].

### Step 8

### Methyl N-(tert-butoxycarbonyl)-O-(tert-butyldiphenylsilyl)-L-serinate 1j

Methyl (2*S*)-2-(tert-butoxycarbonylamino)-3-hydroxypropionate **1i** (6.2 g, 28.3 mmol, Shaoyuan Technology (Shanghai) Co., Ltd.) and imidazole (2.9 g, 42.6 mmol, Shaoyuan Technology (Shanghai) Co., Ltd.) were dissolved in dichloromethane (80 mL). Tert-butyldiphenylchlorosilane (8.6 g, 31.3 mmol, Shaoyuan Technology (Shanghai) Co., Ltd.) was added at 0°C. After the addition was complete, the mixture was reacted under stirring at room temperature for 16 hours. The reaction solution was poured into 100 mL of water and extracted with dichloromethane (100 mL × 2). The organic phases were combined, washed with a saturated sodium chloride solution, and dried over anhydrous sodium sulfate. After filtration, the filtrate was concentrated under reduced pressure. The residue was purified by silica gel column chromatography with eluent system C to obtain the title compound **1j** (12.9 g, yield: 99.6%).

MS m/z (ESI): 458.1 [M+1].

### Step 9

### Tert-butyl (R)-(1-((tert-butyldiphenylsilyl)oxy)-3-hydroxyprop-2-yl)carbamate 1k

Compound **1j** (5.7 g, 12.5 mmol) was dissolved in tetrahydrofuran (60 mL). A lithium borohydride/n-hexane solution (12.4 mL, 24.8 mmol, 2.0 M) was dropwise added at 0°C. After the addition was complete, the mixture was stirred at room temperature for 2 hours. The mixture was quenched with a saturated aqueous ammonium chloride solution and extracted with ethyl acetate (100 mL × 3). The organic phases were combined, washed with a saturated sodium chloride solution, and dried over anhydrous sodium sulfate. After filtration, the filtrate was concentrated under reduced pressure. The residue was purified by silica gel column chromatography with eluent system C to obtain the title compound **1k** (5.08 g, yield: 94.9%).

MS m/z (ESI): 430.1 [M+1].

### Step 10

### (R)-2-((tert-butoxycarbonyl)amino)-3-((tert-butyldiphenylsilyl)oxy)propyl 4-fluorobenzoate 1l

Compound **1k** (5.08 g, 11.8 mmol) and 4-fluorobenzoic acid (1.66 g, 11.8 mmol, Adamas) were dissolved in *N*,*N*-dimethylformamide (60 mL). 4-Dimethylaminopyridine (2.9 g, 23.5 mmol, Shaoyuan Technology (Shanghai) Co., Ltd.) and 1-(3-dimethylaminopropyl)-3-ethylcarbodiimide hydrochloride (4.5 g, 23.5 mmol, Shaoyuan Technology (Shanghai) Co., Ltd.) were added and stirred at room temperature for 16 hours. The reaction solution was poured into 100 mL of water and extracted with ethyl acetate (100 mL × 2). The organic phases were combined, washed with a saturated sodium chloride solution, and dried over anhydrous sodium sulfate. After filtration, the filtrate was concentrated under reduced pressure. The residue was purified by silica gel column chromatography with eluent system C to obtain the title compound **1l** (4.54 g, yield: 69.5%).

MS m/z (ESI): 552.1 [M+1].

### Step 11

### (R)-2-amino-3-((tert-butyldiphenylsilyl)oxy)propyl 4-fluorobenzoate 1m

Compound **1l** (4.54 g, 8.23 mmol) was dissolved in dichloromethane (50 mL). Zinc bromide (5.6 g, 24.9 mmol, Adamas) was added and reacted under stirring at room temperature for 16 hours. The reaction solution was poured into 100 mL of water and extracted with ethyl acetate (100 mL × 3). The organic phases were combined, washed with a saturated sodium chloride solution, and dried over anhydrous sodium sulfate. After filtration, the filtrate was concentrated under reduced pressure. The residue was purified by silica gel column chromatography with eluent system C to obtain the title compound **1m** (3.48 g, yield: 93.6%).

MS m/z (ESI): 452.2 [M+1].

### Step 12

### Tert-butyl (S)-3-(3-((R)-1-((tert-butyldiphenylsilyl)oxy)-3-((4-fluorobenzoyl)oxy)prop-2-yl)ureido)-2-(4-fluoro-3,5-dimethylphenyl)-4-methyl-2,4,6,7-tetrahydro-5H-pyrazolo[4,3-c]pyridine-5-carboxylate 1o

Tert-butyl (*S*)-3-amino-2-(4-fluoro-3,5-dimethylphenyl)-4-methyl-2,4,6,7-tetrahydro-5*H*-pyrazolo[4,3-*c*]pyridine-5-carboxylate **1n** (1.0 g, 2.67 mmol, Shanghai Bidepharm) and *N*,*N*-diisopropylethylamine (1.8 g, 13.9 mmol) were dissolved in tetrahydrofuran (10 mL). Under ice bath condition, phenyl chloroformate (640 mg, 4.09 mmol, Adamas) was dropwise added and reacted under stirring at room temperature for 2 hours. Compound **1m** (3.4 g, 7.52 mmol) was added and stirred at room temperature for 16 hours. The reaction solution was poured into 30 mL of water and extracted with ethyl acetate (30 mL × 3). The organic phases were combined, washed with a saturated sodium chloride solution, and dried over anhydrous sodium sulfate. After filtration, the filtrate was concentrated under reduced pressure. The residue was purified by silica gel column chromatography with eluent system C to obtain the title compound **1o** (2.2 g, yield: 95.6%).

MS m/z (ESI): 850.7 [M-1].

### Step 13

### Tert-butyl (4S)-2-(4-fluoro-3,5-dimethylphenyl)-3-(3-(1-((4-fluorobenzoyl)oxy)-3-hydroxyprop-2-yl)ureido)-4-methyl-2,4,6,7-tetrahydro-5H-pyrazolo[4,3-c]pyridine-5-carboxylate 1p

Compound **1o** (2.2 g, 2.58 mmol) was dissolved in tetrahydrofuran (20 mL). A tetrabutylammonium fluoride/tetrahydrofuran solution (8.0 mL, 8.0 mmol, 1.0 M) was added and the mixture was reacted at room temperature for 1 hour. The reaction solution was poured into 30 mL of water and extracted with ethyl acetate (30 mL × 3). The organic phases were combined, washed with a saturated sodium chloride solution, and dried over anhydrous sodium sulfate. After filtration, the filtrate was concentrated under reduced pressure. The residue was purified by silica gel column chromatography with eluent system C to obtain the title compound **1p** (1.2 g, yield: 72.7%).

MS m/z (ESI): 614.2 [M+1].

### Step 14

### Tert-butyl (4S)-2-(4-fluoro-3,5-dimethylphenyl)-3-(4-(((4-fluorobenzoyl)oxy)methyl)-2-oxoimidazolidin-1-yl)-4-methyl-2,4,6,7-tetrahydro-5H-pyrazolo[4,3-c]pyridine-5-carboxylate 1q

Compound **1p** (1.2 g, 1.96 mmol) and triphenylphosphine (1.0 g, 3.82 mmol, Adamas) were dissolved in tetrahydrofuran (100 mL). Di-tert-butyl azodicarboxylate (900 mg, 3.91 mmol, Adamas) was added at 0°C and the mixture was reacted at room temperature for 16 hours. The reaction solution was poured into 100 mL of water and extracted with ethyl acetate (30 mL × 3). The organic phases were combined, washed with a saturated sodium chloride solution, and dried over anhydrous sodium sulfate. After filtration, the filtrate was concentrated under reduced pressure. The residue was purified by silica gel column chromatography with eluent system C and then purified by preparative high-performance liquid chromatography (instrument model: Waters-2545 chromatographic column: Sharpsil-T, Prep 30 × 150 mm; 5 µm; C18 mobile phase: water (0.1% trifluoroacetic acid) and acetonitrile, with gradient ratio: acetonitrile 50%-95%, flow rate: 30 mL/min) to obtain the title compound **1q** (480 mg, yield: 41.2%).

MS m/z (ESI): 596.2 [M+1].

### Step 15

### Tert-butyl (4S)-2-(4-fluoro-3,5-dimethylphenyl)-3-(4-(hydroxymethyl)-2-oxoimidazolidin-1-yl)-4-methyl-2,4,6,7-tetrahydro-5H-pyrazolo[4,3-c]pyridine-5-carboxylate 1r

Compound **1q** (460 mg, 0.77 mmol) was dissolved in tetrahydrofuran (5.0 mL) and water (1.0 mL). Lithium hydroxide monohydrate (160 mg, 3.81 mmol) was added and the mixture was reacted at room temperature for 3 hours. The reaction solution was poured into 20 mL of water and extracted with ethyl acetate (30 mL × 2). The organic phases were combined, washed with a saturated sodium chloride solution, and dried over anhydrous sodium sulfate. After filtration, the filtrate was concentrated under reduced pressure to obtain the crude title compound **1r** (365 mg).

MS m/z (ESI): 474.3 [M+1].

### Step 16

### Tert-butyl (4S)-2-(4-fluoro-3,5-dimethylphenyl)-4-methyl-3-(4-(((methylsulfonyl)oxy)methyl)-2-oxoimidazolidin-1-yl)-2,4,6,7-tetrahydro-5H-pyrazolo[4,3-c]pyridine-5-carboxylate 1s

Compound **1r** (365 mg, 0.77 mmol) and triethylamine (235 mg, 2.32mmol) were dissolved in dichloromethane (5.0 mL) and water (1.0 mL). Methanesulfonyl chloride (130 mg, 0.88 mmol) was added at 0°C and the mixture was reacted at room temperature for 1 hour. The reaction solution was poured into 20 mL of water and extracted with dichloromethane (30 mL × 2). The organic phases were combined, washed with a saturated sodium chloride solution, and dried over anhydrous sodium sulfate. After filtration, the filtrate was concentrated under reduced pressure to obtain the crude title compound **1s** (420 mg).

MS m/z (ESI): 552.5 [M+1].

### Step 17

### Tert-butyl (4S)-2-(4-fluoro-3,5-dimethylphenyl)-3-(4-(((4-fluoro-5-iodo-1-methyl-1H-indazol-6-yl)oxy)methyl)-2-oxoimidazolidin-1-yl)-4-methyl-2,4,6,7-tetrahydro-5H-pyrazolo[4,3-c]pyridine-5-carboxylate 1t

Compound **1s** (420 mg, 0.76 mmol) and compound **1h** (200 mg, 0.68 mmol) were dissolved in *N,N-*dimethylformamide (5.0 mL). Potassium carbonate (250 mg, 1.81 mmol) was added and reacted at 80°C for 5 hours. The reaction solution was poured into 20 mL of water and extracted with ethyl acetate (30 mL × 2). The organic phases were combined, washed with a saturated sodium chloride solution, and dried over anhydrous sodium sulfate. After filtration, the filtrate was concentrated under reduced pressure. The residue was purified by silica gel column chromatography with eluent system A to obtain the title compound **1t** (280 mg, yield: 54.7%).

MS m/z (ESI): 748.2 [M+1].

### Step 18

### Tert-butyl (4S)-2-(4-fluoro-3,5-dimethylphenyl)-3-(10-fluoro-7-methyl-1-oxo-3a,4-dihydro-1H,7H-imidazo[1',5':4,5][1,4]oxazino[3,2-f]indazol-2(3H)-yl)-4-methyl-2,4,6,7-tetrahydro-5H-pyrazolo[4,3-c]pyridine-5-carboxylate 1u

Compound **1t** (280 mg, 0.37 mmol) and *L*-proline (40 mg, 0.35 mmol) were dissolved in *N,N-*dimethylformamide (15.0 mL). Potassium carbonate (130 mg, 0.94 mmol) and cuprous iodide (36 mg, 0.19 mmol, Leyan) were added. After displacement with nitrogen three times, the mixture was reacted under microwave at 120°C for 3 hours. The reaction solution was poured into 20 mL of water and extracted with ethyl acetate (30 mL × 2). The organic phases were combined, washed with a saturated sodium chloride solution, and dried over anhydrous sodium sulfate. After filtration, the filtrate was concentrated under reduced pressure. The residue was purified by silica gel column chromatography with eluent system C to obtain the title compound **1u** (140 mg, yield: 60.3%).

MS m/z (ESI): 620.2 [M+1].

### Step 19

### 10-Fluoro-2-((S)-2-(4-fluoro-3,5-dimethylphenyl)-4-methyl-4,5,6,7-tetrahydro-2H-pyrazolo[4,3-c]pyridin-3-yl)-7-methyl-2,3,3a,4-tetrahydro-1H,7H-imidazo[1',5':4,5][1,4]oxazino[3,2-f]indazol-1-one 1v

Compound **1u** (140 mg, 0.23 mmol) was dissolved in methanol (2 mL). A 4 M solution of hydrogen chloride in 1,4-dioxane (2 mL) was added and stirred at room temperature for 2 hours. The reaction solution was concentrated under reduced pressure to obtain the crude title compound **1v** (110 mg). The product was directly used in the next reaction without purification.

MS m/z (ESI): 520.2 [M+1].

### Step 20

### 2-((S)-5-(5-((S)-2,2-dimethyltetrahydro-2H-pyran-4-yl)-1-((1S,2S)-2-methyl-1-(5-oxo-4,5-dihydro-1,2,4-oxadiazol-3-yl)cyclopropyl)-1H-indole-2-carbonyl)-2-(4-fluoro-3,5-dimethylphenyl)-4-methyl-4,5,6,7-tetrahydro-2H-pyrazolo[4,3-c]pyridin-3-yl)-10-fluoro-7-methyl-2,3,3a,4-tetrahydro-1H,7H-imidazo[1',5':4,5][1,4]oxazino[3,2-f]indazol-1-one 1

Crude compound **1v** (110 mg, 0.21 mmol) and 5-((*S*)-2,2-dimethyltetrahydro-2*H*-pyran-4-yl)-1-((1*S*,2*S*)-2-methyl-1-(5-oxo-4,5-dihydro-1,2,4-oxadiazol-3-yl)cyclopropyl)-1*H*-indole-2-carboxylic acid **1w** (90 mg, 0.22 mmol, prepared by a method disclosed in Intermediate 47 on page 185 of the description of Patent Application WO 2022017338 A1) were dissolved in *N*,*N*-dimethylformamide (3 mL). *N*,*N*-diisopropylethylamine (140 mg, 1.08 mmol) was added. After the raw materials were fully dissolved, *O*-(7-azabenzotriazol-1-yl)*-N,N,N',N'-*tetramethyluronium hexafluorophosphate (240 mg, 0.63 mmol) was added and stirred at room temperature for 3 hours. The reaction solution was poured into 10 mL of water and extracted with ethyl acetate (10 mL × 3). The organic phases were combined, washed with a saturated sodium chloride solution, and dried over anhydrous sodium sulfate. After filtration, the filtrate was concentrated under reduced pressure to obtain the title compound 1 (a mixture of diastereomers, 140 mg).

MS m/z (ESI): 913.4 [M+1].

### Example 1-1 and Example 1-2

### (S)-2-((S)-5-(5-((S)-2,2-dimethyltetrahydro-2H-pyran-4-yl)-1-((1S,2S)-2-methyl-1-(5-oxo-4,5-dihydro-1,2,4-oxadiazol-3-yl)cyclopropyl)-1H-indole-2-carbonyl)-2-(4-fluoro-3,5-dimethylphenyl)-4-methyl-4,5,6,7-tetrahydro-2H-pyrazolo[4,3-c]pyridin-3-yl)-10-fluoro-7-methyl-2,3,3a,4-tetrahydro-1H,7H-imidazo[1',5':4,5][1,4]oxazino[3,2-f]indazol-1-one 1-1 and (R)-2-((S)-5-(5-((S)-2,2-dimethyltetrahydro-2H-pyran-4-yl)-1-((1S,2S)-2-methyl-1-(5-oxo-4,5-dihydro-1,2,4-oxadiazol-3-yl)cyclopropyl)-1H-indole-2-carbonyl)-2-(4-fluoro-3,5-dimethylphenyl)-4-methyl-4,5,6,7-tetrahydro-2H-pyrazolo[4,3-c]pyridin-3-yl)-10-fluoro-7-methyl-2,3,3a,4-tetrahydro-1H,7H-imidazo[1',5':4,5][1,4]oxazino[3,2-f]indazol-1-one 1-2

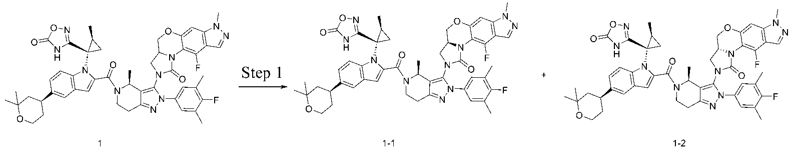

Compound **1** (140 mg, 0.15 mmol) was purified by preparative high-performance liquid chromatography (instrument model: Waters-2545 chromatographic column: Welch Xtimate, Prep 30 × 250 mm; 5 µm; C18, mobile phase: water (10 mM ammonium bicarbonate) and acetonitrile, with gradient ratio: acetonitrile 40%-95%, flow rate: 30 mL/min) to obtain the title compounds (57 mg, yield: 29.5%) and (51 mg, yield: 26.4%).

Single-configuration compound (shorter retention time, retention time: 1.33 minutes, 57 mg, yield: 29.5%):
MS m/z (ESI): 913.8 [M+1].

¹H NMR (500 MHz, CDCl₃): δ 8.03-7.99 (m, 1H), 7.61(d, 1H), 7.54-7.50 (m, 1H), 7.29-7.27 (m, 1H), 7.20-7.17 (m, 2H), 6.72-6.66 (m, 2H), 5.82-5.78 (m, 1H), 4.49-4.45 (m, 1H), 4.27-4.24 (m, 1H), 4.11-4.08 (m, 1H), 4.02-3.96 (m, 3H), 3.91-3.83 (m, 3H), 3.67-3.60 (m, 2H), 3.61-3.00 (m, 4H), 2.37-2.30 (m, 6H), 1.94 (t, 1H), 1.73-1.50 (m, 7H), 1.36-1.25 (m, 10H), 1.07 (d, 1H).

Single-configuration compound (longer retention time, retention time 1.42 minutes, 51 mg, yield: 26.4%):
MS m/z (ESI): 913.8 [M+1].

¹H NMR (500 MHz, CDCl₃): δ 8.06 (s, 1H), 7.79-7.60(m, 2H), 7.53 (d, 1H), 7.33-7.27 (m, 1H), 7.17 (d, 1H), 6.76-6.71 (m, 2H), 5.72-5.68 (m, 1H), 4.48-4.44 (m, 2H), 4.15-4.08 (m, 1H), 4.01-3.96 (m, 3H), 3.92-3.84 (m, 3H), 3.73-3.60 (m, 2H), 3.30 (d,1H), 3.15-3.00(m, 3H), 2.37-2.30 (m, 6H), 1.92 (t, 1H), 1.80-1.54 (m, 7H), 1.40-1.22 (m, 10H), 1.07 (d, 1H).

### Example 2

### 3-((1S,2S)-1-(5-((S)-2,2-dimethyltetrahydro-2H-pyran-4-yl)-2-((S)-2-(4-fluoro-3,5-dimethylphenyl)-4-methyl-3-(2-oxo-1,2-dihydropyridin-3-yl)-4,5,6,7-tetrahydro-2H-pyrazolo[4,3-c]pyridine-5-carbonyl)-1H-indol-1-yl)-2-methylcyclopropyl)-1,2,4-oxadiazol-5(4H)-one 2

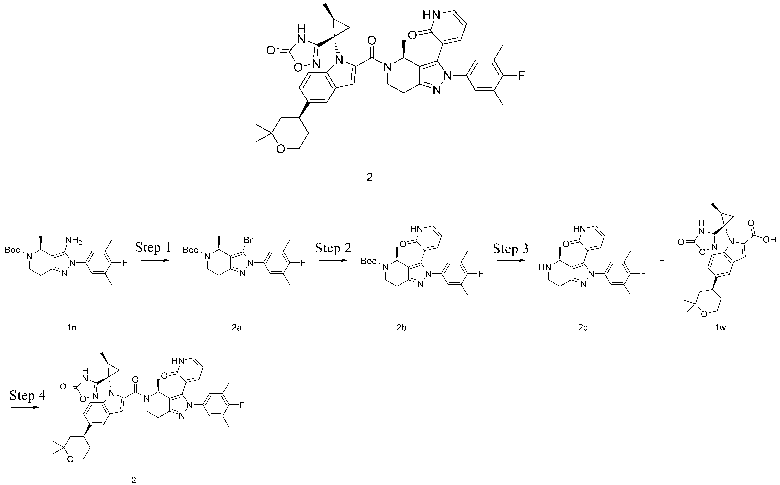

### Step 1

### Tert-butyl (S)-3-bromo-2-(4-fluoro-3,5-dimethylphenyl)-4-methyl-2,4,6,7-tetrahydro-5H-pyrazolo[4,3-c]pyridine-5-carboxylate 2a

Compound **1n** (1.0 g, 2.67 mmol, Bidepharm) was dissolved in acetonitrile (20 mL). Copper bromide (596 mg, 2.67 mmol) was added. The reaction solution was cooled to 0°C. Isoamyl nitrite (469 mg, 4.00 mmol) was dropwise added. After stirring at 0°C for 0.5 hours, the mixture was heated to 60°C, and stirring was continued for 5 hours. The reaction solution was cooled to room temperature and concentrated under reduced pressure. The residue was purified by silica gel column chromatography with eluent system B to obtain the title compound **2a** (350 mg, yield: 29.9%).

MS m/z (ESI): 438.3 [M+1].

### Step 2

### Tert-butyl (S)-2-(4-fluoro-3,5-dimethylphenyl)-4-methyl-3-(2-oxo-1,2-dihydropyridin-3-yl)-2,4,6,7-tetrahydro-5H-pyrazolo[4,3-c]pyridine-5-carboxylate 2b

1,2-Dihydro-2-oxo-pyridin-3-ylboronic acid (317 mg, 2.28 mmol, WuXi AppTec), compound **2a** (500 mg, 1.14 mmol), sodium carbonate (363 mg, 3.42 mmol), and tetrakis(triphenylphosphine)palladium (132 mg, 0.11 mmol) were dissolved in toluene (3 mL) and methanol (3 mL). The reaction solution was purged with nitrogen for 3 minutes, followed by a reaction under microwave at 110°C for 2 hours. The reaction solution was cooled to room temperature and concentrated under reduced pressure. The residue was purified by silica gel column chromatography with eluent system A to obtain the title compound **2b** (100 mg, yield: 19.4%).

MS m/z (ESI): 453.1 [M+1].

### Step 3

### (S)-3-(2-(4-fluoro-3,5-dimethylphenyl)-4-methyl-4,5,6,7-tetrahydro-2H-pyrazolo[4,3-c]pyridin-3-yl)pyridin-2(1H)-one 2c

Compound **2b** (25 mg, 0.055 mmol) was dissolved in dichloromethane (1 mL). A 4M solution of hydrogen chloride in dioxane (1 mL) was added. The reaction was stirred at room temperature for 1 hour. The reaction solution was concentrated under reduced pressure to obtain the crude title compound **2c** (20 mg). The product was directly used in the next reaction without purification.

MS m/z (ESI): 353.0 [M+1].

### Step 4

### 3-((1S,2S)-1-(5-((S)-2,2-dimethyltetrahydro-2H-pyran-4-yl)-2-((S)-2-(4-fluoro-3,5-dimethylphenyl)-4-methyl-3-(2-oxo-1,2-dihydropyridin-3-yl)-4,5,6,7-tetrahydro-2H-pyrazolo[4,3-c]pyridine-5-carbonyl)-1H-indol-1-yl)-2-methylcyclopropyl)-1,2,4-oxadiazol-5(4H)-one 2

Crude compound **2c** (20 mg, 0.057 mmol) and compound **1w** (20 mg, 0.049 mmol) were dissolved in *N*,*N*-dimethylformamide (3 mL). *N,N-*diisopropylethylamine (32 mg, 0.25 mmol) was added. After the raw materials were fully dissolved, *O*-(7-azabenzotriazol-1-yl)-*N*,*N*,*N'*,*N'*-tetramethyluronium hexafluorophosphate (56 mg, 0.15 mmol) was added and stirred at room temperature for 16 hours. The reaction solution was filtered, and the filtrate was collected and purified by preparative high-performance liquid chromatography (instrument model: Waters-2545 chromatographic column: Sharpsil-T, Prep 30 × 150 mm; 5 µm; C18 mobile phase: water (10 mM ammonium acetate) and acetonitrile, with gradient ratio: acetonitrile 27%-47%, flow rate: 30 mL/min) to obtain the title compound **2** (5 mg, yield: 13.8%).

MS m/z (ESI): 746.7 [M+1].

¹H NMR (500 MHz, CD₃OD): δ 7.62-7.42 (m, 3H), 7.39-7.17 (m, 2H), 7.11-6.95 (m, 2H), 6.91-6.74 (m, 1H), 6.45-6.16 (m, 1H), 5.90-5.30 (m, 2H), 4.67-4.38 (m, 2H), 3.93-3.76 (m, 2H), 3.71-3.43 (m, 2H), 3.25-2.88 (m, 3H), 2.33-2.14 (m, 6H), 1.85-1.44 (m, 7H), 1.42-1.32 (m, 5H), 1.27 (s, 2H), 1.19 (d, 1H), 1.06 (d, 1H).

### Example 3

### 3-((1S,2S)-1-(5-((S)-2,2-dimethyltetrahydro-2H-pyran-4-yl)-2-((S)-2-(4-fluoro-3,5-dimethylphenyl)-4-methyl-3-(1-(1-methyl-1H-indazol-5-yl)-2-oxo-1,2-dihydropyridin-3-yl)-4,5,6,7-tetrahydro-2H-pyrazolo[4,3-c]pyridine-5-carbonyl)-1H-indol-1-yl)-2-methylcyclopropyl)-1,2,4-oxadiazol-5(4H)-one 3

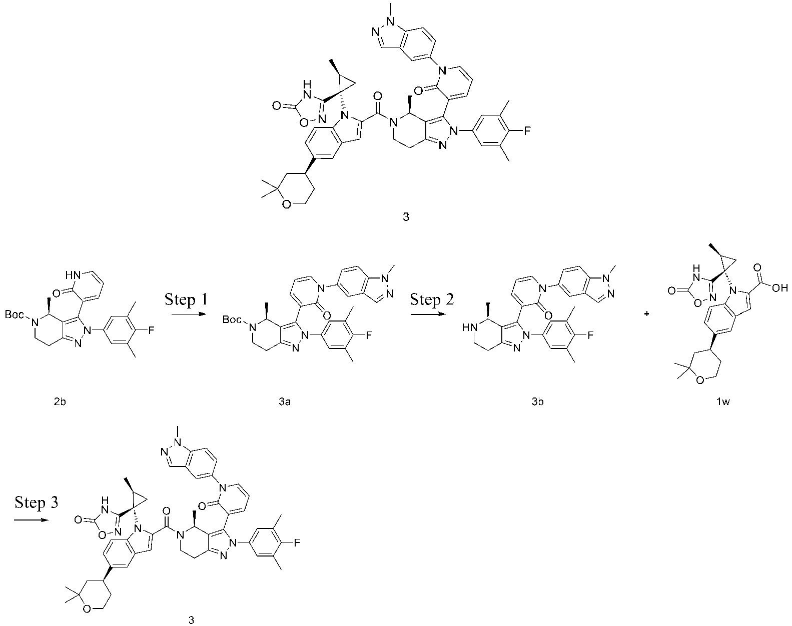

### Step 1

### Tert-butyl (S)-2-(4-fluoro-3,5-dimethylphenyl)-4-methyl-3-(1-(1-methyl-1H-indazol-5-yl)-2-oxo-1,2-dihydropyridin-3-yl)-2,4,6,7-tetrahydro-5H-pyrazolo[4,3-c]pyridine-5-carboxylate 3a

Compound **2b** (50 mg, 0.11 mmol), 1-methylindazole-5-boronic acid (60 mg, 0.34 mmol, Leyan), pyridine (100 mg, 1.26 mmol), and copper acetate (50 mg, 0.25 mmol) were dissolved in 1,2-dichloroethane (2 mL), left open, and stirred at room temperature for 16 hours. The reaction solution was concentrated under reduced pressure. The residue was purified by silica gel column chromatography with eluent system A to obtain the title compound **3a** (35 mg, yield: 54.4%).

MS m/z (ESI): 583.1 [M+1].

### Step 2

### (S)-3-(2-(4-fluoro-3,5-dimethylphenyl)-4-methyl-4,5,6,7-tetrahydro-2H-pyrazolo[4,3-c]pyridin-3-yl)-1-(1-methyl-1H-indazol-5-yl)pyridin-2(1H)-one 3b

Compound **3a** (35 mg, 60.0687 µmol) was dissolved in dichloromethane (2 mL). A 4M solution of hydrogen chloride in dioxane (2 mL) was added. The reaction was stirred at room temperature for 1 hour. The reaction solution was concentrated under reduced pressure to obtain the crude title compound **3b** (29 mg). The product was directly used in the next reaction without purification.

MS m/z (ESI): 483.1 [M+1].

### Step 3

### 3-((1S,2S)-1-(5-((S)-2,2-dimethyltetrahydro-2H-pyran-4-yl)-2-((S)-2-(4-fluoro-3,5-dimethylphenyl)-4-methyl-3-(1-(1-methyl-1H-indazol-5-yl)-2-oxo-1,2-dihydropyridin-3-yl)-4,5,6,7-tetrahydro-2H-pyrazolo[4,3-c]pyridine-5-carbonyl)-1H-indol-1-yl)-2-methylcyclopropyl)-1,2,4-oxadiazol-5(4H)-one 3

Crude compound **3b** (29 mg, 0.073mmol) and compound **1w** (30 mg, 0.12 mmol) were dissolved in *N*,*N*-dimethylformamide (3 mL). *N,N-*diisopropylethylamine (50 mg, 0.39 mmol) was added. After the raw materials were fully dissolved, *O*-(7-azabenzotriazol-1-yl)-*N*,*N*,*N'*,*N*'-tetramethyluronium hexafluorophosphate(84 mg, 0.22 mmol) was added and stirred at room temperature for 4 hours. The reaction solution was filtered, and the filtrate was collected and purified by preparative high-performance liquid chromatography (instrument model: Waters-2545 chromatographic column: Welch Xtimate, Prep 30 × 250 mm; 5 µm; C18 mobile phase: water (10 mM ammonium bicarbonate) and acetonitrile, with gradient ratio: acetonitrile 40%-70%, flow rate: 30 mL/min) to obtain the title compound **3** (15 mg, yield: 23.5%).

MS m/z (ESI): 876.7 [M+1].

¹H NMR (500 MHz, CDCl₃): δ 8.07-7.89 (m, 1H), 7.77-7.26 (m, 7H), 7.16-6.92 (m, 2H), 6.89-6.52 (m, 2H), 6.37-6.14 (m, 1H), 5.79 (q, 1H), 5.29-5.15 (m, 2H), 4.86-4.36 (m, 1H), 4.22-4.01 (m, 3H), 3.92-3.78 (m, 2H), 3.62-3.41 (m, 1H), 3.22-2.93 (m, 3H), 2.39-2.13 (m, 6H), 2.05-1.71(m, 5H), 1.49-1.44 (m, 2H), 1.37-1.26 (m, 8H), 1.18 (d, 2H), 0.98 (d, 1H).

### Example 4

### 3-((1S,2S)-1-(9-((S)-3-(3-(4-fluoro-1-methyl-1H-indazol-5-yl)-2-oxo-2,3-dihydro-1H-imidazol-1-yl)-2-(4-fluoro-3,5-dimethylphenyl)-4-methyl-4,5,6,7-tetrahydro-2H-pyrazolo[4,3-c]pyridine-5-carbonyl)-1,4a,5,11b-tetrahydro-4H-pyrano[4',3':4,5]pyrano[3,2-f]indol-8(2H)-yl)-2-methylcyclopropyl)-1,2,4-oxadiazol-5(4H)-one 4

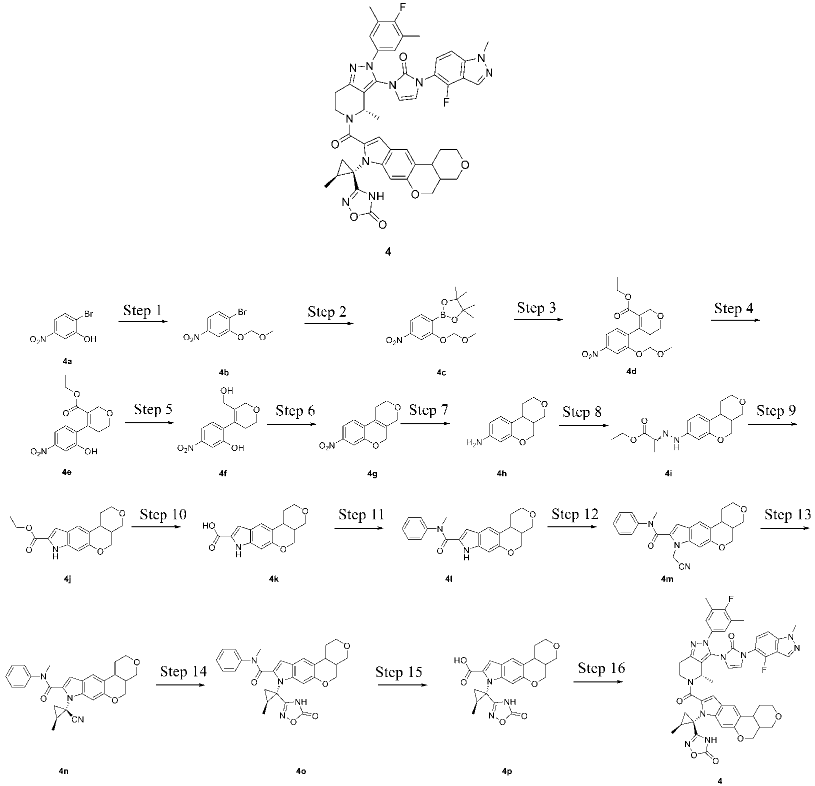

### Step 1

### 1-Bromo-2-(methoxymethoxy)-4-nitrobenzene 4b

2-Bromo-5-nitrophenol **4a** (13 g, 59.63 mmol, Shanghai Bidepharm) and *N,N-*diisopropylethylamine (23.12 g, 178.89 mmol) were dissolved in dichloromethane (100 mL). Bromomethyl methyl ether (8.19 g, 69.59 mmol, Shaoyuan Technology (Shanghai) Co., Ltd.) was dropwise added and the mixture was reacted at room temperature for 16 hours. The reaction mixture was quenched with water and extracted with dichloromethane (100 mL × 3). The organic phase was dried over anhydrous sodium sulfate. After filtration, the filtrate was concentrated under reduced pressure. The residue was purified by silica gel column chromatography with eluent system B to obtain the title compound **4b** (15 g, yield: 95%).

MS m/z (ESI): 262.1 [M+1].

### Step 2

### 2-(2-(Methoxymethoxy)-4-nitrophenyl)-4,4,5,5-tetramethyl-1,3,2-dioxaborolane 4c

Compound **4b** (8.1 g, 30.909 mmol) and bis(pinacolato)diboron (11.78 g, 46.37 mmol) were dissolved in 1,4-dioxane (100 mL). [1,1-Bis(diphenylphosphino)ferrocene]palladium dichloride (2.26 g, 3.09 mmol) and potassium acetate (9.1 g, 92.72 mmol) were separately added. Under nitrogen protection, the mixture was heated to 95°C and reacted for 16 hours. The reaction solution was cooled to room temperature, filtered, and concentrated under reduced pressure. The residue was purified by silica gel column chromatography with eluent system B to obtain the title compound **4c** (8 g, yield: 83%).

MS m/z (ESI): 310.1 [M+1].

### Step 3

### Ethyl 4-(2-(methoxymethoxy)-4-nitrophenyl)-5,6-dihydro-2H-pyran-3-carboxylate 4d

Compound **4c** (8.0 g, 25.88 mmol) and ethyl 4-(((trifluoromethyl)sulfonyl)oxy)-5,6-dihydro-2*H*-pyran-3-carboxylate (7.878 g, 25.884 mmol, preapred by a method disclosed for compound 0025 in step 2 of an example on page 93 in the description of Patent Application WO 2017/103824 A1) were dissolved in toluene (100 mL) and water (30 mL). Tetrakis(triphenylphosphine)palladium (2.99 g, 2.59 mmol) and sodium carbonate (8.23 g, 77.64 mmol) were added and placed under nitrogen protection. The mixture was heated to 100°C and reacted under stirring for 16 hours. The reaction solution was cooled to room temperature. Water was added. The mixture was extracted with ethyl acetate (100 mL × 3). The organic phase was dried over anhydrous sodium sulfate. After filtration, the filtrate was concentrated under reduced pressure. The residue was purified by silica gel column chromatography with eluent system B to obtain the title compound **4d** (7.0 g, yield: 80%).

MS m/z (ESI): 338.3 [M+1].

### Step 4

### Ethyl 4-(2-hydroxy-4-nitrophenyl)-5,6-dihydro-2H-pyran-3-carboxylate 4e

Compound **4d** (6.0 g, 17.79 mmol) was dissolved in ethanol (50 mL) and tetrahydrofuran (10 mL). A 4 M solution of hydrogen chloride in 1,4-dioxane (10 mL) was dropwise added at room temperature and reacted under stirring for 6 hours. The reaction solution was concentrated under reduced pressure to obtain the crude title compound **4e** (3.7 g). The product was directly used in the next reaction without purification.

MS m/z (ESI): 294.2 [M+1].

### Step 5

### 2-(5-(Hydroxymethyl)-3,6-dihydro-2H-pyran-4-yl)-5-nitrophenol 4f

Compound **4e** (3.7 g, 12.616 mmol) was dissolved in tetrahydrofuran (10 mL). The mixture was cooled to -70°C. A solution of diisobutylaluminum hydride at 1 mol/L in n-hexane (6.27 g, 44.16 mmol, 44.16 mL) was dropwise added. After the dropwise addition was complete, the mixture was slowly heated to room temperature and reacted under stirring for 2 hours. After cooling under ice bath condition, the reaction solution was quenched with potassium sodium tartrate tetrahydrate and stirred for 1 hour. After filtration and concentration under reduced pressure, the residue was purified by silica gel column chromatography with eluent system B to obtain the title compound **4f** (2.1 g, yield: 66%).

MS m/z (ESI): 252.3 [M+1].

### Step 6

### 8-Nitro-1,5-dihydro-2H,4H-pyrano[3,4-c]chromene 4g

Compound **4f** (2.1 g, 8.36 mmol) was dissolved in toluene (50 mL). Cyanomethylenetri-n-butylphosphorane (4.04 g, 16.72 mmol, Jiangsu Aikon) was dropwise added. After the dropwise addition was complete, the mixture was heated to 100°C and reacted under stirring for 3 hours. The reaction mixture was cooled to room temperature. Water was added. The mixture was extracted with ethyl acetate (50 mL × 3). The organic phase was dried over anhydrous sodium sulfate. After filtration, the filtrate was concentrated under reduced pressure. The residue was purified by silica gel column chromatography with eluent system B to obtain the title compound **4g** (1.6 g, yield: 82%).

MS m/z (ESI): 234.2 [M+1].

### Step 7

### 1,4a,5,10b-Tetrahydro-2H,4H-pyrano[3,4-c]chromen-8-amine 4h

Compound **4g** (1.6 g, 6.86 mmol) was dissolved in ethanol (20 mL) and tetrahydrofuran (20 mL). Palladium on carbon (0.731 g, 0.69 mmol) was added. After displacement with hydrogen three times, the mixture was reacted under stirring at room temperature in a hydrogen atmosphere for 16 hours. After filtration, the filtrate was concentrated under reduced pressure to obtain the crude title compound **4h** (1.3 g). The product was directly used in the next reaction without purification.

MS m/z (ESI): 206.2 [M+1].

### Step 8

### Ethyl 2-(2-(1,4a,5,10b-tetrahydro-2H,4H-pyrano[3,4-c]chromen-8-yl)hydrazono)propanoate 4i

Compound **4h** (1.3 g, 6.33 mmol) was dissolved in acetonitrile (16 mL). The mixture was cooled to 0°C under ice bath condition. A solution of 6 M hydrogen chloride in water (5.3 mL) was dropwise added. After the dropwise addition was complete, the mixture was stirred for 10 minutes. Sodium nitrite (0.87 g, 12.65 mmol) was dissolved in water (10 mL) and dropwise added to the reaction. The reaction mixture was reacted under stirring at 0°C for 1 hour. Ethyl 2-methylacetoacetate (1.0 g, 6.97 mmol) was dissolved in ethanol (16 mL). The mixture was cooled to 0°C under ice bath condition. Sodium acetate (5.20 g, 63.34 mmol) was added and stirred for 1 hour. The two reaction solutions were combined and mixed under stirring at room temperature for 2 hours. The mixture was neutralized to neutrality with sodium carbonate and extracted with dichloromethane (50 mL × 3). The organic phase was dried over anhydrous sodium sulfate. After filtration, the filtrate was concentrated under reduced pressure. The residue was purified by silica gel column chromatography with eluent system B to obtain an intermediate, which was dissolved in ethanol (20 mL). A 4 M solution of hydrogen chloride in 1,4-dioxane (1 mL) was dropwise added and stirred at room temperature for 2 hours. The reaction solution was concentrated under reduced pressure to obtain the crude title compound **4i** (2 g). The product was directly used in the next reaction without purification.

MS m/z (ESI): 319.3 [M+1].

### Step 9

### Ethyl 1,2,4a,5,8,11b-hexahydro-4H-pyrano[4',3':4,5]pyrano[3,2-f]indole-9-carboxylate 4j

Compound **4i** (2 g, 6.28 mmol) was dissolved in dichloromethane (50 mL). Eaton's reagent (2.99 g, 12.56 mmol) was added. The mixture was heated to 50°C and reacted under stirring for 3 hours. The reaction mixture was quenched with water, neutralized to neutrality with a sodium bicarbonate solution, and extracted with dichloromethane (50 mL × 3). The organic phase was dried over anhydrous sodium sulfate. After filtration, the filtrate was concentrated under reduced pressure. The residue was purified by silica gel column chromatography with eluent system B to obtain the title compound **4j** (900 mg, yield: 47%).

MS m/z (ESI): 302.3 [M+1].

### Step 10

### 1,2,4a,5,8,11b-Hexahydro-4H-pyrano[4',3':4,5]pyrano[3,2-f]indole-9-carboxylic acid 4k

Compound **4j** (900 mg, 2.99 mmol) was dissolved in methanol (10 mL) and water (1 mL). Sodium hydroxide (598 mg, 14.95 mmol) was added. The mixture was heated to 65°C and reacted under stirring for 3 hours. The reaction mixture was quenched with water and adjusted to pH = 3 with a dilute hydrochloric acid solution. A solid was precipitated. After filtration, the solid was dried to obtain the crude title compound **4k** (700 mg). The product was directly used in the next reaction without purification.

MS m/z (ESI): 274.2 [M+1].

### Step 11

### N-methyl-N-phenyl-1,2,4a,5,8,11b-hexahydro-4H-pyrano[4',3":4,5]pyrano[3,2-f]indole-9-carboxamide 4l

Compound **4k** (300 mg, 1.10 mmol) was dissolved in *N*,*N*-dimethylacetamide (10 mL). Under ice bath condition, sulfoxide chloride (458 mg, 3.85 mmol) was added and stirred at room temperature for 5 hours. Triethylamine (666 mg, 6.58 mmol) and N-methylaniline (236 mg, 2.20 mmol) were added and reacted under stirring at room temperature for 16 hours. The reaction mixture was quenched with water and extracted with ethyl acetate (30 mL × 3). The organic phase was dried over anhydrous sodium sulfate. After filtration, the filtrate was concentrated under reduced pressure. The residue was purified by silica gel column chromatography with eluent system B to obtain the title compound **41** (200 mg, yield: 50%).

MS m/z (ESI): 363.4 [M+1].

### Step 12

### 8-(Cyanomethyl)-N-methyl-N-phenyl-1,2,4a,5,8,11b-hexahydro-4H-pyrano[4',3':4,5]pyrano[3,2-f]indole-9-carboxamide 4m

Compound **4l** (300 mg, 0.83 mmol) was dissolved in *N*,*N*-dimethylformamide (10 mL). Under ice bath condition, sodium hydride (332 mg, 8.28 mol, 60% wt) was added and stirred at room temperature for 5 hours. Chloroacetonitrile (625 mg, 8.28 mmol) was added and reacted under stirring at room temperature for 3 hours. The reaction mixture was quenched with water and extracted with ethyl acetate (30 mL × 3). The organic phase was dried over anhydrous sodium sulfate. After filtration, the filtrate was concentrated under reduced pressure. The residue was purified by silica gel column chromatography with eluent system B to obtain the title compound **4m** (200 mg, yield: 60%).

MS m/z (ESI): 402.1 [M+1].

### Step 13

### 8-((1S,2S)-1-cyano-2-methylcyclopropyl)-N-methyl-N-phenyl-1,2,4a,5,8,11b-hexahydro-4H-pyrano[4',3':4,5]pyrano[3,2-f]indole-9-carboxamide 4n

Compound **4m** (300 mg, 0.75 mmol) and (4*R*)-4-methyl-1,3,2-dioxathiolane 2,2-dioxide (362 mg, 0.83 mmol) were dissolved in *N*,*N*-dimethylpropyleneurea (3.832 g, 29.90 mmol). Under ice bath condition, potassium bis(trimethylsilyl)amide (1.19 g, 5.98 mmol, a 1M tetrahydrofuran solution) was added and reacted under stirring at room temperature for 3 hours. The reaction mixture was quenched with water and extracted with ethyl acetate (50 mL × 3). The organic phase was dried over anhydrous sodium sulfate. After filtration, the filtrate was concentrated under reduced pressure. The residue was purified by silica gel column chromatography with eluent system B to obtain the title compound **4n** (170 mg, yield: 51%).

MS m/z (ESI): 442.5 [M+1].

### Step 14

### N-methyl-8-((1S,2S)-2-methyl-1-(5-oxo-4,5-dihydro-1,2,4-oxadiazol-3-yl)cyclopropyl)-N-phenyl-1,2,4a,5,8,11b-hexahydro-4H-pyrano[4',3':4,5]pyrano[3,2-f]indole-9-carboxamide 4o

Compound **4n** (170 mg, 0.39 mmol) and hydroxylamine hydrochloride (267 mg, 3.84 mmol) were dissolved in dimethyl sulfoxide (5 mL). Sodium bicarbonate (323 mg, 3.84 mmol) was added. The mixture was heated to 60°C and reacted under stirring for 3.5 hours. The reaction mixture was cooled to room temperature, quenched with water, and extracted with ethyl acetate (30 mL × 3). The organic phase was dried over anhydrous sodium sulfate. After filtration, the filtrate was concentrated under reduced pressure. The residue was dissolved in dimethyl sulfoxide (5 mL). *N*,*N'*-carbonyldiimidazole (166 mg, 1.15 mmol) and 1,8-diazabicyclo[5,4,0]undec-7-ene (387 mg, 1.54 mmol) were added and reacted under stirring at room temperature for 3 hours. The reaction mixture was quenched with water and extracted with ethyl acetate (30 mL × 3). The organic phase was dried over anhydrous sodium sulfate. After filtration, the filtrate was concentrated under reduced pressure. The residue was purified by silica gel column chromatography with eluent system B to obtain the title compound **4o** (100 mg, yield: 51%).

MS m/z (ESI): 501.5 [M+1].

### Step 15

### 8-((1S,2S)-2-methyl-1-(5-oxo-4,5-dihydro-1,2,4-oxadiazol-3-yl)cyclopropyl)-1,2,4a,5,8,11b-hexahydro-4H-pyrano[4',3":4,5pyrano[3,2-f]indole-9-carboxylic acid 4p

Compound **4o** (50 mg, 0.099 mmol) and potassium hydroxide (561 mg, 10.00 mmol) were dissolved in ethylene glycol monomethyl ether (5 mL) and water (1 mL). The mixture was heated to 120°C and reacted under stirring for 60 hours. The reaction mixture was cooled to room temperature, quenched with water, adjusted to pH = 3 with 1M dilute hydrochloric acid, and extracted with ethyl acetate (30 mL × 3). The organic phase was dried over anhydrous sodium sulfate. After filtration, the filtrate was concentrated under reduced pressure. The residue was purified by silica gel column chromatography with eluent system A to obtain the title compound **4p** (10 mg, yield: 24%).

MS m/z (ESI): 412.4 [M+1].

### Step 16

### 3-((1S,2S)-1-(9-((S)-3-(3-(4-fluoro-1-methyl-1H-indazol-5-yl)-2-oxo-2,3-dihydro-1H-imidazol-1-yl)-2-(4-fluoro-3,5-dimethylphenyl)-4-methyl-4,5,6,7-tetrahydro-2H-pyrazolo[4,3-c]pyridine-5-carbonyl)-1,4a,5,11b-tetrahydro-4H-pyrano[4',3':4,5]pyrano[3,2-f]indol-8(2H)-yl)-2-methylcyclopropyl)-1,2,4-oxadiazol-5(4H)-one 4

Compound **4p** (10 mg, 0.024 mmol) and (*S*)-1-(4-fluoro-1-methyl-1*H*-indazol-5-yl)-3-(2-(4-fluoro-3,5-dimethylphenyl)-4-methyl-4,5,6,7-tetrahydro-2*H-*pyrazolo[4,3-*c*]pyridin-3-yl)-1,3-dihydro-2*H*-imidazol-2-one hydrochloride (12 mg, 0.024 mmol, prepared by a method disclosed for compound 00412 in an example on page 139 of the description of Patent Application WO 2022017338 A1) were dissolved in *N*,*N*-dimethylformamide (2 mL). 2-(7-Azabenzotriazole)-*N*,*N*,*N'*,*N'-*tetramethyluronium hexafluorophosphate(11 mg, 0.026 mmol) and *N,N-*diisopropylethylamine (10 mg, 0.073 mmol) were added and the mixture was reacted at room temperature for 16 hours. The reaction solution was concentrated under reduced pressure. The residue was purified by preparative high-performance liquid chromatography (instrument model: Waters 2489&SQD2, chromatographic column: Welch Xtimate, Prep 30 × 250 mm; 5 µm; C18, mobile phase: water (0.1% TFA) and acetonitrile, with gradient ratio: acetonitrile 45%-95%, flow rate: 30 mL/min) to obtain the title compound **4** (a mixture of stereoisomers, 8 mg, yield: 37%).

MS m/z (ESI): 883.4 [M+1].

¹H NMR (500 MHz, CDCl₃): δ8.19-8.02 (m, 1H), 7.52-7.49 (m, 1H), 7.38(d, 1H), 7.32- 7.29(m, 1H),7.22-7.12(m, 2H), 7.10-7.02(m, 1H), 6.68-6.61(m, 1H), 6.37-6.30 (m, 1H), 5.77 (q, 1H), 4.59-4.38 (m, 3H), 4.19-4.07 (m, 3H), 3.98-3.79 (m, 3H), 3.69-3.54 (m, 2H), 3.23-2.95 (m, 3H), 2.38-2.21 (m, 7H), 1.92 (t, 1H), 1.73-1.50 (m, 5H), 1.36 (s, 3H), 1.13 (d, 2H), 0.91 (d, 1H).

### Example 5

### 3-((1S,2S)-1-(5-((S)-2,2-dimethyltetrahydro-2H-pyran-4-yl)-2-((S)-2-(4-fluoro-3,5-dimethylphenyl)-4-methyl-3-(5-oxo-5H-imidazo[1,5-a]imidazol-6(7H)-yl)-4,5,6,7-tetrahydro-2H-pyrazolo[4,3-c]pyridine-5-carbonyl)-1H-indol-1-yl)-2-methylcyclopropyl)-1,2,4-oxadiazol-5(4H)-one 5

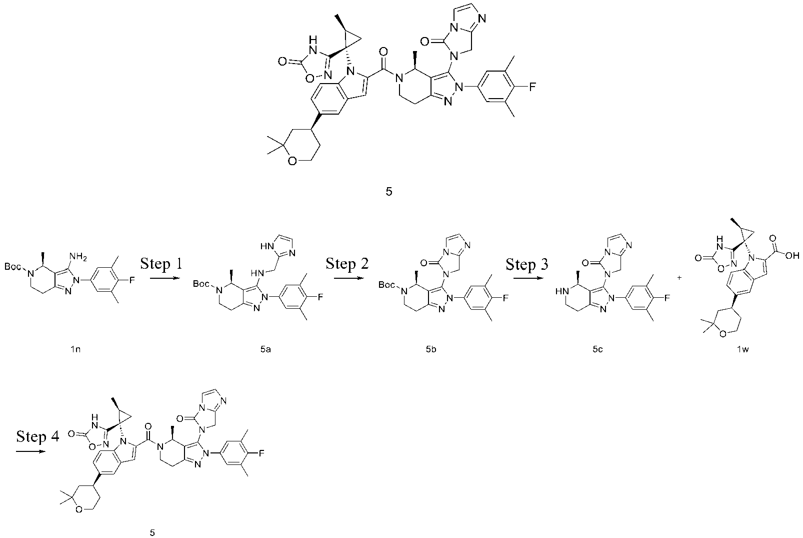

### Step 1

### Tert-butyl (S)-3-(((1H-imidazol-2-yl)methyl)amino)-2-(4-fluoro-3,5-dimethylphenyl)-4-methyl-2,4,6,7-tetrahydro-5H-pyrazolo[4,3-c]pyridine-5-carboxylate 5a

Compound **1n** (500 mg, 1.34 mmol) and 2-imidazolecarboxaldehyde (260 mg, 2.71 mmol, Adamas) were dissolved in 1,2-dichloroethane (20 mL). Tetraisopropyl titanate (1.14 g, 4.01 mmol, Adamas) was added and stirred at 80°C for 16 hours. The reaction solution was cooled to room temperature. Methanol (20 mL) was added. Sodium borohydride (102 mg, 2.70 mmol) was slowly added at room temperature. Stirring was continued for 2 hours. The reaction solution was concentrated under reduced pressure. The residue was purified by silica gel column chromatography with eluent system A to obtain the title compound 5a (500 mg, yield: 82.4%).

MS m/z (ESI): 455.0 [M+1].

### Step 2

### Tert-butyl (S)-2-(4-fluoro-3,5-dimethylphenyl)-4-methyl-3-(5-oxo-5H-imidazo[1,5-a]imidazol-6(7H)-yl)-2,4,6,7-tetrahydro-5H-pyrazolo[4,3-c]pyridine-5-carboxylate 5b

Compound **5a** (100 mg, 0.22 mmol) was dissolved in dichloromethane (5 mL). Triphosgene (33 mg, 0.11 mmol) and triethylamine (89 mg, 0.88 mmol) were successively added at room temperature and stirred at room temperature for 3 hours. The reaction solution was concentrated under reduced pressure. The residue was purified by silica gel column chromatography with eluent system A to obtain the title compound **5b** (60 mg, yield: 56.8%).

MS m/z (ESI): 481.2 [M+1].

### Step 3

### (S)-6-(2-(4-fluoro-3,5-dimethylphenyl)-4-methyl-4,5,6,7-tetrahydro-2H-pyrazolo[4,3-c]pyridin-3-yl)-6,7-dihydro-5H-imidazo[1,5-a]imidazol-5-one 5c

Compound **5b** (25 mg, 0.052 mmol) was dissolved in dichloromethane (1 mL). A 4 M solution of hydrogen chloride in 1,4-dioxane (3 mL) was added and stirred at room temperature for 1 hour. The reaction solution was concentrated under reduced pressure to obtain the crude title compound **5c** (20 mg). The product was directly used in the next reaction without purification.

MS m/z (ESI): 381.1 [M+1].

### Step 4

### 3-((1S,2S)-1-(5-((S)-2,2-dimethyltetrahydro-2H-pyran-4-yl)-2-((S)-2-(4-fluoro-3,5-dimethylphenyl)-4-methyl-3-(5-oxo-5H-imidazo[1,5-a]imidazol-6(7H)-yl)-4,5,6,7-tetrahydro-2H-pyrazolo[4,3-c]pyridine-5-carbonyl)-1H-indol-1-yl)-2-methylcyclopropyl)-1,2,4-oxadiazol-5(4H)-one 5

Crude compound **5c** (20 mg, 0.053 mmol) and compound **1w** (25 mg, 0.061 mmol) were dissolved in *N*,*N*-dimethylformamide (3 mL). *N,N-*diisopropylethylamine (40 mg, 0.30 mmol) was added. After the raw materials were fully dissolved, *O*-(7-azabenzotriazol-1-yl)-*N*,*N*,*N*',*N*'-tetramethyluronium hexafluorophosphate (69 mg, 0.18 mmol) was added and stirred at room temperature for 16 hours. The reaction solution was filtered. The filtrate was purified by preparative high-performance liquid chromatography (instrument model: Gilson-GX-281 chromatographic column: SP-120-5-ODS-BIO, Prep 30 × 150 mm; 5 µm; C18 mobile phase: water (10 mM ammonium bicarbonate) and acetonitrile, with gradient ratio: acetonitrile 55%-80%, flow rate: 30 mL/min) to obtain the title compound **5** (18 mg, yield: 38.3%).

MS m/z (ESI): 772.6 [M-1].

¹H NMR (500 MHz, CDCl₃): δ 7.62-7.41 (m, 3H), 7.28-7.26 (m, 1H), 7.21-6.94 (m, 3H), 6.72-6.63 (m, 1H), 5.75 (q, 1H), 5.36-4.80 (m, 2H), 4.57-4.42 (m, 2H), 4.40-4.31 (m, 1H), 4.23 (q, 1H), 3.90-3.78 (m, 2H), 3.61-3.52 (m, 1H), 3.42-2.96 (m, 4H), 2.27-2.18 (m, 6H), 1.80-1.74 (m, 2H), 1.52 (d, 3H), 1.35-1.32 (m, 3H), 1.29-1.24 (m, 5H), 1.18 (d, 2H), 1.05 (d, 1H).

### Example 6

### 3-((1S,2S)-1-(2-((S)-3-(3-(4-fluoro-1-methyl-1H-indazol-5-yl)-2-oxo-2,3-dihydro-1H-imidazol-1-yl)-2-(4-fluoro-3,5-dimethylphenyl)-4-methyl-4,5,6,7-tetrahydro-2H-pyrazolo[4,3-c]pyridine-5-carbonyl)-8,8-dimethyl-5a,8,9,9a-tetrahydro-5H-pyrano[4',3':4,5]pyrano[2,3-e]indol-1(6H)-yl)-2-methylcyclopropyl)-1,2,4-oxadiazol-5(4H)-one 6

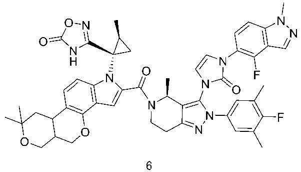

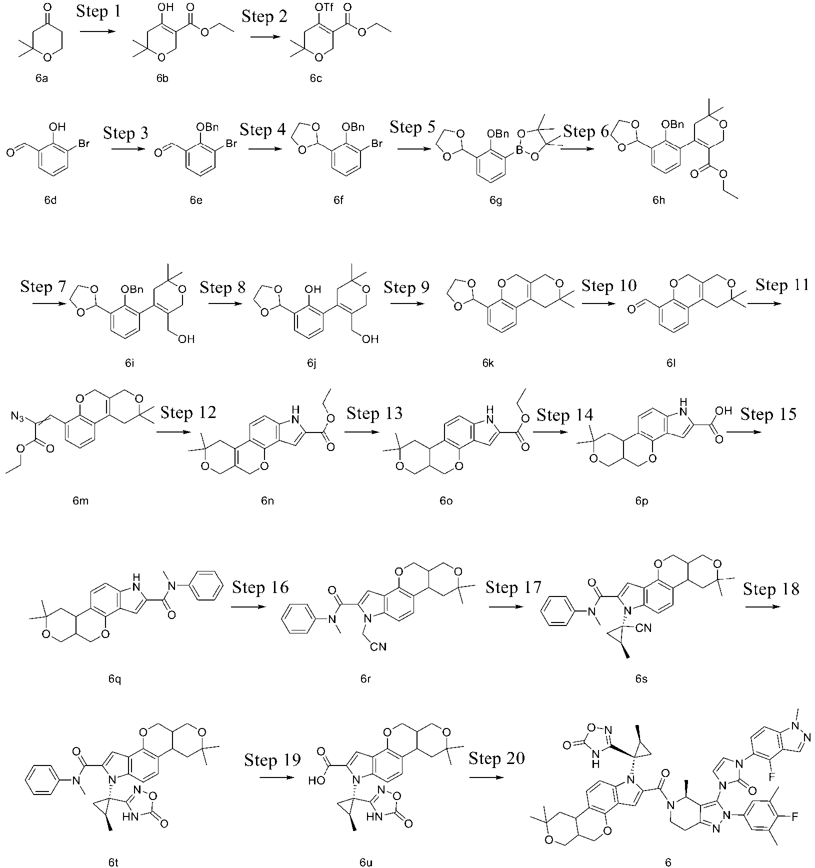

### Step 1

Ethyl 4-hydroxy-6,6-dimethyl-5,6-dihydro-2*H*-pyran-3-carboxylate **6b** 2,2-Dimethyltetrahydro-4*H*-pyran-4-one **6a** (5.0 g, 39.0 mmol, Adamas) was dissolved in tetrahydrofuran (60 mL), and sodium hydride (3.9 g, 97.5 mmol, 60% wt) was added in portions at 0°C and stirred for 0.5 hours. Diethyl carbonate (11.5 g, 97.3 mmol) was dropwise added. After the addition was complete, the mixture was reacted at 65°C for 3 hours. The reaction solution was cooled to room temperature, poured into 100 mL of ice water, quenched with a saturated aqueous ammonium chloride solution, extracted with ethyl acetate (100 mL × 2), and washed with a saturated sodium chloride solution. The organic phase was dried over anhydrous sodium sulfate. After filtration, the filtrate was concentrated under reduced pressure. The residue was purified by silica gel column chromatography with eluent system C to obtain the title compound **6b** (4.68 g, yield: 59.9%).

MS m/z (ESI): 201.1 [M+1].

### Step 2

### Ethyl 6,6-dimethyl-4-(((trifluoromethyl)sulfonyl)oxy)-5,6-dihydro-2H-pyran-3-carboxylate 6c

Compound **6b** (3.6 g, 18.0 mmol) and *N*,*N*-diisopropylethylamine (7.0 g, 54.2 mmol) were dissolved in dichloromethane (50 mL). Trifluoromethanesulfonic anhydride (7.6 g, 26.9 mmol) was dropwise added at -70°C. After the addition was complete, the mixture was slowly heated to room temperature. The reaction solution was quenched with water (50 mL), extracted with dichloromethane (50 mL × 2), and washed with a saturated sodium chloride solution. The organic phase was dried over anhydrous sodium sulfate. After filtration, the filtrate was concentrated under reduced pressure. The residue was purified by silica gel column chromatography with eluent system C to obtain the title compound **6c** (4.37 g, yield: 73.1%).

MS m/z (ESI): 333.1 [M+1].

### Step 3

### 2-Benzyloxy-3-bromobenzaldehyde 6e

2-Hydroxy-3-bromobenzaldehyde **6d** (7.0 g, 34.8 mmol, Shanghai Bidepharm) and benzyl bromide (6.3 g, 36.8 mmol) were dissolved in *N*,*N*-dimethylformamide (70 mL). Potassium carbonate (10 g, 71.9 mmol) was added and the mixture was reacted at room temperature for 16 hours. The reaction mixture was poured into ice water (100 mL) and extracted with ethyl acetate (100 mL × 3). The organic phases were combined and washed with a saturated sodium chloride solution. The organic phase was dried over anhydrous sodium sulfate. After filtration, the filtrate was concentrated under reduced pressure. The residue was stirred with eluent system C to obtain the title compound **6e** (10 g, yield: 98.6%).

MS m/z (ESI): 291.2 [M+1].

### Step 4

### 2-(2-(Benzyloxy)-3-bromophenyl)-1,3-dioxolane 6f

Compound **6e** (3.0 g, 10.3 mmol) and p-toluenesulfonic acid monohydrate (200 mg, 1.05 mmol) were dissolved in toluene (40 mL). A water separator was added. The mixture was reacted at 120°C for 16 hours. The reaction mixture was neutralized with a saturated sodium bicarbonate solution, extracted with ethyl acetate (50 mL × 3), and dried over anhydrous sodium sulfate. After filtration, the filtrate was concentrated under reduced pressure. The residue was purified by silica gel column chromatography with eluent system C to obtain the title compound **6f** (3.0 g, yield: 86.8%).

MS m/z (ESI): 335.0 [M+1].

### Step 5

### 2-(2-Benzyloxy-3-(1,3-dioxolan-2-yl)phenyl)-4,4,5,5-tetramethyl-1,3,2-dioxaborolane 6g

Compound **6f** (2.9 g, 8.65 mmol), potassium acetate (2.5 g, 25.5 mmol), bis(pinacolato)diboron (2.8 g, 11.0 mmol, Shaoyuan Technology (Shanghai) Co., Ltd.), and [1,1'-bis(diphenylphosphino)ferrocene]palladium dichloride (630 mg, 0.86 mmol, Shanghai Bidepharm) were dissolved in dioxane (40 mL). After displacement with nitrogen three times, the mixture was reacted at 85°C for 16 hours. Water (100 mL) was added. The mixture was extracted with ethyl acetate (100 mL × 3). The organic phases were combined and washed with a saturated sodium chloride solution. The organic phase was dried over anhydrous sodium sulfate. After filtration, the filtrate was concentrated under reduced pressure. The residue was purified by silica gel column chromatography with eluent system C to obtain the title compound **6g** (2.5 g, yield: 75.6%).

MS m/z (ESI): 383.1 [M+1].

### Step 6

### Ethyl 4-(2-(benzyloxy)-3-(1,3-dioxolan-2-yl)phenyl)-6,6-dimethyl-5,6-dihydro-2H-pyran-3-carboxylate 6h

Compound **6g** (1.0 g, 2.62 mmol) and **6c** (1.0 g, 3.01 mmol) were dissolved in dioxane (10 mL) and water (2.0 mL). Sodium carbonate (880 mg, 8.00 mmol) and [1,1'-bis(diphenylphosphino)ferrocene]palladium dichloride (190 mg, 0.26 mmol, Shanghai Bidepharm) were added. After displacement with nitrogen three times, the mixture was reacted at 90°C for 16 hours. Water (30 mL) was added. The mixture was extracted with ethyl acetate (20 mL × 3). The organic phases were combined and washed with a saturated sodium chloride solution. The organic phase was dried over anhydrous sodium sulfate. After filtration, the filtrate was concentrated under reduced pressure. The residue was purified by silica gel column chromatography with eluent system C to obtain the title compound **6h** (760 mg, yield: 66.3%).

MS m/z (ESI): 439.3 [M+1].

### Step 7

### (4-(2-(Benzyloxy)-3-(1,3-dioxolan-2-yl)phenyl)-6,6-dimethyl-5,6-dihydro-2H-pyran-3-yl)methanol 6i

Compound **6h** (760 mg, 1.73 mmol) was dissolved in tetrahydrofuran (10mL). A diisobutylaluminum hydride/n-hexane solution (4.3 mL, 4.3 mmol, 1.0 M) was dropwise added at 0°C and stirred at 0°C for 1 hour. The reaction was quenched with a potassium sodium tartrate tetrahydrate solid and stirred for 1 hour. After filtration, the filtrate was concentrated under reduced pressure. The residue was purified by silica gel column chromatography with eluent system C to obtain the title compound **6i** (640 mg, yield: 93.1%).

MS m/z (ESI): 397.2 [M+1].

### Step 8

### 2-(1,3-Dioxolan-2-yl)-6-(5-(hydroxymethyl)-2,2-dimethyl-3,6-dihydro-2H-pyran-4-yl)phenol 6j

Compound **6i** (4.5 g, 11.4 mmol) was dissolved in tetrahydrofuran (50 mL). Palladium hydroxide (1.0 g, 7.12 mmol, Adamas) was added and reacted under stirring in a hydrogen atmosphere at room temperature for 3 hours. After filtration, the filtrate was concentrated under reduced pressure. The residue was purified by silica gel column chromatography with eluent system C to obtain the title compound **6j** (2.6 g, yield: 74.7%).

MS m/z (ESI): 307.3 [M+1].

### Step 9

### 7-(1,3-Dioxolan-2-yl)-2,2-dimethyl-1,5-dihydro-2H,4H-pyrano[3,4-c]chromene 6k

Compound **6j** (2.6 g, 8.49 mmol) and azodicarbonyl dipiperidine (4.3 g, 17.0 mmol, Adamas) were dissolved in toluene (150 mL). Tributylphosphine (3.4 g, 16.8 mmol, Adamas) was added. After displacement with nitrogen three times, the mixture was stirred at 50°C for 1 hour. After concentration under reduced pressure, the residue was purified by silica gel column chromatography with eluent system C to obtain the title compound **6k** (2.25 g, yield: 91.9%).

MS m/z (ESI): 289.3 [M+1].

### Step 10

### 2,2-Dimethyl-1,5-dihydro-2H,4H-pyrano[3,4-c]chromene-7-carbaldehyde 6l

Compound **6k** (2.1 g, 7.28 mmol) was dissolved in tetrahydrofuran (30 mL). Concentrated hydrochloric acid (3.0 mL) was added and stirred at room temperature for 0.5 hours. The reaction solution was poured into 30 mL of water and extracted with ethyl acetate (20 mL × 2). The organic phases were combined, washed with a saturated sodium chloride solution, and dried over anhydrous sodium sulfate. After filtration, the filtrate was concentrated under reduced pressure. The residue was purified by silica gel column chromatography with eluent system C to obtain the title compound **6l** (1.6 g, yield: 89.9%).

MS m/z (ESI): 245.3 [M+1].

### Step 11

### Ethyl 2-azido-3-(2,2-dimethyl-1,5-dihydro-2H,4H-pyrano[3,4-c]chromen-7-yl)acrylate 6m

Compound **6l** (1.6 g, 6.55 mmol) and ethyl azidoacetate (2.6 g, 20.1 mmol) were dissolved in anhydrous ethanol (20 mL). A sodium ethoxide/ethanol solution (7.0 g, 20.5 mmol, 20% wt, Adamas) was dropwise added at -10°C. The temperature was maintained at < -5°C. After the addition was complete, the mixture was reacted under stirring at -5°C for 1 hour. The reaction solution was poured into 50 mL of ice water and extracted with ethyl acetate (50 mL × 3). The organic phases were combined, washed with a saturated sodium chloride solution, and dried over anhydrous sodium sulfate. After filtration, the filtrate was concentrated under reduced pressure. The residue was purified by silica gel column chromatography with eluent system C to obtain the title compound **6m** (a mixture of two isomers, 1.48 g, yield: 63.6%).

MS m/z (ESI): 356.2 [M+1].

### Step 12

### Ethyl 8,8-dimethyl-1,6,8,9-tetrahydro-5H-pyrano[4',3':4,5]pyrano[2,3-e]indole-2-carboxylate 6n

Compound **6m** (1.48 g, 4.16 mmol) was dissolved in xylene (30 mL). The mixture was reacted under reflux for 1 hour and concentrated under reduced pressure. The residue was purified by silica gel column chromatography with eluent system C to obtain the title compound **6n** (760 mg, yield: 55.7%).

MS m/z (ESI): 328.2 [M+1].

### Step 13

### Ethyl 8,8-dimethyl-1,5a,6,8,9,9a-hexahydro-5H-pyrano[4',3':4,5]pyrano[2,3-e]indole-2-carboxylate 6o

Compound **6n** (760 mg, 2.32 mmol) was dissolved in ethanol (10 mL). Palladium/carbon (100 mg, 10% wt) was added. The mixture was reacted in a hydrogen atmosphere at room temperature for 1 hour. After filtration, the filtrate was concentrated under reduced pressure. The residue was purified by silica gel column chromatography with eluent system C to obtain the title compound **6o** (a mixture of stereoisomers, 620 mg, yield: 81.1%).

MS m/z (ESI): 330.2 [M+1].

### Step 14

### 8,8-Dimethyl-1,5a,6,8,9,9a-hexahydro-5H-pyrano[4',3':4,5]pyrano[2,3-e]indole-2-carboxylic acid 6p

Compound **6o** (610 mg, 1.85 mmol) was dissolved in methanol (10 mL)/water (5.0 mL). Sodium hydroxide (250 mg, 6.25 mmol) was added and reacted at 65°C for 1.5 hours. The reaction solution was poured into 30 mL of ice water and adjusted to pH = 3-4 with 2M dilute hydrochloric acid. A solid precipitated out. After filtartion, the solid was dried to obtain the crude title compound **6p** (a mixture of stereoisomers, 530 mg).

MS m/z (ESI): 302.0 [M+1].

### Step 15

### N,8,8-trimethyl-N-phenyl-1,5a,6,8,9,9a-hexahydro-5H-pyrano[4',3':4,5]pyrano[2,3-e]indole-2-carboxamide 6q

Compound **6p** (500 mg, 1.66 mmol) was dissolved in *N*,*N*-dimethylacetamide (10 mL). Under ice bath condition, sulfoxide chloride (500 mg, 4.20 mmol) was added and stirred at room temperature for 5 hours. Triethylamine (840 mg, 8.30 mmol) and N-methylaniline (270 mg, 2.52 mmol) were added and reacted under stirring at room temperature for 16 hours. The reaction mixture was quenched with water and extracted with ethyl acetate (30 mL × 3). The organic phase was dried over anhydrous sodium sulfate. After filtration, the filtrate was concentrated under reduced pressure. The residue was purified by silica gel column chromatography with eluent system C to obtain the title compound **6q** (a mixture of stereoisomers, 180 mg, yield: 27.7%).

MS m/z (ESI): 391.4 [M+1].

### Step 16

### 1-(Cyanomethyl)-N,8,8-trimethyl-N-phenyl-1,5a,6,8,9,9a-hexahydro-5H-pyrano[4',3':4,5]pyrano[2,3-e]indole-2-carboxamide 6r

Compound **6q** (180 mg, 0.46 mmol) was dissolved in *N*,*N-*dimethylformamide (10 mL). Under ice bath condition, sodium hydride (190 mg, 4.75 mol, 60% wt) was added and stirred at room temperature for 5 hours. Chloroacetonitrile (360 mg, 4.77 mmol) was added and reacted under stirring at room temperature for 3 hours. The reaction mixture was quenched with water and extracted with ethyl acetate (30 mL × 3). The organic phase was dried over anhydrous sodium sulfate. After filtration, the filtrate was concentrated under reduced pressure. The residue was purified by silica gel column chromatography with eluent system C to obtain the title compound **6r** (a mixture of stereoisomers, 140 mg, yield: 70.7%).

MS m/z (ESI): 430.1 [M+1].

### Step 17

### 1-((1S,2S)-1-cyano-2-methylcyclopropyl)-N,8,8-trimethyl-N-phenyl-1,5a,6,8,9,9a-hexahydro-5H-pyrano[4',3':4,5]pyrano[2,3-e]indole-2-carboxamide 6s

Compound **6r** (140 mg, 0.33 mmol) and (4*R*)-4-methyl-1,3,2-dioxathiolane 2,2-dioxide (135 mg, 0.98 mmol) were dissolved in *N*,*N-*dimethylpropyleneurea (1.7 g, 13.3 mmol). Under ice bath condition, potassium bis(trimethylsilyl)amide (3.2 mL, 3.2 mmol, a 1M tetrahydrofuran solution) was added and reacted under stirring at room temperature for 3 hours. The reaction mixture was quenched with water and extracted with ethyl acetate (50 mL × 3). The organic phase was dried over anhydrous sodium sulfate. After filtration, the filtrate was concentrated under reduced pressure. The residue was purified by silica gel column chromatography with eluent system B to obtain the title compound **6s** (a mixture of stereoisomers, 40 mg, yield: 26.1%).

MS m/z (ESI): 470.2 [M+1].

### Step 18

### N,8,8-trimethyl-1-((1S,2S)-2-methyl-1-(5-oxo-4,5-dihydro-1,2,4-oxadiazol-3-yl)cyclopropyl)-N-phenyl-1,5a,6,8,9,9a-hexahydro-5H-pyrano[4',3':4,5]pyrano[2,3-e]indole-2-carboxamide 6t

Compound **6s** (40 mg, 0.085 mmol) and hydroxylamine hydrochloride (60 mg, 0.86 mmol) were dissolved in dimethyl sulfoxide (2.0 mL). Sodium bicarbonate (71 mg, 0.85 mmol) was added. The mixture was heated to 60°C and reacted under stirring for 3.5 hours. The reaction mixture was cooled to room temperature, quenched with water, and extracted with ethyl acetate (10 mL × 3). The organic phase was dried over anhydrous sodium sulfate. After filtration, the filtrate was concentrated under reduced pressure. The residue was dissolved in dimethyl sulfoxide (2.0 mL). *N*,*N*'-carbonyldiimidazole (37 mg, 0.26 mmol) and 1,8-diazabicyclo[5,4,0]undec-7-ene (85 mg, 0.34 mmol) were added and reacted under stirring at room temperature for 3 hours. The reaction mixture was quenched with water and extracted with ethyl acetate (30 mL × 3). The organic phase was dried over anhydrous sodium sulfate. After filtration, the filtrate was concentrated under reduced pressure. The residue was purified by silica gel column chromatography with eluent system C to obtain the title compound **6t** (a mixture of stereoisomers, 14 mg, yield: 31.1%).

MS m/z (ESI): 529.5 [M+1].

### Step 19

### 8,8-Dimethyl-1-((1S,2S)-2-methyl-1-(5-oxo-4,5-dihydro-1,2,4-oxadiazol-3-yl)cyclopropyl)-1,5a,6,8,9,9a-hexahydro-5H-pyrano[4',3':4,5]pyrano[2,3-e]indole-2-carboxylic acid 6u

Compound **6t** (14 mg, 0.026 mmol) was dissolved in tetrahydrofuran (1.0 mL). Potassium tert-butoxide (120 mg, 1.07 mmol) and water (4.0 mg) were added and reacted under stirring at room temperature for 16 hours. The reaction mixture was quenched with water and adjusted to pH = 4-5 with 1M dilute hydrochloric acid, and extracted with ethyl acetate (10 mL × 2). The organic phase was dried over anhydrous sodium sulfate. After filtration, the filtrate was concentrated under reduced pressure to obtain the crude title compound **6u** (a mixture of stereoisomers, 10 mg).

MS m/z (ESI): 440.4 [M+1].

### Step 20

### 3-((1S,2S)-1-(2-((S)-3-(3-(4-fluoro-1-methyl-1H-indazol-5-yl)-2-oxo-2,3-dihydro-1H-imidazol-1-yl)-2-(4-fluoro-3,5-dimethylphenyl)-4-methyl-4,5,6,7-tetrahydro-2H-pyrazolo[4,3-c]pyridine-5-carbonyl)-8,8-dimethyl-5a,8,9,9a-tetrahydro-5H-pyrano[4',3':4,5]pyrano[2,3-e]indol-1(6H)-yl)-2-methylcyclopropyl)-1,2,4-oxadiazol-5(4H)-one 6

Compound **6u** (10 mg, 0.023 mmol) and (*S*)-1-(4-fluoro-1-methyl-1*H*-indazol-5-yl)-3-(2-(4-fluoro-3,5-dimethylphenyl)-4-methyl-4,5,6,7-tetrahydro-2*H-*pyrazolo[4,3-*c*]pyridin-3-yl)-1,3-dihydro-2*H*-imidazol-2-one hydrochloride (12 mg, 0.024 mmol, prepared by a method disclosed for compound 00412 in an example on page 139 of the description of Patent Application WO 2022017338 A1) were dissolved in *N*,*N*-dimethylformamide (1.0 mL). 2-(7-Azabenzotriazole)-*N*,*N*,*N'*,*N*'-tetramethyluronium hexafluorophosphate (10 mg, 0.026 mmol) and *N,N-*diisopropylethylamine (10 mg, 0.077 mmol) were added and the mixture was reacted at room temperature for 1 hour. The reaction solution was concentrated under reduced pressure. The residue was purified by preparative high-performance liquid chromatography (instrument model: Waters 2545, chromatographic column: Welch Xtimate, Prep 30 × 250 mm; 5 µm; C18, mobile phase: water (10 mM ammonium bicarbonate) and acetonitrile, with gradient ratio: acetonitrile 30%-95%, flow rate: 30 mL/min) to obtain the title compound **6** (5.0 mg, yield: 24.1%, a mixture of diastereomers).

MS m/z (ESI): 911.3 [M+1].

¹H NMR (500 MHz, CDCl₃): δ 8.15-8.06 (m, 1H), 7.64-7.61 (m, 1H), 7.52-7.39 (m, 2H), 7.33-7.29 (m, 1H), 7.20-7.10 (m, 2H), 7.09-7.01 (m, 1H), 6.80 (s, 1H), 6.63-6.50(m, 1H), 5.80 (q, 1H), 4.55-4.41 (m, 2H), 4.30-4.28 (m, 1H), 4.15-4.06 (m, 3H), 3.92-3.75 (m, 2H), 3.64-3.58 (m, 1H), 3.23-2.90 (m, 3H), 2.36-2.19 (m, 7H), 1.90 (t, 1H), 1.73-1.52 (m, 5H), 1.42-1.21 (m, 8H), 1.09-1.05 (m, 2H), 0.90 (t, 1H).

### Example 7

### 3-((1S,2S)-1-(6-((S)-3-(3-(4-fluoro-1-methyl-1H-imidazol-5-yl)-2-oxo-2,3-dihydro-1H-imidazol-1-yl)-2-(4-fluoro-3,5-dimethylphenyl)-4-methyl-4,5,6,7-tetrahydro-2H-pyrazolo[4,3-c]pyridine-5-carbonyl)-2',3',5',6'-tetrahydro-2H,7H-spiro[furo[3,2-f]indole-3,4'-pyran]-7-yl)-2-methylcyclopropyl)-1,2,4-oxadiazol-5(4H)-one 7

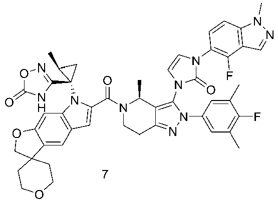

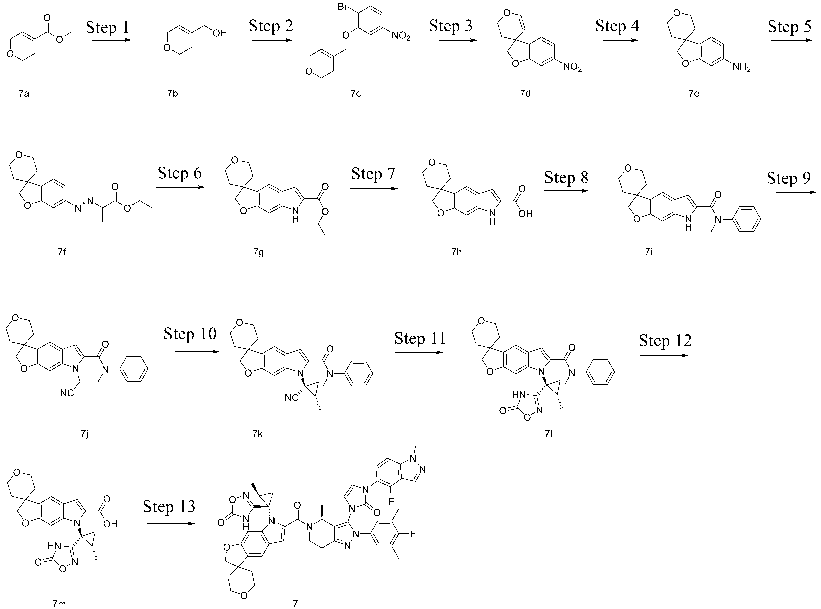

### Step 1

### (3,6-Dihydro-2H-pyran-4-yl)methanol 7b

Methyl 3,6-dihydro-2*H*-pyran-4-carboxylate **7a** (5.00 g, 35.17 mmol, Bidepharm) was dissolved in tetrahydrofuran and *N*,*N*-dimethylformamide (50 mL). A 1M solution of diisobutylaluminum hydride in tetrahydrofuran (77.38 mL, 77.38 mmol, Adamas) was dropwise added at -78°C. The mixture was reacted under stirring at -78°C for 1 hour, then heated to room temperature, and reacted for 16 hours. Under ice-water bath condition, a saturated aqueous ammonium chloride solution (50 mL) was added and stirred in the ice-water bath for three hours. After filtration, the filtrate was concentrated under reduced pressure. The residue was purified by silica gel column chromatography with eluent system C to obtain the title compound **7b** (3.8 g, yield: 94.7%).

### Step 2

### 4-((2-Bromo-5-nitrophenoxy)methyl)-3,6-dihydro-2H-pyran 7c

Compound **7b** (628 mg, 5.50 mmol), 2-bromo-5-nitrophenol (1.0 g, 4.59 mmol, Bidepharm), and triphenylphosphine (2.41 g, 9.17 mmol) were dissolved in tetrahydrofuran (10 mL). Diisopropyl azodicarboxylate (1.86 g, 9.17 mmol) was added and stirred at room temperature for 17 hours. The reaction solution was concentrated under reduced pressure. The residue was purified by silica gel column chromatography with eluent system C to obtain the title compound **7c** (1.2 g, yield: 83.3%).

### Step 3

### 6-Nitro-2',3'-dihydro-2H-spiro[benzofuran-3,4'-pyran] 7d

Compound **7c** (1.0 g, 3.18 mmol), palladium acetate (143 mg, 0.64 mmol), tetra-n-butylammonium bromide (911 mg, 3.18 mmol), and triethylamine (644 mg, 6.36 mmol) were dissolved in *N*,*N-*dimethylacetamide (50 mL), heated to 90°C, and reacted under stirring for 16 hours. The reaction solution was cooled to room temperature. After filtration, the filtrate was concentrated under reduced pressure. The residue was purified by silica gel column chromatography with eluent system C to obtain the title compound **7d** (600 mg, yield: 80.8%).

### Step 4

### 2',3',5',6'-Tetrahydro-2H-spiro[benzofuran-3,4'-pyran]-6-amine 7e

Compound **7d** (300 mg, 1.29 mmol) was dissolved in tetrahydrofuran (5 mL) and ethanol (10 mL). Palladium on carbon with a water content of 10% (31 mg, 0.26 mmol) was added and stirred in a hydrogen atmosphere at room temperature for 17 hours. The reaction solution was filtered. The filtrate was concentrated under reduced pressure. The residue was purified by silica gel column chromatography with eluent system C to obtain the title compound **7e** (200 mg, yield: 75.8%).

MS m/z (ESI): 206.3 [M+1].

### Step 5

### Ethyl ((2',3',5',6'-tetrahydro-2H-spiro[benzofuran-3,4'-pyran]-6-yl)amino)alaninate 7f

Compound **7e** (300 mg, 1.46 mmol) was dissolved in acetonitrile (5 mL). The mixture was cooled to 0°C under ice bath condition. A solution of 6 M hydrogen chloride in water (1.46 mL) was dropwise added. After the dropwise addition was complete, the mixture was stirred for 10 minutes. Sodium nitrite (242 g, 3.51 mmol) was dissolved in water (3 mL) and dropwise added to the reaction. The reaction mixture was reacted under stirring at 0°C for 1 hour. Ethyl 2-methylacetoacetate (278 g, 1.93 mmol) was dissolved in ethanol (5 mL) and cooled to 0°C under ice bath condition. Sodium acetate (1.44 g, 17.54 mmol) was added and stirred for 1 hour. The two reaction solutions were combined and stirred at room temperature for 2 hours. The mixture was neutralized to neutrality with sodium carbonate and extracted with dichloromethane (50 mL × 3). The organic phase was dried over anhydrous sodium sulfate. After filtration, the filtrate was concentrated under reduced pressure. The residue was purified by silica gel column chromatography with eluent system C to obtain an intermediate, which was dissolved in ethanol (6 mL). A 4 M solution of hydrogen chloride in 1,4-dioxane (1 mL) was dropwise added and stirred at room temperature for 2 hours. The reaction solution was concentrated under reduced pressure to obtain the crude title compound **7f** (300 mg). The product was directly used in the next reaction without purification.

MS m/z (ESI): 319.4 [M+1].

### Step 6

### Ethyl 2',3',5',6'-tetrahydro-2H,7H-spiro[furo[3,2-f]indole-3,4'-pyran]-6-carboxylate 7g

Compound **7f** (300 mg, 0.94 mmol) was dissolved in dichloromethane (10 mL). Eaton's reagent (449 g, 1.89 mmol) was added. The mixture was heated to 50°C and reacted under stirring for 3 hours. The reaction mixture was quenched with water, neutralized to neutrality with a sodium bicarbonate solution, and extracted with dichloromethane (50 mL × 3). The organic phase was dried over anhydrous sodium sulfate. After filtration, the filtrate was concentrated under reduced pressure. The residue was purified by silica gel column chromatography with eluent system C to obtain the title compound **7g** (280 mg, yield: 98.6%).

MS m/z (ESI): 302.3 [M+1].

### Step 7

### 2',3',5',6'-Tetrahydro-2H,7H-spiro[furo[3,2-f]indole-3,4'-pyran]-6-carboxylic acid 7h

Compound **7g** (100 mg, 0.33 mmol) was dissolved in methanol (2 mL) and water (2 mL). Sodium hydroxide (66 mg, 1.66 mmol) was added and reacted under stirring at 65°C for 3 hours. The reaction mixture was quenched with water and adjusted to pH = 3 with a dilute hydrochloric acid solution. A solid was precipitated. After filtration, the solid was dried to obtain the crude title compound **7h** (80 mg). The product was directly used in the next reaction without purification.

MS m/z (ESI): 274.2 [M+1].

### Step 8

### N-methyl-N-phenyl-2',3',5',6'-tetrahydro-2H,7H-spiro[furo[3,2-f]indole-3,4'-pyran]-6-carboxamide 7i

Compound **7h** (80 mg, 0.29 mmol) was dissolved in *N*,*N*-dimethylacetamide (2 mL). Under ice bath condition, sulfoxide chloride (139 mg, 3.85 mmol) was added and stirred at room temperature for 5 hours. Triethylamine (237 mg, 2.34 mmol) and N-methylaniline (63 mg, 0.59 mmol) were added and reacted under stirring at room temperature for 16 hours. The reaction mixture was quenched with water and extracted with ethyl acetate (30 mL × 3). The organic phase was dried over anhydrous sodium sulfate. After filtration, the filtrate was concentrated under reduced pressure. The residue was purified by silica gel column chromatography with eluent system C to obtain the title compound **7i** (105 mg, yield: 99.0%).

MS m/z (ESI): 363.4 [M+1].

### Step 9

### 7-(Cyanomethyl)-N-methyl-N-phenyl-2',3',5',6'-tetrahydro-2H,7H-spiro[furo[3,2-f]indole-3,4'-pyran]-6-carboxamide 7j

Compound **7i** (100 mg, 0.28 mmol) was dissolved in *N*,*N*-dimethylformamide (2 mL). Under ice bath condition, sodium hydride (110 mg, 2.75 mol, 60% wt) was added and stirred at room temperature for 5 hours. Chloroacetonitrile (208 mg, 2.75 mmol) was added and reacted under stirring at room temperature for 3 hours. The reaction mixture was quenched with water and extracted with ethyl acetate (30 mL × 3). The organic phase was dried over anhydrous sodium sulfate. After filtration, the filtrate was concentrated under reduced pressure. The residue was purified by silica gel column chromatography with eluent system C to obtain the title compound **7j** (105 mg, yield: 94.8%).

MS m/z (ESI): 402.0 [M+1].

### Step 10

### 7-((1S,2S)-1-cyano-2-methylcyclopropyl)-N-methyl-N-phenyl-2',3',5',6'-tetrahydro-2H,7H-spiro[furo[3,2-f]indole-3,4'-pyran]-6-carboxamide 7k

Compound **7j** (105 mg, 0.26 mmol) and (4*R*)-4-methyl-1,3,2-dioxathiolane 2,2-dioxide (126 mg, 0.91 mmol) were dissolved in *N*,*N*-dimethylpropyleneurea (1.341 g, 10.46 mmol). Under ice bath condition, potassium bis(trimethylsilyl)amide (417 mg, 2.09 mmol, a 1M tetrahydrofuran solution) was added and reacted under stirring at room temperature for 3 hours. The reaction mixture was quenched with water and extracted with ethyl acetate (20 mL × 3). The organic phase was dried over anhydrous sodium sulfate. After filtration, the filtrate was concentrated under reduced pressure. The residue was purified by silica gel column chromatography with eluent system C to obtain the title compound **7k** (60 mg, yield: 52.0%).

MS m/z (ESI): 442.4 [M+1].

### Step 11

### N-methyl-7-((1S,2S)-2-methyl-1-(5-oxo-4,5-dihydro-1,2,4-oxadiazol-3-yl)cyclopropyl)-N-phenyl-2',3',5',6'-tetrahydro-2H,7H-spiro[furo[3,2-f]indole-3,4'-pyran]-6-carboxamide 7l

Compound **7k** (60 mg, 0.14 mmol) and hydroxylamine hydrochloride (94 mg, 1.35 mmol) were dissolved in dimethyl sulfoxide (2 mL). Sodium bicarbonate (114 mg, 1.35 mmol) was added. The mixture was reacted under stirring at 60°C for 3.5 hours, cooled to room temperature, quenched with water, and extracted with ethyl acetate (10 mL × 3). The organic phase was dried over anhydrous sodium sulfate. After filtration, the filtrate was concentrated under reduced pressure. The residue was dissolved in dimethyl sulfoxide (2 mL). *N*,*N*'-carbonyldiimidazole (66 mg, 0.41 mmol) and 1,8-diazabicyclo[5,4,0]undec-7-ene (137 mg, 0.54 mmol) were added and reacted under stirring at room temperature for 3 hours. The reaction mixture was quenched with water and extracted with ethyl acetate (10 mL × 3). The organic phase was dried over anhydrous sodium sulfate. After filtration, the filtrate was concentrated under reduced pressure. The residue was purified by silica gel column chromatography with eluent system C to obtain the title compound **7l** (40 mg, yield: 58.8%).

MS m/z (ESI): 501.4 [M+1].

### Step 12

### 7-((1S,2S)-2-methyl-1-(5-oxo-4,5-dihydro-1,2,4-oxadiazol-3-yl)cyclopropyl)-2',3',5',6'-tetrahydro-2H,7H-spiro[furo[3,2-f]indole-3,4'-pyran]-6-carboxylic acid 7m

Compound **7l** (40 mg, 0.08 mmol) was dissolved in tetrahydrofuran (3.5 mL). Potassium tert-butoxide (314 mg, 2.80 mmol) and water (10 mg, 0.56 mmol) were added and reacted under stirring for 1 hour and adjusted to pH = 3 with 1M dilute hydrochloric acid and extracted with ethyl acetate (30 mL × 3). The organic phase was dried over anhydrous sodium sulfate. After filtration, the filtrate was concentrated under reduced pressure. The residue was purified by silica gel column chromatography with eluent system A to obtain the title compound **7m** (30 mg, yield: 91.2%).

MS m/z (ESI): 412.3 [M+1].

### Step 13

### 3-((1S,2S)-1-(6-((S)-3-(3-(4-fluoro-1-methyl-1H-imidazol-5-yl)-2-oxo-2,3-dihydro-1H-imidazol-1-yl)-2-(4-fluoro-3,5-dimethylphenyl)-4-methyl-4,5,6,7-tetrahydro-2H-pyrazolo[4,3-c]pyridine-5-carbonyl)-2',3',5',6'-tetrahydro-2H,7H-spiro[furo[3,2-f]indole-3,4'-pyran]-7-yl)-2-methylcyclopropyl)-1,2,4-oxadiazol-5(4H)-one 7

Compound **7m** (30 mg, 0.073 mmol) and (*S*)-1-(4-fluoro-1-methyl-1*H*-indazol-5-yl)-3-(2-(4-fluoro-3,5-dimethylphenyl)-4-methyl-4,5,6,7-tetrahydro-2*H-*pyrazolo[4,3-*c*]pyridin-3-yl)-1,3-dihydro-2*H*-imidazol-2-one hydrochloride (34 mg, 0.069 mmol, prepared by a method disclosed for compound 00412 in an example on page 139 of the description of Patent Application WO 2022017338 A1) were dissolved in *N*,*N*-dimethylformamide (1 mL). 2-(7-Azabenzotriazole)-*N*,*N*,*N*',*N*'-tetramethyluronium hexafluorophosphate(83 mg, 0.22 mmol) and *N,N-*diisopropylethylamine (47 mg, 0.36 mmol) were added and the mixture was reacted at room temperature for 16 hours. The reaction solution was concentrated under reduced pressure. The residue was purified by preparative high-performance liquid chromatography (instrument model: Waters 2489&SQD2, chromatographic column: Welch Xtimate, Prep 30 × 250 mm; 5 µm; C18, mobile phase: water (0.1% TFA) and acetonitrile, with gradient ratio: acetonitrile 45%-95%, flow rate: 30 mL/min) to obtain the title compound **7** (25 mg, yield: 38.8%).

MS m/z (ESI): 883.3 [M+1].

¹H NMR (500 MHz, CDCl₃): δ 11.52-11.30 (m, 1H), 8.21-8.00 (m, 1H), 7.52-7.49 (m, 1H), 7.38 (d, 1H), 7.32-7.29 (m, 1H),7.22-7.12 (m, 2H), 7.10-7.02 (m, 1H), 6.70 (s, 1H), 6.65-6.48 (m, 1H), 6.36-6.11 (m, 1H), 5.77 (q, 1H), 4.57-4.49 (m, 2H), 4.14 (s, 3H), 4.10-4.00 (m, 2H), 3.63-3.49 (m, 2H), 3.19-3.11 (m, 1H), 3.06-2.98 (m, 1H), 2.29 (s, 6H), 2.28-2.20 (m, 4H), 2.10-1.98 (m, 2H), 1.89 (t, 1H), 1.76-1.67 (m, 2H), 1.60-1.54 (m, 2H), 1.50-1.45 (m, 1H), 1.22 (d, 2H), 1.07 (d, 1H).

### Example 8-1 or 8-2

### 3-((1S,2S)-1-(5-((S)-2,2-dimethyltetrahydro-2H-pyran-4-yl)-2-((S)-2-(4-fluoro-3,5-dimethylphenyl)-3-(((R,Z)-10-fluoro-7-methyl-3a,4-dihydro-1H,3H,7H-oxazolo[3',4':4,5][1,4]oxazino[3,2-f]indazol-1-ylidene)amino)-4-methyl-4,5,6,7-tetrahydro-2H-pyrazolo[4,3-c]pyridine-5-carbonyl)-1H-indol-1-yl)-2-methylcyclopropyl)-1,2,4-oxadiazol-5(4H)-one 8-1 or 3-((1S,2S)-1-(5-((S)-2,2-dimethyltetrahydro-2H-pyran-4-yl)-2-((S)-2-(4-fluoro-3,5-dimethylphenyl)-3-(((R,E)-10-fluoro-7-methyl-3a,4-dihydro-1H,3H,7H-oxazolo[3',4'4,5][1,4]oxazino[3,2-f]indazol-1-ylidene)amino)-4-methyl-4,5,6,7-tetrahydro-2H-pyrazolo[4,3-c]pyridine-5-carbonyl)-1H-indol-1-yl)-2-methylcyclopropyl)-1,2,4-oxadiazol-5(4H)-one 8-2

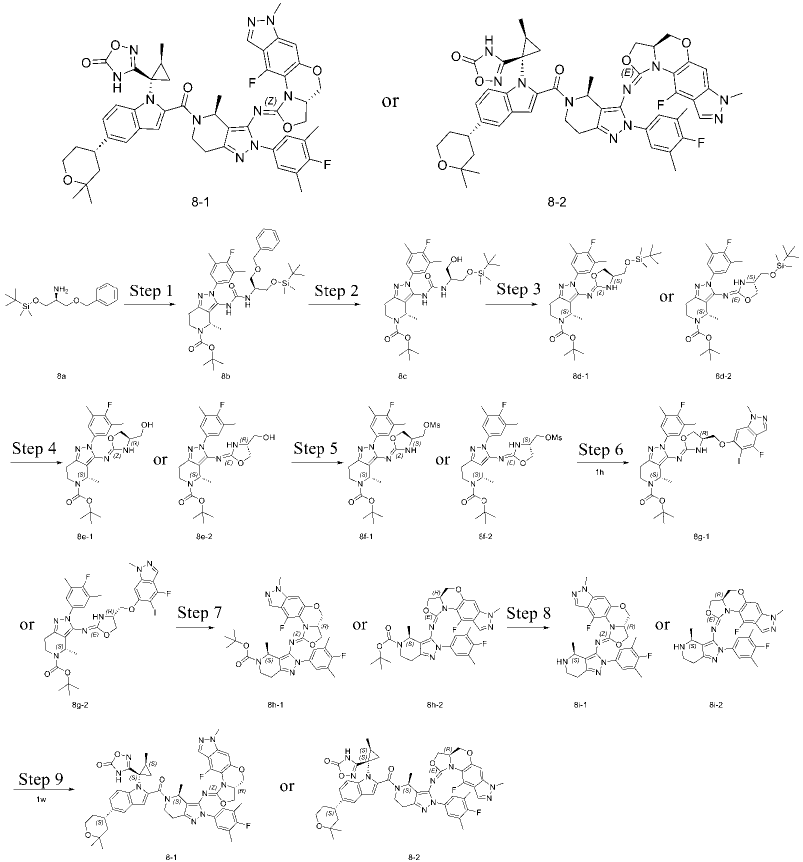

### Step 1

### Tert-butyl (S)-3-(3-((S)-1-(benzyloxy)-3-((tert-butyldimethylsilyl)oxy)prop-2-yl)ureido)-2-(4-fluoro-3,5-dimethylphenyl)-4-methyl-2,4,6,7-tetrahydro-5H-pyrazolo[4,3-c]pyridine-5-carboxylate 8b

Compound **1n** (1.0 g, 2.67 mmol, Shanghai Bidepharm) and *N*,*N-*diisopropylethylamine (1.73 g, 13.4 mmol) was dissolved in tetrahydrofuran (30 mL). Under ice bath condition, phenyl chloroformate (627 mg, 4.0 mmol, Adamas) was dropwise added and reacted under stirring at room temperature for 2 hours. (S)-1-(benzyloxy)-3-((tert-butyldimethylsilyl)oxy)propan-2-amine **8a** (2.36 g, 7.98 mmol, prepared by a method disclosed in a part (Part D) in Intermediate D on page 149 of the description of Patent Application WO 2006074003) was added and stirred at room temperature for 16 hours. The reaction solution was poured into 30 mL of water and extracted with ethyl acetate (30 mL × 3). The organic phases were combined, washed with a saturated sodium chloride solution, and dried over anhydrous sodium sulfate. After filtration, the filtrate was concentrated under reduced pressure. The residue was purified by silica gel column chromatography with eluent system C to obtain the title compound **8b** (1.1 g, yield: 59.8%).

MS m/z (ESI): 696.6 [M+1].

### Step 2

### Tert-butyl (S)-3-(3-((S)-1-((tert-butyldimethylsilyl)oxy)-3-hydroxyprop-2-yl)ureido)-2-(4-fluoro-3,5-dimethylphenyl)-4-methyl-2,4,6,7-tetrahydro-5H-pyrazolo[4,3-c]pyridine-5-carboxylate 8c

Compound **8b** (1.1 g, 7.18 mmol) was dissolved in tetrahydrofuran (30 mL). Palladium on carbon (335 mg, 3.15 mmol, 10 wt% (containing about 55% of water)) was added. The mixture was heated to 60°C in a hydrogen atmosphere and reacted for 8 hours. The reaction mixture was cooled to room temperature and filtered to remove palladium on carbon. The filtrate was concentrated under reduced pressure. The residue was purified by silica gel column chromatography with eluent system B to obtain the title compound **8c** (850 mg, yield: 88%).

MS m/z (ESI): 606.7 [M+1].

### Step 3

### Tert-butyl (S)-3-(((S,Z)-4-(((tert-butyldimethylsilyl)oxy)methyl)oxazolidin-2-ylidene)amino)-2-(4-fluoro-3,5-dimethylphenyl)-4-methyl-2,4,6,7-tetrahydro-5H-pyrazolo[4,3-c]pyridine-5-carboxylate 8d-1, or

### tert-butyl (S)-3-(((S,E)-4-(((tert-butyldimethylsilyl)oxy)methyl)oxazolidin-2-ylidene)amino)-2-(4-fluoro-3,5-dimethylphenyl)-4-methyl-2,4,6,7-tetrahydro-5H-pyrazolo[4,3-c]pyridine-5-carboxylate 8d-2

Compound **8c** (850 mg, 1.40 mmol) was dissolved in dichloromethane (30 mL). Diethylaminosulfur trifluoride (DAST) (271 mg, 1.68 mmol, Adamas) was dropwise added at -78°C and reacted under stirring at -78°C for 1 hour. Subsequently, the reaction mixture was heated to room temperature and stirred for 1 hour. After quenching with a saturated sodium bicarbonate solution (20 mL) and extracted with dichloromethane (30 mL × 3). The organic phases were combined and washed with a saturated sodium chloride solution. The organic phase was dried over anhydrous sodium sulfate. After filtration, the filtrate was concentrated under reduced pressure. The residue was purified by silica gel column chromatography with eluent system A to obtain the title compound **8d-1** or **8d-2** (600 mg, yield: 72.7%).

MS m/z (ESI): 588.6 [M+1].

### Step 4

### Tert-butyl (S)-2-(4-fluoro-3,5-dimethylphenyl)-3-(((R,Z)-4-(hydroxymethyl)oxazolidin-2-ylidene)amino)-4-methyl-2,4,6,7-tetrahydro-5H-pyrazolo[4,3-c]pyridine-5-carboxylate 8e-1, or

### tert-butyl (S)-2-(4-fluoro-3,5-dimethylphenyl)-3-(((R,E)-4-(hydroxymethyl)oxazolidin-2-ylidene)amino)-4-methyl-2,4,6,7-tetrahydro-5H-pyrazolo[4,3-c]pyridine-5-carboxylate 8e-2

Compound **8d-1** or **8d-2** (600 mg, 1.02 mmol) was dissolved in tetrahydrofuran (10 mL). A tetrabutylammonium fluoride/tetrahydrofuran solution (1.12 mL, 1M) was added and the mixture was reacted at room temperature for 1 hour. The reaction solution was poured into 20 mL of water and extracted with ethyl acetate (30 mL × 3). The organic phases were combined, washed with a saturated sodium chloride solution, and dried over anhydrous sodium sulfate. After filtration, the filtrate was concentrated under reduced pressure. The residue was purified by silica gel column chromatography with eluent system B to obtain the crude title compound **8e-1** or **8e-2** (500 mg).

MS m/z (ESI): 474.3 [M+1].

### Step 5

### Tert-butyl (S)-2-(4-fluoro-3,5-dimethylphenyl)-4-methyl-3-(((S,Z)-4-(((methylsulfonyl)oxy)methyl)oxazolidin-2-ylidene)amino)-2,4,6,7-tetrahydro-5H-pyrazolo[4,3-c]pyridine-5-carboxylate 8f-1, or

### tert-butyl (S)-2-(4-fluoro-3,5-dimethylphenyl)-4-methyl-3-(((S,E)-4-(((methylsulfonyl)oxy)methyl)oxazolidin-2-ylidene)amino)-2,4,6,7-tetrahydro-5H-pyrazolo[4,3-c]pyridine-5-carboxylate 8f-2

Compound **8e-1** or **8e-2** (500 mg, 1.06 mmol) and triethylamine (534 mg, 5.27 mmol) were dissolved in dichloromethane (10 mL). Methanesulfonic anhydride(275 mg, 1.58 mmol) was added at 0°C and the mixture was reacted at room temperature for 1 hour. The reaction solution was poured into 10 mL of water and extracted with dichloromethane (20 mL × 2). The organic phases were combined, washed with a saturated sodium chloride solution, and dried over anhydrous sodium sulfate. After filtration, the filtrate was concentrated under reduced pressure to obtain the crude title compound **8f-1** or **8f-2** (582 mg).

MS m/z (ESI): 552.6 [M+1].

### Step 6

### Tert-butyl (S)-2-(4-fluoro-3,5-dimethylphenyl)-3-(((R,Z)-4-(((4-fluoro-5-iodo-1-methyl-1H-indazol-6-yl)oxy)methyl)oxazolidin-2-ylidene)amino)-4-methyl-2,4,6,7-tetrahydro-5H-pyrazolo[4,3-c]pyridine-5-carboxylate 8g-1, or

### tert-butyl (S)-2-(4-fluoro-3,5-dimethylphenyl)-3-(((R,E)-4-(((4-fluoro-5-iodo-1-methyl-1H-indazol-6-yl)oxy)methyl)oxazolidin-2-ylidene)amino)-4-methyl-2,4,6,7-tetrahydro-5H-pyrazolo[4,3-c]pyridine-5-carboxylate 8g-2

Compound **8f-1** or **8f-2** (582 mg, 1.05 mmol) and compound **1h** (308 mg, 1.05mmol) were dissolved in *N*,*N*-dimethylformamide (1.0 mL). Potassium carbonate (437 mg, 3.16 mmol) was added and reacted at 60°C for 2 hours. The reaction solution was poured into 30 mL of water and extracted with ethyl acetate (30 mL × 2). The organic phases were combined, washed with a saturated sodium chloride solution, and dried over anhydrous sodium sulfate. After filtration, the filtrate was concentrated under reduced pressure. The residue was purified by silica gel column chromatography with eluent system A to obtain the title compound **8g-1** or **8g-2** (450 mg, yield: 57.1%).

MS m/z (ESI): 748.4 [M+1].

### Step 7

### Tert-butyl (S)-2-(4-fluoro-3,5-dimethylphenyl)-3-(((R,Z)-10-fluoro-7-methyl-3a,4-dihydro-1H,3H,7H-oxazolo[3 ',4':4,5][1,4]oxazino[3,2-f]indazol-1-ylidene)amino)-4-methyl-2,4,6,7-tetrahydro-5H-pyrazolo[4,3-c]pyridine-5-carboxylate 8h-1, or

### tert-butyl (S)-2-(4-fluoro-3,5-dimethylphenyl)-3-(((R,E)-10-fluoro-7-methyl-3a,4-dihydro-1H,3H,7H-oxazolo[3',4':4,5][1,4]oxazino[3,2-f]indazol-1-ylidene)amino)-4-methyl-2,4,6,7-tetrahydro-5H-pyrazolo[4,3-c]pyridine-5-carboxylate 8h-2

Compound **8g-1** or **8g-2** (350 mg, 0.47 mmol) and (1*S*,2*S*)-(+)-*N*,*N*'-dimethyl-1,2-cyclohexanediamine (66 mg, 0.46 mmol, Adamas) were dissolved in dimethyl sulfoxide (6.0 mL). Potassium carbonate (192 mg, 1.39 mmol) and cuprous iodide (42 mg, 0.22 mmol, Leyan) were added. After displacement with nitrogen three times, the mixture was heated and stirred at 100°C for 2 hours. The reaction solution was poured into 20 mL of water and extracted with ethyl acetate (30 mL × 2). The organic phases were combined, washed with a saturated sodium chloride solution, and dried over anhydrous sodium sulfate. After filtration, the filtrate was concentrated under reduced pressure. The residue was purified by silica gel column chromatography with eluent system A to obtain the title compound **8h-1** or **8h-2** (160 mg, yield: 55.1%).

MS m/z (ESI): 620.6 [M+1].

### Step 8

### (R,Z)-10-fluoro-N-((S)-2-(4-fluoro-3,5-dimethylphenyl)-4-methyl-4,5,6,7-tetrahydro-2H-pyrazolo[4,3-c]pyridin-3-yl)-7-methyl-3a,4-dihydro-1H,3H,7H-oxazolo[3',4':4,5][1,4]oxazino[3,2-f]indazol-1-imine 8i-1, or

### (R,E)-10-fluoro-N-((S)-2-(4-fluoro-3,5-dimethylphenyl)-4-methyl-4,5,6,7-tetrahydro-2H-pyrazolo[4,3-c]pyridin-3-yl)-7-methyl-3a,4-dihydro-1H,3H,7H-oxazolo[3',4':4,5][1,4]oxazino[3,2-f]indazol-1-imine 8i-2

Compound **8h-1** or **8h-2** (80 mg, 0.13 mmol) was dissolved in dichloromethane (5mL). Zinc bromide (88 mg, 0.39 mmol) was added and stirred at room temperature overnight. The reaction solution was concentrated under reduced pressure. The residue was purified by silica gel column chromatography with eluent system A to obtain the crude title compound **8i-1** or **8i-2** (50 mg).

MS m/z (ESI): 520.2 [M+1].

### Step 9

### 3-((1S,2S)-1-(5-((S)-2,2-dimethyltetrahydro-2H-pyran-4-yl)-2-((S)-2-(4-fluoro-3,5-dimethylphenyl)-3-(((R,Z)-10-fluoro-7-methyl-3a,4-dihydro-1H,3H,7H-oxazolo[3',4':4,5][1,4]oxazino[3,2-f]indazol-1-ylidene)amino)-4-methyl-4,5,6,7-tetrahydro-2H-pyrazolo[4,3-c]pyridine-5-carbonyl)-1H-indol-1-yl)-2-methylcyclopropyl)-1,2,4-oxadiazol-5(4H)-one 8-1, or

### 3-((1S,2S)-1-(5-((S)-2,2-dimethyltetrahydro-2H-pyran-4-yl)-2-((S)-2-(4-fluoro-3,5-dimethylphenyl)-3-(((R,E)-10-fluoro-7-methyl-3a,4-dihydro-1H,3H,7H-oxazolo[3',4':4,5][1,4]oxazino[3,2-f]indazol-1-ylidene)amino)-4-methyl-4,5,6,7-tetrahydro-2H-pyrazolo[4,3-c]pyridine-5-carbonyl)-1H-indol-1-yl)-2-methylcyclopropyl)-1,2,4-oxadiazol-5(4H)-one 8-2

Compound **8i-1 or 8i-2** (50 mg, 0.096 mmol) and compound **1w** (43 mg, 0.104 mmol) were dissolved in *N*,*N*-dimethylformamide (5 mL). *N,N-*diisopropylethylamine (37 mg, 0.29 mmol) was added. After the raw materials were fully dissolved, *O*-(7-azabenzotriazol-1-yl)-*N*,*N*,*N*'*,*N'-tetramethyluronium hexafluorophosphate(54 mg, 0.14 mmol) was added and stirred at room temperature for 16 hours. The reaction solution was subjected to suction filtration to remove insoluble substances. The residue was purified by preparative high-performance liquid chromatography (instrument model: Waters-2545 chromatographic column: Sharpsil-T Prep C18 5 µm 30 × 150 mm SN 03212989, mobile phase: water (10 mM ammonium bicarbonate) and acetonitrile, with gradient ratio: acetonitrile 40%-95%, flow rate: 30 mL/min) to obtain the title compound **8-1** or **8-2** (25 mg, yield: 28.4%).

MS m/z (ESI): 913.5 [M+1].

¹H NMR (500 MHz, CDCl₃): δ 8.06-7.89 (m, 1H), 7.66-7.49 (m, 2H), 7.48-7.36 (m, 2H), 7.27-7.20 (m, 1H), 6.78 (s, 1H), 6.71-6.66(m, 1H), 5.63-5.33 (m, 1H), 4.85-4.71 (m, 1H), 4.68-4.51 (m, 1H), 4.38-3.29 (m, 1H), 4.20-3.64 (m, 8H), 3.48-3.27 (m, 1H), 3.12-2.82(m, 3H), 2.50-2.27(m, 6H), 1.87-1.49 (m, 6H), 1.42-1.16 (m, 13H), 1.11-1.04 (m, 1H).

### Example 9

### 3-((1S,2S)-1-(5-((S)-2,2-dimethyltetrahydro-2H-pyran-4-yl)-2-((S)-2-(4-fluoro-3,5-dimethylphenyl)-4-methyl-3-(1-(1-methyl-1H-indazol-5-yl)-6-oxo-1,6-dihydropyrimidin-5-yl)-4,5,6,7-tetrahydro-2H-pyrazolo[4,3-c]pyridine-5-carbonyl)-1H-indol-1-yl)-2-methylcyclopropyl)-1,2,4-oxadiazol-5(4H)-one 9

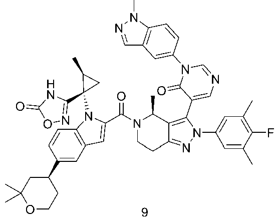

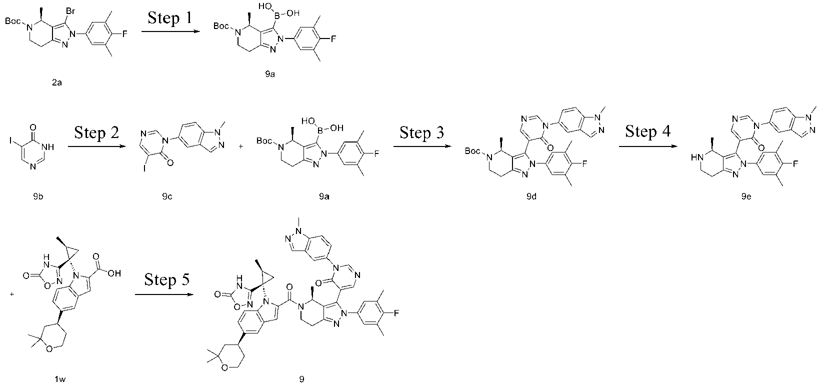

### Step 1

### (S)-(5-(tert-butoxycarbonyl)-2-(4-fluoro-3,5-dimethylphenyl)-4-methyl-4,5,6,7-tetrahydro-2H-pyrazolo[4,3-c]pyridin-3-yl)boronic acid 9a

Compound **2a** (500 mg, 1.14 mmol) was dissolved in tetrahydrofuran (10 mL). After displacement with nitrogen, n-butyllithium (0.92 mL, 2.5M, Adamas) was dropwise added at -78°C and stirred for 1 hour, followed by the addition of trimethyl borate (475 mg, 4.57 mmol, Adamas). The reaction was further stirred at -78°C for 0.5 hours. The reaction solution was quenched with 20 mL of a saturated aqueous ammonium chloride solution and extracted with ethyl acetate (30 mL × 3). The organic phases were combined. The combined organic phase was dried over anhydrous sodium sulfate. After filtration, the filtrate was concentrated under reduced pressure. The residue was purified by silica gel column chromatography with eluent system A to obtain the title compound **9a** (220 mg, yield: 47.8%).

MS m/z (ESI): 404.4 [M+1].

### Step 2

### 5-Iodo-3-(1-methyl-1H-indazol-5-yl)pyrimidin-4(3H)-one 9c

5- Iodopyrimidin-4(3*H*)-one **9b** (1.0 g, 4.50 mmol, Adamas), 1-methylindazole-5-boronic acid (1.2 g, 6.82 mmol, Leyan), pyridine (3.57 g, 45.13 mmol), and copper acetate (1.8 g, 9.02 mmol) were dissolved in 1,2-dichloroethane (30 mL), left open, and stirred at room temperature for 16 hours. The reaction solution was concentrated under reduced pressure. The residue was purified by silica gel column chromatography with eluent system A to obtain a crude product, which was further slurried with dichloromethane to obtain the title compound **9c** (500 mg, yield: 31.5%).

MS m/z (ESI): 353.1 [M+1].

### Step 3

### Tert-butyl (S)-2-(4-fluoro-3,5-dimethylphenyl)-4-methyl-3-(1-(1-methyl-1H-indazol-5-yl)-6-oxo-1,6-dihydropyrimidin-5-yl)-2,4,6,7-tetrahydro-5H-pyrazolo[4,3-c]pyridine-5-carboxylate 9d

Compound **9c** (150 mg, 0.43 mmol), compound **9a** (210 mg, 0.52 mmol), anhydrous potassium carbonate (177 mg, 1.28 mmol), and tetrakis(triphenylphosphine)palladium (132 mg, 0.043 mmol, Bidepharm) were dissolved in dioxane (8 mL) and water (2 mL). After displacement with nitrogen, the mixture was reacted under stirring at 80°C for 3 hours. The reaction solution was cooled to room temperature and concentrated under reduced pressure. The residue was purified by silica gel column chromatography with eluent system A to obtain the crude title compound **9d** (20 mg).

MS m/z (ESI): 584.4 [M+1].

### Step 4

### (S)-5-(2-(4-fluoro-3,5-dimethylphenyl)-4-methyl-4,5,6,7-tetrahydro-2H-pyrazolo[4,3-c]pyridin-3-yl)-3-(1-methyl-1H-indazol-5-yl)pyrimidin-4(3H)-one 9e

Compound **9d** (20 mg, 0.034 mmol) was dissolved in dichloromethane (1 mL). A 4M solution of hydrogen chloride in dioxane (2 mL) was added. The reaction was stirred at room temperature for 1 hour. The reaction solution was concentrated under reduced pressure to obtain the crude title compound **9e** (17 mg). The product was directly used in the next reaction without purification.

MS m/z (ESI): 484.4 [M+1].

### Step 5

### 3-((1S,2S)-1-(5-((S)-2,2-dimethyltetrahydro-2H-pyran-4-yl)-2-((S)-2-(4-fluoro-3,5-dimethylphenyl)-4-methyl-3-(1-(1-methyl-1H-indazol-5-yl)-6-oxo-1,6-dihydropyrimidin-5-yl)-4,5,6,7-tetrahydro-2H-pyrazolo[4,3-c]pyridine-5-carbonyl)-1H-indol-1-yl)-2-methylcyclopropyl)-1,2,4-oxadiazol-5(4H)-one 9

Compound **9e** (17 mg, 0.035 mmol) and compound **1w** (15 mg, 0.036 mmol) were dissolved in *N*,*N*-dimethylformamide (5 mL). *N*,*N*-diisopropylethylamine (24 mg, 0.19 mmol) was added. After the raw materials were fully dissolved, *O*-(7-azabenzotriazol-1-yl)-*N*,*N*,*N*',*N*'-tetramethyluronium hexafluorophosphate (17 mg, 0.045 mmol) was added and stirred at room temperature for 16 hours. The reaction solution was poured into 10 mL of water and extracted with ethyl acetate (10 mL × 3). The organic phases were combined, washed with a saturated sodium chloride solution, and dried over anhydrous sodium sulfate. After filtration, the filtrate was concentrated under reduced pressure. The residue was purified by preparative high-performance liquid chromatography (instrument model: Gilson-GX-281, chromatographic column: Boston Phlex, Prep 30 × 150 mm; 5 µm; C18, mobile phase: water (10 mM ammonium bicarbonate) and acetonitrile, with gradient ratio: acetonitrile 35%-55%, flow rate: 30 mL/min) to obtain the title compound **9** (15 mg, yield: 46.9%).

MS m/z (ESI): 877.8 [M+1].

¹H NMR (500 MHz, CDCl₃): δ 11.44-11.26 (m, 1H), 8.31 (s, 1H), 8.21-8.04 (m, 1H), 7.99-7.88 (m, 1H), 7.74-7.64 (m, 1H), 7.61-7.40 (m, 3H), 7.36-7.27 (m, 1H), 7.16-6.94 (m, 2H), 6.78-6.63 (m, 1H), 5.82 (q, 1H), 5.43-5.18 (m, 1H), 4.89-4.38 (m, 1H), 4.18-4.03 (m, 3H), 3.91-3.76 (m, 2H), 3.64-3.38 (m, 1H), 3.22-2.92 (m, 3H), 2.29-2.21 (m, 6H), 1.90 (t, 1H), 1.73-1.56 (m, 7H), 1.47 (d, 2H), 1.37-1.30 (m, 5H), 1.19 (d, 2H), 1.00 (d, 1H), 0.88 (t, 1H).

### Example 10

### 3-((1S,2S)-1-(5-((S)-2,2-dimethyltetrahydro-2H-pyran-4-yl)-2-((S)-3-(5-fluoro-1-(1-methyl-1H-indazol-5-yl)-2-oxo-1,2-dihydropyridin-3-yl)-2-(4-fluoro-3,5-dimethylphenyl)-4-methyl-4,5,6,7-tetrahydro-2H-pyrazolo[4,3-c]pyridine-5-carbonyl)-1H-indol-1-yl)-2-methylcyclopropyl)-1,2,4-oxadiazol-5(4H)-one 10

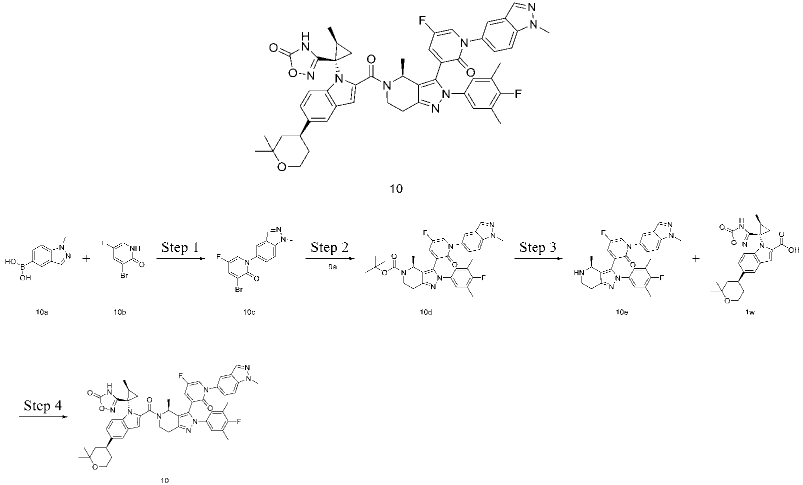

### Step 1

### 3-Bromo-5-fluoro-1-(1-methyl-1H-indazol-5-yl)pyridin-2(1H)-one 10c

3-Bromo-5-fluoropyridin-2(1*H*)-one **10b** (1.0 g, 5.68 mmol, Leyan), (1-methyl-1*H*-indazol-5-yl)boronic acid **10a** (1.3 g, 6.82 mmol, Leyan), 2,2'-bipyridine (1.77 g, 11.33 mmol, Adamas), and copper acetate (2.0 g, 11.33 mmol), sodium carbonate (1.8 g, 7.07 mmol) were dissolved in 1,2-dichloroethane (30 mL), left open, and stirred at room temperature for 16 hours. The reaction solution was concentrated under reduced pressure. The residue was purified by silica gel column chromatography with eluent system B to obtain the title compound **10c** (70 mg, yield: 3.82%).

MS m/z (ESI): 322.1 [M+1].

### Step 2

### Tert-butyl (S)-3-(5-fluoro-1-(1-methyl-1H-indazol-5-yl)-2-oxo-1,2-dihydropyridin-3-yl)-2-(4-fluoro-3,5-dimethylphenyl)-4-methyl-2,4,6,7-tetrahydro-5H-pyrazolo[4,3-c]pyridine-5-carboxylate 10d

Compound **10c** (120 mg, 0.37 mmol), compound **9a** (165 mg, 0.41 mmol), anhydrous potassium carbonate (102 mg, 0.75 mmol), and [1,1'-bis(diphenylphosphino)ferrocene]palladium(II) dichloride (54 mg, 0.07 mmol) were dissolved in dioxane (30 mL) and water (6 mL). After displacement with nitrogen, the mixture was reacted under stirring at 90°C for 16 hours. The reaction solution was cooled to room temperature and concentrated under reduced pressure. The residue was purified by silica gel column chromatography with eluent system B to obtain the crude title compound **10d** (120 mg, yield: 53.6%).

MS m/z (ESI): 601.5 [M+1].

### Step 3

### (S)-5-fluoro-3-(2-(4-fluoro-3,5-dimethylphenyl)-4-methyl-4,5,6,7-tetrahydro-2H-pyrazolo[4,3-c]pyridin-3-yl)-1-(1-methyl-1H-indazol-5-yl)pyridin-2(1H)-one 10e

Compound **10d** (120 mg, 0.05 mmol) was dissolved in dichloromethane (20 mL). Trifluoroacetic acid (5 mL) was added. The reaction was stirred at room temperature for 1 hour. The reaction solution was concentrated under reduced pressure to obtain the crude title compound **10e** (25 mg). The product was directly used in the next reaction without purification.

MS m/z (ESI): 501.4 [M+1].

### Step 4

### 3-((1S,2S)-1-(5-((S)-2,2-dimethyltetrahydro-2H-pyran-4-yl)-2-((S)-3-(5-fluoro-1-(1-methyl-1H-indazol-5-yl)-2-oxo-1,2-dihydropyridin-3-yl)-2-(4-fluoro-3,5-dimethylphenyl)-4-methyl-4,5,6,7-tetrahydro-2H-pyrazolo[4,3-c]pyridine-5-carbonyl)-1H-indol-1-yl)-2-methylcyclopropyl)-1,2,4-oxadiazol-5(4H)-one 10

Compound **10e** (25 mg, 0.05 mmol) and compound **1w** (25 mg, 0.06 mmol) were dissolved in *N*,*N*-dimethylformamide (5 mL). *N*,*N*-diisopropylethylamine (20 mg, 0.15 mmol) was added. After the raw materials were fully dissolved, *O*-(7-azabenzotriazol-1-yl)-*N,N,N',N'*-tetramethyluronium hexafluorophosphate(37 mg, 0.1 mmol) was added and stirred at room temperature for 3 hours. The reaction solution was poured into 10 mL of water and extracted with ethyl acetate (10 mL × 3). The organic phases were combined, washed with a saturated sodium chloride solution, and dried over anhydrous sodium sulfate. After filtration, the filtrate was concentrated under reduced pressure. The residue was purified by preparative high-performance liquid chromatography (instrument model: Waters-2545 chromatographic column: Welch Xtimate, Prep 30 × 150 mm; 5 µm; C18, mobile phase: water (10 mM ammonium bicarbonate) and acetonitrile, with gradient ratio: acetonitrile 30%-65%, flow rate: 30 mL/min) to obtain the title compound **10** (5 mg, yield: 11.1%).

MS m/z (ESI): 894.7 [M+1].

¹H NMR (500 MHz, CDCl₃): δ 11.42 (s, 1H), 11.33 (s, 1H), 8.05 (s, 1H), 7.89 (s, 1H), 7.67 (s, 1H), 7.58-7.47 (m, 2H), 7.40-7.29 (m, 2H), 7.10 (d, 2H), 6.97 (d, 1H), 6.88 (s, 1H), 5.90-5.30 (m, 2H), 4.67-4.38 (m, 2H), 4.12 (s, 3H), 3.93-3.76 (m, 2H), 3.71-3.43 (m, 2H), 3.25-2.88 (m, 3H), 2.33-2.14 (m, 6H), 1.85-1.44 (m, 7H), 1.42-1.32 (m, 5H), 1.27 (s, 2H), 1.19 (d, 1H), 1.06 (d, 1H).

### Example 11

### 2-((S)-5-(5-((S)-2,2-dimethyltetrahydro-2H-pyran-4-yl)-1-((1S,2S)-2-methyl-1-(5-oxo-4,5-dihydro-1,2,4-oxadiazol-3-yl)cyclopropyl)-1H-indole-2-carbonyl)-2-(4-fluoro-3,5-dimethylphenyl)-4-methyl-4,5,6,7-tetrahydro-2H-pyrazolo[4,3-c]pyridin-3-yl)-10-fluoro-7-(trideuteriomethyl)-2,3,3a,4-tetrahydro-1H,7H-imidazo[1',5':4,5][1,4]oxazino[3,2-f]indazol-1-one 11

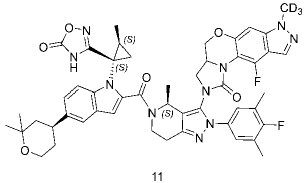

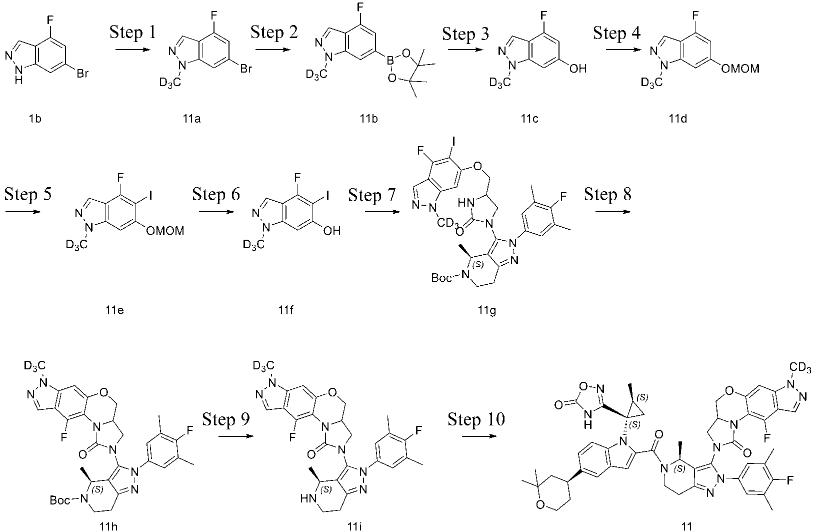

### Step 1

### 6-Bromo-4-fluoro-1-(methyl-d₃)indazole 11a

Compound **1b** (5.0 g, 23.3 mmol) was dissolved in *N*,*N*-dimethylformamide (60 mL). Sodium hydride (1.86 g, 46.5 mmol, 60% dispersed in a mineral oil) was added in portions at 0°C and stirred for 30 minutes. Deuterated iodomethane (6.74 g, 46.5 mmol) was dropwise added. After the addition was complete, the mixture was reacted under stirring at room temperature for 3 hours. The reaction solution was quenched with a saturated aqueous ammonium chloride solution (100 mL), extracted with ethyl acetate (50 mL × 2), and washed with a saturated sodium chloride solution. The organic phase was dried over anhydrous sodium sulfate. After filtration, the filtrate was concentrated under reduced pressure. The residue was purified by silica gel column chromatography with eluent system C to obtain the title compound **11a** (3.6 g, yield: 66.7%).

MS m/z (ESI): 232.2 [M+1].

### Step 2

### 4-Fluoro-1-(methyl-d₃)-6-(4,4,5,5-tetramethyl-1,3,2-dioxaborolan-2-yl)-1-(trideuteriomethyl)indazole 11b

Compound **11a** (3.6 g, 15.5 mmol), potassium acetate (3.8 g, 38.7 mmol), bis(pinacolato)diboron (5.90 g, 23.2 mmol, Shaoyuan Technology (Shanghai) Co., Ltd.), and [1,1'-bis(diphenylphosphino)ferrocene]palladium dichloride (2.27 g, 3.10 mmol, Shanghai Bidepharm) were dissolved in dioxane (100 mL). After displacement with nitrogen three times, the mixture was reacted at 85°C for 16 hours. Water (100 mL) was added. The mixture was extracted with ethyl acetate (100 mL × 3). The organic phases were combined and washed with a saturated sodium chloride solution. The organic phase was dried over anhydrous sodium sulfate. After filtration, the filtrate was concentrated under reduced pressure. The residue was purified by silica gel column chromatography with eluent system C to obtain the title compound **11b** (4.3 g, yield: 99.3%).

MS m/z (ESI): 280.1 [M+1].

### Step 3

### 4-Fluoro-1-(methyl-d₃)-1H-indazol-6-ol 11c

Compound **11b** (500 mg, 1.79 mmol) was dissolved in tetrahydrofuran (10 mL). A solution of sodium perborate tetrahydrate (880 mg, 5.37 mmol) in water (5 mL) was dropwise added at 0°C. After the addition was complete, the mixture was reacted at room temperature for hours. The reaction solution was poured into 50 mL of water, extracted with dichloromethane (30 mL × 3), and dried over anhydrous sodium sulfate. After filtration, the filtrate was concentrated under reduced pressure. The residue was purified by silica gel column chromatography with eluent system C to obtain the title compound 11c (300 mg, yield: 99.0%).

MS m/z (ESI): 170.1 [M+1].

### Step 4

### 4-Fluoro-6-(methoxymethoxy)-1-(methyl-d₃)-1H-indazole 11d

Compound **11c** (2.2 g, 12.8 mmol) and *N*,*N*-diisopropylethylamine (3.3 g, 25.5 mmol) were dissolved in dichloromethane (50 mL). Bromomethyl methyl ether (2.39 g, 19.1 mmol) was dropwise added at 0°C. After the addition was complete, the mixture was stirred at room temperature for 2 hours, quenched with water (50 mL), and extracted with dichloromethane (50 mL × 3). The organic phases were combined and washed with a saturated sodium chloride solution. The organic phase was dried over anhydrous sodium sulfate. After filtration, the filtrate was concentrated under reduced pressure. The residue was purified by silica gel column chromatography with eluent system C to obtain the title compound **11d** (2.0 g, yield: 73.4%).

MS m/z (ESI): 214.2 [M+1].

### Step 5

### 4-Fluoro-5-iodo-6-(methoxymethoxy)-1-(methyl-d₃)-1H-indazole 11e

Compound **11d** (500 mg, 2.34 mmol) was dissolved in tetrahydrofuran (50 mL). Lithium diisopropylamide (1.8 mL, 3.6 mmol, 2.0 M, Adamas) was dropwise added at -78°C in a nitrogen atmosphere. After the addition was complete, stirring was continued at -78°C for 2 hours. A solution of iodine (900 mg, 3.6 mmol) in tetrahydrofuran (5 mL) was then dropwise added. After the addition was complete, stirring was continued at -78°C for 1 hour, and the mixture was slowly heated to room temperature. The reaction was quenched with water (50 mL) and extracted with ethyl acetate (30 mL × 3). The organic phases were combined and washed with a saturated sodium chloride solution. The organic phase was dried over anhydrous sodium sulfate. After filtration, the filtrate was concentrated under reduced pressure. The residue was purified by silica gel column chromatography with eluent system C to obtain the title compound **11e** (600 mg, yield: 75.4%).

MS m/z (ESI): 340.1 [M+1].

### Step 6

### 4-Fluoro-5-iodo-1-(methyl-d₃)-1Hindazol-6-ol 11f

Compound **11**e (600 mg, 1.77 mmol) was dissolved in dichloromethane (10 mL). A hydrogen chloride/dioxane solution (10 mL, 4.0 M) was dropwise added at 0°C. After the addition was complete, the mixture was stirred at room temperature for 1 hour. After concentration, 30 mL of water was added. The mixture was extracted with ethyl acetate (30 mL × 3). The organic phases were combined, washed with a saturated aqueous sodium chloride solution, and dried over anhydrous sodium sulfate. After filtration, the filtrate was concentrated under reduced pressure. The residue was purified by silica gel column chromatography with eluent system C to obtain the title compound **11f (400** mg, yield: 76.6%).

MS m/z (ESI): 296.1 [M+1].

### Step 7

### Tert-butyl (4S)-2-(4-fluoro-3,5-dimethylphenyl)-3-(4-(((4-fluoro-5-iodo-1-(methyl-d₃)-1H-indazol-6-yl))oxy)methyl)-2-oxoimidazolidin-1-yl)-4-methyl-2,4,6,7-tetrahydro-5H-pyrazolo[4,3-c]pyridine-5-carboxylate 11g

Compound **1s** (209 mg, 0.38 mmol) and **11f** (100 mg, 0.34 mmol) were dissolved in *N,N*-dimethylformamide (3.0 mL). Potassium carbonate (130 mg, 0.94 mmol) was added and reacted at 80°C for 5 hours. The reaction solution was poured into 20 mL of water and extracted with ethyl acetate (30 mL × 2). The organic phases were combined, washed with a saturated sodium chloride solution, and dried over anhydrous sodium sulfate. After filtration, the filtrate was concentrated under reduced pressure. The residue was purified by silica gel column chromatography with eluent system A to obtain the title compound **11g** (250 mg, yield: 98.2%).

MS m/z (ESI): 751.2 [M+1].

### Step 8

### Tert-butyl (4S)-2-(4-fluoro-3,5-dimethylphenyl)-3-(10-fluoro-7-(methyl-d₃)-1-oxo-3a,4-dihydro-1H,7H-imidazo[1',5':4,5][1,4]oxazino[3,2-f]indazol-2(3H)-yl)-4-methyl-2,4,6,7-tetrahydro-5H-pyrazolo[4,3-c]pyridine-5-carboxylate 11h

Compound **11g** (250 mg, 0.33mmol) and *L*-proline (10 mg, 0.087 mmol) were dissolved in N,N-dimethylformamide (15.0 mL). Potassium carbonate (140 mg, 1.01mmol) and cuprous iodide (7.0 mg, 0.037 mmol, Leyan) were added. After displacement with nitrogen three times, the mixture was reacted under microwave at 125°C for 1 hour. The reaction solution was poured into 20 mL of water and extracted with ethyl acetate (30 mL × 2). The organic phases were combined, washed with a saturated sodium chloride solution, and dried over anhydrous sodium sulfate. After filtration, the filtrate was concentrated under reduced pressure. The residue was purified by silica gel column chromatography with eluent system C to obtain the title compound **11h** (130 mg, yield: 62.6%).

MS m/z (ESI): 623.3 [M+1].

### Step 9

### 10-Fluoro-2-((S)-2-(4-fluoro-3,5-dimethylphenyl)-4-methyl-4,5,6,7-tetrahydro-2H-pyrazolo[4,3-c]pyridin-3-yl)-7-(methyl-d₃)-2,3,3a,4-tetrahydro-1H,7H-imidazo[1',5':4,5][1,4]oxazino[3,2-f]indazol-1-one 11i

Compound **11h** (130 mg, 0.21 mmol) was dissolved in methanol (2 mL). A 4 M solution of hydrogen chloride in 1,4-dioxane (2 mL) was added and stirred at room temperature for 2 hours. The reaction solution was concentrated under reduced pressure to obtain the crude title compound **11i** (110 mg). The product was directly used in the next reaction without purification.

MS m/z (ESI): 523.2 [M+1].

### Step 10

### 2-((S)-5-(5-((S)-2,2-dimethyltetrahydro-2H-pyran-4-yl)-1-((1S,2S)-2-methyl-1-(5-oxo-4,5-dihydro-1,2,4-oxadiazol-3-yl)cyclopropyl)-1H-indole-2-carbonyl)-2-(4-fluoro-3,5-dimethylphenyl)-4-methyl-4,5,6,7-tetrahydro-2H-pyrazolo[4,3-c]pyridin-3-yl)-10-fluoro-7-(methyl-d₃)-2,3,3a,4-tetrahydro-1H,7H-imidazo[1',5':4,5][1,4]oxazino[3,2-f]indazol-1-one 11

Crude compound **11i** (110 mg, 0.20mmol) and compound **1w** (85 mg, 0.21 mmol) were dissolved in *N,N*-dimethylformamide (3 mL). *N,N-*diisopropylethylamine (130 mg, 1.0 mmol) was added. After the raw materials were fully dissolved, *O*-(7-azabenzotriazol-1-yl)-*N,N,N',N'*-tetramethyluronium hexafluorophosphate(150 mg, 0.39 mmol) was added and stirred at room temperature for 3 hours. The reaction solution was poured into 10 mL of water and extracted with ethyl acetate (10 mL × 3). The organic phases were combined, washed with a saturated sodium chloride solution, and dried over anhydrous sodium sulfate. After filtration, the filtrate was concentrated under reduced pressure to obtain the title compound **11** (a mixture of diastereomers, 150 mg).

MS m/z (ESI): 916.8 [M+1].

### Examples 11-1 and 11-2

### (R)-2-((S)-5-(5-((S)-2,2-dimethyltetrahydro-2H-pyran-4-yl)-1-((1S,2S)-2-methyl-1-(5-oxo-4,5-dihydro-1,2,4-oxadiazol-3-yl)cyclopropyl)-1H-indole-2-carbonyl)-2-(4-fluoro-3,5-dimethylphenyl)-4-methyl-4,5,6,7-tetrahydro-2H-pyrazolo[4,3-c]pyridin-3-yl)-10-fluoro-7-(methyl-d₃)-2,3,3a,4-tetrahydro-1H,7H-imidazo[1',5':4,5][1,4]oxazino[3,2-f]indazol-1-one 11-1, and

### (S)-2-((S)-5-(5-((S)-2,2-dimethyltetrahydro-2H-pyran-4-yl)-1-((1S,2S)-2-methyl-1-(5-oxo-4,5-dihydro-1,2,4-oxadiazol-3-yl)cyclopropyl)-1H-indole-2-carbonyl)-2-(4-fluoro-3,5-dimethylphenyl)-4-methyl-4,5,6,7-tetrahydro-2H-pyrazolo[4,3-c]pyridin-3-yl)-10-fluoro-7-(methyl-d₃)-2,3,3a,4-tetrahydro-1H,7H-imidazo[1',5':4,5][1,4]oxazino[3,2-f]indazol-1-one 11-2

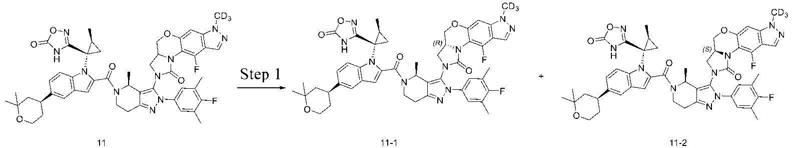

Compound **11** (150 mg, 0.15 mmol) was purified by preparative high-performance liquid chromatography (instrument model: Waters-2545, chromatographic column: Welch Xtimate, Prep 30 × 250 mm; 5 µm; C18, mobile phase: water (10 mM ammonium bicarbonate) and acetonitrile, with gradient ratio: acetonitrile 40%-95%, flow rate: 30 mL/min) to obtain the title compound (48 mg, yield: 26.6%) and (85 mg, yield: 47.1%).

Single-configuration compound (shorter retention time, retention time: 1.35 minutes, 48 mg, yield: 26.6%):
MS m/z (ESI): 916.8 [M+1].

¹H NMR (500 MHz, CDCl₃): δ 8.03-7.99 (m, 1H), 7.61 (d, 1H), 7.54-7.50 (m, 1H), 7.29-7.27 (m, 1H), 7.20-7.17 (m, 2H), 6.72-6.66 (m, 2H), 5.82-5.78 (m, 1H), 4.49-4.45 (m, 1H), 4.27-4.24 (m, 1H), 4.09-3.99 (m, 1H), 3.92-3.80 (m, 2H), 3.67-3.60 (m, 2H), 3.61-3.00 (m, 4H), 2.37-2.30 (m, 6H), 1.94 (t, 1H), 1.73-1.50 (m, 7H), 1.36 -1.25 (m, 11H), 1.07 (d, 1H).

Single-configuration compound (longer retention time, retention time 1.43 minutes, 85 mg, yield: 47.1%):
MS m/z (ESI): 916.8 [M+1].

¹H NMR (500 MHz, CDCl₃): δ 8.06 (s, 1H), 7.79-7.60(m, 2H), 7.53 (d, 1H), 7.33-7.27 (m, 1H), 7.17 (d, 1H), 6.76-6.71 (m, 2H), 5.72-5.68 (m, 1H), 4.48-4.43 (m, 1H), 4.15-4.06 (m, 1H), 3.96-3.82 (m, 3H), 3.73-3.57 (m, 2H), 3.30 (d, 1H), 3.15-3.00 (m, 3H), 2.37-2.30 (m, 6H), 1.92 (t, 1H), 1.80-1.54 (m, 7H), 1.40 -1.22 (m, 11H), 1.07 (d, 1H).

### Example 12

### 2-((S)-5-(5-((S)-2,2-dimethyltetrahydro-2H-pyran-4-yl)-1-((1S,2S)-2-methyl-1-(5-oxo-4,5-dihydro-1,2,4-oxadiazol-3-yl)cyclopropyl)-1H-indole-2-carbonyl)-2-(4-fluoro-3,5-dimethylphenyl)-4-methyl-4,5,6,7-tetrahydro-2H-pyrazolo[4,3-c]pyridin-3-yl)-10-fluoro-7-methyl-2,4-dihydro-1H,7H-imidazo[1', 5':4,5][1,4]oxazino[3,2-f]indazol-1-one 12

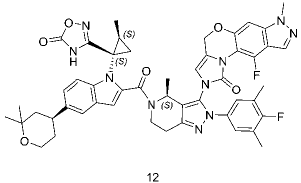

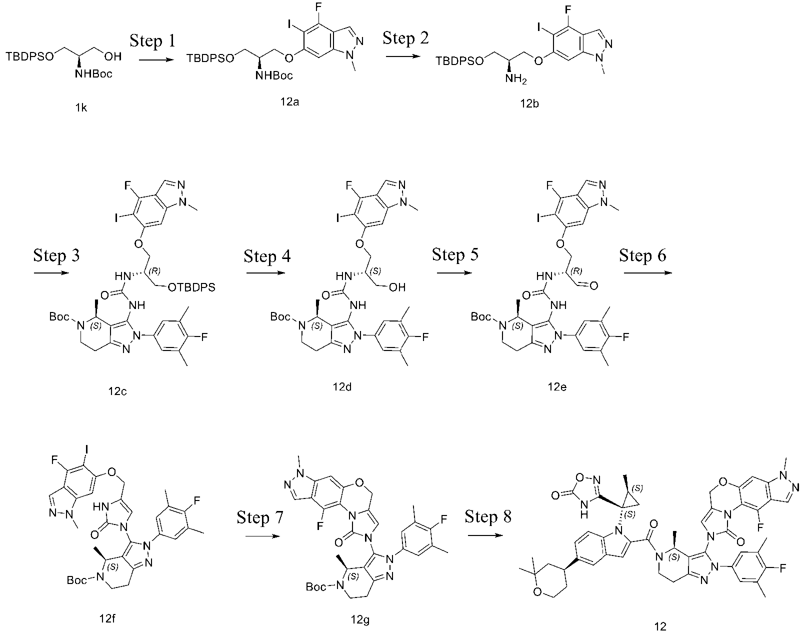

### Step 1

### Tert-butyl (R)-(1-((tert-butyldiphenylsilyl)oxy)-3-((4-fluoro-5-iodo-1-methyl-1H-indazol-6-yl)oxy)prop-2-yl)carbamate 12a

Compound **1k** (1.0 g, 2.38 mmol) and triethylamine (700 mg, 6.9 mmol) were dissolved in dichloromethane (30 mL). Methanesulfonyl chloride (350 mg, 3.05 mmol) was added at 0°C and the mixture was reacted at room temperature for 1 hour. The reaction solution was poured into 20 mL of water and extracted with dichloromethane (30 mL × 2). The organic phases were combined, washed with a saturated sodium chloride solution, and dried over anhydrous sodium sulfate. After filtration, the filtrate was concentrated under reduced pressure to obtain a crude product for later use.

Compound **1h** (500 mg, 1.71 mmol) and the above crude product were dissolved in *N,N*-dimethylformamide (10 mL). Potassium carbonate (600 mg, 4.34 mmol) was added and reacted at 60°C for 5 hours. Water (50 mL) was added. The mixture was extracted with ethyl acetate (30 mL × 2) and washed with a saturated sodium chloride solution. The organic phase was dried over anhydrous sodium sulfate. After filtration, the filtrate was concentrated under reduced pressure. The residue was purified by silica gel column chromatography with eluent system C to obtain the title compound **12a** (1.04 g, yield: 86.3%).

MS m/z (ESI): 704.2 [M+1].

### Step 2

### (R)-1-((tert-butyldiphenylsilyl)oxy)-3-((4-fluoro-5-iodo-1-methyl-1H-indazol-6-yl)oxy)propan-2-amine 12b

Compound **12a** (1.04 g, 1.48 mmol) was dissolved in dichloromethane (15 mL). Zinc bromide (1.0 g, 4.44 mmol, Adamas) was added and reacted under stirring at room temperature for 16 hours. The reaction solution was poured into 100 mL of water and extracted with ethyl acetate (100 mL × 3). The organic phases were combined, washed with a saturated sodium chloride solution, and dried over anhydrous sodium sulfate. After filtration, the filtrate was concentrated under reduced pressure. The residue was purified by silica gel column chromatography with eluent system A to obtain the title compound **12b** (840 mg, yield: 94.1%).

MS m/z (ESI): 604.1 [M+1].

### Step 3

### Tert-butyl (S)-3-(3-((R)-1-((tert-butyldiphenylsilyl)oxy)-3-((4-fluoro-5-iodo-1-methyl-1H-indazol-6-yl)oxy)prop-2-yl)ureido)-2-(4-fluoro-3,5-dimethylphenyl)-4-methyl-2,4,6,7-tetrahydro-5H-pyrazolo[4,3-c]pyridine-5-carboxylate 12c

Compound **1n** (500 mg, 1.33 mmol, Shanghai Bidepharm) and *N,N-*diisopropylethylamine (860 mg, 6.65 mmol) were dissolved in tetrahydrofuran (10 mL). Under ice bath condition, phenyl chloroformate (310 mg, 1.98 mmol, Adamas) was dropwise added and reacted under stirring at room temperature for 2 hours. Compound **12b** (840 mg, 1.39mmol) was added and stirred at room temperature for 16 hours. The reaction solution was poured into 30 mL of water and extracted with ethyl acetate (30 mL × 3). The organic phases were combined, washed with a saturated sodium chloride solution, and dried over anhydrous sodium sulfate. After filtration, the filtrate was concentrated under reduced pressure. The residue was purified by silica gel column chromatography with eluent system C to obtain the title compound **12c** (450 mg, yield: 33.6%).

MS m/z (ESI): 1004.2 [M+1].

### Step 4

### Tert-butyl (S)-2-(4-fluoro-3,5-dimethylphenyl)-3-(3-((S)-1-((4-fluoro-5-iodo-1-methyl-1H-indazol)-6-yl)oxy)-3-hydroxyprop-2-yl)ureido)-4-methyl-2,4,6,7-tetrahydro-5H-pyrazolo[4,3-c]pyridine-5-carboxylate 12d

Compound **12c** (450 mg, 0.45 mmol) was dissolved in tetrahydrofuran (5.0 mL). A tetrabutylammonium fluoride/tetrahydrofuran solution (0.5 mL, 0.5 mmol, 1.0M) was added and the mixture was reacted at room temperature for 1 hour. The reaction solution was poured into 30 mL of water and extracted with ethyl acetate (30 mL × 3). The organic phases were combined, washed with a saturated sodium chloride solution, and dried over anhydrous sodium sulfate. After filtration, the filtrate was concentrated under reduced pressure. The residue was purified by silica gel column chromatography with eluent system A to obtain the title compound **12d** (330 mg, yield: 96.1%).

MS m/z (ESI): 766.2 [M+1].

### Step 5

### Tert-butyl (S)-2-(4-fluoro-3,5-dimethylphenyl)-3-(3-((R)-1-((4-fluoro-5-iodo-1-methyl-1H-indazol-6-yl)oxy)-3-oxoprop-2-yl)ureido)-4-methyl-2,4,6,7-tetrahydro-5H-pyrazolo[4,3-c]pyridine-5-carboxylate 12e

Compound **12d** (340 mg, 0.44 mmol) was dissolved in dichloromethane (10 mL). Dess-Martin periodinane (230 mg, 0.54 mmol, Adamas) was added. After the addition was complete, the mixture was stirred at room temperature for 6 hours. The reaction solution was washed with separately a saturated aqueous sodium bicarbonate solution (10 mL) and a saturated aqueous sodium thiosulfate solution (10 mL). After liquid separation, the organic phase was dried over anhydrous sodium sulfate. After filtration, the filtrate was concentrated under reduced pressure to obtain the crude title compound **12e** (340 mg).

MS m/z (ESI): 764.2 [M+1].

### Step 6

### Tert-butyl (S)-2-(4-fluoro-3,5-dimethylphenyl)-3-(4-(((4-fluoro-5-iodo-1-methyl-1H-indazol-6-yl)oxy)methyl)-2-oxo-2,3-dihydro-1H-imidazol-1-yl)-4-methyl-2,4,6,7-tetrahydro-5H-pyrazolo[4,3-c]pyridine-5-carboxylate 12f

Crude compound **12e** (340 mg, 0.44 mmol) was dissolved in tetrahydrofuran (10 mL). A hydrogen chloride/dioxane solution (1.0 mL, 4.0 M) was dropwise added at 0°C. After the addition was complete, the mixture was stirred at room temperature for 16 hours. After concentration, a crude product was obtained.

The crude product was dissolved in dichloromethane (10 mL). Di-tert-butyl dicarbonate (120 mg, 0.55 mmol) and bis(triethylamine) (140 mg, 1.38 mmol) were added and the mixture was reacted at room temperature for 2 hours. 30 mL of water was added. The mixture was extracted with ethyl acetate (30 mL × 3). The organic phases were combined, washed with a saturated aqueous sodium chloride solution, and dried over anhydrous sodium sulfate. After filtration, the filtrate was concentrated under reduced pressure. The residue was purified by silica gel column chromatography with eluent system A to obtain the title compound **12f** (110 mg, yield: 33.5%).

MS m/z (ESI): 746.1 [M+1].

### Step 7

### Tert-butyl (S)-2-(4-fluoro-3,5-dimethylphenyl)-3-(10-fluoro-7-methyl-1-oxo-1H,7H-imidazo[1',5':4, 5][1,4]oxazino[3,2-f]indazol-2(4H)-yl)-4-methyl-2,4,6,7-tetrahydro-5H-pyrazolo[4,3-c]pyridine-5-carboxylate 12g

Compound **12f** (110 mg, 0.15 mmol) and *L*-proline (7.0 mg, 0.060 mmol) were dissolved in *N*,*N*-dimethylformamide (10 mL). Potassium carbonate (65 mg, 0.47 mmol) and cuprous iodide (6.0 mg, 0.031 mmol, Leyan) were added. After displacement with nitrogen three times, the mixture was reacted under microwave at 125°C for 1 hours. The reaction solution was poured into 10 mL of water and extracted with ethyl acetate (30 mL × 2). The organic phases were combined, washed with a saturated sodium chloride solution, and dried over anhydrous sodium sulfate. After filtration, the filtrate was concentrated under reduced pressure. The residue was purified by silica gel column chromatography with eluent system C to obtain the title compound **12g** (10 mg, yield: 10.9%).

MS m/z (ESI): 618.2 [M+1].

### Step 8

### 2-((S)-5-(5-((S)-2,2-dimethyltetrahydro-2H-pyran-4-yl)-1-((1S,2S)-2-methyl-1-(5-oxo-4,5-dihydro-1,2,4-oxadiazol-3-yl)cyclopropyl)-1H-indole-2-carbonyl)-2-(4-fluoro-3,5-dimethylphenyl)-4-methyl-4,5,6,7-tetrahydro-2H-pyrazolo[4,3-c]pyridin-3-yl)-10-fluoro-7-methyl-2,4-dihydro-1H,7H-imidazo[1', 5':4,5][1,4]oxazino[3,2-f]indazol-1-one 12

Compound **12g** (10 mg, 0.21 mmol) was dissolved in a hydrogen chloride/dioxane solution (1.0 mL, 4M), stirred at room temperature for 1 hour, and concentrated to obtain a crude product.

The above-mentioned crude product and compound **1w** (10 mg, 0.024 mmol) were dissolved in *N,N*-dimethylformamide (1.0 mL). *N,N*-diisopropylethylamine (7.00 mg, 0.054 mmol) was added. After the raw materials were fully dissolved, *O-*(7-azabenzotriazol-1-yl)-*N,N,N',N'*-tetramethyluronium hexafluorophosphate (15 mg, 0.039 mmol) was added and stirred at room temperature for 3 hours. The reaction solution was concentrated under reduced pressure and purified by preparative high-performance liquid chromatography (instrument model: Waters-2545 chromatographic column: Welch Xtimate, Prep 30 × 250 mm; 5 µm; C18, mobile phase: water (10 mM ammonium bicarbonate) and acetonitrile, with gradient ratio: acetonitrile 40%-95%, flow rate: 30 mL/min) to obtain the title compound **12** (2.0 mg, yield: 12.6%).

MS m/z (ESI): 911.2 [M+1].

¹H NMR (500 MHz, CDCl₃): δ 8.10-8.00 (m, 1H), 7.62-7.58(m, 1H), 7.54-7.49 (m, 1H), 7.32-7.29 (m, 1H), 7.22-7.07 (m, 2H), 6.94-6.86(m, 1H), 6.72-6.70 (m, 1H), 6.30-6.04 (m, 1H), 5.78-5.75 (m, 1H), 5.32-5.28 (m, 1H), 4.92-4.81 (m, 2H), 4.51-4.47 (m, 1H), 4.06-3.97 (m, 3H), 3.91-3.85 (m, 2H), 3.19-3.05 (m, 2H), 2.37-2.30 (m, 6H), 1.92 (t, 1H), 1.80-1.54 (m, 7H), 1.40 -1.22 (m, 11H), 1.07 (d, 1H).

### Example 13

### (R)-7-cyclopropyl-2-((S)-5-(5-((S)-2,2-dimethyltetrahydro-2H-pyran-4-yl)-1-((1S,2S)-2-methyl-1-(5-oxo-4,5-dihydro-1,2,4-oxadiazol-3-yl)cyclopropyl)-1H-indole-2-carbonyl)-2-(4-fluoro-3,5-dimethylphenyl)-4-methyl-4,5,6,7-tetrahydro-2H-pyrazolo[4,3-c]pyridin-3-yl)-10-fluoro-2,3,3a,4-tetrahydro-1H,7H-imidazo[1',5':4,5][1,4]oxazino[3,2-f]indazol-1-one 13

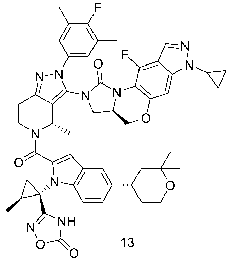

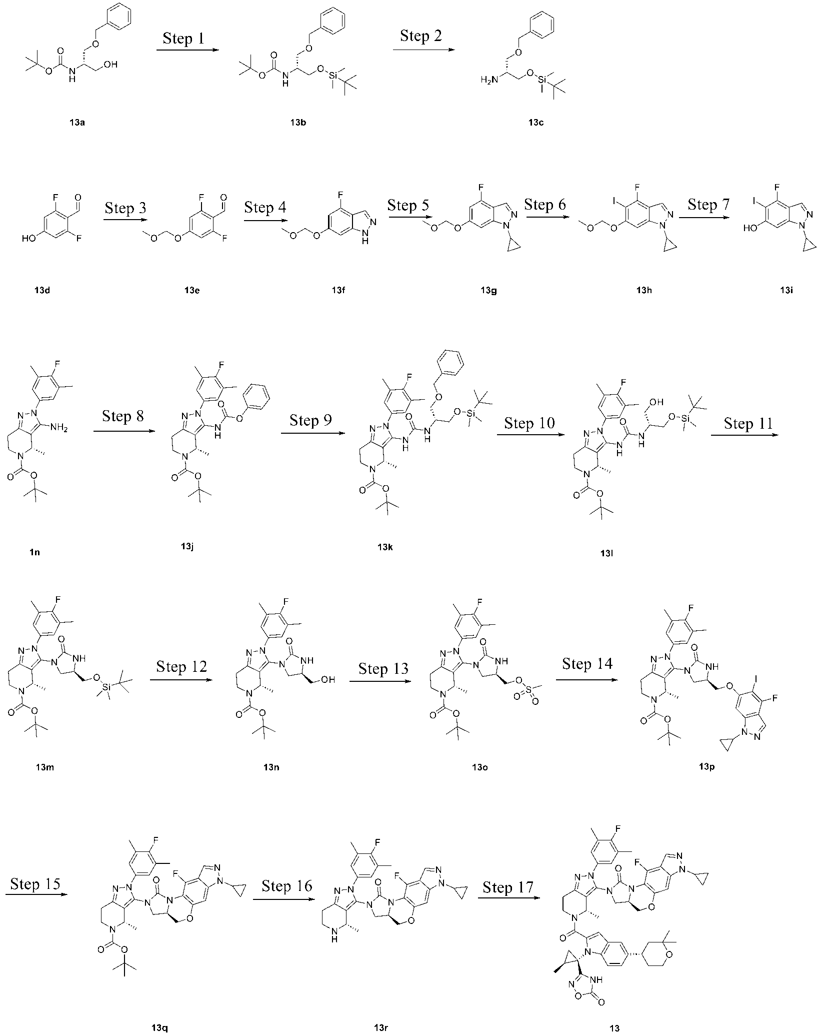

### Step 1

### Tert-butyl (R)-(1-(benzyloxy)-3-((tert-butyldimethylsilyl)oxy)prop-2-yl)carbamate 13b

Tert-butyl (*S*)-(1-(benzyloxy)-3-hydroxyprop-2-yl) **13a** (30 g, 106.63 mmol, WuXi AppTec) and imidazole (14.52 g, 213.25 mmol) were dissolved in *N,N-*dimethylformamide (300 mL). Tert-butyldimethylchlorosilane (19.28 g, 127.95 mmol, Shaoyuan Technology (Shanghai) Co., Ltd.) was added in portions and the mixture was reacted at room temperature for 16 hours. The reaction mixture was quenched with water (50 mL) and extracted with ethyl acetate (150 mL × 3). The organic phase was dried over anhydrous sodium sulfate. After filtration and concentration under reduced pressure, the residue was purified by silica gel column chromatography with eluent system B to obtain the title compound **13b** (40 g, yield: 95%).

MS m/z (ESI): 396.6 [M+1].

### Step 2

### (R)-1-(benzyloxy)-3-((tert-butyldimethylsilyl)oxy)propan-2-amine 13c

Compound **13b** (20 g, 50.55 mmol) was dissolved in dichloromethane (200 mL). Zinc bromide (22 g, 24.9 mmol, Adamas) was added and reacted under stirring at room temperature for 16 hours. After filtration and drying by rotary evaporation, the liquid residue was poured into 200 mL of water and extracted with ethyl acetate (100 mL × 3). The organic phases were combined, washed with a saturated sodium chloride solution, and dried over anhydrous sodium sulfate. After filtration, the filtrate was concentrated under reduced pressure. The residue was purified by silica gel column chromatography with eluent system B to obtain the title compound **13c** (13 g, yield: 87%).

MS m/z (ESI): 296.5 [M+1].

### Step 3

### 2,6-Difluoro-4-(methoxymethoxy)benzaldehyde 13e

2,6-Difluoro-4-hydroxybenzaldehyde **13d** (5.0 g, 31.62 mmol) and *N,N-*diisopropylethylamine (12.26 g, 94.87 mmol) were dissolved in dichloromethane (50 mL). Bromomethyl methyl ether (4.35 g, 34.81 mmol) was dropwise added at 0°C. After the addition was complete, the mixture was stirred at room temperature for 3 hours, quenched with water (100 mL), and extracted with dichloromethane (100 mL × 3). The organic phases were combined and washed with a saturated sodium chloride solution. The organic phase was dried over anhydrous sodium sulfate. After filtration, the filtrate was concentrated under reduced pressure. The residue was purified by silica gel column chromatography with eluent system B to obtain the title compound **13e** (5.3 g, yield: 83%).

MS m/z (ESI): 203.1 [M+1].

### Step 4

### 4-Fluoro-6-(methoxymethoxy)-1H-indazole 13f

Compound **13e** (1 g, 4.95 mmol) and hydrazine hydrate (1.1 g, 17.33 mmol, 85wt%) were dissolved in dioxane (10 mL) and stirred at 100°C for 7 hours. The reaction solution was cooled to room temperature. 200 mL of water was added. A solid was precipitated. After filtration, the solid was washed with water (100 mL × 2) and dried to obtain the crude title compound **13f** (800 mg).

MS m/z (ESI): 197.0 [M+1].

### Step 5

### 1-Cyclopropyl-4-fluoro-6-(methoxymethoxy)-1H-indazole 13g

Compound **13f** (5 g, 25.48 mmol), cyclopropylboronic acid (3.99 g, 17.33 mmol), 2,2'-bipyridine (7.26 g, 25.48 mmol), copper acetate (8.44 g, 46.4758 mmol), and sodium carbonate (7.39 g, 69.72 mmol) were dissolved in 1,2-dichloroethane (50 mL), left open, and stirred at 70°C for 18 hours. The reaction solution was cooled to room temperature, filtered, and concentrated under reduced pressure. The residue was purified by silica gel column chromatography with eluent system B to obtain the title compound **13g** (3.1 g, yield: 51%).

MS m/z (ESI): 237.2 [M+1].

### Step 6

### 1-Cyclopropyl-4-fluoro-5-iodo-6-(methoxymethoxy)-1H-indazole 13h

Compound **13g** (600 mg, 2.53 mmol) was dissolved in tetrahydrofuran (10 mL). Lithium diisopropylamide (2.03 mL, 4.06 mmol, 2M, Energy Chemical) was dropwise added in a nitrogen atmosphere at -78°C. After the addition was complete, stirring was continued at -78°C for 2 hours. A solution of iodine (967 mg, 3.81 mmol)/tetrahydrofuran (10 mL) was then dropwise added. After the addition was complete, stirring was continued at -78°C for 1 hour. The reaction was quenched with a saturated aqueous sodium thiosulfate solution (40 mL) and extracted with ethyl acetate (100 mL × 3). The organic phases were combined and washed with a saturated sodium chloride solution. The organic phase was dried over anhydrous sodium sulfate. After filtration, the filtrate was concentrated under reduced pressure. The residue was purified by silica gel column chromatography with eluent system B to obtain the title compound **13h** (450 mg, yield: 49%).

MS m/z (ESI): 363.1 [M+1].

### Step 7

### 1-Cyclopropyl-4-fluoro-5-iodo-1H-indazol-6-ol 13i

Compound **13h** (400 mg, 1.11 mmol) was dissolved in dichloromethane (5.0 mL). A hydrogen chloride/dioxane solution (5.0 mL, 4M) was dropwise added at 0°C. After the addition was complete, the mixture was stirred at room temperature for 1 hour. After concentration, 30 mL of water was added. The mixture was extracted with ethyl acetate (30 mL × 3). The organic phases were combined, washed with a saturated aqueous sodium chloride solution, and dried over anhydrous sodium sulfate. After filtration, the filtrate was concentrated under reduced pressure. The residue was purified by silica gel column chromatography with eluent system B to obtain the title compound **13i** (300 mg, yield: 85%).

MS m/z (ESI): 319.1 [M+1].

### Step 8

### Tert-butyl (S)-2-(4-fluoro-3,5-dimethylphenyl)-4-methyl-3-((phenoxycarbonyl)amino)-2,4,6,7-tetrahydro-5H-pyrazolo[4,3-c]pyridine-5-carboxylate 13j

Compound **1n** (10 g, 26.71 mmol, Shanghai Bidepharm) and *N,N-*diisopropylethylamine (10.35 g, 80.12 mmol) were dissolved in tetrahydrofuran (100 mL). Under ice bath condition, phenyl chloroformate (7.11 g, 45.39 mmol, Adamas) was dropwise added and reacted under stirring at room temperature for 2 hours. The reaction solution was poured into 30 mL of water and extracted with ethyl acetate (30 mL × 3). The organic phases were combined, washed with a saturated sodium chloride solution, and dried over anhydrous sodium sulfate. After filtration, the filtrate was concentrated under reduced pressure. The residue was stirred with petroleum ether and filtered to obtain the crude title compound **13j** (12 g). The crude title compound was directly used in the next reaction.

MS m/z (ESI): 495.5 [M+1].

### Step 9

### Tert-butyl (S)-3-(3-((R)-1-(benzyloxy)-3-((tert-butyldimethylsilyl)oxy)prop-2-yl)ureido)-2-(4-fluoro-3,5-dimethylphenyl)-4-methyl-2,4,6,7-tetrahydro-5H-pyrazolo[4,3-c]pyridine-5-carboxylate 13k

Compound **13j** (5 g, 10.11 mmol) and *N,N-*diisopropylethylamine (3.92 g, 30.33 mmol) were dissolved in tetrahydrofuran (50 mL). Compound **13c** (8.96 g, 30.33 mmol) was added and stirred at room temperature for 5 hours. The reaction solution was poured into 30 mL of water and extracted with ethyl acetate (50 mL × 3). The organic phases were combined, washed with a saturated sodium chloride solution, and dried over anhydrous sodium sulfate. After filtration, the filtrate was concentrated under reduced pressure. The residue was purified by silica gel column chromatography with eluent system B to obtain the title compound **13k** (6.0 g, yield: 85%).

MS m/z (ESI): 696.6 [M+1].

### Step 10

### Tert-butyl (S)-3-(3-((R)-1-((tert-butyldimethylsilyl)oxy)-3-hydroxyprop-2-yl)ureido)-2-(4-fluoro-3,5-dimethylphenyl)-4-methyl-2,4,6,7-tetrahydro-5H-pyrazolo[4,3-c]pyridine-5-carboxylate 13l

Compound **13k** (5 g, 7.18 mmol) was dissolved in ethanol (50 mL). Palladium on carbon (2.29 g, 2.15 mmol, 10 wt% (containing about 55% of water)) was added. The mixture was heated to 50°C in a hydrogen atmosphere and reacted fro 3 hours. The mixture was cooled to room temperature and filtered to remove palladium on carbon. The filtrate was concentrated under reduced pressure. The residue was purified by silica gel column chromatography with eluent system B to obtain the title compound **13l** (3.8 g, yield: 87%).

MS m/z (ESI): 606.6 [M+1].

### Step 11

### Tert-butyl (S)-3-((R)-4-(((tert-butyldimethylsilyl)oxy)methyl)-2-oxoimidazolidin-1-yl)-2-(4-fluoro-3,5-dimethylphenyl)-4-methyl-2,4,6,7-tetrahydro-5H-pyrazolo[4,3-c]pyridine-5-carboxylate 13m

Compound **13l** (2.8 g, 4.62 mmol) and triphenylphosphine (1.27 g, 4.85 mmol) were dissolved in dichloromethane (50 mL). Bis(4-chlorobenzyl) azodicarboxylate (1.78 mg, 4.85 mmol) was added at 35°C and the mixture was reacted at room temperature for 5 hours. After filtration, the filtrate was concentrated under reduced pressure. The residue was purified by silica gel column chromatography with eluent system B and then purified by preparative high-performance liquid chromatography (instrument model: Waters-2545 chromatographic column: Sharpsil-T, Prep 30 × 150 mm; 5 µm; C18 mobile phase: water (0.1% trifluoroacetic acid) and acetonitrile, with gradient ratio: acetonitrile 50%-95%, flow rate: 30 mL/min) to obtain the title compound **13m** (1.5 g, yield: 55%).

MS m/z (ESI): 588.6 [M+1].

### Step 12

### Tert-butyl (S)-2-(4-fluoro-3,5-dimethylphenyl)-3-((R)-4-(hydroxymethyl)-2-oxoimidazolidin-1-yl)-4-methyl-2,4,6,7-tetrahydro-5H-pyrazolo[4,3-c]pyridine-5-carboxylate 13n

Compound **13m** (600 mg, 1.02 mmol) was dissolved in tetrahydrofuran (10 mL). Tetrabutylammonium fluoride/tetrahydrofuran solution (1.02 mL, 1.0M) was added and the mixture was reacted at room temperature for 1 hour. The reaction solution was poured into 30 mL of water and extracted with ethyl acetate (30 mL × 3). The organic phases were combined, washed with a saturated sodium chloride solution, and dried over anhydrous sodium sulfate. After filtration, the filtrate was concentrated under reduced pressure. The residue was purified by silica gel column chromatography with eluent system B to obtain the title compound **13n** (430 mg, yield: 89%).

MS m/z (ESI): 474.4 [M+1].

### Step 13

### Tert-butyl (S)-2-(4-fluoro-3,5-dimethylphenyl)-4-methyl-3-((R)-4-(((methylsulfonyl)oxy)methyl)-2-oxoimidazolidin-1-yl)-2,4,6,7-tetrahydro-5H-pyrazolo[4,3-c]pyridine-5-carboxylate 13o

Compound **13n** (230 mg, 0.48 mmol) and pyridine (115 mg, 1.45 mmol) were dissolved in dichloromethane (5.0 mL) and tetrahydrofuran (2.0 mL). Methanesulfonic anhydride(109 mg, 0.62 mmol) was added at 0°C and the mixture was reacted at room temperature for 3 hours. The reaction solution was poured into 20 mL of water and extracted with dichloromethane (20 mL × 2). The organic phases were combined, washed with a saturated sodium chloride solution, and dried over anhydrous sodium sulfate. After filtration, the filtrate was concentrated under reduced pressure to obtain the crude title compound **13o** (230 mg).

MS m/z (ESI): 552.1 [M+1].

### Step 14

### Tert-butyl (S)-3-((R)-4-(((1-cyclopropyl-4-fluoro-5-iodo-1H-indazol-6-yl)oxy)methyl)-2-oxoimidazolidin-1-yl)-2-(4-fluoro-3,5-dimethylphenyl)-4-methyl-2,4,6,7-tetrahydro-5H-pyrazolo[4,3-c]pyridine-5-carboxylate 13p

Compound **13o** (150 mg, 0.27 mmol) and compound **13i** (91 mg, 0.28 mmol) were dissolved in *N,N-*dimethylformamide (5.0 mL). Potassium carbonate (113 mg, 0.81 mmol) was added and reacted at 80°C for 3 hours. The reaction solution was poured into 20 mL of water and extracted with ethyl acetate (30 mL × 2). The organic phases were combined, washed with a saturated sodium chloride solution, and dried over anhydrous sodium sulfate. After filtration, the filtrate was concentrated under reduced pressure. The residue was purified by silica gel column chromatography with eluent system A to obtain the title compound **13p** (150 mg, yield: 71%).

MS m/z (ESI): 774.2 [M+1].

### Step 15

### Tert-butyl (S)-3-((R)-7-cyclopropyl-10-fluoro-1-oxo-3a,4-dihydro-1H,7H-imidazo[1',5':4,5][1,4]oxazino[3,2-f]indazol-2(3H)-yl)-2-(4-fluoro-3,5-dimethylphenyl)-4-methyl-2,4,6,7-tetrahydro-5H-pyrazolo[4,3-c]pyridine-5-carboxylate 13q

Compound **13p** (150 mg, 0.19 mmol) and *L*-proline (23 mg, 0.19 mmol) were dissolved in *N*,*N*-dimethylformamide (5.0 mL). Potassium carbonate (81 mg, 0.58 mmol) and cuprous iodide (19 mg, 0.09 mmol, Leyan) were added. After displacement with nitrogen three times, the mixture was heated to 120°C and reacted under stirring for 3 hours. The reaction solution was poured into 20 mL of water and extracted with ethyl acetate (30 mL × 2). The organic phases were combined, washed with a saturated sodium chloride solution, and dried over anhydrous sodium sulfate. After filtration, the filtrate was concentrated under reduced pressure. The residue was purified by silica gel column chromatography with eluent system C to obtain the title compound **13q** (80 mg, yield: 63%).

MS m/z (ESI): 646.5 [M+1].

### Step 16

### (R)-7-cyclopropyl-10-fluoro-2-((S)-2-(4-fluoro-3,5-dimethylphenyl)-4-methyl-4,5,6,7-tetrahydro-2H-pyrazolo[4,3-c]pyridin-3-yl)-2,3,3a,4-tetrahydro-1H,7H-imidazo[1',5':4,5][1,4]oxazino[3,2-f]indazol-1-one 13r

Compound **13q** (80 mg, 0.12 mmol) was dissolved in methanol (2 mL). A 4 M solution of hydrogen chloride in 1,4-dioxane (2 mL) was added and stirred at room temperature for 2 hours. The reaction solution was concentrated under reduced pressure to obtain the crude title compound **13r** (60 mg). The product was directly used in the next reaction without purification.

MS m/z (ESI): 546.5 [M+1].

### Step 17

### (R)-7-cyclopropyl-2-((S)-5-(5-((S)-2,2-dimethyltetrahydro-2H-pyran-4-yl)-1-((1S,2S)-2-methyl-1-(5-oxo-4,5-dihydro-1,2,4-oxadiazol-3-yl)cyclopropyl)-1H-indole-2-carbonyl)-2-(4-fluoro-3,5-dimethylphenyl)-4-methyl-4,5,6,7-tetrahydro-2H-pyrazolo[4,3-c]pyridin-3-yl)-10-fluoro-2,3,3a,4-tetrahydro-1H,7H-imidazo[1',5':4,5][1,4]oxazino[3,2-f]indazol-1-one 13

Compound **13r** (60 mg, 0.11mmol) and compound **1w** (45 mg, 0.11 mmol) were dissolved in *N,N*-dimethylformamide (3 mL). *N,N*-diisopropylethylamine (42 mg, 0.33 mmol) was added. After the raw materials were fully dissolved, *O*-(7-azabenzotriazol-1-yl)-*N,N,N',N'*-tetramethyluronium hexafluorophosphate (41 mg, 0.12 mmol) was added and stirred at room temperature for 16 hours. The reaction solution was poured into 10 mL of water and extracted with ethyl acetate (10 mL × 3). The organic phases were combined, washed with a saturated sodium chloride solution, and dried over anhydrous sodium sulfate. After filtration, the filtrate was concentrated under reduced pressure. The residue was purified by preparative high-performance liquid chromatography (instrument model: Waters-2545 chromatographic column: Welch Xtimate, Prep 30 × 250 mm; 5 µm; C18, mobile phase: water (10 mM ammonium bicarbonate) and acetonitrile, with gradient ratio: acetonitrile 40%-95%, flow rate: 30 mL/min) to obtain the title compound **13** (60 mg, yield: 58%).

MS m/z (ESI): 939.7 [M+1].

¹H NMR (500 MHz, CDCl₃): δ 7.98 (s, 1H), 7.61(d, 1H), 7.55-7.48 (m, 2H), 7.21-7.15 (m, 2H), 6.93 (s, 1H),6.71(s, 1H), 5.81-5.76 (m, 1H), 4.48-4.42 (m, 1H), 4.29-4.20 (m, 1H), 4.11-3.95 (m, 3H), 3.92-3.76 (m, 3H), 3.71-3.60 (m, 2H), 3.54-3.48(m, 1H), 3.18-2.95(m, 5H), 2.30-2.26 (m, 6H), 1.94 (t, 1H), 1.83-1.55 (m, 7H), 1.40 -1.22 (m, 12H), 1.06 (d, 1H).

### Example 14

### (R)-2-((S)-5-(5-((S)-2,2-dimethyltetrahydro-2H-pyran-4-yl)-1-((1S,2S)-2-methyl-1-(5-oxo-4,5-dihydro-1,2,4-oxadiazol-3-yl)cyclopropyl)-1H-indole-2-carbonyl)-2-(4-fluoro-3,5-dimethylphenyl)-4-methyl-4,5,6,7-tetrahydro-2H-pyrazolo[4,3-c]pyridin-3-yl)-11-fluoro-8-methyl-2,3,3a,4,6,8-hexahydro-1H-imidazo[5',1':3,4][1,4]oxazepino[5,6-f]indazol-1-one 14

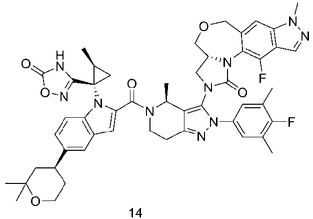

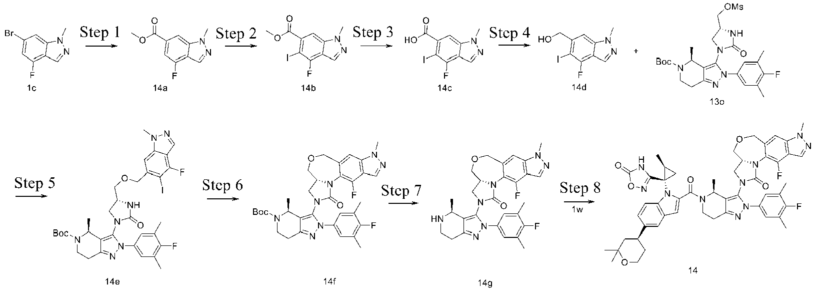

### Step 1

Methyl 4-fluoro-1-methyl-1*H*-indazole-6-carboxylate **14a** 6-Bromo-4-fluoro-1-methyl-1*H*-indazole **1c** (1.00 g, 4.37 mmol) was dissolved in *N*,*N*-dimethylformamide (20 mL) and methanol (20 mL). [1,1'-Bis(diphenylphosphino)ferrocene]palladium dichloride (639 mg, 0.87 mmol, Shanghai Bidepharm) and triethylamine (1.76 g, 17.39 mmol) were added. After displacement with carbon monoxide three times, the mixture was heated to 90°C and reacted for 16 hours. After filtration and concentration under reduced pressure, the residue was purified by silica gel column chromatography with eluent system B to obtain the title compound **14a** (400 mg, yield: 44%).

MS m/z (ESI): 209.1 [M+1].

### Step 2

Methyl 4-fluoro-5-iodo-1-methyl-1*H*-indazole-6-carboxylate **14b** Compound **14a** (570 mg, 2.74 mmol) was dissolved in tetrahydrofuran (30 mL). Lithium diisopropylamide (2.05 mL, 4.11 mmol, 2M, Energy Chemical) was dropwise added at -78°C in a nitrogen atmosphere. After the addition was complete, stirring was continued at -78°C for 1 hour. A solution of iodine (1.04 g, 4.10 mmol)/tetrahydrofuran (20 mL) was then dropwise added. After the addition was complete, stirring was continued at -78°C for 1 hour. The reaction was quenched with a saturated aqueous sodium thiosulfate solution (50 mL) and extracted with ethyl acetate (100 mL × 3). The organic phases were combined and washed with a saturated sodium chloride solution. The organic phase was dried over anhydrous sodium sulfate. After filtration, the filtrate was concentrated under reduced pressure. The residue was purified by silica gel column chromatography with eluent system B to obtain the title compound **14b** (360 mg, yield: 39%).

MS m/z (ESI): 335.1 [M+1].

### Step 3

4-Fluoro-5-iodo-1-methyl-1*H*-indazole-6-carboxylic acid **14c** Compound **14b** (310 mg, 0.93 mmol) was dissolved in tetrahydrofuran (10 mL) and water (10 mL). Lithium hydroxide monohydrate (156 g, 3.71 mmol) was added. After the addition was complete, the mixture was stirred at 35°C for 16 hours. The reaction solution was adjusted to pH = 6 with dilute hydrochloric acid and concentrated under reduced pressure to obtain the title compound **14c** (280 mg, yield: 94%).

MS m/z (ESI): 321.0 [M+1].

### Step 4

### (4-Fluoro-5-iodo-1-methyl-1H-indazol-6-yl)methanol 14d

Compound **14c** (300 mg, 0.94 mmol) was dissolved in tetrahydrofuran (10 mL). Borane tetrahydrofuran (3.75 mL, 3.75 mmol, 1M, adamas) was dropwise added at 0°C and stirred at room temperature for 16 hours. The reaction solution was cooled to room temperature. 20 mL of methanol was added and stirred for 1 hour. The mixture was concentrated under reduced pressure. The residue was purified by silica gel column chromatography with eluent system B to obtain the title compound **14d** (280 mg, yield: 98%).

MS m/z (ESI): 307.0 [M+1].

### Step 5

### Tert-butyl (S)-2-(4-fluoro-3,5-dimethylphenyl)-3-((R)-4-(((4-fluoro-5-iodo-1-methyl-1H-indazol-6-yl)methoxy)methyl)-2-oxoimidazolidin-1-yl)-4-methyl-2,4,6,7-tetrahydro-5H-pyrazolo[4,3-c]pyridine -5-carboxylate 14e

Compound **14d** (122 mg, 0.40 mmol) was dissolved in *N,N-*dimethylformamide (5.0 mL). Sodium hydride (23 mg, 0.60 mmol, 60% dispersed in a mineral oil) was added at 0°C. The mixture was stirred at 0°C for 30 minutes, and a solution of compound **13o** (220 mg, 0.40 mmol) in N,N-dimethylformamide (2.0 mL) was added. The mixture was stirred at 0°C for 30 minutes and at room temperature for 1 hour. The reaction solution was poured into 30 mL of water and extracted with ethyl acetate (50 mL × 3). The organic phases were combined, washed with a saturated sodium chloride solution, and dried over anhydrous sodium sulfate. After filtration, the filtrate was concentrated under reduced pressure. The residue was purified by silica gel column chromatography with eluent system B to obtain the title compound **14e** (20 mg, yield: 7%).

MS m/z (ESI): 762.4 [M+1].

### Step 6

### Tert-butyl (S)-2-(4-fluoro-3,5-dimethylphenyl)-3-((R)-11-fluoro-8-methyl-1-oxo-3a,4,6,8-tetrahydro-1H-imidazo[5',1':3,4][1,4]oxazepino[5,6-f]indazol-2(3H)-yl)-4-methyl-2,4,6,7-tetrahydro-5H-pyrazolo[4,3-c]pyridine-5-carboxylate 14f

Compound **14e** (20 mg, 0.026 mmol) and *L*-proline (6 mg, 0.052 mmol) were dissolved in *N,N*-dimethylformamide (5.0 mL). Potassium carbonate (11 mg, 0.079 mmol) and cuprous iodide (5 mg, 0.026 mmol, Leyan) were added. After displacement with nitrogen three times, the mixture was heated to 120°C and reacted under stirring for 3 hours. The reaction solution was poured into 20 mL of water and extracted with ethyl acetate (30 mL × 2). The organic phases were combined, washed with a saturated sodium chloride solution, and dried over anhydrous sodium sulfate. After filtration, the filtrate was concentrated under reduced pressure. The residue was purified by silica gel column chromatography with eluent system A to obtain the title compound **14f** (16 mg, yield: 96%).

MS m/z (ESI): 634.4 [M+1].

### Step 7

### (R)-11-fluoro-2-((S)-2-(4-fluoro-3,5-dimethylphenyl)-4-methyl-4,5,6,7-tetrahydro-2H-pyrazolo[4,3-c]pyridin-3-yl)-8-methyl-2,3,3a,4,6,8-hexahydro-1H-imidazo[5',1':3,4][1,4]oxazepino[5,6-f]indazol-1-one 14g

Compound **14f** (16 mg, 0.025 mmol) was dissolved in methanol (2 mL). A 4 M solution of hydrogen chloride in 1,4-dioxane (2 mL) was added and stirred at room temperature for 2 hours. The reaction solution was concentrated under reduced pressure to obtain the crude title compound **14g** (14 mg, yield: 97%). The product was directly used in the next reaction without purification.

MS m/z (ESI): 534.4 [M+1].

### Step 8

### (R)-2-((S)-5-(5-((S)-2,2-dimethyltetrahydro-2H-pyran-4-yl)-1-((1S,2S)-2-methyl-1-(5-oxo-4,5-dihydro-1,2,4-oxadiazol-3-yl)cyclopropyl)-1H-indole-2-carbonyl)-2-(4-fluoro-3,5-dimethylphenyl)- 4-methyl-4,5,6,7-tetrahydro-2H-pyrazolo[4,3-c]pyridin-3-yl)-11-fluoro-8-methyl-2,3,3a,4,6,8 -hexahydro-1H-imidazo[5',1':3,4][1,4]oxazepino[5,6-f]indazol-1-one 14

Compound **14g** (14 mg, 0.025 mmol) and compound **1w** (10 mg, 0.025 mmol) were dissolved in *N,N*-dimethylformamide (5 mL). *N,N*-diisopropylethylamine (16 mg, 0.12 mmol) was added. After the raw materials were fully dissolved, *O*-(7-azabenzotriazol-1-yl)-*N,N,N',N'*-tetramethyluronium hexafluorophosphate(14 mg, 0.037 mmol) was added and stirred at room temperature for 16 hours. The reaction solution was poured into 10 mL of water and extracted with ethyl acetate (10 mL × 3). The organic phases were combined, washed with a saturated sodium chloride solution, and dried over anhydrous sodium sulfate. After filtration, the filtrate was concentrated under reduced pressure. The residue was purified by preparative high-performance liquid chromatography (instrument model: Waters-2545, chromatographic column: Sharpsil-T, Prep 30 × 150 mm; 5 µm; C18, mobile phase: water (10 mM ammonium bicarbonate) and acetonitrile, with gradient ratio: acetonitrile 40%-90%, flow rate: 30 mL/min) to obtain the title compound **14** (5 mg, yield: 22%).

MS m/z (ESI): 927.5 [M+1].

¹H NMR (500 MHz, CDCl₃): δ11.36-11.22(m, 1H), 8.04-7.98(m, 1H), 7.60-7.58(m, 1H), 7.50-7.47(m, 1H), 7.27-7.26(m, 1H), 7.20-7.13(m, 2H), 6.70-6.69(m, 1H), 5.79-5.75(m, 1H), 5.38-5.21(m, 1H), 4.87-4.77(m, 1H), 4.60-4.51(m, 1H), 4.47-4.43(m, 1H), 4.25-4.23(m, 3H), 4.12-3.96(m, 2H), 3.90-3.58(m, 3H), 3.40-2.94(m, 4H), 2.34-2.30(m, 6H), 2.03-1.91(m, 1H), 1.80-1.51(m, 8H), 1.35 -1.22(m, 12H).

### Example 15

### 3-((1S,2S)-1-(5-((S)-2,2-dimethyltetrahydro-2H-pyran-4-yl)-2-((S)-3-(2-(4-fluoro-1-methyl-1H-indazol-5-yl)-5-oxo-5H-imidazo[1,5-a]imidazol-6(7H)-yl)-2-(4-fluoro-3,5-dimethylphenyl)-4-methyl-4,5,6,7-tetrahydro-2H-pyrazolo[4,3-c]pyridine-5-carbonyl)-1H-indol-1-yl)-2-methylcyclopropyl)-1,2,4-oxadiazol-5(4H)-one 15

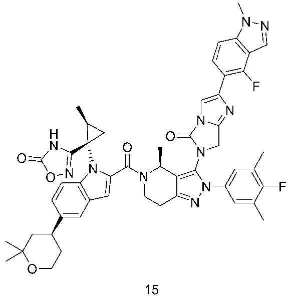

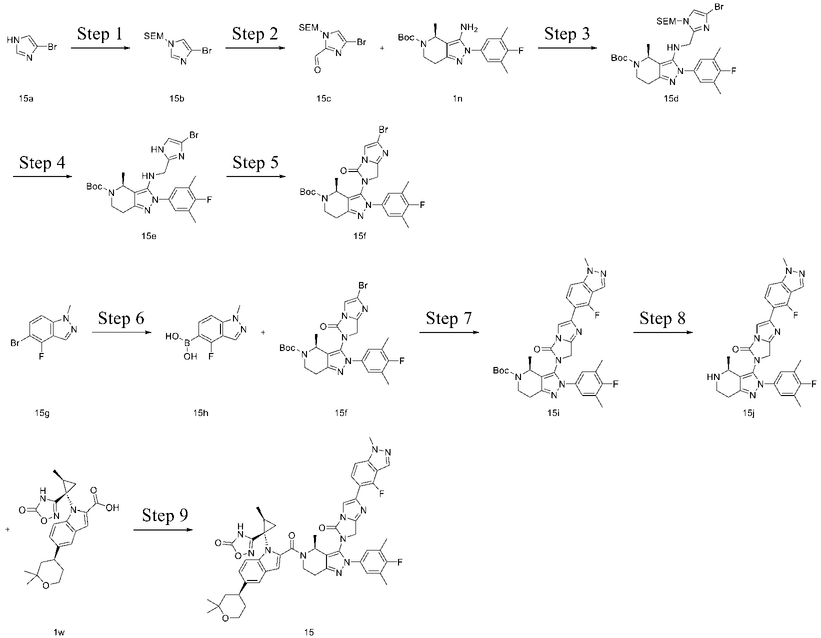

### Step 1

### 4-Bromo-1-((2-(trimethylsilyl)ethoxy)methyl)-1H-imidazole 15b

4-Bromo-1*H*-imidazole **15a** (4.0 g, 27.21 mmol, Accela) was dissolved in tetrahydrofuran (60 mL). Sodium hydride (3.0 g, 69.19 mmol, 60% dispersed in a mineral oil) was added at 0°C and stirred for 1 hour, followed by the addition of 2-(trimethylsilyl)ethoxymethyl chloride (6.8 g, 40.79 mmol, Accela) and further stirring at 0°C for 1 hour. The reaction solution was quenched with 50 mL of a saturated aqueous ammonium chloride solution and extracted with ethyl acetate (80 mL × 3). The organic phases were combined. The combined organic phase was dried over anhydrous sodium sulfate. After filtration, the filtrate was concentrated under reduced pressure. The residue was purified by silica gel column chromatography with eluent system B to obtain the title compound **15b** (4.8 g, yield: 63.6%).

MS m/z (ESI): 277.1 [M+1].

### Step 2

### 4-Bromo-1-((2-(trimethylsilyl)ethoxy)methyl)-1H-imidazole-2-carboxaldehyde 15c

Compound **15b** (3.3 g, 11.90 mmol) was dissolved in tetrahydrofuran (30 mL). After displacement with nitrogen, lithium diisopropylamide (8.96 mL, 2.0M) was dropwise added at -78°C and stirred for 1 hour, followed by the dropwise addition of N,N-dimethylformamide (1.74 g, 23.81 mmol). The reaction was further stirred at -78°C for 1 hour. The reaction mixture was quenched with 30 mL of a saturated aqueous ammonium chloride solution and extracted with ethyl acetate (50 mL × 3). The organic phases were combined. The combined organic phase was dried over anhydrous sodium sulfate. After filtration, the filtrate was concentrated under reduced pressure. The residue was purified by silica gel column chromatography with eluent system A to obtain the title compound **15c** (2.9 g, yield: 79.8%).

### Step 3

### Tert-butyl (S)-3-(((4-bromo-1-((2-(trimethylsilyl)ethoxy)methyl)-1H-imidazol-2-yl)methyl)amino)-2-(4-fluoro-3,5-dimethylphenyl)-4-methyl-2,4,6,7-tetrahydro-5H-pyrazolo[4,3-c]pyridine-5-carboxylate 15d

Compound **1n** (500 mg, 1.34 mmol) and compound **15c** (1.23 g, 4.03 mmol) were dissolved in 1,2-dichloroethane (20 mL). Tetraisopropyl titanate (1.9 g, 6.69 mmol) was added and stirred at 80°C overnight. The reaction solution was cooled to room temperature. Methanol (20 mL) was added. Sodium borohydride (350 mg, 2.69 mmol) was slowly added under ice-water bath condition. The mixture was further stirred at room temperature for 0.5 hours. The reaction solution was concentrated under reduced pressure. The residue was purified by silica gel column chromatography with eluent system A to obtain the title compound **15d** (886 mg, yield: 99.9%).

MS m/z (ESI): 663.5 [M+1].

### Step 4

### Tert-butyl (S)-3-(((4-bromo-1H-imidazol-2-yl)methyl)amino)-2-(4-fluoro-3,5-dimethylphenyl)-4-methyl-2,4,6,7-tetrahydro-5H-pyrazolo[4,3-c]pyridine-5-carboxylate 15e

Compound **15d** (886 mg, 1.34 mmol) was dissolved in tetrahydrofuran (20 mL). A tetrabutylammonium fluoride/tetrahydrofuran solution (6.70 mL, 1.0M, Adamas) was added at room temperature. The mixture was heated to 70°C and reacted fro 1 hour. The reaction solution was cooled to room temperature and poured into 40 mL of water. The mixture was extracted with ethyl acetate (50 mL × 3). The organic phases were combined, washed with a saturated sodium chloride solution, and dried over anhydrous sodium sulfate. After filtration, the filtrate was concentrated under reduced pressure. The residue was purified by silica gel column chromatography with eluent system A to obtain the title compound **15e** (600 mg, yield: 84.3%).

MS m/z (ESI): 533.3 [M+1].

### Step 5

### Tert-butyl (S)-3-(2-bromo-5-oxo-5H-imidazo[1,5-a]imidazol-6(7H)-yl)-2-(4-fluoro-3,5-dimethylphenyl)-4-methyl-2,4,6,7-tetrahydro-5H-pyrazolo[4,3-c]pyridine-5-carboxylate 15f

Compound **15e** (300 mg, 0.56 mmol) and triphosgene (167 mg, 0.56 mmol, Adamas) were dissolved in dichloromethane (20 mL). Triethylamine (285 mg, 2.82 mmol) was added under ice-water bath condition. The reaction was heated to room temperature and stirred for 1 hour. The reaction mixture was diluted with dichloromethane (30 mL) and washed with water (10 mL × 2). The organic phase was dried over anhydrous sodium sulfate. After filtration, the filtrate was concentrated under reduced pressure. The residue was purified by silica gel column chromatography with eluent system B to obtain the title compound **15f** (200 mg, yield: 63.6%).

MS m/z (ESI): 559.3 [M+1].

### Step 6

### (4-Fluoro-1-methyl-1H-indazol-5-yl)boronic acid 15h

5-Bromo-4-fluoro-1-methyl-1*H*-indazole **15g** (250 mg, 1.09 mmol, Leyan) was dissolved in tetrahydrofuran (5 mL). After displacement with nitrogen, n-butyllithium (0.52 mL, 2.5M, Adamas) was dropwise added at -78°C and stirred for 1 hour, followed by the addition of trimethyl borate(227 mg, 2.18 mmol, Adamas). The mixture was further stirred at -78°C for 1 hour. The reaction solution was quenched with 10 mL of a saturated aqueous ammonium chloride solution and extracted with ethyl acetate (20 mL × 3). The organic phases were combined. The combined organic phase was dried over anhydrous sodium sulfate. After filtration, the filtrate was concentrated under reduced pressure. The residue was purified by silica gel column chromatography with eluent system A to obtain the title compound **15h** (120 mg, yield: 56.7%).

MS m/z (ESI): 195.2 [M+1].

### Step 7

### Tert-butyl (S)-3-(2-(4-fluoro-1-methyl-1H-indazol-5-yl)-5-oxo-5H-imidazo[1,5-a]imidazol-6(7H)-yl)-2-(4-fluoro-3,5-dimethylphenyl)-4-methyl-2,4,6,7-tetrahydro-5H-pyrazolo[4,3-c]pyridine-5-carboxylate 15i

Compound **15h** (30 mg, 0.15 mmol), compound **15f** (70 mg, 0.13 mmol), anhydrous potassium carbonate (52 mg, 0.38 mmol), and [1,1'-bis(diphenylphosphino)ferrocene]palladium dichloride (10 mg, 0.014 mmol, Bidepharm) were dissolved in dioxane (4 mL) and water (1 mL). After displacement with nitrogen, the reaction was stirred at 100°C for 3 hours. The reaction solution was cooled to room temperature and concentrated under reduced pressure. The residue was purified by silica gel column chromatography with eluent system A to obtain the title compound **15i** (60 mg, yield: 76.3%).

MS m/z (ESI): 629.5 [M+1].

### Step 8

### (S)-2-(4-fluoro-1-methyl-1H-indazol-5-yl)-6-(2-(4-fluoro-3,5-dimethylphenyl)-4-methyl-4,5,6,7-tetrahydro-2H-pyrazolo[4,3-c]pyridin-3-yl)-6,7-dihydro-5H-imidazo[1,5-a]imidazol-5-one 15j

Compound **15i** (30 mg, 0.048 mmol) was dissolved in dichloromethane (1 mL). A 4M solution of hydrogen chloride in dioxane (4 mL) was added. The reaction was stirred at room temperature for 1 hour. The reaction solution was concentrated under reduced pressure to obtain the crude title compound **15j** (25 mg). The product was directly used in the next reaction without purification.

MS m/z (ESI): 529.5 [M+1].

### Step 9

### 3-((1S,2S)-1-(5-((S)-2,2-dimethyltetrahydro-2H-pyran-4-yl)-2-((S)-3-(2-(4-fluoro-1-methyl-1H-indazol-5-yl)-5-oxo-5H-imidazo[1,5-a]imidazol-6(7H)-yl)-2-(4-fluoro-3,5-dimethylphenyl)-4-methyl-4,5,6,7-tetrahydro-2H-pyrazolo[4,3-c]pyridine-5-carbonyl)-1H-indol-1-yl)-2-methylcyclopropyl)-1,2,4-oxadiazol-5(4H)-one 15

Compound **15j** (25 mg, 0.047 mmol) and compound **1w** (20 mg, 0.049 mmol) were dissolved in *N,N*-dimethylformamide (3 mL). *N,N*-diisopropylethylamine (32 mg, 0.25 mmol) was added. After the raw materials were fully dissolved, *O*-(7-azabenzotriazol-1-yl)-*N,N,N',N'*-tetramethyluronium hexafluorophosphate (22 mg, 0.058 mmol) was added and stirred at room temperature for 16 hours. The reaction solution was poured into 10 mL of water and extracted with ethyl acetate (10 mL × 3). The organic phases were combined, washed with a saturated sodium chloride solution, and dried over anhydrous sodium sulfate. After filtration, the filtrate was concentrated under reduced pressure. The residue was purified by preparative high-performance liquid chromatography (instrument model: Gilson-GX-281, chromatographic column: Boston Phlex, Prep 30 × 150 mm; 5 µm; C18, mobile phase: water (10 mM ammonium bicarbonate) and acetonitrile, with gradient ratio: acetonitrile 45%-80%, flow rate: 30 mL/min) to obtain the title compound **15** (20 mg, yield: 44.6%).

MS m/z (ESI): 920.7 [M-1].

¹H NMR (500 MHz, CDCl₃): δ 11.19 (s, 1H), 8.21-7.98 (m, 2H), 7.95-7.67 (m, 1H), 7.62-7.55 (m, 1H), 7.53-7.43 (m, 1H), 7.31-7.27 (m, 1H), 7.25-7.19 (m, 1H), 7.16-7.03 (m, 2H), 6.75-6.64 (m, 1H), 5.79 (q, 1H), 5.45-5.13 (m, 1H), 4.90-4.39 (m, 3H), 4.31(q, 1H), 4.15-4.03 (m, 3H), 3.91-3.77 (m, 2H), 3.63-3.34 (m, 1H), 3.21-2.92 (m, 3H), 2.29-2.20 (m, 6H), 1.93-1.61 (m, 8H), 1.36-1.30 (m, 5H), 1.18 (d, 2H), 1.07 (d, 1H), 0.88 (t, 1H).

### Example 16-1 or 16-2

### 3-((1S,2S)-1-(5-((S)-2,2-dimethyltetrahydro-2H-pyran-4-yl)-2-((S)-2-(4-fluoro-3,5-dimethylphenyl)-3-(((S,Z)-10-fluoro-7-methyl-3a,4-dihydro-1H,3H,7H-oxazolo[3',4':4,5][1,4]oxazino[3,2-f]indazol-1-ylidene)amino)-4-methyl-4,5,6,7-tetrahydro-2H-pyrazolo[4,3-c]pyridine-5-carbonyl)-1H-indol-1-yl)-2-methylcyclopropyl)-1,2,4-oxadiazol-5(4H)-one 16-1, or

### 3-((1S,2S)-1-(5-((S)-2,2-dimethyltetrahydro-2H-pyran-4-yl)-2-((S)-2-(4-fluoro-3,5-dimethylphenyl)-3-(((S,E)-10-fluoro-7-methyl-3a,4-dihydro-1H,3H,7H-oxazolo[3',4':4,5][1,4]oxazino[3,2-f]indazol-1-ylidene)amino)-4-methyl-4,5,6,7-tetrahydro-2H-pyrazolo[4,3-c]pyridine-5-carbonyl)-1H-indol-1-yl)-2-methylcyclopropyl)-1,2,4-oxadiazol-5(4H)-one 16-2

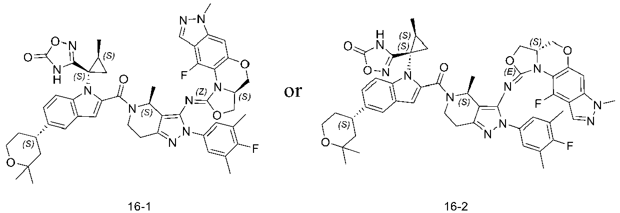

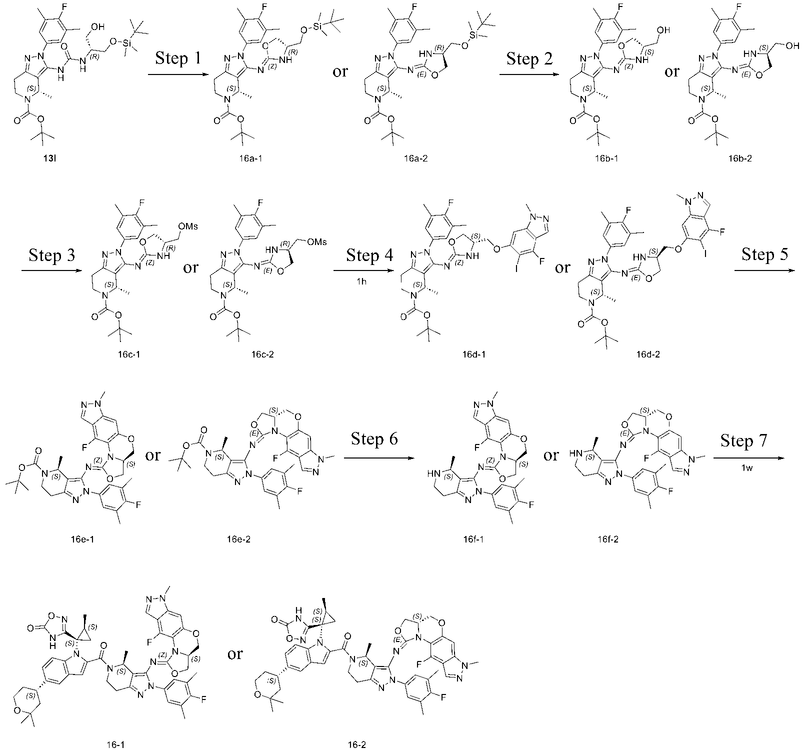

### Step 1

### Tert-butyl (S)-3-(((R,Z)-4-(((tert-butyldimethylsilyl)oxy)methyl)oxazolidin-2-ylidene)amino)-2-(4-fluoro-3,5-dimethylphenyl)-4-methyl-2,4,6,7-tetrahydro-5H-pyrazolo[4,3-c]pyridine-5-carboxylate 16a-1 or

### tert-butyl (S)-3-(((R,E)-4-(((tert-butyldimethylsilyl)oxy)methyl)oxazolidin-2-ylidene)amino)-2-(4-fluoro-3,5-dimethylphenyl)-4-methyl-2,4,6,7-tetrahydro-5H-pyrazolo[4,3-c]pyridine-5-carboxylate 16a-2

Compound **13l** (800 mg, 1.32 mmol) was dissolved in dichloromethane (30 mL). Diethylaminosulfur trifluoride (DAST)(255 mg, 1.58 mmol, Adamas) was dropwise added at -78°C and stirred for 1 hour. Subsequently, the reaction mixture was heated to room temperature and stirred for 1 hour. After quenching with a saturated sodium bicarbonate solution (20 mL) and extracted with dichloromethane (30 mL × 3). The organic phases were combined and washed with a saturated sodium chloride solution. The organic phase was dried over anhydrous sodium sulfate. After filtration, the filtrate was concentrated under reduced pressure. The residue was purified by silica gel column chromatography with eluent system A to obtain the title compound **16a-1** or **16a-2** (637 mg, yield: 82.1%).

MS m/z (ESI): 588.6 [M+1].

### Step 2

### Tert-butyl (S)-2-(4-fluoro-3,5-dimethylphenyl)-3-(((S,Z)-4-(hydroxymethyl)oxazolidin-2-ylidene)amino)-4-methyl-2,4,6,7-tetrahydro-5H-pyrazolo[4,3-c]pyridine-5-carboxylate 16b-1 or

### tert-butyl (S)-2-(4-fluoro-3,5-dimethylphenyl)-3-(((S,E)-4-(hydroxymethyl)oxazolidin-2-ylidene)amino)-4-methyl-2,4,6,7-tetrahydro-5H-pyrazolo[4,3-c]pyridine-5-carboxylate 16b-2

Compound **16a-1** or **16a-2** (230 mg, 0.39 mmol) was dissolved in tetrahydrofuran (5 mL). A tetrabutylammonium fluoride/tetrahydrofuran solution (0.40 mL, 1.0M, Adamas) was added and the mixture was reacted at room temperature for 1 hour. The reaction solution was poured into 20 mL of water and extracted with ethyl acetate (30 mL × 3). The organic phases were combined, washed with a saturated sodium chloride solution, and dried over anhydrous sodium sulfate. After filtration, the filtrate was concentrated under reduced pressure. The residue was purified by silica gel column chromatography with eluent system B to obtain the title compound **16b-1** or **16b-2** (110 mg, yield: 59.4%).

MS m/z (ESI): 474.3 [M+1].

### Step 3

### Tert-butyl (S)-2-(4-fluoro-3,5-dimethylphenyl)-4-methyl-3-(((R,Z)-4-(((methylsulfonyl)oxy)methyl)oxazolidin-2-ylidene)amino)-2,4,6,7-tetrahydro-5H-pyrazolo[4,3-c]pyridine-5-carboxylate 16c-1 or

### tert-butyl (S)-2-(4-fluoro-3,5-dimethylphenyl)-4-methyl-3-(((R,E)-4-(((methylsulfonyl)oxy)methyl)oxazolidin-2-ylidene)amino)-2,4,6,7-tetrahydro-5H-pyrazolo[4,3-c]pyridine-5-carboxylate 16c-2

Compound **16b-1** or **16b-2** (150 mg, 0.32 mmol) and triethylamine (130 mg, 1.28 mmol) were dissolved in dichloromethane (10 mL). Methanesulfonic anhydride(72 mg, 0.41 mmol) was added at 0°C and the mixture was reacted at room temperature for 1 hour. The reaction solution was poured into water (20 mL) and extracted with dichloromethane (20 mL × 2). The organic phases were combined, washed with a saturated sodium chloride solution, and dried over anhydrous sodium sulfate. After filtration, the filtrate was concentrated under reduced pressure to obtain the crude title compound **16c-1** or **16c-2** (174 mg).

MS m/z (ESI): 552.3 [M+1].

### Step 4

### Tert-butyl (S)-2-(4-fluoro-3,5-dimethylphenyl)-3-(((S,Z)-4-(((4-fluoro-5-iodo-1-methyl-1H-indazol-6-yl)oxy)methyl)oxazolidin-2-ylidene)amino)-4-methyl-2,4,6,7-tetrahydro-5H-pyrazolo[4,3-c]pyridine-5-carboxylate 16d-1 or

### tert-butyl (S)-2-(4-fluoro-3,5-dimethylphenyl)-3-(((S,E)-4-(((4-fluoro-5-iodo-1-methyl-1H-indazol-6-yl)oxy)methyl)oxazolidin-2-ylidene)amino)-4-methyl-2,4,6,7-tetrahydro-5H-pyrazolo[4,3-c]pyridine-5-carboxylate 16d-2

Compound **16c-1** or **16c-2** (174 mg, 0.32 mmol) and compound **1h** (92 mg, 0.32 mmol) were dissolved in *N,N*-dimethylformamide (5.0 mL). Potassium carbonate (131 mg, 0.95 mmol) was added and reacted at 60°C for 2 hours. The reaction solution was poured into 20 mL of water and extracted with ethyl acetate (30 mL × 2). The organic phases were combined, washed with a saturated sodium chloride solution, and dried over anhydrous sodium sulfate. After filtration, the filtrate was concentrated under reduced pressure. The residue was purified by silica gel column chromatography with eluent system A to obtain the title compound **16d-1** or **16d-2** (110 mg, yield: 46.6%).

MS m/z (ESI): 748.4 [M+1].

### Step 5

### Tert-butyl (S)-2-(4-fluoro-3,5-dimethylphenyl)-3-(((S,Z)-10-fluoro-7-methyl-3a,4-dihydro-1H,3H,7H-oxazolo[3',4':4,5][1,4]oxazino[3,2-f]indazol-1-ylidene)amino)-4-methyl-2,4,6,7-tetrahydro-5H-pyrazolo[4,3-c]pyridine-5-carboxylate 16e-1, or tert-butyl (S)-2-(4-fluoro-3,5-dimethylphenyl)-3-(((S,E)-10-fluoro-7-methyl-3a,4-dihydro-1H,3H,7H-oxazolo[3',4':4,5][1,4]oxazino[3,2-f]indazol-1-ylidene)amino)-4-methyl-2,4,6,7-tetrahydro-5H-pyrazolo[4,3-c]pyridine-5-carboxylate 16e-2

Compound **16d-1** or **16d-2** (100 mg, 0.13 mmol) and L-proline (31 mg, 0.27 mmol, Accela) were dissolved in *N,N*-dimethylformamide (5.0 mL). Potassium carbonate (56 mg, 0.40 mmol) and cuprous iodide (26 mg, 0.13 mmol, Leyan) were added. After displacement with nitrogen three times, the mixture was heated. The reaction was stirred at 100°C overnight. The reaction solution was poured into 20 mL of water and extracted with ethyl acetate (30 mL × 2). The organic phases were combined, washed with a saturated sodium chloride solution, and dried over anhydrous sodium sulfate. After filtration, the filtrate was concentrated under reduced pressure. The residue was purified by silica gel column chromatography with eluent system A to obtain the title compound **16e-1** or **16e-2** (10 mg, yield: 12.1%).

MS m/z (ESI): 620.3 [M+1].

### Step 6

### (S,Z)-10-fluoro-N-((S)-2-(4-fluoro-3,5-dimethylphenyl)-4-methyl-4,5,6,7-tetrahydro-2H-pyrazolo[4,3-c]pyridin-3-yl)-7-methyl-3a,4-dihydro-1H,3H,7H-oxazolo[3',4':4,5][1,4]oxazino[3,2-f]indazol-1-imine 16f-1 or

### (S,E)-10-fluoro-N-((S)-2-(4-fluoro-3,5-dimethylphenyl)-4-methyl-4,5,6,7-tetrahydro-2H-pyrazolo[4,3-c]pyridin-3-yl)-7-methyl-3a,4-dihydro-1H,3H,7H-oxazolo[3',4':4,5][1,4]oxazino[3,2-f]indazol-1-imine 16f-2

Compound **16e-1 or 16e-2** (20 mg, 0.02 mmol) was dissolved in dichloromethane (3 mL). Zinc bromide (16 mg, 0.07 mmol, Adamas) was added and stirred at room temperature overnight. The reaction solution was concentrated under reduced pressure. The residue was purified by silica gel column chromatography with eluent system A to obtain the crude title compound **16f-1** or **16f-2** (25 mg).

MS m/z (ESI): 520.2 [M+1].

### Step 7

### 3-((1S,2S)-1-(5-((S)-2,2-dimethyltetrahydro-2H-pyran-4-yl)-2-((S)-2-(4-fluoro-3,5-dimethylphenyl)-3-(((S,Z)-10-fluoro-7-methyl-3a,4-dihydro-1H,3H,7H-oxazolo[3',4':4,5][1,4]oxazino[3,2-f]indazol-1-ylidene)amino)-4-methyl-4,5,6,7-tetrahydro-2H-pyrazolo[4,3-c]pyridine-5-carbonyl)-1H-indol-1-yl)-2-methylcyclopropyl)-1,2,4-oxadiazol-5(4H)-one 16-1, or

### 3-((1S,2S)-1-(5-((S)-2,2-dimethyltetrahydro-2H-pyran-4-yl)-2-((S)-2-(4-fluoro-3,5-dimethylphenyl)-3-(((S,E)-10-fluoro-7-methyl-3a,4-dihydro-1H,3H,7H-oxazolo[3',4':4,5][1,4]oxazino[3,2-f]indazol-1-ylidene)amino)-4-methyl-4,5,6,7-tetrahydro-2H-pyrazolo[4,3-c]pyridine-5-carbonyl)-1H-indol-1-yl)-2-methylcyclopropyl)-1,2,4-oxadiazol-5(4H)-one 16-2

Compound **16f-1** or **16f-2** (25 mg, 0.049 mmol) and compound **1w** (20 mg, 0.049 mmol) were dissolved in *N,N*-dimethylformamide (5 mL). *N,N-*diisopropylethylamine (19 mg, 0.15 mmol) was added. After the raw materials were fully dissolved, *O*-(7-azabenzotriazol-1-yl)-*N,N,N',N'*-tetramethyluronium hexafluorophosphate (28 mg, 0.073 mmol) was added and stirred at room temperature for 16 hours. The reaction solution was poured into 10 mL of water and extracted with ethyl acetate (10 mL × 3). The organic phases were combined, washed with a saturated sodium chloride solution, and dried over anhydrous sodium sulfate. After filtration, the filtrate was concentrated under reduced pressure. The residue was purified by preparative high-performance liquid chromatography (instrument model: Gilson-GX-281, chromatographic column: Boston Phlex, Prep 30 × 150 mm; 5 µm; C18, mobile phase: water (10 mM ammonium bicarbonate) and acetonitrile, with gradient ratio: acetonitrile 40%-60%, flow rate: 30 mL/min) to obtain the title compound **16-1** or **16-2** (12 mg, yield: 27.0%).

MS m/z (ESI): 913.7 [M+1].

¹H NMR (500 MHz, CDCl₃): δ 12.00-11.00 (m, 1H), 8.10-7.94 (m, 1H), 7.62-7.48 (m, 2H), 7.25-7.12 (m, 3H), 6.78-6.62 (m, 2H), 5.79-5.11 (m, 2H), 4.98-4.75 (m, 1H), 4.44-4.33 (m, 1H), 4.29-4.18 (m, 1H), 4.16-4.07 (m, 1H), 4.05-3.96 (m, 3H), 3.92-3.71 (m, 3H), 3.60-3.25 (m, 2H), 3.12-2.81 (m, 3H), 2.22 (t, 1H), 2.08-1.97 (m, 1H), 1.96-1.87 (m, 6H), 1.82-1.58 (m, 7H), 1.35-1.30 (m, 5H), 1.23 (d, 2H), 1.08 (d, 1H), 0.88 (t, 1H).

### Example 17

### 3-((1S,2S)-1-(5-((S)-2,2-dimethyltetrahydro-2H-pyran-4-yl)-2-((S)-3-(2-(4-fluoro-1-methyl-1H-indazol-5-yl)-3-oxo-2,3-dihydrohydropyridazin-4-yl)-2-(4-fluoro-3,5-dimethylphenyl)-4-methyl-4,5,6,7-tetrahydro-2H-pyrazolo[4,3-c]pyridine-5-carbonyl)-1H-indol-1-yl)-2-methylcyclopropyl)-1,2,4-oxadiazol-5(4H)-one 17

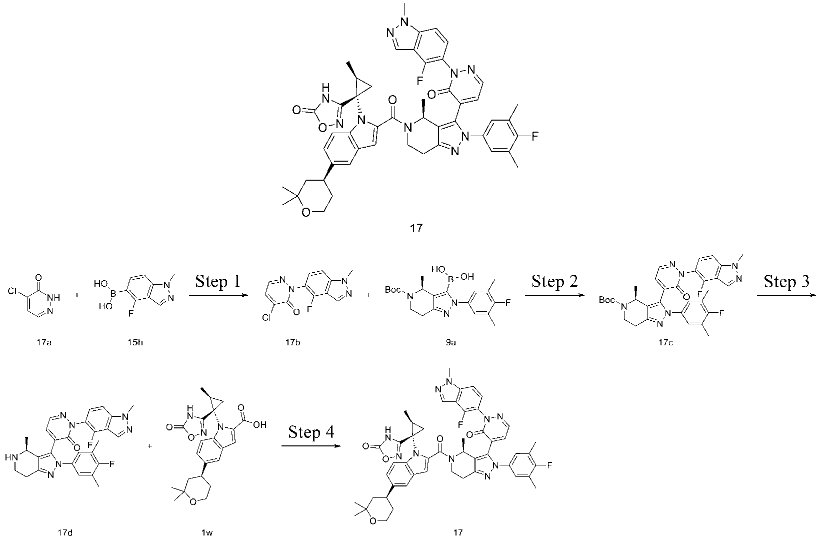

### Step 1

### 4-Chloro-2-(4-fluoro-1-methyl-1H-indazol-5-yl)pyridazin-3(2H)-one 17b

4-Chloropyridazin-3(2*H*)-one **17a** (105 mg, 0.80 mmol, Bidepharm), compound **15h** (163 mg, 0.84 mmol), pyridine (128 mg, 1.61 mmol), and copper acetate (293 mg, 1.61 mmol) were dissolved in 1,2-dichloroethane (5 mL), left open, and stirred at room temperature for 16 hours. The reaction solution was concentrated under reduced pressure. The residue was purified by silica gel column chromatography with eluent system A to obtain the title compound **17b** (25 mg, yield: 11.2%).

MS m/z (ESI): 279.2 [M+1].

### Step 2

### Tert-butyl (S)-3-(2-(4-fluoro-1-methyl-1H-indazol-5-yl)-3-oxo-2,3-dihydropyridazin-4-yl)-2-(4-fluoro-3,5-dimethylphenyl)-4-methyl-2,4,6,7-tetrahydro-5H-pyrazolo[4,3-c]pyridine-5-carboxylate 17c

Compound **17b** (15 mg, 0.054 mmol), compound **9a** (30 mg, 0.074 mmol), anhydrous potassium carbonate (23 mg, 0.17 mmol), [1,1'-bis(diphenylphosphino)ferrocene]palladium dichloride (40 mg, 0.055 mmol, Bidepharm), and tetrakis(triphenylphosphine)palladium (62 mg, 0.054 mmol, Bidepharm) were dissolved in dioxane (5 mL) and water (1 mL). After displacement with nitrogen, the reaction was stirred at 90°C overnight. The reaction solution was cooled to room temperature and concentrated under reduced pressure. The residue was purified by silica gel column chromatography with eluent system A to obtain the title compound **17c** (15 mg, yield: 46.3%).

MS m/z (ESI): 602.4 [M+1].

### Step 3

### (S)-2-(4-fluoro-1-methyl-1H-indazol-5-yl)-4-(2-(4-fluoro-3,5-dimethylphenyl)-4-methyl-4,5,6,7-tetrahydro-2H-pyrazolo[4,3-c]pyridin-3-yl)pyridazin-3(2H)-one 17d

Compound **17c** (15 mg, 0.025 mmol) was dissolved in dichloromethane (1 mL). A 4M solution of hydrogen chloride in dioxane (1 mL) was added. The reaction was stirred at room temperature for 1 hour. The reaction solution was concentrated under reduced pressure to obtain the crude title compound **17d** (13 mg). The product was directly used in the next reaction without purification.

MS m/z (ESI): 502.4 [M+1].

### Step 4

### 3-((1S,2S)-1-(5-((S)-2,2-dimethyltetrahydro-2H-pyran-4-yl)-2-((S)-3-(2-(4-fluoro-1-methyl-1H-indazol-5-yl)-3-oxo-2,3-dihydrohydropyridazin-4-yl)-2-(4-fluoro-3,5-dimethylphenyl)-4-methyl-4,5,6,7-tetrahydro-2H-pyrazolo[4,3-c]pyridine-5-carbonyl)-1H-indol-1-yl)-2-methylcyclopropyl)-1,2,4-oxadiazol-5(4H)-one 17

Compound **17d** (13 mg, 0.026 mmol) and compound **1w** (15 mg, 0.036 mmol) were dissolved in *N,N*-dimethylformamide (4 mL). *N,N*-diisopropylethylamine (18 mg, 0.14 mmol) was added. After the raw materials were fully dissolved, *O*-(7-azabenzotriazol-1-yl)-*N,N,N',N'*-tetramethyluronium hexafluorophosphate (15 mg, 0.040 mmol) was added and stirred at room temperature for 16 hours. The reaction solution was poured into 10 mL of water and extracted with ethyl acetate (10 mL × 3). The organic phases were combined, washed with a saturated sodium chloride solution, and dried over anhydrous sodium sulfate. After filtration, the filtrate was concentrated under reduced pressure. The residue was purified by preparative high-performance liquid chromatography (instrument model: Gilson-GX-281, chromatographic column: Boston Phlex, Prep 30 × 150 mm; 5 µm; C18, mobile phase: water (10 mM ammonium bicarbonate) and acetonitrile, with gradient ratio: acetonitrile 30%-70%, flow rate: 30 mL/min) to obtain the title compound **17** (20 mg, yield: 86.2%).

MS m/z (ESI): 895.5 [M+1].

¹H NMR (500 MHz, CDCl₃): δ 11.50-11.25 (m, 1H), 8.21-7.74 (m, 2H), 7.63-7.37 (m, 3H), 7.34-7.28 (m, 1H), 7.25-7.18 (m, 1H), 7.17-6.84 (m, 3H), 6.75-6.65 (m, 1H), 5.96-5.48 (m, 1H), 5.47-5.11 (m, 1H), 4.95-4.37 (m, 1H), 4.17-4.04 (m, 3H), 3.95-3.77 (m, 2H), 3.63-3.32 (m, 1H), 3.23-2.97 (m, 3H), 2.33-2.23 (m, 6H), 2.22-1.86 (m, 2H), 1.79-1.62 (m, 5H), 1.45 (d, 2H), 1.39-1.31 (m, 5H), 1.19 (d, 2H), 1.01 (d, 1H), 0.88 (t, 1H).

### Example 18

### 3-((1S,2S)-1-(5-((S)-2,2-dimethyltetrahydro-2H-pyran-4-yl)-2-((S)-2-(4-fluoro-3,5-dimethylphenyl)-4-methyl-3-(2-(1-methyl-1H-indazol-5-yl)-3-oxo-2,3-dihydropyridazin-4-yl)-4,5,6,7-tetrahydro-2H-pyrazolo[4,3-c]pyridine-5-carbonyl)-1H-indol-1-yl)-2-methylcyclopropyl)-1,2,4-oxadiazol-5(4H)-one 18

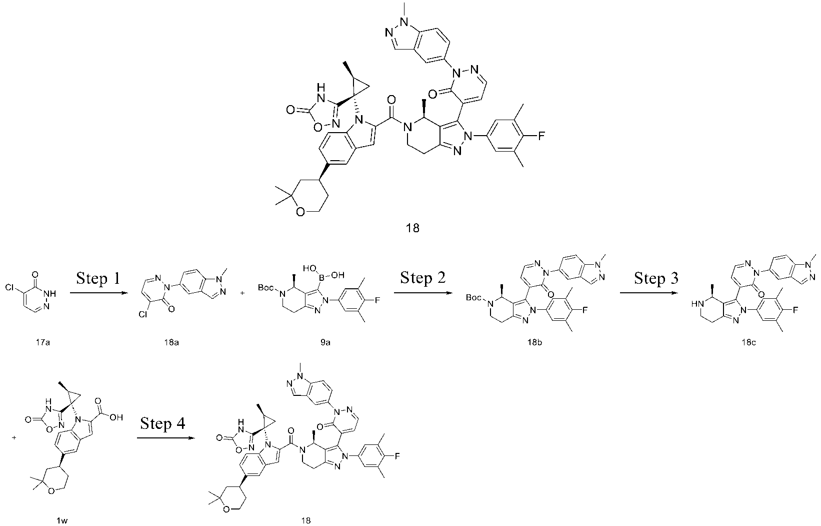

### Step 1

### 4-Chloro-2-(1-methyl-1H-indazol-5-yl)pyridazin-3(2H)-one 18a

Compound **17a** (60 mg, 0.46 mmol, Bidepharm), 1-methylindazole-5-boronic acid (82 mg, 0.47 mmol, Leyan), pyridine (200 mg, 2.53 mmol), and copper acetate (50 mg, 0.28 mmol) were dissolved in 1,2-dichloroethane (3 mL), left open, and stirred at room temperature for 16 hours. The reaction solution was concentrated under reduced pressure. The residue was purified by silica gel column chromatography with eluent system A to obtain the crude title compound **18a** (120 mg).

MS m/z (ESI): 261.1 [M+1].

### Step 2

### Tert-butyl (S)-2-(4-fluoro-3,5-dimethylphenyl)-4-methyl-3-(2-(1-methyl-1H-indazol-5-yl)-3-oxo-2,3-dihydropyridazin-4-yl)-2,4,6,7-tetrahydro-5H-pyrazolo[4,3-c]pyridine-5-carboxylate 18b

Compound **18a** (80 mg, 0.31 mmol), compound **9a** (100 mg, 0.25 mmol), anhydrous potassium carbonate (103 mg, 0.75 mmol), [1,1'-bis(diphenylphosphino)ferrocene]palladium dichloride (36 mg, 0.049 mmol, Bidepharm), and tetrakis(triphenylphosphine)palladium (58 mg, 0.049 mmol, Bidepharm) were dissolved in dioxane (10 mL) and water (2 mL). After displacement with nitrogen, the reaction was stirred at 90°C overnight. The reaction solution was cooled to room temperature and concentrated under reduced pressure. The residue was purified by silica gel column chromatography with eluent system A to obtain the title compound **18b** (15 mg, yield: 10.4%).

MS m/z (ESI): 584.4 [M+1].

### Step 3

### (S)-4-(2-(4-fluoro-3,5-dimethylphenyl)-4-methyl-4,5,6,7-tetrahydro-2H-pyrazolo[4,3-c]pyridin-3-yl)-2-(1-methyl-1H-indazol-5-yl)pyridazin-3(2H)-one 18c

Compound **18b** (15 mg, 0.026 mmol) was dissolved in dichloromethane (1 mL). A 4M solution of hydrogen chloride in dioxane (2 mL) was added. The reaction was stirred at room temperature for 1 hour. The reaction solution was concentrated under reduced pressure to obtain the crude title compound **18c** (13 mg). The product was directly used in the next reaction without purification.

MS m/z (ESI): 484.3 [M+1].

### Step 4

### 3-((1S,2S)-1-(5-((S)-2,2-dimethyltetrahydro-2H-pyran-4-yl)-2-((S)-2-(4-fluoro-3,5-dimethylphenyl)-4-methyl-3-(2-(1-methyl-1H-indazol-5-yl)-3-oxo-2,3-dihydropyridazin-4-yl)-4,5,6,7-tetrahydro-2H-pyrazolo[4,3-c]pyridine-5-carbonyl)-1H-indol-1-yl)-2-methylcyclopropyl)-1,2,4-oxadiazol-5(4H)-one 18

Compound **18c** (13 mg, 0.027 mmol) and compound **1w** (15 mg, 0.036 mmol) were dissolved in *N,N*-dimethylformamide (3 mL). *N,N*-diisopropylethylamine (18 mg, 0.13 mmol) was added. After the raw materials were fully dissolved, O-(7-azabenzotriazol-1-yl)-*N,N,N',N'*-tetramethyluronium hexafluorophosphate (16 mg, 0.041 mmol) was added and stirred at room temperature for 16 hours. The reaction solution was poured into 10 mL of water and extracted with ethyl acetate (10 mL × 3). The organic phases were combined, washed with a saturated sodium chloride solution, and dried over anhydrous sodium sulfate. After filtration, the filtrate was concentrated under reduced pressure. The residue was purified by preparative high-performance liquid chromatography (instrument model: Gilson-GX-281, chromatographic column: Boston Phlex, Prep 30 × 150 mm; 5 µm; C18, mobile phase: water (10 mM ammonium bicarbonate) and acetonitrile, with gradient ratio: acetonitrile 30%-65%, flow rate: 30 mL/min) to obtain the title compound **18** (14 mg, yield: 59.4%).

MS m/z (ESI): 877.8 [M+1].

¹H NMR (500 MHz, CDCl₃): δ 11.36 (s, 1H), 8.14-7.64 (m, 3H), 7.62-7.44 (m, 3H), 7.38-7.28 (m, 1H), 7.25-7.03 (m, 3H), 6.99-6.81 (m, 1H), 6.72-6.63 (m, 1H), 5.98-5.46 (m, 1H), 5.40-5.19 (m, 1H), 4.91-4.37 (m, 1H), 4.19-4.01 (m, 3H), 3.92-3.77 (m, 2H), 3.63-3.36 (m, 1H), 3.23-2.93 (m, 3H), 2.32-2.17 (m, 7H), 2.05-1.86 (m, 2H), 1.83-1.67 (m, 4H), 1.44 (d, 2H), 1.37-1.32 (m, 5H), 1.18 (d, 2H), 0.99 (d, 1H), 0.88 (t, 1H).

### Example 19-1 or 19-2

### 3-((1S,2S)-1-(5-((S)-2,2-dimethyltetrahydro-2H-pyran-4-yl)-2-((S)-3-(((S,Z)-4-(((4-fluoro-1-methyl-1H-indazol-6-yl)oxy)methyl)oxazolidin-2-ylidene)amino)-2-(4-fluoro-3,5-dimethylphenyl)-4-methyl-4,5,6,7-tetrahydro-2H-pyrazolo[4,3-c]pyridine-5-carbonyl)-1H-indol-1-yl)-2-methylcyclopropyl)-1,2,4-oxadiazol-5(4H)-one 19-1 or

### 3-((1S,2S)-1-(5-((S)-2,2-dimethyltetrahydro-2H-pyran-4-yl)-2-((S)-3-(((S,E)-4-(((4-fluoro-1-methyl-1H-indazol-6-yl)oxy)methyl)oxazolidin-2-ylidene)amino)-2-(4-fluoro-3,5-dimethylphenyl)-4-methyl-4,5,6,7-tetrahydro-2H-pyrazolo[4,3-c]pyridine-5-carbonyl)-1H-indol-1-yl)-2-methylcyclopropyl)-1,2,4-oxadiazol-5(4H)-one 19-2

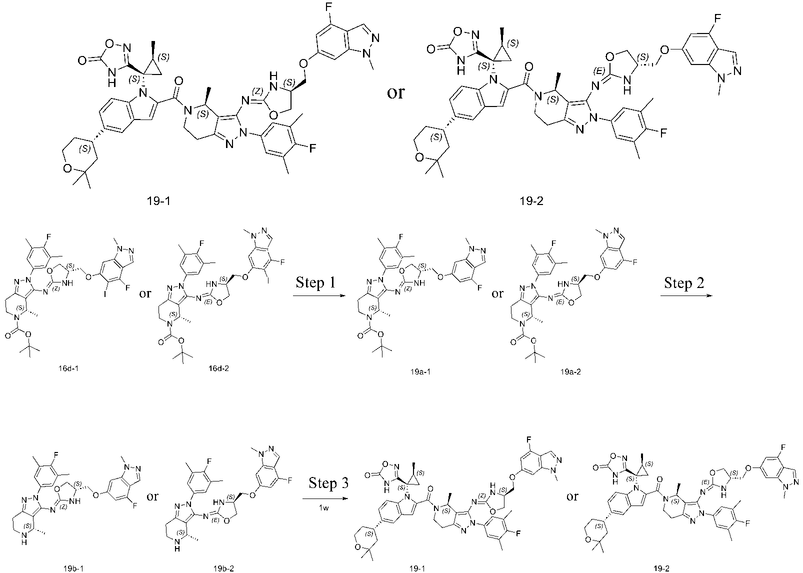

### Step 1

### Tert-butyl (S)-3-(((S,Z)-4-(((4-fluoro-1-methyl-1H-indazol-6-yl)oxy)methyl)oxazolidin-2-ylidene)amino)-2-(4-fluoro-3,5-dimethylphenyl)-4-methyl-2,4,6,7-tetrahydro-5H-pyrazolo[4,3-c]pyridine-5-carboxylate 19a-1 or

### tert-butyl (S)-3-(((S,E)-4-(((4-fluoro-1-methyl-1H-indazol-6-yl)oxy)methyl)oxazolidin-2-ylidene)amino)-2-(4-fluoro-3,5-dimethylphenyl)-4-methyl-2,4,6,7-tetrahydro-5H-pyrazolo[4,3-c]pyridine-5-carboxylate 19a-2

Compound **16d-1** or **16d-2** (100 mg, 0.13 mmol) and *L*-proline (31 mg, 0.27 mmol, Adamas) were dissolved in *N,N*-dimethylformamide (5.0 mL). Potassium carbonate (56 mg, 0.40 mmol) and cuprous iodide (26 mg, 0.13 mmol, Leyan) were added. After displacement with nitrogen three times, the mixture was heated to 120°C and reacted under stirring for 2 hours. The reaction solution was poured into 20 mL of water and extracted with ethyl acetate (30 mL × 2). The organic phases were combined, washed with a saturated sodium chloride solution, and dried over anhydrous sodium sulfate. After filtration, the filtrate was concentrated under reduced pressure. The residue was purified by silica gel column chromatography with eluent system A to obtain the title compound **19a-1** or **19a-2** (80 mg, yield: 96.2%).

MS m/z (ESI): 622.3 [M+1].

### Step 2

### (S,Z)-4-(((4-fluoro-1-methyl-1H-indazol-6-yl)oxy)methyl)-N-((S)-2-(4-fluoro-3,5-dimethylphenyl)-4-methyl-4,5,6,7-tetrahydro-2H-pyrazolo[4,3-c]pyridin-3-yl)oxazolidin-2-imine 19b-1

### or (S,E)-4-(((4-fluoro-1-methyl-1H-indazol-6-yl)oxy)methyl)-N-((S)-2-(4-fluoro-3,5-dimethylphenyl)-4-methyl-4,5,6,7-tetrahydro-2H-pyrazolo[4,3-c]pyridin-3-yl)oxazolidin-2-imine 19b-2

Compound **19a-1** or **19a-2** (160 mg, 0.26 mmol) was dissolved in dichloromethane (5 mL). Zinc bromide (174 mg, 0.78 mmol, Adamas) was added and stirred at room temperature overnight. The reaction solution was concentrated under reduced pressure. The residue was purified by silica gel column chromatography with eluent system A to obtain the crude title compound **19b-1** or **19b-2** (89 mg).

MS m/z (ESI): 522.2 [M+1].

### Step 3

### 3-((1S,2S)-1-(5-((S)-2,2-dimethyltetrahydro-2H-pyran-4-yl)-2-((S)-3-(((S,Z)-4-(((4-fluoro-1-methyl-1H-indazol-6-yl)oxy)methyl)oxazolidin-2-ylidene)amino)-2-(4-fluoro-3,5-dimethylphenyl)-4-methyl-4,5,6,7-tetrahydro-2H-pyrazolo[4,3-c]pyridine-5-carbonyl)-1H-indol-1-yl)-2-methylcyclopropyl)-1,2,4-oxadiazol-5(4H)-one 19-1 or

### 3-((1S,2S)-1-(5-((S)-2,2-dimethyltetrahydro-2H-pyran-4-yl)-2-((S)-3-(((S,E)-4-(((4-fluoro-1-methyl-1H-indazol-6-yl)oxy)methyl)oxazolidin-2-ylidene)amino)-2-(4-fluoro-3,5-dimethylphenyl)-4-methyl-4,5,6,7-tetrahydro-2H-pyrazolo[4,3-c]pyridine-5-carbonyl)-1H-indol-1-yl)-2-methylcyclopropyl)-1,2,4-oxadiazol-5(4H)-one 19-2

Compound **19b-1** or **19b-2** (50 mg, 0.096 mmol) and compound **1w** (40 mg, 0.096 mmol) were dissolved in *N,N*-dimethylformamide (3 mL). *N,N-*diisopropylethylamine (38 mg, 0.29 mmol) was added. After the raw materials were fully dissolved, *O*-(7-azabenzotriazol-1-yl)-*N,N,N',N'*-tetramethyluronium hexafluorophosphate (55 mg, 0.14 mmol) was added and stirred at room temperature for 16 hours. The reaction solution was poured into 10 mL of water and extracted with ethyl acetate (10 mL × 3). The organic phases were combined, washed with a saturated sodium chloride solution, and dried over anhydrous sodium sulfate. After filtration, the filtrate was concentrated under reduced pressure. The residue was purified by preparative high-performance liquid chromatography (instrument model: Gilson-GX-281, chromatographic column: Boston Phlex, Prep 30 × 150 mm; 5 µm; C18, mobile phase: water (10 mM ammonium bicarbonate) and acetonitrile, with gradient ratio: acetonitrile 35%-55%, flow rate: 30 mL/min) to obtain the title compound **19-1** or **19-2** (15 mg, yield: 17.1%).

MS m/z (ESI): 915.6 [M+1].

¹H NMR (500 MHz, CDCl₃): δ 11.56 (s, 1H), 8.10-7.79 (m, 1H), 7.65-7.30 (m, 3H), 7.24-7.10 (m, 2H), 6.71-6.59 (m, 1H), 6.57-6.24 (m, 2H), 5.77-5.51 (m, 1H), 5.43-5.23 (m, 1H), 5.18-4.78 (m, 1H), 4.73-4.48 (m, 1H), 4.46-4.23 (m, 2H), 4.09-3.93 (m, 4H), 3.91-3.44 (m, 4H), 3.09-2.83 (m, 3H), 2.35-2.09 (m, 7H), 2.04-1.84 (m, 2H), 1.64-1.52 (m, 4H), 1.45 (d, 2H), 1.37-1.29 (m, 5H), 1.22-1.10 (m, 2H), 1.07 (d, 1H), 0.88 (t, 1H).

### Example 20

### 3-((1S,2S)-1-(5-((S)-2,2-dimethyltetrahydro-2H-pyran-4-yl)-2-((S)-3-(6-(4-fluoro-1-methyl-1H-indazol-5-yl)pyridin-2-yl)-2-(4-fluoro-3,5-dimethylphenyl)-4-methyl-4,5,6,7-tetrahydro-2H-pyrazolo[4,3-c]pyridine-5-carbonyl)-1H-indol-1-yl)-2-methylcyclopropyl)-1,2,4-oxadiazol-5(4H)-one 20

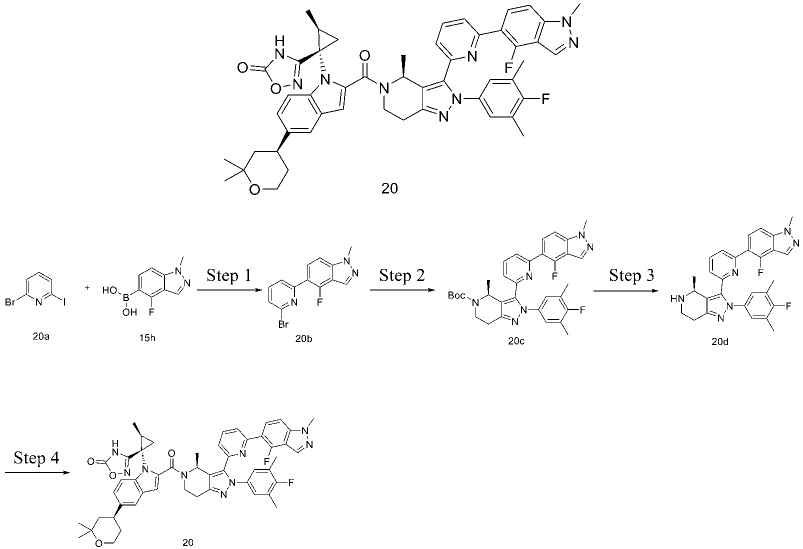

### Step 1

### 5-(6-Bromopyridin-2-yl)-4-fluoro-1-methyl-1H-indazole 20b

Compound **15h** (279 mg, 1.06 mmol), 2-bromo-6-iodopyridine **20a** (300 mg, 1.05 mmol, Adamas), anhydrous sodium carbonate (225 mg, 2.12 mmol), and [1,1'-bis(diphenylphosphino)ferrocene]palladium dichloride (78 mg, 0.106 mmol, Accela) were dissolved in dioxane (5 mL) and water (0.5 mL). After displacement with nitrogen, the reaction was stirred at 90°C for 3 hours. The reaction solution was cooled to room temperature and concentrated under reduced pressure. The residue was purified by silica gel column chromatography with eluent system A to obtain the title compound **20b** (300 mg, yield: 92.7%).

MS m/z (ESI): 306.1 [M+1].

### Step 2

### Tert-butyl (S)-3-(6-(4-fluoro-1-methyl-1H-indazol-5-yl)pyridin-2-yl)-2-(4-fluoro-3,5-dimethylphenyl)-4-methyl-2,4,6,7-tetrahydro-5H-pyrazolo[4,3-c]pyridine-5-carboxylate 20c

Compound **20b** (150 mg, 0.49 mmol), compound **9a** (195 mg, 0.48 mmol), anhydrous potassium carbonate (201 mg, 1.45 mmol), and [1,1'-bis(diphenylphosphino)ferrocene]palladium dichloride (36 mg, 0.048 mmol, Accela) were dissolved in dioxane (5 mL) and water (1 mL). After displacement with nitrogen, the mixture was reacted under stirring at 100°C for 3 hours. The reaction solution was cooled to room temperature and concentrated under reduced pressure. The residue was purified by silica gel column chromatography with eluent system A to obtain the title compound **20c** (100 mg, yield: 34.9%).

MS m/z (ESI): 585.5 [M+1].

### Step 3

### (5)-3-(6-(4-fluoro-1-methyl-1H-indazol-5-yl)pyridin-2-yl)-2-(4-fluoro-3,5-dimethylphenyl)-4-methyl-4,5,6,7-tetrahydro-2H-pyrazolo[4,3-c]pyridine 20d

Compound **20c** (30 mg, 0.051 mmol) was dissolved in a 4 M solution of hydrogen chloride in dioxane (2 mL). The reaction was stirred at room temperature for 16 hours. The reaction solution was concentrated under reduced pressure to obtain the crude title compound **20d** (25 mg). The product was directly used in the next reaction without purification.

MS m/z (ESI): 485.5 [M+1].

### Step 4

### 3-((1S,2S)-1-(5-((S)-2,2-dimethyltetrahydro-2H-pyran-4-yl)-2-((S)-3-(6-(4-fluoro-1-methyl-1H-indazol-5-yl)pyridin-2-yl)-2-(4-fluoro-3,5-dimethylphenyl)-4-methyl-4,5,6,7-tetrahydro-2H-pyrazolo[4,3-c]pyridine-5-carbonyl)-1H-indol-1-yl)-2-methylcyclopropyl)-1,2,4-oxadiazol-5(4H)-one 20

Compound **20d** (25 mg, 0.052 mmol) and compound **1w** (21 mg, 0.051 mmol) were dissolved in *N,N*-dimethylformamide (3 mL). *N,N*-diisopropylethylamine (20 mg, 0.155 mmol) was added. After the raw materials were fully dissolved, *O*-(7-azabenzotriazol-1-yl)-*N,N,N',N'*-tetramethyluronium hexafluorophosphate (29 mg, 0.076 mmol) was added and stirred at room temperature for 16 hours. The reaction solution was filtered. The filtrate was purified by preparative high-performance liquid chromatography (instrument model: Gilson-GX-281, chromatographic column: Boston Phlex, Prep C18 5 µm 30 × 150 mm; mobile phase: water (10 mM ammonium bicarbonate) and acetonitrile, with gradient ratio: acetonitrile 50%-85%, flow rate: 30 mL/min) to obtain the title compound **20** (20 mg, yield: 44.1%).

MS m/z (ESI): 878.5 [M+1].

¹H NMR (500 MHz, CDCl₃): δ 11.92-11.30 (m, 1H), 8.18-8.70 (m, 4H), 7.67-7.58 (m, 1H), 7.55-7.45 (m, 1H), 7.37-7.19 (m, 2H), 7.09-7.04 (m, 1H), 7.04-6.94 (m, 1H), 7.82-6.72 (m, 1H), 6.56-6.47 (m,1H), 6.14-5.77 (m, 1H), 4.95-4.44 (m, 2H), 4.20-3.98 (m, 3H), 3.93-3.78 (m, 2H), 3.73-2.99 (m, 5H), 2.32-2.16 (m, 6H), 1.95-1.73 (m, 4H), 1.46-1.24 (m, 10H), 1.22-1.06 (m, 3H).

### Example 21-1 or 21-2

### 3-((1S,2S)-1-((R)-(6-((S)-3-(3-(4-fluoro-1-methyl-1H-indazol-5-yl)-2-oxo-2,3-dihydro-1H-imidazol-1-yl)-2-(4-fluoro-3,5-dimethylphenyl)-4-methyl-4,5,6,7-tetrahydro-2H-pyrazolo[4,3-c]pyridine-5-carbonyl)-2',2'-dimethyl-2',3',5',6'-tetrahydro-2H,7H-spiro[furo[3,2-f]indole-3,4'-pyran]-7-yl)-2-methylcyclopropyl)-1,2,4-oxadiazol-5(4H)-one 21-1 or

### 3-((1S,2S)-1-((S)-(6-((S)-3-(3-(4-fluoro-1-methyl-1H-indazol-5-yl)-2-oxo-2,3-dihydro-1H-imidazol-1-yl)-2-(4-fluoro-3,5-dimethylphenyl)-4-methyl-4,5,6,7-tetrahydro-2H-pyrazolo[4,3-c]pyridine-5-carbonyl)-2',2'-dimethyl-2',3',5',6'-tetrahydro-2H,7H-spiro[furo[3,2-f]indole-3,4'-pyran]-7-yl)-2-methylcyclopropyl)-1,2,4-oxadiazol-5(4H)-one 21-2

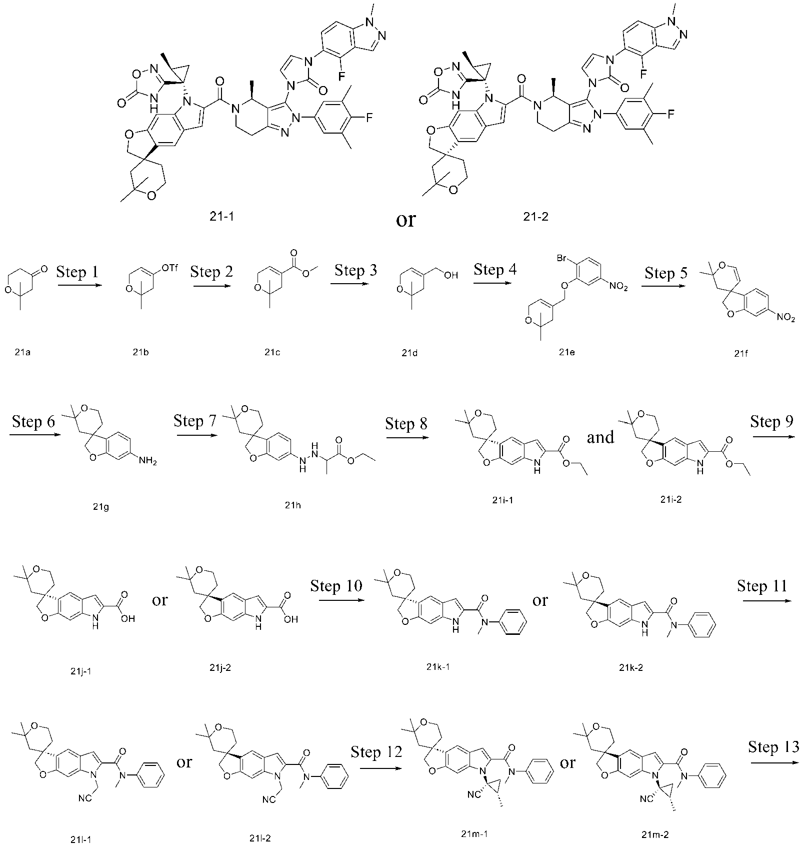

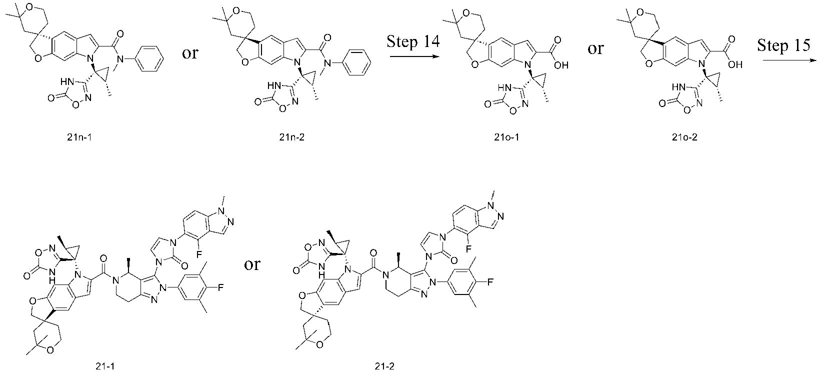

### Step 1

### 2,2-Dimethyl-3,6-dihydro-2H-pyran-4-yl trifluoromethanesulfonate 21b

2,2-Dimethyltetrahydro-4*H*-pyran-4-one **21a** (10.00 g, 78.02 mmol, Bidepharm) was dissolved in tetrahydrofuran (100 mL). A 2 M solution of lithium diisopropylamide in tetrahydrofuran (46.82 mL, 93.63 mmol, Adamas) was added dropwise added at -78°C. The mixture was reacted under stirring at -78°C for 1 hour. A solution of 1,1,1-trifluoro-*N*-phenyl-*N-*(trifluoromethylsulfonyl)methanesulfonamide (30.66 g, 85.83 mmol, Bidepharm) in tetrahydrofuran (250 mL) was then added. The mixture was slowly heated to room temperature and reacted for 16 hours. A saturated aqueous ammonium chloride solution (50 mL) was added under ice-water bath condition. The mixture was extracted with ethyl acetate (150 mL×3). The organic phases were combined and dried over anhydrous sodium sulfate. After filtration, the filtrate was concentrated under reduced pressure. The residue was purified by silica gel column chromatography with eluent system C to obtain the title compound **21b** (10 g, yield: 49.3%).

### Step 2

### Methyl 2,2-dimethyl-3,6-dihydro-2H-pyran-4-carboxylate 21c

**21b** (6.00 g, 23.06 mmol) was dissolved in methanol (15 mL) and *N,N-*dimethylformamide (20 mL). Palladium acetate (414 mg, 3.18 mmol), triphenylphosphine (968 mg, 3.69 mmol), and triethylamine (4.67 g, 46.11 mmol) were added and reacted in a carbon monoxide atmosphere at room temperature for 16 hours. The reaction mixture was concentrated under reduced pressure. The residue was purified by silica gel column chromatography with eluent system C to obtain the title compound **21c** (3 g, yield: 76.4%).

### Step 3

### (2,2-Dimethyl-3,6-dihydro-2H-pyran-4-yl)methanol 21d

Compound **21c** (1.00 g, 5.88 mmol) was dissolved in tetrahydrofuran (20 mL). A 1 M solution of diisobutylaluminum hydride in tetrahydrofuran (12.92 mL, 12.92 mmol, Adamas) was dropwise added at -78°C and stirred for 1 hour. The mixture was then heated to room temperature and reacted for 16 hours. Under ice-water bath condition, a saturated aqueous ammonium chloride solution (10 mL) was added and stirred for three hours. After filtration, the filtrate was concentrated under reduced pressure. The residue was purified by silica gel column chromatography with eluent system C to obtain the title compound **21d** (650 mg, yield: 77.8%).

### Step 4

### 4-((2-Bromo-5-nitrophenoxy)methyl)-2,2-dimethyl-3,6-dihydro-2H-pyran 21e

Compound **21d** (717 mg, 5.04 mmol), 2-bromo-5-nitrophenol (1.0 g, 4.59 mmol, Bidepharm), and triphenylphosphine (2.41 g, 9.17 mmol) were dissolved in tetrahydrofuran (10 mL). Diisopropyl azodicarboxylate (1.86 g, 9.17 mmol) was added and stirred at room temperature for 17 hours. The reaction solution was concentrated under reduced pressure. The residue was purified by silica gel column chromatography with eluent system C to obtain the title compound **21e** (1.3 g, yield: 82.8%).

### Step 5

### 2',2'-Dimethyl-6-nitro-2',3'-dihydro-2H-spiro[benzofuran-3,4'-pyran] 21f

Compound **21e** (1.4 g, 4.09 mmol), palladium acetate (276 mg, 1.23 mmol), tetra-n-butylammonium bromide (1.17 g, 4.09 mmol), and triethylamine (828 mg, 8.18 mmol) were dissolved in *N*,*N*-dimethylacetamide (20 mL), heated to 90°C, and reacted under stirring for 16 hours. The reaction solution was cooled to room temperature. After filtration, the filtrate was concentrated under reduced pressure. The residue was purified by silica gel column chromatography with eluent system C to obtain the title compound **21f** (600 mg, yield: 56.1%).

### Step 6

### 2',2'-Dimethyl-2',3',5',6'-tetrahydro-2H-spiro[benzofuran-3,4'-pyran]-6-amine 21g

Compound **21f** (480 mg, 1.84 mmol) was dissolved in tetrahydrofuran (5 mL) and ethanol (10 mL). Palladium on carbon with a water content of 10% (112 mg, 0.92 mmol) was added and stirred in a hydrogen atmosphere at room temperature for 6 days. The reaction solution was filtered. The filtrate was concentrated under reduced pressure. The residue was purified by silica gel column chromatography with eluent system C to obtain the title compound **21g** (400 mg, yield: 93.3%).

MS m/z (ESI): 234.2 [M+1].

### Step 7

### Ethyl ((2',2'-dimethyl-2',3',5',6'-tetrahydro-2H-spiro[benzofuran-3,4'-pyran]-6-yl)amino)alaninate 21h

Compound **21g** (450 mg, 1.93 mmol) was dissolved in acetonitrile (6 mL). A 6 M solution of hydrogen chloride in water (1.61 mL) was dropwise added under ice bath condition. After the dropwise addition was complete, the mixture was stirred for 10 minutes. Sodium nitrite (266 mg, 3.86 mmol) was dissolved in water (3.5 mL) and dropwise added to the reaction. The reaction mixture was reacted under stirring at 0°C for 1 hour. Ethyl 2-methylacetoacetate (306 mg, 2.12 mmol) was dissolved in ethanol (6 mL) and cooled to 0°C under ice bath condition. Sodium acetate (1.58 g, 19.28 mmol) was added and stirred for 0.5 hour. The two reaction solutions were combined and stirred at room temperature for 2 hours. The mixture was neutralized to pH = 7 with sodium carbonate and extracted with dichloromethane (50 mL × 3). The organic phase was dried over anhydrous sodium sulfate. After filtration, the filtrate was concentrated under reduced pressure. The residue was purified by silica gel column chromatography with eluent system C to obtain an intermediate and dissolved in ethanol (6 mL). A 4 M solution of hydrogen chloride in 1,4-dioxane (1 mL) was dropwise added and stirred at room temperature 0.5 hours. The reaction solution was concentrated under reduced pressure to obtain the crude title compound **21h** (450 mg). The product was directly used in the next reaction without purification.

MS m/z (ESI): 347.2 [M+1].

### Step 8

### Ethyl (R)-2',2'-dimethyl-2',3',5',6'-tetrahydro-2H,7H-spiro[furo[3,2-f]indole-3,4'-pyran]-6-carboxylate 21i-1 and

### ethyl (S)-2',2'-dimethyl-2',3',5',6'-tetrahydro-2H,7H-spiro[furo[3,2-f]indole-3,4'-pyran]-6-carboxylate 21i-2

Compound **21h** (450 mg, 1.30 mmol) was dissolved in dichloromethane (10 mL). Eaton's reagent (618 mg, 2.60 mmol) was added and reacted under stirring at 50°C for 3 hours. The reaction solution was cooled to room temperature and quenched with water (20 mL). The reaction mixture was neutralized to neutrality with a sodium bicarbonate solution and extracted with dichloromethane (50 mL × 3). The organic phase was dried over anhydrous sodium sulfate. After filtration, the filtrate was concentrated under reduced pressure. The residue was purified by silica gel column chromatography with eluent system C to obtain a compound, which was separated by a chiral preparative column (instrument model: Gilson-281 chromatographic column: Columntek O ptichiral A1(AD), 10 µm, 20 mm × 250 mm; mobile phase: A - n-hexane; B - ethanol, elution in a ratio of (70% ethanol and 30% n-hexane), flow rate: 30 mL/min) to obtain the title compound **21i-1** (100 mg, yield 23.4%) and **21i-2** (100 mg, yield 23.4%).

Single-configuration compound (shorter retention time, retention time: 2.980 minutes, 100 mg, yield: 23.4%);
MS m/z (ESI): 328.2 [M-1].

Chiral HPLC analysis: retention time: 2.980 minutes, purity: 100% ( chromatographic column: CHIRALPAK IF 150 × 4.6 mm, 5 µm; mobile phase: ethanol, acetonitrile, with gradient ratio: ethanol: acetonitrile = 80%:20%).

Single-configuration compound (longer retention time, retention time: 8.537 minutes, 100 mg, yield: 23.4%).

MS m/z (ESI): 328.2 [M-1].

Chiral HPLC analysis: retention time: 8.537 minutes, purity: 100% ( chromatographic column: CHIRALPAK IF 150 × 4.6 mm, 5 µm; mobile phase: ethanol, acetonitrile, with gradient ratio: ethanol: acetonitrile = 80%:20%).

### Step 9

### (R)-2',2'-dimethyl-2',3',5',6'-tetrahydro-2H,7H-spiro[furo[3,2-f]indole-3,4'-pyran]-6-carboxylic acid 21j-1 or (S)-2',2'-dimethyl-2',3',5',6'-tetrahydro-2H,7H-spiro[furo[3,2-f]indole-3,4'-pyran]-6-carboxylic acid 21j-2

The compound with shorter retention time (109 mg, 0.33 mmol) among compounds **21i-1** and **21i-2** was dissolved in methanol (2 mL) and water (2 mL). Sodium hydroxide (66 mg, 1.65 mmol) was added and reacted under stirring at 65°C for 3 hours. The reaction solution was cooled to room temperature, quenched with water, and adjusted to pH = 3 with a dilute hydrochloric acid solution. A solid was precipitated. After filtration, the solid was dried to obtain the crude title compound **21j-1 or 21j-2** (100 mg). The product was directly used in the next reaction without purification.

MS m/z (ESI): 302.3 [M+1].

### Step 10

### (R)-N,2',2'-trimethyl-N-phenyl-2',3',5',6'-tetrahydro-2H,7H-spiro[furo[3,2-f]indole-3,4'-pyran]-6-carboxamide 21k-1 or

### (S)-N,2',2'-trimethyl-N-phenyl-2',3',5',6'-tetrahydro-2H,7H-spiro[furo[3,2-f]indole-3,4'-pyran]-6-carboxamide 21k-2

Compound **21j-1** or **21j-2** (100 mg, 0.33 mmol, prepared in one step from the compound with a shorter retention time, among compounds **21i-1** and **21i-2**) was dissolved in *N,N-*dimethylacetamide (3 mL). Sulfoxide chloride (158 mg, 0.33 mmol) was added under ice bath condition and stirred at room temperature for 5 hours. Triethylamine (336 mg, 3.32 mmol) and *N*-methylaniline (35 mg, 0.33 mmol) were added and reacted under stirring at room temperature for 16 hours. The reaction mixture was quenched with water (10 mL) and extracted with ethyl acetate (30 mL × 3). The organic phase was dried over anhydrous sodium sulfate. After filtration, the filtrate was concentrated under reduced pressure. The residue was purified by silica gel column chromatography with eluent system C to obtain the title compound **21k-1 or 21k-2** (100 mg, yield: 77.2%).

MS m/z (ESI): 391.5 [M+1].

### Step 11

### (R)-7-(cyanomethyl)-N,2',2'-trimethyl-N-phenyl-2',3',5',6'-tetrahydro-2H,7H-spiro[furo[3,2-f]indole-3,4'-pyran]-6-carboxamide 21l-1 or

### (S)-7-(cyanomethyl)-N,2',2'-trimethyl-N-phenyl-2',3',5',6'-tetrahydro-2H,7H-spiro[furo[3,2-f]indole-3,4'-pyran]-6-carboxamide 21l-2

Compound **21k-1** or **21k-2** (120 mg, 0.31 mmol, prepared in two steps from the compound with a shorter retention time among compounds **21i-1** and **21i-2**) was dissolved in *N*,*N*-dimethylformamide (3 mL). Sodium hydride (123 mg, 3.08 mol, 60% wt) was added under ice bath condition and stirred at room temperature for 5 hours. Chloroacetonitrile (232 mg, 3.07 mmol) was added and reacted under stirring at room temperature for 3 hours. The reaction mixture was quenched with water (10 mL) and extracted with ethyl acetate (30 mL × 3). The organic phase was dried over anhydrous sodium sulfate. After filtration, the filtrate was concentrated under reduced pressure. The residue was purified by silica gel column chromatography with eluent system C to obtain the title compound **21l-1** or **21l-2** (60 mg, yield: 45.5%).

MS m/z (ESI): 430.5 [M+1].

### Step 12

### (R)-7-((1S,2S)-1-cyano-2-methylcyclopropyl)-N,2',2'-trimethyl-N-phenyl-2',3',5',6'-tetrahydro-2H,7H-spiro[furo[3,2-f]indole-3,4'-pyran]-6-carboxamide 21m-1 or

### (S)-7-((1S,2S)-1-cyano-2-methylcyclopropyl)-N,2',2'-trimethyl-N-phenyl-2',3',5',6'-tetrahydro-2H,7H-spiro[furo[3,2-f]indole-3,4'-pyran]-6-carboxamide 21m-2

Compound **211-1** or **211-2** (60 mg, 0.14 mmol, prepared in three steps from the compound with a shorter retention time, among compounds **21i-1** and **21i-2**) and (*R*)-4-methyl-1,3,2-dioxathiolane 2,2-dioxide (68 mg, 0.49 mmol) were dissolved in *N,N*-dimethylpropyleneurea (716 mg, 5.59 mmol). Potassium bis(trimethylsilyl)amide (223 mg, 1.12 mmol, a 1M tetrahydrofuran solution) was added under ice bath condition and reacted under stirring under ice bath condition for 3 hours. The reaction mixture was quenched with water (10 mL) and extracted with ethyl acetate (20 mL × 3). The organic phase was dried over anhydrous sodium sulfate. After filtration, the filtrate was concentrated under reduced pressure. The residue was purified by silica gel column chromatography with eluent system C to obtain the title compound **21m-1** or **21m-2** (15 mg, yield: 22.9%).

MS m/z (ESI): 470.5 [M+1].

### Step 13

### (R)-N,2',2'-trimethyl-7-((1S,2S)-2-methyl-1-(5-oxo-4,5-dihydro-1,2,4-oxadiazol-3-yl)cyclopropyl)-N-phenyl-2',3',5',6'-tetrahydro-2H,7H-spiro[furo[3,2-f]indole-3,4'-pyran]-6-carboxamide 21n-1 or (S)-N,2',2'-trimethyl-7-((1S,2S)-2-methyl-1-(5-oxo-4,5-dihydro-1,2,4-oxadiazol-3-yl)cyclopropyl)-N-phenyl-2',3',5',6'-tetrahydro-2H,7H-spiro[furo[3,2-f]indole-3,4'-pyran]-6-carboxamide 21n-2

Compound **21m-1** or **21m-2** (15 mg, 0.032 mmol, prepared in four steps from the compound with a shorter retention time, among compounds **21i-1** and **21i-2**) and hydroxylamine hydrochloride (22 mg, 0.32 mmol) were dissolved in dimethyl sulfoxide (1 mL). Sodium bicarbonate (27 mg, 0.32 mmol) was added and reacted under stirring at 60°C for 3.5 hours. The reaction mixture was cooled to room temperature, quenched with water (5 mL), and extracted with ethyl acetate (10 mL × 3). The organic phase was dried over anhydrous sodium sulfate. After filtration, the filtrate was concentrated under reduced pressure. The residue was dissolved in dimethyl sulfoxide (1 mL). N,N'-carbonyldiimidazole(16 mg, 0.096 mmol) and 1,8-diazabicyclo[5,4,0]undec-7-ene (32 mg, 0.13 mmol) were added and reacted under stirring at room temperature for 3 hours. The reaction mixture was quenched with water (5 mL) and extracted with ethyl acetate (10 mL × 3). The organic phase was dried over anhydrous sodium sulfate. After filtration, the filtrate was concentrated under reduced pressure. The residue was purified by silica gel column chromatography with eluent system C to obtain the title compound **21n-1** or **21n-2** (9 mg, yield: 53.3%).

MS m/z (ESI): 529.4 [M+1].

### Step 14

### (R)-2',2'-dimethyl-7-((1S,2S)-2-methyl-1-(5-oxo-4,5-dihydro-1,2,4-oxadiazol-3-yl)cyclopropyl)-2',3',5',6'-tetrahydro-2H,7H-spiro[furo[3,2-f]indole-3,4'-pyran]-6-carboxylic acid 21o-1 or

### (S)-2',2'-dimethyl-7-((1S,2S)-2-methyl-1-(5-oxo-4,5-dihydro-1,2,4-oxadiazol-3-yl)cyclopropyl)-2',3',5',6'-tetrahydro-2H,7H-spiro[furo[3,2-f]indole-3,4'-pyran]-6-carboxylic acid 21o-2

Compound **21n-1** or **21n-2** (9 mg, 0.017 mmol, prepared in five steps from the compound with a shorter retention time, among compounds **21i-1** and **21i-2)** was dissolved in tetrahydrofuran (1 mL). Potassium tert-butoxide (67 mg, 0.60 mmol) and water (2 mg, 0.11 mmol) were added and reacted under stirring for 1 hour. The reaction mixture was adjusted to pH = 3 with 1M dilute hydrochloric acid and extracted with ethyl acetate (30 mL × 3). The organic phase was dried over anhydrous sodium sulfate. After filtration, the filtrate was concentrated under reduced pressure. The residue was purified by silica gel column chromatography with eluent system A to obtain the title compound **21o-1** or **21o-2** (7 mg, yield: 93.6%).

MS m/z (ESI): 440.3 [M+1].

### Step 15

### 3-((1S,2S)-1-((R)-(6-((S)-3-(3-(4-fluoro-1-methyl-1H-indazol-5-yl)-2-oxo-2,3-dihydro-1H-imidazol-1-yl)-2-(4-fluoro-3,5-dimethylphenyl)-4-methyl-4,5,6,7-tetrahydro-2H-pyrazolo[4,3-c]pyridine-5-carbonyl)-2',2'-dimethyl-2',3',5',6'-tetrahydro-2H,7H-spiro[furo[3,2-f]indole-3,4'-pyran]-7-yl)-2-methylcyclopropyl)-1,2,4-oxadiazol-5(4H)-one 21-1 or

### 3-((1S,2S)-1-((S)-(6-((S)-3-(3-(4-fluoro-1-methyl-1H-indazol-5-yl)-2-oxo-2,3-dihydro-1H-imidazol-1-yl)-2-(4-fluoro-3,5-dimethylphenyl)-4-methyl-4,5,6,7-tetrahydro-2H-pyrazolo[4,3-c]pyridine-5-carbonyl)-2',2'-dimethyl-2',3',5',6'-tetrahydro-2H,7H-spiro[furo[3,2-f]indole-3,4'-pyran]-7-yl)-2-methylcyclopropyl)-1,2,4-oxadiazol-5(4H)-one 21-2

Compound **21o-1 or 21o-2** (7 mg, 0.016 mmol, prepared in six steps from the compound with a shorter retention time, among compounds **21i-1** and **21i-2)** and (*S*)-1-(4-fluoro-1-methyl-1*H*-indazol-5-yl)-3-(2-(4-fluoro-3,5-dimethylphenyl)-4-methyl-4,5,6,7-tetrahydro-2*H*-pyrazolo[4,3-c]pyridin-3-yl)-1,3-dihydro-2*H-*imidazol-2-one hydrochloride (8 mg, 0.016 mmol, prepared by a method disclosed for compound 00412 in an example on page 139 of the description of Patent Application WO 2022017338 A1) were dissolved in *N,N*-dimethylformamide (1 mL). 2-(7-azabenzotriazole)-*N,N,N',N'*-tetramethyluronium hexafluorophosphate (18 mg, 0.047 mmol) and *N,N*-diisopropylethylamine (10 mg, 0.077 mmol) were added and the mixture was reacted at room temperature for 16 hours. The reaction solution was concentrated under reduced pressure. The residue was purified by preparative high-performance liquid chromatography (instrument model: Waters-515-2489-2767-SQ2, chromatographic column: Welch Xtimate, Prep 30 × 250 mm; 5 µm; C18, mobile phase: water (10 mM ammonium bicarbonate) and acetonitrile, with gradient ratio: acetonitrile 60%-85%, flow rate: 30 mL/min) to obtain the title compound **21-1 or 21-2** (3 mg, yield: 20.7%, prepared in seven steps from the compound with a shorter retention time among compounds **21i-1** and **21i-2).**

MS m/z (ESI): 911.4 [M+1].

¹H NMR (500 MHz, CDCl₃): δ 8.13-8.08 (m, 1H), 7.52-7.48 (m, 1H), 7.35-7.29 (m, 2H), 7.18-7.07 (m, 2H), 7.04-6.99 (m, 1H), 6.68 (s, 1H), 6.63-6.49 (m, 1H), 6.32-6.12 (m, 1H), 5.77 (q, 1H), 4.74-4.76 (m, 1H), 4.55-4.50 (m, 1H), 4.39-4.36 (m, 1H), 4.13 (s, 3H), 4.09 (s, 1H), 3.86-3.79 (m, 2H), 3.64-3.58 (m, 1H), 3.17-3.11 (m, 1H), 3.05-3.00 (m, 1H), 2.29 (s, 6H), 2.14-2.03 (m, 2H), 1.90-1.86 (m, 1H), 1.73-1.66 (m, 3H), 1.51-1.46 (m, 1H), 1.35-1.17 (m, 11H).

### Example 21-2 or 21-1

### 3-((1S,2S)-1-((S)-(6-((S)-3-(3-(4-fluoro-1-methyl-1H-indazol-5-yl)-2-oxo-2,3-dihydro-1H-imidazol-1-yl)-2-(4-fluoro-3,5-dimethylphenyl)-4-methyl-4,5,6,7-tetrahydro-2H-pyrazolo[4,3-c]pyridine-5-carbonyl)-2',2'-dimethyl-2',3',5',6'-tetrahydro-2H,7H-spiro[furo[3,2-f]indole-3,4'-pyran]-7-yl)-2-methylcyclopropyl)-1,2,4-oxadiazol-5(4H)-one 21-2 or

### 3-((1S,2S)-1-((R)-(6-((S)-3-(3-(4-fluoro-1-methyl-1H-indazol-5-yl)-2-oxo-2,3-dihydro-1H-imidazol-1-yl)-2-(4-fluoro-3,5-dimethylphenyl)-4-methyl-4,5,6,7-tetrahydro-2H-pyrazolo[4,3-c]pyridine-5-carbonyl)-2',2'-dimethyl-2',3',5',6'-tetrahydro-2H,7H-spiro[furo[3,2-f]indole-3,4'-pyran]-7-yl)-2-methylcyclopropyl)-1,2,4-oxadiazol-5(4H)-one 21-1

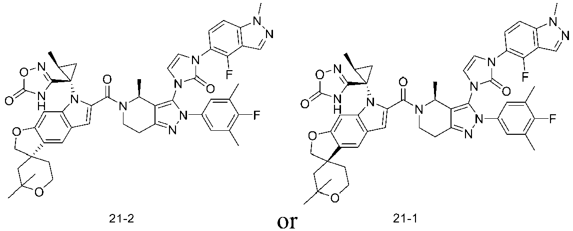

By means of the synthesis route in the above Examples 21-1 and 21-2, the raw material in step 9 was replaced with the compound with a longer retention time among compounds **21i-2** and **21i-1** to prepare the title compound **21-2** or **21-1** (15 mg, the yield of the last reaction: 43.6%, prepared in seven steps from the compound with a longer retention time among compounds **21i-2** and **21i-1).**

MS m/z (ESI): 911.6 [M+1].

¹H NMR (500 MHz, CDCl₃): δ 8.14-8.09 (m, 1H), 7.52-7.48 (m, 1H), 7.36-7.29 (m, 2H), 7.18-7.07 (m, 2H), 7.04-6.99 (m, 1H), 6.67 (s, 1H), 6.62-6.49 (m, 1H), 6.32-6.11 (m, 1H), 5.78 (q, 1H), 4.74-4.76 (m, 1H), 4.55-4.50 (m, 1H), 4.39-4.36 (m, 1H), 4.13 (s, 3H), 4.09 (s, 1H), 3.86-3.79 (m, 2H), 3.64-3.58 (m, 1H), 3.17-3.11 (m, 1H), 3.05-3.00 (m, 1H), 2.29 (s, 6H), 2.13-2.03 (m, 2H), 1.91-1.86 (m, 1H), 1.73-1.66 (m, 3H), 1.50-1.46 (m, 1H), 1.37-1.15 (m, 11H).

### Example 22

### 3-((1S,2S)-1-(5-((S)-2,2-dimethyltetrahydro-2H-pyran-4-yl)-2-((S)-2-(4-fluoro-3,5-dimethylphenyl)-4-methyl-3-(2-methyl-1-(1-methyl-1H-indazol-5-yl)-6-oxo-1,6-dihydropyrimidin-5-yl)-4,5,6,7-tetrahydro-2H-pyrazolo[4,3-c]pyridine-5-carbonyl)-1H-indol-1-yl)-2-methylcyclopropyl)-1,2,4-oxadiazol-5(4H)-one 22

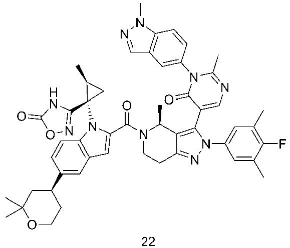

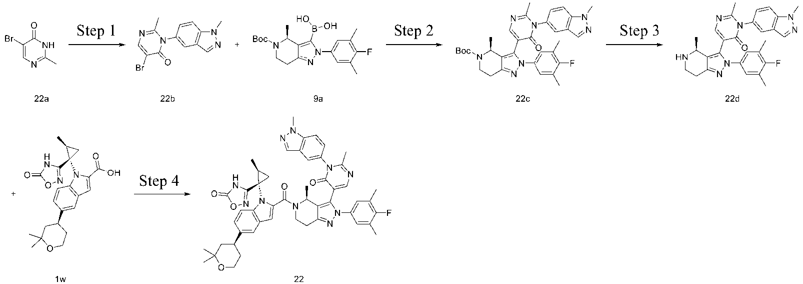

### Step 1

### 5-Bromo-2-methyl-3-(1-methyl-1H-indazol-5-yl)pyrimidin-4(3H)-one 22b

5-Bromo-2-methylpyrimidin-4(3*H*)-one **22a** (500 mg, 2.65 mmol, Bidepharm), 1-methylindazole-5-boronic acid (932 mg, 5.29 mmol, Leyan), pyridine (2.09 g, 26.45 mmol), and copper acetate (1.06 g, 5.29 mmol) were dissolved in 1,2-dichloroethane (20 mL), left open, and stirred at room temperature for 16 hours. The reaction solution was concentrated under reduced pressure. The residue was purified by silica gel column chromatography with eluent system A to obtain the title compound **22b** (15 mg, yield: 1.8%).

MS m/z (ESI): 319.0 [M+1].

### Step 2

### Tert-butyl (S)-2-(4-fluoro-3,5-dimethylphenyl)-4-methyl-3-(2-methyl-1-(1-methyl-1H-indazol-5-yl)-6-oxo-1,6-dihydropyrimidin-5-yl)-2,4,6,7-tetrahydro-5H-pyrazolo[4,3-c]pyridine-5-carboxylate 22c

Compound **22b** (10 mg, 0.031 mmol), compound **9a** (25 mg, 0.062 mmol), anhydrous potassium carbonate (13 mg, 0.094 mmol), tetrakis(triphenylphosphine)palladium (36 mg, 0.031 mmol, Bidepharm), and [1,1'-bis(diphenylphosphino)ferrocene]palladium dichloride (23 mg, 0.031 mmol, Accela) were dissolved in dioxane (10 mL) and water (2 mL). After displacement with nitrogen, the mixture was reacted under stirring at 90°C for 16 hours. The reaction solution was cooled to room temperature and concentrated under reduced pressure. The residue was purified by silica gel column chromatography with eluent system A to obtain the crude title compound **22c** (40 mg). The product was directly used in the next reaction without purification.

MS m/z (ESI): 598.5 [M+1].

### Step 3

### (S)-5-(2-(4-fluoro-3,5-dimethylphenyl)-4-methyl-4,5,6,7-tetrahydro-2H-pyrazolo[4,3-c]pyridin-3-yl)-2-methyl-3-(1-methyl-1H-indazol-5-yl)pyrimidin-4(3H)-one 22d

Compound **22c** (40 mg, 0.067 mmol) was dissolved in dichloromethane (1 mL). A 4M solution of hydrogen chloride in dioxane (2 mL) was added. The reaction was stirred at room temperature for 1 hour. The reaction solution was concentrated under reduced pressure to obtain the crude title compound **22d** (33 mg). The product was directly used in the next reaction without purification.

MS m/z (ESI): 498.5 [M+1].

### Step 4

### 3-((1S,2S)-1-(5-((S)-2,2-dimethyltetrahydro-2H-pyran-4-yl)-2-((S)-2-(4-fluoro-3,5-dimethylphenyl)-4-methyl-3-(2-methyl-1-(1-methyl-1H-indazol-5-yl)-6-oxo-1,6-dihydropyrimidin-5-yl)-4,5,6,7-tetrahydro-2H-pyrazolo[4,3-c]pyridine-5-carbonyl)-1H-indol-1-yl)-2-methylcyclopropyl)-1,2,4-oxadiazol-5(4H)-one 22

Compound **22d** (33 mg, 0.066 mmol) and compound **1w** (30 mg, 0.073 mmol) were dissolved in *N,N*-dimethylformamide (3 mL). *N,N*-diisopropylethylamine (43 mg, 0.33 mmol) was added. After the raw materials were fully dissolved, O-(7-azabenzotriazol-1-yl)-*N,N,N',N'*-tetramethyluronium hexafluorophosphate (38 mg, 0.099 mmol) was added and stirred at room temperature for 3 hours. The reaction solution was poured into 10 mL of water and extracted with ethyl acetate (10 mL × 3). The organic phases were combined, washed with a saturated sodium chloride solution, and dried over anhydrous sodium sulfate. After filtration, the filtrate was concentrated under reduced pressure. The residue was purified by preparative high-performance liquid chromatography (instrument model: Waters-2545, chromatographic column: SP-120-5-ODS-BIO, Prep 30 × 150 mm; 5 µm; C18, mobile phase: water (0.1% trifluoroacetic acid) and acetonitrile, with gradient ratio: acetonitrile 60%-78%, flow rate: 30 mL/min) to obtain the title compound **22** (5 mg, yield: 8.5%).

MS m/z (ESI): 891.6 [M+1].

¹H NMR (500 MHz, CDCl₃): δ 11.52-11.26 (m, 1H), 8.19-7.82 (m, 2H), 7.72-7.47 (m, 4H), 7.27-7.23 (m, 1H), 7.18-6.88 (m, 3H), 6.74-6.54 (m, 1H), 6.26-5.50 (m, 2H), 5.43-5.10 (m, 1H), 4.90-4.37 (m, 1H), 4.27-4.08 (m, 3H), 3.97-3.79 (m, 2H), 3.66-3.55 (m, 1H), 3.20-2.99 (m, 3H), 2.34-2.22 (m, 9H), 2.18 (s, 1H), 2.06-1.90 (m, 2H), 1.82-1.72 (m, 3H), 1.53-1.49 (m, 2H), 1.39-1.35 (m, 4H), 1.22 (d, 2H), 1.02 (t, 1H), 0.90 (t, 2H).

### Example 23

### (R)-7-((S)-5-(5-((S)-2,2-dimethyltetrahydro-2H-pyran-4-yl)-1-((1S,2S)-2-methyl-1-(5-oxo-4,5-dihydro-1,2,4-oxadiazol-3-yl)cyclopropyl)-1H-indole-2-carbonyl)-2-(4-fluoro-3,5-dimethylphenyl)-4-methyl-4,5,6,7-tetrahydro-2H-pyrazolo[4,3-c]pyridin-3-yl)-1-methyl-5,5a,6,7-tetrahydro-1H,8H-imidazo[1',5':4,5][1,4]oxazino[2,3-e]indazol-8-one 23

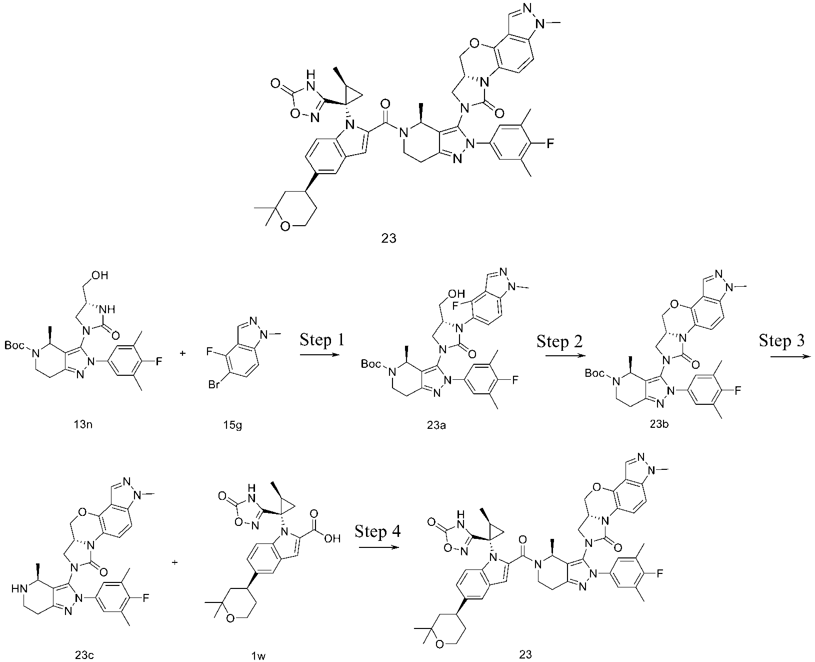

### Step 1

### Tert-butyl (S)-3-((R)-3-(4-fluoro-1-methyl-1H-indazol-5-yl)-4-(hydroxymethyl)-2-oxoimidazolidin-1-yl)-2-(4-fluoro-3,5-dimethylphenyl)-4-methyl-2,4,6,7-tetrahydro-5H-pyrazolo[4,3-c]pyridine-5-carboxylate 23a

Compound **13n** (500 mg, 1.06 mmol), compound **15g** (726 mg, 3.17 mmol, Leyan), and (1*S*,2*S*)-(-)-*N,N'*-dimethyl-1,2-cyclohexanediamine (120 mg, 0.84 mmol) were dissolved in *N,N*-dimethylformamide (5.0 mL). Potassium carbonate (437 mg, 3.17 mmol) and cuprous iodide (80 mg, 0.42 mmol, Leyan) were added. After displacement with nitrogen three times, the reaction was stirred at 120°C for 16 hours. The reaction solution was poured into 50 mL of water and extracted with ethyl acetate (30 mL × 2). The organic phases were combined, washed with a saturated sodium chloride solution, and dried over anhydrous sodium sulfate. After filtration, the filtrate was concentrated under reduced pressure. The residue was purified by silica gel column chromatography with eluent system B to obtain the title compound **23a** (450 mg, yield: 69%).

MS m/z (ESI): 622.5 [M+1].

### Step 2

### Tert-butyl (S)-2-(4-fluoro-3,5-dimethylphenyl)-4-methyl-3-((R)-1-methyl-8-oxo-5a,6-dihydro-1H,5H-imidazo[1',5':4,5][1,4]oxazino[2,3-e]indazol-7(8H)-yl)-2,4,6,7-tetrahydro-5H-pyrazolo[4,3-c]pyridine-5-carboxylate 23b

Compound **23a** (100 mg, 0.16 mmol) was dissolved in dimethyl sulfoxide (10.0 mL). Cesium carbonate (157 mg, 0.48 mmol) was added. After displacement with nitrogen three times, the mixture was heated to 120°C and reacted under stirring 5 hours. The reaction solution was poured into 50 mL of water and extracted with ethyl acetate (30 mL × 2). The organic phases were combined, washed with a saturated sodium chloride solution, and dried over anhydrous sodium sulfate. After filtration, the filtrate was concentrated under reduced pressure. The residue was purified by silica gel column chromatography with eluent system B to obtain the title compound **23b** (96 mg, yield: 99%).

MS m/z (ESI): 602.5 [M+1].

### Step 3

### (R)-7-((S)-2-(4-fluoro-3,5-dimethylphenyl)-4-methyl-4,5,6,7-tetrahydro-2H-pyrazolo[4,3-c]pyridin-3-yl)-1-methyl-5,5a,6,7-tetrahydro-1H,8H-imidazo[1',5':4,5][1,4]oxazino[2,3-e]indazol-8-one 23c

Compound **23b** (50 mg, 0.083 mmol) was dissolved in dichloromethane (4 mL). A 4 M solution of hydrogen chloride in 1,4-dioxane (4 mL) was added and stirred at room temperature for 2 hours. The reaction solution was concentrated under reduced pressure to obtain the crude title compound **23c** (41 mg). The product was directly used in the next reaction without purification.

MS m/z (ESI): 502.4 [M+1].

### Step 4

### (R)-7-((S)-5-(5-((S)-2,2-dimethyltetrahydro-2H-pyran-4-yl)-1-((1S,2S)-2-methyl-1-(5-oxo-4,5-dihydro-1,2,4-oxadiazol-3-yl)cyclopropyl)-1H-indole-2-carbonyl)-2-(4-fluoro-3,5-dimethylphenyl)-4-methyl-4,5,6,7-tetrahydro-2H-pyrazolo[4,3-c]pyridin-3-yl)-1-methyl-5,5a,6,7-tetrahydro-1H,8H-imidazo[1',5':4,5][1,4]oxazino[2,3-e]indazol-8-one 23

Compound **23c** (41 mg, 0.083 mmol) and compound **1w** (34 mg, 0.083 mmol) were dissolved in *N,N*-dimethylformamide (5 mL). *N,N*-diisopropylethylamine (55 mg, 0.43 mmol) was added. After the raw materials were fully dissolved, *O*-(7-azabenzotriazol-1-yl)-*N,N,N',N'*-tetramethyluronium hexafluorophosphate (39 mg, 0.10 mmol) was added and stirred at room temperature for 16 hours. After filtration, the residue was purified by preparative high-performance liquid chromatography (instrument model: Waters-2545 chromatographic column: SP-120-5-ODS-BIO, Prep 30 × 150 mm; 5 µm; C18, mobile phase: water (10 mM ammonium bicarbonate) and acetonitrile, with gradient ratio: acetonitrile 30%-60%, flow rate: 30 mL/min) to obtain the title compound **23** (30 mg, yield: 41%).

MS m/z (ESI): 895.5 [M+1].

¹H NMR (500 MHz, DMSO-*d*₆): δ 11.36-11.22 (m, 1H), 7.99-7.93 (m, 1H), 7.86-7.62 (m, 1H), 7.52-7.46 (m, 1H), 7.40-7.38 (m, 1H), 7.25-7.06 (m, 4H), 6.93-6.66 (m, 1H), 5.81-5.68 (m, 1H), 5.33-4.58 (m, 2H), 4.50-4.30 (m, 1H), 4.25-4.16 (m, 1H), 4.06-3.94 (m, 3H), 3.93-3.55 (m, 4H), 3.43-2.79 (m, 4H), 2.28-2.14 (m, 6H), 1.82-1.42 (m, 9H), 1.34-1.11 (m, 10H).

### Example 24

### (R)-1-cyclopropyl-7-((S)-5-(5-((S)-2,2-dimethyltetrahydro-2H-pyran-4-yl)-1-((1S,2S)-2-methyl-1-(5-oxo-4,5-dihydro-1,2,4-oxadiazol-3-yl)cyclopropyl)-1H-indole-2-carbonyl)-2-(4-fluoro-3,5-dimethylphenyl)-4-methyl-4,5,6,7-tetrahydro-2H-pyrazolo[4,3-c]pyridin-3-yl)-5,5a,6,7-tetrahydro-1H,8H-imidazo[1',5':4,5][1,4]oxazino[2,3-e]indazol-8-one 24

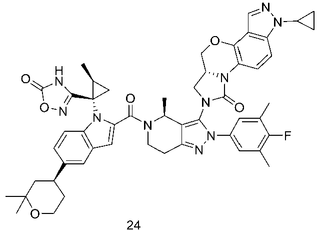

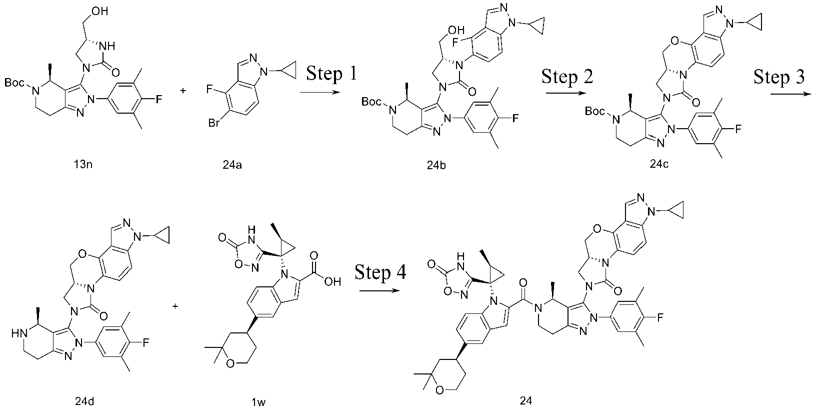

### Step 1

### Tert-butyl (S)-3-((R)-3-(1-cyclopropyl-4-fluoro-1H-indazol-5-yl)-4-(hydroxymethyl)-2-oxoimidazolidin-1-yl)-2-(4-fluoro-3,5-dimethylphenyl)-4-methyl-2,4,6,7-tetrahydro-5H-pyrazolo[4,3-c]pyridine-5-carboxylate 24b

Compound **13n** (500 mg, 1.06 mmol), 5-bromo-1-cyclopropyl-4-fluoro-1*H-*indazole **24a** (808 mg, 3.17 mmol, prepared by a method disclosed for intermediate 8-3 on page 33 in the description of Patent Application CN 111303118 A), and (1S,2S)-(-)-*N,N'*-dimethyl-1,2-cyclohexanediamine (120 mg, 0.84 mmol) were dissolved in *N,N-*dimethylformamide (5.0 mL). Potassium carbonate (437 mg, 3.17 mmol) and cuprous iodide (80 mg, 0.42 mmol, Leyan) were added. After displacement with nitrogen three times, the reaction was stirred at 120°C for 16 hours. The reaction solution was poured into 50 mL of water and extracted with ethyl acetate (30 mL × 2). The organic phases were combined, washed with a saturated sodium chloride solution, and dried over anhydrous sodium sulfate. After filtration, the filtrate was concentrated under reduced pressure. The residue was purified by silica gel column chromatography with eluent system B to obtain the title compound **24b** (330 mg, yield: 48%).

MS m/z (ESI): 648.5 [M+1].

### Step 2

### Tert-butyl (S)-3-((R)-1-cyclopropyl-8-oxo-5a,6-dihydro-1H,5H-imidazo[1',5':4,5][1,4]oxazino[2,3-e]indazol-7(8H)-yl)-2-(4-fluoro-3,5-dimethylphenyl)-4-methyl-2,4,6,7-tetrahydro-5H-pyrazolo[4,3-c]pyridine-5-carboxylate 24c

Compound **24b** (130 mg, 0.20 mmol) was dissolved in dimethyl sulfoxide (10.0 mL). Cesium carbonate (196 mg, 0.60 mmol) was added. After displacement with nitrogen three times,the mixture was reacted under stirring at 120°C for 2 hours. The reaction solution was poured into 50 mL of water and extracted with ethyl acetate (30 mL × 2). The organic phases were combined, washed with a saturated sodium chloride solution, and dried over anhydrous sodium sulfate. After filtration, the filtrate was concentrated under reduced pressure. The residue was purified by silica gel column chromatography with eluent system B to obtain the title compound **24c** (125 mg, yield: 99%).

MS m/z (ESI): 628.5 [M+1].

### Step 3

### (R)-1-cyclopropyl-7-((S)-2-(4-fluoro-3,5-dimethylphenyl)-4-methyl-4,5,6,7-tetrahydro-2H-pyrazolo[4,3-c]pyridin-3-yl)-5,5a,6,7-tetrahydro-1H,8H-imidazo[1',5':4,5][1,4]oxazino[2,3-e]indazol-8-one 24d

Compound **24c** (70 mg, 0.11 mmol) was dissolved in dichloromethane (4 mL). A 4 M solution of hydrogen chloride in 1,4-dioxane (4 mL) was added and stirred at room temperature for 1 hours. The reaction solution was concentrated under reduced pressure to obtain the crude title compound **24d** (58 mg). The product was directly used in the next reaction without purification.

MS m/z (ESI): 528.5 [M+1].

### Step 4

### (R)-1-cyclopropyl-7-((S)-5-(5-((S)-2,2-dimethyltetrahydro-2H-pyran-4-yl)-1-((1S,2S)-2-methyl-1-(5-oxo-4,5-dihydro-1,2,4-oxadiazol-3-yl)cyclopropyl)-1H-indole-2-carbonyl)-2-(4-fluoro-3,5-dimethylphenyl)-4-methyl-4,5,6,7-tetrahydro-2H-pyrazolo[4,3-c]pyridin-3-yl)-5,5a,6,7-tetrahydro-1H,8H-imidazo[1',5':4,5][1,4]oxazino[2,3-e]indazol-8-one 24

Compound **24d** (58 mg, 0.11 mmol) and compound **1w** (45 mg, 0.11 mmol) were dissolved in *N,N*-dimethylformamide (5 mL). *N,N*-diisopropylethylamine (71 mg, 0.55 mmol) was added. After the raw materials were fully dissolved, *O*-(7-azabenzotriazol-1-yl)-*N,N,N',N'*-tetramethyluronium hexafluorophosphate (50 mg, 0.13 mmol) was added and stirred at room temperature for 16 hours. After filtration, the residue was purified by preparative high-performance liquid chromatography (instrument model: Gilson281 chromatographic column: Phlex, Prep 30 × 150 mm; 5 µm; C18, mobile phase: water (10 mM ammonium bicarbonate) and acetonitrile, with gradient ratio: acetonitrile 35%-65%, flow rate: 30 mL/min) to obtain the title compound **24** (60 mg, yield: 60%).

MS m/z (ESI): 921.5 [M+1].

¹H NMR (500 MHz, CDCl₃): δ11.33-11.27 (m, 1H), 8.05-7.79 (m, 2H), 7.59-7.46 (m, 2H), 7.27-7.13 (m, 4H), 6.69-6.68 (m, 1H), 5.81-5.21 (m, 1H), 4.84-4.35 (m, 2H), 4.17-3.50 (m, 6H), 3.36-2.95 (m, 4H), 2.37-2.07 (m, 6H), 2.02-1.46 (m, 10H), 1.43-0.85 (m, 14H).

### Example 25

### (R)-7-(difluoromethyl)-2-((S)-5-(5-((S)-2,2-dimethyltetrahydro-2H-pyran-4-yl)-1-((1S,2S)-2-methyl-1-(5-oxo-4,5-dihydro-1,2,4-oxadiazol-3-yl)cyclopropyl)-1H-indole-2-carbonyl)-2-(4-fluoro-3,5-dimethylphenyl)-4-methyl-4,5,6,7-tetrahydro-2H-pyrazolo[4,3-c]pyridin-3-yl)-10-fluoro-2,3,3a,4-tetrahydro-1H,7H-imidazo[1',5':4,5][1,4]oxazino[3,2-f]indazol-1-one 25

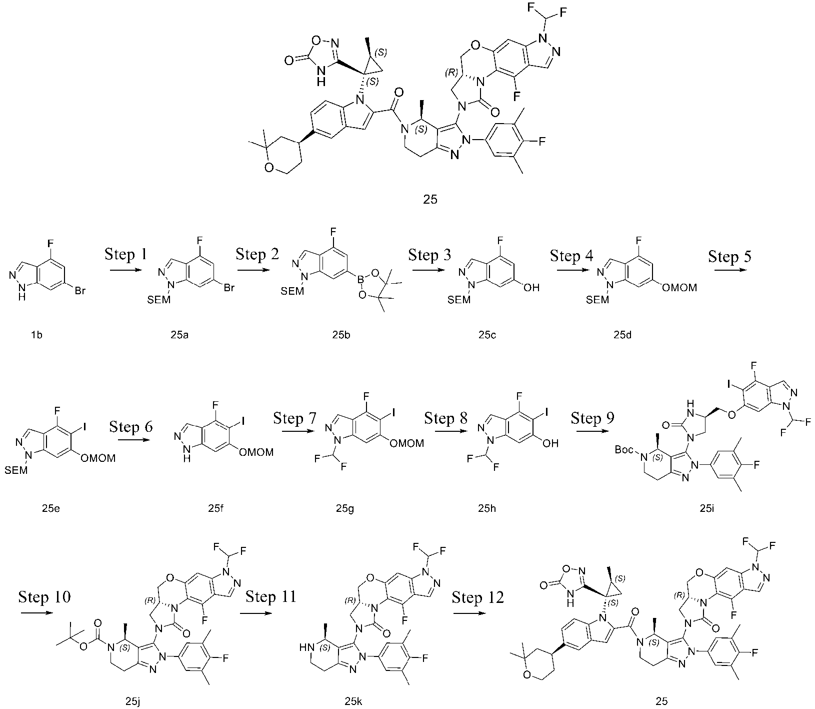

### Step 1

### 6-Bromo-4-fluoro-1-((2-(trimethylsilyl)ethoxy)methyl)-1H-indazole 25a

Compound **1b** (2.0 g, 9.30 mmol) was dissolved in *N,N*-dimethylformamide (20 mL). Sodium hydride (558 mg, 13.95 mmol) was added in portions at 0°C and stirred for 30 minutes. 2-(Trimethylsilyl)ethoxymethyl chloride (2 g, 12.05 mmol) was dropwise added. After the addition was complete, the mixture was reacted under stirring at room temperature for 3 hours. The reaction solution was quenched with a saturated aqueous ammonium chloride solution (100 mL), extracted with ethyl acetate (50 mL × 2), and washed with a saturated sodium chloride solution. The organic phase was dried over anhydrous sodium sulfate. After filtration, the filtrate was concentrated under reduced pressure. The residue was purified by silica gel column chromatography with eluent system B to obtain the title compound **25a** (3.2 g, yield: 99.6%).

### Step 2

### 4-Fluoro-6-(4,4,5,5-tetramethyl-1,3,2-dioxaborolan-2-yl)-1-((2-(trimethylsilyl)ethoxy)methyl)-1H-indazole 25b

Compound **25a** (3.0 g, 8.68 mmol), potassium acetate (2.1 g, 21.7mmol), bis(pinacolato)diboron (3.3 g, 13.0 mmol, Shaoyuan Technology (Shanghai) Co., Ltd.), and [1,1'-bis(diphenylphosphino)ferrocene]palladium dichloride (1.27 g, 1.73 mmol, Shanghai Bidepharm) were dissolved in dioxane (100 mL). After displacement with nitrogen three times, the mixture was reacted at 85°C for 16 hours. The reaction solution was cooled to room temperature. Water (100 mL) was added. The mixture was extracted with ethyl acetate (100 mL × 3). The organic phases were combined and washed with a saturated sodium chloride solution. The organic phase was dried over anhydrous sodium sulfate. After filtration, the filtrate was concentrated under reduced pressure. The residue was purified by silica gel column chromatography with eluent system **B** to obtain the title compound **25b** (3.0 g, yield: 99.3%).

MS m/z (ESI): 393.4 [M+1].

### Step 3

### 4-Fluoro-1-((2-(trimethylsilyl)ethoxy)methyl)-1H-indazol-6-ol 25c

Compound **25b** (3 g, 7.65 mmol) was dissolved in tetrahydrofuran (20 mL). A solution of sodium perborate tetrahydrate (3.86 g, 23.56 mmol) in water (5 mL) was dropwise added at 0°C. After the addition was complete, the mixture was reacted at room temperature for 16 hours. The reaction solution was poured into 50 mL of water, extracted with dichloromethane (30 mL × 3), and dried over anhydrous sodium sulfate. After filtration, the filtrate was concentrated under reduced pressure. The residue was purified by silica gel column chromatography with eluent system B to obtain the title compound **25c** (1.9 g, yield: 90.4%).

MS m/z (ESI): 281.1 [M-1].

### Step 4

### 4-Fluoro-6-(methoxymethoxy)-1-((2-(trimethylsilyl)ethoxy)methyl)-1H-indazole 25d

Compound **25c** (1.9 g, 6.72 mmol) and N,N-diisopropylethylamine (1.73 g, 13.38 mmol) were dissolved in dichloromethane (50 mL). Bromomethyl methyl ether (1.26 g, 10.08 mmol) was dropwise added at 0°C. After the addition was complete, the mixture was stirred at room temperature for 2 hours. The mixture was quenched with water (50 mL) and extracted with dichloromethane (50 mL × 3). The organic phases were combined and washed with a saturated sodium chloride solution. The organic phase was dried over anhydrous sodium sulfate. After filtration, the filtrate was concentrated under reduced pressure. The residue was purified by silica gel column chromatography with eluent system C to obtain the title compound **25d** (1.55 g, yield: 70.6%).

### Step 5

### 4-Fluoro-5-iodo-6-(methoxymethoxy)-1-((2-(trimethylsilyl)ethoxy)methyl)-1H-indazole 25e

Compound **25d** (1.43 g, 4.38 mmol) was dissolved in tetrahydrofuran (50 mL). Lithium diisopropylamide (3.3 mL, 6.6 mmol, 2.0 M, Adamas) was dropwise added in a nitrogen atmosphere at -78°C. Stirring was continued for 2 hours. A solution of iodine (1.49 g, 6.54 mmol) in tetrahydrofuran (5 mL) was then dropwise added. After the addition was complete, stirring was continued at -78°C for 1 hour, and the mixture was slowly heated to room temperature. The reaction was quenched with water (50 mL) and extracted with ethyl acetate (30 mL × 3). The organic phases were combined and washed with a saturated sodium chloride solution. The organic phase was dried over anhydrous sodium sulfate. After filtration, the filtrate was concentrated under reduced pressure. The residue was purified by silica gel column chromatography with eluent system **B** to obtain the title compound **25e** (1.5 g, yield: 75.7%).

MS m/z (ESI): 453.2 [M+1].

### Step 6

### 4-Fluoro-5-iodo-6-(methoxymethoxy)-1H-indazole 25f

Compound **25e** (1.6 g, 3.53 mmol) and tetrabutylammonium fluoride (17.6 mL, 17.6 mmol, 1.0 M, Adamas) were dissolved in tetrahydrofuran (20 mL) and reacted at 60°C for 5 hours. The reaction solution was cooled to room temperature. 30 mL of water was added. The mixture was extracted with ethyl acetate (30 mL × 3). The organic phases were combined, washed with a saturated aqueous sodium chloride solution, and dried over anhydrous sodium sulfate. After filtration, the filtrate was concentrated under reduced pressure. The residue was purified by silica gel column chromatography with eluent system C to obtain the title compound **25f** (720 mg, yield: 63.2%).

MS m/z (ESI): 323.0 [M+1].

### Step 7

### 1-(Difluoromethyl)-4-fluoro-5-iodo-6-(methoxymethoxy)-1H-indazole 25g

Compound **25f** (700 mg, 2.17 mmol), sodium difluorochloroacetate (662.7 mg, 4.34 mmol, Shaoyuan Technology (Shanghai) Co., Ltd.), and cesium carbonate (1.41 g, 4.32 mmol, Shaoyuan Technology (Shanghai) Co., Ltd.) were dissolved in *N,N*-dimethylacetamide (20 mL) and stirred at 130°C for 5 hours. The reaction solution was cooled to room temperature. 60 mL of water was added. The mixture was extracted with ethyl acetate (30 mL × 3). The organic phases were combined, washed with a saturated aqueous sodium chloride solution, and dried over anhydrous sodium sulfate. After filtration, the filtrate was concentrated under reduced pressure. The residue was purified by silica gel column chromatography with eluent system C to obtain the title compound **25g** (50 mg, yield: 6.18%).

MS m/z (ESI): 373.0 [M+1].

### Step 8

### 1-(Difluoromethyl)-4-fluoro-5-iodo-1H-indazol-6-ol 25h

Compound **25g** (65 mg, 0.17 mmol) was dissolved in dichloromethane (6 mL). A hydrogen chloride/dioxane solution (6 mL, 4.0 M) was dropwise added at 0°C. After the addition was complete, the mixture was stirred at room temperature for 1 hour. After concentration, 30 mL of water was added. The mixture was extracted with ethyl acetate (30 mL × 3). The organic phases were combined, washed with a saturated aqueous sodium chloride solution, and dried over anhydrous sodium sulfate. After filtration, the filtrate was concentrated under reduced pressure. The residue was purified by silica gel column chromatography with eluent system B to obtain the title compound **25h** (57 mg, yield: 100%).

MS m/z (ESI): 329.0 [M+1].

### Step 9

### Tert-butyl (S)-3-((R)-4-(((1-(difluoromethyl)-4-fluoro-5-iodo-1H-indazol-6-yl)oxy)methyl)-2-oxoimidazolidin-1-yl)-2-(4-fluoro-3,5-dimethylphenyl)-4-methyl-2,4,6,7-tetrahydro-5H-pyrazolo[4,3-c]pyridine-5-carboxylate 25i

Compound **13o** (129 mg, 0.23 mmol) and compound **25h** (70 mg, 0.21 mmol) were dissolved in *N,N-*dimethylformamide (10.0 mL). Potassium carbonate (88 mg, 0.64 mmol) was added and reacted at 80°C for 5 hours. The reaction solution was cooled to room temperature and poured into 20 mL of water. The mixture was extracted with ethyl acetate (30 mL × 2). The organic phases were combined, washed with a saturated sodium chloride solution, and dried over anhydrous sodium sulfate. After filtration, the filtrate was concentrated under reduced pressure. The residue was purified by silica gel column chromatography with eluent system A to obtain the title compound **25i** (100 mg, yield: 59.8%).

MS m/z (ESI): 784.3 [M+1].

### Step 10

### Tert-butyl (S)-3-((R)-7-(difluoromethyl)-10-fluoro-1-oxo-3a,4-dihydro-1H,7H-imidazo[1',5':4,5][1,4]oxazino[3,2-f]indazol-2(3H)-yl)-2-(4-fluoro-3,5-dimethylphenyl)-4-methyl-2,4,6,7-tetrahydro-5H-pyrazolo[4,3-c]pyridine-5-carboxylate 25j

Compound **25i** (70 mg, 0.09 mmol) and (1*R*,2*R*)-(-)-*N,N'*-dimethyl-1,2-cyclohexanediamine (17 mg, 0.09 mmol) were dissolved in *N,N-*dimethylformamide (10.0 mL). Potassium carbonate (37 mg, 0.27 mmol) and cuprous iodide (12 mg, 0.09 mmol, Leyan) were added. After displacement with nitrogen three times, the mixture was reacted at 125°C for 2 hours. The reaction solution was cooled to room temperature and poured into 20 mL of water. The mixture was extracted with ethyl acetate (30 mL × 2). The organic phases were combined, washed with a saturated sodium chloride solution, and dried over anhydrous sodium sulfate. After filtration, the filtrate was concentrated under reduced pressure. The residue was purified by silica gel column chromatography with eluent system C to obtain the title compound **25j** (35 mg, yield: 59.7%).

MS m/z (ESI): 656.5 [M+1].

### Step 11

### (R)-7-(difluoromethyl)-10-fluoro-2-((S)-2-(4-fluoro-3,5-dimethylphenyl)-4-methyl-4,5,6,7-tetrahydro-2H-pyrazolo[4,3-c]pyridin-3-yl)-2,3,3a,4-tetrahydro-1H,7H-imidazo[1',5':4,5][1,4]oxazino[3,2-f]indazol-1-one 25k

Compound **25j** (50 mg, 0.07 mmol) was dissolved in ethyl acetate (2 mL). A 4 M solution of hydrogen chloride in 1,4-dioxane (2 mL) was added and stirred at room temperature for 2 hours. The reaction solution was concentrated under reduced pressure to obtain the crude title compound **25k** (40 mg). The product was directly used in the next reaction without purification.

MS m/z (ESI): 556.4 [M+1].

### Step 12

### (R)-7-(difluoromethyl)-2-((S)-5-(5-((S)-2,2-dimethyltetrahydro-2H-pyran-4-yl)-1-((1S,2S)-2-methyl-1-(5-oxo-4,5-dihydro-1,2,4-oxadiazol-3-yl)cyclopropyl)-1H-indole-2-carbonyl)-2-(4-fluoro-3,5-dimethylphenyl)-4-methyl-4,5,6,7-tetrahydro-2H-pyrazolo[4,3-c]pyridin-3-yl)-10-fluoro-2,3,3a,4-tetrahydro-1H,7H-imidazo[1',5':4,5][1,4]oxazino[3,2-f]indazol-1-one 25

Crude compound **25k** (42 mg, 0.07 mmol) and compound **1w** (37 mg, 0.09 mmol) were dissolved in *N,N*-dimethylformamide (3 mL). *N,N-*diisopropylethylamine (48 mg, 0.38 mmol) was added. After the raw materials were fully dissolved, *O*-(7-azabenzotriazol-1-yl)-*N,N,N',N'*-tetramethyluronium hexafluorophosphate (57 mg, 0.15 mmol) was added and stirred at room temperature for 3 hours. The reaction solution was poured into 10 mL of water and extracted with ethyl acetate (10 mL × 3). The organic phases were combined, washed with a saturated sodium chloride solution, and dried over anhydrous sodium sulfate. After filtration, the filtrate was concentrated under reduced pressure. The residue was purified by preparative high-performance liquid chromatography (instrument model: Waters-2545, chromatographic column: Sharpsil-T, Prep 30 × 150 mm; 5 µm; C18, mobile phase: water (10 mM ammonium bicarbonate) and acetonitrile, with gradient ratio: acetonitrile 40%-65%, flow rate: 30 mL/min) to obtain the title compound **25** (10 mg, yield: 13.9%).

MS m/z (ESI): 949.6 [M+1].

¹H NMR (500 MHz, CDCl₃): δ 11.29-11.22 (m, 1H), 8.41-8.38 (m, 1H), 7.60-7.58 (m, 1H), 7.53-7.47 (m, 2H), 7.41-7.37 (m, 1H), 7.20-7.15 (m, 2H), 7.05-6.95 (m, 1H), 6.69 (s, 1H), 5.80-5.76 (m, 1H), 4.47-4.43 (m, 1H), 4.22-4.17 (m, 1H), 4.08-4.06 (m, 2H), 3.87-3.80 (m, 2H), 3.64-3.58 (m, 2H), 3.15-2.96 (m, 5H), 2.28 (s, 6H), 2.23-2.20 (m, 1H), 2.05-1.90 (m, 3H), 1.80-1.60 (m, 7H), 1.34-1.21 (m, 6H), 1.05 (d, 1H).

### Examples 26-1 and 26-2

### (R)-7-cyclopropyl-2-((S)-5-(7-((S)-2,2-dimethyltetrahydro-2H-pyran-4-yl)-3-(1-(5-oxo-4,5-dihydro-1,2,4-oxadiazol-3-yl)cyclopropyl)indolizine-2-carbonyl)-2-(4-fluoro-3,5-dimethylphenyl)-4-methyl-4,5,6,7-tetrahydro-2H-pyrazolo[4,3-c]pyridin-3-yl)-10-fluoro-2,3,3a,4-tetrahydro-1H,7H-imidazo[1',5':4,5][1,4]oxazino[3,2-f]indazol-1-one 26-1 or

### (R)-7-cyclopropyl-2-((S)-5-(7-((R)-2,2-dimethyltetrahydro-2H-pyran-4-yl)-3-(1-(5-oxo-4,5-dihydro-1,2,4-oxadiazol-3-yl)cyclopropyl)indolizine-2-carbonyl)-2-(4-fluoro-3,5-dimethylphenyl)-4-methyl-4,5,6,7-tetrahydro-2H-pyrazolo[4,3-c]pyridin-3-yl)-10-fluoro-2,3,3a,4-tetrahydro-1H,7H-imidazo[1',5':4,5][1,4]oxazino[3,2-f]indazol-1-one 26-2

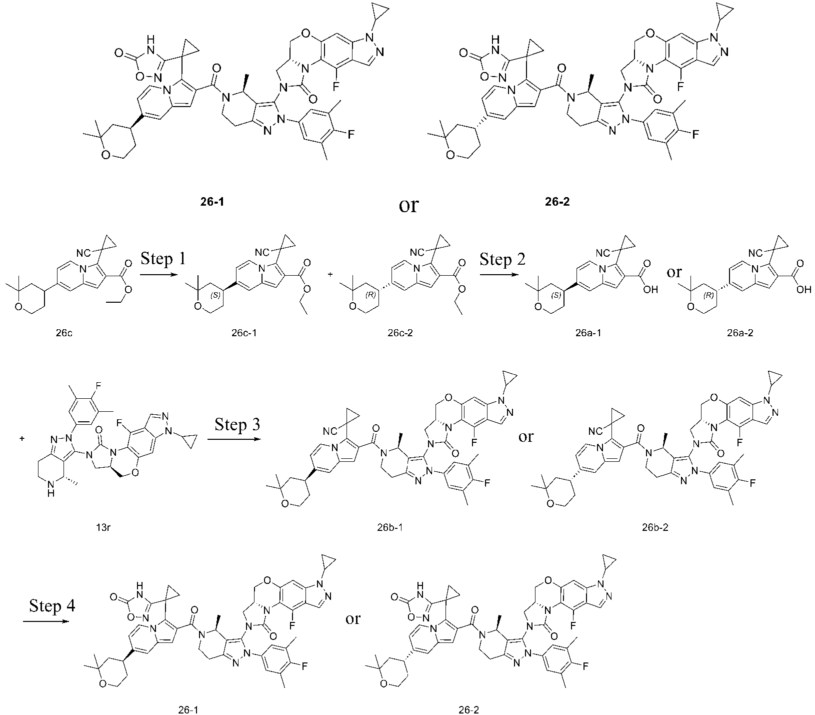

### Step 1

### Ethyl (S)-3-(1-cyanocyclopropyl)-7-(2,2-dimethyltetrahydro-2H-pyran-4-yl)indolizine-2-carboxylate 26c-1

### Ethyl (R)-3-(1-cyanocyclopropyl)-7-(2,2-dimethyltetrahydro-2H-pyran-4-yl)indolizine-2-carboxylate 26c-2

Ethyl 3-(1-cyanocyclopropyl)-7-(2,2-dimethyltetrahydro-2*H*-pyran-4-yl)indolizine-2-carboxylate **26c** (160 mg, prepared by a method disclosed for intermediate 31-G on page 164 in the description of Patent Application WO 2022/17338) was subjected to chiral HPLC resolution (instrument model: Gilson-281 Prep system, chromatographic column: ChiralPak IE, Prep 20 × 250 mm; 5 µm; mobile phase: n-hexane and ethanol, with gradient ratio: ethanol 30%-50%, flow rate: 15 mL/min) to obtain the title compound (80 mg, yield: 50%, retention time: 12.4 minutes) and (70 mg, yield: 43.8%, retention time: 18.9 minutes).

MS m/z (ESI): 367.3 [M+1]. Single-configuration compound (shorter retention time).

MS m/z (ESI): 367.4 [M+1]. Single-configuration compound (longer retention time).

### Step 2

### (S)-3-(1-cyanocyclopropyl)-7-(2,2-dimethyltetrahydro-2H-pyran-4-yl)indolizine-2-carboxylic acid 26a-1 or

### (R)-3-(1-cyanocyclopropyl)-7-(2,2-dimethyltetrahydro-2H-pyran-4-yl)indolizine-2-carboxylic acid 26a-2

The compound with a shorter retention time (80 mg, 0.22 mmol) among compounds **26c-1** and **26c-2** was dissolved in water (1 mL) and methanol (5 mL). Lithium hydroxide monohydrate (183 mg, 4.36 mmol) was added and reacted under stirring at 50°C for 16 hours. The reaction solution was cooled to room temperature and concentrated under reduced pressure to remove the organic solvent, followed by dilution with water. The diluted solution was adjusted to pH 3 with 2M dilute hydrochloric acid and extracted with dichloromethane (20 mL × 3). The organic phase was dried over anhydrous sodium sulfate. After filtration, the filtrate was concentrated under reduced pressure. The residue was purified by silica gel column chromatography with eluent system B to obtain the title compound **26a-1** or **26a-2** (39 mg, yield: 52.8%).

MS m/z (ESI): 339.2 [M+1].

### Step 3

### 1-(2-((S)-3-((R)-7-cyclopropyl-10-fluoro-1-oxo-3a,4-dihydro-1H,7H-imidazo[1',5':4,5][1,4]oxazino[3,2-f]indazol-2(3H)-yl)-2-(4-fluoro-3,5-dimethylphenyl)-4-methyl-4,5,6,7-tetrahydro-2H-pyrazolo[4,3-c]pyridine-5-carbonyl)-7-((S)-2,2-dimethyltetrahydro-2H-pyran-4-yl)indolizin-3-yl)cyclopropane-1-carbonitrile 26b-1 or

### 1-(2-((S)-3-((R)-7-cyclopropyl-10-fluoro-1-oxo-3a,4-dihydro-1H,7H-imidazo[1',5':4,5][1,4]oxazino[3,2-f]indazol-2(3H)-yl)-2-(4-fluoro-3,5-dimethylphenyl)-4-methyl-4,5,6,7-tetrahydro-2H-pyrazolo[4,3-c]pyridine-5-carbonyl)-7-((R)-2,2-dimethyltetrahydro-2H-pyran-4-yl)indolizin-3-yl)cyclopropane-1-carbonitrile 26b-2

Compound **13r** (63 mg, 0.115 mmol) and compound **26a-1** or **26a-2** (39 mg, 0.115 mmol, prepared in one step from the compound with a shorter retention time, among compounds **26c-1** and **26c-2)** were dissolved in *N,N*-dimethylformamide (3 mL). *O*-(7-azabenzotriazol-1-yl)-*N,N,N',N'*-tetramethyluronium hexafluorophosphate(51 mg, 0.134 mmol) and *N,N*-diisopropylethylamine (50 mg, 0.387 mmol) were added and stirred at room temperature for 16 hours. The reaction solution was filtered. The residue was purified by silica gel column chromatography with eluent system B to obtain the title compound **26b-1 or 26b-2** (50 mg, yield: 50%).

MS m/z (ESI): 866.6 [M+1].

### Step 4

### (R)-7-cyclopropyl-2-((S)-5-(7-((S)-2,2-dimethyltetrahydro-2H-pyran-4-yl)-3-(1-(5-oxo-4,5-dihydro-1,2,4-oxadiazol-3-yl)cyclopropyl)indolizine-2-carbonyl)-2-(4-fluoro-3,5-dimethylphenyl)-4-methyl-4,5,6,7-tetrahydro-2H-pyrazolo[4,3-c]pyridin-3-yl)-10-fluoro-2,3,3a,4-tetrahydro-1H,7H-imidazo[1',5':4,5][1,4]oxazino[3,2-f]indazol-1-one 26-1 or

### (R)-7-cyclopropyl-2-((S)-5-(7-((R)-2,2-dimethyltetrahydro-2H-pyran-4-yl)-3-(1-(5-oxo-4,5-dihydro-1,2,4-oxadiazol-3-yl)cyclopropyl)indolizine-2-carbonyl)-2-(4-fluoro-3,5-dimethylphenyl)-4-methyl-4,5,6,7-tetrahydro-2H-pyrazolo[4,3-c]pyridin-3-yl)-10-fluoro-2,3,3a,4-tetrahydro-1H,7H-imidazo[1',5':4,5][1,4]oxazino[3,2-f]indazol-1-one 26-2

Compound **26b-1** or **26b-2** (50 mg, 0.057 mmol, prepared in two steps from the compound with a shorter retention time, among compounds **26c-1** and **26c-2)** and hydroxylamine hydrochloride (40 mg, 0.577 mmol) were dissolved in dimethyl sulfoxide (2 mL). Sodium bicarbonate (48 mg, 0.577 mmol) was added and reacted under stirring at 60°C for 3.5 hours. The reaction mixture was cooled to room temperature, quenched with water (10 mL), and extracted with ethyl acetate (30 mL × 3). The organic phase was dried over anhydrous sodium sulfate. After filtration, the filtrate was concentrated under reduced pressure. The residue was dissolved in dimethyl sulfoxide (2 mL). *N,N'*-carbonyldiimidazole (25 mg, 0.173 mmol) and 1,8-diazabicyclo[5,4,0]undec-7-ene (58 mg, 0.23 mmol) were added and reacted under stirring at room temperature for 3 hours. The reaction solution was quenched with water (10 mL) and extracted with ethyl acetate (30 mL × 3). The organic phase was dried over anhydrous sodium sulfate. After filtration, the filtrate was concentrated under reduced pressure. The filtrate was purified by preparative high-performance liquid chromatography (instrument model: Gilson-GX-281 chromatographic column: SP-120-5-ODS-BIO, Prep 30 × 150 mm; 5 µm; C18 mobile phase: water (10 mM ammonium bicarbonate) and acetonitrile, with gradient ratio: acetonitrile 55%-80%, flow rate: 30 mL/min) to obtain the title compound **26-1** or **26-2** (23 mg, yield: 43%, prepared in three steps from the compound with a shorter retention time among compounds **26c-1** and **26c-2).**

MS m/z (ESI): 923.5 [M-1].

¹H NMR (500 MHz, CDCl₃): δ 8.18 (d, 1H), 7.98 (s, 1H), 7.26 (s, 1H), 7.18 (d, 2H), 6.92 (s, 1H), 6.65 (d, 1H), 6.39 (s,1H), 5.87-5.76 (m, 1H), 5.44-5.31 (m, 2H), 4.40-4.33 (m, 1H), 4.29-4.20 (m, 1H), 4.17-4.09 (m, 2H), 4.01 (t, 1H), 3.71-3.60 (m, 3H), 3.54-3.38 (m, 4H), 3.18-2.85 (m, 5H), 2.30-2.26 (m, 6H), 2.23 (t, 1H), 1.83-1.55 (m, 7H), 1.40-1.22 (m, 6H), 0.98 (d, 2H).

### Example 26-2 or 26-1

### (R)-7-cyclopropyl-2-((S)-5-(7-((R)-2,2-dimethyltetrahydro-2H-pyran-4-yl)-3-(1-(5-oxo-4,5-dihydro-1,2,4-oxadiazol-3-yl)cyclopropyl)indolizine-2-carbonyl)-2-(4-fluoro-3,5-dimethylphenyl)-4-methyl-4,5,6,7-tetrahydro-2H-pyrazolo[4,3-c]pyridin-3-yl)-10-fluoro-2,3,3a,4-tetrahydro-1H,7H-imidazo[1',5':4,5][1,4]oxazino[3,2-f]indazol-1-one 26-2 or

### (R)-7-cyclopropyl-2-((S)-5-(7-((S)-2,2-dimethyltetrahydro-2H-pyran-4-yl)-3-(1-(5-oxo-4,5-dihydro-1,2,4-oxadiazol-3-yl)cyclopropyl)indolizine-2-carbonyl)-2-(4-fluoro-3,5-dimethylphenyl)-4-methyl-4,5,6,7-tetrahydro-2H-pyrazolo[4,3-c]pyridin-3-yl)-10-fluoro-2,3,3a,4-tetrahydro-1H,7H-imidazo[1',5':4,5][1,4]oxazino[3,2-f]indazol-1-one 26-1

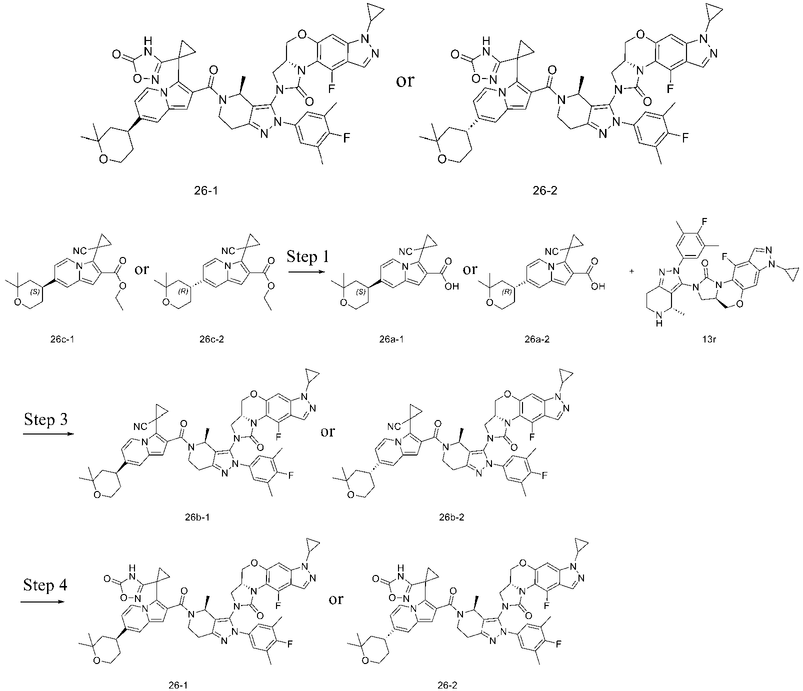

### Step 1

### (R)-3-(1-cyanocyclopropyl)-7-(2,2-dimethyltetrahydro-2H-pyran-4-yl)indolizine-2-carboxylic acid 26a-2 or

### (S)-3-(1-cyanocyclopropyl)-7-(2,2-dimethyltetrahydro-2H-pyran-4-yl)indolizine-2-carboxylic acid 26a-1

The compound with a longer retention time (70 mg, 0.19 mmol, with a shorter retention time) among compounds **26c-2** and **26c-1** was dissolved in water (2 mL), tetrahydrofuran (10 mL), and methanol (2 mL). Lithium hydroxide monohydrate (160 mg, 3.82 mmol) was added and reacted under stirring at 50°C for 16 hours. The reaction solution was cooled to room temperature and concentrated under reduced pressure to remove the organic solvent, followed by dilution with water. The diluted solution was adjusted to pH 5 with 2M dilute hydrochloric acid and extracted with dichloromethane (20 mL × 3). The organic phase was dried over anhydrous sodium sulfate. After filtration, the filtrate was concentrated under reduced pressure. The residue was purified by silica gel column chromatography with eluent system B to obtain the title compound **26a-2** or **26a-1** (60 mg, yield: 92.8%).

MS m/z (ESI): 339.3 [M+1].

### Step 2

### 1-(2-((S)-3-((R)-7-cyclopropyl-10-fluoro-1-oxo-3a,4-dihydro-1H,7H-imidazo[1',5':4,5][1,4]oxazino[3,2-f]indazol-2(3H)-yl)-2-(4-fluoro-3,5-dimethylphenyl)-4-methyl-4,5,6,7-tetrahydro-2H-pyrazolo[4,3-c]pyridine-5-carbonyl)-7-((R)-2,2-dimethyltetrahydro-2H-pyran-4-yl)indolizin-3-yl)cyclopropane-1-carbonitrile 26b-2 or

### 1-(2-((S)-3-((R)-7-cyclopropyl-10-fluoro-1-oxo-3a,4-dihydro-1H,7H-imidazo[1',5':4,5][1,4]oxazino[3,2-f]indazol-2(3H)-yl)-2-(4-fluoro-3,5-dimethylphenyl)-4-methyl-4,5,6,7-tetrahydro-2H-pyrazolo[4,3-c]pyridine-5-carbonyl)-7-((S)-2,2-dimethyltetrahydro-2H-pyran-4-yl)indolizin-3-yl)cyclopropane-1-carbonitrile 26b-1

Compound **13r** (71 mg, 0.13 mmol) and compound **26a-2** or **26a-1** (40 mg, 0.11 mmol, prepared in one step from the compound with a longer retention time among compounds **26c-2** and **26c-1)** were dissolved in *N,N*-dimethylformamide (3 mL). *O*-(7-azabenzotriazol-1-yl)-*N,N,N',N'*-tetramethyluronium hexafluorophosphate (89 mg, 0.24 mmol) and *N,N*-diisopropylethylamine (45 mg, 0.35 mmol) were added and stirred at room temperature for 16 hours. The reaction solution was filtered. The residue was purified by silica gel column chromatography with eluent system B to obtain the title compound **26b-2 or 26b-1** (100 mg, yield: 97.6%).

MS m/z (ESI): 866.8 [M+1].

### Step 3

### (R)-7-cyclopropyl-2-((S)-5-(7-((R)-2,2-dimethyltetrahydro-2H-pyran-4-yl)-3-(1-(5-oxo-4,5-dihydro-1,2,4-oxadiazol-3-yl)cyclopropyl)indolizine-2-carbonyl)-2-(4-fluoro-3,5-dimethylphenyl)-4-methyl-4,5,6,7-tetrahydro-2H-pyrazolo[4,3-c]pyridin-3-yl)-10-fluoro-2,3,3a,4-tetrahydro-1H,7H-imidazo[1',5':4,5][1,4]oxazino[3,2-f]indazol-1-one 26-2 or

### (R)-7-cyclopropyl-2-((S)-5-(7-((S)-2,2-dimethyltetrahydro-2H-pyran-4-yl)-3-(1-(5-oxo-4,5-dihydro-1,2,4-oxadiazol-3-yl)cyclopropyl)indolizine-2-carbonyl)-2-(4-fluoro-3,5-dimethylphenyl)-4-methyl-4,5,6,7-tetrahydro-2H-pyrazolo[4,3-c]pyridin-3-yl)-10-fluoro-2,3,3a,4-tetrahydro-1H,7H-imidazo[1',5':4,5][1,4]oxazino[3,2-f]indazol-1-one 26-1

Compound **26b-2** or **26b-1** (100 mg, 0.11 mmol, prepared in two steps from the compound corresponding to a longer retention time, among compounds **26c-2** and **26c-1),** hydroxylamine hydrochloride (80 mg, 1.15 mmol) were dissolved in dimethyl sulfoxide (10 mL). Sodium bicarbonate (97 mg, 1.15 mmol) was added and reacted under stirring at 60°C for 16 hours. The reaction solution was cooled to room temperature, quenched with water (10 mL), and extracted with ethyl acetate (30 mL × 3). The organic phase was dried over anhydrous sodium sulfate. After filtration, the filtrate was concentrated under reduced pressure. The residue was dissolved in dimethyl sulfoxide (10 mL). *N,N'*-carbonyldiimidazole (50 mg, 0.35 mmol) and 1,8-diazabicyclo[5,4,0]undec-7-ene (87 mg, 0.35 mmol) were added and reacted under stirring at room temperature for 16 hours. The reaction mixture was quenched with water (10 mL) and extracted with ethyl acetate (30 mL × 3). The organic phase was dried over anhydrous sodium sulfate. After filtration, the filtrate was concentrated under reduced pressure. The filtrate was purified by preparative high-performance liquid chromatography (instrument model: Gilson-GX-281 chromatographic column: SP-120-5-ODS-BIO, Prep 30 × 150 mm; 5 µm; C18 mobile phase: water (10 mM ammonium bicarbonate) and acetonitrile, with gradient ratio: acetonitrile 55%-80%, flow rate: 30 mL/min) to obtain the title compound **26-2** or **26-1** (23 mg, yield: 43%, prepared in three steps from the compound with a longer retention time among compounds **26c-2** and **26c-1).**

MS m/z (ESI): 925.8 [M+1].

¹H NMR (500 MHz, CDCl₃): δ 11.35-10.90 (m, 1H), 8.16-8.06 (m, 1H), 7.96 (s, 1H), 7.26 (s, 1H), 7.17 (d, 2H), 6.90-6.85 (m, 1H), 6.63-6.56 (m, 1H), 6.37-6.31 (m, 1H), 5.80 (s, 1H), 5.42-5.29 (m, 2H), 4.36-4.23 (m, 2H), 4.07-3.98 (m, 2H), 3.87-3.50 (m, 5H), 3.13-2.89 (m, 4H), 2.27-2.12 (m, 6H), 1.78-1.55 (m, 7H), 1.32-1.14 (m, 12H).

### Example 27

### 2-((4S)-5-(5-((S)-2,2-dimethyltetrahydro-2H-pyran-4-yl)-1-((1S,2S)-2-methyl-1-(5-oxo-4,5-dihydro-1,2,4-oxadiazol-3-yl)cyclopropyl)-1H-indole-2-carbonyl)-2-(4-fluoro-3,5-dimethylphenyl)-4-methyl-4,5,6,7-tetrahydro-2H-pyrazolo[4,3-c]pyridin-3-yl)-6-(4-fluoro-1-methyl-1H-indazol-5-yl)pyridine 1-oxide 27

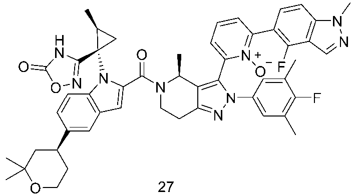

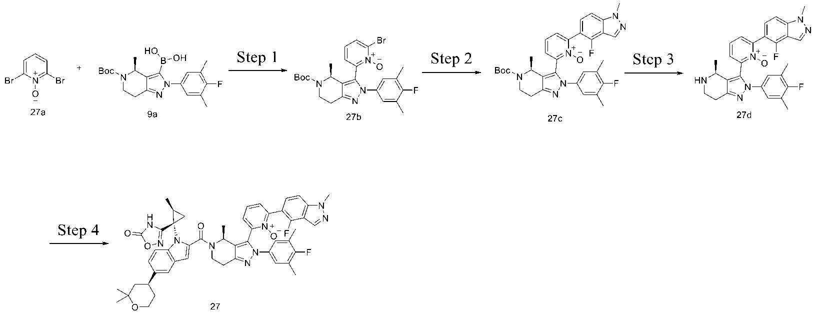

### Step 1

### (S)-2-bromo-6-(5-(tert-butoxycarbonyl)-2-(4-fluoro-3,5-dimethylphenyl)-4-methyl-4,5,6,7-tetrahydro-2H-pyrazolo[4,3-c]pyridin-3-yl)pyridine 1-oxide 27b

Compound **9a** (1.59 g, 3.94 mmol), 2,6-dibromopyridine 1-oxide**27a** (500 mg, 1.97 mmol, Adamas), anhydrous potassium carbonate (815 mg, 5.89 mmol), and [1,1'-bis(diphenylphosphino)ferrocene]palladium dichloride (140 mg, 0.191 mmol, Accela) were dissolved in dioxane (15 mL) and water (1.5 mL). After displacement with nitrogen, the mixture was reacted under stirring at 100°C for 2 hours. The reaction solution was cooled to room temperature, poured into 30 mL of water, and extracted with ethyl acetate (30 mL × 3). The organic phases were combined. The combined organic phase was dried over anhydrous sodium sulfate. After filtration, the filtrate was concentrated under reduced pressure. The residue was purified by silica gel column chromatography with eluent system A to obtain the crude title compound **27b** (150 mg, yield: 14.3%).

MS m/z (ESI): 531.2 [M+2].

### Step 2

### (S)-2-(5-(tert-butoxycarbonyl)-2-(4-fluoro-3,5-dimethylphenyl)-4-methyl-4,5,6,7-tetrahydro-2H-pyrazolo[4,3-c]pyridin-3-yl)-6-(4-fluoro-1-methyl-1H-indazol-5-yl)pyridine 1-oxide 27c

Compound **27b** (150 mg, 0.282 mmol), compound **15h** (108 mg, 0.48 mmol), anhydrous potassium carbonate (117 mg, 0.846 mmol), and [1,1'-bis(diphenylphosphino)ferrocene]palladium dichloride (21 mg, 0.028 mmol, Accela) were dissolved in dioxane (3 mL) and water (0.3 mL). After displacement with nitrogen, the reaction was stirred at 95°C for 1 hour. The reaction solution was cooled to room temperature, poured into 20 mL of water, and extracted with ethyl acetate (30 mL × 3). The organic phases were combined. The combined organic phase was dried over anhydrous sodium sulfate. After filtration, the filtrate was concentrated under reduced pressure. The residue was purified by silica gel column chromatography with eluent system A to obtain the title compound **27c** (70 mg, yield: 41.3%).

MS m/z (ESI): 601.5 [M+1].

### Step 3

### (S)-2-(4-fluoro-1-methyl-1H-indazol-5-yl)-6-(2-(4-fluoro-3,5-dimethylphenyl)-4-methyl-4,5,6,7-tetrahydro-2H-pyrazolo[4,3-c]pyridin-3-yl)pyridine 1-oxide 27d

Compound **27c** (35 mg, 0.058 mmol) was dissolved in a 4 M solution of hydrogen chloride in dioxane (2 mL). The reaction was stirred at room temperature for 1 hours. The reaction solution was concentrated under reduced pressure to obtain the crude title compound **27d** (30 mg). The product was directly used in the next reaction without purification.

MS m/z (ESI): 501.3 [M+1].

### Step 4

### 2-((4S)-5-(5-((S)-2,2-dimethyltetrahydro-2H-pyran-4-yl)-1-((1S,2S)-2-methyl-1-(5-oxo-4,5-dihydro-1,2,4-oxadiazol-3-yl)cyclopropyl)-1H-indole-2-carbonyl)-2-(4-fluoro-3,5-dimethylphenyl)-4-methyl-4, 5,6,7-tetrahydro-2H-pyrazolo[4,3-c]pyridin-3-yl)-6-(4-fluoro-1-methyl-1H-indazol-5-yl)pyridine 1-oxide 27

Compound **27d** (30 mg, 0.059 mmol) and compound **1w** (27 mg, 0.065 mmol) were dissolved in *N,N*-dimethylformamide (3 mL). *N,N*-diisopropylethylamine (24 mg, 0.185 mmol) was added. After the raw materials were fully dissolved, *O*-(7-azabenzotriazol-1-yl)-*N,N,N',N'*-tetramethyluronium hexafluorophosphate (34 mg, 0.089 mmol) was added and stirred at room temperature for 16 hours. The reaction solution was filtered. The filtrate was purified by preparative high-performance liquid chromatography (instrument model: SHIMADZU LC, chromatographic column: Boston Phlex, Prep 30 × 150 mm; 5 µm; C18, mobile phase: water (0.1% aqueous ammonia) and acetonitrile, with gradient ratio: acetonitrile 35%-55%, flow rate: 30 mL/min) to obtain the title compound **27** (10 mg, yield: 18.6%).

MS m/z (ESI): 894.5 [M+1].

¹H NMR (500 MHz, CDCl₃): δ 11.37 (s, 1H), 8.21-7.79 (m, 1H), 7.71-7.32 (m, 5H), 7.25-6.84 (m, 3H), 6.79-6.16 (m, 2H), 5.81-5.42 (m, 1H), 4.90-4.40 (m, 1H), 4.18-4.02 (m, 3H), 3.96-3.35 (m, 4H), 3.27-2.96 (m, 3H), 2.33-2.12 (m, 6H), 2.10-1.62 (m, 7H), 1.40-1.09 (m, 11H), 1.02-0.82 (m, 1H).

### Examples 28-1 and 28-2

### 3-((1S,2S)-1-(5-((S)-2,2-dimethyltetrahydro-2H-pyran-4-yl)-2-((S)-3-(((Z)-3-(4-fluoro-1-methyl-1H-indazol-5-yl)oxazolidin-2-ylidene)amino)-2-(4-fluoro-3,5-dimethylphenyl)-4-methyl-4,5,6,7-tetrahydro-2H-pyrazolo[4,3-c]pyridine-5-carbonyl)-1H-indol-1-yl)-2-methylcyclopropyl)-1,2,4-oxadiazol-5(4H)-one 28-1

### 3-((1S,2S)-1-(5-((S)-2,2-dimethyltetrahydro-2H-pyran-4-yl)-2-((S)-3-(((E)-3-(4-fluoro-1-methyl-1H-indazol-5-yl)oxazolidin-2-ylidene)amino)-2-(4-fluoro-3,5-dimethylphenyl)-4-methyl-4,5,6,7-tetrahydro-2H-pyrazolo[4,3-c]pyridine-5-carbonyl)-1H-indol-1-yl)-2-methylcyclopropyl)-1,2,4-oxadiazol-5(4H)-one 28-2

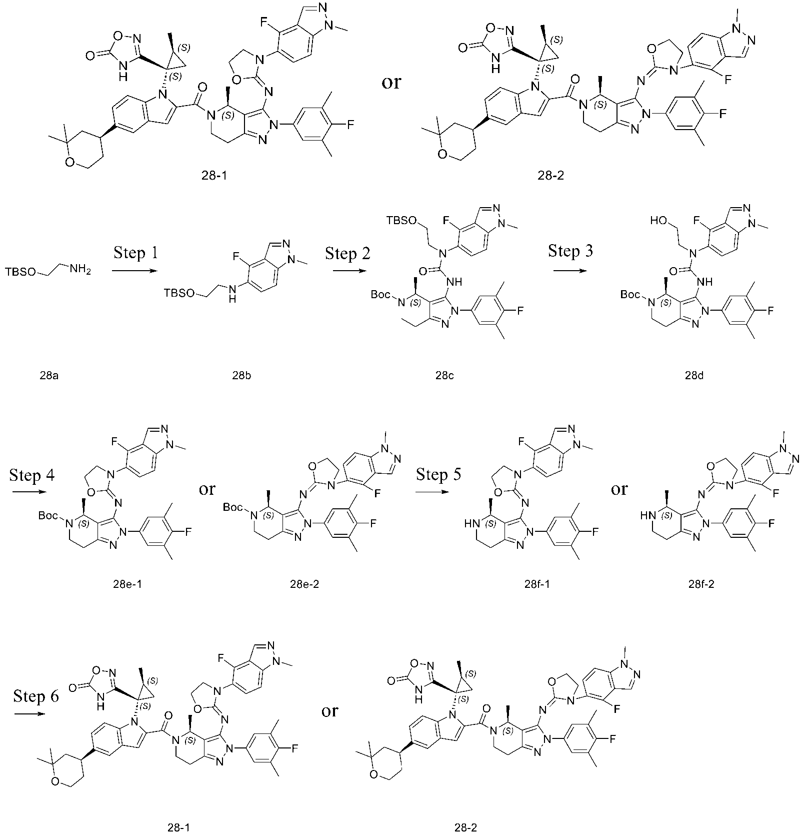

### Step 1

### (N-(2-((tert-butyldimethylsilyl)oxy)ethyl)-4-fluoro-1-methyl-1H-indazol-5-amine 28b

2-(Tert-butyldimethylsilyloxy)ethylamine **28a** (500 mg, 2.85 mmol, Shanghai Bidepharm) and 5-bromo-4-fluoro-1-methylindazole (700 mg, 3.05 mmol, prepared by a method disclosed for intermediate 1 on page 135 in the description of Patent Application WO 2022017338 A1) were dissolved in dioxane (10.0 mL). Cesium carbonate (2.3 g, 7.05 mmol) was added. Tris(dibenzylideneacetone)dipalladium (260 mg, 0.28 mmol, Adamas) and 4,5-bis(diphenylphosphino)-9,9-dimethylxanthene (170 mg, 0.29 mmol, Shaoyuan Technology (Shanghai) Co., Ltd.) were added. After displacement with nitrogen three times, the mixture was reacted at 110°C for 16 hours. The reaction solution was poured into 20 mL of water and extracted with ethyl acetate (30 mL × 2). The organic phases were combined, washed with a saturated sodium chloride solution, and dried over anhydrous sodium sulfate. After filtration, the filtrate was concentrated under reduced pressure. The residue was purified by silica gel column chromatography with eluent system C to obtain the title compound **28b** (430 mg, yield: 46.6%).

MS m/z (ESI): 324.1 [M+1].

### Step 2

### Tert-butyl (S)-3-(3-(2-((tert-butyldimethylsilyl)oxy)ethyl)-3-(4-fluoro-1-methyl-1H-indazol-5-yl)ureido)-2-(4-fluoro-3,5-dimethylphenyl)-4-methyl-2,4,6,7-tetrahydro-5H-pyrazolo[4,3-c]pyridine-5-carboxylate 28c

Compound **28b** (400 mg, 1.23 mmol) and compound **13j** (200 mg, 0.40 mmol) were dissolved in tetrahydrofuran (5.0 mL). *N,N*-diisopropylethylamine (160 mg, 1.24 mmol) was added and the mixture was reacted at room temperature for 16 hours. The reaction solution was poured into 30 mL of water and extracted with ethyl acetate (30 mL × 3). The organic phases were combined, washed with a saturated sodium chloride solution, and dried over anhydrous sodium sulfate. After filtration, the filtrate was concentrated under reduced pressure. The residue was purified by silica gel column chromatography with eluent system C to obtain the title compound **28c** (140 mg, yield: 47.8%).

MS m/z (ESI): 724.2 [M+1].

### Step 3

### Tert-butyl (S)-3-(3-(4-fluoro-1-methyl-1H-indazol-5-yl)-3-(2-hydroxyethyl)ureido)-2-(4-fluoro-3,5-dimethylphenyl)-4-methyl-2,4,6,7-tetrahydro-5H-pyrazolo[4,3-c]pyridine-5-carboxylate 28d

Compound **28c** (140 mg, 0.19 mmol) was dissolved in tetrahydrofuran (2.0 mL). A tetrabutylammonium fluoride/tetrahydrofuran solution (0.2 mL, 0.2 mmol, 1.0M) was added and the mixture was reacted at room temperature for 1 hour. The reaction solution was poured into 10 mL of water and extracted with ethyl acetate (10 mL × 2). The organic phases were combined, washed with a saturated sodium chloride solution, and dried over anhydrous sodium sulfate. After filtration, the filtrate was concentrated under reduced pressure. The residue was purified by silica gel column chromatography with eluent system C to obtain the title compound **28d** (50 mg, yield: 42.4%).

MS m/z (ESI): 610.3 [M+1].

### Step 4

### Tert-butyl (S,Z)-3-((3-(4-fluoro-1-methyl-1H-indazol-5-yl)oxazolidin-2-ylidene)amino)-2-(4-fluoro-3,5-dimethylphenyl)-4-methyl-2,4,6,7-tetrahydro-5H-pyrazolo[4,3-c]pyridine-5-carboxylate 28e-1 or

### tert-butyl (S,E)-3-((3-(4-fluoro-1-methyl-1H-indazol-5-yl)oxazolidin-2-ylidene)amino)-2-(4-fluoro-3,5-dimethylphenyl)-4-methyl-2,4,6,7-tetrahydro-5H-pyrazolo[4,3-c]pyridine-5-carboxylate 28e-2

Compound **28d** (50 mg, 0.082 mmol) was dissolved in dichloromethane (5.0 mL). Diethylaminosulfur trifluoride (15 mg, 0.093 mmol, Adamas) was added at - 78°C. After the addition was complete, the mixture was reacted at -78°C for 1 hour and then at room temperature for 1 hour. The reaction solution was poured into 50 mL of a saturated aqueous sodium bicarbonate solution and extracted with dichloromethane (30 mL × 3). The organic phases were combined, washed with a saturated sodium chloride solution, and dried over anhydrous sodium sulfate. After filtration, the filtrate was concentrated under reduced pressure. The residue was purified by silica gel column chromatography with eluent system A to obtain the title compound **28e-1** or **28e-2** (45 mg, yield: 92.7%).

MS m/z (ESI): 592.2 [M+1].

### Step 5

### (S,Z)-3-(4-fluoro-1-methyl-1H-indazol-5-yl)-N-(2-(4-fluoro-3,5-dimethylphenyl)-4-methyl-4,5,6,7-tetrahydro-2H-pyrazolo[4,3-c]pyridin-3-yl)oxazolidin-2-imine 28f-1 or

### (S,E)-3-(4-fluoro-1-methyl-1H-indazol-5-yl)-N-(2-(4-fluoro-3,5-dimethylphenyl)-4-methyl-4,5,6,7-tetrahydro-2H-pyrazolo[4,3-c]pyridin-3-yl)oxazolidin-2-imine 28f-2

Compound **28e-1** or **28e-2** (45 mg, 0.076 mmol) was dissolved in dichloromethane (3.0 mL). Zinc bromide (60 mg, 0.27 mmol) was added and stirred at room temperature overnight. The reaction solution was concentrated under reduced pressure. The residue was purified by silica gel column chromatography with eluent system A to obtain the title compound **28f-1** or **28f-2** as a crude compound (20 mg).

MS m/z (ESI): 492.2 [M+1].

### Step 6

### 3-((1S,2S)-1-(5-((S)-2,2-dimethyltetrahydro-2H-pyran-4-yl)-2-((S)-3-(((Z)-3-(4-fluoro-1-methyl-1H-indazol-5-yl)oxazolidin-2-ylidene)amino)-2-(4-fluoro-3,5-dimethylphenyl)-4-methyl-4,5,6,7-tetrahydro-2H-pyrazolo[4,3-c]pyridine-5-carbonyl)-1H-indol-1-yl)-2-methylcyclopropyl)-1,2,4-oxadiazol-5(4H)-one 28-1 or

### 3-((1S,2S)-1-(5-((S)-2,2-dimethyltetrahydro-2H-pyran-4-yl)-2-((S)-3-(((E)-3-(4-fluoro-1-methyl-1H-indazol-5-yl)oxazolidin-2-ylidene)amino)-2-(4-fluoro-3,5-dimethylphenyl)-4-methyl-4,5,6,7-tetrahydro-2H-pyrazolo[4,3-c]pyridine-5-carbonyl)-1H-indol-1-yl)-2-methylcyclopropyl)-1,2,4-oxadiazol-5(4H)-one 28-2

Compound **28f-1** or **28f-2** (110 mg, 0.041 mmol) and compound **1w** (20 mg, 0.048 mmol) were dissolved in *N,N*-dimethylformamide (2.0 mL). *N,N-*diisopropylethylamine (16 mg, 0.12 mmol) was added. After the raw materials were fully dissolved, *O*-(7-azabenzotriazol-1-yl)-*N,N,N',N'*-tetramethyluronium hexafluorophosphate (30 mg, 0.079 mmol) was added and stirred at room temperature for 3 hours. The reaction solution was poured into 10 mL of water and extracted with ethyl acetate (10 mL × 3). The organic phases were combined, washed with a saturated sodium chloride solution, and dried over anhydrous sodium sulfate. After filtration, the filtrate was concentrated under reduced pressure. The residue was purified by preparative high-performance liquid chromatography (instrument model: Waters-2545 chromatographic column: Welch Xtimate, Prep 30 × 250 mm; 5 µm; C18, mobile phase: water (10 mM ammonium bicarbonate) and acetonitrile, with gradient ratio: acetonitrile 40%-95%, flow rate: 30 mL/min) to obtain the title compound **28-1** or **28-2** (15 mg, yield: 41.6%).

MS m/z (ESI): 885.8 [M+1].

¹H NMR (500 MHz, CDCl₃): δ 11.92-11.24 (m, 1H), 8.12-8.04 (m, 1H), 7.61-7.53 (m, 2H), 7.44-7.41 (m, 1H), 7.30-7.13 (m, 4H), 6.71-6.70 (m, 1H), 5.75-5.71 (m, 1H), 4.87-4.79 (m, 1H), 4.67-4.62 (m, 1H), 4.38-4.35 (m, 1H), 4.24-4.19 (m, 1H), 4.13-4.05 (m, 3H), 3.92-3.85 (m, 2H), 3.76-3.73 (m, 1H), 3.51-3.45 (m, 1H), 3.10-3.01 (m, 2H), 2.91-2.85 (m, 1H), 2.25-2.18 (m, 6H), 1.93 (t, 1H), 1.81-1.46 (m, 7H), 1.37-1.07 (m, 11H).

### Example 29

### (R)-2-((S)-5-(5-((S)-2,2-dimethyltetrahydro-2H-pyran-4-yl)-1-((1S,2S)-2-methyl-1-(5-oxo-4,5-dihydro-1,2,4-oxadiazol-3-yl)cyclopropyl)-1H-indole-2-carbonyl)-2-(4-fluoro-3,5-dimethylphenyl)-4-methyl-4,5,6,7-tetrahydro-2H-pyrazolo[4,3-c]pyridin-3-yl)-10-fluoro-7-(2,2,2-trifluoroethyl)-2,3,3a,4-tetrahydro-1H,7H-imidazo[1',5':4,5][1,4]oxazino[3,2-f]indazol-1-one 29

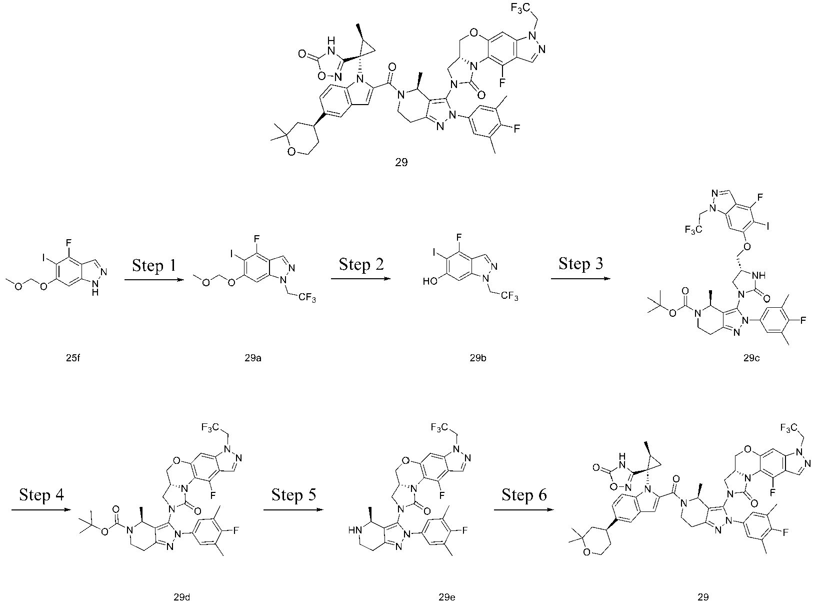

### Step 1

### 4-Fluoro-5-iodo-6-(methoxymethoxy)-1-(2,2,2-trifluoroethyl)-1H-indazole 29a

Compound **25f** (230 mg, 0.71 mmol) was dissolved in *N,N-*dimethylacetamide (20 mL). Sodium hydride (43 mg, 1.07 mmol, 60% dispersed in a mineral oil) was added under ice bath condition and stirred for 30 minutes. 2,2,2-Trifluoroethyl trifluoromethanesulfonate (250 mg, 1.08 mmol, Shanghai Bidepharm) was added. The reaction was stirred at room temperature for 3 hours. 30 mL of water was added. The mixture was extracted with ethyl acetate (30 mL × 3). The organic phases were combined, washed with a saturated aqueous sodium chloride solution, and dried over anhydrous sodium sulfate. After filtration, the filtrate was concentrated under reduced pressure. The residue was purified by silica gel column chromatography with eluent system C to obtain the title compound **29a** (180 mg, yield: 62%).

MS m/z (ESI): 404.8 [M+1].

### Step 2

### 4-Fluoro-5-iodo-1-(2,2,2-trifluoroethyl)-1H-indazol-6-ol 29b

Compound **29a** (180 mg, 0.45 mmol) was dissolved in dichloromethane (2 mL). A hydrogen chloride/dioxane solution (2 mL, 4.0 M) was dropwise added at 0°C and then stirred at room temperature for 12 hours. After concentration, 30 mL of water was added. The mixture was extracted with ethyl acetate (30 mL × 3). The organic phases were combined, washed with a saturated aqueous sodium chloride solution, and dried over anhydrous sodium sulfate. After filtration, the filtrate was concentrated under reduced pressure. The residue was purified by silica gel column chromatography with eluent system B to obtain the title compound **29b** (100 mg, yield: 62%).

MS m/z (ESI): 361.0 [M+1].

### Step 3

### Tert-butyl (S)-2-(4-fluoro-3,5-dimethylphenyl)-3-((R)-4-(((4-fluoro-5-iodo-1-(2,2,2-trifluoroethyl)-1H-indazol-6-yl)oxy)methyl)-2-oxoimidazolidin-1-yl)-4-methyl-2,4,6,7-tetrahydro-5H-pyrazolo[4,3-c]pyridine-5-carboxylate 29c

Compound **13o** (139 mg, 0.25 mmol) and compound **29b** (100 mg, 0.27 mmol) were dissolved in *N,N*-dimethylformamide (5.0 mL). Potassium carbonate (106 mg, 0.76 mmol) was added and reacted at 80°C for 3 hours. The reaction solution was poured into 20 mL of water and extracted with ethyl acetate (30 mL × 2). The organic phases were combined, washed with a saturated sodium chloride solution, and dried over anhydrous sodium sulfate. After filtration, the filtrate was concentrated under reduced pressure. The residue was purified by silica gel column chromatography with eluent system A to obtain the title compound **29c** (150 mg, yield: 72%).

MS m/z (ESI): 816.2 [M+1].

### Step 4

### Tert-butyl (S)-3-((R)-10-fluoro-1-oxo-7-(2,2,2-trifluoroethyl)-3a,4-dihydro-1H,7H-imidazo[1',5':4,5][1,4]oxazino[3,2-f]indazol-2(3H)-yl)-2-(4-fluoro-3,5-dimethylphenyl)-4-methyl-2,4,6,7-tetrahydro-5H-pyrazolo[4,3-c]pyridine-5-carboxylate 29d

Compound **29c** (150 mg, 0.18 mmol) and (1*S*,2*S*)-(+)-*N,N'*-dimethyl-1,2-cyclohexanediamine (13 mg, 0.09 mmol) were dissolved in *N*-methylpyrrolidone (5.0 mL). Potassium carbonate (77 mg, 0.55 mmol) and cuprous iodide (9 mg, 0.05 mmol) were added. After displacement with nitrogen three times, the mixture was heated to 125°C and reacted under stirring for 3 hours. The reaction solution was poured into 20 mL of water and extracted with ethyl acetate (30 mL × 2). The organic phases were combined, washed with a saturated sodium chloride solution, and dried over anhydrous sodium sulfate. After filtration, the filtrate was concentrated under reduced pressure. The residue was purified by silica gel column chromatography with eluent system C to obtain the title compound **29d** (100 mg, yield: 79%).

MS m/z (ESI): 688.4 [M+1].

### Step 5

### (R)-10-fluoro-2-((S)-2-(4-fluoro-3,5-dimethylphenyl)-4-methyl-4,5,6,7-tetrahydro-2H-pyrazolo[4,3-c]pyridin-3-yl)-7-(2,2,2-trifluoroethyl)-2,3,3a,4-tetrahydro-1H,7H-imidazo[1',5':4,5][1,4]oxazino[3,2-f]indazol-1-one 29e

Compound **29d** (100 mg, 0.15 mmol) was dissolved in methanol (2 mL). A 4 M solution of hydrogen chloride in 1,4-dioxane (2 mL) was added and stirred at room temperature for 2 hours. The reaction solution was concentrated under reduced pressure to obtain the crude title compound **29e** (70 mg). The product was directly used in the next reaction without purification.

MS m/z (ESI): 588.3 [M+1].

### Step 6

### (R)-2-((S)-5-(5-((S)-2,2-dimethyltetrahydro-2H-pyran-4-yl)-1-((1S,2S)-2-methyl-1-(5-oxo-4,5-dihydro-1,2,4-oxadiazol-3-yl)cyclopropyl)-1H-indole-2-carbonyl)-2-(4-fluoro-3,5-dimethylphenyl)-4-methyl-4,5,6,7-tetrahydro-2H-pyrazolo[4,3-c]pyridin-3-yl)-10-fluoro-7-(2,2,2-trifluoroethyl)-2,3,3a,4-tetrahydro-1H,7H-imidazo[1',5':4,5][1,4]oxazino[3,2-f]indazol-1-one 29

Compound **29e** (70 mg, 0.12 mmol) and compound **1w** (50 mg, 0.12 mmol) were dissolved in *N,N*-dimethylformamide (3 mL). *N,N*-diisopropylethylamine (47 mg, 0.36 mmol) was added. After the raw materials were fully dissolved, *O*-(7-azabenzotriazol-1-yl)-*N,N,N',N'*-tetramethyluronium hexafluorophosphate(46 mg, 0.12 mmol) was added and stirred at room temperature for 16 hours. The reaction solution was poured into 10 mL of water and extracted with ethyl acetate (10 mL × 3). The organic phases were combined, washed with a saturated sodium chloride solution, and dried over anhydrous sodium sulfate. After filtration, the filtrate was concentrated under reduced pressure. The residue was purified by preparative high-performance liquid chromatography (instrument model: Waters-2545 chromatographic column: Welch Xtimate, Prep 30 × 250 mm; 5 µm; C18, mobile phase: water (10 mM ammonium bicarbonate) and acetonitrile, with gradient ratio: acetonitrile 40%-95%, flow rate: 30 mL/min) to obtain the title compound **29** (50 mg, yield: 43%).

MS m/z (ESI): 981.6 [M+1].

¹H NMR (500 MHz, CDCl₃): δ 7.98 (s, 1H), 7.61 (d, 1H), 7.55-7.48 (m, 2H), 7.21-7.15 (m, 2H), 6.93 (s, 1H),6.71(s, 1H), 5.81-5.76 (m, 1H), 4.48-4.42 (m, 1H), 4.29-4.20 (m, 1H), 4.11-3.95 (m, 3H), 3.92-3.76 (m, 3H), 3.71-3.60 (m, 2H), 3.54-3.48 (m, 1H), 3.18-2.95 (m, 4H), 2.30-2.26 (m, 5H), 1.94 (t, 1H), 1.83-1.55 (m, 6H), 1.40-1.22 (m, 12H), 1.06 (d, 1H).

### Example 30

### (R)-2-((S)-5-(5-((S)-2,2-dimethyltetrahydro-2H-pyran-4-yl)-1-((1S,2S)-2-methyl-1-(5-oxo-4,5-dihydro-1,2,4-oxadiazol-3-yl)cyclopropyl)-1H-indole-2-carbonyl)-2-(4-fluoro-3,5-dimethylphenyl)-4-methyl-4,5,6,7-tetrahydro-2H-pyrazolo[4,3-c]pyridin-3-yl)-10-fluoro-7-(2-methoxyethyl)-2,3,3a,4-tetrahydro-1H,7H-imidazo[1',5':4,5][1,4]oxazino[3,2-f]indazol-1-one 30

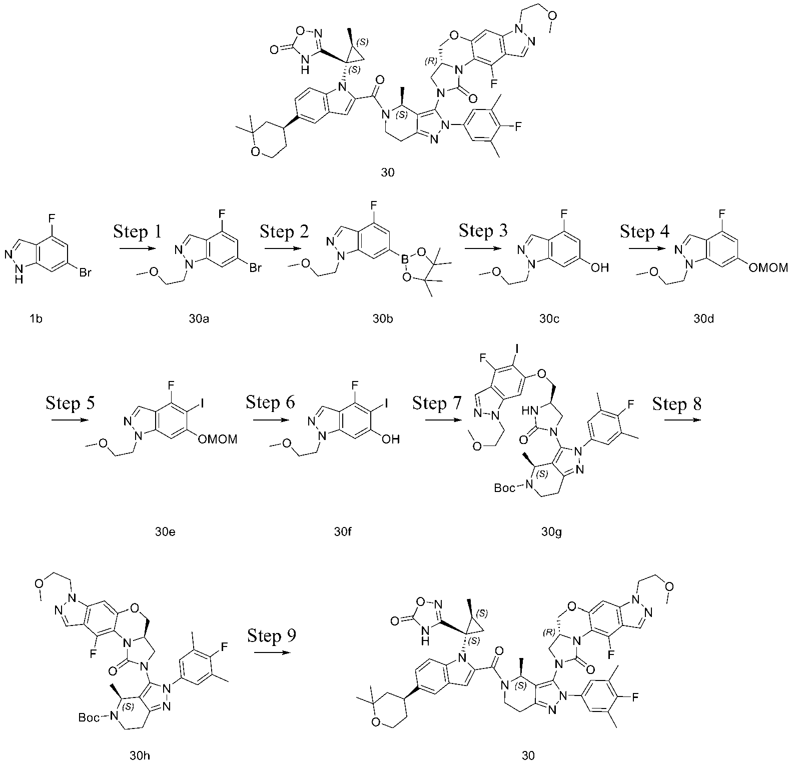

### Step 1

### 6-Bromo-4-fluoro-1-(2-methoxyethyl)-1H-indazole 30a

Compound **1b** (2.0 g, 9.3 mmol) was dissolved in *N,N-*dimethylformamide (60 mL). Sodium hydride (560 mg, 14.0 mmol, 60% dispersed in a mineral oil) was added in portions at 0°C and stirred for 30 minutes. 2-Bromoethyl methyl ether (1.55 g, 11.2 mmol) was dropwise added. After the addition was complete, the mixture was reacted under stirring at room temperature for 3 hours. The reaction solution was quenched with a saturated aqueous ammonium chloride solution (100 mL), extracted with ethyl acetate (50 mL × 2), and washed with a saturated sodium chloride solution. The organic phase was dried over anhydrous sodium sulfate. After filtration, the filtrate was concentrated under reduced pressure. The residue was purified by silica gel column chromatography with eluent system C to obtain the title compound **30a** (1.6 g, yield: 62.9%).

MS m/z (ESI): 273.1 [M+1].

### Step 2

### 4-Fluoro-1-(2-methoxyethyl)-6-(4,4,5,5-tetramethyl-1,3,2-dioxaborolan-2-yl)-1H-indazole 30b

Compound **30a** (1.6 g, 5.85 mmol), potassium acetate (1.43 g, 14.6 mmol), bis(pinacolato)diboron (2.23 g, 8.78 mmol), and [1,1'-bis(diphenylphosphino)ferrocene]palladium dichloride (857 mg, 1.17 mmol) were dissolved in dioxane (50 mL). After displacement with nitrogen three times, the mixture was reacted at 85°C for 16 hours. Water (100 mL) was added. The mixture was extracted with ethyl acetate (100 mL × 3). The organic phases were combined and washed with a saturated sodium chloride solution. The organic phase was dried over anhydrous sodium sulfate. After filtration, the filtrate was concentrated under reduced pressure. The residue was purified by silica gel column chromatography with eluent system C to obtain the title compound **30b** (1.6 g, yield: 85.3%).

MS m/z (ESI): 321.1 [M+1].

### Step 3

### 4-Fluoro-1-(2-methoxyethyl)-1H-indazol-6-ol 30c

Compound **30b** (1.6 g, 4.99 mmol) was dissolved in tetrahydrofuran (40 mL). A solution of sodium perborate tetrahydrate (2.1 g, 12.8 mmol) in water (20 mL) was dropwise added at 0°C. After the addition was complete, the mixture was reacted at room temperature for 16 hours. The reaction solution was poured into 50 mL of water, extracted with ethyl acetate (50 mL × 3), and dried over anhydrous sodium sulfate. After filtration, the filtrate was concentrated under reduced pressure. The residue was purified by silica gel column chromatography with eluent system C to obtain the title compound **30c** (1.0 g, yield: 95.2%).

MS m/z (ESI): 211.1 [M+1].

### Step 4

### 4-Fluoro-1-(2-methoxyethyl)-6-(methoxymethoxy)-1H-indazole 30d

Compound **30c** (1.5 g, 7.13 mmol) and *N,N*-diisopropylethylamine (1.84 g, 14.2 mmol) were dissolved in dichloromethane (60 mL). Bromomethyl methyl ether (1.33 g, 10.6 mmol) was dropwise added at 0°C. After the addition was complete, the mixture was stirred at room temperature for 2 hours, quenched with water (50 mL), and extracted with dichloromethane (50 mL × 3). The organic phases were combined and washed with a saturated sodium chloride solution. The organic phase was dried over anhydrous sodium sulfate. After filtration, the filtrate was concentrated under reduced pressure. The residue was purified by silica gel column chromatography with eluent system C to obtain the title compound **30d** (1.0 g, yield: 55.1%).

MS m/z (ESI): 255.2 [M+1].

### Step 5

### 4-Fluoro-5-iodo-1-(2-methoxyethyl)-6-(methoxymethoxy)-1H-indazole 30e

Compound **30d** (500 mg, 1.96 mmol) was dissolved in tetrahydrofuran (30 mL). Lithium diisopropylamide (1.5 mL, 3.0 mmol, 2.0M, Adamas) was dropwise added in a nitrogen atmosphere at -78°C. After the addition was complete, stirring was continued at -78°C for 1 hour. A solution of an iodine (750 mg, 2.95 mmol)/tetrahydrofuran solution (5.0 mL) was then dropwise added. After the addition was complete, stirring was continued at -78°C for 1 hour, and the mixture was slowly heated to room temperature. The reaction was quenched with water (5 mL) and extracted with ethyl acetate (30 mL × 3). The organic phases were combined and washed with a saturated sodium chloride solution. The organic phase was dried over anhydrous sodium sulfate. After filtration, the filtrate was concentrated under reduced pressure. The residue was purified by silica gel column chromatography with eluent system **C** to obtain the title compound **30e** (200 mg, yield: 26.7%).

MS m/z (ESI): 381.1 [M+1].

### Step 6

### 4-Fluoro-5-iodo-1-(2-methoxyethyl)-1H-indazol-6-ol 30f

Compound **30e** (180 mg, 0.47 mmol) was dissolved in dichloromethane (5.0 mL). A hydrogen chloride/dioxane solution (3.0 mL, 4.0M) was dropwise added at 0°C. After the addition was complete, the mixture was stirred at room temperature for 1 hour. The mixture was concentrated to obtain the crude title compound **30f** (160 mg).

MS m/z (ESI): 335.0 [M-1].

### Step 7

Tert-butyl (*S*)-2-(4-fluoro-3,5-dimethylphenyl)-3-((*R*)-4-(((4-fluoro-5-iodo-1-(2-methoxyethyl)-1*H*-indazol-6-yl)oxy)methyl)-2-oxoimidazolidin-1-yl)-4-methyl-2,4,6,7-tetrahydro-5*H*-pyrazolo[4,3-*c*]pyridine-5-carboxylate **30g** Compound **13o** (290 mg, 0.53 mmol) and compound **30f** (160 mg, 0.48 mmol) were dissolved in *N,N*-dimethylformamide (3.0 mL). Potassium carbonate (200 mg, 1.45 mmol) was added and reacted at 80°C for 5 hours. The reaction solution was poured into 20 mL of water and extracted with ethyl acetate (30 mL × 2). The organic phases were combined, washed with a saturated sodium chloride solution, and dried over anhydrous sodium sulfate. After filtration, the filtrate was concentrated under reduced pressure. The residue was purified by silica gel column chromatography with eluent system A to obtain the title compound **30g** (250 mg, yield: 66.3%).

MS m/z (ESI): 792.2 [M+1].

### Step 8

### Tert-butyl (S)-2-(4-fluoro-3,5-dimethylphenyl)-3-((R)-10-fluoro-7-(2-methoxyethyl)-1-oxo-3a,4-dihydro-1H,7H-imidazo[1',5':4,5][1,4]oxazino[3,2-f]indazol-2(3H)-yl)-4-methyl-2,4,6,7-tetrahydro-5H-pyrazolo[4,3-c]pyridine-5-carboxylate 30h

Compound **30g** (250 mg, 0.31 mmol) and (1*S*,2*S*)-(+)-*N,N*'-dimethyl-1,2-cyclohexanediamine (25 mg, 0.18 mmol) were dissolved in *N,N-*dimethylformamide (15.0 mL). Potassium carbonate (130 mg, 0.94 mmol) and cuprous iodide (20 mg, 0.11 mmol) were added. After displacement with nitrogen three times, the mixture was reacted at 125°C for 3 hours. The reaction solution was poured into 20 mL of water and extracted with ethyl acetate (30 mL × 2). The organic phases were combined, washed with a saturated sodium chloride solution, and dried over anhydrous sodium sulfate. After filtration, the filtrate was concentrated under reduced pressure. The residue was purified by silica gel column chromatography with eluent system C to obtain the title compound **30h** (170 mg, yield: 81.1%).

MS m/z (ESI): 664.3 [M+1].

### Step 9

### (R)-2-((S)-5-(5-((S)-2,2-dimethyltetrahydro-2H-pyran-4-yl)-1-((1S,2S)-2-methyl-1-(5-oxo-4,5-dihydro-1,2,4-oxadiazol-3-yl)cyclopropyl)-1H-indole-2-carbonyl)-2-(4-fluoro-3,5-dimethylphenyl)-4-methyl-4,5,6,7-tetrahydro-2H-pyrazolo[4,3-c]pyridin-3-yl)-10-fluoro-7-(2-methoxyethyl)-2,3,3a,4-tetrahydro-1H,7H-imidazo[1',5':4,5][1,4]oxazino[3,2-f]indazol-1-one 30

Compound **30h** (130 mg, 0.26 mmol) was dissolved in methanol (2 mL). A 4M solution of hydrogen chloride in 1,4-dioxane (2 mL) was added and stirred at room temperature for 2 hours. The reaction solution was concentrated under reduced pressure to obtain a crude product.

The above-mentioned crude product (60 mg) and compound **1w** (50 mg, 0.12 mmol) were dissolved in *N,N*-dimethylformamide (3 mL). *N,N-*diisopropylethylamine (60 mg, 0.46 mmol) was added. After the raw materials were fully dissolved, *O*-(7-azabenzotriazol-1-yl)-*N,N,N',N'*-tetramethyluronium hexafluorophosphate (80 mg, 0.21 mmol) was added and stirred at room temperature for 3 hours. The reaction solution was poured into 10 mL of water and extracted with ethyl acetate (10 mL × 3). The organic phases were combined, washed with a saturated sodium chloride solution, and dried over anhydrous sodium sulfate. After filtration, the filtrate was concentrated under reduced pressure and purified by preparative high-performance liquid chromatography (instrument model: Waters-2545 chromatographic column: Welch Xtimate, Prep 30 × 250 mm; 5 µm; C18, mobile phase: water (10 mM ammonium bicarbonate) and acetonitrile, with gradient ratio: acetonitrile 40%-95%, flow rate: 30 mL/min) to obtain the title compound **30** (60 mg, yield: 62.6%).

MS m/z (ESI): 957.6 [M+1].

¹H NMR (500 MHz, CDCl₃): δ 8.06-8.02 (m, 1H), 7.61 (d, 1H), 7.55-7.51 (m, 1H), 7.29-7.27 (m, 1H), 7.19-7.17 (m, 2H), 6.82-6.71 (m, 2H), 5.82-5.78 (m, 1H), 4.48-4.40 (m, 3H), 4.27-4.22 (m, 1H), 4.07-3.99 (m, 2H), 3.91-3.80 (m, 4H), 3.66-3.60 (m, 2H), 3.33-3.28 (m, 3H), 3.15-3.00 (m, 3H), 2.35-2.30 (m, 6H), 1.94-1.55 (m, 9H), 1.36 -1.25 (m, 10H), 1.07 (d, 1H).

### Example 31-1 or 31-2

### (R)-7-cyclopropyl-2-((S)-5-((R)-2',2'-dimethyl-7-((1S,2S)-2-methyl-1-(5-oxo-4,5-dihydro-1,2,4-oxadiazol-3-yl)cyclopropyl)-2',3',5',6'-tetrahydro-2H,7H-spiro[furo[3,2-f]indole-3,4'-pyran]-6-carbonyl)-2-(4-fluoro-3,5-dimethylphenyl)-4-methyl-4,5,6,7-tetrahydro-2H-pyrazolo[4,3-c]pyridin-3-yl)-10-fluoro-2,3,3a,4-tetrahydro-1H,7H-imidazo[1',5':4,5][1,4]oxazino[3,2-f]indazol-1-one 31-1 or

### (R)-7-cyclopropyl-2-((S)-5-((S)-2',2'-dimethyl-7-((1S,2S)-2-methyl-1-(5-oxo-4,5-dihydro-1,2,4-oxadiazol-3-yl)cyclopropyl)-2',3',5',6'-tetrahydro-2H,7H-spiro[furo[3,2-f]indole-3,4'-pyran]-6-carbonyl)-2-(4-fluoro-3,5-dimethylphenyl)-4-methyl-4,5,6,7-tetrahydro-2H-pyrazolo[4,3-c]pyridin-3-yl)-10-fluoro-2,3,3a,4-tetrahydro-1H,7H-imidazo[1',5':4,5][1,4]oxazino[3,2-f]indazol-1-one 31-2

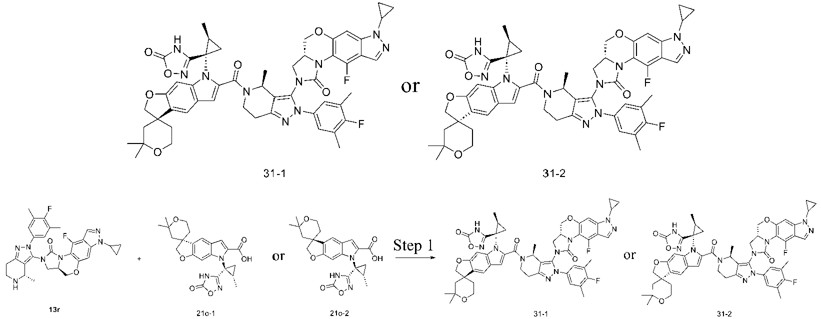

Compound **21o-1 or 21o-2** (25 mg, 0.057 mmol, prepared in six steps from the compound with a shorter retention time, among compounds **21i-1** and **21i-2)** and compound **13r** (34 mg, 0.06 mmol) were dissolved in N,N-dimethylformamide (3 mL). *N,N*-diisopropylethylamine (22 mg, 0.17 mmol) was added. After the raw materials were fully dissolved, *O*-(7-azabenzotriazol-1-yl)-*N,N,N',N'-*tetramethyluronium hexafluorophosphate (32 mg, 0.084 mmol) was added and stirred at room temperature for 16 hours. The reaction solution was filtered. The filtrate was concentrated under reduced pressure. The residue was purified by preparative high-performance liquid chromatography (instrument model: Wasters 2545, chromatographic column: Sharpsil-T, Prep 30 × 150 mm; 5 µm; C18, mobile phase: water (10 mM ammonium bicarbonate) and acetonitrile, with gradient ratio: acetonitrile 40%-60%, flow rate: 30 mL/min) to obtain the title compound **31-1** or **31-2** (15 mg, yield: 27.2%, prepared in seven steps from the compound with a shorter retention time, among compounds **21i-1** and **21i-2).**

MS m/z (ESI): 967.7 [M+1].

¹H NMR (500 MHz, CDCl₃): δ 8.01-7.92 (m, 1H), 7.52-7.29 (m, 2H), 7.22-7.12 (m, 2H), 7.07-6.95 (m, 1H), 6.94-6.85 (m, 1H), 6.74-6.64 (m, 1H), 5.82-5.71 (m, 1H), 4.83-4.67 (m, 1H), 4.55-4.18 (m, 3H), 4.12-3.91 (m, 2H), 3.89-3.42 (m, 6H), 3.17-2.93 (m, 3H), 2.30 (s, 6H), 2.17-1.85(m, 3H), 1.63-1.41 (m, 3H), 1.40-0.83(m, 16H).

### Example 32

### (R)-7-cyclopropyl-2-((S)-5-(5-((S)-2,2-dimethyltetrahydro-2H-pyran-4-yl)-1-((1S,2S)-2-methyl-1-(5-oxo-4,5-dihydro-1,2,4-oxadiazol-3-yl)cyclopropyl)-1H-indole-2-carbonyl)-2-(4-fluoro-3,5-dimethylphenyl)-4-methyl-4,5,6,7-tetrahydro-2H-pyrazolo[4,3-c]pyridin-3-yl)-6-fluoro-2,3,3a,4-tetrahydro-1H,7H-imidazo[1',5':4,5][1,4]oxazino[3,2-f]indazol-1-one 32

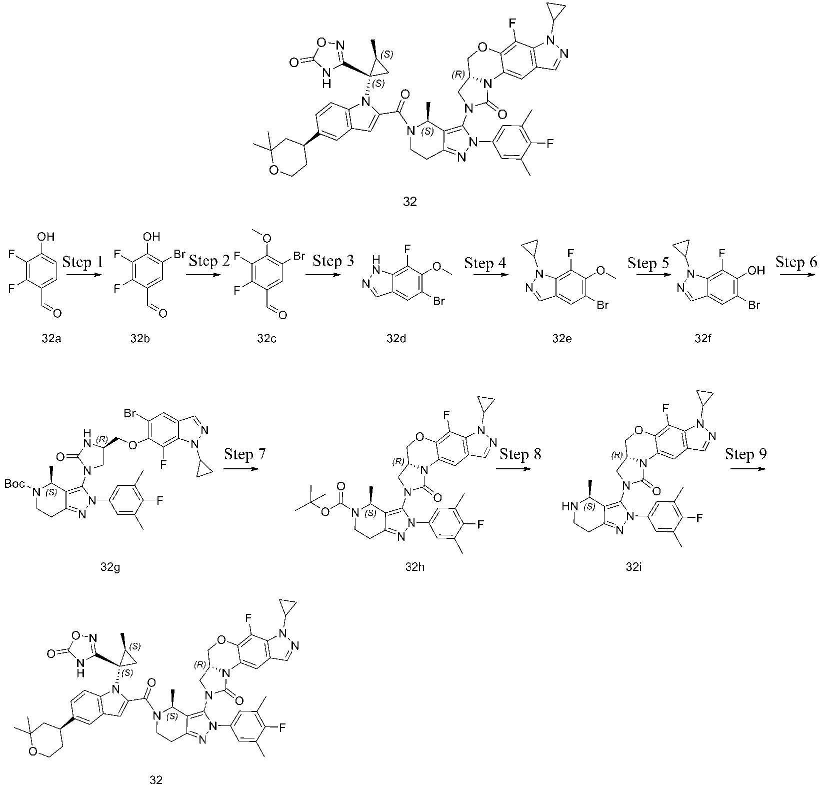

### Step 1

### 5-bromo-2,3-difluoro-4-hydroxybenzaldehyde 32b

2,3-Difluoro-4-hydroxybenzaldehyde **32a** (4.6 g, 20.09 mmol) was dissolved in anhydrous dichloromethane (20 mL). Diisopropylamine (294.42 mg, 2.90 mmol) was added at 0°C, followed by the slow dropwise addition of a solution of *N-*bromosuccinimide (5.4 g, 30.56 mmol) in dichloromethane (130 mL). The reaction was continued at 0°C for 1 hour. The reaction solution was quenched with a saturated aqueous ammonium chloride solution (100 mL), extracted with ethyl acetate (50 mL × 2), and washed with a saturated sodium chloride solution. The organic phase was dried over anhydrous sodium sulfate. After filtration, the filtrate was concentrated under reduced pressure. The residue was purified by silica gel column chromatography with eluent system B to obtain the title compound **32b** (6.0 g, yield: 87.0%).

MS m/z (ESI): 234.8 [M-1].

### Step 2

### 5-Bromo-2,3-difluoro-4-methoxybenzaldehyde 32c

Compound **32b** (6.0 g, 25.31 mmol), cesium carbonate (12.38 g, 37.99 mmol), and iodomethane (7.2 g, 50.65 mmol, Shaoyuan Technology (Shanghai) Co., Ltd.) were dissolved in *N,N*-dimethylformamide (60 mL) and the mixture was reacted at room temperature for 16 hours. Water (100 mL) was added. The mixture was extracted with ethyl acetate (100 mL × 3). The organic phases were combined and washed with a saturated sodium chloride solution. The organic phase was dried over anhydrous sodium sulfate. After filtration, the filtrate was concentrated under reduced pressure. The residue was purified by silica gel column chromatography with eluent system B to obtain the title compound **32c** (3.5 g, yield: 55.07%).

### Step 3

### 5-Bromo-7-fluoro-6-methoxy-1H-indazole 32d

Compound **32c** (124 mg, 0.49 mmol) and hydrazine hydrate (247 mg, 4.93 mmol, 85% wt aqueous solution) were dissolved in N,Ndimethylacetamide (10 mL) and the mixture was reacted at room temperature for 2 hours. The reaction was continued at 110°C for 16 hours. The reaction solution was poured into 50 mL of water, extracted with ethyl acetate (30 mL × 3), washed once with a saturated aqueous sodium chloride solution, and dried over anhydrous sodium sulfate. After filtration, the filtrate was concentrated under reduced pressure. The residue was purified by silica gel column chromatography with eluent system B to obtain the title compound **32d** (60 mg, yield: 49.5%).

MS m/z (ESI): 245.0 [M+1].

### Step 4

5-Bromo-1-cyclopropyl-7-fluoro-6-methoxy-1*H*-indazole **32e** Compound **32d** (120 mg, 0.49 mmol), cyclopropylboronic acid (84 mg, 0.98 mmol, Shaoyuan Technology (Shanghai) Co., Ltd.), anhydrous copper acetate (178 mg, 0.98 mmol, Adamas), 2,2'-bipyridine (153 mg, 0.98 mmol, Shaoyuan Technology (Shanghai) Co., Ltd.), and cesium carbonate (156 mg, 1.47 mmol) were dissolved in 1,2-dichloroethane (30 mL) and the mixture was reacted at room temperature for 16 hours. The mixture was quenched with water (50 mL) and extracted with dichloromethane (50 mL × 3). The organic phases were combined and washed with a saturated sodium chloride solution. The organic phase was dried over anhydrous sodium sulfate. After filtration, the filtrate was concentrated under reduced pressure. The residue was purified by silica gel column chromatography with eluent system B to obtain the title compound **32e** (120 mg, yield: 85.9%).

MS m/z (ESI): 284.9 [M+1].

### Step 5

### 5-Bromo-1-cyclopropyl-7-fluoro-1H-indazol-6-ol 32f

Compound **32e** (100 mg, 0.35 mmol) was dissolved in boron tribromide (10 mL, a 1M dichloromethane solution) and stirred at room temperature for 16 hours. The mixture was quenched with ice water, adjusted to pH = 8-9 with an excess of a saturated sodium bicarbonate solution, and extracted with dichloromethane (3 × 30 mL). The organic phases were combined and washed with a saturated sodium chloride solution. The organic phase was dried over anhydrous sodium sulfate. After filtration, the filtrate was concentrated under reduced pressure. The residue was purified by silica gel column chromatography with eluent system B to obtain the title compound **32f** (80 mg, yield: 84.1%).

MS m/z (ESI): 271.0 [M+1].

### Step 6

### Tert-butyl (S)-3-((R)-4-(((5-bromo-1-cyclopropyl-7-fluoro-1H-indazol-6-yl)oxy)methyl)-2-oxoimidazolidin-1-yl)-2-(4-fluoro-3,5-dimethylphenyl)-4-methyl-2,4,6,7-tetrahydro-5H-pyrazolo[4,3-c]pyridine-5-carboxylate 32g

Compound **13o** (244 mg, 0.44 mmol) and compound **32f** (100 mg, 0.37 mmol) were dissolved in *N,N-*dimethylformamide (10.0 mL). Potassium carbonate (157 mg, 1.11 mmol) was added and reacted at 80°C for 5 hours. The reaction solution was poured into 50 mL of water and extracted with ethyl acetate (30 mL × 2). The organic phases were combined, washed with a saturated sodium chloride solution, and dried over anhydrous sodium sulfate. After filtration, the filtrate was concentrated under reduced pressure. The residue was purified by silica gel column chromatography with eluent system A to obtain the title compound **32g** (260 mg, yield: 97.0%).

MS m/z (ESI): 726.4 [M+1].

### Step 7

### Tert-butyl (S)-3-((R)-7-cyclopropyl-6-fluoro-1-oxo-3a,4-dihydro-1H,7H-imidazo[1',5':4,5][1,4]oxazino[3,2-f]indazol-2(3H)-yl)-2-(4-fluoro-3,5-dimethylphenyl)-4-methyl-2,4,6,7-tetrahydro-5H-pyrazolo[4,3-c]pyridine-5-carboxylate 32h

Compound **32g** (250 mg, 0.34 mmol) and (1*R*,2*R*)-(-)-*N,N'*-dimethyl-1,2-cyclohexanediamine (49 mg, 0.34 mmol) were dissolved in *N,N-*dimethylformamide (10.0 mL). Potassium carbonate (142 mg, 1.03 mmol) and cuprous iodide (65 mg, 0.34 mmol) were added. After displacement with nitrogen three times, the mixture was reacted at 125°C for 2 hours. The reaction solution was poured into 20 mL of water and extracted with ethyl acetate (30 mL × 2). The organic phases were combined, washed with a saturated sodium chloride solution, and dried over anhydrous sodium sulfate. After filtration, the filtrate was concentrated under reduced pressure. The residue was purified by silica gel column chromatography with eluent system C to obtain the crude title compound **32h** (230 mg).

MS m/z (ESI): 646.5 [M+1].

### Step 8

### (R)-7-cyclopropyl-6-fluoro-2-((S)-2-(4-fluoro-3,5-dimethylphenyl)-4-methyl-4,5,6,7-tetrahydro-2H-pyrazolo[4,3-c]pyridin-3-yl)-2,3,3a,4-tetrahydro-1H,7H-imidazo[1',5':4,5][1,4]oxazino[3,2-f]indazol-1-one 32i

Compound **32h** (50 mg, 0.07 mmol) was dissolved in ethyl acetate (2 mL). A 4 M solution of hydrogen chloride in 1,4-dioxane (2 mL) was added and stirred at room temperature for 2 hours. The reaction solution was concentrated under reduced pressure to obtain the crude title compound **32i** (40 mg). The product was directly used in the next reaction without purification.

MS m/z (ESI): 546.4 [M+1].

### Step 9

### (R)-7-cyclopropyl-2-((S)-5-(5-((S)-2,2-dimethyltetrahydro-2H-pyran-4-yl)-1-((1S,2S)-2-methyl-1-(5-oxo-4,5-dihydro-1,2,4-oxadiazol-3-yl)cyclopropyl)-1H-indole-2-carbonyl)-2-(4-fluoro-3,5-dimethylphenyl)-4-methyl-4,5,6,7-tetrahydro-2H-pyrazolo[4,3-c]pyridin-3-yl)-6-fluoro-2,3,3a,4-tetrahydro-1H,7H-imidazo[1',5':4,5][1,4]oxazino[3,2-f]indazol-1-one 32

Crude compound **32i** (67 mg, 0.12 mmol) and compound **1w** (60.6 mg, 147.38 mmol) were dissolved in *N,N*-dimethylformamide (3 mL). *N,N-*diisopropylethylamine (79 mg, 0.61 mmol) was added. After the raw materials were fully dissolved, *O*-(7-azabenzotriazol-1-yl)-*N,N,N',N'*-tetramethyluronium hexafluorophosphate (57 mg, 0.15 mmol) was added and stirred at room temperature for 3 hours. The reaction solution was poured into 10 mL of water and extracted with ethyl acetate (10 mL × 3). The organic phases were combined, washed with a saturated sodium chloride solution, and dried over anhydrous sodium sulfate. After filtration, the filtrate was concentrated under reduced pressure. The residue was purified by preparative high-performance liquid chromatography (instrument model: Waters-2545, chromatographic column: Sharpsil-T, Prep 30 × 150 mm; 5 µm; C18, mobile phase: water (10 mM ammonium bicarbonate) and acetonitrile, with gradient ratio: acetonitrile 50%-75%, flow rate: 30 mL/min) to obtain the title compound **32** (60 mg, yield: 52.0%).

MS m/z (ESI): 937.5 [M-1].

¹H NMR (500 MHz, CDCl₃): δ 11.32-11.26 (m, 1H), 8.14-7.72 (m, 2H), 7.59-7.43 (m, 2H), 7.17-7.12 (m, 2H), 6.69-6.66 (m, 1H), 5.80-5.76 (m, 1H), 5.34-4.83 (m, 2H), 4.49-4.39 (m, 2H), 4.26-4.04 (m, 1H), 3.89-3.76 (m, 3H), 3.67-3.57 (m, 2H), 3.51-3.37 (m, 1H), 3.15-2.96 (m, 4H), 2.25-2.20 (m, 6H), 2.02-1.72 (m, 3H), 1.67-1.44 (m, 8H), 1.34-0.87 (m, 11H).

### Example 33

### (R)-2-((S)-5-(5-((S)-2,2-dimethyltetrahydro-2H-pyran-4-yl)-1-((1S,2S)-2-methyl-1-(5-oxo-4,5-dihydro-1,2,4-oxadiazol-3-yl)cyclopropyl)-1H-indole-2-carbonyl)-2-(4-fluoro-3,5-dimethylphenyl)-4-methyl-4,5,6,7-tetrahydro-2H-pyrazolo[4,3-c]pyridin-3-yl)-8-fluoro-7-(isopropylamino)-2,3,3a,4-tetrahydro-1H-benzo[b]imidazo[1,5-d][1,4]oxazin-1-one 33

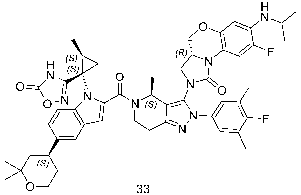

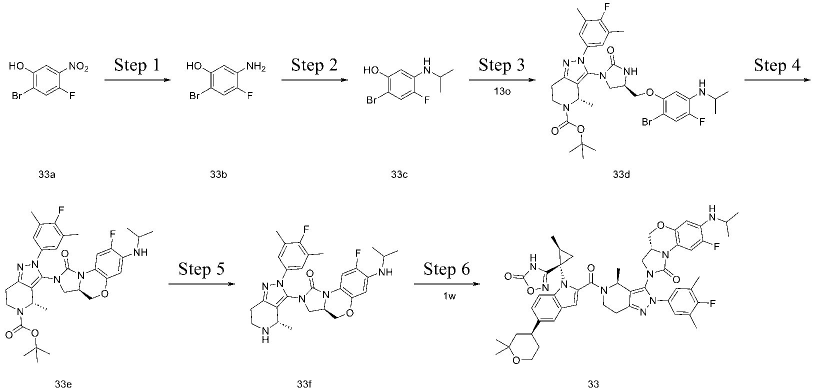

### Step 1

### 5-Amino-2-bromo-4-fluorophenol 33b

2-Bromo-4-fluoro-5-nitrophenol **33a** (5.0 g, 21.2 mmol, Accela), iron powder (6.0 g, 107.4 mmol), and ammonium chloride (11.3 g, 211.2 mmol) were dissolved in ethanol (100 mL) and water (40 mL). The reaction solution was reacted under stirring at 80°C for 16 hours. The reaction solution was filtered. The filtrate was concentrated under reduced pressure. The residue was purified by silica gel column chromatography with eluent system C to obtain the crude title compound **33b** (2.7 g).

MS m/z (ESI): 203.8 [M-1].

### Step 2

### 2-Bromo-4-fluoro-5-(isopropylamino)phenol 33c

Compound **33b** (2.0 g, 9.7 mmol) and acetone (1.7 g, 29.3 mmol) were dissolved in 1,2-dichloroethane (30 mL). Sodium triacetoxyborohydride (6.2 g, 29.3 mmol) and acetic acid (583 mg, 9.7 mmol) were added. The reaction was stirred at room temperature for 16 hours. The reaction solution was concentrated under reduced pressure. The residue was purified by silica gel column chromatography with eluent system C to obtain the title compound **33c** (500 mg, yield: 20.8%).

MS m/z (ESI): 247.9 [M+1].

### Step 3

### Tert-butyl (S)-3-((R)-4-((2-bromo-4-fluoro-5-(isopropylamino)phenoxy)methyl)-2-oxoimidazolidin-1-yl)-2-(4-fluoro-3,5-dimethylphenyl)-4-methyl-2,4,6,7-tetrahydro-5H-pyrazolo[4,3-c]pyridine-5-carboxylate 33d

Compound **13o** (120 mg, 0.22 mmol) and compound **33c** (90 mg, 0.36 mmol) were dissolved in N,N-dimethylformamide (5.0 mL). Potassium carbonate (91 mg, 0.66 mmol) was added and reacted at 60°C for 2 hours. The reaction solution was poured into 20 mL of water and extracted with ethyl acetate (30 mL × 2). The organic phases were combined, washed with a saturated sodium chloride solution, and dried over anhydrous sodium sulfate. After filtration, the filtrate was concentrated under reduced pressure. The residue was purified by silica gel column chromatography with eluent system A to obtain the title compound **33d** (50 mg, yield: 32.7%).

MS m/z (ESI): 703.4 [M+1].

### Step 4

### Tert-butyl (S)-2-(4-fluoro-3,5-dimethylphenyl)-3-((R)-8-fluoro-7-(isopropylamino)-1-oxo-3a,4-dihydro-1H-benzo[b]imidazo[1,5-d][1,4]oxazin-2(3H)-yl)-4-methyl-2,4,6,7-tetrahydro-5H-pyrazolo[4,3-c]pyridine-5-carboxylate 33e

Compound **33d** (50 mg, 0.07 mmol) and (1S,2S)-(+)-*N,N*-dimethyl-1,2-cyclohexanediamine (11 mg, 0.08 mmol) were dissolved in *N*-methylpyrrolidone (5.0 mL). Potassium carbonate (30 mg, 0.22 mmol) and cuprous iodide (14 mg, 0.07 mmol) were added. After displacement with nitrogen three times, the mixture was heated to 110°C and reacted under stirring for 3 hours. The reaction solution was poured into 10 mL of water and extracted with ethyl acetate (20 mL × 2). The organic phases were combined, washed with a saturated sodium chloride solution, and dried over anhydrous sodium sulfate. After filtration, the filtrate was concentrated under reduced pressure. The residue was purified by silica gel column chromatography with eluent system A to obtain the title compound **33e** (40 mg, yield: 90.4%).

MS m/z (ESI): 623.5 [M+1].

### Step 5

### (R)-8-fluoro-2-((S)-2-(4-fluoro-3,5-dimethylphenyl)-4-methyl-4,5,6,7-tetrahydro-2H-pyrazolo[4,3-c]pyridin-3-yl)-7-(isopropylamino)-2,3,3a,4-tetrahydro-1H-benzo[b]imidazo[1,5-d][1,4]oxazin-1-one 33f

Compound **33e** (40 mg, 0.064 mmol) was dissolved in dichloromethane (1 mL). A 4 M solution of hydrogen chloride in 1,4-dioxane (3 mL) was added and stirred at room temperature for 1 hour. The reaction solution was concentrated under reduced pressure to obtain the crude title compound **33f** (34 mg). The product was directly used in the next reaction without purification.

MS m/z (ESI): 523.5 [M+1].

### Step 6

### (R)-2-((S)-5-(5-((S)-2,2-dimethyltetrahydro-2H-pyran-4-yl)-1-((1S,2S)-2-methyl-1-(5-oxo-4,5-dihydro-1,2,4-oxadiazol-3-yl)cyclopropyl)-1H-indole-2-carbonyl)-2-(4-fluoro-3,5-dimethylphenyl)-4-methyl-4,5,6,7-tetrahydro-2H-pyrazolo[4,3-c]pyridin-3-yl)-8-fluoro-7-(isopropylamino)-2,3,3a,4-tetrahydro-1H-benzo[b]imidazo[1,5-d][1,4]oxazin-1-one 33

Compound **33f** (34 mg, 0.065 mmol) and compound **1w** (27 mg, 0.066 mmol) were dissolved in *N,N*-dimethylformamide (5 mL). *N,N*-diisopropylethylamine (43 mg, 0.33 mmol) was added. After the raw materials were fully dissolved, *O*-(7-azabenzotriazol-1-yl)-*N,N,N',N'*-tetramethyluronium hexafluorophosphate (38 mg, 0.10 mmol) was added and stirred at room temperature for 16 hours. The reaction solution was poured into 10 mL of water and extracted with ethyl acetate (10 mL × 3). The organic phases were combined, washed with a saturated sodium chloride solution, and dried over anhydrous sodium sulfate. After filtration, the filtrate was concentrated under reduced pressure. The residue was purified by preparative high-performance liquid chromatography (instrument model: Waters-2545, chromatographic column: Welch Xtimate, Prep 30 × 150 mm; 5 µm; C18, mobile phase: water (10 mM ammonium bicarbonate) and acetonitrile, with gradient ratio: acetonitrile 50%-85%, flow rate: 30 mL/min) to obtain the title compound **33** (16 mg, yield: 26.8%).

MS m/z (ESI): 916.7 [M+1].

¹H NMR (500 MHz, CDCl₃): δ 11.30 (s, 1H), 7.71-7.37 (m, 3H), 7.25-7.21 (m, 1H), 7.18-7.07 (m, 2H), 6.72-6.61 (m, 1H), 6.32-6.15 (m, 1H), 5.79-5.18 (m, 2H), 4.89-4.37 (m, 1H), 4.29-4.14 (m, 1H), 4.12-4.03 (m, 1H), 3.92-3.74 (m, 3H), 3.68-3.29 (m, 4H), 3.16-2.84 (m, 4H), 2.29-2.17 (m, 6H), 2.04-1.46 (m, 16H), 1.39-1.30 (m, 5H), 1.05 (d, 1H), 0.88 (t, 1H).

### Example 34-1 or 34-2

### 3-((1S,2S)-1-((R)-6-((S)-2-(4-fluoro-3,5-dimethylphenyl)-4-methyl-3-(1-(1-methyl-1H-indazol-5-yl)-6-oxo-1,6-dihydropyrimidin-5-yl)-4,5,6,7-tetrahydro-2H-pyrazolo[4,3-c]pyridine-5-carbonyl)-2',2'-dimethyl-2',3',5',6'-tetrahydro-2H,7H-spiro[furo[3,2-f]indole-3,4'-pyran]-7-yl)-2-methylcyclopropyl)-1,2,4-oxadiazol-5(4H)-one 34-1 or

### 3-((1S,2S)-1-((S)-6-((S)-2-(4-fluoro-3,5-dimethylphenyl)-4-methyl-3-(1-(1-methyl-1H-indazol-5-yl)-6-oxo-1,6-dihydropyrimidin-5-yl)-4,5,6,7-tetrahydro-2H-pyrazolo[4,3-c]pyridine-5-carbonyl)-2',2'-dimethyl-2',3',5',6'-tetrahydro-2H,7H-spiro[furo[3,2-f]indole-3,4'-pyran]-7-yl)-2-methylcyclopropyl)-1,2,4-oxadiazol-5(4H)-one 34-2

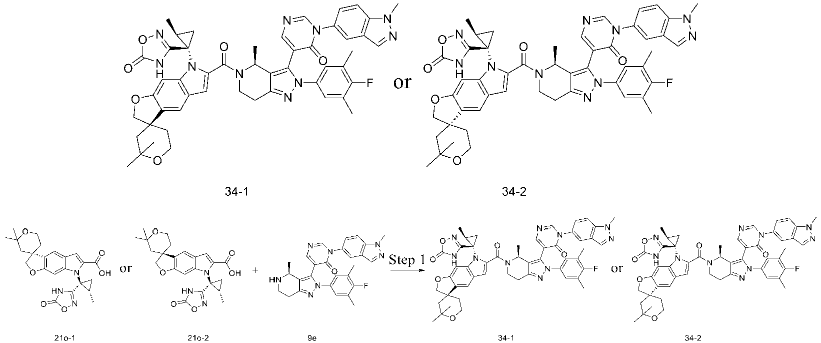

Compound **21o-1 or 21o-2** (23 mg, 0.052 mmol, prepared in six steps from the compound with a shorter retention time among compounds **21i-1** and **21i-2** #1) and compound **9e** (25 mg, 0.052 mmol) were dissolved in *N,N*-dimethylformamide (3 mL). 2-(7-Azabenzotriazole)-*N,N,N',N'*-tetramethyluronium hexafluorophosphate(30 mg, 0.079 mmol) and *N,N*-diisopropylethylamine (35 mg, 0.27 mmol) were added and the mixture was reacted at room temperature for 16 hours. The reaction solution was concentrated under reduced pressure. The residue was purified by preparative high-performance liquid chromatography (instrument model: Gilson-GX-281, chromatographic column: Boston phlex, Prep 30 × 250 mm; 5 µm; C18, mobile phase: water (10 mM ammonium bicarbonate) and acetonitrile, with gradient ratio: acetonitrile 35%-60%, flow rate: 30 mL/min) to obtain the title compound **34-1** or **34-2** (15 mg, yield: 31.7%, prepared in seven steps from the compound with a shorter retention time among compounds **21i-1** and **21i-2).**

MS m/z (ESI): 905.5 [M+1].

¹H NMR (500 MHz, CDCl₃): δ 11.39 (s, 1H), 8.35-8.15 (m, 1H), 8.12-7.62 (m, 3H), 7.60-7.29 (m, 3H), 7.10-6.76 (m, 3H), 6.69-6.58 (m, 1H), 5.82-5.21 (m, 2H), 4.85-4.32 (m, 3H), 4.19-4.08 (m, 3H), 3.87-3.74 (m, 2H), 3.66-3.36 (m, 1H), 3.19-2.93 (m, 2H), 2.29-2.20 (m, 6H), 2.08 (d, 1H), 2.04-1.97 (m, 1H), 1.86 (t, 1H), 1.76-1.65 (m, 4H), 1.46 (d, 3H), 1.33-1.28 (m, 4H), 1.17 (d, 2H), 1.00 (d, 1H), 0.88 (t, 1H).

### Example 35-1 or 35-2

### (R)-1-cyclopropyl-7-((S)-5-((R)-2',2'-dimethyl-7-((1S,2S)-2-methyl-1-(5-oxo-4,5-dihydro-1,2,4-oxadiazol-3-yl)cyclopropyl)-2',3',5',6'-tetrahydro-2H,7H-spiro[furo[3,2-f]indole-3,4'-pyran]-6-carbonyl)-2-(4-fluoro-3,5-dimethylphenyl)-4-methyl-4,5,6,7-tetrahydro-2H-pyrazolo[4,3-c]pyridin-3-yl)-5,5a,6,7-tetrahydro-1H,8H-imidazo[1',5':4,5][1,4]oxazino[2,3-e]indazol-8-one 35-1 or

### (R)-1-cyclopropyl-7-((S)-5-((S)-2',2'-dimethyl-7-((1S,2S)-2-methyl-1-(5-oxo-4,5-dihydro-1,2,4-oxadiazol-3-yl)cyclopropyl)-2',3',5',6'-tetrahydro-2H,7H-spiro[furo[3,2-f]indole-3,4'-pyran]-6-carbonyl)-2-(4-fluoro-3,5-dimethylphenyl)-4-methyl-4,5,6,7-tetrahydro-2H-pyrazolo[4,3-c]pyridin-3-yl)-5,5a,6,7-tetrahydro-1H,8H-imidazo[1',5':4,5][1,4]oxazino[2,3-e]indazol-8-one 35-2

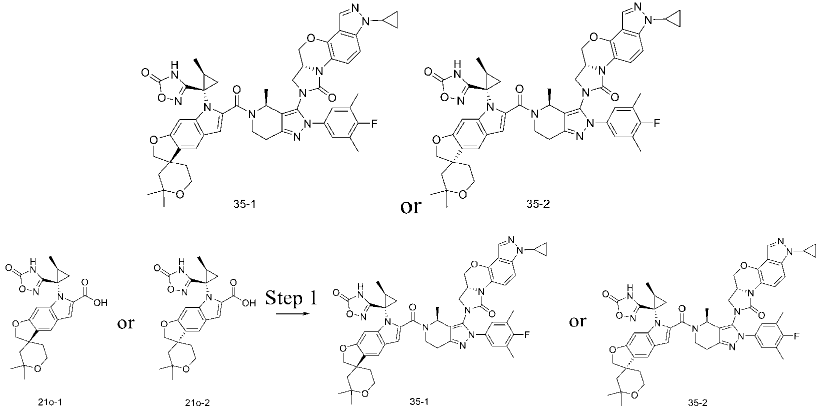

Compound **21o-1 or 21o-2** (42 mg, 0.096 mmol, prepared in six steps from the compound with a shorter retention time among compounds **21i-1** and **21i-2)** and compound **24d** (50 mg, 0.096 mmol) were dissolved in *N,N*-dimethylformamide (5 mL). 2-(7-Azabenzotriazole)-*N,N,N',N*'-tetramethyluronium hexafluorophosphate (44 mg, 0.115 mmol) and *N,N*-diisopropylethylamine (62 mg, 0.478 mmol) were added and the mixture was reacted at room temperature for 16 hours. The reaction solution was concentrated under reduced pressure. The residue was purified by preparative high-performance liquid chromatography (instrument model: Waters-2545-2767-2489, chromatographic column: Sharpsil-T, Prep 30 × 250 mm; 5 µm; C18, mobile phase: water (10 mM ammonium bicarbonate) and acetonitrile, with gradient ratio: acetonitrile 45%-65%, flow rate: 30 mL/min) to obtain the title compound **35-1** or **35-2** (35 mg, yield: 39%, prepared in seven steps from the compound with a shorter retention time among compounds **21i-1** and **21i-2).**

MS m/z (ESI): 949.5 [M+1].

¹H NMR (500 MHz, CDCl₃): δ 11.60-10.80 (m, 1H), 8.03-7.83 (m, 2H), 7.35-7.25 (m, 2H), 7.21-7.11 (m, 2H), 7.05-6.97 (m, 1H), 6.66-6.65 (m, 1H), 5.75-5.23 (m, 1H), 4.80-4.70 (m, 1H), 4.50-4.32 (m, 3H), 4.17-4.11 (m, 1H), 3.92-3.88 (m, 1H), 3.84-3.77 (m, 2H), 3.68-3.50 (m, 3H), 3.14-2.91 (m, 3H), 2.24-2.17 (m, 6H), 2.11-1.98 (m, 1H), 1.88-1.85 (m, 1H), 1.78-1.44 (m, 8H), 1.39-1.14 (m, 12H), 1.05-0.86 (m, 1H).

### Example 36

### 2-((S)-5-(5-((S)-2,2-dimethyltetrahydro-2H-pyran-4-yl)-1-((1S,2S)-2-methyl-1-(5-oxo-4,5-dihydro-1,2,4-oxadiazol-3-yl)cyclopropyl)-1H-indole-2-carbonyl)-2-(4-fluoro-3,5-dimethylphenyl)-4-methyl-4,5,6,7-tetrahydro-2H-pyrazolo[4,3-c]pyridin-3-yl)-8-methyl-8H-pyrimido[1',2':4,5][1,4]oxazino[3,2-f]indazol-1(5H)-one 36

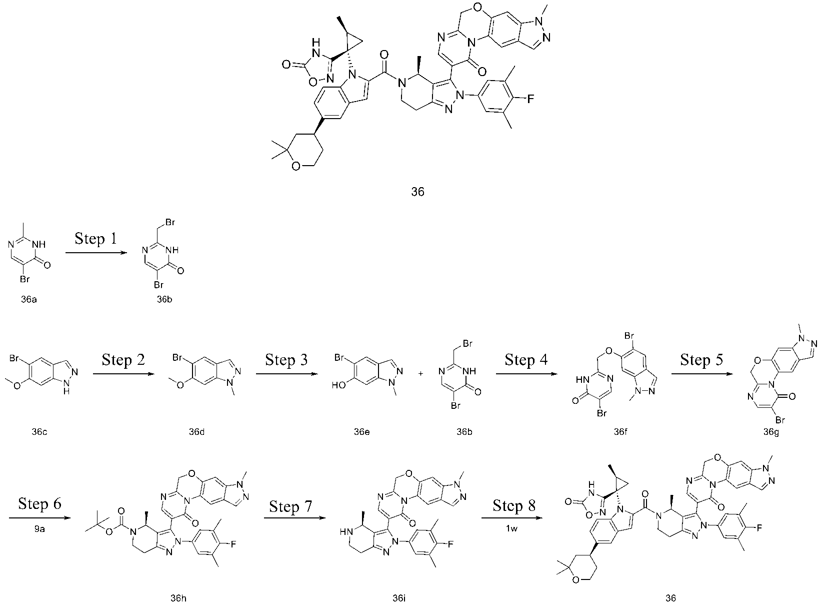

### Step 1

### 5-Bromo-2-(bromomethyl)pyrimidin-4(3H)-one 36b

5-Bromo-2-methylpyrimidin-4(3*H*)-one **36a** (1.0 g, 5.29 mmol, Accela), *N-*bromosuccinimide (1.25 g, 7.02 mmol), and azodiisobutyronitrile (100 mg, 0.61 mmol) were dissolved in tetrahydrofuran (30 mL). The reaction was stirred at 30°C for 16 hours. The reaction solution was concentrated under reduced pressure. The residue was purified by silica gel column chromatography with eluent system A to obtain the title compound **36b** (110 mg, yield: 7.8%).

MS m/z (ESI): 266.8 [M+1].

### Step 2

### 5-Bromo-6-methoxy-1-methyl-1H-indazole 36d

5-Bromo-6-methoxy-1*H*-indazole **36c** (2.0 g, 8.81 mmol, Accela) was dissolved in *N,N*-dimethylformamide (20 mL). Sodium hydride (406 mg, 10.60 mmol, 60% dispersed in a mineral oil) was added in portions 0°C and stirred for 1 hour. Iodomethane (1.5 g, 10.57 mmol) was dropwise added. After the addition was complete, the reaction was stirred at 0°C for 2 hours. The reaction solution was quenched with a saturated aqueous ammonium chloride solution (50 mL), extracted with ethyl acetate (50 mL × 2), and washed with a saturated sodium chloride solution. The organic phase was dried over anhydrous sodium sulfate. After filtration, the filtrate was concentrated under reduced pressure. The residue was purified by silica gel column chromatography with eluent system C to obtain the title compound **36d** (3.6 g, yield: 66.7%).

MS m/z (ESI): 241.0 [M+1].

### Step 3

### 5-Bromo-1-methyl-1H-indazol-6-ol 36e

Compound **36d** (800 mg, 3.32 mmol) was dissolved in boron tribromide (25.0 mL, 25.0 mmol, a 1 M dichloromethane solution). The reaction solution was stirred at 50°C for 16 hours. The reaction solution was cooled to room temperature, quenched with ice water (10 mL), adjusted to approximately pH 8-9, and an excess of a saturated sodium bicarbonate solution, and extracted with dichloromethane (30 mL × 2). The organic phase was dried over anhydrous sodium sulfate. After filtration, the filtrate was concentrated under reduced pressure. The residue was purified by silica gel column chromatography with eluent system A to obtain the title compound **36e** (550 mg, yield: 73.0%).

MS m/z (ESI): 227.1 [M+1].

### Step 4

### 5-Bromo-2-(((5-bromo-1-methyl-1H-indazol-6-yl)oxy)methyl)pyrimidin-4(3H)-one 36f

Compound **36e** (260 mg, 1.15 mmol) was dissolved in *N,N-*dimethylformamide (10 mL). Sodium hydride (132 mg, 3.44 mmol, 60% dispersed in a mineral oil) was added in portions at room temperature and stirred at room temperature for 1 hour. Compound **36b** (250 mg, 0.93 mmol) was added and reacted under stirring at room temperature for 3 hours. The reaction solution was quenched with a saturated aqueous ammonium chloride solution (10 mL), extracted with ethyl acetate (20 mL × 2), and washed with a saturated sodium chloride solution. The organic phase was dried over anhydrous sodium sulfate. After filtration, the filtrate was concentrated under reduced pressure. The residue was purified by silica gel column chromatography with eluent system A to obtain the title compound **36f** (90 mg, yield: 19.0%).

MS m/z (ESI): 412.9 [M+1].

### Step 5

### 2-Bromo-8-methyl-8H-pyrimido[1',2':4,5][1,4]oxazino[3,2-f]indazol-1(5H)-one 36g

Compound **36f** (20 mg, 0.048 mmol) and *N*,*N*-dimethylethylenediamine (8 mg, 0.091 mmol) were dissolved in *N*,*N*-dimethylformamide (8.0 mL) and 1,4-dioxane (4.0 mL). Anhydrous potassium phosphate (31 mg, 0.15 mmol) and cuprous iodide (15 mg, 0.079 mmol) were added. After displacement with nitrogen three times, the mixture was heated to 90°C and reacted under stirring for 3 hours. The reaction solution was poured into 5 mL of water and extracted with ethyl acetate (20 mL × 2). The organic phases were combined, washed with a saturated sodium chloride solution, and dried over anhydrous sodium sulfate. After filtration, the filtrate was concentrated under reduced pressure. The residue was purified by silica gel column chromatography with eluent system A to obtain the title compound **36g** (10 mg, yield: 62.1%).

MS m/z (ESI): 333.1 [M+1].

### Step 6

### Tert-butyl (S)-2-(4-fluoro-3,5-dimethylphenyl)-4-methyl-3-(8-methyl-1-oxo-1,5-dihydro-8H-pyrimido[1',2':4,5][1,4]oxazino[3,2-f]indazol-2-yl)-2,4,6,7-tetrahydro-5H-pyrazolo[4,3-c]pyridine-5-carboxylate 36h

Compound **36g** (20 mg, 0.060 mmol), compound **9a** (36 mg, 0.089 mmol), anhydrous potassium carbonate (25 mg, 0.18 mmol), and 1,1'-bis(di-tert-butylphosphino)ferrocenepalladium dichloride (21 mg, 0.031 mmol, Adamas) were dissolved in dioxane (20 mL) and water (4 mL). After displacement with nitrogen, the reaction was stirred at 70°C for 6 hours. The reaction solution was cooled to room temperature and concentrated under reduced pressure. The residue was purified by silica gel column chromatography with eluent system A to obtain the title compound **36h** (20 mg, yield: 54.5%).

MS m/z (ESI): 612.4 [M+1].

### Step 7

### (S)-2-(2-(4-fluoro-3,5-dimethylphenyl)-4-methyl-4,5,6,7-tetrahydro-2H-pyrazolo[4,3-c]pyridin-3-yl)-8-methyl-8H-pyrimido[1',2':4,5][1,4]oxazino[3,2-f]indazol-1(5H)-one 36i

Compound **36h** (20 mg, 0.033 mmol) was dissolved in dichloromethane (1 mL). A 4 M solution of hydrogen chloride in 1,4-dioxane (3 mL) was added and stirred at room temperature for 1 hour. The reaction solution was concentrated under reduced pressure to obtain the crude title compound **36i** (17 mg). The product was directly used in the next reaction without purification.

MS m/z (ESI): 512.4 [M+1].

### Step 8

### 2-((S)-5-(5-((S)-2,2-dimethyltetrahydro-2H-pyran-4-yl)-1-((1S,2S)-2-methyl-1-(5-oxo-4,5-dihydro-1,2,4-oxadiazol-3-yl)cyclopropyl)-1H-indole-2-carbonyl)-2-(4-fluoro-3,5-dimethylphenyl)-4-methyl-4,5,6,7-tetrahydro-2H-pyrazolo[4,3-c]pyridin-3-yl)-8-methyl-8H-pyrimido[1',2':4,5][1,4]oxazino[3,2-f]indazol-1(5H)-one 36

Compound **36i** (17 mg, 0.033 mmol) and compound **1w** (17 mg, 0.041 mmol) were dissolved in *N*,*N*-dimethylformamide (3 mL). *N*,*N*-diisopropylethylamine (27 mg, 0.21 mmol) was added. After the raw materials were fully dissolved, *O*-(7-azabenzotriazol-1-yl)-*N*,*N*,*N*',*N*'-tetramethyluronium hexafluorophosphate (25 mg, 0.066 mmol) was added and stirred at room temperature for 16 hours. The reaction solution was poured into 10 mL of water and extracted with ethyl acetate (10 mL × 3). The organic phases were combined, washed with a saturated sodium chloride solution, and dried over anhydrous sodium sulfate. After filtration, the filtrate was concentrated under reduced pressure. The residue was purified by preparative high-performance liquid chromatography (instrument model: Gilson-GX-281, chromatographic column: Boston phlex, Prep 30 × 150 mm; 5 µm; C18, mobile phase: water (10 mM ammonium bicarbonate) and acetonitrile, with gradient ratio: acetonitrile 35%-60%, flow rate: 30 mL/min) to obtain the title compound 36 (18 mg, yield: 48.1%).

MS m/z (ESI): 905.6 [M+1].

¹H NMR (500 MHz, CDCl₃): δ 11.41-11.29 (m, 1H), 8.99-7.74 (m, 2H), 7.65-7.29 (m, 3H), 7.26-7.23 (m, 1H), 7.15-6.92 (m, 3H), 6.73-6.62 (m, 1H), 5.88-5.39 (m, 1H), 4.98 (q, 1H), 4.93-4.38 (m, 2H), 4.12-3.95 (m, 3H), 3.92-3.77 (m, 2H), 3.65-3.41 (m, 1H), 3.23-2.97 (m, 3H), 2.29-2.16 (m, 6H), 2.05-1.98 (m, 1H), 1.89 (t, 1H), 1.84-1.71 (m, 3H), 1.69-1.58 (m, 3H), 1.48-1.43 (m, 2H), 1.38-1.29 (m, 5H), 1.17 (d, 2H), 1.03 (d, 1H), 0.88 (t, 1H).

### Example 37

### (R)-1-cyclopropyl-7-((S)-5-(5-((S)-2,2-dimethyltetrahydro-2H-pyran-4-yl)-1-((1S,2S)-2-methyl-1-(5-oxo-4,5-dihydro-1,2,4-oxadiazol-3-yl)cyclopropyl)-1H-indole-2-carbonyl)-2-(4-fluoro-3,5-dimethylphenyl)-4-methyl-4,5,6,7-tetrahydro-2H-pyrazolo[4,3-c]pyridin-3-yl)-10-fluoro-5,5a,6,7-tetrahydro-1H,8H-imidazo[1',5':4,5][1,4]oxazino[2,3-e]indazol-8-one 37

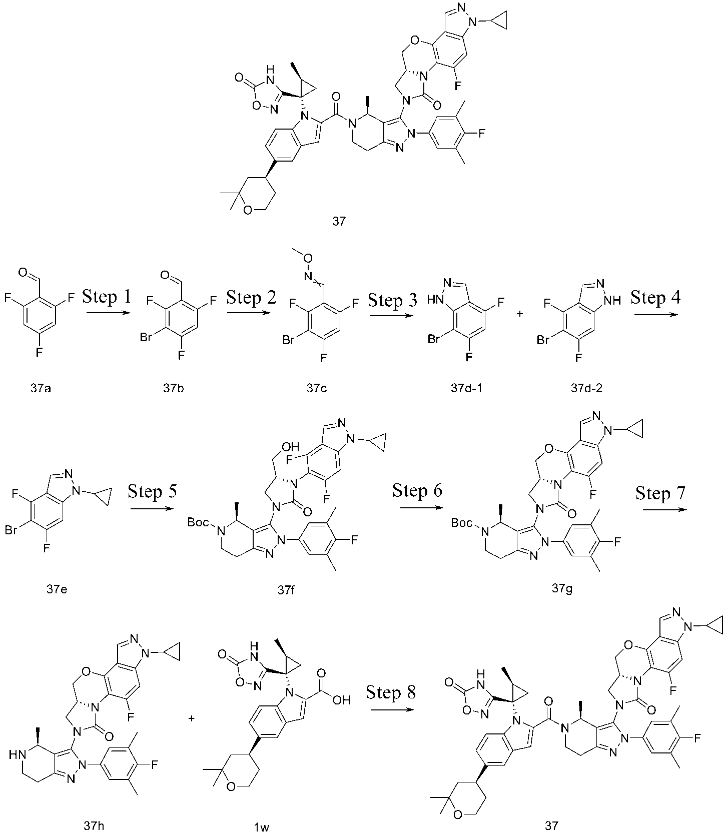

### Step 1

### 3-Bromo-2,4,6-trifluorobenzaldehyde 37b

2,4,6-Trifluorobenzaldehyde **37a** (22.50 g, 140.5 mmol, Adamas) was dissolved in concentrated sulfuric acid (66 mL) and heated to 60°C. *N-*bromosuccinimide (30.02 g, 168.7 mmol) was added at 60°C. The reaction was stirred at 60°C for 5 hours. The reaction solution was poured into 200 mL of ice water and extracted with ethyl acetate (100 mL × 3). The organic phases were combined, washed with a saturated sodium chloride solution, and dried over anhydrous sodium sulfate. After filtration, the filtrate was concentrated under reduced pressure. The residue was purified by silica gel column chromatography with eluent system B to obtain the title compound **37b** (28.60 g, yield: 85%).

### Step 2

### 3-Bromo-2,4,6-trifluorobenzaldehyde O-methyloxime 37c

Compound **37b** (28.60 g, 119.7 mmol) was dissolved in ethylene glycol dimethyl ether (300 mL). Potassium carbonate (21.50 g, 155.6 mmol) and methoxyamine hydrochloride (10.99 g, 131.6 mmol). The reaction was stirred at 60°C for 16 hours. After filtration, the filtrate was concentrated under reduced pressure. The residue was purified by silica gel column chromatography with eluent system B to obtain the title compound **37c** (31 g, yield: 97%).

### Step 3

### 7-Bromo-4,6-difluoro-1H-indazole 37d-1

### 5-Bromo-4,6-difluoro-1H-indazole 37d-2

Compound **37c** (31.00 g, 115.7 mmol) was dissolved in tetrahydrofuran (100 mL). Hydrazine hydrate (100 mL, 85% wt aqueous solution) was added and stirred at 60°C for 16 hours. The reaction solution was poured into 200 mL of water and extracted with ethyl acetate (100 mL × 3). The organic phases were combined, washed with a saturated sodium chloride solution, and dried over anhydrous sodium sulfate. After filtration, the filtrate was concentrated under reduced pressure. The residue was purified by silica gel column chromatography with eluent system B to obtain the title compound **37d-1** (15.00 g, yield: 56%) and the title compound **37d-2** (1.40 g, yield: 5%).

MS m/z (ESI): 230.7 [M-1].

### Step 4

### 5-Bromo-1-cyclopropyl-4,6-difluoro-1H-indazole 37e

Compound **37d-2** (800 mg, 3.43 mmol), cyclopropylboronic acid (590 mg, 6.87 mmol), 2,2'-bipyridine (1.07 g, 6.85 mmol), copper acetate (1.25 g, 6.88 mmol), and sodium carbonate(1.09 g, 10.28 mmol) were dissolved in 1,2-dichloroethane (50 mL), left open, and stirred at 70°C for 18 hours. The reaction solution was cooled to room temperature, filtered, and concentrated under reduced pressure. The residue was purified by silica gel column chromatography with eluent system B to obtain the title compound **37e** (650 mg, yield: 69%).

### Step 5

### Tert-butyl (S)-3-((R)-3-(1-cyclopropyl-4,6-difluoro-1H-indazol-5-yl)-4-(hydroxymethyl)-2-oxoimidazolidin-1-yl)-2-(4-fluoro-3,5-dimethylphenyl)-4-methyl-2,4,6,7-tetrahydro-5H-pyrazolo[4,3-c]pyridine-5-carboxylate 37f

Compound **13n** (150 mg, 0.32 mmol), compound **37e** (260 mg, 0.95 mmol), and (1*S*,2*S*)-(-)-*N*,*N*-dimethyl-1,2-cyclohexanediamine (36 mg, 0.25 mmol) were dissolved in *N*-methylpyrrolidone (10.0 mL). Potassium carbonate (131 mg, 0.95 mmol) and cuprous iodide (24 mg, 0.13 mmol) were added. After displacement with nitrogen three times, the reaction was stirred in a sealed tube at 120°C for 16 hours. The reaction solution was poured into 50 mL of water and extracted with ethyl acetate (30 mL × 2). The organic phases were combined, washed with a saturated sodium chloride solution, and dried over anhydrous sodium sulfate. After filtration, the filtrate was concentrated under reduced pressure. The residue was purified by silica gel column chromatography with eluent system B to obtain the title compound **37f (30** mg, yield: 14%).

MS m/z (ESI): 666.5 [M+1].

### Step 6

### Tert-butyl (S)-3-((R)-1-cyclopropyl-10-fluoro-8-oxo-5a,6-dihydro-1H,5H-imidazo[1',5':4,5][1,4]oxazino[2,3-e]indazol-7(8H)-yl)-2-(4-fluoro-3,5-dimethylphenyl)-4-methyl-2,4,6,7-tetrahydro-5H-pyrazolo[4,3-c]pyridine-5-carboxylate 37g

Compound **37f** (30 mg, 0.045 mmol) was dissolved in dimethyl sulfoxide (10.0 mL). Cesium carbonate (44 mg, 0.14 mmol) was added. After displacement with nitrogen three times,the mixture was reacted under stirring at 120°C for 2 hours. The reaction solution was poured into 50 mL of water and extracted with ethyl acetate (30 mL × 2). The organic phases were combined, washed with a saturated sodium chloride solution, and dried over anhydrous sodium sulfate. After filtration, the filtrate was concentrated under reduced pressure. The residue was purified by silica gel column chromatography with eluent system B to obtain the title compound **37g** (25 mg, yield: 86%).

MS m/z (ESI): 646.4 [M+1].

### Step 7

### (R)-1-cyclopropyl-10-fluoro-7-((S)-2-(4-fluoro-3,5-dimethylphenyl)-4-methyl-4,5,6,7-tetrahydro-2H-pyrazolo[4,3-c]pyridin-3-yl)-5,5a,6,7-tetrahydro-1H,8H-imidazo[1',5':4,5][1,4]oxazino[2,3-e]indazol-8-one 37h

Compound **37g** (25 mg, 0.039 mmol) was dissolved in dichloromethane (2 mL). A 4 M solution of hydrogen chloride in 1,4-dioxane (2 mL) was added and stirred at room temperature for 1 hour. The reaction solution was concentrated under reduced pressure to obtain the crude title compound **37h** (21 mg). The product was directly used in the next reaction without purification.

MS m/z (ESI): 546.4 [M+1].

### Step 8

### (R)-1-cyclopropyl-7-((S)-5-(5-((S)-2,2-dimethyltetrahydro-2H-pyran-4-yl)-1-((1S,2S)-2-methyl-1-(5-oxo-4,5-dihydro-1,2,4-oxadiazol-3-yl)cyclopropyl)-1H-indole-2-carbonyl)-2-(4-fluoro-3,5-dimethylphenyl)-4-methyl-4,5,6,7-tetrahydro-2H-pyrazolo[4,3-c]pyridin-3-yl)-10-fluoro-5,5a,6,7-tetrahydro-1H,8H-imidazo[1',5':4,5][1,4]oxazino[2,3-e]indazol-8-one 37

Compound **37h** (20 mg, 0.036 mmol) and compound **1w** (15 mg, 0.036 mmo) were dissolved in *N*,*N*-dimethylformamide (4 mL). *N*,*N*-diisopropylethylamine (24 mg, 0.18 mmol) was added. After the raw materials were fully dissolved, *O*-(7-azabenzotriazol-1-yl)-*N*,*N*,*N*',*N*'-tetramethyluronium hexafluorophosphate (21 mg, 0.055 mmol) was added and stirred at room temperature for 16 hours. After filtration, the residue was purified by preparative high-performance liquid chromatography (instrument model: Waters-2545-2767-2489, chromatographic column: Sharpsil-T, Prep 30 × 250 mm; 5 µm; C18, mobile phase: water (10 mM ammonium bicarbonate) and acetonitrile, with gradient ratio: acetonitrile 45%-65%, flow rate: 30 mL/min) to obtain the title compound **37** (13 mg, yield: 38%).

MS m/z (ESI): 939.6 [M+1].

¹H NMR (500 MHz, CDCl₃): δ 11.25 (br, 1H), 7.92-7.86 (m, 1H), 7.60-7.48 (m, 2H), 7.28-7.25 (m, 1H), 7.20-7.14 (m, 2H), 7.03-6.95 (m, 2H), 6.69-6.68 (m, 1H), 5.80-4.82 (m, 1H), 4.46-4.32 (m, 1H), 4.10-3.30 (m, 6H), 3.18-2.92 (m, 4H), 2.33-2.20 (m, 6H), 2.05-1.50 (m, 10H), 1.48-0.86 (m, 14H).

### Example 38

### 3-((1S,2S)-1-(5-((S)-2,2-dimethyltetrahydro-2H-pyran-4-yl)-2-((S)-2-(4-fluoro-3,5-dimethylphenyl)-4-methyl-3-(4-(1-methyl-1H-indazol-5-yl)-3-oxo-3,4-dihydropyrazin-2-yl)-4,5,6,7-tetrahydro-2H-pyrazolo[4,3-c]pyridine-5-carbonyl)-1H-indol-1-yl)-2-methylcyclopropyl)-1,2,4-oxadiazol-5(4H)-one 38

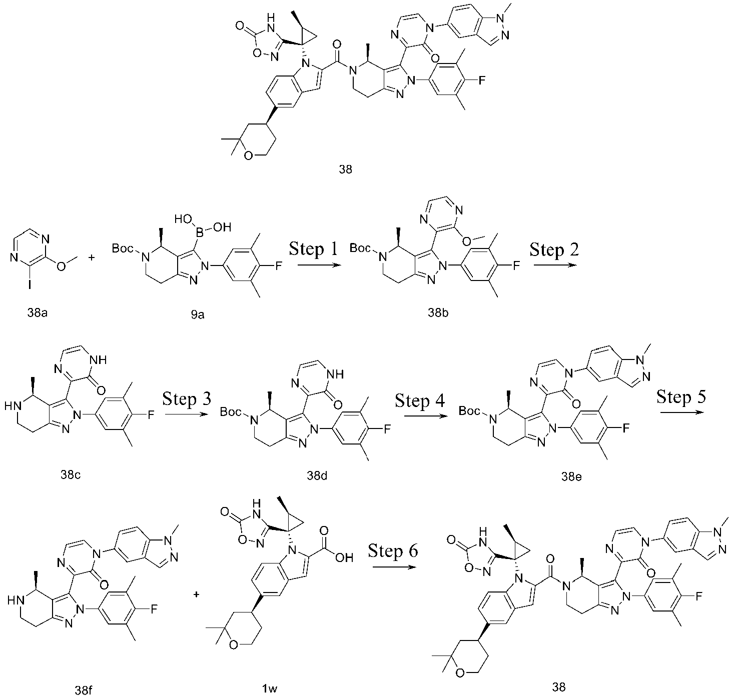

### Step 1

### Tert-butyl (S)-2-(4-fluoro-3,5-dimethylphenyl)-3-(3-methoxypyrazin-2-yl)-4-methyl-2,4,6,7-tetrahydro-5H-pyrazolo[4,3-c]pyridine-5-carboxylate 38b

2-Iodo-3-methoxypyrazine **38a** (155 mg, 0.66 mmol, Aikon), compound **9a** (450 mg, 1.12 mmol), anhydrous potassium carbonate (272 mg, 1.97 mmol), [1,1'-bis(diphenylphosphino)ferrocene]palladium(II) dichloride (144 mg, 0.20 mmol), and tetrakis(triphenylphosphine)palladium (228 mg, 0.20 mmol) were dissolved in dioxane (25 mL) and water (2 mL). After displacement with nitrogen, the reaction was stirred at 80°C for 16 hours. The reaction solution was cooled to room temperature and concentrated under reduced pressure. The residue was purified by silica gel column chromatography with eluent system B to obtain the crude title compound **38b** (100 mg, yield: 33%).

MS m/z (ESI): 468.3 [M+1].

### Step 2

### (S)-3-(2-(4-fluoro-3,5-dimethylphenyl)-4-methyl-4,5,6,7-tetrahydro-2H-pyrazolo[4,3-c]pyridin-3-yl)pyrazin-2-(1H)-one 38c

Compound **38b** (90 mg, 0.19 mmol) was dissolved in acetonitrile(15 mL). Sodium iodide (87 mg, 0.58 mmol) was added, followed by trimethylchlorosilane (63 mg, 0.58 mmol). The mixture was heated to 70°C and reacted under stirring for 1.5 hours. After the reaction solution was cooled, 3 mL of saturated sodium bicarbonate and 3 mL of a saturated sodium sulfite solution were added to quench the reaction. The aqueous phase was directly used for the next reaction.

MS m/z (ESI): 354.1 [M+1].

### Step 3

### Tert-butyl (S)-2-(4-fluoro-3,5-dimethylphenyl)-4-methyl-3-(3-oxo-3,4-dihydropyrazin-2-yl)-2,4,6,7-tetrahydro-5H-pyrazolo[4,3-c]pyridine-5-carboxylate 38d

Methanol (4 mL), triethylamine (97 mg, 0.96 mmol), and di-tert-butyl dicarbonate (84 mg, 0.38 mmol) were added to the aqueous phase containing compound **38c** (150 mg, 0.43 mmol) from the previous step and reacted under stirring at room temperature for 16 hours. The reaction mixture was extracted with ethyl acetate (10 mL × 3). The organic phases were combined, washed with a saturated sodium chloride solution, and dried over anhydrous sodium sulfate. After filtration, the filtrate was concentrated under reduced pressure. The residue was purified by silica gel column chromatography with eluent system A to obtain the title compound **38d** (60 mg, yield: 69%).

MS m/z (ESI): 454.2 [M+1].

### Step 4

### Tert-butyl (S)-2-(4-fluoro-3,5-dimethylphenyl)-4-methyl-3-(4-(1-methyl-1H-indazol-5-yl)-3-oxo-3,4-dihydropyrazin-2-yl)-2,4,6,7-tetrahydro-5H-pyrazolo[4,3-c]pyridine-5-carboxylate 38e

Compound **38d** (60 mg, 0.13 mmol), (1-methyl-1*H*-indazol-5-yl)boronic acid (35 mg, 0.20 mmol), pyridine (105 mg, 1.32 mmol), and copper acetate (53 mg, 0.26 mmol) were dissolved in 1,2-dichloroethane (10 mL), left open, and stirred at room temperature for 16 hours. The reaction solution was concentrated under reduced pressure. The residue was purified by silica gel column chromatography with eluent system A to obtain the title compound **38e** (70 mg, yield: 91%).

MS m/z (ESI): 584.4 [M+1].

### Step 5

### (S)-3-(2-(4-fluoro-3,5-dimethylphenyl)-4-methyl-4,5,6,7-tetrahydro-2H-pyrazolo[4,3-c]pyridin-3-yl)-1-(1-methyl-1H-indazol-5-yl)pyrazin-2(1H)-one 38f

Compound **38e** (70 mg, 0.12 mmol) was dissolved in dichloromethane (2 mL). A 4M solution of hydrogen chloride in dioxane (2 mL) was added. The reaction was stirred at room temperature for 1 hour. The reaction solution was concentrated under reduced pressure to obtain the crude title compound **38f** (58 mg). The product was directly used in the next reaction without purification.

MS m/z (ESI): 484.2 [M+1].

### Step 6

### 3-((1S,2S)-1-(5-((S)-2,2-dimethyltetrahydro-2H-pyran-4-yl)-2-((S)-2-(4-fluoro-3,5-dimethylphenyl)-4-methyl-3-(4-(1-methyl-1H-indazol-5-yl)-3-oxo-3,4-dihydropyrazin-2-yl)-4,5,6,7-tetrahydro-2H-pyrazolo[4,3-c]pyridine-5-carbonyl)-1H-indol-1-yl)-2-methylcyclopropyl)-1,2,4-oxadiazol-5(4H)-one 38

Compound **38f** (58 mg, 0.12 mmol) and compound **1w** (50 mg, 0.12 mmol) were dissolved in *N*,*N*-dimethylformamide (5 mL). *N*,*N*-diisopropylethylamine (79 mg, 0.61 mmol) was added. After the raw materials were fully dissolved, O-(7-azabenzotriazol-1-yl)-*N*,*N*,*N*',*N*'-tetramethyluronium hexafluorophosphate (69 mg, 0.18 mmol) was added and stirred at room temperature for 16 hours. The reaction solution was poured into 10 mL of water and extracted with ethyl acetate (10 mL × 3). The organic phases were combined, washed with a saturated sodium chloride solution, and dried over anhydrous sodium sulfate. After filtration, the filtrate was concentrated under reduced pressure. The residue was purified by preparative high-performance liquid chromatography (instrument model: Waters-2545 chromatographic column: Welch Xtimate, Prep 30 × 150 mm; 5 µm; C18, mobile phase: water (10 mM ammonium bicarbonate) and acetonitrile, with gradient ratio: acetonitrile 35%-60%, flow rate: 30 mL/min) to obtain the title compound **38** (3 mg, yield: 3%).

MS m/z (ESI): 877.4 [M+1].

¹H NMR (500 MHz, CDCl₃): δ 11.47-11.36 (m, 1H), 8.05-7.94 (m, 1H), 7.62-7.38 (m, 5H), 7.25-7.18 (m, 2H), 7.07-7.05 (m, 1H), 7.00-6.80 (m, 1H), 6.69-6.68 (m, 1H), 5.94 (q, 1H), 5.37-5.33 (m, 1H), 4.87-4.39 (m, 1H), 4.17-4.08 (m, 3H), 3.90-3.80 (m, 2H), 3.66-3.47 (m, 1H), 3.19-3.01 (m, 3H), 2.34-2.20 (m, 6H), 2.04-1.99 (m, 1H), 1.90 (t, 1H), 1.80-1.51 (m, 6H), 1.35-1.25 (m, 7H), 1.19 (d, 2H), 1.01 (d, 1H), 0.88 (t, 1H).

### Example 39

### (R)-1-cyclopropyl-7-((S)-5-(5-((S)-2,2-dimethyltetrahydro-2H-pyran-4-yl)-1-((1S,2S)-2-methyl-1-(5-oxo-4,5-dihydro-1,2,4-oxadiazol-3-yl)cyclopropyl)-1H-indole-2-carbonyl)-2-(4-fluoro-3,5-dimethylphenyl)-4-methyl-4,5,6,7-tetrahydro-2H-pyrazolo[4,3-c]pyridin-3-yl)-11-fluoro-5,5a,6,7-tetrahydro-1H,8H-imidazo[1',5':4,5][1,4]oxazino[2,3-e]indazol-8-one 39

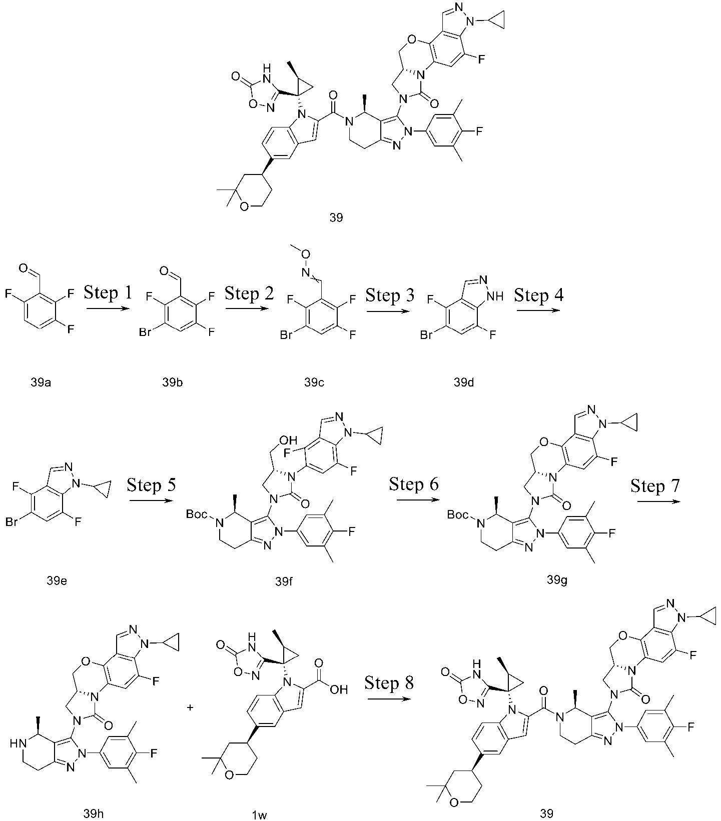

### Step 1

### 3-Bromo-2,5,6-trifluorobenzaldehyde 39b

2,5,6-Trifluorobenzaldehyde **39a** (5.00 g, 31.23 mmol, adamas) was dissolved in concentrated sulfuric acid (15 mL). The mixture was heated to 60°C. *N-*bromosuccinimide (6.67 g, 37.48 mmol) was added at 60°C. The reaction was stirred at 60°C for 5 hours. The reaction solution was poured into 200 mL of ice water and extracted with ethyl acetate (100 mL × 3). The organic phases were combined, washed with a saturated sodium chloride solution, and dried over anhydrous sodium sulfate. After filtration, the filtrate was concentrated under reduced pressure. The residue was purified by silica gel column chromatography with eluent system B to obtain the title compound **39b** (2.10 g, yield: 28%).

### Step 2

### 3-Bromo-2,5,6-trifluorobenzaldehyde O-methyloxime 39c

Compound **39b** (1.90 g, 7.95 mmol) was dissolved in ethylene glycol dimethyl ether (30 mL). Potassium carbonate (1.43 g, 10.35 mmol) and methoxyamine hydrochloride (730 mg, 8.74 mmol). The reaction was stirred at 60°C for 16 hours. After filtration, the filtrate was concentrated under reduced pressure. The residue was purified by silica gel column chromatography with eluent system B to obtain the title compound **39c** (1.20 g, yield: 56%).

### Step 3

### 5-Bromo-4,7-difluoro-1H-indazole 39d

Compound **39c** (1.20 g, 4.48 mmol) was dissolved in tetrahydrofuran (15 mL). Hydrazine hydrate (4.5 mL, 85% wt aqueous solution) was added and stirred at 60°C for 16 hours. The reaction solution was poured into 50 mL of water and extracted with ethyl acetate (30 mL × 3). The organic phases were combined, washed with a saturated sodium chloride solution, and dried over anhydrous sodium sulfate. After filtration, the filtrate was concentrated under reduced pressure. The residue was purified by silica gel column chromatography with eluent system B to obtain the title compound **39d** (900 mg, yield: 86%).

MS m/z (ESI): 230.7 [M-1].

### Step 4

### 5-Bromo-1-cyclopropyl-4,7-difluoro-1H-indazole 39e

Compound **39d** (500 mg, 2.15 mmol), cyclopropylboronic acid (369 mg, 4.30 mmol), 2,2'-bipyridine (670 mg, 4.29 mmol), copper acetate (779 mg, 4.29 mmol), and sodium carbonate (682 mg, 6.43 mmol) were dissolved in 1,2-dichloroethane (30 mL), left open, and stirred at 70°C for 18 hours. The reaction solution was cooled to room temperature, filtered, and concentrated under reduced pressure. The residue was purified by silica gel column chromatography with eluent system B to obtain the title compound **39e** (400 mg, yield: 68%).

### Step 5

### Tert-butyl (S)-3-((R)-3-(1-cyclopropyl-4,7-difluoro-1H-indazol-5-yl)-4-(hydroxymethyl)-2-oxoimidazolidin-1-yl)-2-(4-fluoro-3,5-dimethylphenyl)-4-methyl-2,4,6,7-tetrahydro-5H-pyrazolo[4,3-c]pyridine-5-carboxylate 39f

Compound **13n** (120 mg, 0.25 mmol), compound **39e** (208 mg, 0.76 mmol), and (1*S*,2*S*)-(-)-*N*,*N*-dimethyl-1,2-cyclohexanediamine (23 mg, 0.16 mmol) were dissolved in *N*-methylpyrrolidone (10.0 mL). Potassium carbonate (105 mg, 0.76 mmol) and cuprous iodide (19 mg, 0.10 mmol) were added. After displacement with nitrogen three times, the reaction was stirred in a sealed tube at 120°C for 16 hours. The reaction solution was poured into 50 mL of water and extracted with ethyl acetate (30 mL × 2). The organic phases were combined, washed with a saturated sodium chloride solution, and dried over anhydrous sodium sulfate. After filtration, the filtrate was concentrated under reduced pressure. The residue was purified by silica gel column chromatography with eluent system B to obtain the title compound **39f** (90 mg, yield: 53%).

MS m/z (ESI): 666.4 [M+1].

### Step 6

### Tert-butyl (S)-3-((R)-1-cyclopropyl-11-fluoro-8-oxo-5a,6-dihydro-1H,5H-imidazo[1',5':4,5][1,4]oxazino[2,3-e]indazol-7(8H)-yl)-2-(4-fluoro-3,5-dimethylphenyl)-4-methyl-2,4,6,7-tetrahydro-5H-pyrazolo[4,3-c]pyridine-5-carboxylate 39g

Compound **39f** (90 mg, 0.135 mmol) was dissolved in dimethyl sulfoxide (10.0 mL). Cesium carbonate (132 mg, 0.406 mmol) was added. After displacement with nitrogen three times,the mixture was reacted under stirring at 120°C for 2 hours. The reaction solution was poured into 50 mL of water and extracted with ethyl acetate (30 mL × 2). The organic phases were combined, washed with a saturated sodium chloride solution, and dried over anhydrous sodium sulfate. After filtration, the filtrate was concentrated under reduced pressure. The residue was purified by silica gel column chromatography with eluent system B to obtain the title compound **39g** (87 mg, yield: 100%).

MS m/z (ESI): 646.4 [M+1].

### Step 7

### (R)-1-cyclopropyl-11-fluoro-7-((S)-2-(4-fluoro-3,5-dimethylphenyl)-4-methyl-4,5,6,7-tetrahydro-2H-pyrazolo[4,3-c]pyridin-3-yl)-5,5a,6,7-tetrahydro-1H,8H-imidazo[1',5':4,5][1,4]oxazino[2,3-e]indazol-8-one 39h

Compound **39g** (45 mg, 0.070 mmol) was dissolved in dichloromethane (2 mL). A 4 M solution of hydrogen chloride in 1,4-dioxane (2 mL) was added and stirred at room temperature for 1 hour. The reaction solution was concentrated under reduced pressure to obtain the crude title compound **39h** (38 mg). The product was directly used in the next reaction without purification.

MS m/z (ESI): 546.3 [M+1].

### Step 8

### (R)-1-cyclopropyl-7-((S)-5-(5-((S)-2,2-dimethyltetrahydro-2H-pyran-4-yl)-1-((1S,2S)-2-methyl-1-(5-oxo-4,5-dihydro-1,2,4-oxadiazol-3-yl)cyclopropyl)-1H-indole-2-carbonyl)-2-(4-fluoro-3,5-dimethylphenyl)-4-methyl-4,5,6,7-tetrahydro-2H-pyrazolo[4,3-c]pyridin-3-yl)-11-fluoro-5,5a,6,7-tetrahydro-1H,8H-imidazo[1',5':4,5][1,4]oxazino[2,3-e]indazol-8-one 39

Compound **39h** (38 mg, 0.070 mmol) and compound **1w** (29 mg, 0.070 mmo) were dissolved in *N*,*N*-dimethylformamide (4 mL). *N*,*N-*diisopropylethylamine (46 mg, 0.352 mmol) was added. After the raw materials were fully dissolved, *O*-(7-azabenzotriazol-1-yl)-*N*,*N*,*N*',*N*'-tetramethyluronium hexafluorophosphate (40 mg, 0.106 mmol) was added and stirred at room temperature for 16 hours. After filtration, the residue was purified by preparative high-performance liquid chromatography (instrument model: Waters-2545-2767-2489, chromatographic column: Sharpsil-T, Prep 30 × 250 mm; 5 µm; C18, mobile phase: water (10 mM ammonium bicarbonate) and acetonitrile, with gradient ratio: acetonitrile 45%-65%, flow rate: 30 mL/min) to obtain the title compound **39** (15 mg, yield: 23%).

MS m/z (ESI): 939.6 [M+1].

¹H NMR (500 MHz, CDCl₃): δ 11.30-11.25 (s, 1H), 7.91-7.85 (m, 1H), 7.81-7.56 (m, 2H), 7.51-7.47 (m, 1H), 7.27-7.23 (m, 1H), 7.16-7.12 (m, 2H), 6.69-6.68 (m, 1H), 5.79-4.82 (m, 2H), 4.47-4.33 (m, 2H), 4.17-4.11 (m, 1H), 3.96-3.36 (m, 4H), 3.16-2.94 (m, 4H), 2.30-2.20 (m, 6H), 2.01-1.40 (m, 10H), 1.38-0.87 (m, 14H).

### Example 40

### (R)-2-((S)-5-(5-((S)-2,2-dimethyltetrahydro-2H-pyran-4-yl)-1-((1S,2S)-2-methyl-1-(5-oxo-4,5-dihydro-1,2,4-oxadiazol-3-yl)cyclopropyl)-1H-indole-2-carbonyl)-2-(4-fluoro-3,5-dimethylphenyl)-4-methyl-4,5,6,7-tetrahydro-2H-pyrazolo[4,3-c]pyridin-3-yl)-10-fluoro-7-isopropyl-2,3,3a,4-tetrahydro-1H,7H-imidazo[1',5':4,5][1,4]oxazino[3,2-f]indazol-1-one 40

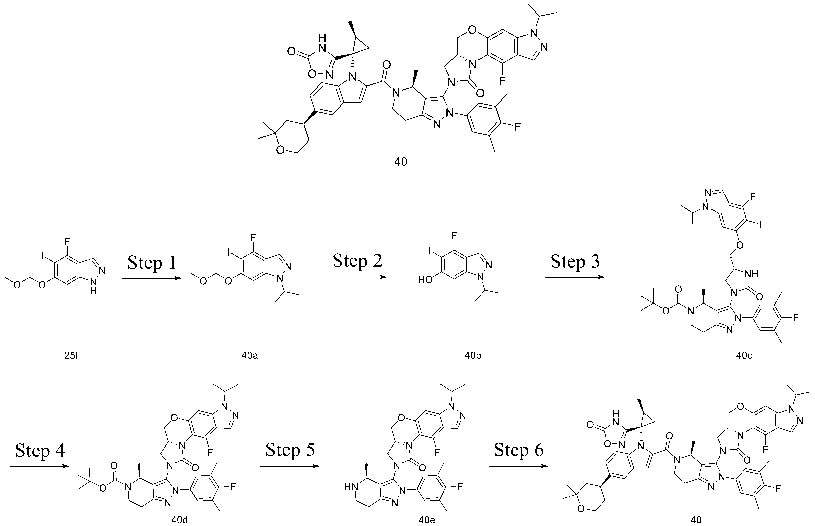

### Step 1

### 4-Fluoro-5-iodo-1-isopropyl-6-(methoxymethoxy)-1H-indazole 40a

Compound 25f (300 mg, 0.93 mmol), 2-iodopropane (314 mg, 1.86mmol), and cesium carbonate (913 mg, 2.79 mmol) were dissolved in *N*,*N*-dimethylacetamide (20 mL). The reaction was heated to 70°C and reacted under stirring for 12 hours. The reaction mixture was cooled to room temperature. 30 mL of water was added. The mixture was extracted with ethyl acetate (30 mL × 3). The organic phases were combined, washed with a saturated aqueous sodium chloride solution, and dried over anhydrous sodium sulfate. After filtration, the filtrate was concentrated under reduced pressure. The residue was purified by silica gel column chromatography with eluent system C to obtain the title compound **40a** (200 mg, yield: 60%).

MS m/z (ESI): 365.1 [M+1].

### Step 2

### 4-Fluoro-5-iodo-1-isopropyl-1H-indazol-6-ol 40b

Compound **40a** (200 mg, 0.55 mmol) was dissolved in dichloromethane (2 mL). A hydrogen chloride/dioxane solution (2 mL, 4.0 M) was dropwise added at 0°C. After the addition was complete, the mixture was stirred at room temperature for 12 hours. After concentration, 30 mL of water was added. The mixture was extracted with ethyl acetate (30 mL × 3). The organic phases were combined, washed with a saturated aqueous sodium chloride solution, and dried over anhydrous sodium sulfate. After filtration, the filtrate was concentrated under reduced pressure. The residue was purified by silica gel column chromatography with eluent system B to obtain the title compound **40b** (110 mg, yield: 62%).

MS m/z (ESI): 320.9 [M+1].

### Step 3

### Tert-butyl (S)-2-(4-fluoro-3,5-dimethylphenyl)-3-((R)-4-(((4-fluoro-5-iodo-1-isopropyl-1H-indazol-6-yl)oxy)methyl)-2-oxoimidazolidin-1-yl)-4-methyl-2,4,6,7-tetrahydro-5H-pyrazolo[4,3-c]pyridine-5-carboxylate 40c

Compound **13o** (180 mg, 0.32 mmol) and compound **40b** (105 mg, 0.32 mmol) were dissolved in *N*,*N-*dimethylformamide (5.0 mL). Potassium carbonate (319 mg, 0.98 mmol) was added and reacted at 80°C for 3 hours. The reaction solution was poured into 20 mL of water and extracted with ethyl acetate (30 mL × 2). The organic phases were combined, washed with a saturated sodium chloride solution, and dried over anhydrous sodium sulfate. After filtration, the filtrate was concentrated under reduced pressure. The residue was purified by silica gel column chromatography with eluent system A to obtain the title compound **40c** (150 mg, yield: 59%).

MS m/z (ESI): 776.3 [M+1].

### Step 4

### Tert-butyl (S)-2-(4-fluoro-3,5-dimethylphenyl)-3-((R)-10-fluoro-7-isopropyl-1-oxo-3a,4-dihydro-1H,7H-imidazo[1',5':4,5][1,4]oxazino[3,2-f]indazol-2(3H)-yl)-4-methyl-2,4,6,7-tetrahydro-5H-pyrazolo[4,3-c]pyridine-5-carboxylate 40d

Compound **40c** (160 mg, 0.21 mmol) and (1*S*,2*S*)-(+)-*N*,*N*'-dimethyl-1,2-cyclohexanediamine (10 mg, 0.09 mmol) were dissolved in *N*-methylpyrrolidone (5.0 mL). Potassium carbonate (77 mg, 0.62 mmol) and cuprous iodide (10 mg, 0.05 mmol) were added. After displacement with nitrogen three times, the mixture was heated to 125°C and reacted under stirring for 3 hours. The reaction solution was poured into 20 mL of water and extracted with ethyl acetate (30 mL × 2). The organic phases were combined, washed with a saturated sodium chloride solution, and dried over anhydrous sodium sulfate. After filtration, the filtrate was concentrated under reduced pressure. The residue was purified by silica gel column chromatography with eluent system C to obtain the title compound **40d** (100 mg, yield: 75%).

MS m/z (ESI): 648.5 [M+1].

### Step 5

### (R)-10-fluoro-2-((S)-2-(4-fluoro-3,5-dimethylphenyl)-4-methyl-4,5,6,7-tetrahydro-2H-pyrazolo[4,3-c]pyridin-3-yl)-7-isopropyl-2,3,3a,4-tetrahydro-1H,7H-imidazo[1',5':4,5][1,4]oxazino[3,2-f]indazol-1-one 40e

Compound **40d** (100 mg, 0.15 mmol) was dissolved in methanol (2 mL). A 4 M solution of hydrogen chloride in 1,4-dioxane (2 mL) was added and stirred at room temperature for 2 hours. The reaction solution was concentrated under reduced pressure to obtain the crude title compound **40e** (70 mg). The product was directly used in the next reaction without purification.

MS m/z (ESI): 548.4 [M+1].

### Step 6

### (R)-2-((S)-5-(5-((S)-2,2-dimethyltetrahydro-2H-pyran-4-yl)-1-((1S,2S)-2-methyl-1-(5-oxo-4,5-dihydro-1,2,4-oxadiazol-3-yl)cyclopropyl)-1H-indole-2-carbonyl)-2-(4-fluoro-3,5-dimethylphenyl)-4-methyl-4,5,6,7-tetrahydro-2H-pyrazolo[4,3-c]pyridin-3-yl)-10-fluoro-7-isopropyl-2,3,3a,4-tetrahydro-1H,7H-imidazo[1',5':4,5][1,4]oxazino[3,2-f]indazol-1-one 40

Compound **40e** (70 mg, 0.12mmol) and compound **1w** (53 mg, 0.12 mmol) were dissolved in *N*,*N*-dimethylformamide (3 mL). *N*,*N*-diisopropylethylamine (50 mg, 0.36 mmol) was added. After the raw materials were fully dissolved, *O*-(7-azabenzotriazol-1-yl)-*N*,*N*,*N*',*N'*-tetramethyluronium hexafluorophosphate(50 mg, 0.13 mmol) was added and stirred at room temperature for 16 hours. The reaction solution was poured into 10 mL of water and extracted with ethyl acetate (10 mL × 3). The organic phases were combined, washed with a saturated sodium chloride solution, and dried over anhydrous sodium sulfate. After filtration, the filtrate was concentrated under reduced pressure. The residue was purified by preparative high-performance liquid chromatography (instrument model: Waters-2545 chromatographic column: Welch Xtimate, Prep 30 × 250 mm; 5 µm; C18, mobile phase: water (10 mM ammonium bicarbonate) and acetonitrile, with gradient ratio: acetonitrile 40%-95%, flow rate: 30 mL/min) to obtain the title compound **40** (50 mg, yield: 41%).

MS m/z (ESI): 941.6 [M+1].

¹H NMR (500 MHz, CDCl₃): δ 7.98 (s, 1H), 7.61(d, 1H), 7.55-7.48 (m, 2H), 7.21-7.15 (m, 2H), 6.77 (s, 1H),6.71(s, 1H), 5.81-5.76 (m, 1H), 4.48-4.42 (m, 1H), 4.29-4.20 (m, 1H), 4.11-3.95 (m, 3H), 3.92-3.76 (m, 3H), 3.71-3.60 (m, 2H), 3.54-3.48(m, 1H), 3.18-2.95(m, 5H), 2.30-2.26 (m, 6H), 1.94 (t, 1H), 1.83-1.55 (m, 7H), 1.40 -1.22 (m, 13H), 1.06 (d, 1H), 0.91(t, 1H).

### Example 41

### 3-((1S,2S)-1-(2-((S)-3-(1-(1-cyclopropyl-1H-indazol-5-yl)-6-oxo-1,6-dihydropyrimidin-5-yl)-2-(4-fluoro-3,5-dimethylphenyl)-4-methyl-4,5,6,7-tetrahydro-2H-pyrazolo[4,3-c]pyridine-5-carbonyl)-5-((S)-2,2-dimethyltetrahydro-2H-pyran-4-yl)-1H-indol-1-yl)-2-methylcyclopropyl)-1,2,4-oxadiazol-5(4H)-one 41

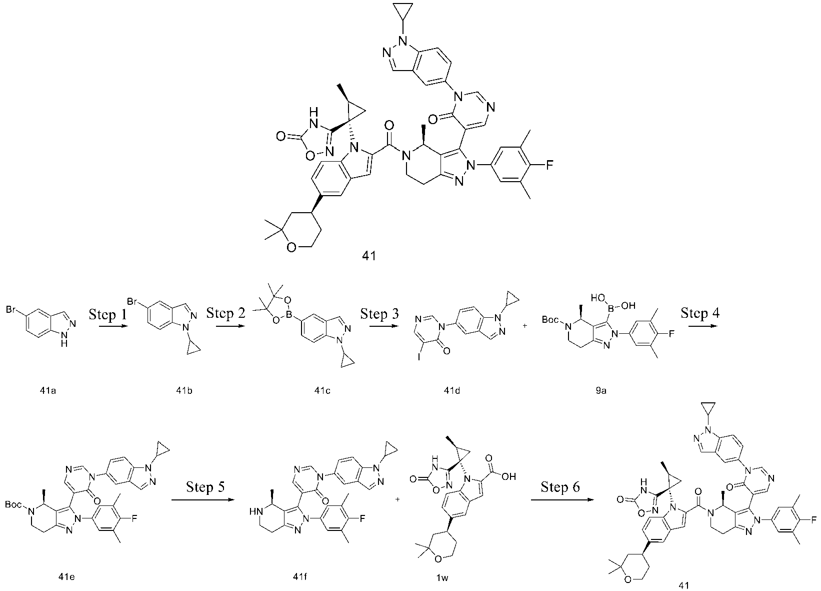

### Step 1

### 5-Bromo-1-cyclopropyl-1H-indazole 41b

5-Bromo-1*H*-indazole **41a** (5.0 g, 25.37 mmol), cyclopropylboronic acid (2.8 g, 32.94 mmol), anhydrous copper acetate (6.9 g, 38.09 µmol), 2,2'-bipyridine (5.9 g, 38.03 mmol), and cesium carbonate (6.7 g, 63.4 mmol) were dissolved in 1,2-dichloroethane (100 mL) and reacted at 70°C for 16 hours. The mixture was quenched with water (100 mL) and extracted with dichloromethane (50 mL × 3). The organic phases were combined and washed with a saturated sodium chloride solution. The organic phase was dried over anhydrous sodium sulfate. After filtration, the filtrate was concentrated under reduced pressure. The residue was purified by silica gel column chromatography with eluent system B to obtain the title compound **41b** (5.0 g, yield: 83.1%).

MS m/z (ESI): 236.9 [M+1].

### Step 2

### 1-Cyclopropyl-5-(4,4,5,5-tetramethyl-1,3,2-dioxaborolan-2-yl)-1H-indazole 41c

Compound **41b** (3.0 g, 12.65 mmol), potassium acetate (2.5 g, 25.27 mmol), bis(pinacolato)diboron (4.8 g, 18.98 mmol), and [1,1'-bis(diphenylphosphino)ferrocene]palladium dichloride (1.85 g, 2.53 mmol) were dissolved in dioxane (100 mL). After displacement with nitrogen three times, the mixture was reacted at 85°C for 16 hours. Water (100 mL) was added. The mixture was extracted with ethyl acetate (60 mL × 3). The organic phases were combined and washed with a saturated sodium chloride solution. The organic phase was dried over anhydrous sodium sulfate. After filtration, the filtrate was concentrated under reduced pressure. The residue was purified by silica gel column chromatography with eluent system B to obtain the title compound **41c** (3.0 g, yield: 99.3%).

MS m/z (ESI): 285.0 [M+1].

### Step 3

### 3-(1-Cyclopropyl-1H-indazol-5-yl)-5-iodopyrimidin-4(3H)-one 41d

4-Hydroxy-5-iodopyrimidine (200 mg, 0.90 mmol, Leyan), compound **41c** (0.38 g, 1.35 mmol), 2,2'-bipyridine (0.28 g, 1.8 mmol), copper acetate (0.33 g, 1.8 mmol), and sodium carbonate (0.28 g, 2.7 mmol) were dissolved in 1,2-dichloroethane (30 mL), left open, and stirred at 70°C for 16 hours. The reaction solution was concentrated under reduced pressure. The residue was purified by silica gel column chromatography with eluent system B to obtain the title compound **41d** (150 mg, yield: 44%).

MS m/z (ESI): 378.9 [M+1].

### Step 4

### Tert-butyl (S)-3-(1-(1-cyclopropyl-1H-indazol-5-yl)-6-oxo-1,6-dihydropyrimidin-5-yl)-2-(4-fluoro-3,5-dimethylphenyl)-4-methyl-2,4,6,7-tetrahydro-5H-pyrazolo[4,3-c]pyridine-5-carboxylate 41e

Compound **41d** (140 mg, 0.37 mmol), compound **9a** (220 mg, 0.55 mmol), anhydrous potassium carbonate (153 mg, 1.11 mmol), and [1,1'-bis(di-tert-butylphosphino)ferrocene]palladium dichloride (50.5 mg, 0.07 mmol, Bidepharm) were dissolved in dioxane (20 mL) and water (4 mL). After displacement with nitrogen, the reaction was stirred at 70°C for 3 hours. The reaction solution was cooled to room temperature and concentrated under reduced pressure. The residue was purified by silica gel column chromatography with eluent system A to obtain the crude title compound **41e** (130 mg, yield: 57.6%).

MS m/z (ESI): 610.4 [M+1].

### Step 5

### (S)-3-(1-cyclopropyl-1H-indazol-5-yl)-5-(2-(4-fluoro-3,5-dimethylphenyl)-4-methyl-4,5,6,7-tetrahydro-2H-pyrazolo[4,3-c]pyridin-3-yl)pyrimidin-4(3H)-one 41f

Compound **41e** (38 mg, 62.33 µmol) was dissolved in ethyl acetate (2 mL). A 4 M solution of hydrogen chloride in 1,4-dioxane (2 mL) was added and stirred at room temperature for 2 hours. The reaction solution was concentrated under reduced pressure to obtain the crude title compound **41f** (40 mg). The product was directly used in the next reaction without purification.

MS m/z (ESI): 510.3 [M+1].

### Step 6

### 3-((1S,2S)-1-(2-((S)-3-(1-(1-cyclopropyl-1H-indazol-5-yl)-6-oxo-1,6-dihydropyrimidin-5-yl)-2-(4-fluoro-3,5-dimethylphenyl)-4-methyl-4,5,6,7-tetrahydro-2H-pyrazolo[4,3-c]pyridine-5-carbonyl)-5-((S)-2,2-dimethyltetrahydro-2H-pyran-4-yl)-1H-indol-1-yl)-2-methylcyclopropyl)-1,2,4-oxadiazol-5(4H)-one 41

Compound **41f** (31 mg, 0.06 mmol) and compound **1w** (30 mg, 0.073 mmol) were dissolved in *N*,*N*-dimethylformamide (5 mL). *N*,*N*-diisopropylethylamine (24 mg, 0.19 mmol) was added. After the raw materials were fully dissolved, *O*-(7-azabenzotriazol-1-yl)-*N*,*N*,*N*',*N*'-tetramethyluronium hexafluorophosphate (46 mg, 0.12 mmol) was added and stirred at room temperature for 16 hours. The reaction solution was poured into 10 mL of water and extracted with ethyl acetate (10 mL × 3). The organic phases were combined, washed with a saturated sodium chloride solution, and dried over anhydrous sodium sulfate. After filtration, the filtrate was concentrated under reduced pressure. The residue was purified by preparative high-performance liquid chromatography (instrument model: Gilson-GX-281, chromatographic column: Boston Phlex, Prep 30 × 150 mm; 5 µm; C18, mobile phase: water (10 mM ammonium bicarbonate) and acetonitrile, with gradient ratio: acetonitrile 40%-70%, flow rate: 30 mL/min) to obtain the title compound **41** (5 mg, yield: 9.1%).

MS m/z (ESI): 903.6 [M+1].

¹H NMR (500 MHz, CDCl₃): δ 11.16 (br, 1H), 8.30-8.17 (m, 1H), 8.04-7.72 (m, 3H), 7.66-7.24 (m, 5H), 7.08-6.98 (m, 2H), 6.69-6.66 (m, 1H), 5.83-5.79 (m, 1H), 5.35-4.84 (m, 2H), 4.45-4.41 (m, 1H), 3.89-3.80 (m, 2H), 3.67-3.57 (m, 2H), 3.19-2.99 (m, 4H), 2.27-2.17 (m, 6H), 2.04-1.89 (m, 2H), 1.78-1.47 (m, 6H), 1.35-0.87 (m, 12H).

### Example 42

### 3-((1S,2S)-1-(2-((S)-2-(3-cyclopropyl-4-fluorophenyl)-4-methyl-3-(1-(1-methyl-1H-indazol-5-yl)-6-oxo-1,6-dihydropyrimidin-5-yl)-4,5,6,7-tetrahydro-2H-pyrazolo[4,3-c]pyridine-5-carbonyl)-5-((S)-2,2-dimethyltetrahydro-2H-pyran-4-yl)-1H-indol-1-yl)-2-methylcyclopropyl)-1,2,4-oxadiazol-5(4H)-one 42

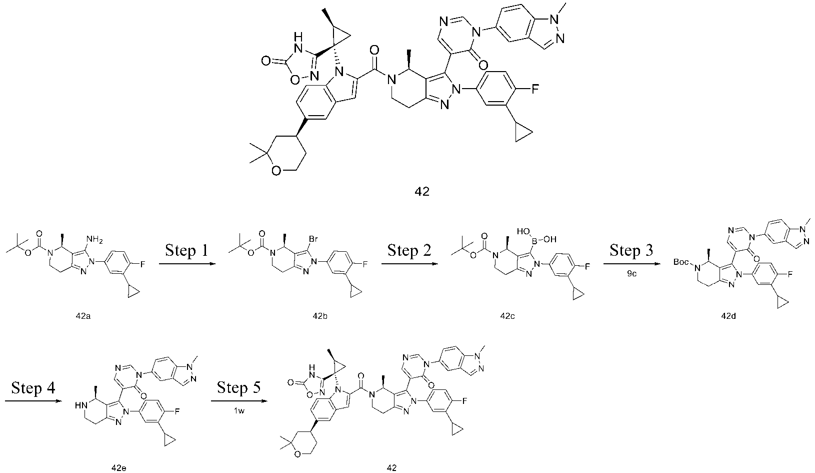

### Step 1

### Tert-butyl (S)-3-bromo-2-(3-cyclopropyl-4-fluorophenyl)-4-methyl-2,4,6,7-tetrahydro-5H-pyrazolo[4,3-c]pyridine-5-carboxylate 42b

Tert-butyl (*S*)-3-amino-2-(3-cyclopropyl-4-fluorophenyl)-4-methyl-2,4,6,7-tetrahydro-5*H*-pyrazolo[4,3-*c*]pyridine-5-carboxylate **42a** (500 mg, 1.29 mmol, prepared by a method for intermediate 1-E in an example on page 9 of the description of Patent Application CN 117447493 A) was dissolved in acetonitrile (20 mL). Cuprous bromide (650 mg, 4.53 mmol) was added. Isoamyl nitrite (600 mg, 4.61 mmol) was dropwise added at room temperature. The mixture was reacted under stirring at room temperature for 1 hour. The reaction solution was poured into 20 mL of an ammonium chloride solution and extracted with ethyl acetate (40 mL × 2). The organic phases were combined, washed with a saturated sodium chloride solution, and dried over anhydrous sodium sulfate. After filtration, the filtrate was concentrated under reduced pressure. The residue was purified by silica gel column chromatography with eluent system C to obtain the title compound **42b** (460 mg, yield: 78.9%).

MS m/z (ESI): 450.3 [M+1].

### Step 2

### (S)-(5-(tert-butoxycarbonyl)-2-(3-cyclopropyl-4-fluorophenyl)-4-methyl-4,5,6,7-tetrahydro-2H-pyrazolo[4,3-c]pyridin-3-yl)boronic acid 42c

Compound **42b** (400 mg, 0.89 mmol) was dissolved in tetrahydrofuran (10 mL). After displacement with nitrogen, n-butyllithium (0.54 mL, 2.5M) was dropwise added at -78°C and stirred for 1 hour, followed by the addition of trimethyl borate (277 mg, 2.67 mmol). The reaction was further stirred at -78°C for 0.5 hours. The reaction solution was quenched with 20 mL of a saturated aqueous ammonium chloride solution and extracted with ethyl acetate (30 mL × 3). The organic phases were combined and dried over anhydrous sodium sulfate. After filtration, the filtrate was concentrated under reduced pressure. The residue was purified by silica gel column chromatography with eluent system A to obtain the title compound **42c** (300 mg, yield: 81.3%).

MS m/z (ESI): 416.1 [M+1].

### Step 3

### Tert-butyl (S)-2-(3-cyclopropyl-4-fluorophenyl)-4-methyl-3-(1-(1-methyl-1H-indazol-5-yl)-6-oxo-1,6-dihydropyrimidin-5-yl)-2,4,6,7-tetrahydro-5H-pyrazolo[4,3-c]pyridine-5-carboxylate 42d

Compound **42c** (140 mg, 0.34 mmol), compound **9c** (70 mg, 0.20 mmol), anhydrous potassium carbonate (83 mg, 0.60 mmol), and 1,1'-bis(di-tert-butylphosphino)ferrocenepalladium dichloride (70 mg, 0.10 mmol) were dissolved in dioxane (50 mL) and water (10 mL). After displacement with nitrogen, the reaction was stirred at 70°C for 5 hours. The reaction solution was cooled to room temperature and concentrated under reduced pressure. The residue was purified by silica gel column chromatography with eluent system A to obtain the title compound **42d** (100 mg, yield: 84.4%).

MS m/z (ESI): 596.5 [M+1].

### Step 4

### (S)-5-(2-(3-cyclopropyl-4-fluorophenyl)-4-methyl-4,5,6,7-tetrahydro-2H-pyrazolo[4,3-c]pyridin-3-yl)-3-(1-methyl-1H-indazol-5-yl)pyrimidin-4(3H)-one 42e

Compound **42d** (100 mg, 0.17 mmol) was dissolved in dichloromethane (2 mL). A 4 M solution of hydrogen chloride in 1,4-dioxane (3 mL) was added and stirred at room temperature for 1 hour. The reaction solution was concentrated under reduced pressure to obtain the crude title compound **42e** (83 mg). The product was directly used in the next reaction without purification.

MS m/z (ESI): 496.4 [M+1].

### Step 5

### 3-((1S,2S)-1-(2-((S)-2-(3-cyclopropyl-4-fluorophenyl)-4-methyl-3-(1-(1-methyl-1H-indazol-5-yl)-6-oxo-1,6-dihydropyrimidin-5-yl)-4,5,6,7-tetrahydro-2H-pyrazolo[4,3-c]pyridine-5-carbonyl)-5-((S)-2,2-dimethyltetrahydro-2H-pyran-4-yl)-1H-indol-1-yl)-2-methylcyclopropyl)-1,2,4-oxadiazol-5(4H)-one 42

Compound **42e** (83 mg, 0.17mmol) and compound **1w** (100 mg, 0.024 mmol) were dissolved in *N*,*N*-dimethylformamide (5 mL). *N,*N-diisopropylethylamine (100 mg, 0.85 mmol) was added. After the raw materials were fully dissolved, *O*-(7-azabenzotriazol-1-yl)-*N*,*N*,*N*',*N*'-tetramethyluronium hexafluorophosphate (100 mg, 0.26 mmol) was added and stirred at room temperature for 16 hours. The reaction solution was poured into 10 mL of water and extracted with ethyl acetate (10 mL × 3). The organic phases were combined, washed with a saturated sodium chloride solution, and dried over anhydrous sodium sulfate. After filtration, the filtrate was concentrated under reduced pressure. The residue was purified by preparative high-performance liquid chromatography (instrument model: Gilson-GX-281, chromatographic column: Boston phlex, Prep 30 × 150 mm; 5 µm; C18, mobile phase: water (10 mM ammonium bicarbonate) and acetonitrile, with gradient ratio: acetonitrile 40%-50%, flow rate: 30 mL/min) to obtain the title compound **42** (50 mg, yield: 33.6%).

MS m/z (ESI): 889.2 [M+1].

¹H NMR (500 MHz, CDCl₃): δ 11.92-10.81 (m, 1H), 8.40-7.47 (m, 6H), 7.46-7.27 (m, 2H), 7.20-6.60 (m, 5H), 5.85-5.18 (m, 1H), 4.92-4.36 (m, 1H), 4.18-4.04 (m, 3H), 3.95-3.75 (m, 2H), 3.69-3.38 (m, 1H), 3.24-2.87 (m, 3H), 2.26-1.69 (m, 7H), 1.50-1.43 (m, 3H), 1.39-1.31 (m, 5H), 1.23-1.12 (m, 3H), 1.07-0.86 (m, 4H), 0.75-0.56 (m, 2H).

### Example 43

### 3-((1S,2S)-1-(5-((S)-2,2-dimethyltetrahydro-2H-pyran-4-yl)-2-((S)-2-(4-fluoro-3,5-dimethylphenyl)-4-methyl-3-(1-(1-methyl-1H-indazol-6-yl)-6-oxo-1,6-dihydropyrimidin-5-yl)-4,5,6,7-tetrahydro-2H-pyrazolo[4,3-c]pyridine-5-carbonyl)-1H-indol-1-yl)-2-methylcyclopropyl)-1,2,4-oxadiazol-5(4H)-one 43

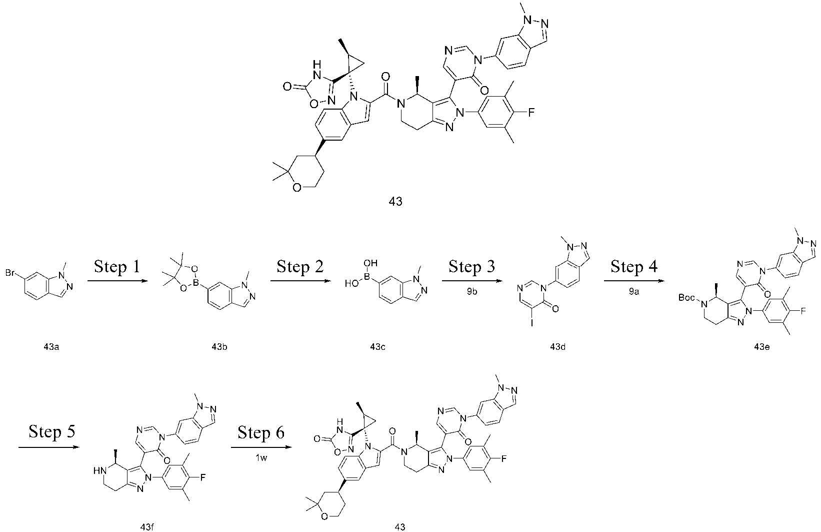

### Step 1

### 1-Methyl-6-(4,4,5,5-tetramethyl-1,3,2-dioxaborolan-2-yl)-1H-indazole 43b

6-Bromo-1-methyl-1*H*-indazole **43a** (2.0 g, 11.42 mmol), bis(pinacolato)diboron (2.9 g, 11.42 mmol), potassium acetate (1.4 g, 14.26 mmol), and [1,1'-bis(diphenylphosphino)ferrocene]palladium(II) dichloride (700 mg, 1.00 mmol, Adamas) were dissolved in dioxane (40 mL). After displacement with nitrogen, the mixture was reacted under stirring at 100°C for 6 hours. The reaction solution was cooled to room temperature and concentrated under reduced pressure. The residue was purified by silica gel column chromatography with eluent system C to obtain the title compound **43b** (2.4 g, yield: 98.1%).

MS m/z (ESI): 259.1 [M+1].

### Step 2

### (1-Methyl-1H-indazol-6-yl)boronic acid 43c

Compound **43b** (2.0 g, 7.75 mmol) was dissolved in acetone (30 mL) and water (30 mL). Sodium periodate (5.0 g, 23.38 mmol) and ammonium acetate (1.8 g, 23.35 mmol) were added. The reaction was stirred at room temperature overnight. The reaction solution was filtered. The filtrate was concentrated under reduced pressure to remove the organic solvent and extracted with ethyl acetate (50 mL × 3). The organic phases were combined. The combined organic phase was dried over anhydrous sodium sulfate. After filtration, the filtrate was concentrated under reduced pressure. The residue was purified by silica gel column chromatography with eluent system A to obtain the title compound **43c** (1.2 g, yield: 88.0%).

MS m/z (ESI): 177.1 [M+1].

### Step 3

### 5-Iodo-3-(1-methyl-1H-indazol-6-yl)pyrimidin-4(3H)-one 43d

Compound **9b** (500 mg, 2.25 mmol), compound **43c** (600 mg, 3.41 mmol), pyridine (1.8 g, 22.76 mmol), and copper acetate (900 mg, 4.51 mmol) were dissolved in 1,2-dichloroethane (20 mL), left open, and stirred at room temperature for 16 hours. The reaction solution was concentrated under reduced pressure. The residue was purified by silica gel column chromatography with eluent system A to obtain a crude product, which was further slurried with dichloromethane to obtain the title compound **43d** (270 mg, yield: 34.0%).

MS m/z (ESI): 352.9 [M+1].

### Step 4

### Tert-butyl (S)-2-(4-fluoro-3,5-dimethylphenyl)-4-methyl-3-(1-(1-methyl-1H-indazol-6-yl)-6-oxo-1,6-dihydropyrimidin-5-yl)-2,4,6,7-tetrahydro-5H-pyrazolo[4,3-c]pyridine-5-carboxylate 43e

Compound **43d** (100 mg, 0.28 mmol), compound **9a** (200 mg, 0.50 mmol), anhydrous potassium carbonate (118 mg, 0.85 mmol), and 1,1'-bis(di-tert-butylphosphino)ferrocenepalladium dichloride (100 mg, 0.15 mmol) were dissolved in dioxane (80 mL) and water (16 mL). After displacement with nitrogen, the reaction was stirred at 70°C for 6 hours. The reaction solution was cooled to room temperature and concentrated under reduced pressure. The residue was purified by silica gel column chromatography with eluent system A to obtain the title compound **43e** (110 mg, yield: 66.4%).

MS m/z (ESI): 584.4 [M+1].

### Step 5

### (S)-5-(2-(4-fluoro-3,5-dimethylphenyl)-4-methyl-4,5,6,7-tetrahydro-2H-pyrazolo[4,3-c]pyridin-3-yl)-3-(1-methyl-1H-indazol-6-yl)pyrimidin-4(3H)-one 43f

Compound **43e** (110 mg, 0.19 mmol) was dissolved in dichloromethane (2 mL). A 4 M solution of hydrogen chloride in 1,4-dioxane (5 mL) was added and stirred at room temperature for 1 hour. The reaction solution was concentrated under reduced pressure to obtain the crude title compound **43f** (91 mg). The product was directly used in the next reaction without purification.

MS m/z (ESI): 484.5 [M+1].

### Step 6

### 3-((1S,2S)-1-(5-((S)-2,2-dimethyltetrahydro-2H-pyran-4-yl)-2-((S)-2-(4-fluoro-3,5-dimethylphenyl)-4-methyl-3-(1-(1-methyl-1H-indazol-6-yl)-6-oxo-1,6-dihydropyrimidin-5-yl)-4,5,6,7-tetrahydro-2H-pyrazolo[4,3-c]pyridine-5-carbonyl)-1H-indol-1-yl)-2-methylcyclopropyl)-1,2,4-oxadiazol-5(4H)-one 43

Compound **43f** (91 mg, 0.19 mmol) and compound **1w** (116 mg, 0.28 mmol) were dissolved in *N*,*N-*dimethylformamide (5 mL). *N*,*N*-diisopropylethylamine (122 mg, 0.94 mmol) was added. After the raw materials were fully dissolved, *O*-(7-azabenzotriazol-1-yl)-*N*,*N*,*N*',*N*'-tetramethyluronium hexafluorophosphate (108 mg, 0.28 mmol) was added and stirred at room temperature for 16 hours. The reaction solution was poured into 10 mL of water and extracted with ethyl acetate (10 mL × 3). The organic phases were combined, washed with a saturated sodium chloride solution, and dried over anhydrous sodium sulfate. After filtration, the filtrate was concentrated under reduced pressure. The residue was purified by preparative high-performance liquid chromatography (instrument model: Gilson-GX-281, chromatographic column: Boston phlex, Prep 30 × 150 mm; 5 µm; C18, mobile phase: water (10 mM ammonium bicarbonate) and acetonitrile, with gradient ratio: acetonitrile 35%-50%, flow rate: 30 mL/min) to obtain the title compound **43** (45 mg, yield: 27.3%).

MS m/z (ESI): 877.5 [M+1].

¹H NMR (500 MHz, CDCl₃): δ 11.84-10.59 (m, 1H), 8.36-8.15 (m, 1H), 8.13-8.00 (m, 1H), 7.98-7.34 (m, 5H), 7.25-7.14 (m, 1H), 7.12-6.52 (m, 4H), 5.85-5.19 (m, 1H), 4.89-4.38 (m, 1H), 4.18-4.00 (m, 3H), 3.93-3.77 (m, 2H), 3.66-3.37 (m, 1H), 3.22-2.93 (m, 3H), 2.32-2.18 (m, 6H), 1.90 (t, 1H), 1.82-1.51 (m, 9H), 1.49 (d, 2H), 1.39-1.30 (m, 4H), 1.19 (d, 2H), 1.01 (d, 1H).

### Example 44

### (R)-9-cyclopropyl-2-((S)-5-(5-((S)-2,2-dimethyltetrahydro-2H-pyran-4-yl)-1-((1S,2S)-2-methyl-1-(5-oxo-4,5-dihydro-1,2,4-oxadiazol-3-yl)cyclopropyl)-1H-indole-2-carbonyl)-2-(4-fluoro-3,5-dimethylphenyl)-4-methyl-4,5,6,7-tetrahydro-2H-pyrazolo[4,3-c]pyridin-3-yl)-2,3,3a,4-tetrahydro-1H,9H-imidazo[1',5':4,5][1,4]oxazino[2,3-f]indazol-1-one 44

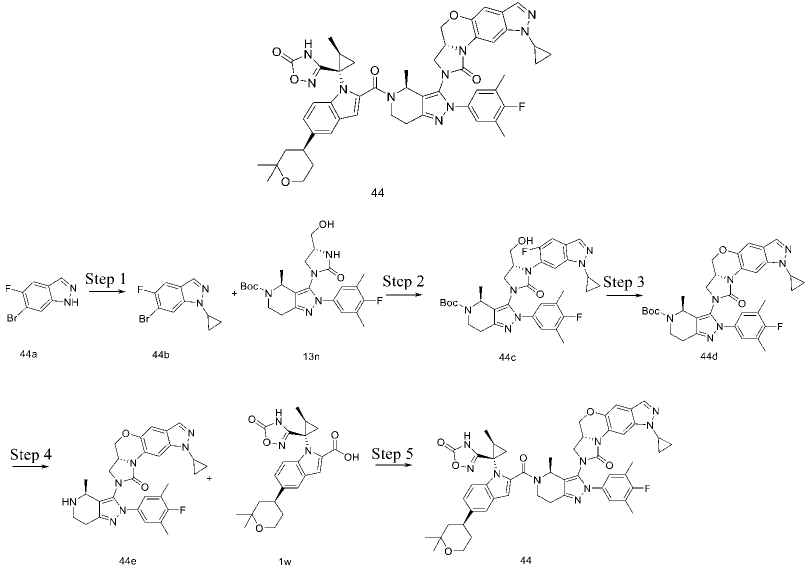

### Step 1

### 6-Bromo-1-cyclopropyl-5-fluoro-1H-indazole 44b

6-Bromo-5-fluoro-1*H*-indazole **44a** (1.00 g, 4.65 mmol, adamas), cyclopropylboronic acid (799 mg, 9.30 mmol), 2,2'-bipyridine (1.45 mg, 9.30 mmol), copper acetate (1.69 mg, 9.30 mmol), and sodium carbonate(1.48 mg, 13.95 mmol) were dissolved in 1,2-dichloroethane (30 mL), left open, and stirred at 70°C for 18 hours. The reaction solution was cooled to room temperature, filtered, and concentrated under reduced pressure. The residue was purified by silica gel column chromatography with eluent system B to obtain the title compound **44b** (1.10 g, yield: 93%).

MS m/z (ESI): 255.0 [M+1].

### Step 2

### Tert-butyl (S)-3-((R)-3-(1-cyclopropyl-5-fluoro-1H-indazol-6-yl)-4-(hydroxymethyl)-2-oxoimidazolidin-1-yl)-2-(4-fluoro-3,5-dimethylphenyl)-4-methyl-2,4,6,7-tetrahydro-5H-pyrazolo[4,3-c]pyridine-5-carboxylate 44c

Compound **13n** (100 mg, 0.21 mmol), compound **44b** (162 mg, 0.63 mmol), and (1*S*,2*S*)-(-)-*N*,*N*-dimethyl-1,2-cyclohexanediamine (24 mg, 0.17 mmol) were dissolved in *N*,*N*-dimethylformamide (5.0 mL). Potassium carbonate (87 mg, 0.63 mmol) and cuprous iodide (16 mg, 0.084 mmol) were added. After displacement with nitrogen three times, the reaction was stirred at 120°C for 16 hours. The reaction solution was poured into 50 mL of water and extracted with ethyl acetate (30 mL × 2). The organic phases were combined, washed with a saturated sodium chloride solution, and dried over anhydrous sodium sulfate. After filtration, the filtrate was concentrated under reduced pressure. The residue was purified by silica gel column chromatography with eluent system B to obtain the title compound **44c** (30 mg, yield: 22%).

MS m/z (ESI): 648.4 [M+1].

### Step 3

### Tert-butyl (S)-3-((R)-9-cyclopropyl-1-oxo-3a,4-dihydro-1H,9H-imidazo[1',5':4,5][1,4]oxazino[2,3-f]indazol-2(3H)-yl)-2-(4-fluoro-3,5-dimethylphenyl)-4-methyl-2,4,6,7-tetrahydro-5H-pyrazolo[4,3-c]pyridine-5-carboxylate 44d

Compound **44c** (30 mg, 0.045 mmol) was dissolved in dimethyl sulfoxide (5.0 mL). Cesium carbonate (44 mg, 0.135 mmol) was added. After displacement with nitrogen three times,the mixture was reacted under stirring at 120°C for 2 hours. The reaction solution was poured into 50 mL of water and extracted with ethyl acetate (30 mL × 2). The organic phases were combined, washed with a saturated sodium chloride solution, and dried over anhydrous sodium sulfate. After filtration, the filtrate was concentrated under reduced pressure. The residue was purified by silica gel column chromatography with eluent system B to obtain the title compound **44d** (10 mg, yield: 35%).

MS m/z (ESI): 628.4 [M+1].

### Step 4

### (R)-9-cyclopropyl-2-((S)-2-(4-fluoro-3,5-dimethylphenyl)-4-methyl-4,5,6,7-tetrahydro-2H-pyrazolo[4,3-c]pyridin-3-yl)-2,3,3a,4-tetrahydro-1H,9H-imidazo[1',5':4,5][1,4]oxazino[2,3-f]indazol-1-one 44e

Compound **44d** (6 mg, 0.010 mmol) was dissolved in dichloromethane (4 mL). A 4 M solution of hydrogen chloride in 1,4-dioxane (4 mL) was added and stirred at room temperature for 1 hours. The reaction solution was concentrated under reduced pressure to obtain the crude title compound **44e** (5 mg). The product was directly used in the next reaction without purification.

MS m/z (ESI): 528.2 [M+1].

### Step 5

### (R)-9-cyclopropyl-2-((S)-5-(5-((S)-2,2-dimethyltetrahydro-2H-pyran-4-yl)-1-((1S,2S)-2-methyl-1-(5-oxo-4,5-dihydro-1,2,4-oxadiazol-3-yl)cyclopropyl)-1H-indole-2-carbonyl)-2-(4-fluoro-3,5-dimethylphenyl)-4-methyl-4,5,6,7-tetrahydro-2H-pyrazolo[4,3-c]pyridin-3-yl)-2,3,3a,4-tetrahydro-1H,9H-imidazo[1',5':4,5][1,4]oxazino[2,3-f]indazol-1-one 44

Compound **44e** (5 mg, 0.010 mmol) and compound **1w** (4 mg, 0.010 mmo) were dissolved in *N*,*N*-dimethylformamide (5 mL). *N*,*N*-diisopropylethylamine (6 mg, 0.049 mmol) was added. After the raw materials were fully dissolved, *O*-(7-azabenzotriazol-1-yl)-*N*,*N*,*N*',*N*'-tetramethyluronium hexafluorophosphate (6 mg, 0.014 mmol) was added and stirred at room temperature for 16 hours. After filtration, the residue was purified by preparative high-performance liquid chromatography (instrument model: Waters-2545 chromatographic column: Welch Xtimate, Prep 30 × 150 mm; 5 µm; C18, mobile phase: water (10 mM ammonium bicarbonate) and acetonitrile, with gradient ratio: acetonitrile 25%-65%, flow rate: 30 mL/min) to obtain the title compound **44** (5 mg, yield: 56%).

MS m/z (ESI): 921.4 [M+1].

¹H NMR (500 MHz, CDCl₃): δ 11.32 (s, 1H), 8.42-8.13 (m, 1H), 7.82-7.74 (m, 1H), 7.59-7.14 (m, 2H), 7.26-7.15 (m, 4H), 6.69-6.64 (m, 1H), 5.82-4.80 (m, 2H), 4.47-4.10 (m, 2H), 3.87-2.95 (m, 9H), 2.33-2.20 (m, 6H), 2.01-1.46 (m, 10H), 1.43-0.85 (m, 14H).

### Example 45

### 3-((1S,2S)-1-(5-((S)-2,2-dimethyltetrahydro-2H-pyran-4-yl)-2-((S)-2-(4-fluoro-3,5-dimethylphenyl)-4-methyl-3-(1-(1-methyl-1H-indazol-7-yl)-6-oxo-1,6-dihydropyrimidin-5-yl)-4,5,6,7-tetrahydro-2H-pyrazolo[4,3-c]pyridine-5-carbonyl)-1H-indol-1-yl)-2-methylcyclopropyl)-1,2,4-oxadiazol-5(4H)-one 45

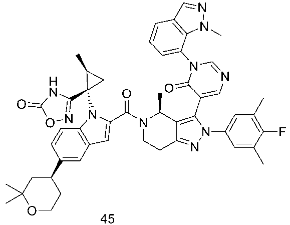

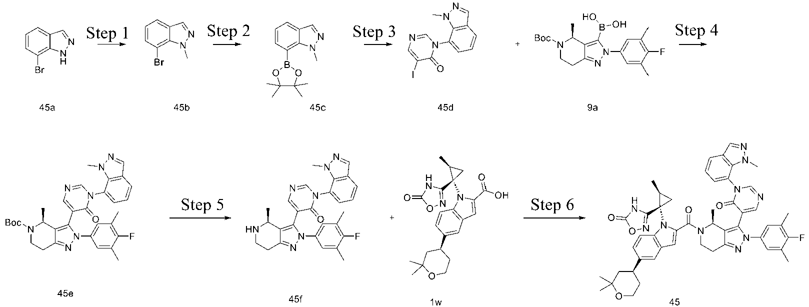

### Step 1

### 7-Bromo-1-methyl-1H-indazole 45b

7-Bromo-1*H*-indazole **45a** (3.0 g, 15.22 mmol) was dissolved in *N,N-*dimethylformamide (50 mL). Sodium hydride (0.9 g, 22.84 mmol, 60% dispersed in a mineral oil) was added at 0°C. Stirring was continued at 0°C 1 hour. Iodomethane (2.59 g, 18.25 mmol, 1.14 mL) was added. The reaction was continued for 16 hours. The mixture was quenched with water (100 mL) and extracted with dichloromethane (50 mL × 3). The organic phases were combined and washed with a saturated sodium chloride solution. The organic phase was dried over anhydrous sodium sulfate. After filtration, the filtrate was concentrated under reduced pressure. The residue was purified by silica gel column chromatography with eluent system B to obtain the title compound **45b** (2.2 g, yield: 68.4%).

MS m/z (ESI): 211.0 [M+1].

### Step 2

### 1-Methyl-7-(4,4,5,5-tetramethyl-1,3,2-dioxaborolan-2-yl)-1H-indazole 45c

Compound **45b** (2.2 g, 10.42 mmol), potassium acetate (2.3 g, 22.93 mmol), bis(pinacolato)diboron (3.9 g, 15.63 mmol), and [1,1'-bis(diphenylphosphino)ferrocene]palladium dichloride (1.52 g, 2.08 mmol) were dissolved in dioxane (100 mL). After displacement with nitrogen three times, the mixture was reacted at 85°C for 16 hours. Water (100 mL) was added. The mixture was extracted with ethyl acetate (60 mL × 3). The organic phases were combined and washed with a saturated sodium chloride solution. The organic phase was dried over anhydrous sodium sulfate. After filtration, the filtrate was concentrated under reduced pressure. The residue was purified by silica gel column chromatography with eluent system B to obtain the title compound **45c** (2.0 g, yield: 74.3%).

MS m/z (ESI): 258.9 [M+1].

### Step 3

### 5-Iodo-3-(1-methyl-1H-indazol-7-yl)pyrimidin-4(3H)-one 45d

4-Hydroxy-5-iodopyrimidine (559 mg, 2.51 mmol), compound **45c** (650 mg, 2.51 mmol), 2,2'-bipyridine (0.78 g, 5.03 mmol), copper acetate (1.0 g, 5.06 mmol), and sodium carbonate (0.8 g, 7.55 mmol) were dissolved in 1,2-dichloroethane (30 mL), left open, and stirred at 70°C for 16 hours. The reaction solution was concentrated under reduced pressure. The residue was purified by silica gel column chromatography with eluent system B to obtain the title compound **45d** (50 mg, yield: 5.6%).

MS m/z (ESI): 353.0 [M+1].

### Step 4

### Tert-butyl (S)-2-(4-fluoro-3,5-dimethylphenyl)-4-methyl-3-(1-(1-methyl-1H-indazol-7-yl)-6-oxo-1,6-dihydropyrimidin-5-yl)-2,4,6,7-tetrahydro-5H-pyrazolo[4,3-c]pyridine-5-carboxylate 45e

Compound **45d** (50 mg, 0.14 mmol), compound **9a** (114 mg, 0.28 mmol), anhydrous potassium carbonate (59 mg, 0.43 mmol), and [1,1'-bis(di-tert-butylphosphino)ferrocene]palladium dichloride (19 mg, 0.03 mmol) were dissolved in dioxane (20 mL) and water (4 mL). After displacement with nitrogen, the reaction was stirred at 70°C for 3 hours. The reaction solution was cooled to room temperature and concentrated under reduced pressure. The residue was purified by silica gel column chromatography with eluent system A to obtain the crude title compound **45e** (20 mg, yield: 24.1%).

MS m/z (ESI): 584.5 [M+1].

### Step 5

### (S)-5-(2-(4-fluoro-3,5-dimethylphenyl)-4-methyl-4,5,6,7-tetrahydro-2H-pyrazolo[4,3-c]pyridin-3-yl)-3-(1-methyl-1H-indazol-7-yl)pyrimidin-4(3H)-one 45f

Compound **45e** (20 mg, 34.26 µmol) was dissolved in ethyl acetate (2 mL). A 4 M solution of hydrogen chloride in 1,4-dioxane (2 mL) was added and stirred at room temperature for 2 hours. The reaction solution was concentrated under reduced pressure to obtain the crude title compound **45f** (17 mg). The product was directly used in the next reaction without purification.

MS m/z (ESI): 484.1 [M+1].

### Step 6 3-((1S,2S)-1-(5-((S)-2,2-dimethyltetrahydro-2H-pyran-4-yl)-2-((S)-2-(4-fluoro-3,5-dimethylphenyl)-4-methyl-3-(1-(1-methyl-1H-indazol-7-yl)-6-oxo-1,6-dihydropyrimidin-5-yl)-4,5,6,7-tetrahydro-2H-pyrazolo[4,3-c]pyridine-5-carbonyl)-1H-indol-1-yl)-2-methylcyclopropyl)-1,2,4-oxadiazol-5(4H)-one 45

Compound **45f (17** mg, 42.18 µmol) and compound **1w** (17 mg, 35.15 µmol) were dissolved in *N*,*N*-dimethylformamide (5 mL). *N*,*N*-diisopropylethylamine (13 mg, 0.11 mmol) was added. After the raw materials were fully dissolved, *O*-(7-azabenzotriazol-1-yl)-*N*,*N*,*N*',*N*'-tetramethyluronium hexafluorophosphate (26 mg, 0.07 mmol) was added and stirred at room temperature for 16 hours. The reaction solution was poured into 10 mL of water and extracted with ethyl acetate (10 mL × 3). The organic phases were combined, washed with a saturated sodium chloride solution, and dried over anhydrous sodium sulfate. After filtration, the filtrate was concentrated under reduced pressure. The residue was purified by preparative high-performance liquid chromatography (instrument model: Gilson-GX-281, chromatographic column: Boston Phlex, Prep 30 × 150 mm; 5 µm; C18, mobile phase: water (10 mM ammonium bicarbonate) and acetonitrile, with gradient ratio: acetonitrile 40%-75%, flow rate: 30 mL/min) to obtain the title compound **45** (5 mg, yield: 9.1%).

MS m/z (ESI): 875.6 [M-1].

¹H NMR (500 MHz, CDCl₃): δ 11.22 (s, 1H), 8.79 (s, 1H), 8.61 (s, 1H), 8.32 (s, 1H), 7.97 (s, 1H), 7.90 (s, 1H), 7.62-7.58 (m, 1H), 7.50 (s, 1H), 7.27-7.23 (m, 2H), 6.97-6.89 (m, 2H), 6.73-6.70 (m, 1H), 5.94-5.90 (m, 1H), 5.75-5.59 (m, 2H), 5.36-5.34 (m, 1H), 4.90-4.88 (m, 1H), 4.53-4.50 (m, 1H), 3.90-3.80 (m, 2H), 3.70-3.60 (m, 3H), 3.43-3.39 (m, 1H), 3.24-3.18 (m, 1H), 3.11-3.05 (m, 2H),2.23-2.19 (m, 6H), 2.01-1.71 (m, 4H), 1.68-1.49 (m, 3H), 1.45-1.19 (m, 6H), 0.99 (d, 1H), 0.88 (t, 1H).

### Example 46

### (R)-7-cyclopropyl-2-((S)-2-(3-cyclopropyl-4-fluorophenyl)-5-(5-((S)-2,2-dimethyltetrahydro-2H-pyran-4-yl)-1-((1S,2S)-2-methyl-1-(5-oxo-4,5-dihydro-1,2,4-oxadiazol-3-yl)cyclopropyl)-1H-indole-2-carbonyl)-4-methyl-4,5,6,7-tetrahydro-2H-pyrazolo[4,3-c]pyridin-3-yl)-10-fluoro-2,3,3a,4-tetrahydro-1H,7H-imidazo[1',5':4,5][1,4]oxazino[3,2-f]indazol-1-one 46

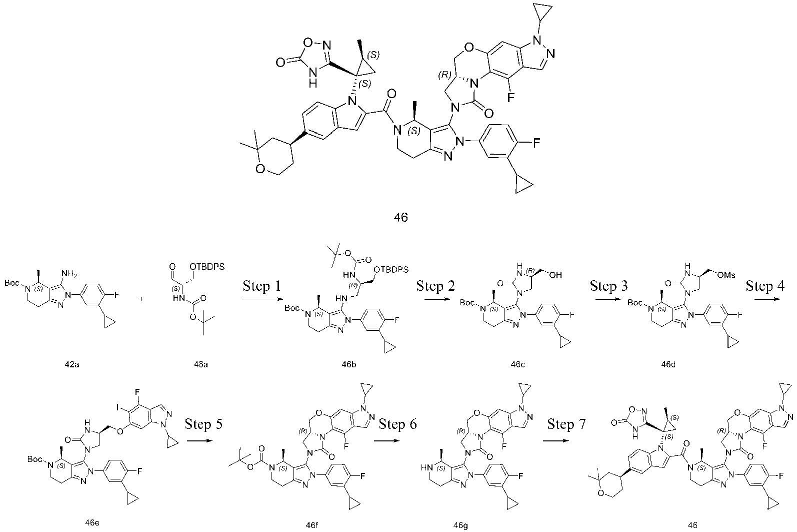

### Step 1

### Tert-butyl (S)-3-(((R)-2-((tert-butoxycarbonyl)amino)-3-((tert-butyldiphenylsilyl)oxy)propyl)amino)-2-(3-cyclopropyl-4-fluorophenyl)-4-methyl-2,4,6,7-tetrahydro-5H-pyrazolo[4,3-c]pyridine-5-carboxylate 46b

Compound **42a** (500 mg, 1.29 mmol) and tert-butyl (*S*)-(1-((tert-butyldiphenylsilyl)oxy)-3-oxopropyl-2-yl)carbamate **46a** (1.1 g, 2.58 mmol, prepared by a method in the document European Journal of Organic Chemistry, 1998, 6, p945-959) were dissolved in acetic acid (50 mL) and stirred at 0°C for 1 hour. Sodium cyanoborohydride (232 mg, 2.88 mmol) was added. The reaction was continued for 3 hours. The mixture was quenched with water (100 mL) and extracted with dichloromethane (50 mL × 3). The organic phases were combined and washed with a saturated sodium chloride solution. The organic phase was dried over anhydrous sodium sulfate. After filtration, the filtrate was concentrated under reduced pressure. The residue was purified by silica gel column chromatography with eluent system B to obtain the title compound **46b** (400 mg, yield: 38.7%).

MS m/z (ESI): 798.4 [M+1].

### Step 2

### Tert-butyl (S)-2-(3-cyclopropyl-4-fluorophenyl)-3-((R)-4-(hydroxymethyl)-2-oxoimidazolidin-1-yl)-4-methyl-2,4,6,7-tetrahydro-5H-pyrazolo[4,3-c]pyridine-5-carboxylate 46c

Compound **46b** (400 mg, 0.50 mmol) and cesium carbonate (326 mg, 1.0 mmol) were dissolved in *N*-methylpyrrolidone (30 mL). After displacement with nitrogen three times, the mixture was reacted at 100°C for 16 hours. The reaction mixture was cooled to room temperature. Water (100 mL) was added. The mixture was extracted with ethyl acetate (60 mL × 3). The organic phases were combined and washed with a saturated sodium chloride solution. The organic phase was dried over anhydrous sodium sulfate. After filtration, the filtrate was concentrated under reduced pressure. The residue was purified by silica gel column chromatography with eluent system B to obtain the title compound **46c** (60 mg, yield: 24.6%).

MS m/z (ESI): 486.5 [M+1].

### Step 3

### Tert-butyl(S)-2-(3-cyclopropyl-4-fluorophenyl)-4-methyl-3-((R)-4-(((methylsulfonyl)oxy)methyl)-2-oxoimidazolidin-1-yl)-2,4,6,7-tetrahydro-5H-pyrazolo[4,3-c]pyridine-5-carboxylate 46d

Compound **46c** (60 mg, 0.12 mmol) and triethylamine (37 mg, 0.37 mmol) were dissolved in anhydrous dichloromethane (10 mL) and cooled to 0°C. Methanesulfonyl chloride (21 mg, 0.18 mmol) was added and stirred for 1 hour. 50 mL of water was added. The mixture was extracted with dichloromethane (30 mL × 3). The organic phases were combined and washed with a saturated sodium chloride solution. The organic phase was dried over anhydrous sodium sulfate and filtered. The reaction solution was concentrated under reduced pressure to obtain the crude title compound **46d** (70 mg). The product was directly used in the next reaction without purification.

MS m/z (ESI): 564.2 [M+1].

### Step 4

### Tert-butyl (S)-3-((R)-4-(((1-cyclopropyl-4-fluoro-5-iodo-1H-indazol-6-yl)oxy)methyl)-2-oxoimidazolidin-1-yl)-2-(3-cyclopropyl-4-fluorophenyl)-4-methyl-2,4,6,7-tetrahydro-5H-pyrazolo[4,3-c]pyridine-5-carboxylate 46e

Compound **46d** (70 mg, 0.13 mmol) and compound **13i** (40 mg, 0.13 mmol) were dissolved in *N*,*N*-dimethylformamide (5.0 mL). Potassium carbonate (52 mg, 0.37 mmol) was added and reacted at 80°C for 3 hours. The reaction solution was poured into 20 mL of water and extracted with ethyl acetate (30 mL × 2). The organic phases were combined, washed with a saturated sodium chloride solution, and dried over anhydrous sodium sulfate. After filtration, the filtrate was concentrated under reduced pressure. The residue was purified by silica gel column chromatography with eluent system A to obtain the title compound **46e** (80 mg, yield: 80.9%).

MS m/z (ESI): 786.3 [M+1].

### Step 5

### Tert-butyl (S)-3-((R)-7-cyclopropyl-10-fluoro-1-oxo-3a,4-dihydro-1H,7H-imidazo[1',5':4,5][1,4]oxazino[3,2-f]indazol-2(3H)-yl)-2-(3-cyclopropyl-4-fluorophenyl)-4-methyl-2,4,6,7-tetrahydro-5H-pyrazolo[4,3-c]pyridine-5-carboxylate 46f

Compound **46e** (80 mg, 0.10 mmol) and (1*R*,2*R*)-(-)-*N*,*N*-dimethyl-1,2-cyclohexanediamine (15 mg, 0.10 mmol) were dissolved in *N*,*N*-dimethylformamide (10.0 mL). Potassium carbonate (42 mg, 0.31 mmol) and cuprous iodide (19 mg, 0.10 mmol) were added. After displacement with nitrogen three times, the mixture was reacted at 125°C for 2 hours. The reaction solution was poured into 20 mL of water and extracted with ethyl acetate (30 mL × 2). The organic phases were combined, washed with a saturated sodium chloride solution, and dried over anhydrous sodium sulfate. After filtration, the filtrate was concentrated under reduced pressure. The residue was purified by silica gel column chromatography with eluent system C to obtain the crude title compound **46f** (70 mg).

MS m/z (ESI): 658.3 [M+1].

### Step 6

### (R)-7-cyclopropyl-2-((S)-2-(3-cyclopropyl-4-fluorophenyl)-4-methyl-4,5,6,7-tetrahydro-2H-pyrazolo[4,3-c]pyridin-3-yl)-10-fluoro-2,3,3a,4-tetrahydro-1H,7H-imidazo[1',5':4,5][1,4]oxazino[3,2-f]indazol-1-one 46g

Compound **46f** (70 mg, 0.10 mmol) was dissolved in ethyl acetate (2 mL). A 4 M solution of hydrogen chloride in 1,4-dioxane (2 mL) was added and stirred at room temperature for 2 hours. The reaction solution was concentrated under reduced pressure to obtain the crude title compound **46g** (60 mg). The product was directly used in the next reaction without purification.

MS m/z (ESI): 558.3 [M+1].

### Step 7

### (R)-7-cyclopropyl-2-((S)-2-(3-cyclopropyl-4-fluorophenyl)-5-(5-((S)-2,2-dimethyltetrahydro-2H-pyran-4-yl)-1-((1S,2S)-2-methyl-1-(5-oxo-4,5-dihydro-1,2,4-oxadiazol-3-yl)cyclopropyl)-1H-indole-2-carbonyl)-4-methyl-4,5,6,7-tetrahydro-2H-pyrazolo[4,3-c]pyridin-3-yl)-10-fluoro-2,3,3a,4-tetrahydro-1H,7H-imidazo[1',5':4,5][1,4]oxazino[3,2-f]indazol-1-one 46

Crude compound **46g** (60 mg, 0.11 mmol) and compound **1w** (53 mg, 0.13 mmol) were dissolved in *N*,*N*-dimethylformamide (5 mL). *N,N-*diisopropylethylamine (41 mg, 0.32 mmol) was added. After the raw materials were fully dissolved, O-(7-azabenzotriazol-1-yl)-*N*,*N*,*N*',*N*'-tetramethyluronium hexafluorophosphate (81 mg, 0.21 mmol) was added and stirred at room temperature for 3 hours. The reaction solution was poured into 10 mL of water and extracted with ethyl acetate (10 mL × 3). The organic phases were combined, washed with a saturated sodium chloride solution, and dried over anhydrous sodium sulfate. After filtration, the filtrate was concentrated under reduced pressure. The residue was purified by preparative high-performance liquid chromatography (instrument model: Gilson-GX-281, chromatographic column: Welch xtimate phenly-hexyl, 30 × 250 mm; 5 µm; mobile phase: water (10 mM ammonium bicarbonate) and acetonitrile, with gradient ratio: acetonitrile 40%-50%, flow rate: 30 mL/min to obtain the title compound **46** (10 mg, yield: 9.8%).

MS m/z (ESI): 951.8 [M+1].

¹H NMR (500 MHz, CDCl₃): δ 11.30-11.24 (m, 1H), 7.97-7.94 (m, 1H), 7.60-7.52 (m, 1H), 7.51-7.47 (m, 1H), 7.27-7.21 (m, 2H), 7.10 (t, 1H), 7.05-7.02 (m, 1H), 6.91-6.85 (m, 1H), 6.72-6.68 (m, 1H), 5.79-5.75 (m, 1H), 5.36-5.16 (m, 1H), 4.46-4.43 (m, 1H), 4.21-4.18 (m, 1H), 4.07-3.97 (m, 2H), 3.89-3.80 (m, 2H), 3.64-3.48 (m, 2H), 3.15-2.96 (m, 4H), 2.23-2.20 (t, 1H), 2.11-1.99 (m, 2H), 1.92 (t, 1H), 1.79-1.71 (m, 3H), 1.68-1.52 (m, 6H), 1.34-1.07 (m, 11H), 1.00-0.94 (m, 1H), 0.89-0.86 (m, 1H), 0.71-0.61 (m, 2H).

### Example 47

### 3-((1S,2S)-1-(5-((S)-2,2-dimethyltetrahydro-2H-pyran-4-yl)-2-((S)-2-(4-fluoro-3,5-dimethylphenyl)-4-methyl-3-(1-(1-methyl-1H-indazol-4-yl)-6-oxo-1,6-dihydropyrimidin-5-yl)-4,5,6,7-tetrahydro-2H-pyrazolo[4,3-c]pyridine-5-carbonyl)-1H-indol-1-yl)-2-methylcyclopropyl)-1,2,4-oxadiazol-5(4H)-one 47

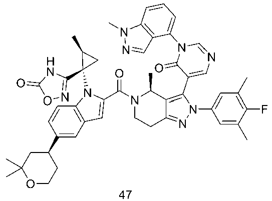

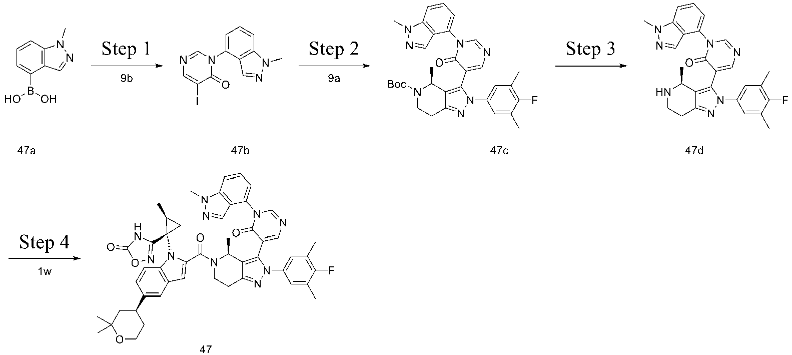

### Step 1

### 5-Iodo-3-(1-methyl-1H-indazol-4-yl)pyrimidin-4(3H)-one 47b

Compound **9b** (400 mg, 1.80 mmol), (1-methyl-1*H*-indazol-4-yl)boronic acid **47a** (480 mg, 2.73 mmol), pyridine (1.42 g, 18.02 mmol), and copper acetate (720 mg, 3.61 mmol) were dissolved in 1,2-dichloroethane (10 mL), left open, and stirred at room temperature for 16 hours. The reaction solution was concentrated under reduced pressure. The residue was purified by silica gel column chromatography with eluent system A to obtain a crude product, which was further slurried with dichloromethane to obtain the title compound **47b** (250 mg, yield: 39.4%).

MS m/z (ESI): 353.0 [M+1].

### Step 2

### Tert-butyl (S)-2-(4-fluoro-3,5-dimethylphenyl)-4-methyl-3-(1-(1-methyl-1H-indazol-4-yl)-6-oxo-1,6-dihydropyrimidin-5-yl)-2,4,6,7-tetrahydro-5H-pyrazolo[4,3-c]pyridine-5-carboxylate 47c

Compound **47b** (100 mg, 0.28 mmol), compound **9a** (180 mg, 0.45 mmol), anhydrous potassium carbonate (118 mg, 0.85 mmol), and 1,1'-bis(di-tert-butylphosphino)ferrocenepalladium dichloride (100 mg, 0.15 mmol) were dissolved in dioxane (40 mL) and water (8 mL). After displacement with nitrogen, the reaction was stirred at 70°C for 6 hours. The reaction solution was cooled to room temperature and concentrated under reduced pressure. The residue was purified by silica gel column chromatography with eluent system A to obtain the title compound **47c** (35 mg, yield: 21.1%).

MS m/z (ESI): 584.4 [M+1].

### Step 3

### (S)-5-(2-(4-fluoro-3,5-dimethylphenyl)-4-methyl-4,5,6,7-tetrahydro-2H-pyrazolo[4,3-c]pyridin-3-yl)-3-(1-methyl-1H-indazol-4-yl)pyrimidin-4(3H)-one 47d

Compound **47c** (35 mg, 0.060 mmol) was dissolved in dichloromethane (1 mL). A 4 M solution of hydrogen chloride in 1,4-dioxane (3 mL) was added and stirred at room temperature for 1 hour. The reaction solution was concentrated under reduced pressure to obtain the crude title compound **47d** (30 mg). The product was directly used in the next reaction without purification.

MS m/z (ESI): 484.3 [M+1].

### Step 4

### 3-((1S,2S)-1-(5-((S)-2,2-dimethyltetrahydro-2H-pyran-4-yl)-2-((S)-2-(4-fluoro-3,5-dimethylphenyl)-4-methyl-3-(1-(1-methyl-1H-indazol-4-yl)-6-oxo-1,6-dihydropyrimidin-5-yl)-4,5,6,7-tetrahydro-2H-pyrazolo[4,3-c]pyridine-5-carbonyl)-1H-indol-1-yl)-2-methylcyclopropyl)-1,2,4-oxadiazol-5(4H)-one 47

Compound **47d** (30 mg, 0.062mmol) and compound **1w** (30 mg, 0.073 mmol) were dissolved in *N*,*N*-dimethylformamide (4 mL). *N*,*N*-diisopropylethylamine (40 mg, 0.31 mmol) was added. After the raw materials were fully dissolved, *O*-(7-azabenzotriazol-1-yl)-*N*,*N*,*N*',*N*'-tetramethyluronium hexafluorophosphate (35 mg, 0.093 mmol) was added and stirred at room temperature for 16 hours. The reaction solution was poured into 10 mL of water and extracted with ethyl acetate (10 mL × 3). The organic phases were combined, washed with a saturated sodium chloride solution, and dried over anhydrous sodium sulfate. After filtration, the filtrate was concentrated under reduced pressure. The residue was purified by preparative high-performance liquid chromatography (instrument model: Waters-2545, chromatographic column: Welch Xtimate, Prep 30 × 150 mm; 5 µm; C18, mobile phase: water (10 mM ammonium bicarbonate) and acetonitrile, with gradient ratio: acetonitrile 40%-70%, flow rate: 30 mL/min) to obtain the title compound **47** (13 mg, yield: 23.9%).

MS m/z (ESI): 877.5 [M+1].

¹H NMR (500 MHz, CDCl₃): δ 11.29 (s, 1H), 8.38-8.17 (m, 1H), 8.08-7.76 (m, 1H), 7.63-7.48 (m, 4H), 7.36-7.29 (m, 1H), 7.28-7.23 (m, 1H), 7.18-6.78 (m, 3H), 6.75-6.66 (m, 1H), 5.89-5.21 (m, 2H), 4.92-4.39 (m, 1H), 4.22-4.10 (m, 3H), 3.95-3.56 (m, 3H), 3.50-3.13 (m, 1H), 3.12-2.97 (m, 2H), 2.33-2.26 (m, 6H), 2.24 (t, 1H), 2.07-1.99 (m, 1H), 1.92 (t, 1H), 1.83-1.73 (m, 3H), 1.70-1.63 (m, 3H), 1.52 (d, 2H), 1.38-1.35 (m, 3H), 1.22 (d, 2H), 1.05 (d, 1H), 0.90 (t, 1H).

### Example 48

### 3-((1S,2S)-1-(5-((S)-2,2-dimethyltetrahydro-2H-pyran-4-yl)-2-((S)-2-(4-fluoro-3,5-dimethylphenyl)-4-methyl-3-(1-(1-methyl-1H-benzo[d]imidazol-5-yl)-6-oxo-1,6-dihydropyrimidin-5-yl)-4,5,6,7-tetrahydro-2H-pyrazolo[4,3-c]pyridine-5-carbonyl)-1H-indol-1-yl)-2-methylcyclopropyl)-1,2,4-oxadiazol-5(4H)-one 48

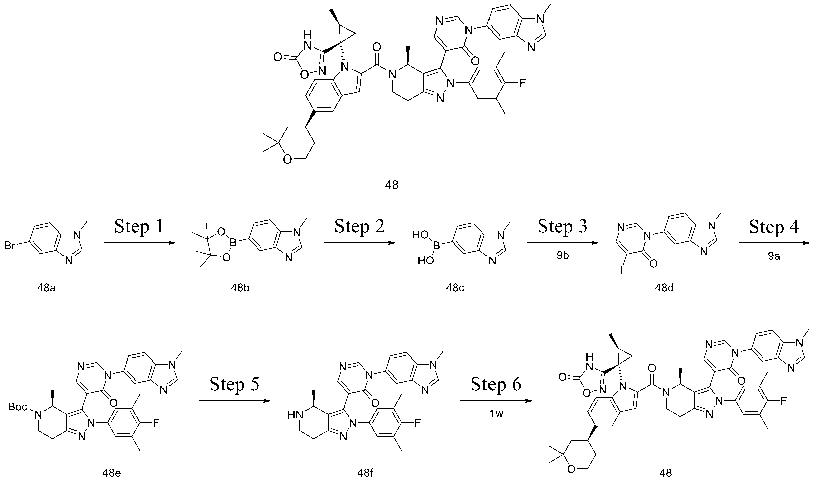

### Step 1

### 1-Methyl-5-(4,4,5,5-tetramethyl-1,3,2-dioxaborolan-2-yl)-1H-benzo[d]imidazole 48b

5-Bromo-1-methylbenzimidazole **48a** (1.0 g, 4.74 mmol), bis(pinacolato)diboron (1.5 g, 5.91 mmol), potassium acetate (0.7 g, 7.13 mmol), and [1,1'-bis(diphenylphosphino)ferrocene]palladium(II) dichloride (350 mg, 0.48 mmol) were dissolved in dioxane (30 mL). After displacement with nitrogen, the mixture was reacted under stirring at 100°C for 6 hours. The reaction solution was cooled to room temperature and concentrated under reduced pressure. The residue was purified by silica gel column chromatography with eluent system C to obtain the title compound **48b** (840 mg, yield: 68.7%).

MS m/z (ESI): 259.1 [M+1].

### Step 2

### (1-Methyl-1H-benzo[d]imidazol-5-yl)boronic acid 48c

Compound **48b** (840 mg, 3.25 mmol) was dissolved in acetone (20 mL) and water (20 mL). Sodium periodate (2.1 g, 9.82 mmol) and ammonium acetate (753 mg, 9.77 mmol) were added. The reaction was stirred at room temperature overnight. The reaction solution was filtered. The filtrate was concentrated under reduced pressure to remove the organic solvent and extracted with ethyl acetate (30 mL × 3). The organic phases were combined and dried over anhydrous sodium sulfate. After filtration, the filtrate was concentrated under reduced pressure. The residue was purified by silica gel column chromatography with eluent system A to obtain the title compound **48c** (260 mg, yield: 45.4%).

MS m/z (ESI): 177.0 [M+1].

### Step 3

### 5-Iodo-3-(1-methyl-1H-benzo[d]imidazol-5-yl)pyrimidin-4(3H)-one 48d

Compound **9b** (300 mg, 1.35 mmol), compound **48c** (260 mg, 1.48 mmol), pyridine (1.1 g, 13.91 mmol), and copper acetate (540 mg, 2.70 mmol) were dissolved in 1,2-dichloroethane (10 mL), left open, and stirred at room temperature for 16 hours. The reaction solution was concentrated under reduced pressure. The residue was purified by silica gel column chromatography with eluent system A to obtain a crude product, which was further slurried with dichloromethane to obtain the title compound **48d** (70 mg, yield: 14.7%).

MS m/z (ESI): 353.0 [M+1].

### Step 4

### Tert-butyl (S)-2-(4-fluoro-3,5-dimethylphenyl)-4-methyl-3-(1-(1-methyl-1H-benzo[d]imidazol-5-yl)-6-oxo-1,6-dihydropyrimidin-5-yl)-2,4,6,7-tetrahydro-5H-pyrazolo[4,3-c]pyridine-5-carboxylate 48e

Compound **48d** (70 mg, 0.19 mmol), compound **9a** (121 mg, 0.30 mmol), anhydrous potassium carbonate (84 mg, 0.61 mmol), and 1,1'-bis(di-tert-butylphosphino)ferrocenepalladium dichloride (70 mg, 0.10 mmol) were dissolved in dioxane (50 mL) and water (10 mL). After displacement with nitrogen, the reaction was stirred at 70°C for 5 hours. The reaction solution was cooled to room temperature and concentrated under reduced pressure. The residue was purified by silica gel column chromatography with eluent system A to obtain the title compound **48e** (110 mg, yield: 66.4%).

MS m/z (ESI): 584.3 [M+1].

### Step 5

### (S)-5-(2-(4-fluoro-3,5-dimethylphenyl)-4-methyl-4,5,6,7-tetrahydro-2H-pyrazolo[4,3-c]pyridin-3-yl)-3-(1-methyl-1H-benzo[d]imidazol-5-yl)pyrimidin-4(3H)-one 48f

Compound **48e** (30 mg, 0.051 mmol) was dissolved in dichloromethane (2 mL). A 4 M solution of hydrogen chloride in 1,4-dioxane (3 mL) was added and stirred at room temperature for 1 hour. The reaction solution was concentrated under reduced pressure to obtain the crude title compound **48f** (25 mg). The product was directly used in the next reaction without purification.

MS m/z (ESI): 484.2 [M+1].

### Step 6

### 3-((1S,2S)-1-(5-((S)-2,2-dimethyltetrahydro-2H-pyran-4-yl)-2-((S)-2-(4-fluoro-3,5-dimethylphenyl)-4-methyl-3-(1-(1-methyl-1H-benzo[d]imidazol-5-yl)-6-oxo-1,6-dihydropyrimidin-5-yl)-4,5,6,7-tetrahydro-2H-pyrazolo[4,3-c]pyridine-5-carbonyl)-1H-indol-1-yl)-2-methylcyclopropyl)-1,2,4-oxadiazol-5(4H)-one 48

Compound **48f** (25 mg, 0.052 mmol) and compound **1w** (20 mg, 0.049 mmol) were dissolved in *N*,*N*-dimethylformamide (4 mL). *N*,*N*-diisopropylethylamine (35 mg, 0.27 mmol) was added. After the raw materials were fully dissolved, *O-*(7-azabenzotriazol-1-yl)-*N*,*N*,*N*',*N*'-tetramethyluronium hexafluorophosphate (30 mg, 0.079 mmol) was added and stirred at room temperature for 16 hours. The reaction solution was poured into 10 mL of water and extracted with ethyl acetate (10 mL × 3). The organic phases were combined, washed with a saturated sodium chloride solution, and dried over anhydrous sodium sulfate. After filtration, the filtrate was concentrated under reduced pressure. The residue was purified by preparative high-performance liquid chromatography (instrument model: Waters-2545, chromatographic column: Welch Xtimate, Prep 30 × 150 mm; 5 µm; C18, mobile phase: water (10 mM ammonium bicarbonate) and acetonitrile, with gradient ratio: acetonitrile 30%-50%, flow rate: 30 mL/min) to obtain the title compound **48** (5 mg, yield: 11.0%).

MS m/z (ESI): 875.6 [M-1].

¹H NMR (500 MHz, CDCl₃): δ 11.37 (s, 1H), 8.38-8.17 (m, 1H), 8.05-7.46 (m, 5H), 7.35-7.28 (m, 1H), 7.24-7.16 (m, 1H), 7.11-6.97 (m, 2H), 6.83-6.61 (m, 2H), 5.89-5.77 (m, 1H), 5.31-5.16 (m, 1H), 4.88-4.39 (m, 1H), 3.98-3.76 (m, 5H), 3.62-3.39 (m, 1H), 3.19-2.96 (m, 3H), 2.31-2.16 (m, 8H), 1.80-1.72 (m, 3H), 1.47 (d, 2H), 1.36-1.33 (m, 5H), 1.19 (d, 2H), 1.02 (d, 1H), 0.88 (t, 3H).

### Example 49

### 3-((1S,2S)-1-(5-((S)-2,2-dimethyltetrahydro-2H-pyran-4-yl)-2-((S)-2-(4-fluoro-3,5-dimethylphenyl)-4-methyl-3-(1-(1-methyl-1H-indol-5-yl)-6-oxo-1,6-dihydropyrimidin-5-yl)-4,5,6,7-tetrahydro-2H-pyrazolo[4,3-c]pyridine-5-carbonyl)-1H-indol-1-yl)-2-methylcyclopropyl)-1,2,4-oxadiazol-5(4H)-one 49

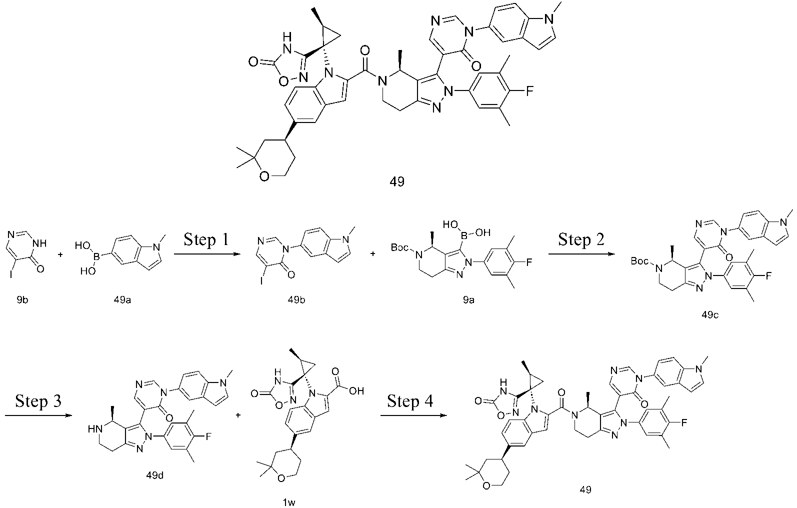

### Step 1

### 5-Iodo-3-(1-methyl-1H-indol-5-yl)pyrimidin-4(3H)-one 49b

Compound **9b** (500 mg, 2.25 mmol), (1-methyl-1*H*-indol-5-yl)boronic acid **49a** (600 mg, 3.43 mmol, WuXi AppTec), pyridine (1.76 g, 22.26 mmol), and copper acetate (900 mg, 4.51 mmol) were dissolved in 1,2-dichloroethane (20 mL), left open, and stirred at room temperature for 16 hours. The reaction solution was concentrated under reduced pressure. The residue was purified by silica gel column chromatography with eluent system A to obtain the title compound **49b** (550 mg, yield: 69.5%).

MS m/z (ESI): 351.9 [M+1].

### Step 2

### Tert-butyl (S)-2-(4-fluoro-3,5-dimethylphenyl)-4-methyl-3-(1-(1-methyl-1H-indol-5-yl)-6-oxo-1,6-dihydropyrimidin-5-yl)-2,4,6,7-tetrahydro-5H-pyrazolo[4,3-c]pyridine-5-carboxylate 49c

Compound **49b** (100 mg, 0.28 mmol), compound **9a** (180 mg, 0.45 mmol), anhydrous potassium carbonate (118 mg, 0.85 mmol), and [1,1'-bis(di-tert-butylphosphino)ferrocene]palladium dichloride (94 mg, 0.14 mmol) were dissolved in dioxane (40 mL) and water (8 mL). After displacement with nitrogen, the mixture was reacted under stirring at 70°C for 6 hours. The reaction solution was cooled to room temperature and concentrated under reduced pressure. The residue was purified by silica gel column chromatography with eluent system A to obtain the title compound **49c** (90 mg, yield: 54.2%).

MS m/z (ESI): 583.3 [M+1].

### Step 3

### (S)-5-(2-(4-fluoro-3,5-dimethylphenyl)-4-methyl-4,5,6,7-tetrahydro-2H-pyrazolo[4,3-c]pyridin-3-yl)- 3-(1-methyl-1H-indol-5-yl)pyrimidin-4(3H)-one 49d

Compound **49c** (90 mg, 0.15 mmol) was dissolved in dichloromethane (5 mL). A 4M solution of hydrogen chloride in dioxane (4 mL) was added. The reaction was stirred at room temperature for 1 hour. The reaction solution was concentrated under reduced pressure to obtain the crude title compound **49d** (74 mg). The product was directly used in the next reaction without purification.

MS m/z (ESI): 483.4 [M+1].

### Step 4

### 3-((1S,2S)-1-(5-((S)-2,2-dimethyltetrahydro-2H-pyran-4-yl)-2-((S)-2-(4-fluoro-3,5-dimethylphenyl)-4-methyl-3-(1-(1-methyl-1H-indol-5-yl)-6-oxo-1,6-dihydropyrimidin-5-yl)-4,5,6,7-tetrahydro-2H-pyrazolo[4,3-c]pyridine-5-carbonyl)-1H-indol-1-yl)-2-methylcyclopropyl)-1,2,4-oxadiazol-5(4H)-one 49

Compound **49d** (50 mg, 0.10 mmol) and compound **1w** (65 mg, 0.16 mmol) were dissolved in *N*,*N-*dimethylformamide (5 mL). *N*,*N*-diisopropylethylamine (73 mg, 0.56 mmol) was added. After the raw materials were fully dissolved, *O*-(7-azabenzotriazol-1-yl)-*N*,*N*,*N*',*N*'-tetramethyluronium hexafluorophosphate (62 mg, 0.16 mmol) was added and stirred at room temperature for 4 hours. The reaction solution was poured into 10 mL of water and extracted with ethyl acetate (10 mL × 3). The organic phases were combined, washed with a saturated sodium chloride solution, and dried over anhydrous sodium sulfate. After filtration, the filtrate was concentrated under reduced pressure. The residue was purified by preparative high-performance liquid chromatography (instrument model: (Waters-2545, chromatographic column Welch Xtimate C18, 30 × 150 mm, 5 µm; mobile phase: aqueous phase (10 mM ammonium bicarbonate) and acetonitrile, with gradient ratio: acetonitrile 40%-60%, flow rate: 30 mL/min) to obtain the title compound **49** (11 mg, yield: 12.1%).

MS m/z (ESI): 876.7 [M+1].

¹H NMR (500 MHz, CDCl₃): δ 8.41-8.13 (m, 1H), 7.98-7.66 (m, 1H), 7.64-7.31 (m, 4H), 7.27-6.93 (m, 5H), 6.77-6.46 (m, 2H), 5.90-5.14 (m, 2H), 4.93-4.33 (m, 1H), 3.96-3.73 (m, 5H), 3.66-3.38 (m, 1H), 3.21-2.96 (m, 3H), 2.34-2.14 (m, 7H), 2.08-1.97 (m, 1H), 1.95-1.85 (m, 1H), 1.82-1.73 (m, 3H), 1.52-1.49 (m, 2H), 1.40-1.32 (m, 6H), 1.23-1.17 (m, 2H), 1.06-0.99 (m, 1H), 0.92-0.85 (m, 1H).

### Example 50

### (R)-7-(tert-butyl)-2-((S)-5-(5-((S)-2,2-dimethyltetrahydro-2H-pyran-4-yl)-1-((1S,2S)-2-methyl-1-(5-oxo-4,5-dihydro-1,2,4-oxadiazol-3-yl)cyclopropyl)-1H-indole-2-carbonyl)-2-(4-fluoro-3,5-dimethylphenyl)-4-methyl-4,5,6,7-tetrahydro-2H-pyrazolo[4,3-c]pyridin-3-yl)-10-fluoro-2,3,3a,4-tetrahydro-1H,7H-imidazo[1',5':4,5][1,4]oxazino[3,2-f]indazol-1-one 50

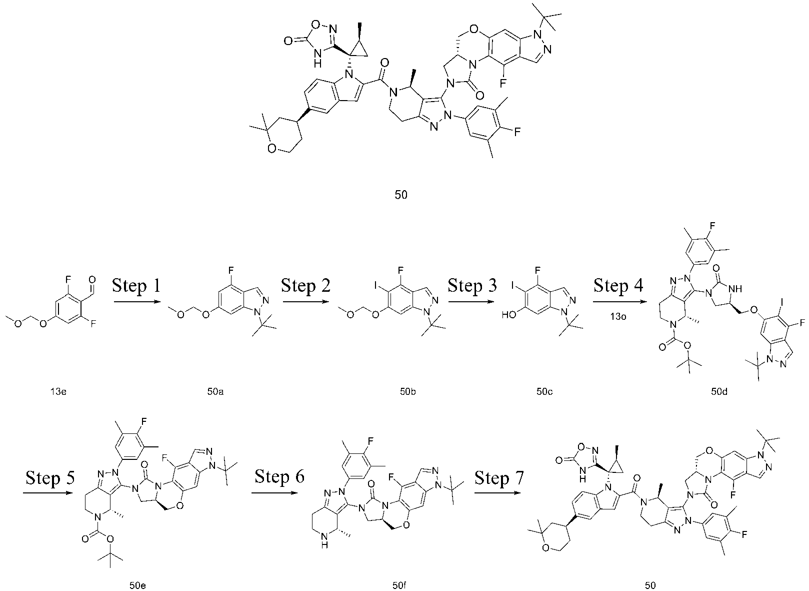

### Step 1

### 1-(Tert-butyl)-4-fluoro-6-(methoxymethoxy)-1H-indazole 50a

Compound **13e** (2.0 g, 9.89 mmol), tert-butylhydrazine hydrochloride (6.5 g, 52.16 mmol, Adamas), and potassium carbonate (14.0 g, 101.30 mmol) were dissolved in *N*,*N*-dimethylformamide (20 mL). The reaction was heated to 140°C and reacted under stirring for 4 hours. The reaction solution was cooled to room temperature. The reaction solution was cooled to room temperature. After filtration, the filtrate was concentrated under reduced pressure. The residue was purified by silica gel column chromatography with eluent system B to obtain the title compound **50a** (720 mg, yield: 28.8%).

MS m/z (ESI): 253.3 [M+1].

### Step 2

### 1-(Tert-butyl)-4-fluoro-5-iodo-6-(methoxymethoxy)-1H-indazole 50b

Compound **50a** (400 mg, 1.59 mmol) was dissolved in tetrahydrofuran (10 mL). Lithium diisopropylamide (1.59 mL, 3.17 mmol, 2M, Energy Chemical) was dropwise added in a nitrogen atmosphere at -78°C. After the addition was complete, stirring was continued at -78°C for 2 hours. A solution of iodine (805 mg, 3.17 mmol)/tetrahydrofuran (2 mL) was then dropwise added. After the addition was complete, stirring was continued at -78°C for 1 hour. The reaction was quenched with a saturated aqueous sodium thiosulfate solution (30 mL) and extracted with ethyl acetate (40 mL × 3). The organic phases were combined and washed with a saturated sodium chloride solution. The organic phase was dried over anhydrous sodium sulfate. After filtration, the filtrate was concentrated under reduced pressure. The residue was purified by silica gel column chromatography with eluent system B to obtain the title compound **50b** (550 mg, yield: 91.7%).

MS m/z (ESI): 379.0 [M+1].

### Step 3

### 1-(Tert-butyl)-4-fluoro-5-iodo-1H-indazol-6-ol 50c

Compound **50b** (300 mg, 0.79 mmol) was dissolved in dichloromethane (3.0 mL). A hydrogen chloride/dioxane solution (3.0 mL, 4M) was dropwise added at 0°C. After the addition was complete, the mixture was stirred at room temperature for 1 hour. After concentration, 20 mL of water was added. The mixture was extracted with ethyl acetate (30 mL × 3). The organic phases were combined, washed with a saturated aqueous sodium chloride solution, and dried over anhydrous sodium sulfate. After filtration, the filtrate was concentrated under reduced pressure. The residue was purified by silica gel column chromatography with eluent system B to obtain the title compound **50c** (300 mg, yield: 85%).

MS m/z (ESI): 334.9 [M+1].

### Step 4

### Tert-butyl (S)-3-((R)-4-(((1-(tert-butyl)-4-fluoro-5-iodo-1H-indazol-6-yl)oxy)methyl)-2-oxoimidazolidin-1-yl)-2-(4-fluoro-3,5-dimethylphenyl)-4-methyl-2,4,6,7-tetrahydro-5H-pyrazolo[4,3-c]pyridine-5-carboxylate 50d

Compound **13o** (120 mg, 0.22 mmol) and compound **50c** (60 mg, 0.18 mmol) were dissolved in *N*,*N*-dimethylformamide (5.0 mL). Potassium carbonate (75 mg, 0.54 mmol) was added and reacted at 60°C for 2 hours. The reaction solution was poured into 20 mL of water and extracted with ethyl acetate (30 mL × 2). The organic phases were combined, washed with a saturated sodium chloride solution, and dried over anhydrous sodium sulfate. After filtration, the filtrate was concentrated under reduced pressure. The residue was purified by silica gel column chromatography with eluent system A to obtain the title compound **50d** (75 mg, yield: 52.9%).

MS m/z (ESI): 790.5 [M+1].

### Step 5

### Tert-butyl (S)-3-((R)-7-(tert-butyl)-10-fluoro-1-oxo-3a,4-dihydro-1H,7H-imidazo[1',5':4,5][1,4]oxazino[3,2-f]indazol-2(3H)-yl)-2-(4-fluoro-3,5-dimethylphenyl)-4-methyl-2,4,6,7-tetrahydro-5H-pyrazolo[4,3-c]pyridine-5-carboxylate 50e

Compound **50d** (75 mg, 0.095 mmol) and (1S,25)-(+)-*N*,*N*'-dimethyl-1,2-cyclohexanediamine (14 mg, 0.098 mmol) were dissolved in *N*-methylpyrrolidone (5.0 mL). Potassium carbonate (40 mg, 0.29 mmol) and cuprous iodide (19 mg, 0.10 mmol, Leyan) were added. After displacement with nitrogen three times, the mixture was heated to 110°C and reacted under stirring for 3 hours. The reaction solution was poured into 20 mL of water and extracted with ethyl acetate (30 mL × 2). The organic phases were combined, washed with a saturated sodium chloride solution, and dried over anhydrous sodium sulfate. After filtration, the filtrate was concentrated under reduced pressure. The residue was purified by silica gel column chromatography with eluent system C to obtain the title compound **50e** (45 mg, yield: 71.6%).

MS m/z (ESI): 662.3 [M+1].

### Step 6

### (R)-7-(tert-butyl)-10-fluoro-2-((S)-2-(4-fluoro-3,5-dimethylphenyl)-4-methyl-4,5,6,7-tetrahydro-2H-pyrazolo[4,3-c]pyridin-3-yl)-2,3,3a,4-tetrahydro-1H,7H-imidazo[1',5':4,5][1,4]oxazino[3,2-f]indazol-1-one 50f

Compound **50e** (45 mg, 0.068 mmol) was dissolved in dichloromethane (1 mL). A 4M solution of hydrogen chloride in 1,4-dioxane (3 mL) was added and stirred at room temperature for 1 hour. The reaction solution was concentrated under reduced pressure to obtain the crude title compound **50f** (38 mg). The product was directly used in the next reaction without purification.

MS m/z (ESI): 562.4 [M+1].

### Step 7

### (R)-7-(tert-butyl)-2-((S)-5-(5-((S)-2,2-dimethyltetrahydro-2H-pyran-4-yl)-1-((1S,2S)-2-methyl-1-(5-oxo-4,5-dihydro-1,2,4-oxadiazol-3-yl)cyclopropyl)-1H-indole-2-carbonyl)-2-(4-fluoro-3,5-dimethylphenyl)-4-methyl-4,5,6,7-tetrahydro-2H-pyrazolo[4,3-c]pyridin-3-yl)-10-fluoro-2,3,3a,4-tetrahydro-1H,7H-imidazo[1',5':4,5][1,4]oxazino[3,2-f]indazol-1-one 50

Compound **50f** (38 mg, 0.068 mmol) and compound **1w** (30 mg, 0.073 mmol) were dissolved in *N*,*N*-dimethylformamide (3 mL). *N*,*N*-diisopropylethylamine (90 mg, 0.70 mmol) was added. After the raw materials were fully dissolved, *O*-(7-azabenzotriazol-1-yl)-*N*,*N*,*N*',*N*'-tetramethyluronium hexafluorophosphate (40 mg, 0.11 mmol) was added and stirred at room temperature for 2 hours. The reaction solution was poured into 10 mL of water and extracted with ethyl acetate (10 mL × 3). The organic phases were combined, washed with a saturated sodium chloride solution, and dried over anhydrous sodium sulfate. After filtration, the filtrate was concentrated under reduced pressure. The residue was purified by preparative high-performance liquid chromatography (instrument model: Gilson-GX-281, chromatographic column: Welch Xtimate Phenyl-Hexyl, Prep 30 × 250 mm; 5 µm; C18, mobile phase: water (10 mM ammonium bicarbonate) and acetonitrile, with gradient ratio: acetonitrile 45%-72%, flow rate: 30 mL/min) to obtain the title compound **50** (15 mg, yield: 23.2%).

MS m/z (ESI): 955.7 [M+1].

¹H NMR (500 MHz, CDCl₃): δ 11.28 (s, 1H), 8.05-7.93 (m, 1H), 7.65-7.47 (m, 2H), 7.27-7.13 (m, 3H), 7.07-6.94 (m, 1H), 6.82-6.68 (m, 1H), 5.84-5.14 (m, 2H), 4.90-4.43 (m, 1H), 4.32-4.18 (m, 1H), 4.10-3.77 (m, 4H), 3.72-3.29 (m, 2H), 3.19-2.91 (m, 4H), 2.31 (s, 6H), 2.24 (t, 1H), 2.09-1.99 (m, 1H), 1.94 (t, 1H), 1.83-1.74 (m, 11H), 1.40-1.31 (m, 7H), 1.27-1.21 (m, 4H), 1.07 (d, 1H), 0.90 (t, 1H).

### Example 51

### (R)-7-(cyclopropylmethyl)-2-((S)-5-(5-((S)-2,2-dimethyltetrahydro-2H-pyran-4-yl)-1-((1S,2S)-2-methyl-1-(5-oxo-4,5-dihydro-1,2,4-oxadiazol-3-yl)cyclopropyl)-1H-indole-2-carbonyl)-2-(4-fluoro-3,5-dimethylphenyl)-4-methyl-4,5,6,7-tetrahydro-2H-pyrazolo[4,3-c]pyridin-3-yl)-10-fluoro-2,3,3a,4-tetrahydro-1H,7H-imidazo[1',5':4,5][1,4]oxazino[3,2-f]indazol-1-one 51

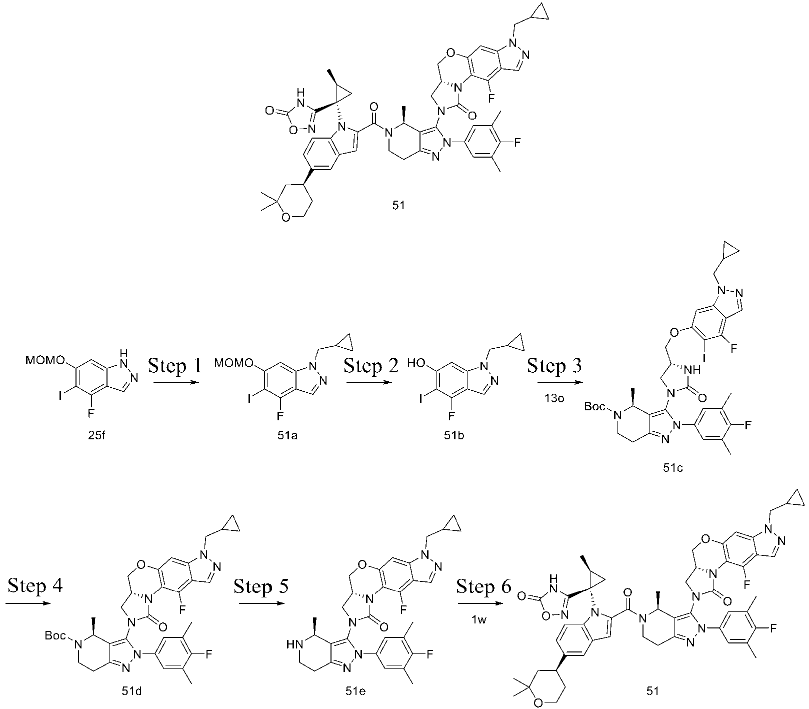

### Step 1

1-(Cyclopropylmethyl)-4-fluoro-5-iodo-6-(methoxymethoxy)-1*H*-indazole **51a** Compound **25f** (500 mg, 1.55 mmol) was dissolved in tetrahydrofuran (50 mL). Sodium hydride (124 mg, 3.10 mmol, 60% dispersed in a mineral oil) was added under ice bath condition and stirred for 30 minutes, followed by the addition of bromomethylcyclopropane (420 mg, 3.10 mmol, Accela). The mixture was heated to 50°C and reacted overnight. After the reaction was complete, 50 mL of water was added and extracted with ethyl acetate (80 mL × 3). The organic phases were combined, washed with a saturated aqueous sodium chloride solution, and dried over anhydrous sodium sulfate. After filtration, the filtrate was concentrated under reduced pressure. The residue was purified by silica gel column chromatography with eluent system B to obtain the title compound **51a** (22 mg, yield: 38%).

MS m/z (ESI): 376.9 [M+1].

### Step 2

### 1-(Cyclopropylmethyl)-4-fluoro-5-iodo-1H-indazol-6-ol 51b

Compound **51a** (220 mg, 0.58 mmol) was dissolved in dichloromethane (5 mL). A hydrogen chloride/dioxane solution (5 mL, 4.0M) was added and then stirred at room temperature for 4 hours. The reaction solution was concentrated under reduced pressure to obtain the crude title compound **51b** (190 mg). The product was directly used in the next reaction without purification.

MS m/z (ESI): 333.2 [M+1].

### Step 3

### Tert-butyl (S)-3-((R)-4-(((1-(cyclopropylmethyl)-4-fluoro-5-iodo-1H-indazol-6-yl)oxy)methyl)-2-oxoimidazolidin-1-yl)-2-(4-fluoro-3,5-dimethylphenyl)-4-methyl-2,4,6,7-tetrahydro-5H-pyrazolo[4,3-c]pyridine-5-carboxylate 51c

Compound **13o** (160 mg, 0.29 mmol) and compound **51b** (95 mg, 0.29 mmol) were dissolved in *N*,*N-*dimethylformamide (5.0 mL). Potassium carbonate (120 mg, 0.87 mmol) was added and reacted at 80°C for 3 hours. The reaction solution was poured into 20 mL of water and extracted with ethyl acetate (30 mL × 2). The organic phases were combined, washed with a saturated sodium chloride solution, and dried over anhydrous sodium sulfate. After filtration, the filtrate was concentrated under reduced pressure. The residue was purified by silica gel column chromatography with eluent system A to obtain the title compound **51c** (150 mg, yield: 67%).

MS m/z (ESI): 788.4 [M+1].

### Step 4

### Tert-butyl (S)-3-((R)-7-(cyclopropylmethyl)-10-fluoro-1-oxo-3a,4-dihydro-1H,7H-imidazo[1',5':4,5][1,4]oxazino[3,2-f]indazol-2(3H)-yl)-2-(4-fluoro-3,5-dimethylphenyl)-4-methyl-2,4,6,7-tetrahydro-5H-pyrazolo[4,3-c]pyridine-5-carboxylate 51d

Compound **51c** (150 mg, 0.19 mmol) and (1*S*,2*S*)-(+)-*N*,*N*'-dimethyl-1,2-cyclohexanediamine (14 mg, 0.10 mmol) were dissolved in *N*-methylpyrrolidone (5.0 mL). Potassium carbonate (80 mg, 0.58 mmol) and cuprous iodide (10 mg, 0.05 mmol) were added. After displacement with nitrogen three times, the mixture was heated to 125°C and reacted under stirring for 3 hours. The reaction solution was poured into 20 mL of water and extracted with ethyl acetate (30 mL × 2). The organic phases were combined, washed with a saturated sodium chloride solution, and dried over anhydrous sodium sulfate. After filtration, the filtrate was concentrated under reduced pressure. The residue was purified by silica gel column chromatography with eluent system C to obtain the title compound **51d** (64 mg, yield: 51%).

MS m/z (ESI): 660.6 [M+1].

### Step 5

### (R)-7-(cyclopropylmethyl)-10-fluoro-2-((S)-2-(4-fluoro-3,5-dimethylphenyl)-4-methyl-4,5,6,7-tetrahydro-2H-pyrazolo[4,3-c]pyridin-3-yl)-2,3,3a,4-tetrahydro-1H,7H-imidazo[1',5':4,5][1,4]oxazino[3,2-f]indazol-1-one51e

Compound **51d** (64 mg, 0.10 mmol) was dissolved in dichloromethane (2 mL). A 4M solution of hydrogen chloride in 1,4-dioxane (2 mL) was added and stirred at room temperature for 2 hour. The reaction solution was concentrated under reduced pressure to obtain the crude title compound **51e** (54 mg). The product was directly used in the next reaction without purification.

MS m/z (ESI): 560.2 [M+1].

### Step 6

### (R)-7-(cyclopropylmethyl)-2-((S)-5-(5-((S)-2,2-dimethyltetrahydro-2H-pyran-4-yl)-1-((1S,2S)-2-methyl-1-(5-oxo-4,5-dihydro-1,2,4-oxadiazol-3-yl)cyclopropyl)-1H-indole-2-carbonyl)-2-(4-fluoro-3,5-dimethylphenyl)-4-methyl-4,5,6,7-tetrahydro-2H-pyrazolo[4,3-c]pyridin-3-yl)-10-fluoro-2,3,3a,4-tetrahydro-1H,7H-imidazo[1',5':4,5][1,4]oxazino[3,2-f]indazol-1-one 51

Compound **51e** (54 mg, 0.10 mmol) and compound **1w** (50 mg, 0.12 mmol) were dissolved in *N*,*N*-dimethylformamide (5 mL). *N*,*N*-diisopropylethylamine (62 mg, 0.48 mmol) was added. After the raw materials were fully dissolved, *O*-(7-azabenzotriazol-1-yl)-*N*,*N*,*N*',*N*'-tetramethyluronium hexafluorophosphate (50 mg, 0.13 mmol) was added and stirred at room temperature for 2 hours. The reaction solution was poured into 10 mL of water and extracted with ethyl acetate (10 mL × 3). The organic phases were combined, washed with a saturated sodium chloride solution, and dried over anhydrous sodium sulfate. After filtration, the filtrate was concentrated under reduced pressure. The residue was purified by preparative high-performance liquid chromatography (instrument model: Gilson-GX-281, chromatographic column: Boston Phlex Prep C18, 30 × 150 mm; 5 µm; mobile phase: water (10 mM ammonium bicarbonate) and acetonitrile, with gradient ratio: acetonitrile 40%-70%, flow rate: 30 mL/min) to obtain the title compound **51** (40 mg, yield: 43%).

MS m/z (ESI): 953.8 [M+1].

¹H NMR (500 MHz, CDCl₃): δ 11.30 (s, 1H), 8.08-7.98 (m, 1H), 7.65-7.50 (m, 2H), 7.30-7.28 (m, 1H), 7.23-7.17 (m, 2H), 6.79-6.60 (m, 2H), 5.85-5.13 (m, 1H), 4.90-4.43 (m, 1H), 4.29-3.78 (m, 7H), 3.73-3.31 (m, 2H), 3.21-2.93 (m, 4H), 2.30 (s, 6H), 2.06-1.90 (m, 1H), 1.83-1.74 (m, 3H), 1.71-1.68 (m, 3H), 1.64-1.61 (m, 2H), 1.58-1.55 (m, 1H), 1.38-1.34 (m, 3H), 1.31-1.29 (m, 3H), 1.26-1.23 (m, 3H), 1.09-0.87 (m, 1H), 0.65-0.54 (m, 2H), 0.45-0.36 (m, 2H).

### Example 52-1 and Example 52-2

### (R)-7-cyclopropyl-2-((R)-5-(5-((S)-2,2-dimethyltetrahydro-2H-pyran-4-yl)-1-((1S,2S)-2-methyl-1-(5-oxo-4,5-dihydro-1,2,4-oxadiazol-3-yl)cyclopropyl)-1H-indole-2-carbonyl)-2-(4-fluoro-3,5-dimethylphenyl)-4-methyl-4,5,6,7-tetrahydropyrazolo[1,5-a]pyrazin-3-yl)-10-fluoro-2,3,3a,4-tetrahydro-1H,7H-imidazo[1',5':4,5][1,4]oxazino[3,2-f]indazol-1-one 52-1 and

### (R)-7-cyclopropyl-2-((S)-5-(5-((S)-2,2-dimethyltetrahydro-2H-pyran-4-yl)-1-((1S,2S)-2-methyl-1-(5-oxo-4,5-dihydro-1,2,4-oxadiazol-3-yl)cyclopropyl)-1H-indole-2-carbonyl)-2-(4-fluoro-3,5-dimethylphenyl)-4-methyl-4,5,6,7-tetrahydropyrazolo[1,5-a]pyrazin-3-yl)-10-fluoro-2,3,3a,4-tetrahydro-1H,7H-imidazo[1',5':4,5][1,41oxazino[3,2-f]indazol-1-one 52-2

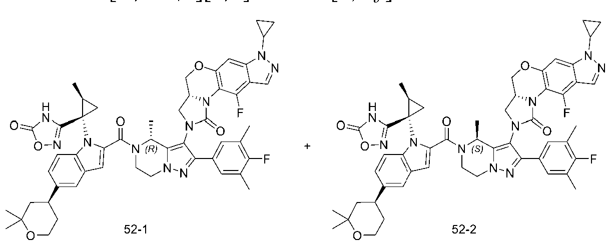

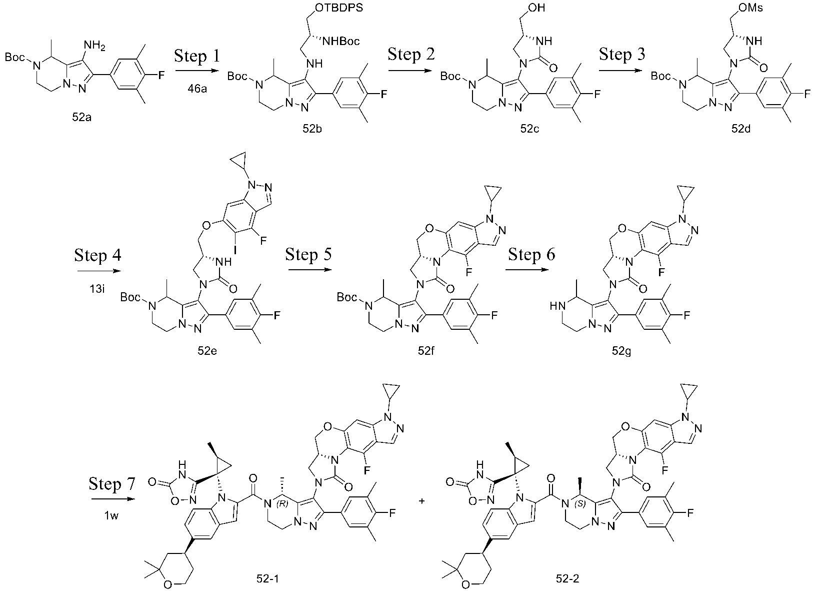

### Step 1

### Tert-butyl 3-(((R)-2-((tert-butoxycarbonyl)amino)-3-((tert-butyldiphenylsilyl)oxy)propyl)amino)-2-(4-fluoro-3,5-dimethylphenyl)-4-methyl-6,7-dihydropyrazolo[1,5-a]pyrazine-5(4H)-carboxylate 52b

Tert-butyl 3-amino-2-(4-fluoro-3,5-dimethylphenyl)-4-methyl-6,7-dihydropyrazolo[1,5-*a*]pyrazine-5(4*H*)-carboxylate **52a** (170 mg, 0.45 mmol, prepared by a method disclosed for compound 108a on page 113 of the description of Patent Application WO 2022052958 A1) and tert-butyl (*S*)-(1-((tert-butyldiphenylsilyl)oxy)-3-oxopropyl-2-yl)carbamate **46a** (500 mg, 1.17 mmol, prepared by a method in the document European Journal of Organic Chemistry, 1998, 6, p945-959) were dissolved in methanol (8 mL) and stirred at room temperature overnight. Sodium cyanoborohydride (100 mg, 1.59 mmol) was then added. The reaction was continued at room temperature for 4 hours. The mixture was quenched with water (100 mL) and extracted with dichloromethane (50 mL × 3). The organic phases were combined, washed with a saturated sodium chloride solution, and dried over anhydrous sodium sulfate. After filtration, the filtrate was concentrated under reduced pressure. The residue was purified by silica gel column chromatography with eluent system B to obtain the title compound **52b** (330 mg, yield: 92%).

MS m/z (ESI): 786.8 [M+1].

### Step 2

### Tert-butyl 2-(4-fluoro-3,5-dimethylphenyl)-3-((R)-4-(hydroxymethyl)-2-oxoimidazolidin-1-yl)-4-methyl-6,7-dihydropyrazolo[1,5-a]pyrazine-5(4H)-carboxylate 52c

Compound **52b** (330 mg, 0.42 mmol) and cesium carbonate (500 mg, 1.53 mmol) were dissolved in *N*-methylpyrrolidone (15 mL). After displacement with nitrogen three times, the mixture was reacted at 110°C for 16 hours. The reaction mixture was cooled to room temperature. Water (100 mL) was added. The mixture was extracted with ethyl acetate (60 mL × 3). The organic phases were combined, washed with a saturated sodium chloride solution, and dried over anhydrous sodium sulfate. After filtration, the filtrate was concentrated under reduced pressure. The residue was purified by silica gel column chromatography with eluent system A to obtain the title compound **52c** (155 mg, yield: 78%).

MS m/z (ESI): 474.5 [M+1].

### Step 3

### Tert-butyl 2-(4-fluoro-3,5-dimethylphenyl)-4-methyl-3-((R)-4-(((methylsulfonyl)oxy)methyl)-2-oxoimidazolidin-1-yl)-6,7-dihydropyrazolo[1,5-a]pyrazine-5(4H)-carboxylate 52d

Compound **52c** (70 mg, 0.15 mmol) and triethylamine (73 mg, 0.72 mmol) were dissolved in anhydrous dichloromethane (5 mL) and cooled to 0°C. Methanesulfonyl chloride (59 mg, 0.52 mmol) was added and stirred for 1 hour. 50 mL of water was added. The mixture was extracted with dichloromethane (30 mL × 3). The organic phases were combined, washed with a saturated sodium chloride solution, and dried over anhydrous sodium sulfate and filtered. The reaction solution was concentrated under reduced pressure to obtain the crude title compound **52d** (60 mg). The product was directly used in the next reaction without purification.

MS m/z (ESI): 552.4 [M+1].

### Step 4

### Tert-butyl 3-((R)-4-(((1-cyclopropyl-4-fluoro-5-iodo-1H-indazol-6-yl)oxy)methyl)-2-oxoimidazolidin-1-yl)-2-(4-fluoro-3,5-dimethylphenyl)-4-methyl-6,7-dihydropyrazolo[1,5-a]pyrazine-5(4H)-carboxylate 52e

Compound **52d** (60 mg, 0.11 mmol) and compound **13i** (45 mg, 0.14 mmol) were dissolved in *N,N-*dimethylformamide (5.0 mL). Potassium carbonate (50 mg, 0.36 mmol) was added and reacted at 80°C for 3 hours. The reaction solution was poured into 20 mL of water and extracted with ethyl acetate (30 mL × 2). The organic phases were combined, washed with a saturated sodium chloride solution, and dried over anhydrous sodium sulfate. After filtration, the filtrate was concentrated under reduced pressure. The residue was purified by silica gel column chromatography with eluent system A to obtain the title compound **52e** (68 mg, yield: 81%).

MS m/z (ESI): 774.6 [M+1].

### Step 5

### Tert-butyl 3-((R)-7-cyclopropyl-10-fluoro-1-oxo-3a,4-dihydro-1H,7H-imidazo[1',5':4,5][1,4]oxazino[3,2-f]indazol-2(3H)-yl)-2-(4-fluoro-3,5-dimethylphenyl)-4-methyl-6,7-dihydropyrazolo[1,5-a]pyrazine-5(4H)-carboxylate

### 52f

Compound **52e** (68 mg, 0.09 mmol) and (1*S*,2*S*)-(+)-N,N'-dimethyl-1,2-cyclohexanediamine (12 mg, 0.08 mmol, Adamas) were dissolved in *N-*methylpyrrolidone (5.0 mL). Potassium carbonate (40 mg, 0.29 mmol) and cuprous iodide (8 mg, 0.4 mmol) were added. After displacement with nitrogen three times, the mixture was reacted at 125°C for 2 hours. The reaction solution was poured into 20 mL of water and extracted with ethyl acetate (30 mL × 2). The organic phases were combined, washed with a saturated sodium chloride solution, and dried over anhydrous sodium sulfate. After filtration, the filtrate was concentrated under reduced pressure. The residue was purified by silica gel column chromatography with eluent system B to obtain the title compound **52f** (35 mg, yield: 62%).

MS m/z (ESI): 646.5 [M+1].

### Step 6

### (3aR)-7-cyclopropyl-10-fluoro-2-(2-(4-fluoro-3,5-dimethylphenyl)-4-methyl-4,5,6,7-tetrahydropyrazolo[1,5-a]pyrazin-3-yl)-2,3,3a,4-tetrahydro-1H,7H-imidazo[1',5':4,5][1,4]oxazino[3,2-f]indazol-1-one 52g

Compound **52f** (35 mg, 0.05 mmol) was dissolved in ethyl acetate (2 mL). A 4 M solution of hydrogen chloride in 1,4-dioxane (2 mL) was added and stirred at room temperature for 2 hours. The reaction solution was concentrated under reduced pressure to obtain the crude title compound **52g** (30 mg). The product was directly used in the next reaction without purification.

MS m/z (ESI): 546.4 [M+1].

### Step 7

### (R)-7-cyclopropyl-2-((R)-5-(5-((S)-2,2-dimethyltetrahydro-2H-pyran-4-yl)-1-((1S,2S)-2-methyl-1-(5-oxo-4,5-dihydro-1,2,4-oxadiazol-3-yl)cyclopropyl)-1H-indole-2-carbonyl)-2-(4-fluoro-3,5-dimethylphenyl)-4-methyl-4,5,6,7-tetrahydropyrazolo[1,5-a]pyrazin-3-yl)-10-fluoro-2,3,3a,4-tetrahydro-1H,7H-imidazo[1',5':4,5][1,4]oxazino[3,2-f]indazol-1-one 52-1 and

### (R)-7-cyclopropyl-2-((S)-5-(5-((S)-2,2-dimethyltetrahydro-2H-pyran-4-yl)-1-((1S,2S)-2-methyl-1-(5-oxo-4,5-dihydro-1,2,4-oxadiazol-3-yl)cyclopropyl)-1H-indole-2-carbonyl)-2-(4-fluoro-3,5-dimethylphenyl)-4-methyl-4,5,6,7-tetrahydropyrazolo[1,5-a]pyrazin-3-yl)-10-fluoro-2,3,3a,4-tetrahydro-1H,7H-imidazo[1',5':4,5][1,4]oxazino[3,2-f]indazol-1-one 52-2

Compound **52g** (30 mg, 0.05 mmol) and compound **1w** (25 mg, 0.06 mmol) were dissolved in *N,N*-dimethylformamide (3 mL). *N,N*-diisopropylethylamine (36 mg, 0.28 mmol) was added. After the raw materials were fully dissolved, *O*-(7-azabenzotriazol-1-yl)-*N,N,N',N'-*tetramethyluronium hexafluorophosphate (40 mg, 0.1 mmol) was added and stirred at room temperature overnight. The reaction solution was poured into 10 mL of water and extracted with ethyl acetate (10 mL × 3). The organic phases were combined, washed with a saturated sodium chloride solution, and dried over anhydrous sodium sulfate. After filtration, the filtrate was concentrated under reduced pressure. The residue was purified by preparative high-performance liquid chromatography (instrument model: Gilson-GX-281, chromatographic column: Welch Xtimate Phenyl-Hexyl, 30 × 250 mm; 5 µm; mobile phase: water (10 mM ammonium bicarbonate) and acetonitrile, with gradient ratio: acetonitrile 40%-60%, flow rate: 30 mL/min) to obtain the title compound (11 mg, yield: 23%) and (7 mg, yield: 14%).

Single-configuration compound (shorter retention time, 11 mg):
MS m/z (ESI): 939.8 [M+1].

¹H NMR (500 MHz, CDCl₃): δ 11.29-10.87 (m, 1H), 8.08-7.89 (m, 1H), 7.70-7.46 (m, 2H), 7.40-7.30 (m, 3H), 7.00-6.89 (m, 1H), 6.84-6.61 (m, 1H), 6.49-6.10 (m, 1H), 5.48-4.88 (m, 2H), 4.51-4.29 (m, 2H), 4.28-4.12 (m, 1H), 4.08-3.63 (m, 7H), 3.58-3.43 (m, 1H), 3.40-3.22 (m, 1H), 3.20-2.96 (m, 1H), 2.38-2.31 (m, 6H), 2.08-2.00 (m, 1H), 1.98-1.90 (m, 1H), 1.87-1.73 (m, 3H), 1.69-1.63 (m, 4H), 1.53-1.45 (m, 2H), 1.25-1.19 (m, 4H), 1.18-1.14 (m, 4H), 0.90 (t, 2H).

Single-configuration compound (longer retention time, 7 mg):
MS m/z (ESI): 939.7 [M+1].

¹H NMR (500 MHz, CDCl₃): δ 10.94 (s, 1H), 8.02-7.91 (m, 1H), 7.68-7.45 (m, 2H), 7.32 (s, 2H), 7.25 (s, 1H), 6.99-6.85 (m, 1H), 6.83-6.65 (m, 1H), 5.95-5.85 (m, 1H), 5.52-5.16 (m, 3H), 4.76-4.62 (m, 1H), 4.53-4.19 (m, 4H), 4.13-3.78 (m, 7H), 3.74-3.41 (m, 2H), 3.17 (d, 1H), 3.10-3.00 (m, 1H), 2.30 (s, 6H), 2.10-1.99 (m, 2H), 1.98-1.93 (m, 1H), 1.84-1.73 (m, 7H), 1.22-1.02 (m, 5H), 0.90 (t, 2H).

### Example 53

### 3-(1-(7-((S)-2,2-dimethyltetrahydro-2H-pyran-4-yl)-2-((S)-2-(4-fluoro-3,5-dimethylphenyl)-4-methyl-3-(1-(1-methyl-1H-indazol-5-yl)-6-oxo-1,6-dihydropyrimidin-5-yl)-4,5,6,7-tetrahydro-2H-pyrazolo[4,3-c]pyridine-5-carbonyl)indolizin-3-yl)cyclopropyl)-1,2,4-oxadiazol-5(4H)-one 53-1 or

### 3-(1-(7-((R)-2,2-dimethyltetrahydro-2H-pyran-4-yl)-2-((S)-2-(4-fluoro-3,5-dimethylphenyl)-4-methyl-3-(1-(1-methyl-1H-indazol-5-yl)-6-oxo-1,6-dihydropyrimidin-5-yl)-4,5,6,7-tetrahydro-2H-pyrazolo[4,3-c]pyridine-5-carbonyl)indolizin-3-yl)cyclopropyl)-1,2,4-oxadiazol-5(4H)-one 53-2

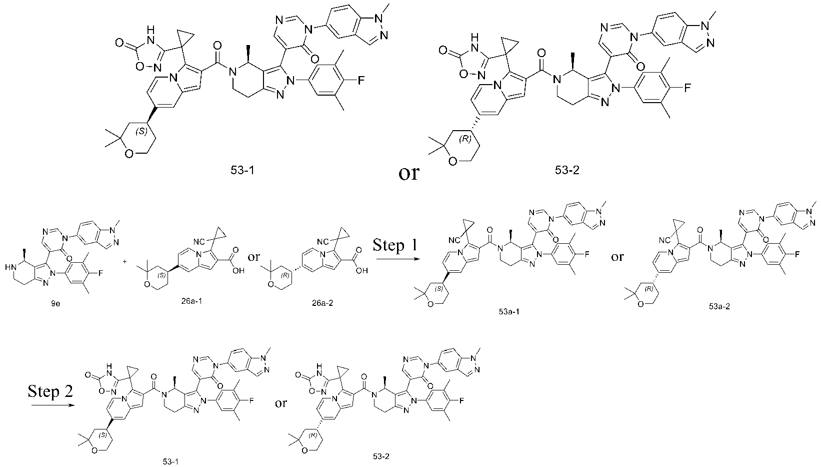

### Step 1

### 1-(7-((S)-2,2-dimethyltetrahydro-2H-pyran-4-yl)-2-((S)-2-(4-fluoro-3,5-dimethylphenyl)-4-methyl-3-(1-(1-methyl-1H-indazol-5-yl)-6-oxo-1,6-dihydropyrimidin-5-yl)-4,5,6,7-tetrahydro-2H-pyrazolo[4,3-c]pyridine-5-carbonyl)indolizin-3-yl)cyclopropane-1-carbonitrile 53a-1 or

### 1-(7-((R)-2,2-dimethyltetrahydro-2H-pyran-4-yl)-2-((S)-2-(4-fluoro-3,5-dimethylphenyl)-4-methyl-3-(1-(1-methyl-1H-indazol-5-yl)-6-oxo-1,6-dihydropyrimidin-5-yl)-4,5,6,7-tetrahydro-2H-pyrazolo[4,3-c]pyridine-5-carbonyl)indolizin-3-yl)cyclopropane-1-carbonitrile 53a-2

Compound **9e** (65 mg, 0.13 mmol) and compound **26a-2 or 26a-1** (40 mg, 0.12 mmol, prepared in one step from the compound with a longer retention time among compounds **26c-2** and **26c-1)** were dissolved in *N,N*-dimethylformamide (3 mL). *O-*(7-azabenzotriazol-1-yl)-*N,N,N',N'*-tetramethyluronium hexafluorophosphate (135 mg, 0.36 mmol) and *N,N*-diisopropylethylamine (77 mg, 0.59 mmol) were added and stirred at room temperature for 3 hours. The reaction solution was poured into 10 mL of water and extracted with ethyl acetate (10 mL × 3). The organic phases were combined, washed with a saturated sodium chloride solution, and dried over anhydrous sodium sulfate. After filtration, the filtrate was concentrated under reduced pressure. The residue was purified by silica gel column chromatography with eluent system B to obtain the title compound **53a-1** or **53a-2** (85 mg, yield: 89%).

MS m/z (ESI): 804.8 [M+1].

### Step 2

### 3-(1-(7-((S)-2,2-dimethyltetrahydro-2H-pyran-4-yl)-2-((S)-2-(4-fluoro-3,5-dimethylphenyl)-4-methyl-3-(1-(1-methyl-1H-indazol-5-yl)-6-oxo-1,6-dihydropyrimidin-5-yl)-4,5,6,7-tetrahydro-2H-pyrazolo[4,3-c]pyridine-5-carbonyl)indolizin-3-yl)cyclopropyl)-1,2,4-oxadiazol-5(4H)-one 53-1 or

### 3-(1-(7-((R)-2,2-dimethyltetrahydro-2H-pyran-4-yl)-2-((S)-2-(4-fluoro-3,5-dimethylphenyl)-4-methyl-3-(1-(1-methyl-1H-indazol-5-yl)-6-oxo-1,6-dihydropyrimidin-5-yl)-4,5,6,7-tetrahydro-2H-pyrazolo[4,3-c]pyridine-5-carbonyl)indolizin-3-yl)cyclopropyl)-1,2,4-oxadiazol-5(4H)-one 53-2

Compound **53a-1** or **53a-2** (85 mg, 0.11 mmol, prepared in two steps from the compound corresponding to a longer retention time among compounds **26c-2** and **26c-1**) and hydroxylamine hydrochloride (75 mg, 1.07 mmol) were dissolved in dimethyl sulfoxide (3 mL). Sodium bicarbonate (90 mg, 1.07 mmol) was added and reacted under stirring at 60°C for 2 hours. The reaction mixture was cooled to room temperature, quenched with water (10 mL), and extracted with ethyl acetate (30 mL × 3). The organic phase was dried over anhydrous sodium sulfate. After filtration, the filtrate was concentrated under reduced pressure. The residue was dissolved in dimethyl sulfoxide (3 mL). *N,N'*-carbonyldiimidazole (45 mg, 0.28 mmol) and bicyclo[5.4.0]-1,8-diaza-7-nonene (51 mg, 0.33 mmol) were added and reacted under stirring at room temperature for 1 hour. The reaction solution was quenched with water (10 mL) and extracted with ethyl acetate (30 mL × 3). The organic phase was dried over anhydrous sodium sulfate. After filtration, the filtrate was concentrated under reduced pressure. The residue was purified by preparative high-performance liquid chromatography (instrument model: Waters-2545, chromatographic column: Welch Xtimate, Prep 30 × 250 mm; 5 µm; C18, mobile phase: water (10 mM ammonium bicarbonate) and acetonitrile, with gradient ratio: acetonitrile 35%-50%, flow rate: 30 mL/min) to obtain the title compound **53-1** or **53-2** (25 mg, yield: 27%).

MS m/z (ESI): 861.7 [M-1].

¹H NMR (500 MHz, CDCl₃): δ8.32-8.10 (m, 2H), 8.09-7.99 (m, 1H), 7.95 (s, 1H), 7.68 (d, 1H), 7.60-7.49 (m, 1H), 7.31 (dd, 1H), 7.23 (s, 1H), 7.10-7.03 (m, 2H), 6.61 (dd, 1H), 6.40-6.34 (m, 1H), 5.83 (s, 1H), 4.38-4.27 (m, 1H), 4.17-4.10 (m, 3H), 3.90-3.85 (m, 1H), 3.81 (td, 1H), 3.55-3.37 (m, 1H), 3.17-3.04 (m, 1H), 3.00-2.83 (m, 2H), 2.33-2.18 (m, 7H), 2.16-2.04 (m, 1H), 1.78-1.73 (m, 2H), 1.69-1.67 (m, 1H), 1.58-1.53 (m, 2H), 1.45 (d, 3H), 1.33 (s, 3H), 1.29 (s, 3H), 1.20-1.08 (m, 2H).

### Biological evaluation

### Test Example 1: Evaluation of agonist activity against GLP-1 receptor

### I. Purpose

This experiment was intended to test the agonist activity of compound molecules against hGLP-1 receptor and evaluate the in vitro activity of the molecules according to EC₅₀. In this experiment, ONE-Glo^{™} Luciferase Assay System (ONE-Glo^{™} Luciferase Assay System, Promega, E6110) was used. Under the action of the compound molecules, GLP-1R downstream signaling pathways were activated to cause elevated cAMP level. The binding of cAMP to CRE could initiate the transcriptional expression of a CRE downstream luciferase gene. The luciferase could emit fluorescence when reacting with a substrate thereof. The fluorescence signal, which reflected the activity of the compound for agonizing GLP-1 receptor, was measured by means ONE-Glo^{™} reagent.

### II. Experimental method

A stably transfected CHO-K1/CRE-luc/hGLP-1 receptor cell strain (hGLP-1 receptor plasmid constructed in-house and CRE-luc plasmid Promega E8471) was constructed. CHO-K1/CRE-luc/hGLP-1 receptor cells were digested, centrifuged, and then resuspended. The single cell suspension was uniformly mixed and adjusted to a viable cell density of 2.78 × 10⁵ cells/ml with a cell culture medium (DME/F-12 + 10% FBS). The resulting solution was added to a 96-well cell culture plate at 90 µL/well (Corning, #3903). The culture plate was incubated in an incubator for 16 h (37°C, 5% CO₂).

The compound was dissolved in DMSO to prepare a stock solution with an initial concentration of 20 mM. The starting concentration of the small molecule compound was 0.02 mM. The compound was subjected to 3-fold dilution for 10 points, wherein the 11th point was DMSO. Another 96-well plate was taken, to which was added 95 µL/well of a cell culture medium (DME/F-12 + 10% FBS), followed by 5 µL/well of test samples with different concentrations. The mixtures were uniformly mixed. Next, 10 µL/well of test samples with different concentrations were added to the cell culture plate, with two duplicate wells for each sample. The culture plate was incubated in an incubator for 5 h (37°C, 5% CO₂). The 96-well cell culture plate was taken out. 100 µL/well of ONE-Glo^{™} reagent was added, followed by incubation at room temperature for 10 minutes. Chemiluminescence was measured in and by a microplate reader (PHERAstar FS, BMG labtech).

### III. Data analysis

Data were processed and analyzed using Microsoft Excel and Graphpad Prism 10. EC₅₀ values of the compounds were obtained. The results are shown in Table 1 below.

**Table 1. Agonist activity of the compounds of the present invention against hGLP-1 receptor**

| Example No. | EC₅₀ (nM) | Emax (fold) |
|---|---|---|
| Compound with shorter retention time among compounds 1-1 and 1-2 | 0.02 | 19.03 |
| 3 | 0.09 | 19.88 |
| 4 | 0.39 | 16.51 |
| 7 | 0.03 | 14.46 |
| 9 | 0.06 | 18.96 |
| Compound with shorter retention time among compounds 11-1 and 11-2 | 0.02 | 21.12 |
| 13 | 0.01 | 21.50 |
| 21-1 or 21-2 (prepared in seven steps from the compound with a shorter retention time among compounds 21i-1 and 21i-2) | 0.01 | 22.02 |
| 23 | 0.02 | 22.76 |
| 24 | 0.03 | 18.51 |
| 25 | 0.02 | 18.46 |
| 26-1 or 26-2 (prepared in three steps from the compound with a longer retention time among compounds **26c-1** and **26c-2**) | 0.02 | 17.54 |
| 29 | 0.02 | 19.58 |
| 30 | 0.02 | 20.59 |
| 31-1 or 31-2 (prepared in seven steps from the compound with a shorter retention time among compounds **21i-1 and 21i-2**) | 0.02 | 20.23 |
| 34-1 or 34-2 (prepared in seven steps from the compound with a shorter retention time among compounds **21i-1 and 21i-2**) | 0.04 | 21.19 |
| 35-1 or 35-2 (prepared in seven steps from the compound with a shorter retention time among compounds **21i-1 and 21i-2**) | 0.02 | |
| 37 | 0.03 | 18.06 |
| 40 | 0.03 | 19.36 |
| 41 | 0.05 | 18.56 |
| 42 | 0.05 | 19.67 |
| 46 | 0.05 | 16.76 |
| 50 | 0.04 | 18.87 |
| 51 | 0.02 | 21.19 |
| Compound with longer retention time among compounds 52-1 and 52-2 | 0.03 | 20.70 |

| | | |
|---|---|---|
| Conclusion: The compounds of the present invention have high agonist activity against GLP-1 receptor. | | |

### Test Example 2: Pharmacokinetic evaluation

### I. Monkey test

### 1. Abstract

Using cynomolgus monkeys as test animals, the plasma concentrations of the example compounds at different time points were determined by an LC-MS/MS method following administration to the monkeys by intragastric gavage (i.g.). The pharmacokinetic behaviors of the compounds of the present invention in monkeys were investigated, and the pharmacokinetic profiles thereof were evaluated.

### 2. Experimental protocol

### 2.1 Experimental compounds

Compound 9.

### 2.2 Experimental animals

Compound 9: Three cynomolgus monkeys (two males and one female) were provided by Guangxi Nanning Tongling Shengwu Keji Youxian Gongsi, with production license SCXK Gui-2020-0002.

Compound (5 mpk): Equal numbers of male and female cynomolgus monkeys were provided by Suzhou Guochen Biotechnology Co., Ltd., with production license SYXK (Su) 2020-0017.

### 2.3 Drug formulation

Compound 9: A certain amount of the test compound was separately weighed. 5% DMSO + 30% PG + 30% PEG400 + 35% normal saline were added to prepare a 0.4 mg/mL colorless and clear solution.

Compound (5 mpk): A certain amount of the test compound was separately weighed. 10% PEG400 + 10% PG + 80% glycine buffer (100 mM glycine, 26 mM NaOH, pH 9) were added to prepare a 1.0 mg/mL solution.

### 2.4. Administration

Compound 9: Administration dose was 1 mg/kg and administration volume was 2.5 mL/kg.

Compound (5 mpk): Administration dose was 5 mg/kg and administration volume was 5 mL/kg.

### 3. Operations

0.5 mL of blood samples were collected from the vein before administration and at 0.25, 0.5, 1.0, 2.0, 4.0, 6.0, 8.0, 11.0, and 24.0 hours after administration and placed in EDTA-K2 anticoagulant tubes, followed by centrifugation at 10000 rpm for 2 minutes (4°C). The plasma was separated within 1 hour and stored at -20°C or -80°C for analysis. The process from blood collection to centrifugation was operated under ice bath condition.

Determination of test compound contents in rat plasma after administration of different compounds:
Compound 9: 50 µL of the monkey plasma sample collected at each time point after administration was taken. 25 µL of an internal standard acetonitrile solution (tolbutamide, 1 ug/mL) was added, followed by 450 µL of an acetonitrile solution. The mixture was vortexed and mixed, followed by centrifugation at 3700 rpm for 10 min. The supernatant was taken for LC-MS/MS analysis.

Compound (5 mpk): 30 µL of the monkey plasma sample collected at each time point after administration was taken. 30.0 µL of an internal standard solution (verapamil) and 200 µL of acetonitrile were added. The mixture was vortexed and mixed, followed by centrifugation at 2600 g for 10 min. The supernatant was taken. An equal volume of pure water was added for LC-MS/MS analysis.

### 4. Pharmacokinetic parameter results

**Table 2. Pharmacokinetic parameters of compounds of the present invention in monkeys**

| Compound No. | Administ ration dose (mg/kg) | Plasma concentration Cmax (ng/mL) | Area under the curve AUC₀₋ₜ (h*ng/m L) | Half life T1/2 (h) | Clearance CL/F (mL/min/ kg) | Apparent volume of distribution Vz/F (mL/kg) | Bioavaila bility F (%) |
|---|---|---|---|---|---|---|---|
| 9 | 1 | 178 | 1184 | 5.88 | 12.4 | 6115 | 44.5 |

| | | | | | | | |
|---|---|---|---|---|---|---|---|
| Conclusion: The compounds of the present invention exhibit high exposure in monkeys, demonstrating pharmacokinetic advantages. | | | | | | | |

## Claims

1. A compound represented by general formula (I) or a pharmaceutically acceptable salt thereof: 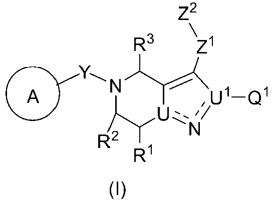 **characterized in that**: 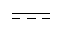
is a single bond or a double bond;
Q¹ is aryl or heteroaryl, and the aryl and heteroaryl are each independently and optionally substituted with one or more R^{Q1};
Z² is selected from the group consisting of a hydrogen atom, a deuterium atom, cycloalkyl, heterocyclyl, 6- to 10-membered aryl, and 5- to 10-membered heteroaryl, wherein the cycloalkyl, heterocyclyl, 6- to 10-membered aryl, and 5- to 10-membered heteroaryl are each independently and optionally substituted with one or more R^{Z2};
Z¹ is selected from the group consisting of a bond, -heterocyclyl-, - heterocyclyl-L^{2A}-, -L^{2A}-heterocyclyl-, -L^{2A}-heterocyclyl-L^{2A}-, -heteroaryl-L^{2A}-, - L^{2A}-heteroaryl-L^{2A}-, -N=, -NR^{aa}-L^{2A}-, and -NR^{aa}-C(O)-NR^{bb}-L^{2A}-, wherein the heterocyclyl and heteroaryl are each independently and optionally substituted with one or more R^{Z1};
L^{2A} is selected from the group consisting of a bond, alkyl, deuterioalkyl, heteroalkyl, and -N=; 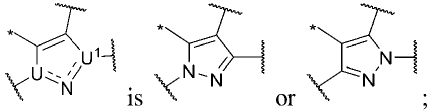
Y is selected from the group consisting of C(O), S(O), S(O)₂, deuterioalkylene, and alkylene;
R¹, R², and R³ are the same or different and are each independently selected from the group consisting of a hydrogen atom, a deuterium atom, halogen, alkyl, alkenyl, alkynyl, alkoxy, alkoxyalkyl, haloalkyl, haloalkoxy, hydroxyalkyl, cyano, amino, nitro, hydroxyl, oxo, -(CH₂)ₓNR^{11a}R^{11b}, -(CH₂)ₓC(O)NR^{11a}R^{11b}, - NR⁰⁰C(O)R¹², -C(O)R¹², -C(O)OR¹², -OC(O)R¹², -S(O)ₜR¹², -S(O)ₜNR^{11a}R^{11b}, - NR⁰⁰S(O)ₜR¹², -(CH₂)ₓP(O)R¹²R¹³, -S(O)ₜOR¹², -OS(O)ₜR¹², -OR¹², =CR^{12a}R^{13a}, =NR⁰⁰, C(=NR⁰⁰)R¹², S(=NR⁰⁰)(O)R¹², S(=NR⁰⁰)R¹², cycloalkyl, cycloalkylalkyl, heterocyclyl, heterocyclylalkyl, aryl, and heteroaryl; the alkyl, alkenyl, alkynyl, alkoxy, cycloalkyl, cycloalkylalkyl, heterocyclyl, heterocyclylalkyl, aryl, and heteroaryl are each independently and optionally substituted with one or more R⁰¹;
ring A is selected from the group consisting of 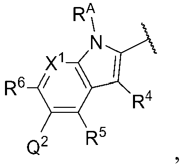 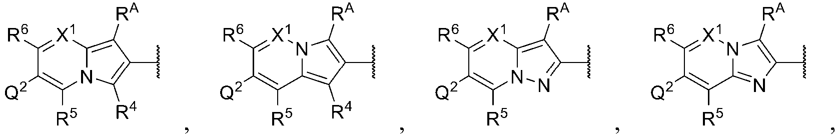 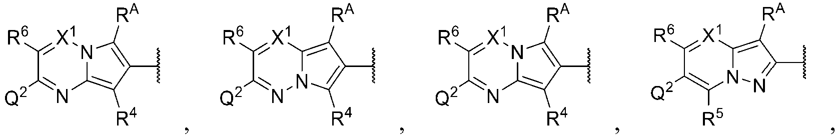 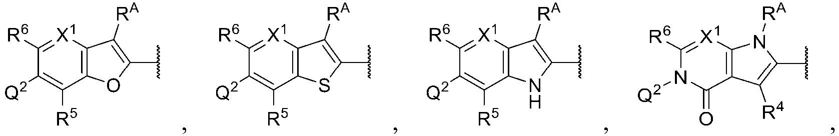 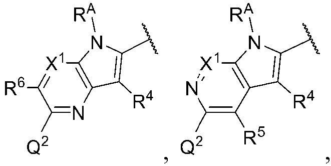 and bicyclic heteroaryl substituted with R^{A} and Q²; the bicyclic heteroaryl is optionally substituted with one or more R⁰¹; 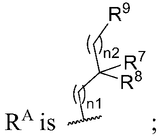
Q² is selected from the group consisting of cycloalkyl, heterocyclyl, aryl, and heteroaryl, wherein the cycloalkyl, heterocyclyl, aryl, and heteroaryl are each independently and optionally substituted with one or more R^{Q2};
X¹ is CR^{x1} or N;
R⁴, R⁵, R⁶, and R^{x1}, when present, are the same or different and are each independently selected from the group consisting of a hydrogen atom, a deuterium atom, halogen, alkyl, alkenyl, alkynyl, alkoxy, alkoxyalkyl, haloalkyl, haloalkoxy, hydroxyalkyl, cyano, amino, nitro, hydroxyl, -(CH₂)ₓNR^{11a}R^{11b}, -NR⁰⁰C(O)R¹², - C(O)R¹², -C(O)OR¹², -(CH₂)ₓC(O)NR^{11a}R^{11b}, -OC(O)R¹², -S(O)ₜR¹², - S(O)ₜNR^{11a}R^{11b}, -NR⁰⁰S(O)ₜR¹², -(CH₂)ₓP(O)R¹²R¹³, -S(O)ₜOR¹², -OS(O)ₜR¹², -OR¹², C(=NR⁰⁰)R¹², S(=NR⁰⁰)(O)R¹², S(=NR⁰⁰)R¹², cycloalkyl, cycloalkylalkyl, heterocyclyl, heterocyclylalkyl, aryl, and heteroaryl; the alkyl, alkenyl, alkynyl, alkoxy, cycloalkyl, cycloalkylalkyl, heterocyclyl, heterocyclylalkyl, aryl, and heteroaryl are each independently and optionally substituted with one or more R⁰¹;
or Q² and R⁶, together with the carbon atoms to which they are attached, form ring B, or Q² and R⁵, together with the carbon atoms to which they are attached, form ring B; or R^{Q2} and R⁶, together with the ring atoms to which they are attached, form ring B⁰, or R^{Q2} and R⁵, together with the ring atoms to which they are attached, form ring B⁰;
each ring B is independently selected from the group consisting of cycloalkyl, heterocyclyl, aryl, and heteroaryl, and the ring B is optionally substituted with one or more R^{B}; each ring B⁰ is independently selected from the group consisting of cycloalkyl, heterocyclyl, aryl, and heteroaryl, and the ring B⁰ is optionally substituted with one or more R^{B};
R⁹ is selected from the group consisting of -C(O)OR¹², -C(O)NR^{11a}R^{11b}, - S(O)ₜR¹², -S(O)ₜNR^{11a}R^{11b}, heterocyclyl, and heteroaryl, wherein the heterocyclyl and heteroaryl are each independently and optionally substituted with one or more R^{9a};
R⁷ and R⁸ are the same or different and are each independently selected from the group consisting of a hydrogen atom, a deuterium atom, halogen, alkyl, alkenyl, alkynyl, alkoxy, alkoxyalkyl, haloalkyl, haloalkoxy, hydroxyalkyl, cyano, amino, nitro, hydroxyl, oxo, -(CH₂)ₓNR^{11a}R^{11b}, -(CH₂)ₓC(O)NR^{11a}R^{11b}, -NR⁰⁰C(O)R¹², - C(O)R¹², -C(O)OR¹², -OC(O)R¹², -S(O)ₜR¹², -S(O)ₜNR^{11a}R^{11b}, -NR⁰⁰S(O)ₜR¹², - (CH₂)ₓP(O)R¹²R¹³, -S(O)ₜOR¹², -OS(O)ₜR¹², -OR¹², =CR^{12a}R^{13a}, =NR⁰⁰, C(=NR⁰⁰)R¹², S(=NR⁰⁰)(O)R¹², S(=NR⁰⁰)R¹², cycloalkyl, cycloalkylalkyl, heterocyclyl, heterocyclylalkyl, aryl, and heteroaryl; the alkyl, alkenyl, alkynyl, alkoxy, cycloalkyl, cycloalkylalkyl, heterocyclyl, heterocyclylalkyl, aryl, and heteroaryl are each independently and optionally substituted with one or more R⁰¹; or
R⁷ and R⁸, together with the atoms to which they are attached, form 3- to 5-membered cycloalkyl or heterocyclyl, wherein the 3- to 5-membered cycloalkyl and heterocyclyl are each independently and optionally substituted with one or more R⁷⁸;
n1 is 0, 1, 2, 3, 4, or 5; n2 is 0, 1, 2, 3, 4, or 5;
R^{Q1}, R^{Z2}, R^{Z1}, R⁰¹, R^{Q2}, R^{B}, R^{9a}, and R⁷⁸ are each the same or different and are each independently selected from the group consisting of a deuterium atom, halogen, alkyl, alkenyl, alkynyl, alkoxy, haloalkyl, deuterioalkyl, haloalkoxy, hydroxyalkyl, cyano, amino, nitro, hydroxyl, oxo, =S, -(CH₂)ₓNR^{11a}R^{11b}, -NR⁰⁰C(O)R¹², -C(O)R¹², -C(O)OR¹², -OC(O)R¹², -(CH₂)ₓC(O)NR^{11a}R^{11b}, -S(O)ₜR¹², -S(O)ₜNR^{11a}R^{11b}, - NR⁰⁰S(O)ₜR¹², -(CH₂)ₓP(O)R¹²R¹³, -S(O)ₜOR¹², -OS(O)ₜR¹², -OR¹², =CR^{12a}R^{13a}, =NR⁰⁰, C(=NR⁰⁰)R¹², S(=NR⁰⁰)(O)R¹², S(=NR⁰⁰)R¹², cycloalkyl, heterocyclyl, 6- to 10-membered aryl, and 5- to 10-membered heteroaryl, wherein the alkyl, alkenyl, alkynyl, alkoxy, cycloalkyl, heterocyclyl, 6- to 10-membered aryl, and 5- to 10-membered heteroaryl are each independently and optionally substituted with one or more R*;
or R^{Z2} and R^{Z1}, together with the ring atoms to which they are attached, form ring C;
ring C is selected from the group consisting of cycloalkyl, heterocyclyl, aryl, and heteroaryl, and the ring C is optionally substituted with one or more R^{C};
R* and R^{C} are each the same or different and are each independently selected from the group consisting of a deuterium atom, halogen, alkyl, alkenyl, alkynyl, alkoxy, haloalkyl, haloalkoxy, hydroxyalkyl, cyano, amino, nitro, hydroxyl, oxo, =S, -(CH₂)ₓNR^{11a}R^{11b}, -NR⁰⁰C(O)R¹², -C(O)R¹², -C(O)OR¹², -(CH₂)ₓC(O)NR^{11a}R^{11b}, - S(O)ₜR¹², -S(O)ₜNR^{11a}R^{11b}, -NR⁰⁰S(O)ₜR¹², -(CH₂)ₓP(O)R¹²R¹³, -OC(O)R¹², - S(O)ₜOR¹², -OS(O)ₜR¹², -OR¹², =CR^{12a}R^{13a}, =NR⁰⁰, C(=NR⁰⁰)R¹², S(=NR⁰⁰)(O)R¹², S(=NR⁰⁰)R¹², cycloalkyl, heterocyclyl, aryl, and heteroaryl, wherein the alkyl, alkenyl, alkynyl, alkoxy, cycloalkyl, heterocyclyl, aryl, and heteroaryl are each independently and optionally substituted with one or more identical or different substituents selected from the group consisting of a deuterium atom, oxo, halogen, cyano, amino, hydroxyl, alkyl, haloalkyl, hydroxyalkyl, alkoxy, haloalkoxy, cycloalkyl, heterocyclyl, aryl, and heteroaryl;
R^{11a}, R^{11b}, R¹², and R¹³ are each the same or different and are each independently selected from the group consisting of a hydrogen atom, a deuterium atom, alkyl, alkoxy, hydroxyalkyl, haloalkyl, -C(O)R¹⁵, -C(O)OR¹⁵, -OC(O)R¹⁵, - (CH₂)_{y}NR^{14a}R^{14b}, -(CH₂)_{y}C(O)NR^{14a}R^{14b}, -NR⁰C(O)R¹⁵, -S(O)ₜR¹⁵, -S(O)ₜNR^{14a}R^{14b}, -NR⁰S(O)ₜR¹⁵, -S(O)ₜOR¹⁵, - OS(O)ₜR¹⁵, -OR¹⁵, 4- to 12-membered cycloalkyl, and heterocyclyl; or R^{11a} and R^{11b}, together with the nitrogen atom to which they are attached, form heterocyclyl, wherein the heterocyclyl is optionally substituted with one or more identical or different substituents selected from the group consisting of a deuterium atom, oxo, halogen, cyano, amino, hydroxyl, alkyl, haloalkyl, hydroxyalkyl, alkoxy, and haloalkoxy;
R^{aa}, R^{bb}, R⁰⁰, and R⁰ are each the same or different and are each independently selected from the group consisting of a hydrogen atom, a deuterium atom, alkyl, hydroxyalkyl, haloalkyl, C(O) alkyl, amido, cycloalkyl, and heterocyclyl;
R^{12a} and R^{13a} are each the same or different and are each independently selected from the group consisting of a hydrogen atom, a deuterium atom, halogen, cyano, amino, hydroxyl, alkyl, haloalkyl, hydroxyalkyl, alkoxy, haloalkoxy, cycloalkyl, and heterocyclyl; or R^{12a} and R^{13a}, together with the carbon atoms to which they are attached, form cycloalkyl or heterocyclyl, wherein the cycloalkyl and heterocyclyl are each independently and optionally substituted with one or more identical or different substituents selected from the group consisting of a deuterium atom, halogen, cyano, amino, hydroxyl, oxo, alkenyl, alkynyl, alkyl, haloalkyl, hydroxyalkyl, alkoxy, haloalkoxy, cycloalkyl, and heterocyclyl;
R¹⁵, R^{14a}, and R^{14b} are each the same or different and are each independently selected from the group consisting of a hydrogen atom, a deuterium atom, alkyl, alkoxy, hydroxyalkyl, haloalkyl, C(O) alkyl, amino, amido, cycloalkyl, heterocyclyl, aryl, and heteroaryl, wherein the alkyl, alkoxy, cycloalkyl, heterocyclyl, aryl, and heteroaryl are each independently and optionally substituted with one or more identical or different substituents selected from the group consisting of a deuterium atom, halogen, cyano, nitro, amino, hydroxyl, oxo, alkenyl, alkynyl, alkyl, haloalkyl, hydroxyalkyl, alkoxy, haloalkoxy, cycloalkyl, and heterocyclyl;
x is 0, 1, 2, 3, 4, 5, or 6; y is 0, 1, 2, 3, 4, 5, or 6; and
t is 0, 1, or 2.

2. The compound or the pharmaceutically acceptable salt thereof according to claim 1, **characterized in that** the compound or the pharmaceutically acceptable salt thereof is a compound represented by general formula (IN) or a pharmaceutically acceptable salt thereof: 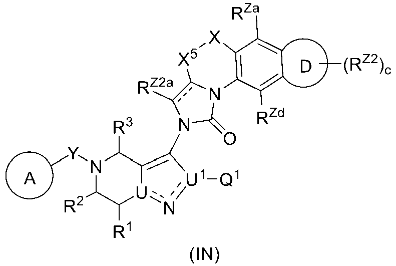
wherein -̅ -̅ -̅ is a single bond or a double bond;
X is selected from the group consisting of a bond, CR^{Z2c}R^{Z2d}CR^{Z2c}R^{Z2d}, CR^{Z2c}R^{Z2d}, O, NR^{cc}, CR^{Z2c}R^{Z2d}O, OCR^{Z2c}R^{Z2d}, and C(O); X⁵ is selected from the group consisting of a bond, CR^{Z2e}R^{Z2f}CR^{Z2e}R^{Z2f}, CR^{Z2e}R^{Z2f}, O, NR^{cc}, CR^{Z2e}R^{Z2f}O, OCR^{Z2e}R^{Z2f}, and C(O);
R^{Z2a}, R^{Z2c}, R^{Z2d}, R^{Z2e}, and R^{Z2f} are the same or different and are each independently a hydrogen atom or R^{Z2};
R^{Za} and R^{Zd} are the same or different and are each independently a hydrogen atom or R^{Z2};
ring D is selected from the group consisting of cycloalkyl, heterocyclyl, aryl, and heteroaryl;
R^{cc} is selected from the group consisting of a hydrogen atom, alkyl, hydroxyalkyl, and cycloalkyl; c is 0, 1, 2, 3, 4, 5, or 6; and 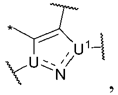 ring A, Y, R¹, R², R³, Q¹, and R^{Z2} are as defined in claim 1.

3. The compound or the pharmaceutically acceptable salt thereof according to claim 1 or 2, **characterized in that** ring A is 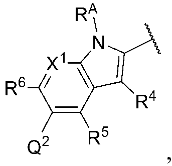 and X¹, Q², R^{A}, R⁴, R⁵, and R⁶ are as defined in claim 1.

4. The compound or the pharmaceutically acceptable salt thereof according to any one of claims 1 to 3, **characterized in that** Q² is tetrahydropyranyl optionally substituted with one or more R^{Q2}, and R^{Q2} is as defined in claim 1; preferably Q² is 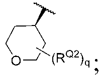 and q is 1, 2, 3, 4, 5, or 6, and R^{Q2} is as defined in claim 1.

5. The compound or the pharmaceutically acceptable salt thereof according to any one of claims 2 to 4, **characterized in that** ring D is 3- to 10-membered heterocyclyl or 5- to 10-membered heteroaryl; preferably 5-membered heteroaryl; and more preferably pyrazolyl.

6. The compound or the pharmaceutically acceptable salt thereof according to any one of claims 2 to 5, **characterized in that** 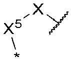 is selected from the group consisting of 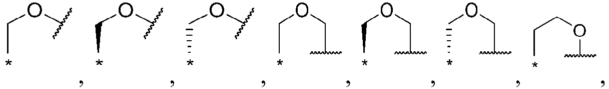 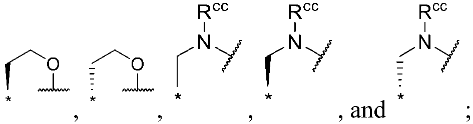 R^{cc} is a hydrogen atom or C₁₋₆ alkyl (preferably a hydrogen atom or methyl); and preferably 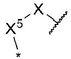 is selected from the group consisting of

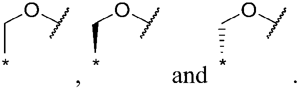

7. The compound or the pharmaceutically acceptable salt thereof according to claim 1 or 3, **characterized in that** the compound or the pharmaceutically acceptable salt thereof is a compound represented by general formula (IM) or a pharmaceutically acceptable salt thereof: 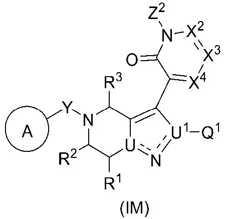 wherein X², X³, and X⁴ are the same or different and are each independently CR^{Z1a} or N; R^{Z1a} is a hydrogen atom or R^{Z1}; and -̅ -̅ -̅, 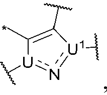 ring A, Y, R¹, R², R³, Q¹, Z², and R^{Z1} are as defined in claim 1.

8. The compound or the pharmaceutically acceptable salt thereof according to claim 1 or 3, **characterized in that** the compound or the pharmaceutically acceptable salt thereof is a compound represented by general formula (IV) or (IV') or a pharmaceutically acceptable salt thereof: 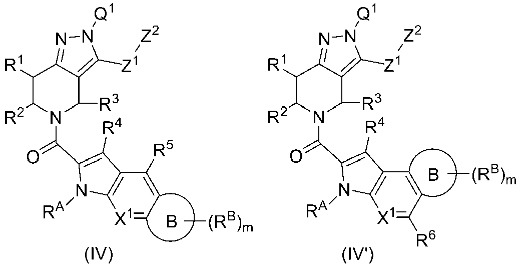 wherein
m is 0, 1, 2, 3, 4, 5, or 6; and
ring B, X¹, Q¹, Z¹, Z², R¹ to R⁴, R⁵, R⁶, R^{A}, and R^{B} are as defined in claim 1.

9. The compound or the pharmaceutically acceptable salt thereof according to claim 8, **characterized in that** ring B is 3- to 12-membered heterocyclyl.

10. The compound or the pharmaceutically acceptable salt thereof according to claim 8, **characterized in that** Z¹ is 3- to 12-membered heterocyclyl optionally substituted with one or more R^{Z1}, and R^{Z1} is as defined in claim 1.

11. The compound or the pharmaceutically acceptable salt thereof according to any one of claims 7 to 10, **characterized in that** Z² is a hydrogen atom or 5- to 10-membered heteroaryl optionally substituted with one or more R^{Z2}, and R^{Z2} is as defined in claim 1; preferably, Z² is a hydrogen atom or 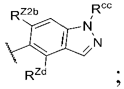 and R^{Z2b} is a hydrogen atom, R^{Zd} is selected from the group consisting of a hydrogen atom, halogen, and C₁₋₆ alkyl, and R^{cc} is selected from the group consisting of a hydrogen atom, C₁₋₆ alkyl, 3- to 6-membered cycloalkyl, and C₁₋₆ deuterioalkyl.

12. The compound or the pharmaceutically acceptable salt thereof according to any one of claims 1 to 11, **characterized in that** Q¹ is phenyl or 6-membered heteroaryl, wherein the phenyl or 6-membered heteroaryl is optionally substituted with one or more R^{Q1}, and R^{Q1} is as defined in claim 1.

13. The compound or the pharmaceutically acceptable salt thereof according to any one of claims 1 to 12, **characterized in that** R^{A} is 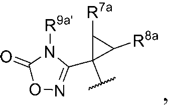 and R^{7a} and R^{8a} are the same or different and are each independently a hydrogen atom or R⁷⁸; R^{9a}' is a hydrogen atom or C₁₋₆ alkyl, and R⁷⁸ is as defined in claim 1; and preferably, R^{A} is 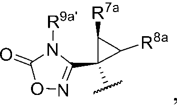 more preferably R^{A} is

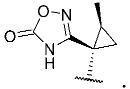

14. The compound or the pharmaceutically acceptable salt thereof according to any one of claims 1 to 13, **characterized in that** R¹ is a hydrogen atom, and/or R² is a hydrogen atom, and/or R³ is C₁₋₆ alkyl, and/or R⁴, R⁵, and R⁶ are hydrogen atoms, and/or X¹ is CH.

15. The compound or the pharmaceutically acceptable salt thereof according to claim 1, **characterized in that** the compound or the pharmaceutically acceptable salt thereof is selected from the group consisting of the following compounds: 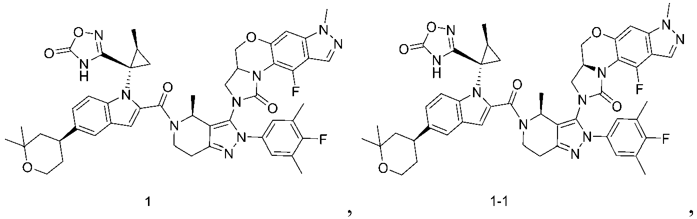 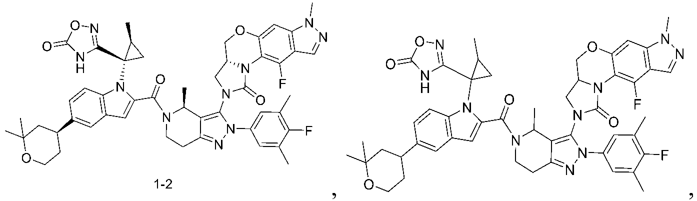 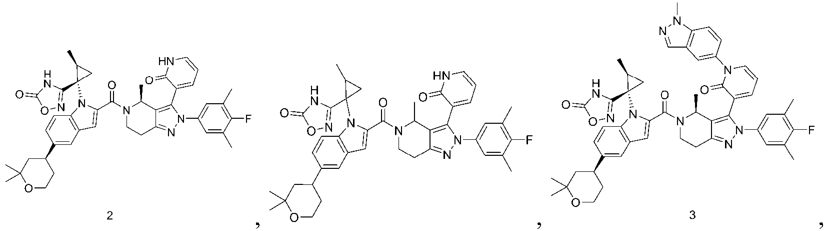 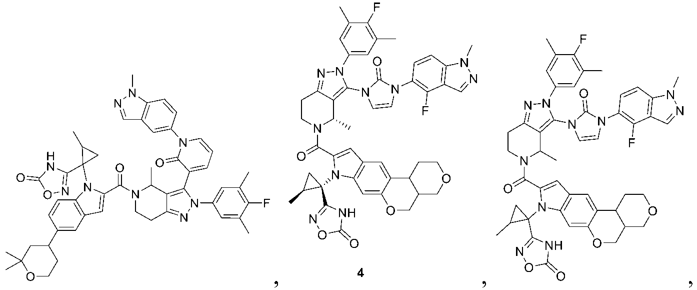 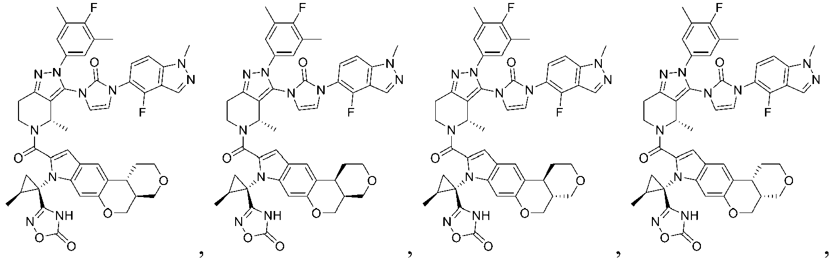 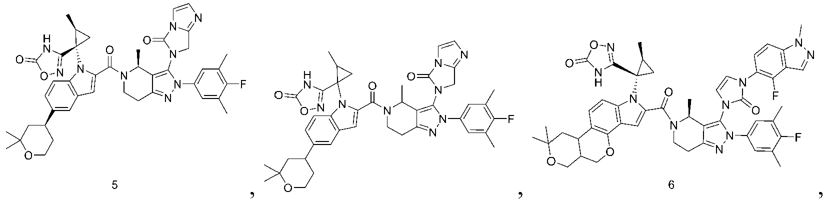 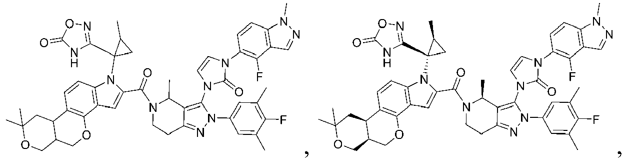 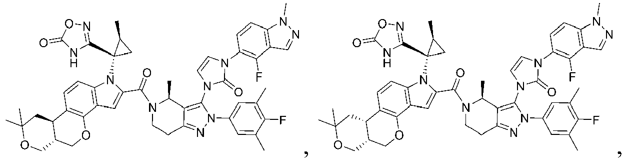 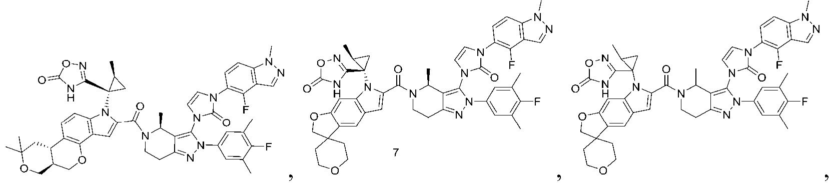 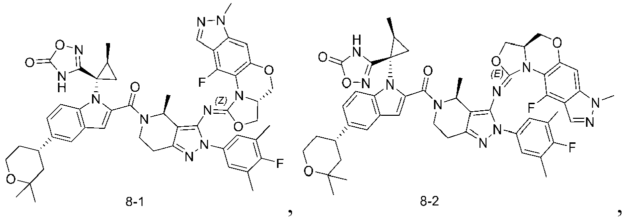 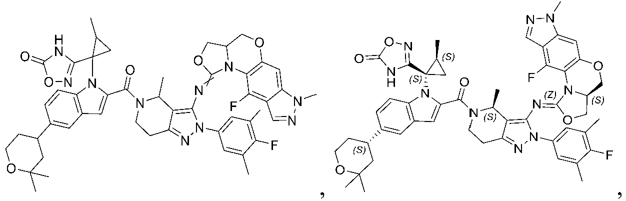 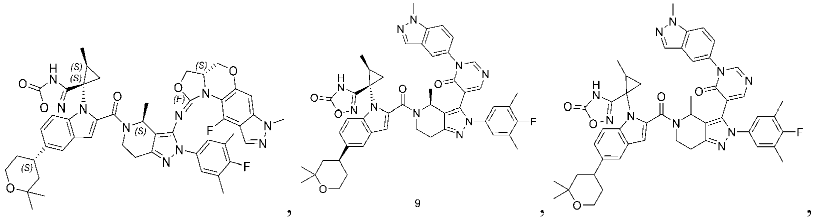 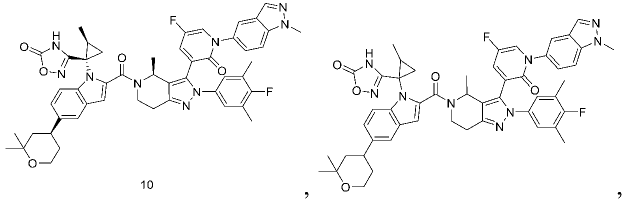 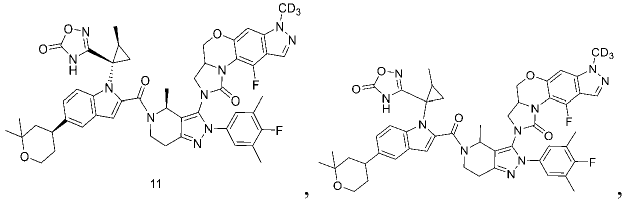 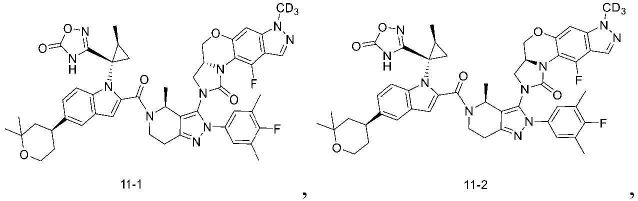 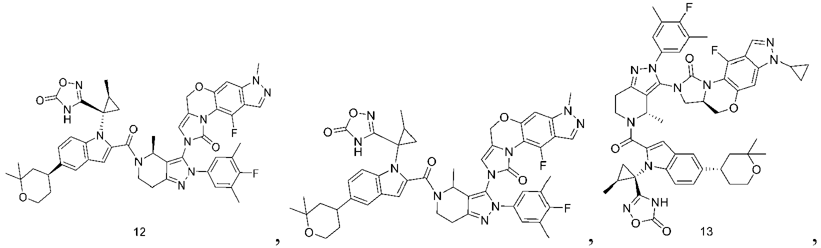 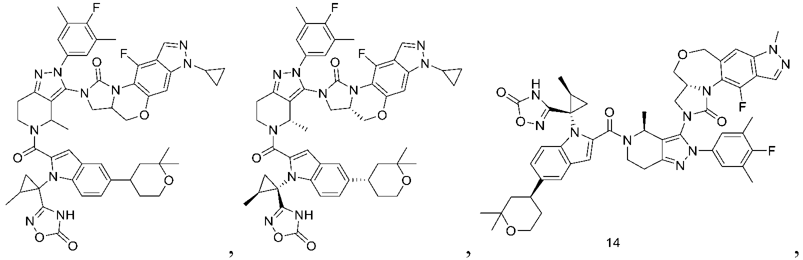 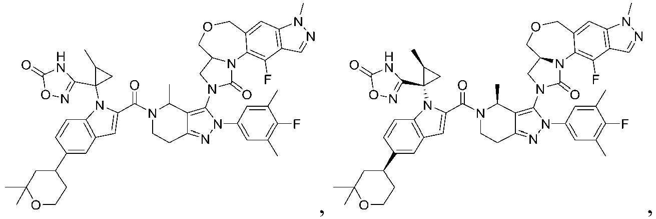 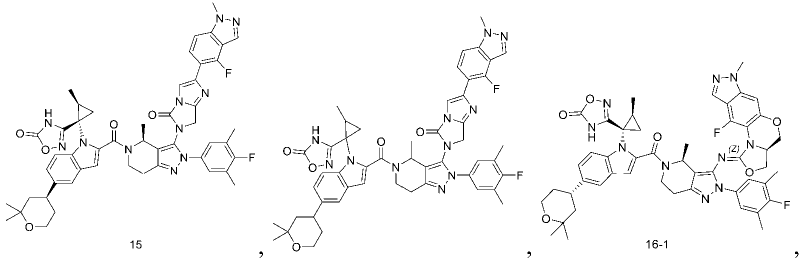 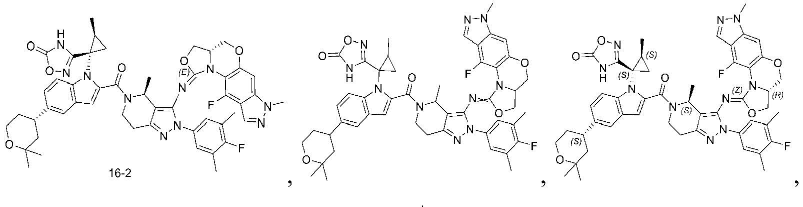 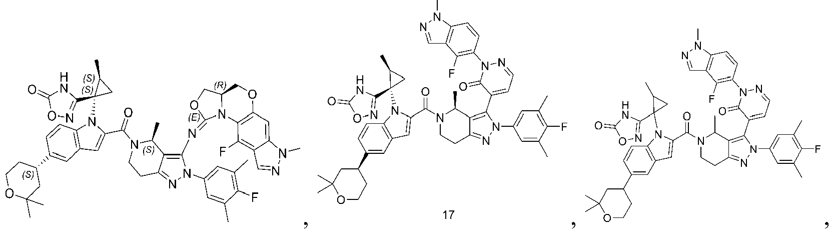 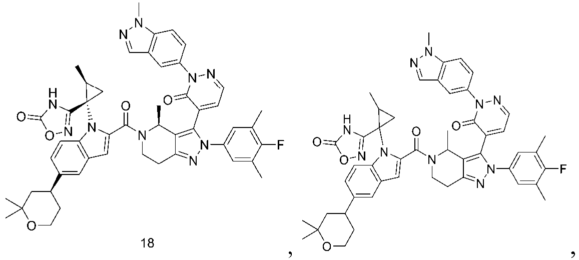 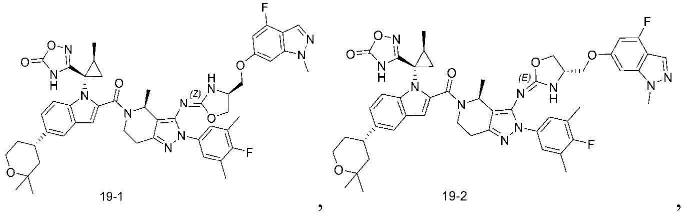 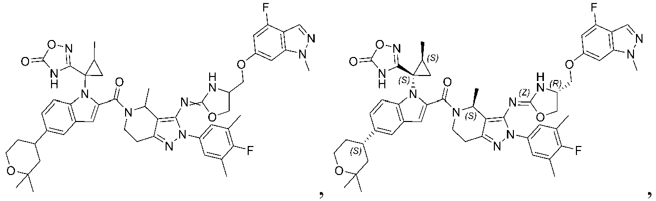 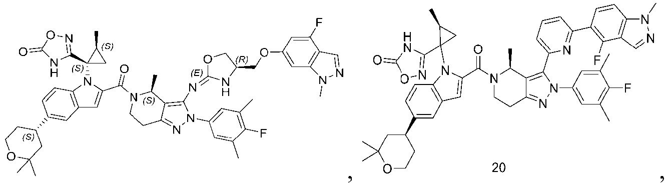 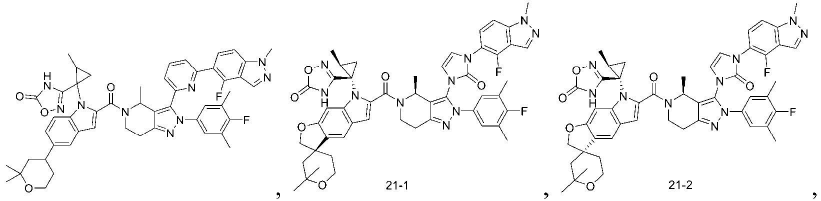 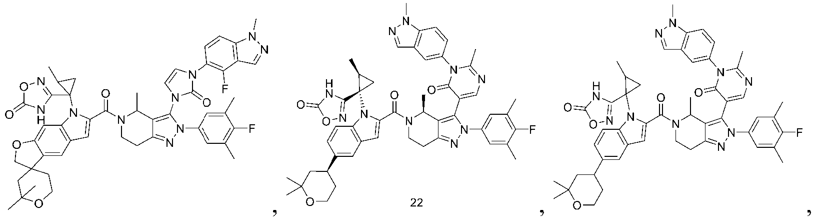 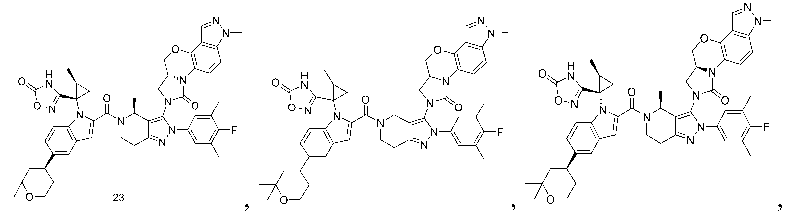 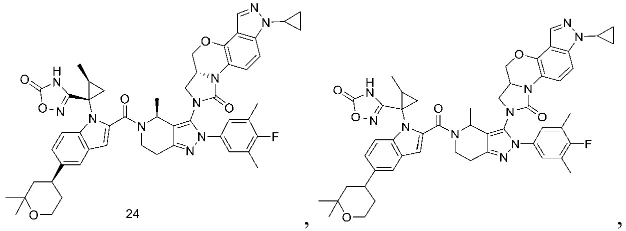 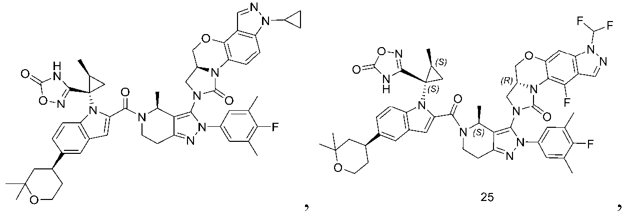 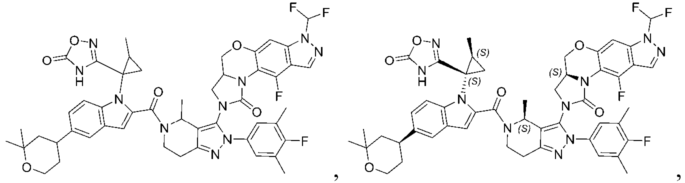 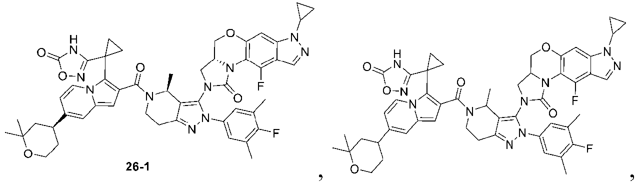 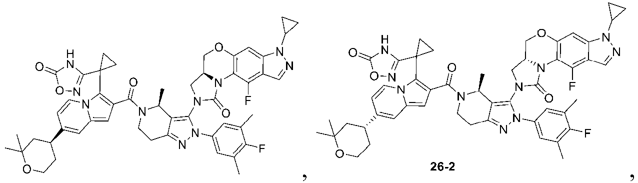 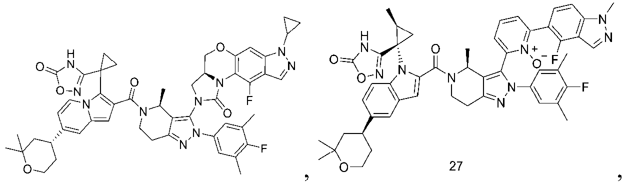 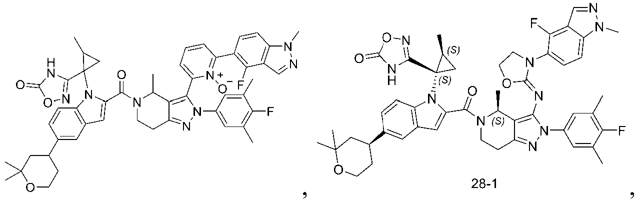 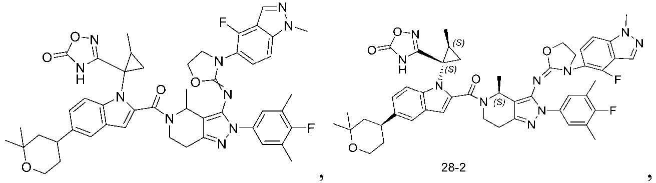 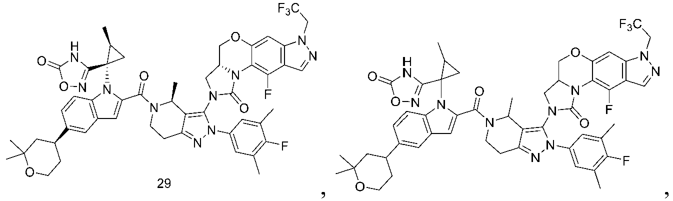 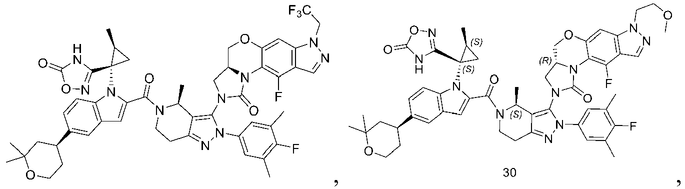 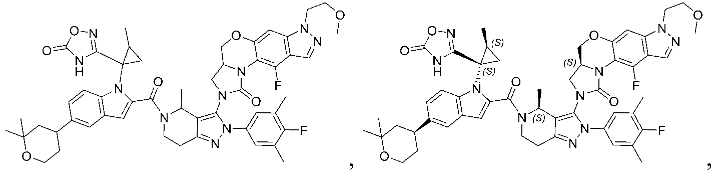 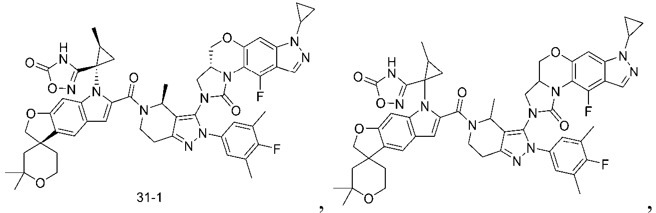 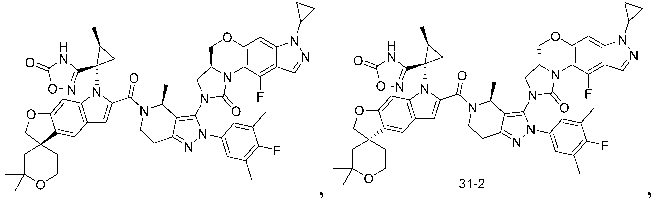 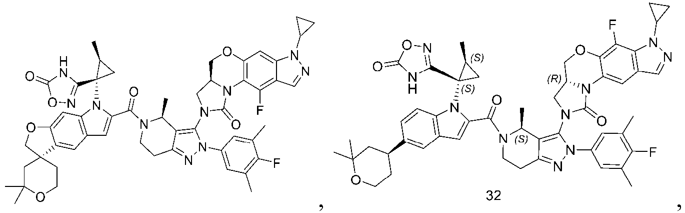 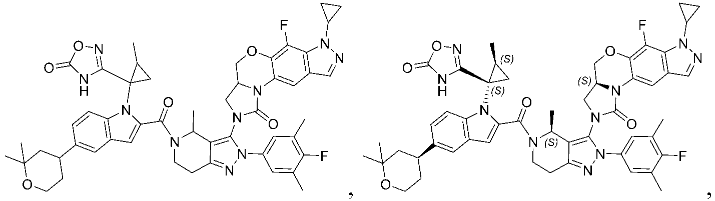 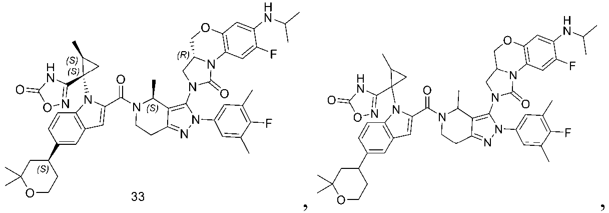 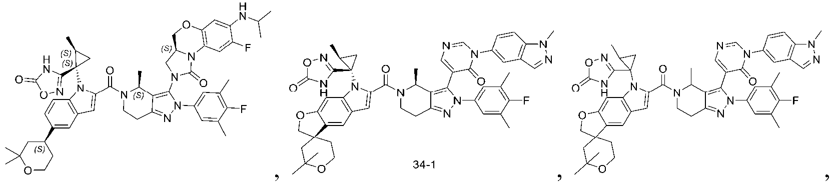 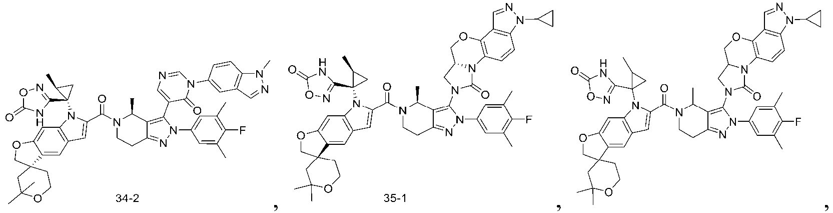 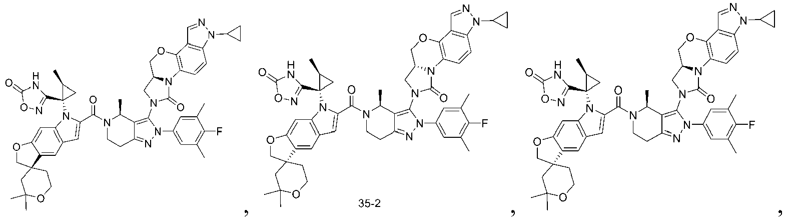 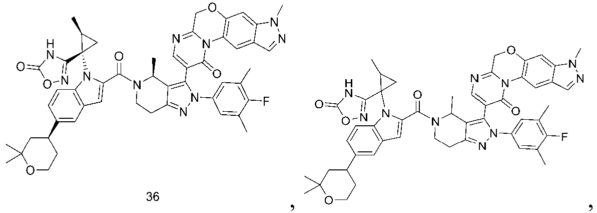 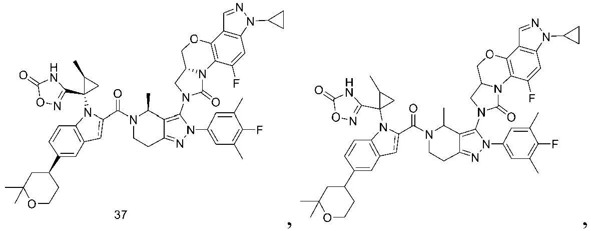 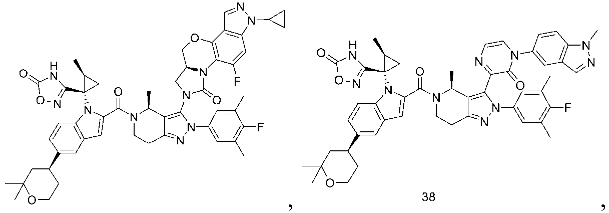 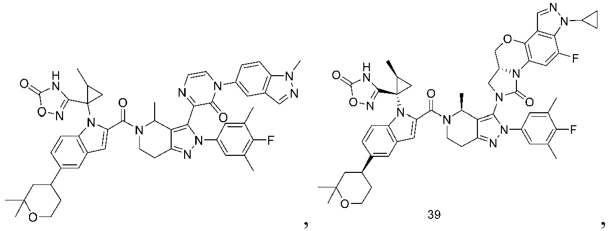 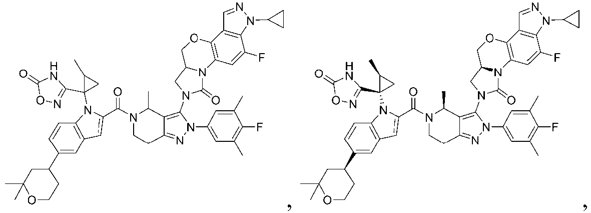 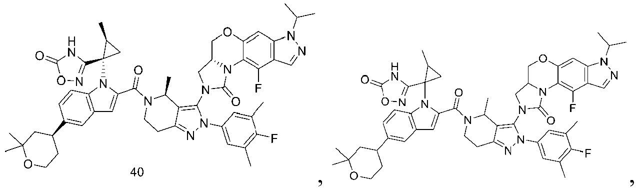 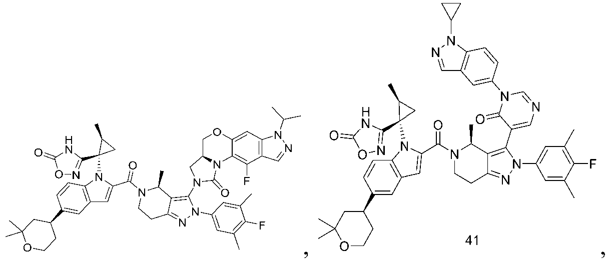 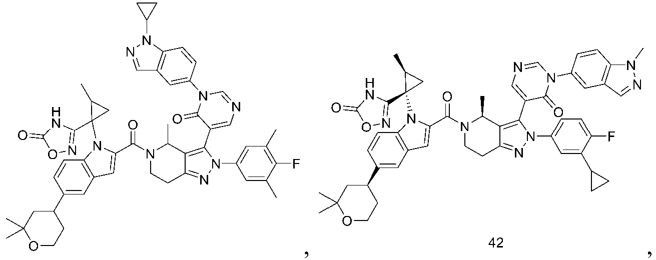 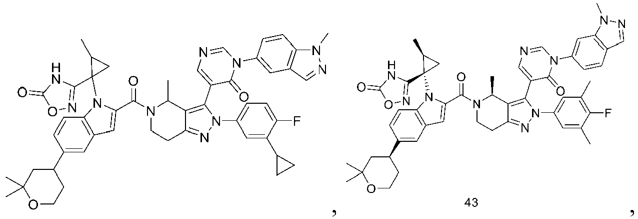 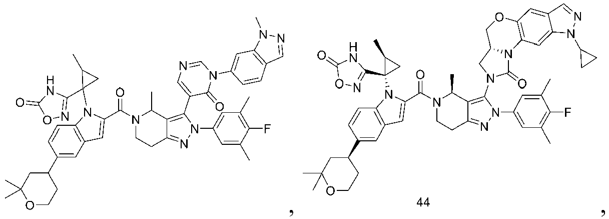 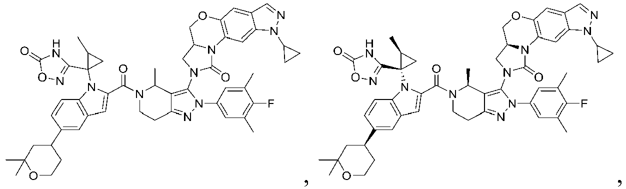 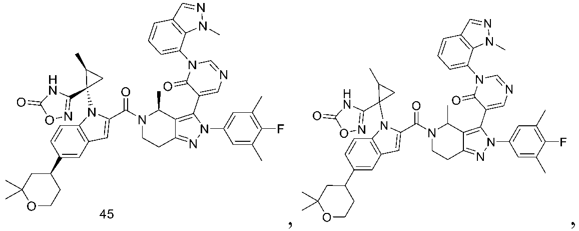 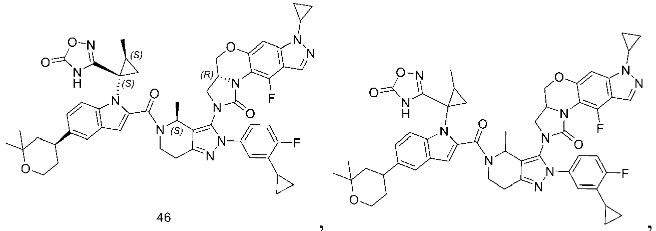 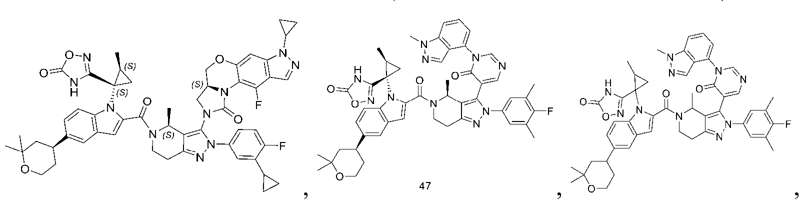 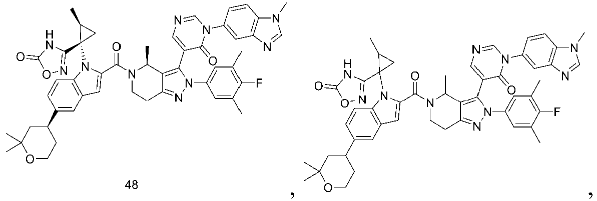 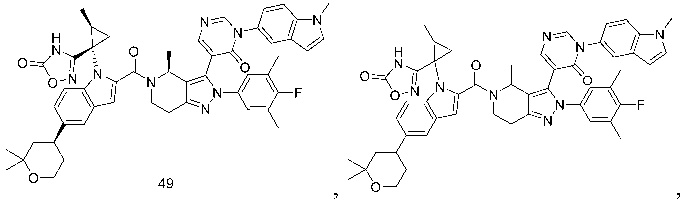 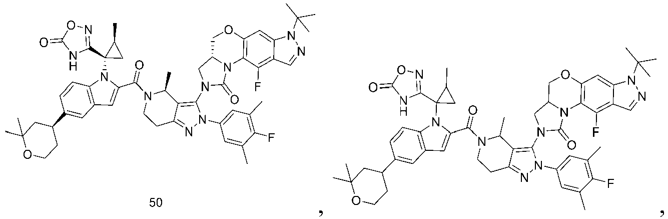 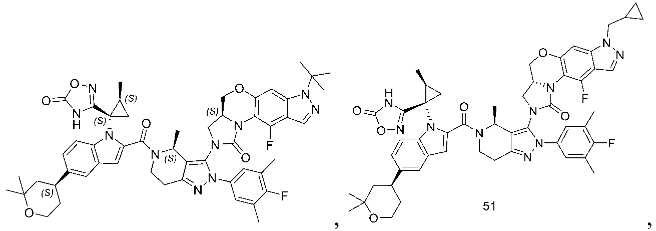 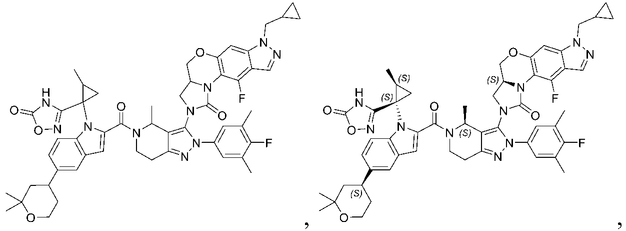 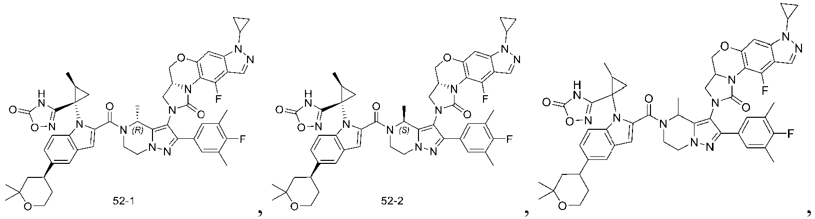 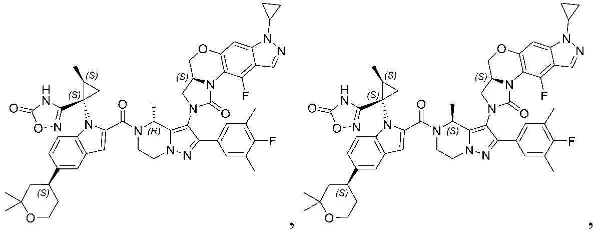 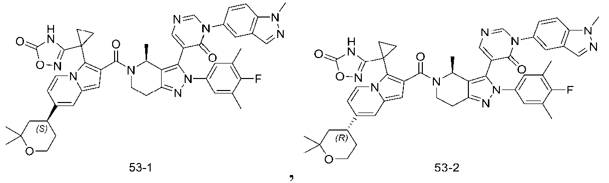 and

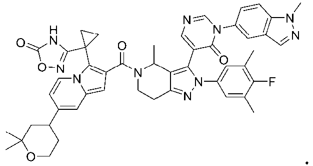

16. A compound represented by general formula (IA) or a salt thereof: 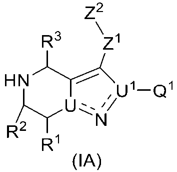 **characterized in that** -̅ -̅ -̅, 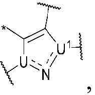 Q¹, Z¹, Z², R¹, R², and R³ are as defined in claim 1.

17. A compound represented by general formula (IVB) or (IV'B) or a salt thereof: 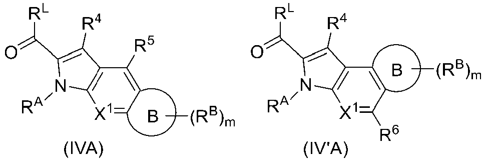
**characterized in that** R^{L} is selected from the group consisting of halogen, hydroxyl, and alkoxy; preferably hydroxyl; and
ring B, X¹, R^{A}, R⁴, R⁵, R⁶, R^{B}, and m are as defined in claim 8.

18. A compound represented by general formula (INA) or a salt thereof: 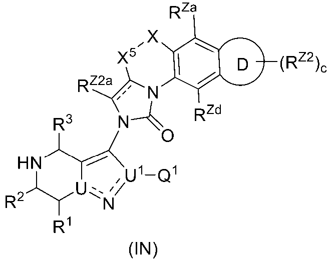 **characterized in that** 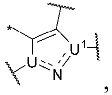 Q¹, R¹, R², R³, R^{Z2a}, -̅ -̅ -̅, X, X⁵, R^{Za}, R^{Zd}, ring D, R^{Z2}, and c are as defined in claim 2.

19. A compound or a salt thereof, **characterized in that** the structure of the compound or the salt thereof is as follows:

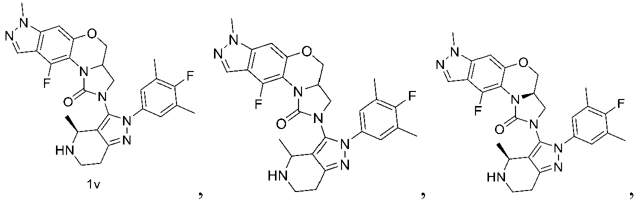

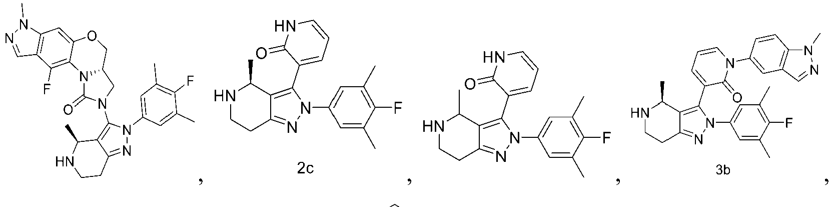

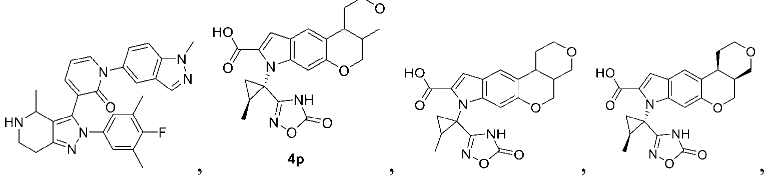

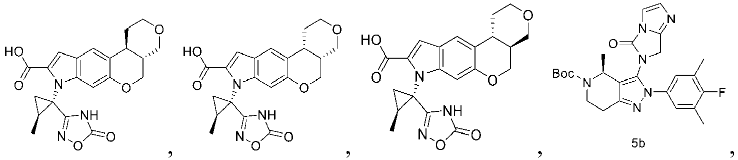

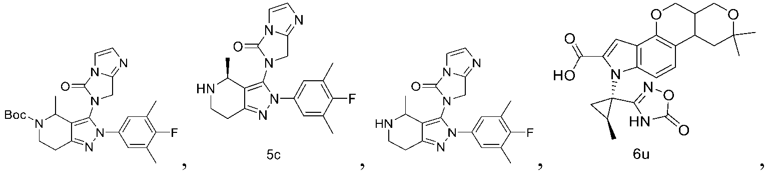

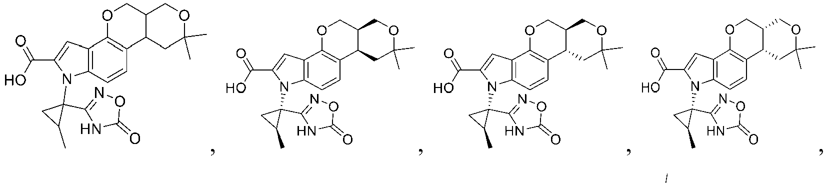

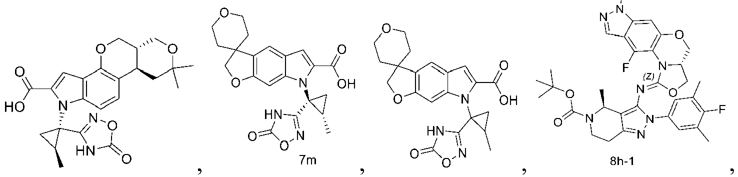

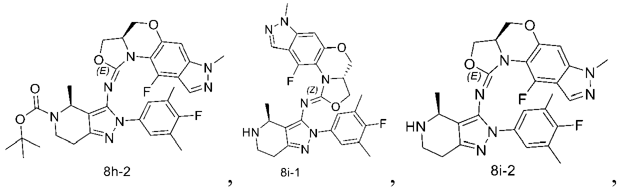

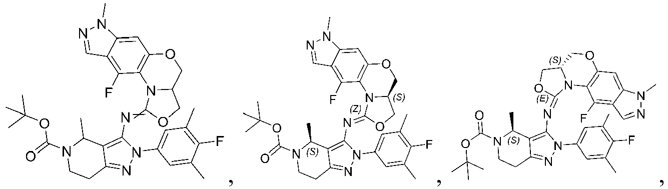

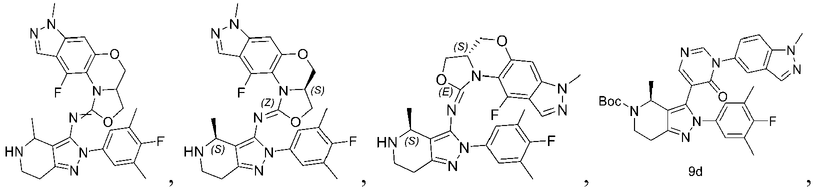

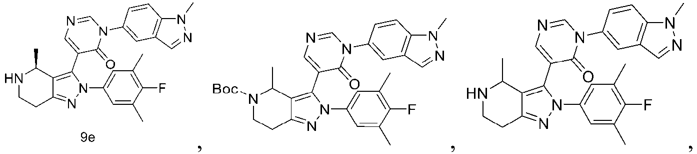

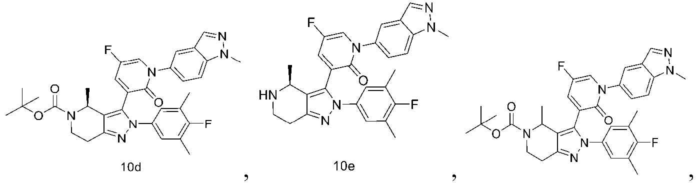

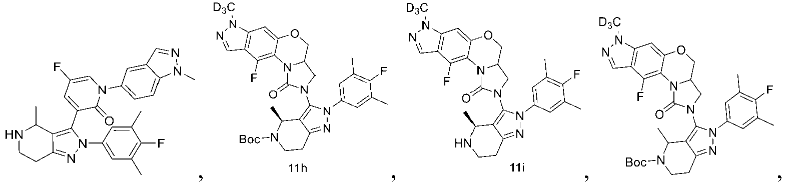

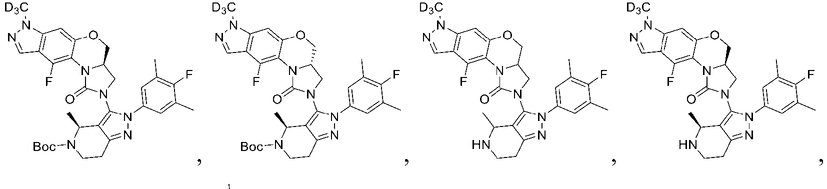

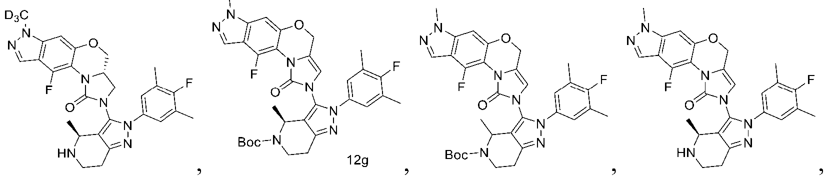

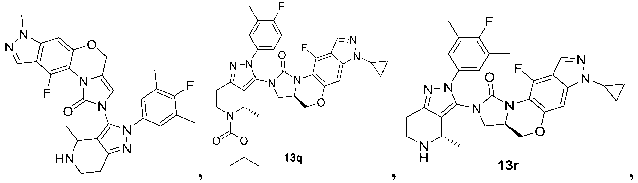

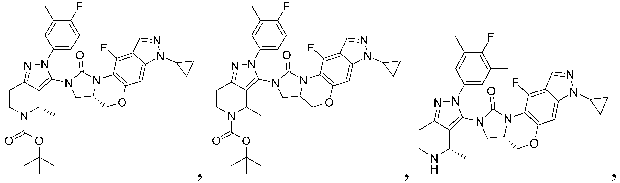

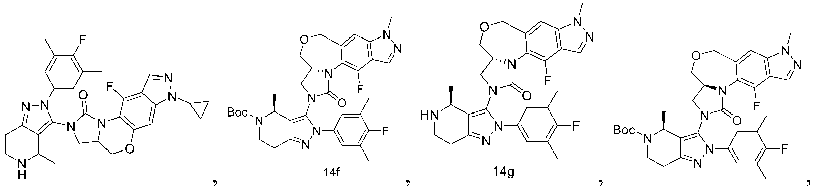

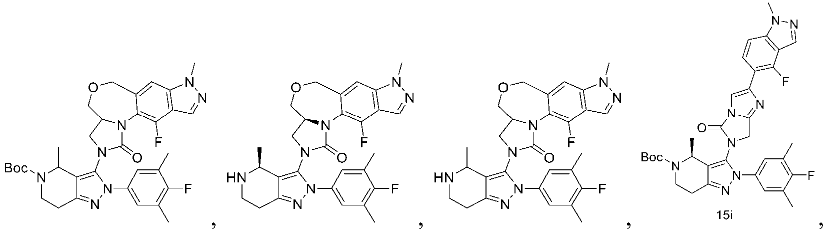

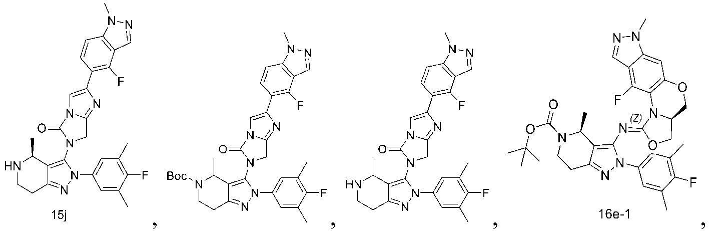

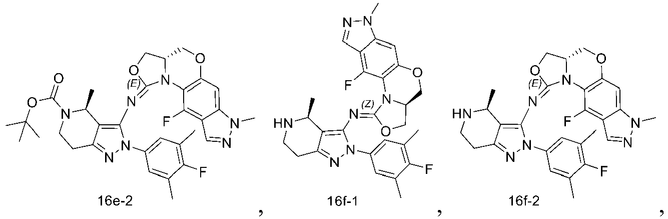

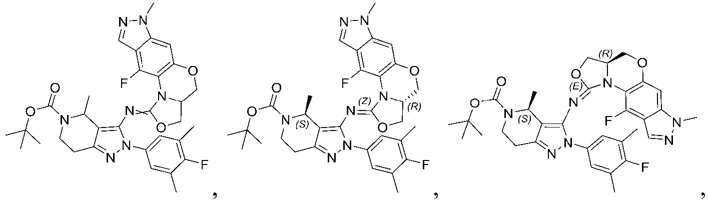

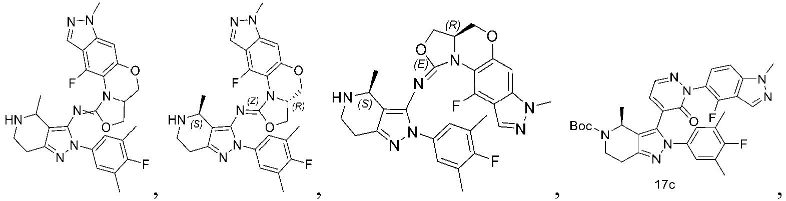

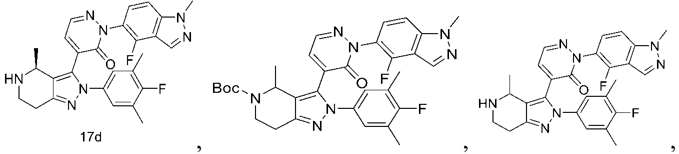

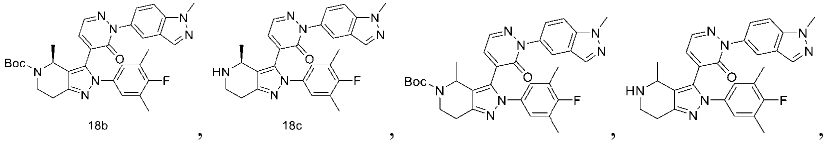

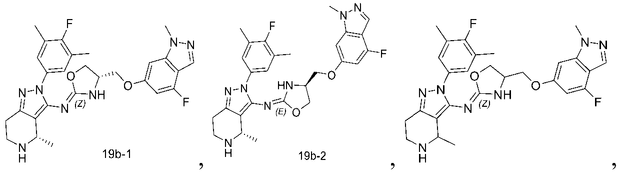

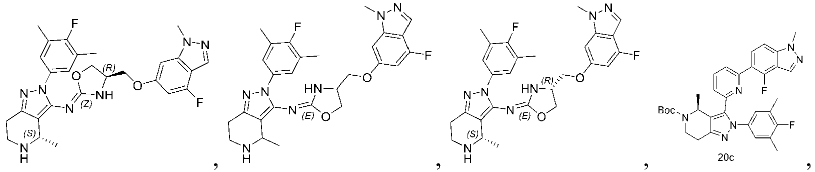

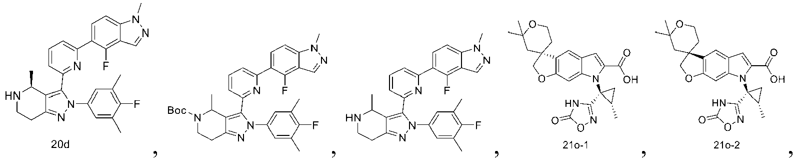

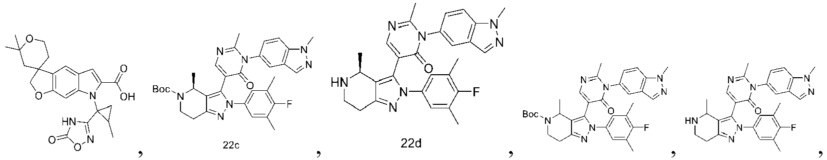

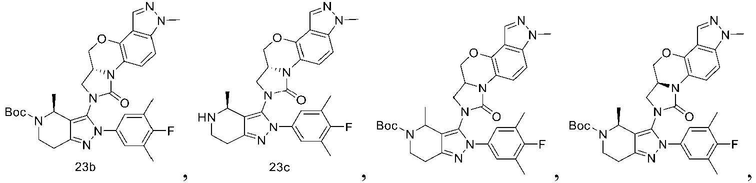

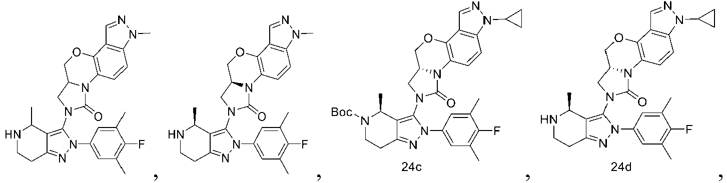

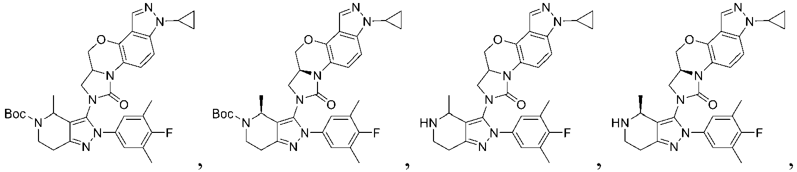

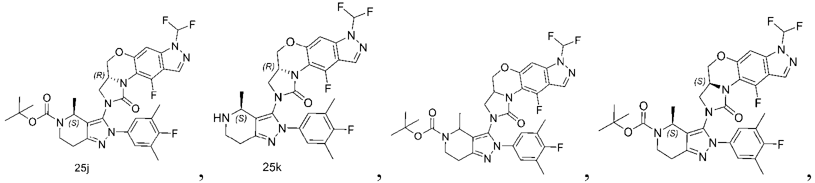

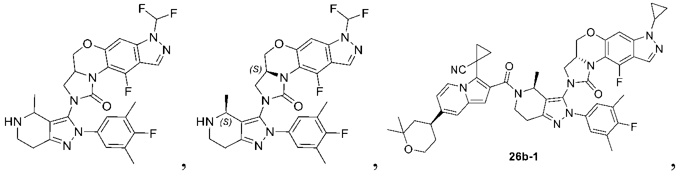

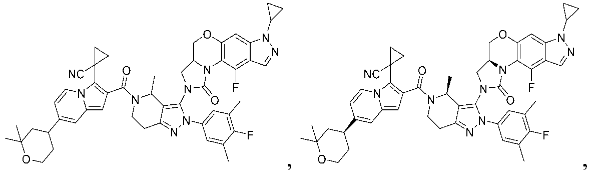

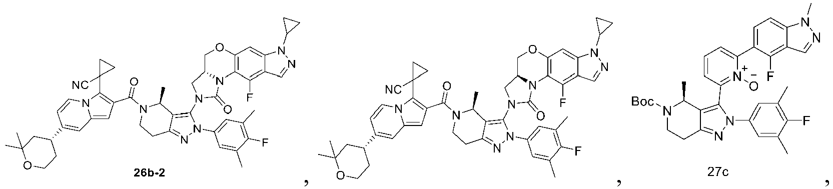

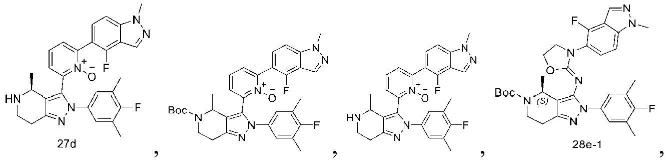

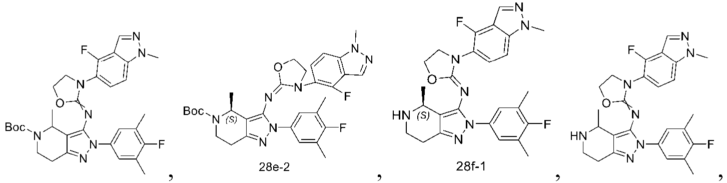

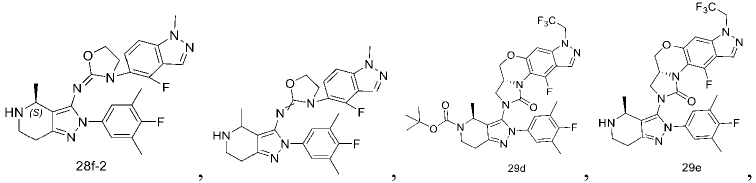

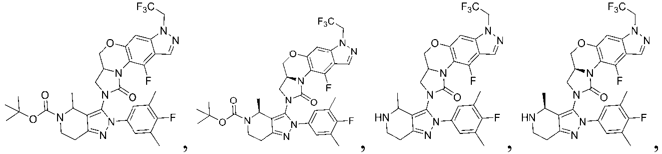

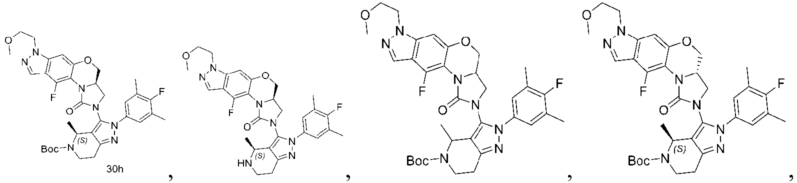

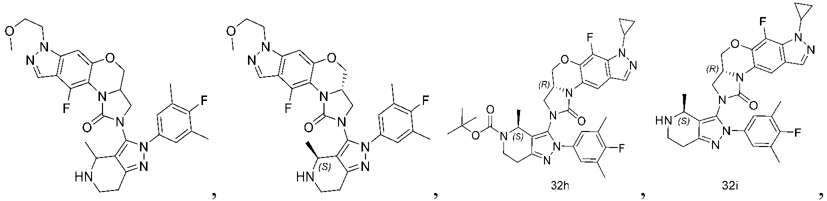

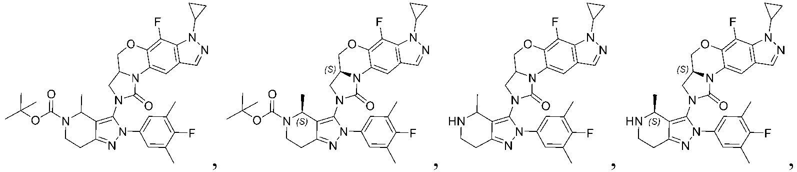

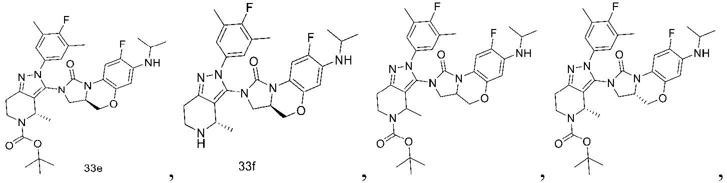

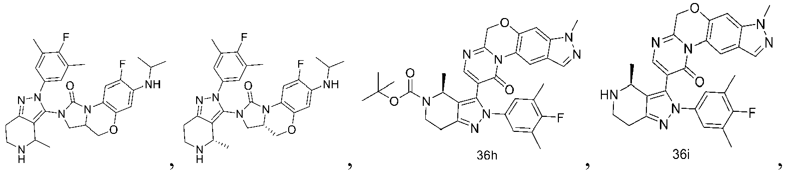

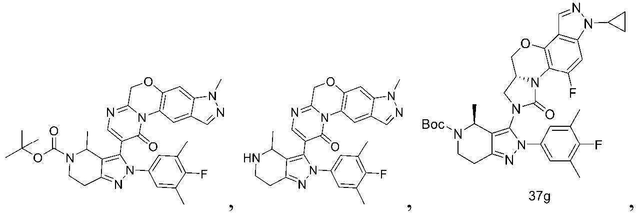

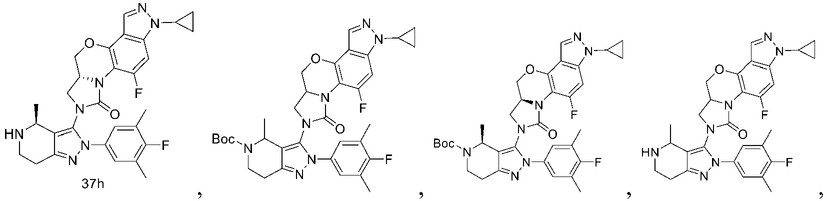

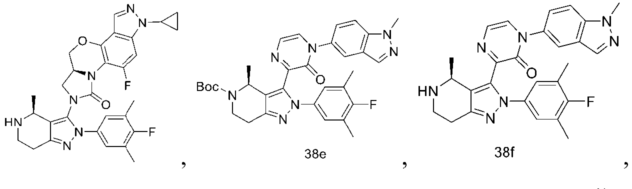

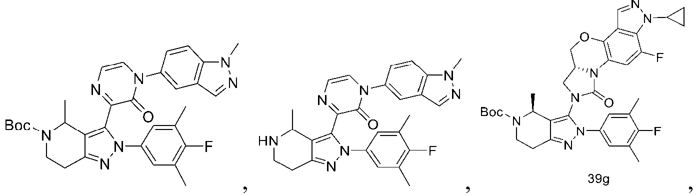

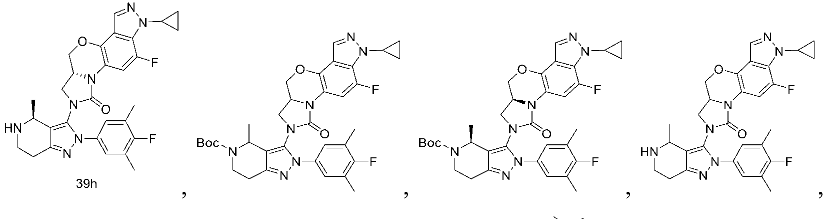

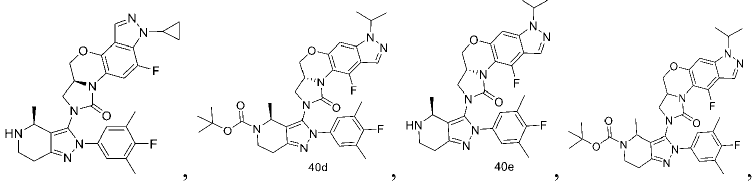

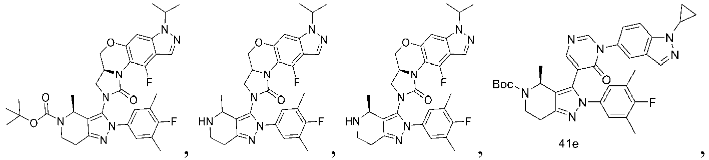

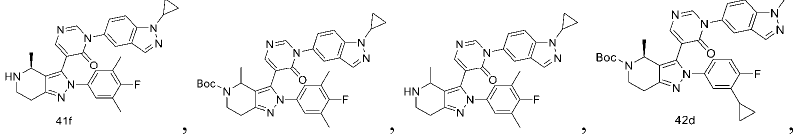

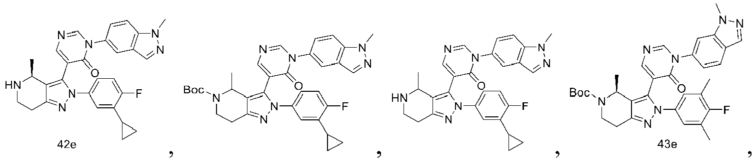

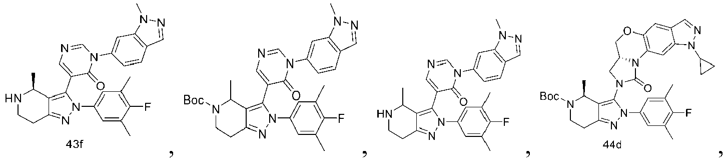

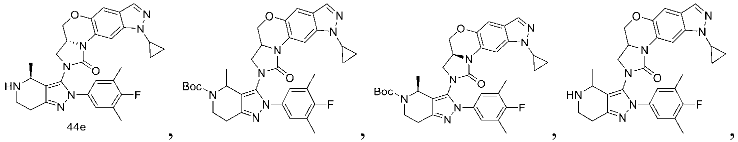

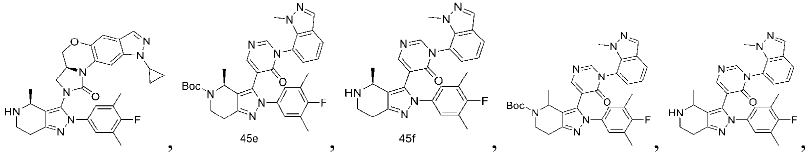

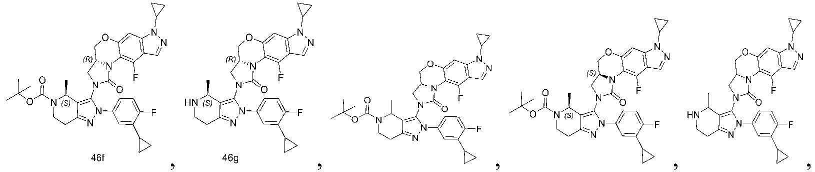

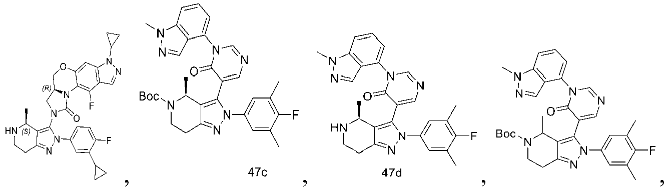

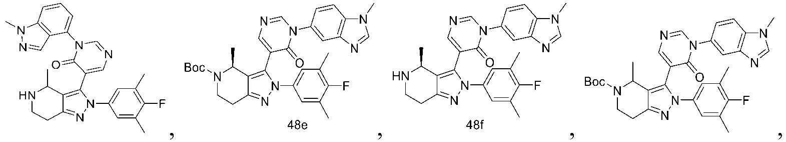

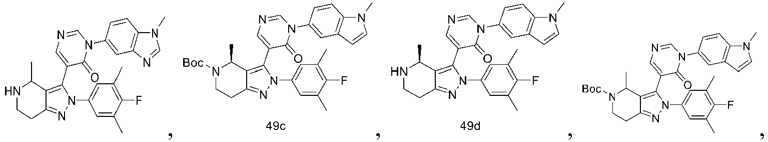

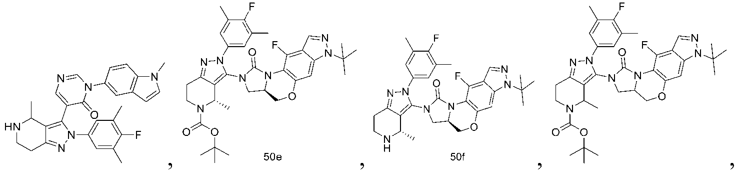

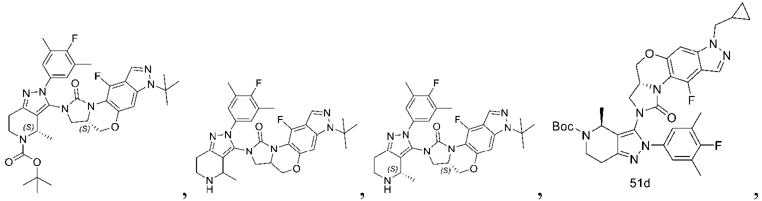

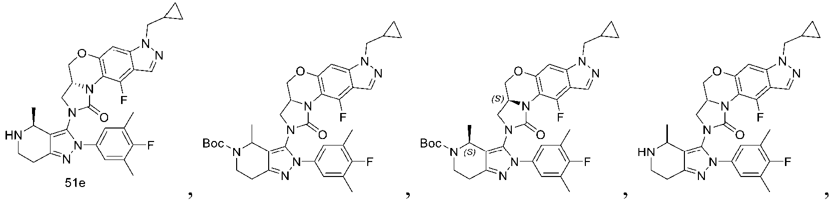

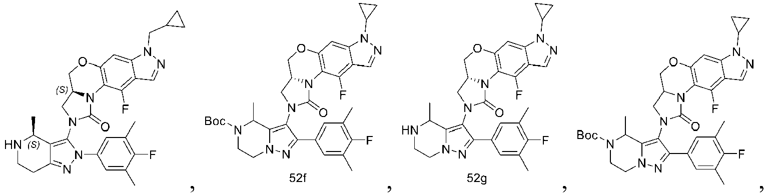

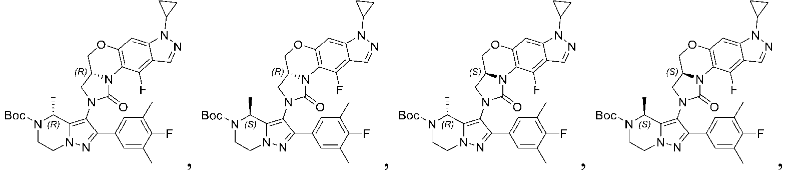

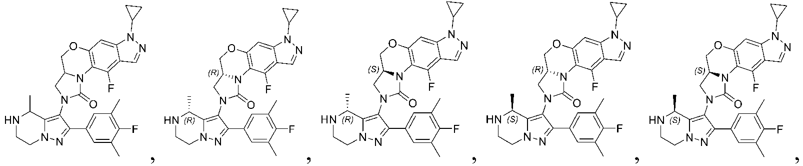

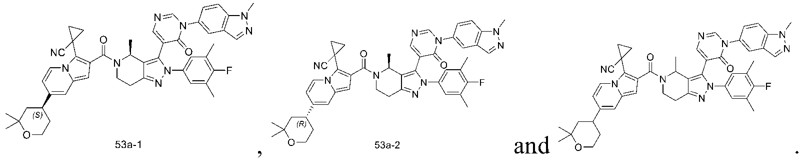

20. A method for preparing the compound represented by general formula (I) or the pharmaceutically acceptable salt thereof according to claim 1, **characterized in that** the method comprises: 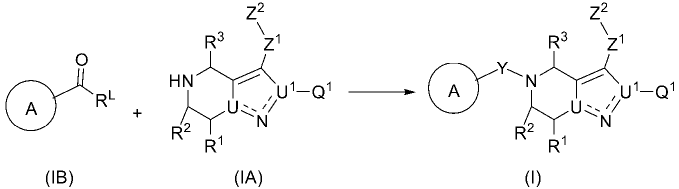
subjecting a compound represented by general formula (IA) or a salt thereof and a compound represented by general formula (IB) or a salt thereof to a condensation reaction to obtain the compound represented by general formula (I) or the pharmaceutically acceptable salt thereof,
wherein R^{L} is selected from the group consisting of halogen, hydroxyl, and alkoxy, preferably hydroxyl; Y is C(O); and 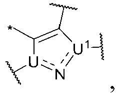 ring A, Q¹, Z¹, Z², R¹, R², and R³ are as defined in claim 1.

21. A pharmaceutical composition, **characterized in that** the pharmaceutical composition comprises the compound or the pharmaceutically acceptable salt thereof according to any one of claims 1 to 15, and one or more pharmaceutically acceptable carriers, diluents or excipients.

22. Use of the compound or the pharmaceutically acceptable salt thereof according to any one of claims 1 to 15 or the pharmaceutical composition according to claim 21 in the preparation of a medicament for agonizing a GLP-1 receptor.

23. Use of the compound or the pharmaceutically acceptable salt thereof according to any one of claims 1 to 15 or the pharmaceutical composition according to claim 21 in the preparation of a medicament for treating and/or preventing type I diabetes, type II diabetes, malnutrition-related diabetes, diabetic complications, obesity, hyperglycemia, glucose intolerance, cardiovascular diseases, hyperlipidemia, cerebral infarction, stroke, non-alcoholic steatohepatitis (NASH), Parkinson's disease, dementia, glaucoma, diarrhea, insulin resistance, and liver insulin resistance; and preferably in the preparation of a medicament for treating and/or preventing type I diabetes, type II diabetes, obesity, diabetic complications, non-alcoholic steatohepatitis, and cardiovascular diseases.

24. Use of the compound or the pharmaceutically acceptable salt thereof according to any one of claims 1 to 15 or the pharmaceutical composition according to claim 21 in the preparation of a medicament for treating and/or preventing idiopathic type I diabetes, latent immune diabetes in adults (LADA), maturity-onset diabetes of the young (MODY), gestational diabetes, non-alcoholic fatty liver disease (NAFLD), atherosclerosis, hypertension, and coronary heart disease.
